# EUROPEAN PATENT SPECIFICATION

(11) **EP 1 780 267 B1**
(45) Date of publication and mention of the grant of the patent: **13.01.2016**
(21) Application number: 06252751.0
(22) Date of filing: 26.05.2006
(51) Int. Cl.: C12N 5/073

(54) **Amniotic fluid derived cells**
Zellen aus Fruchtwasser
Cellules isolés de liquide amniotique

(30) Priority: 27.05.2005 US 685607 P; 27.03.2006 US 743821 P
(43) Date of publication of application: 02.05.2007
(60) Divisional application: 10184689.7
(73) Proprietor: LifeScan, Inc., Milpitas, CA 95035 (US)
(72) Inventor: Rezania, Alireza, Hillsborough, NJ 08844 (US); Xu, Jean, Hillsborough, NJ 08844 (US)
(74) Representative: Wise, Daniel Joseph

(56) References cited:
- EP-A1- 1 391 505
- WO-A-2005/017117
- WEI JUN PING ET AL: "Human amnion-isolated cells normalize blood glucose in streptozotocin-induced diabetic mice." CELL TRANSPLANTATION, vol. 12, no. 5, 2003, pages 545-552, XP009085264 ISSN: 0963-6897
- CHEN LI-BO ET AL: "Differentiation of rat marrow mesenchymal stem cells into pancreatic islet beta-cells" WORLD JOURNAL OF GASTROENTEROLOGY, XX, XX, vol. 10, no. 20, 15 October 2004 (2004-10-15), pages 3016-3020, XP002363979 ISSN: 1007-9327
- HELD K R ET AL: "THE EFFECT OF OXYGEN TENSION ON COLONY FORMATION AND CELL PROLIFERATION OF AMNIOTIC FLUID CELLS IN-VITRO" PRENATAL DIAGNOSIS, vol. 4, no. 3, 1984, pages 171-180, XP009085380 ISSN: 0197-3851
- TSAI MING-SONG ET AL: "Isolation of human multipotent mesenchymal stem cells from second-trimester amniotic fluid using a novel two-stage culture protocol" HUMAN REPRODUCTION, IRL PRESS, OXFORD, GB, vol. 19, no. 6, June 2004 (2004-06), pages 1450-1456, XP002404972 ISSN: 0268-1161
- IN 'T ANKER PIETERNELLA S ET AL: "Amniotic fluid as a novel source of mesenchymal stem cells for therapeutic transplantation." BLOOD 15 AUG 2003, vol. 102, no. 4, 15 August 2003 (2003-08-15), pages 1548-1549, XP009085337 ISSN: 0006-4971
- FAUZA D: "Amniotic fluid and placental stem cells" BAILLIERE'S BEST PRACTICE AND RESEARCH. CLINICAL OBSTETRICS AND GYNAECOLOGY, BAILLIERE TINDALL, LONDON, GB, vol. 18, no. 6, December 2004 (2004-12), pages 877-891, XP004676339 ISSN: 1521-6934

## Description

### FIELD OF THE INVENTION

This invention relates to an expandable population of amniotic fluid-derived cells that can be differentiated into a β-cell lineage. Also disclosed are methods for isolating and expanding such amniotic fluid-derived cells, as well as related methods and compositions for utilizing such cells in the therapeutic treatment of diabetes.

### BACKGROUND

Loss of organ function can result from congenital defects, injury or disease. One example of a disease causing loss of organ function is diabetes mellitus, or diabetes. Most cases of diabetes fall into two clinical types: Type 1, also known as juvenile-onset diabetes, or insulin dependent diabetes mellitus (IDDM), and Type 2, also known as adult-onset diabetes. Each type has a different prognosis, treatment, and cause. Both types are characterized by the patient's inability to regulate their blood glucose levels. As a consequence, blood glucose levels rise to high values because glucose cannot enter cells to meet metabolic demands. This inability to properly metabolize blood sugar causes a complex series of early and late-stage symptomologies, beginning with, for example, hyperglycemia, abnormal hunger, thirst, polyuria, and glycouria, and then escalating to, for example, neuropathy, macro-vascular disease, and micro-vascular disease.

A common method of treatment of Type 1 diabetes involves the exogenous administration of insulin, typically by injection with either a syringe or a pump. This method does not completely normalize blood glucose levels and is often associated with an increased risk of hypoglycemia. More effective glycemic control can be achieved if the function of the pancreas can be restored or rejuvenated via transplantation or cell-based therapies.

There are many transplantation therapies currently used to treat diabetes: One such treatment involves transplanting isolated islets of Langerhans into the diabetic patient. One of the main hurdles to human islet transplantation has been the lack of sufficient number of islets to treat the large number of diabetic patients. One possible solution to the shortage of islets is the generation of islets from alternate cellular sources.

It has been documented that progenitor cells derived from adult tissues are capable of differentiation into a pancreatic β-cell phenotype. See, for example, WO2004/087885 A2, Hess et al. (Nature Biotechnology 21, 763 - 770, 2003), and Ianus et al. (J. Clin. Invest. 111: 843-850, 2003), which report the capacity of adult bone marrow-derived cells (mesenchymal and hematopoetic cells) to differentiate into cells having characteristics of a pancreatic β-cell *in vitro,* or secrete trophic factors that help regenerate a damaged pancreas *in vivo.*

Among other sources of progenitor cells that can be differentiated into pancreatic cells include rodent liver oval stem cells (WO03/033697) and post-partum placenta (U.S. Published Application 2004/0161419 A1).

The endocrine cells of the islets of Langerhans, including β-cells, are constantly turning over by processes of apoptosis and the proliferation of new islet cells (neogenesis). As such, the pancreas is thought to be a source of progenitor cells that are capable of differentiating into pancreatic hormone producing cells. There are three distinct tissue types, isolated from a pancreas, that are a potential source of pancreatic progenitor cells: an islet rich fraction, a ductal cell rich fraction, and an acinar cell rich fraction.

Isolation of progenitor cells or partially differentiated cells from crude pancreatic tissue extracts may be achieved using antibodies raised against cell surface markers. For example, U.S. Published Application 2004/0241761 discloses isolation of murine cells that expressed ErbB2, ErbB3, ErbB4, Msx-2, PDX-1 and insulin.

Gershengorn et al. (Science 306: 2261-2264, 2004) teach the production of proliferating cells that were able to form islet-like cell aggregates. The cells were derived from a heterogeneous population of adherent cells that emerged from the culture of isolated human pancreatic islets *in vitro.* The isolated islets of Langerhans were initially seeded onto tissue culture dishes and cultured in medium containing 10% serum. Fibroblast-like cells were observed to migrate out of the cultured islets and form a monolayer. These cells expressed Nestin, smooth muscle actin and vimentin.

Pancreatic progenitor cells may also arise from the culture of pancreatic islet and ductal tissue that has been dissociated into single cells, as disclosed by Seaberg et al. (Nature Biotechnology 22: 1115 - 1124, 2004). The murine progenitor cells disclosed by Seaberg *et al.* expressed Nestin during proliferation.

U.S. Published Application 2003/0082155 discloses methods to isolate and identify a population of cells from the islets of Langerhans of human pancreas, which have the functional and molecular characteristics of stem cells. In particular, these cells were characterized by Nestin-positive staining, Nestin gene expression, GLP-1R-positive staining, GLP- 1R gene expression, ABCG2 positive staining, ABCG2 gene expression, Oct3/4 positive staining, Oct3/4 gene expression, latrophilin (type 2) positive staining, latrophilin (type 2) gene expression, Hes-1 positive staining, Hes-1 gene expression, Integrin subunits α6 and β1 positive staining, Integrin subunits α6 and β1 gene expression, c-kit positive staining, c-kit gene expression, MDR-1 positive staining, MDR-1 gene expression, SST-R, 2, 3, 4 positive staining, SST-R, 2, 3, 4 gene expression, SUR-1 positive staining, SUR-1 gene expression, Kir 6.2 positive staining, Kir 6.2 gene expression, CD34 negative staining, CD45 negative staining, CD133 negative staining, MHC class I negative staining, MHC class II negative staining, cytokeratin-19 negative staining, long-term proliferation in culture, and the ability to differentiate into pseudo-islets in culture.

In another approach, as disclosed in U.S. Patent 5,834,308, U.S. Patent 6,001,647 and U.S. Patent 6,703,017, crude preparations of islet cultures from NOD mice may be used to establish epithelial-like cultures, which can be maintained in growing cultures for greater than 1 year and which appear to demonstrate the ability to differentiate into islet-like clusters, capable of secreting insulin.

Islet-like structures may be generated from fractions of digested human pancreata enriched for ductal tissue, as disclosed in Bonner-Weir et al. (Proc Nat Acad Sci 97: 7999-8004, 2000) and U.S. Patent 6,815,203 B1. Islet-like clusters disclosed in these publications stained positive for cytokeratin-19 and showed immunoreactivity for insulin.

WO2004/011621 discloses the generation of insulin negative adherent cells from human pancreatic ductal fragments.

WO03/102134 discloses the generation of an epithelial cell positive for cytokeratin-19 from an acinar fraction of a human pancreatic digest. The cells generated are capable of limited expansion and differentiate into an insulin-producing cell in the presence of an induction media.

U.S. Published Application 2004/015805 A1 reports that a subset of human pancreatic stem cells may be isolated using ligands to the cell surface marker CD56 (also known as NCAM). These cells can differentiate into insulin producing cells and insulin producing aggregates.

It has been documented that progenitor cells, derived from fetal or embryonic tissues, have the potential to differentiate into a pancreatic hormone-producing cell. See, for example, U.S. Patent 6,436,704, WO03/062405, WO02/092756 and EP 0 363 125 A2, which report the potential of human fetal and embryonic derived cells to differentiate into a β-cell lineage.

Human Embryonic Stem cells (hES) are derived from the inner cell mass of the blastocyst, the earliest stage of embryonic development of the fertilized egg. The blastocyst is a pre-implantation stage of the embryo, a stage before the embryo would implant in the uterine wall. When cultured on an inactivated feeder layer of cells according to conditions described by Thompson and colleagues (Thomson, et al. (Proc. Natl. Acad. Sci. U.S.A. 92: 7844-7848, 1995); Thomson, et al. (Science 282:1145-1147, 1998), Marshall, et al., (Methods Mol. Biol. 158:11-18, 2001), the inner layer cells of the blastocyst may be grown *in vitro* indefinitely in an undifferentiated state. Properly propagated hES cells have unlimited potential to double while maintaining their pluiripotency; namely their capacity of differentiating into the three layers of the embryo, Ectoderm (Ec), Mesoderm (Me) and Endoderm (En). When grown as pluripotent hES, the cells maintain a euploid karyotype and are not prone to senescence.

Human embryonic stem cells display a distinct group of cell surface antigens, SSEA-3, SSEA-4, TRA-2-54 (alkaline phosphatase), TRA-1-60 and TRA-1-81, in addition to expressing specific transcription factors OCT-4, NANOG, SOX-2, FGF-4 and REX-1 (Henderson, et al., (Stem Cells 20:329-337, 2002), Draper, et al., (J. Anat. 200:249-258, 2002), Mitsui et al., (Cell 113:631-642, 2003), Chambers et al., (Cell 113:643-655, 2003).

It is important to note from these publications, however, that human embryonic cells often require a feeder layer for expansion and maintenance of pluripotency or combination of a complex extracellular matrix, such as, for example, MATRIGEL™, plus conditioned media. These conditions do not allow the facile scale up of cells and an eventual cell therapy for treating diabetes.

Researchers have found that non-embryonic types of stem cells ("adult stem cells") are not as capable of differentiating into many different tissue types, as are embryonic stem cells, so embryonic stem cells still have many advantages over the use of adult stem cells. However, one obstacle with the isolation of embryonic stem cells is that the cells are derived from embryos at the "blastocyst" stage. Human embryonic stem cell research is encumbered by an emotionally charged political and ethics debate and is likely to remain so for years to come.

Additionally, human embryonic stem cells (hES) have been found to be tumorigenic when injected into immunologically impaired animals, i.e. in the context of post-natal tissues, whereas adult stem cells are not. The tumorigenic attributes of hES cells are not frequently addressed, though this issue may burden their use in replacement cell therapy in the future. The political, moral and ethical issues around hES cells and their tumorigenic properties, as well as the perceived difficulties of expanding undifferentiated adult stem cells in culture, while maintaining a genetically normal genome, are major barriers in the development of human cell replacement therapy.

Pluripotent or multipotent stem cells have been isolated from chorionic villus, and amniotic fluid. Many amniotic and placental cells share a common origin, namely the inner cell mass of the morula, which gives rise to the embryo itself, the yolk sac, the mesenchymal core of the chorionic villi, the chorion and the amnion (Crane & Cheung, Prenatal Diagnosis 8: 119-129, 1988). Embryonic and fetal cells from all three germ layers have long been identified in the amniotic fluid (Milunsky, Genetic Disorder of the Fetus. New York: Plenum Press, 75-84, 1979; Hoehn & Salk, Methods in Cell Biology 26, 11-34, 1982; Gosden, British Medical Bulletin 39, 348-354, 1983; Prusa et al, Human Reproduction 18, 1489-1493, 2003). Thus, amniotic fluid may provide the least invasive access to embryonic-like and fetal-like stem cells.

Amniotic fluid derived cells have been routinely used for detecting chromosomal abnormality of the fetus. Amniotic fluid is typically sampled during the 2nd trimester (16 to 22 weeks of gestation). Previous art clearly demonstrates presence of three subpopulation with distinct cell morphologies: "fibroblastic" (F), "amniotic fluid" (AF) cells, and "epithelial" (E) cells. The F and AF cells rapidly expand whereas the E cells display a much slower growth curve and have poor clonal efficiency.

For example, PCT application WO2003/042405 discloses isolation of c-Kit positive stem cells from chorionic villus, amniotic fluid and placenta (Cell 1, **Table I**).

In another example, U.S. Published Application 2005/0054093 discloses the isolation of stem cells from amniotic fluid. These cells express stage-specific embryonic antigen 3 (SSEA3), stage-specific embryonic antigen 4 (SSEA4), Tral-60, Tral-81, Tra2-54, Oct-4, HLA class I, CD13, CD44 CD49b and CD105 (Cell 2, **Table I**).

In another example, fetal cells have been isolated from amniotic fluid (in't Anker et al, Blood 102, 1548-1549, 2003). The cells disclosed were positive for expression of the following markers: CD44, CD73, CD90, CD105, CD106, HLA-A,B, & C. The cells were negative for expression of the following markers: c-Kit (CD117), CD11, CD31, CD34, CD45 and HLA-D (Cell 3, **Table I**).

A population of mesenchymal stem cells isolated from amniotic fluid has also been reported in a publication to Tsai *et al* (Tsai et al, Human Reproduction 19, 1450-1456, 2004). The cells disclosed were positive for expression of the following markers: CD29, CD44, CD73, CD90, HLA-A,B, & C. The cells were also positive for the embryonic transcription factor Oct-4. The cells were negative for expression of the following markers: c-Kit (CD117), CD34 and HLA-D (Cell 4, **Table I**).

Although recent publications and patents have suggested that within the fibroblastic, amniotic fluid, or epithelial subpopulations there exists a cell population that display some characteristics of human embryonic cells, such as expression of surface markers SSEA3 and -4, expression of transcription factor Oct-4, strong expansion potential, and differentiation into multiple cell types; none of the previously published art has demonstrated the existence of a subpopulation of the cells that display expression of key early endodermal markers, such as HNF-1 beta, HNF-3 beta, SOX-17, and GATA-6, while maintaining expression of ES markers SSEA-4.

Co expression of HNF-1 beta, HNF-3 beta (also known as FOXa2), SOX-17, and GATA-6 is regarded as the key step to define the formation of definitive endoderm during gastrulation. Thus, expression of these markers may be key in the generation of a pancreatic β-cell population, or a population of pancreatic hormone-producing cells, or a gut hormone-producing cell from an amniotic fluid-derived cell.

Therefore, there still remains a significant need to develop culture conditions for establishing amniotic fluid-derived cell lines that can be expanded to address the current clinical needs, while retaining the potential to differentiate into definitive endoderm, or a population of pancreatic hormone-producing cells, or a gut hormone-producing cell, or a β-cell lineage.

Wei Jun Ping et al. (Cell Transplantation, 2003,vol. 12(5), p545-552) disclose human amnion-isolated cells that can normalize blood glucose in streptozotocin-induced diabetic mice. Chen Li-Bo et al. (World J. Gastroenterology, 2004, vol. 10(20), p3016-3020) disclose the differentiation of rat marrow mesenchymal stem cells into pancreatic islet beta-cells. Held KR et al. (Prenatal Diagnosis, 1984, vol. 4(3), p171-180) disclose the effect of oxygen tension on colony formation and cell proliferation of amniotic fluid cells in vitro. Fauza D (Bailliere's Best Practice and Research: Clinical Obstetrics and Gynaecology, 2004, vol. 18(6), p877-891) reviews the amniotic fluid and the placenta as unique sources of different populations of stem cells--mesenchymal, hematopoietic, trophoblastic--and, possibly, of more primitive stem cells.

WO 2005/017117 discloses a source of multipotent amniotic fluid/fetal stem cells (MAFSCs). MAFSC are of fetal origin and are characterized by the following cell surface markers: SSEA3, SSEA4, Tra-1-60, Tra-1-81, Tra-2-54, HLA class I, CD13, CD44, CD49b, CD 105 and are distinguished by the absence of the antigen markers CD34, CD45, and HLA Class II.

EP1391505A1 relates to a method for separating pancreatic stem cell of a mammal and an identification method thereof.

### SUMMARY OF INVENTION

The invention provides a substantially pure population of amniotic fluid-derived cells, wherein said cells are: capable of differentiating into cells displaying the characteristics of the beta-cell lineage, substantially negative for the expression of the CD117 and Oct-4 protein markers, wherein the markers are not present or expressed in at least 70% of the total cell population, substantially positive for the expression of GATA-6 and SSEA-4, wherein GATA-6 and SSEA-4 are present or expressed in at least about 50% of the total cell population, and substantially either: i) positive for the expression of SOX-17 and cytokeratin, wherein SOX-17 and cytokeratin are present or expressed in at least about 50% of the total cell population; ii) negative for the expression of SOX-17, wherein SOX-17 is not present or expressed in at least 70% of the total cell population, and positive in the expression of cytokeratin, wherein cytokeratin is present or expressed in at least about 50% of the total cell population; or iii) negative for the expression of SOX-17 and cytokeratin, wherein SOX-17 and cytokeratin are not present or expressed in at least 70% of the total cell population.

The invention also provides a population of amniotic fluid-derived cells according to the present invention for use in a method of treating a patient with diabetes mellitus or at risk of developing diabetes.

The invention also provides the in vitro use of the population of cells according to the present invention, in a method wherein the cells of said population are differentiated into pancreatic hormone producing cells.

### SUMMARY OF DISCLOSURE

Disclosed herein is a method for isolating mammalian amniotic fluid-derived cells. Amniotic fluid-derived cells are obtained from amniotic fluid samples of about 14 to about 23 weeks gestation. Alternatively, the amniotic fluid-derived cells are obtained from amniotic fluid samples of about 23 to about 40 weeks gestation.

In one embodiment, the cultures are left undisturbed for at least 5 to 10 days under hypoxic conditions (3% O₂). Alternatively, the cultures are left undisturbed for at least 5 to 10 days under normoxic conditions (approximately 20% O₂).

In an alternate embodiment, amniotic fluid-derived cells are obtained from amniotic fluid samples from the second trimester of gestation. Alternatively, the amniotic fluid-derived cells are obtained from amniotic fluid samples from the third trimester of gestation.

In one embodiment, the cultured amniotic fluid-derived cells are isolated as single cells, and clonally expanded.

The amniotic fluid-derived isolated according to the methods disclosed herein can be contacted, for example, with an agent (such as an antibody) that specifically recognizes a protein marker expressed by amniotic fluid cells, to identify and select amniotic fluid-derived cells, thereby obtaining a substantially pure population of amniotic fluid-derived cells, i.e., wherein a recognized protein marker is expressed in at least 50% of the cell population.

In one embodiment, the resulting amniotic fluid-derived cell population is substantially positive for at least one ofthe following markers: HNF-1 beta, HNF-3 beta, SOX-17, or GATA-6. The amniotic fluid-derived cell population is substantially negative for at least one of the following markers: CD117, Oct-4, or Tra2-54. The amniotic fluid-derived cell population can be expanded for more than 50 population doublings without losing the capacity to express HNF-1 beta, HNF-3 beta, SOX-17, or GATA-6.

In one embodiment, the amniotic fluid-derived cell population is substantially positive for the following markers: SSEA4 and CD44. The amniotic fluid-derived cell population can be expanded for more than 50 population doublings without losing the capacity to express HNF-1 beta, HNF-3 beta, SOX-17, or GATA-6.

In one embodiment, the amniotic fluid-derived cell population isolated according to the methods disclosed herein is substantially negative for at least one of the following markers: SOX-17, CD117, Oct-4, or Tra2-54. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods disclosed herein is substantially negative for cytokeratin and at least one ofthe following markers: SOX-17, CD117, Oct-4, or Tra2-54. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods disclosed herein is substantially negative for SOX-17. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods disclosed herein is substantially negative for the following markers: cytokeratin, and SOX-17. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods disclosed herein is substantially negative for SOX-17. The amniotic fluid-derived cell population is further negative for at least one of the following markers: CD 117, Oct-4, or Tra2-54. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods disclosed herein is substantially negative for the following markers: cytokeratin, and SOX-17. The amniotic fluid-derived cell population is further negative for at least one of the following markers: CD117, Oct-4, or Tra2-54. The amniotic fluid-derived cell population is substantially positive for the following markers: SSEA4 and CD44. The amniotic fluid-derived cell population can be expanded for more than 50 population doublings.

In another embodiment, the present disclosure provides an isolated pure population of amniotic fluid-derived cells that are substantially negative for at least one of the following markers: CD117, Oct-4, or Tra2-54.

In another embodiment, the present disclosure provides an isolated pure population of amniotic fluid-derived cells that are substantially negative for at least one of the following markers: SOX-17, CD117, Oct-4, or Tra2-54.

In another embodiment, the present disclosure provides an isolated pure population of amniotic fluid-derived cells that are substantially negative for SOX-17.

In another embodiment, the present disclosure provides an isolated pure population of amniotic fluid-derived cells that are substantially negative for SOX-17, and substantially negative for at least one of the following markers: CD117, Oct-4, or Tra2-54.

In one embodiment, the amniotic fluid-derived cells isolated according to the methods disclosed herein may also express at least one of the following: Musashi-1 and Hesl.

The amniotic fluid-derived cells isolated and expanded as disclosed herein can be induced to differentiate into cells of the β cell lineage under appropriate *in vitro* or *in vivo* conditions. Accordingly, the amniotic fluid-derived cells selected and expanded according to the present disclosure, as well as the differentiated cells derived from the amniotic fluid-derived cells, are useful for treating Type 1 and 2 diabetes.

The amniotic fluid-derived cells isolated and expanded as disclosed herein can be induced to gut hormone-producing cells under appropriate *in vitro* or *in vivo* conditions. In one embodiment, the amniotic fluid-derived cells isolated and expanded as disclosed herein can be induced to gut hormone-producing cells under appropriate *in vitro* or *in vivo* conditions and may express insulin in a glucose responsive manner.

### BRIEF DESCRIPTION OF THE DRAWINGS

**Figure 1** shows the isolation and culturing steps used to isolate the amniotic fluid derived cells of the present invention.
**Figure 2** shows three distinct morphologies of cells isolated from an amniotic fluid sample at passage 0. a) AF morphology, b) epithelial morphology, and c) fibroblast morphology.
**Figure 3** depicts the expression of cell surface markers on AF-I cells derived from amniotic fluid. The markers are indicated on panels a-n.
**Figure 4** depicts the expression of cell surface markers on F cells derived from amniotic fluid. The markers are indicated on panels a-l.
**Figure 5** depicts the expression of cell surface markers on E cells derived from amniotic fluid. The markers are indicated on panels a-m.
**Figure 6** depicts immunofluoresence images of the F cells derived from amniotic fluid samples. F cells stained positive for a) vimentin, b) SSEA-4, and c) beta III tubulin.
**Figure 7** depicts immunofluoresence images of the E cells derived from amniotic fluid samples. E cells stained positive for a) vimentin and nestin, b) SSEA-4, c) beta III tubulin, d) pan-cytokeratin, e) smooth muscle actin, and f) cytokeratin 19.
**Figure 8** depicts immunofluoresence images of the AF-I cells derived from amniotic fluid samples. AF-I cells stained positive for a) vimentin and nestin, b) beta III tubulin, c) cytokeratin 19 and HES-1, d) pan-cytokeratin, e) SSEA-4, f) SOX-17 and ZO-1, g) GATA-6, h) HNF-1 beta, i) smooth muscle actin and HES-2.
**Figure 9** shows the expression profile of AF-I, AF-II, and AF-III cells of the present disclosure.
**Figure 10** depicts the population doubling curve of early passage AF-I cells.

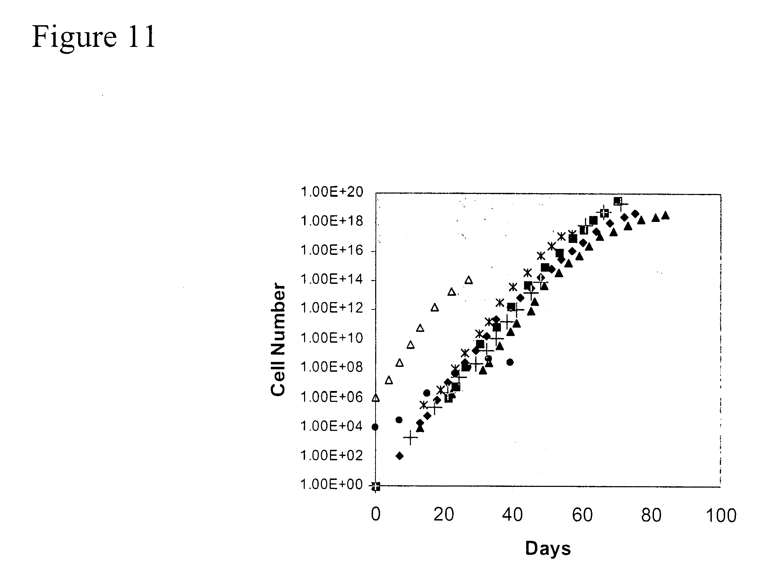

**Figure 11** depicts the expansion potential of AF, F, or E cell derived from different donors. ◆ shows the cell number of amniotic fluid-derived cells with AF-I morphology obtained from amniotic fluid from one donor at 14-23 weeks gestation. Cells were cultured in media number 5 (**Table II**). ▲ shows the cell number of amniotic fluid-derived cells with AF-I morphology obtained from amniotic fluid from a second donor at 14-23 weeks gestation. Cells were cultured in media number 5 (**Table II**). ■ shows the cell number of amniotic fluid-derived cells with F morphology obtained from amniotic fluid from a third donor at 14-23 weeks gestation. Cells were cultured in media number 15 (**Table II**). 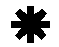 shows the cell number of amniotic fluid-derived cells with F morphology obtained from amniotic fluid from a fourth donor at 14-23 weeks gestation. Cells were cultured in media number 16 (**Table II**). ● shows the cell number of amniotic fluid-derived cells with E morphology obtained from amniotic fluid from a donor at 14-23 weeks gestation. Cells were cultured in media number 5. + shows the cell number of amniotic fluid-derived cells with AF-II morphology obtained from amniotic fluid from a second donor at 14-23 weeks gestation. Cells were cultured in media number 5 (**Table II**). Δ shows the cell number of amniotic fluid-derived cells with AF-III morphology obtained from amniotic fluid from a second donor at 14-23 weeks gestation. Cells were cultured in media number 5 (**Table II**)

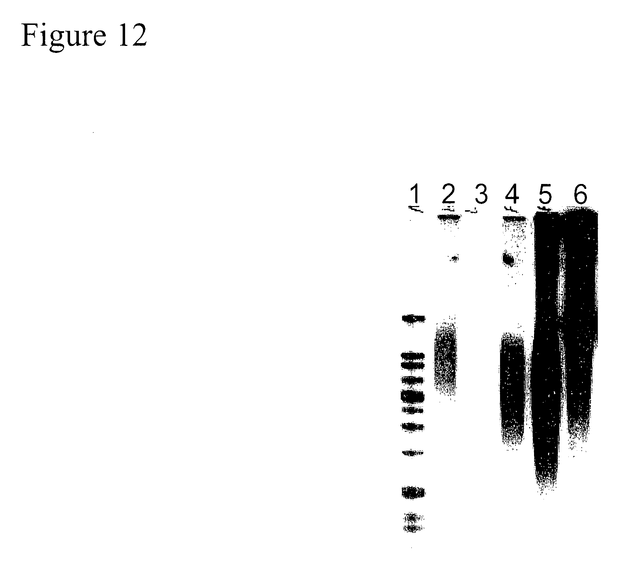

**Figure 12** depicts the telomere length of an AF-I cell line cultured either in AMNIOMAX or DM-LG + 10 % FBS at an intermediate passage level (approximately 40 population doublings). Lane 1 is the molecular weight ladder, lane 2 is the high telomere length control, lane 3 is the low telomere length control, lane 4 is amniotic fluid-derived cells from a donor at Passage 12, cultured in DMEM-LG + 10% FBS, lane 5 is amniotic fluid-derived cells from the same donor at passage 12, cultured in media #5, and lane 6 is an embryonic carcinoma cell line (NTERA cells) that serves as a positive control.
**Figure 13** shows the karyotype of a) AF-I, b) AF-II, and c) AF-III cells cultured at passage 7-9 (approximately 30-35 population doublings).
**Figure 14** depicts the expansion potential of a single AF derived cell from one donor at term (approximately 38 weeks). Cells were cultured in media number 5 (**Table II**).
**Figure 15** depicts the scatter plot gene expression profiles between the different amniotic fluid cell types. The Pearson correlation coefficient for each plot is also listed.
**Figure 16** shows the effects of growth factors on gene expression in amniotic fluid-derived cells. Amniotic fluid-derived cells were obtained from a single donor and cultured for 12 days in conditioned media that was obtained from cultures of PANC-1 cells. The media was supplemented with the growth factors indicated. The levels of expression of HNF-3 beta and somatostatin were determined by real-time PCR. Human pancreas total RNA was included as a calibrator. Panel a shows the changes in HNF-3 beta expression. Panel b shows the changes in somatostatin expression.
**Figure 17** shows the effects of L685,458 on cultured amniotic fluid-derived cells having the AF morphology. Panel a shows the relative differences in RNA expression of human Hes-1 in cultured AF cells treated with the concentrations of L685,458 indicated. Panel b shows the effects of L685,458 on the viability of the cultured cells following a treatment with L685,458. Cells were treated for three days, at the concentrations indicated. Changes in viability, corresponding to cytotoxicity were detected using an MTS assay, where a decrease in cell viability corresponds to a decrease in A490nm.

### DETAILED DESCRIPTION

For clarity of disclosure, and not by way of limitation, the detailed description of the invention is divided into the following subsections that describe or illustrate certain features, embodiments or applications of, or relating to, the present invention.

Disclosed herein are methods for isolating an amniotic fluid-derived cell population that is highly proliferative, and displays embryonic-like characteristics. Similar cells may also be present in the chorionic villus. Some embodiments disclosed herein describe three morphologically distinct populations of amniotic fluid-derived cells: "fibroblastic" (F), epithelial" (E) cells, and "amniotic fluid" (AF) cells.

### Definitions

"β-cell lineage" refer to cells with positive gene expression for the transcription factor PDX-1 and at least one of the following transcription factors: NGN-3, Nkx2.2, Nkx6.1, NeuroD, Isl-1, HNF-3 beta, MAFA, Pax4, and Pax6. Characteristics of cells of the beta cell lineage are well known to those skilled in the art, and additional characteristics of the beta cell lineage continue to be identified. These transcription factors are well established in prior art for identification of endocrine cells (Nature Reviews Genetics, Vol3, 524-632, 2002).

"Pancreatic islet-like structure" refers to a three-dimensional clusters of cells derived by practicing the methods of the disclosure, which has the appearance of a pancreatic islet. The cells in a pancreatic islet-like structure express at least the PDX-1 gene and one hormone selected from the list glucagon, somatostatin, or insulin.

The term "hypoxic" refers to oxygen levels less than 20%, preferably less than 10%, and more preferably less than 5% but more than 1%.

The term "normoxia" refers to atmospheric oxygen levels of about 20%.

The term "substantially positive," when used in connection with a population of cells with respect to the expression of certain marker (such as a membrane receptor, cytoplasmic or nuclear protein, or a transcription factor), means that the marker is present or expressed in at least about 50%, alternatively at least about 60%, and alternatively at least about 70%, of the total cell population.

The term "substantially negative," when used in connection with a population of cells with respect to the expression of certain marker (such as a membrane receptor, cytoplasmic or nuclear protein, or a transcription factor), means that the marker is not present or expressed in at least about 70%, alternatively about 80%, alternatively about 90%, of the total cell population.

A "stem cell" as used herein refers to an undifferentiated cell that is capable of extensive propagation either *in vivo* or *ex vivo* and capable of differentiation to other cell types.

A "progenitor cell" refers to a cell that is derived from a stem cell by differentiation and is capable of further differentiation to more mature cell types. Progenitor cells typically have more restricted proliferation capacity as compared to stem cells.

"Expandable population" refers to the ability of an isolated cell population to be propagated through at least 50 or more cell divisions in a cell culture system.

By "undifferentiated cells," when used in connection with cells isolated from a amniotic fluid, are meant a population of amniotic fluid-derived cells that are substantially negative for the expression of PDX-1, or insulin.

By "differentiated cells," when used in connection with cells isolated from amniotic fluid, are meant a population of amniotic fluid-derived cells that are substantially positive for the expression of PDX-1, or insulin.

"Markers" as used herein, are nucleic acid or polypeptide molecules that are differentially expressed in a cell of interest. In this context, differential expression means an increased level of the marker for a positive marker, and a decreased level for a negative marker. The detectable level of the marker nucleic acid or polypeptide is sufficiently higher or lower in the cells of interest, compared to other cells, such that the cell of interest can be identified and distinguished from other cells, using any of a variety of methods known in the art.

"c-Kit" and "CD 117" both refer to a cell surface receptor tyrosine kinase having a sequence disclosed in Genbank Accession No. X06182, or a naturally occurring variant sequence thereof (e.g., allelic variant).

"CD9" is also referred to as "Motility-related protein-1 (MRP-1)" and is a transmembrane glycoprotein that has been implicated in cell adhesion, motility, proliferation, and differentiation.

"CD10" is also referred to as "Common Acute Lymphocytic Leukemia Antigen (CALLA)". CD10 is a cell surface enzyme with neutral metalloendopeptidase activity and it is expressed in lymphoblastic, Burkitt's, and follicular germinal center lymphomas and in patients with chronic myelocytic leukemia. It is also expressed on the surface of normal early lymphoid progenitor cells, immature B Cells within adult bone marrow and germinal center B Cells within lymphoid tissue. CD10 is also present on breast myoepithelial cells, bile canaliculi, fibroblasts, brush border of kidney and gut epithelial cells.

"CD44" is also referred to as "Hermes antigen" and is the main cell surface receptor for hyaluronan. This CD is primarily expressed in most cell types, except for tissues/cells such as hepatocytes, some epithelial cells, and cardiac muscle.

"CD49f" is also referred to as "a6 integrin" and "VLA-6," and associates with integrin subunit beta 1 to bind laminin. CD49f is expressed primarily on epithelial cells, trophoblasts, platelets, and monocytes.

"CD73" is also referred to as "ecto-5'-nucleotidase" and is primarily expressed on a subset of-B and T cells, bone marrow stromal cells, various epithelial cells, fibroblasts, and endothelial cells.

"CD90" is also referred to as "Thy-1" and is primarily expressed on hematopoietic stem cells, connective tissue cells, and various fibroblastic and stromal cells.

"SSEA-1" (Stage Specific Embryonic Antigen-1) is a glycolipid surface antigen present on the surface of murine teratocarcinoma stem cells (EC), murine and human embryonic germ cells (EG), and murine embryonic stem cells (ES).

"SSEA-3" (Stage Specific Embryonic Antigen-3) is a glycolipid surface antigen present on the surface of human teratocarcinoma stem cells (EC), human embryonic germ cells (EG), and human embryonic stem cells (ES).

"SSEA-4" (Stage Specific Embryonic Antigen-4) is a glycolipid surface antigen present on the surface of human teratocarcinoma stem cells (EC), human embryonic germ cells (EG), and human embryonic stem cells (ES).

"TRA1-60" is a keratin sulfate related antigen that is expressed on the surface of human teratocarcinoma stem cells (EC), human embryonic germ cells (EG), and human embryonic stem cells (ES).

"TRA1-81" is a keratin sulfate related antigen that is expressed on the surface of human teratocarcinoma stem cells (EC), human embryonic germ cells (EG), and human embryonic stem cells (ES).

"TRA2-49" is an alkaline phosphatase isozyme expressed on the surface of human teratocarcinoma stem cells (EC), and human embryonic stem cells (ES).

"Oct-4" is a member of the POU-domain transcription factor and is widely regarded as a hallmark of pluripotent stem cells. The relationship of Oct-4 to pluripotent stem cells is indicated by its tightly restricted expression to undifferentiated pluripotent stem cells. Upon differentiation to somatic lineages, the expression of Oct-4 disappears rapidly.

"EPCAM" " is also referred to as "Epithelial Cell Adhesion Molecule" is broadly expressed on cells of epithelial origin and epithelial derived tumor cells.

"Rex-1" is a developmentally regulated acidic zinc finger gene (*Zfp-42*). Rex-1 message level is high in embryonic stem cells and reduced upon induction of differentiation. As expected for a stem-cell-specific message, Rex-1 mRNA is present in the inner cell mass (ICM) of blastocyst, polar trophoblast of the blastocyst and later in the ectoplacental cone and extraembryonic ectoderm of the egg cylinder (trophoblast-derived tissues), but its abundance is much reduced in the embryonic ectoderm, which is directly descended from the ICM.

"HNF-1 alpha", "HNF-1 beta" and "HNF-3 beta" belong to the hepatic nuclear factor family of transcription factors, which is characterized by a highly conserved DNA binding domain and two short carboxy-terminal domains.

"GATA-4" and "GATA-6" are members of the GATA transcription factor family. This family of transcription factors are induced by TGF β signaling and contribute to the maintenance of early endoderm markers, Sox17α and HNF-1 beta, and the later marker HNF-3 beta.

"SOX-17" is a transcription factor, which is implicated in the formation of endoderm during embryogenesis.

By "basic defined cell culture medium" is meant a serum free or serum containing, chemically defined cell growth medium. Such medium includes, but is not limited to, Dulbecco's Modified Eagle's Medium (DMEM), alpha modified Minimum Essential Medium (alpha MMEM), Basal Medium Essential (BME), CMRL-1066, RPMI 1640, M199 medium, Ham's F10 nutrient medium, KNOCKOUT™ DMEM, Advanced DMEM, MCDB based media such as MCDB -151, -153, -201, and -302 (Sigma, MO), and DMEM/F12. These and other useful media are available from GIBCO, Grand Island, New York, U.S.A., for example. A number of these media are reviewed in Methods in Enzymology, Volume LVIII, "Cell Culture," pp. 62-72, edited by William B. Jakoby and Ira H. Pastan, published by Academic Press Inc.

"Hes-1", also known as "hairy/enhancer of split-1" is a transcription factor that may influence cell fate determination.

"Musashi-1" is a member of a subfamily of RNA binding proteins that are highly conserved across species. Musashi-1 expression is highly enriched in proliferative cells within the developing central nervous system, and may be a stem cell marker in intestinal cells.

"Pharmaceutical carrier" refers to a biodegradable or non-degradable porous or nonporous matrix that can act as a carrier for transplantation of mammalian cells.

"Transplantation" as used herein, can include the steps of introducing a cell or a population of cells or tissue into a mammal such as a human patient. "Transplantation" may also include incorporating cells or tissue into a pharmaceutical carrier, and implanting the carrier in a mammal such as a human patient.

### Isolation of Amniotic Fluid-Derived Cells

Amniotic fluid-derived cells may be isolated by a multi-stage method, which essentially involves:
- Isolation of amniotic fluid,
- Centrifugation of the amniotic fluid, followed by removal of the supernatant,
- Resuspending the cell pellet in growth medium,
- Culturing the tissues and cells in a low oxygen environment,
- Leaving the culture undisturbed for about 5 to 10 days without any media changes,
- Isolation of distinct colonies using cloning rings,
- Culturing the isolated colonies in growth media
- Serial dilution cloning and identification of single cells that give rise to proliferating colonies, and
- Culturing the clones in growth media.

In an alternate embodiment, amniotic fluid-derived cells are isolated by a multi-stage method, which essentially involves:
- Isolation of amniotic fluid,
- Centrifugation of the amniotic fluid, followed by removal of the supernatant,
- Resuspending the cell pellet in growth medium,
- Culturing the tissues and cells in a normoxic environment,
- Leaving the culture undisturbed for about 5 to 10 days without any media changes,
- Isolation of distinct colonies using cloning rings,
- Culturing the isolated colonies in growth media,
- Serial dilution cloning and identification of single cells that give rise to proliferating colonies, and
- Culturing the clones in growth media.

The culture plates may be pre-coated with agents such as, for example, fibronectin, vitronectin, laminin, collagen, gelatin, thrombospondin, placenta extracts, MATRIGEL™, tenascin, human serum, or combinations thereof.

If desirable, the amniotic fluid may be exposed, for example, to an agent (such as an antibody) that specifically recognizes a protein marker expressed by amniotic fluid cells, to identify and select amniotic fluid-derived cells, thereby obtaining a substantially pure population of amniotic fluid-derived cells.

Amniotic fluid-derived cells may be cultured in AMNIOMAX™ complete medium (Invitrogen). Alternatively, the cells may be cultured in Chang B/C medium (Irvine Scientific). Alternatively, the cells may be cultured in low glucose DMEM, supplemented with insulin-transferrin-selenium-X (ITS-X, Invitrogen, CA), 2% fetal bovine serum (FBS), 1% penicillin/streptomycin (P/S) + 25 ng/ml bFGF. Alternatively, the cells may be cultured in, DM-KNOCKOUT™ media (Invitrogen, CA), supplemented with 20% KNOCKOUT™ serum replacement (Invitrogen, CA), 10 ng/ml bFGF. Alternatively, the cells may be cultured in Williams' medium E supplemented with 2% defined FBS, 2mM L-glutamine, ITS, 55 µM 2-mercaptoethanol, 10ng/ml EGF, 4ng/ml bFGF, and 4ng/ml dexamethasone. Alternatively, the cells may be cultured in 1:1 DMEM-LG/MCDB 201, 2% FBS, ITS-X, βme 55 µM, 100 µM ascorbic acid-2-phosphate, 4ng/ml bFGF, 10ng/ml EGF, and 4ng/ml dexamethasone. Alternatively, the cells may be cultured in low glucose DMEM, supplemented with 20% FBS. Alternatively, the cell may be cultured in low glucose DMEM, supplemented with 5% FBS. The cells may also be cultured in low glucose DMEM/MCDB 201 medium (1:1), supplemented with 2% defined FBS, ITS-X, 1nM dexamethasone, 100 mM ascorbic acid 2-phosphate, 10ng/ml EGF, 10ng/ml PDGF-bb and 100 mM 2-mercaptoethanol. The media may be supplemented with bFGF, at concentrations from about 5 ng/ml to about 100 ng/ml. Alternatively, the cells may be cultured in 20% KNOCKOUT™ serum replacement + 80% KNOCKOUT™ DMEM, supplemented with 1 mM L-glutamine, 1% non-essential amino acids and 0.1 mM 2-mercaptoethanol. The medium may be conditioned overnight, on human or murine embryonic fibroblasts, human bone marrow derived stromal cells, or human placenta derived cells and supplemented with 4 ng/ml bFGF. Alternatively, the cells may be cultured in high glucose DMEM, supplemented with 20% defined FBS with 0.1 mM 2- mercaptoethanol. **Table II** lists the various media formulations used to culture the amniotic fluid-derived cells of the present invention.

During culture in growth media, the cells may be cultured under hypoxic or normoxic conditions. Under hypoxic conditions, oxygen levels are lower than 20%, alternatively lower than 10%, alternatively lower than 5%, but more than 1%.

Preferably, the culture should be maintained in the growth media undisturbed for about 5 to 14 days without any media changes, at which point the cells have typically become adherent to the culture substrate used. At which point, cells may be sub-cultured.

Subculture can be achieved with any of the enzymatic solutions well known to those skilled in the art. An example of an enzymatic solution suitable for use in the present invention is TrypLE EXPRESS™ (Invitrogen, Ca).

Furthermore, the amniotic fluid-derived cells may be expanded by culturing in a defined growth media containing agent(s) that stimulate the proliferation of the cells of the present invention. These factors may include, for example, nicotinamide, members of TGF-β family, including TGF-β1, 2, and 3, bone morphogenic proteins (BMP-2, -4, 6, - 7, -11, -12, and -13), serum albumin, fibroblast growth factor family, platelet-derived growth factor-AA, and -BB, platelet rich plasma, insulin growth factor (IGF-I, II) growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, testosterone, estrogen, aprotinin, hydrocortisone, ethanolamine, beta mercaptoethanol, epidermal growth factor (EGF), gastrin I and II, copper chelators such as triethylene pentamine, TGF-α, forskolin, Na-Butyrate, activin, betacellulin, noggin, neuron growth factor, nodal, insulin/transferring/selenium (ITS), hepatocyte growth factor (HGF), keratinocyte growth factor (KGF), bovine pituitary extract, islet neogenesis-associated protein (INGAP), proteasome inhibitors, notch pathway inhibitors, sonic hedgehog inhibitors, GSK-3 beta inhibitors, or combinations thereof. Alternatively, the amniotic fluid-derived cells may be expanded by culturing in conditioned media. By "conditioned media" is meant that a population of cells is grown in a basic defined cell culture medium and contributes soluble factors to the medium. In one such use, the cells are removed from the medium, while the soluble factors the cells produce remain. This medium is then used to nourish a different population of cells.

In certain embodiments, the amniotic fluid-derived cells are cultured on standard tissue culture plates. Alternatively, the culture plates may be coated with extracellular matrix proteins, such as, for example, MATRIGEL ®, growth factor reduced MATRIGEL ®, laminin, collagen, gelatin, tenascin, fibronectin, vitronectin, thrombospondin, placenta extracts, human serum, or combinations thereof.

### Characterization of The Isolated Amniotic Fluid-Derived Cells

Methods for assessing expression of protein and nucleic acid markers in cultured or isolated cells are standard in the art. These include quantitative reverse transcriptase polymerase chain reaction (RT-PCR), Northern blots, *in situ* hybridization (see, e.g., Current Protocols in Molecular Biology (Ausubel et al., eds. 2001 supplement)), and immunoassays, such as immunohistochemical analysis of sectioned material, Western blotting, and for markers that are accessible in intact cells, flow cytometry analysis (FACS) (see, e.g., Harlow and Lane, Using Antibodies: A Laboratory Manual, New York: Cold Spring Harbor Laboratory Press (1998)).

Examples of antibodies useful for detecting certain protein markers are listed in **Table III.** It should be noted that other antibodies directed to the same markers that are recognized by the antibodies listed in **Table III** are available, or can be readily developed. Such other antibodies can also be employed for assessing expression of markers in the cells isolated in accordance with the present invention.

Characteristics of cells of the β-cell lineage are well known to those skilled in the art, and additional characteristics of the β-cell lineage continue to be identified. These characteristics can be used to confirm that the amniotic fluid-derived cells isolated in accordance with the present invention have differentiated to acquire the properties characteristic of the β-cell lineage. β-cell lineage specific characteristics include the expression of one or more transcription factors such as, for example, PDX-1 (pancreatic and duodenal homeobox gene-1), NGN-3 (neurogenin-3), Hlxb9, Nkx6, Isl1, Pax6, NeuroD, Hnf1a, Hnf6, Hnf3 Beta, and Mafa, among others. These transcription factors are well established in the art for identification of endocrine cells. See, e.g., Edlund (Nature Reviews Genetics 3: 524-632 (2002)).

Characteristics of cells of the intestinal cell lineage are well known to those skilled in the art, and additional characteristics of this lineage continue to be identified. These characteristics can be used to confirm that the differentiated or undifferentiated amniotic fluid-derived cells isolated in accordance with the present invention have some of the properties characteristic of the intestinal cell lineage. Intestinal cell lineage characteristics include the expression of one or more transcription factors such as, for example, HES-1 (hairy/enhancer of split-1), NGN-3, Pax6, NeuroD, Math-1, and Musashi-1, among others. In addition, gut cells express hormones such as secretin, cholecystokinin, GLP-1, neurotensin, gastric inhibitory peptide (GIP), serotonin, somatostatin, and gastrin, among others. These transcription factors and gut hormones are well established in the art for identification of intestinal cells. See, e.g., Schonhoff (Endocrinology 145: 2639-2644 (2004)).

The present inventors have identified and isolated a population of amniotic fluid-derived cells that is highly proliferative, and displays embryonic cell-like characteristics, and may express at least one ofthe following markers: HNF-1 beta, HNF-3 beta, SOX-17, or GATA 6. In particular, the amniotic fluid-derived cells isolated as disclosed herein are characterized as, *inter alia*, substantially lacking at least one of the following protein markers: CD117, Oct-4 or Tra2-54. Populations of amniotic fluid-derived cells with these characteristics are referred to herein as AF-I (ATCC accession number PTA-6975).

Under the above growth conditions for expansion, the amniotic fluid cells isolated as disclosed herein may be expanded for more than 50 population doublings, while maintaining the potential to express at least one of the following markers: HNF-1 beta, HNF-3 beta, SOX-17, or GATA-6.

The present inventors have also identified and isolated populations of amniotic fluid-derived cells that is highly proliferative, displays embryonic cell-like characteristics, and do not express at least one of following markers: HNF-3 beta, SOX-17, GATA-4, CD 117, Oct-4 or Tra2-54. In particular, the amniotic fluid-derived cells isolated in accordance with the present disclosure are characterized as, *inter alia,* substantially lacking at least one ofthe following protein markers: CD117, Oct-4 or Tra2-54. Populations of amniotic fluid-derived cells with these characteristics are referred to herein as AF-II.

The present inventors have also identified and isolated populations of amniotic fluid-derived cells that is highly proliferative, displays embryonic cell-like characteristics, and do not express any of the following markers: HNF-3beta, SOX-17, GATA-4, CD117, Oct-4 or Tra2-54. In particular, the amniotic fluid-derived cells isolated in accordance with the present disclosure are characterized as, *inter alia*, substantially lacking at least one of the following protein markers: CD117, Oct-4 or Tra2-54. Populations of amniotic fluid-derived cells with these characteristics are referred to herein as AF-II.

The present inventors have also identified and isolated populations of amniotic fluid-derived cells that is highly proliferative, displays embryonic cell-like characteristics, and do not express cytokeratin and at least one of following markers: HNF-3 beta, SOX-17, GATA-4, CD117, Oct-4 or Tra2-54. In particular, the amniotic fluid-derived cells isolated in accordance with the present disclosure are characterized as, *inter alia,* substantially lacking at least one of the following protein markers: CD117, Oct-4 or Tra2-54. Populations of amniotic fluid-derived cells with these characteristics are refered to herein as AF-III.

The present inventors have also identified and isolated populations of amniotic fluid-derived cells that is highly proliferative, displays embryonic cell-like characteristics, and do not express any of the following markers: cytokeratin, HNF-3beta, SOX-17, GATA-4, CD 117, Oct-4 or Tra2-54. In particular, the amniotic fluid-derived cells isolated in accordance with the present disclosure are characterized as, *inter alia,* substantially lacking at least one of the following protein markers: CD117, Oct-4 or Tra2-54. Populations of amniotic fluid-derived cells with these characteristics are refered to herein as AF-III.

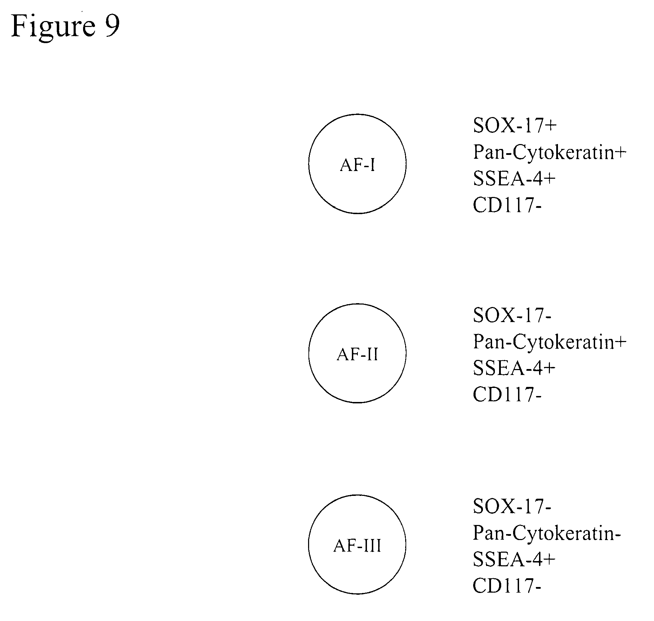

A summay of the expression profile of AF-I, AF-II and AF-III cells is shown in Figure 9.

Amniotic fluid-derived cells of the present invention may be expanded for more than 50 population doublings, while maintaining the potential to differentiate into definitive endoderm, or cells with characteristics of a pancreatic β- cell lineage, or the capacity to differentiate into a gut hormone-producing cell.

### Differentiation Of Amniotic Fluid-Derived Cells

In one aspect, the present disclosure provides compositions capable of differentiating the expanded amniotic fluid-derived cells of this invention into cells bearing markers characteristic of the βcell lineage.

In another aspect, the present disclosure provides compositions capable of differentiating the expanded amniotic fluid-derived cells of this invention into cells bearing markers characteristic of definitive endoderm.

In another aspect, the present disclosure provides compositions capable of differentiating the expanded amniotic fluid-derived cells of this invention into cells bearing markers characteristic of a gut hormone-producing cell.

A basic defined culture medium, when supplied with one or more components, that support the growth of amniotic fluid-derived cells, supplemented with differentiation-inducing amounts of one or more growth factors, is referred to as an "induction medium." In accordance with the present invention, the induction medium contains less than or equal to 20% serum. In one embodiment, fetal calf serum may be used. Alternatively, fetal bovine serum may be replaced by serum from any mammal, or by albumin, bovine albumin or other compounds that permit or enhance differentiation of amniotic fluid-derived cells to the β cell lineage. Alternatively, the induction medium may be conditioned medium.

Factors appropriate for use in the induction medium may include, for example, nicotinamide, members of TGF-β family, including TGF-β1, 2, and 3, bone morphogenic proteins (BMP-2, -4, 6, -7, -11, -12, and -13), serum albumin, fibroblast growth factor family, platelet-derived growth factor-AA, and -BB, platelet rich plasma, insulin growth factor (IGF-I, II) growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as triethylene pentamine, TGF-α, forskolin, Na-Butyrate, activin, betacellulin, ITS, noggin, neurite growth factor, nodal, valporic acid, trichostatin A, sodium butyrate, hepatocyte growth factor (HGF), sphingosine-1, Wnt proteins such as Wnt-1, -3, -3a, 07a, and -8, keratinocyte growth factor (KGF), Dickkopf protein family, bovine pituitary extract, islet neogenesis-associated protein (INGAP), Indian hedgehog, sonic hedgehog, proteasome inhibitors, notch pathway inhibitors, sonic hedgehog inhibitors, or combinations thereof.

In one aspect of the present disclosure, a combination of growth factors and chemical agents, including bFGF, Activin-A, FGF5, N2 and B27 supplements (Gibco, CA), steroid alkaloid such as, for example, cyclopamine (EMD, CA) that inhibits sonic hedgehog signaling, and a proteasome inhibitor such as, for example MG132 (EMD, CA), is supplied to a basic defined medium to support differentiation of amniotic fluid-derived cells into a β-cell lineage. In one aspect, the cells are cultured in an induction media composed of DMEM (low glucose, 5.5 mM) containing 10 micromolar MG-132 for 1-2 days, followed by additional incubation for 3-7 days in an induction media supplemented with 1X B27 (Gibco, CA) and 1X N2 (Gibco, CA) and further supplemented with Cyclopamine (10 µM; EMD, CA), bFGF (20 ng/ml; R&D Systems, MN), Activin A (20 nM; R&D Systems, MN) or FGF5 (20 ng/ml; R&D Systems, MN) for an additional five days.

In another aspect of the disclosure, the cells of the current invention can be treated with conditioned media isolated from cultures of primary fetal intestinal or pancreatic rudiments to induce further differentiation into the intestinal or pancreatic lineages, respectively. The cells may also be induce to differentiate with conditioned media from pancreatic cells lines such as PANC-1, CAPAN-1, BxPC-3, HPAF-II, hepatic cell lines such as HepG2, and intestinal cell lines such as, for example, FHs 74 and HS738. Altrenatively, the cells of the present invention can be treated with conditioned media isolated from human or mouse embryonic stem cells indiced to differentiate into an endodermal lineage. These cell lines can be purchased from the ATCC (VA).

The combination and concentrations of growth factors, the length of culture, and other culture conditions can be optimized by those skilled in the art to achieve effective differentiation by, e.g., monitoring the percentage of cells that have differentiated into cells characteristic of the β-cell lineage. The one or more growth factors may be added in an amount sufficient to induce the differentiation of the amniotic fluid-derived cells of the present invention into cells bearing markers of a β-cell lineage over a time period of about one to four weeks.

### Therapeutic Use of The Cells of The Present Invention.

In one aspect, the present disclosure includes a method for treating a patient suffering from, or at risk of developing Type 1 diabetes. This method involves isolating and culturing amniotic fluid-derived cells, expanding the isolated population of cells, differentiating the cultured cells *in vitro* into a β-cell lineage, and implanting the differentiated cells either directly or in a pharmaceutical carrier into the patient. If appropriate, the patient can be further treated with pharmaceutical agents or bioactives that facilitate the survival and function of the transplanted cells. These agents may include, for example, insulin, members of the TGF-β family, including TGF-β1, 2, and 3, bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, glucagon like peptide-I (GLP-1) and II, GLP-1 and 2 mimetibody, Exendin-4, retinoic acid, parathyroid hormone, MAPK inhibitors, such as, for example, compounds disclosed in U.S. Published Application 2004/0209901 and U.S. Published Application 2004/0132729.

In yet another aspect, this disclosure includes a method for treating a patient suffering from, or at risk of developing Type 2 diabetes. The method involves isolating and culturing amniotic fluid-derived cells according to the present invention, expanding the isolated population of cells, differentiating the cultured cells *in vitro* into a β-cell lineage, and implanting the differentiated cells either directly or in a pharmaceutical carrier into said patient.

The amniotic fluid-derived cells of the present invention may be cryopreserved using commercially available medium containing DMSO (dimethylsulfoxide) or glycerol. The banked and frozen cells may be stored in the vapor phase of a liquid nitrogen storage tank until needed.

The amniotic fluid-derived cells of the present invention may be transplanted with mature islets of the same or different animal species to enhance the survival of the amniotic fluid-derived cells or to induce further differentiation of the amniotic fluid-derived cells into a pancreatic β cell lineage. The source of amniotic fluid from which the cells are isolated may be autologous in relation to the patient undergoing the therapeutic treatment. Alternatively, the source may be allogeneic, or xenogeneic. Cells to be administered to a patient may also be genetically modified to enhance proliferation and/or differentiation or prevent or lessen the risk of immune rejection. Alternatively, the amniotic fluid-derived cells obtained in accordance with the present invention can be used to modulate the recipient's immune response, prior to transplantation of differentiated cells prepared in accordance with the present invention. See, for example, U.S. Patent 6,328,960, U.S. Patent 6,281,012. The amniotic fluid-derived cells of the present invention may be differentiated into an insulin-producing cell prior to transplantation into a recipient. The amniotic fluid-derived cells of the present invention may be fully differentiated into β-cells, prior to transplantation into a recipient. Alternatively, the amniotic fluid-derived cells of the present invention may be transplanted into a recipient in an undifferentiated or partially differentiated state. Further differentiation may take place in the recipient. The amniotic fluid-derived cells of the present invention may be genetically modified. For example, the cells may be engineered to over express markers characteristic of a cell of a β-cell lineage, such as, for example, PDX-1 or insulin. The cells may be engineered to over express with any suitable gene of interest. Furthermore, the cells may be engineered to over express markers characteristic of an intestinal cell, such as MATH-1. Alternatively, the cells of the present invention can be differentiated into a GIP expressing cell population and further modified with an insulin gene under control of the GIP promoter to become glucose responsive and insulin-producing cell population.

Techniques useful to genetically modify the amniotic fluid-derived cells of the present invention can be found, for example, in standard textbooks and reviews in cell biology. Methods in molecular genetics and genetic engineering are described, for example, in Molecular Cloning: A Laboratory Manual, 2nd Ed. (Sambrook et al., 1989); Oligonucleotide Synthesis (M. J. Gait, ed., 1984); Animal Cell Culture (R. I. Freshney, ed., 1987); the series Methods in Enzymology (Academic Press, Inc.); Gene Transfer Vectors for Mammalian Cells (I. M. Miller &M. P. Calos, eds. , 1987); Current Protocols in Molecular Biology and Short Protocols in Molecular Biology, 3rd Edition (F. M. Ausubel et al., eds., 1987 &1995); and Recombinant DNA Methodology II (R. Wu ed. , Academic Press 1995).

The nucleic acid molecule, encoding the gene of interest may be stably integrated into the genome of the host amniotic fluid-derived cell, or the nucleic acid molecule may be present as an extrachromosomal molecule, such as a vector or plasmid. Such an extrachromosomal molecule may be auto- replicating. The term "transfection," as used herein, refers to a process for introducing heterologous nucleic acid into the host amniotic fluid-derived cell.

The cells, undifferentiated or otherwise, may be used as dispersed cells or formed into clusters that may be infused into the hepatic portal vein. Alternatively, the cells may be provided in biocompatible degradable polymeric supports, porous non-degradable devices or encapsulated to protect from host immune response. The cells may be implanted into an appropriate site in a recipient. The implantation sites include, for example, the liver, natural pancreas, renal subcapsular space, omentum, peritoneum, subserosal space, intestine, stomach, or a subcutaneous pocket.

To enhance further differentiation, survival or activity of implanted cells, additional factors, such as growth factors, antioxidants or anti-inflammatory agents, can be administered before, simultaneously with, or after the administration of the cells. In certain embodiments, growth factors are utilized to differentiate the administered cells *in vivo*. These factors can be secreted by endogenous cells and exposed to the administered amniotic fluid-derived cells *in situ*. Implanted amniotic fluid-derived cells can be induced to differentiate by any combination of endogenous and exogenously administered growth factors known in the art.

The amount of cells used in implantation depends on a number of factors including the patient's condition and response to the therapy, and can be determined by one skilled in the art.

In one aspect, this disclosure includes a method for treating a patient suffering from, or at risk of developing diabetes. The method includes isolating and culturing amniotic fluid-derived cells according to the present invention, expanding the isolated population of cells, differentiating *in vitro* the cultured amniotic fluid-derived cells into a β-cell lineage, and incorporating the cells into a three-dimensional support. The cells can be maintained *in vitro* on this support prior to implantation into the patient. Alternatively, the support containing the cells can be directly implanted in the patient without additional *in vitro* culturing. The support can optionally be incorporated with at least one pharmaceutical agent that facilitates the survival and function of the transplanted cells.

Suitable support materials include tissue templates, conduits, barriers, and reservoirs useful for tissue repair. In particular, synthetic and natural materials in the form of foams, sponges, gels, hydrogels, textiles, and nonwoven structures, which have been used *in vitro* and *in vivo* to reconstruct or regenerate biological tissue, as well as to deliver chemotactic agents for inducing tissue growth, are suitable for use in practicing the methods of the present invention. See, e.g., the materials disclosed in U.S. Patent 5,770,417, U.S. Patent 6,022,743, U.S. Patent 5,567,612, U.S. Patent 5,759,830, U.S. Patent 6,626,950, U.S. Patent 6,534,084, U.S. Patent 6,306,424, U.S. Patent 6,365,149, U.S. Patent 6,599,323, U.S. Patent 6,656,488, and U.S. Patent 6,333,029. Exemplary polymers suitable for use in the present invention are disclosed in U.S. Published Application 2004/0062753 A1 and U.S. Patent 4,557,264. To form a support incorporated with a pharmaceutical agent, the pharmaceutical agent can be mixed with the polymer solution prior to forming the support. Alternatively, a pharmaceutical agent could be coated onto a fabricated support, preferably in the presence of a pharmaceutical carrier. The pharmaceutical agent may be present as a liquid, a finely divided solid, or any other appropriate physical form. Alternatively, excipients may be added to the support to alter the release rate of the pharmaceutical agent. In an alternate embodiment, the support is incorporated with at least one pharmaceutical compound that is an anti-inflammatory compound, such as, for example compounds disclosed in U.S. Patent 6,509,369.

The support may be incorporated with at least one pharmaceutical compound that is an anti-apoptotic compound, such as, for example, compounds disclosed in U.S. Patent 6,793,945.

The support may be incorporated with at least one pharmaceutical compound that is an inhibitor of fibrosis, such as, for example, compounds disclosed in U.S. Patent 6,331,298.

In a further embodiment, the support is incorporated with at least one pharmaceutical compound that is capable of enhancing angiogenesis, such as, for example, compounds disclosed in U.S. Published Application 2004/0220393 and U.S. Published Application 2004/0209901.

The support may be incorporated with at least one pharmaceutical compound that is an immunosuppressive compound, such as, for example, compounds disclosed in U.S. Published Application 2004/0171623.

The support may be incorporated with at least one pharmaceutical compound that is a growth factor, such as, for example, members of the TGF-β family, including TGF-β1, 2, and 3, bone morphogenic proteins (BMP-2, -3, -4, -5, -6, -7, -11, - 12, and -13), fibroblast growth factors-1 and -2, platelet-derived growth factor-AA, and - BB, platelet rich plasma, insulin growth factor (IGF-I, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, hypoxia inducible factor 1-alpha, glucagon like peptide-I (GLP-1), GLP-1 and GLP-2 mimetibody, and II, Exendin-4, nodal, noggin, NGF, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in U.S. Published Application 2004/0209901 and U.S. Published Application 2004/0132729.

The incorporation of the cells of the present invention into a scaffold can be achieved by the simple depositing of cells onto the scaffold. Cells can enter into the scaffold by simple diffusion (J. Pediatr. Surg. 23 (1 Pt 2): 3-9 (1988)). Several other approaches have been developed to enhance the efficiency of cell seeding. For example, spinner flasks have been used in seeding of chondrocytes onto polyglycolic acid scaffolds (Biotechnol. Prog. 14(2): 193-202 (1998)). Another approach for seeding cells is the use of centrifugation, which yields minimum stress to the seeded cells and enhances seeding efficiency. For example, Yang et al. developed a cell seeding method (J. Biomed. Mater. Res. 55(3): 379-86 (2001)), referred to as Centrifugational Cell Immobilization (CCI).

The present invention is further illustrated, but not limited by, the following examples.

### Example 1

### The Establishment of Human Amniotic Fluid-Derived Cell Lines

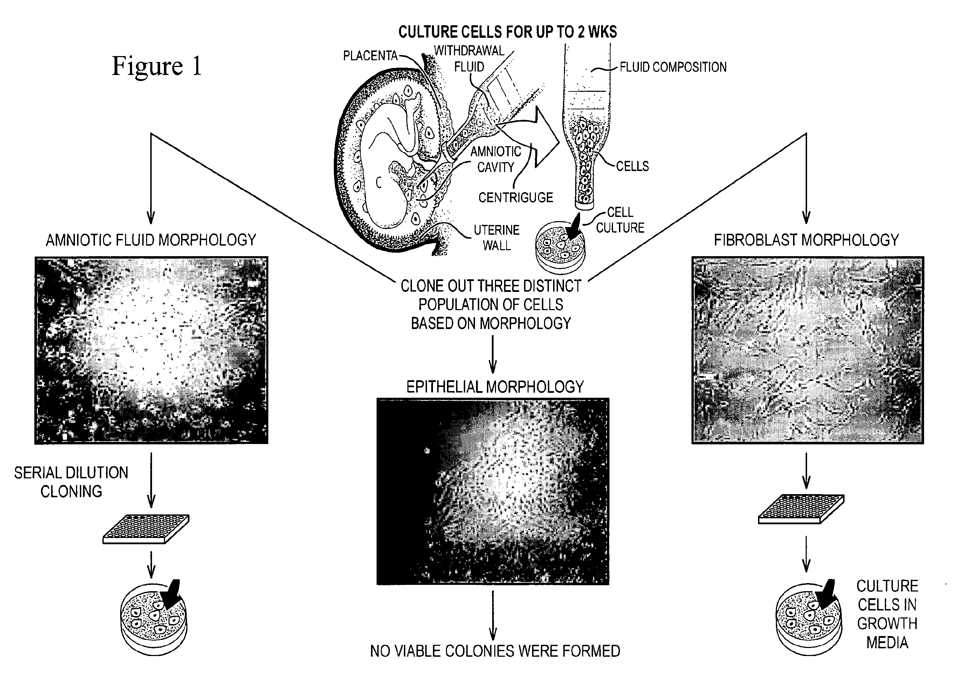

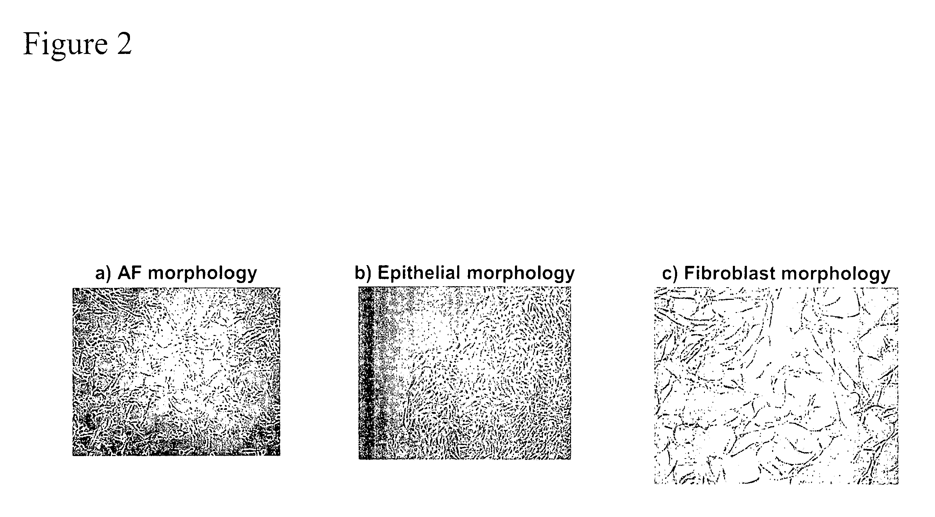

Amniotic fluid used to isolate the cells of the present invention was taken from samples taken from routine amniocentesis performed at 16 to 22 weeks of gestation for fetal karyotyping. The multi-stage method used to isolate the amniotic fluid-derived cells is outlined in **Figure 1**. The amniotic fluid was centrifuged for 7 minutes at 400 *x g* and the supernatant removed. The resulting cell pellet was resuspended in the growth media indicated in **Table III** for the amniotic fluid samples used in the present invention. The cells were cultured either on collagen type IV (1mg/100 mm plate), or on collagen type I (1 microgram/cm²), vitronectin (10 microgram/ml) or fibronectin (10 micrograms/ml) coated plates. The cell yield from amniotic fluid samples had a large variation (8000-300000 cell/sample) and some samples also contained a significant number of blood cell contamination. The cultures were left undisturbed for at least 5-10 days under hypoxic conditions (3% 02). In parallel, cultures were established under similar conditions in normoxic conditions. Next, the cultures were fed with the same growth media and cultured until the cultures reached 70-80% confluency. Cells at this stage were referred to as "P0". In some cultures, colonies of cells were isolated by a cloning ring and sub cultured into a different culture plate. Distinct colonies were present with morphologies characteristic of fibroblast (F), amniotic fluid (AF), and epithelial (E) cells (Figure 2). Cells were released from P0 culture by using TrypLE Express™ (Invitrogen) and seeded into fibronectin, vitronectin, or collagen type IV coated flaks/dishes/plates at various densities (50-10,000 cell/cm2). Some of the P0 cells were used for serial dilution cloning. The population doubling time of the fastest growing cells was approximately 24 hrs at early passages. The expanded cells cultured under various media conditions (**Table II**) were analyzed for cell surface markers (**Table III**). Cells were typically split at ∼70% confluency and reseeded at 100-10000 cells/cm². RNA was collected at various stages of cell growth and analyzed for embryonic and germ layer markers (**Table V**).

Amniotic fluid cells of the present invention were present at various gestational ages. **Table VI** lists the presence or absence of AF, E, and F morphologies in amniotic fluid samples obtained at 17 weeks to 41 weeks of gestation.

Amniotic fluid cells of the present invention were also obtained from amniotic fluid obtained at term (approximately 40 wks of gestation). Amniotic fluid samples were obtained from 38-40 wk deliveries and cultured according to the protocols outlined above. The resulting adherent cell populations displayed very similar characteristics to the cells isolated from 16-22 wks of gestation.

### Example 2

### Clonal Expansion of the Cells of the Present Invention

Using methods described in **Example 1**, cells with AF-like morphologies were harvested from P0 cultures using cloning rings. Three distinct populations of cells exhibiting different expression of surface receptors, cytoskeletal proteins, and transcription factors were identified. For sake of clarity, these populations are referred to as AF-I, AF-II, and AF-III cells. Subsequent examples highlight the differences between AF-I, -II, and -III populations.

### Example 3

### Fluorescence-Activated Cell Sorting (FACS) Analysis

Adhered cells were removed from culture plates by five-minute incubation with the TRYPLE™ express solution (Gibco, CA). Released cells were resuspended in DMEM supplemented with 10% FBS and recovered by centrifugation, followed by washing and resuspending the cells in a staining buffer consisting of 2% BSA, 0.05% sodium azide (Sigma, MO) in PBS. If appropriate, the cells were Fc-receptor blocked using a 0.1 % γ-globulin (Sigma) solution for 15 min. Aliquots (approximately 10⁵ cells) were incubated with either phycoerythirin (PE) or allophycocyanin (APC) conjugated monoclonal antibodies (5 µl antibody per 10⁶ cells), as indicated in **Table III-A**, or with an unconjugated primary antibody. Controls included appropriate isotype matched antibodies, non-stained cells, and cells only stained with secondary conjugated antibody. All incubations with antibodies were performed for 30 mins at 4°C, after which the cells were washed with the staining buffer. Samples that were stained with unconjugated primary antibodies were incubated for additional 30 mins at 4°C with secondary conjugated PE or -APC labeled antibodies. See **Table III-B** for a list of secondary antibodies used. Washed cells were pelleted and resuspended in the staining buffer and the cell surface molecules were identified by using a FACS Array (BD Biosciences) by collecting at least 10,000 events.

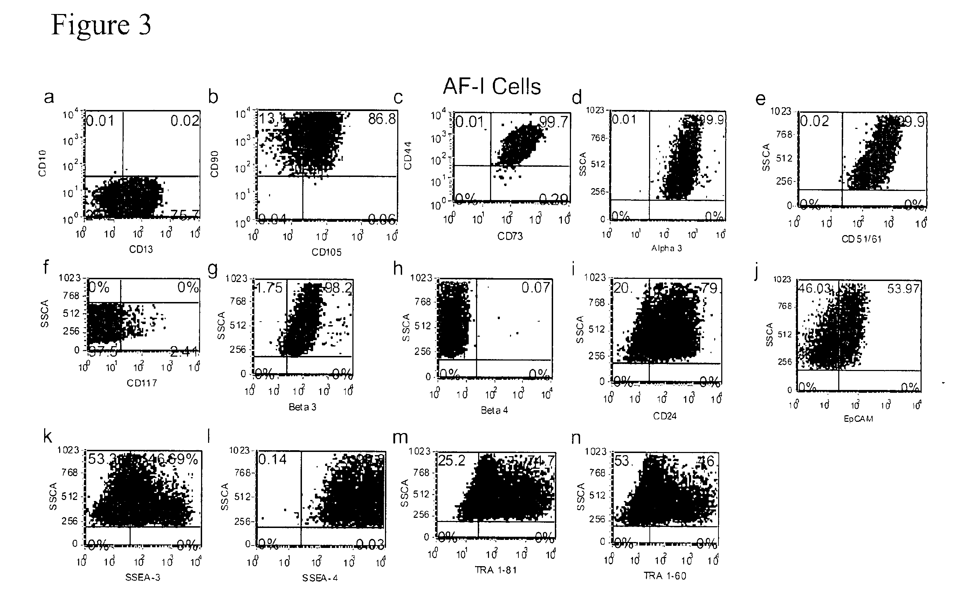

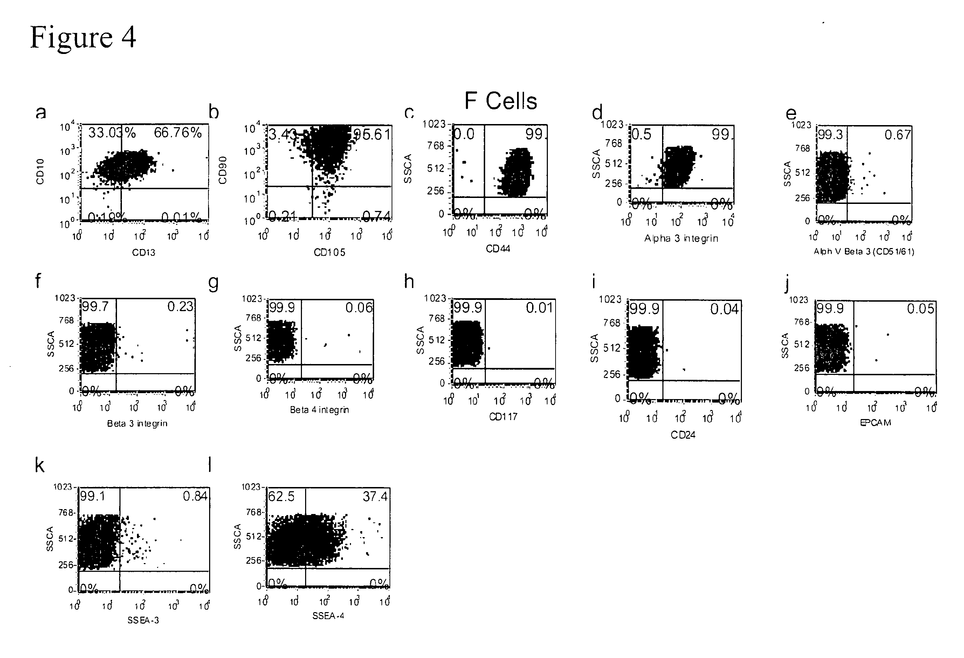

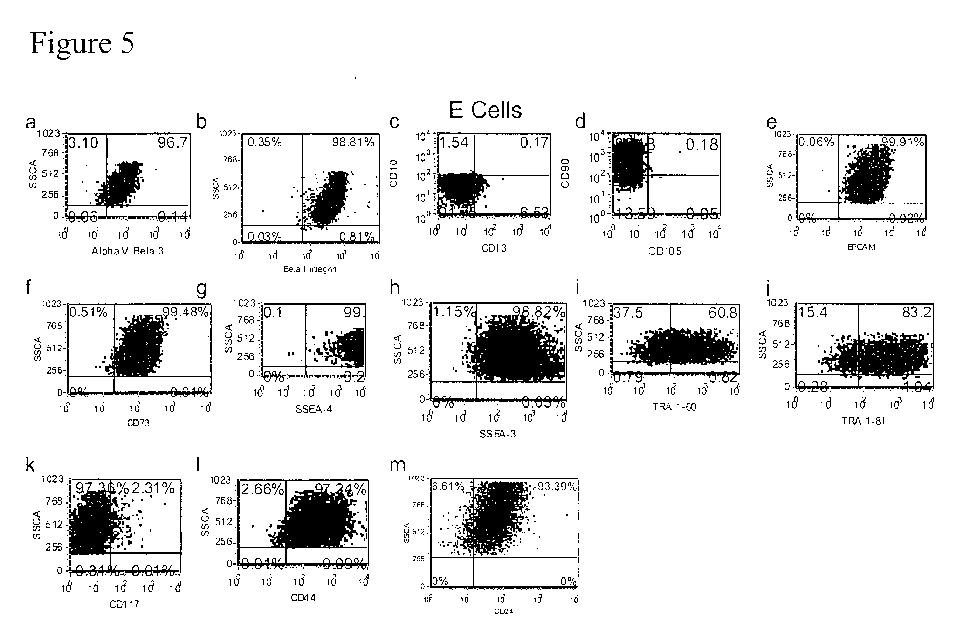

For intracellular staining, cells were first fixed for 10 mins with 4% paraformaldheyde, followed by two rinses in the staining buffer, centrifugation of cells and resuspension of the cells in a permeabilization buffer containing 0.5% Triton-X (Sigma) in PBS for 5 mins at room temperature (RT). The permeabilized cells were rinsed twice with a rinsing buffer, centrifuged, and resuspended in the staining buffer and incubated with an appropriate conjugated antibody (5 µl antibody per 10⁶ cells), for 30 mins at 4°C. Samples that were stained with unconjugated primary antibodies were incubated for additional 30 mins at 4°C with secondary conjugated PE or -APC labeled antibodies (**Table III B**). Washed cells were pelleted and resuspended in the staining buffer and the internal proteins were identified by using a FACSArray (BD Biosciences) by collecting at least 10,000 events. The expression level of examined surface and internal markers is listed in **Table IV A and** B**.** FACS analysis allowed identification of signature markers to distinguish amniotic fluid cells (AF-I, -II, and -III), fibroblasts (F), and epithelial cells (E) (**Figures 3-5**). Table **IV C** lists the cell surface expression profile of AF-I cells isolated from term (38-40 wks) amniotic fluid. The expression level of cell surface receptors is very similar to AF-I cells isolated from 16-22 wks amniotic fluid.

### Example 4

### Immunostaining of Undifferentiated Cells

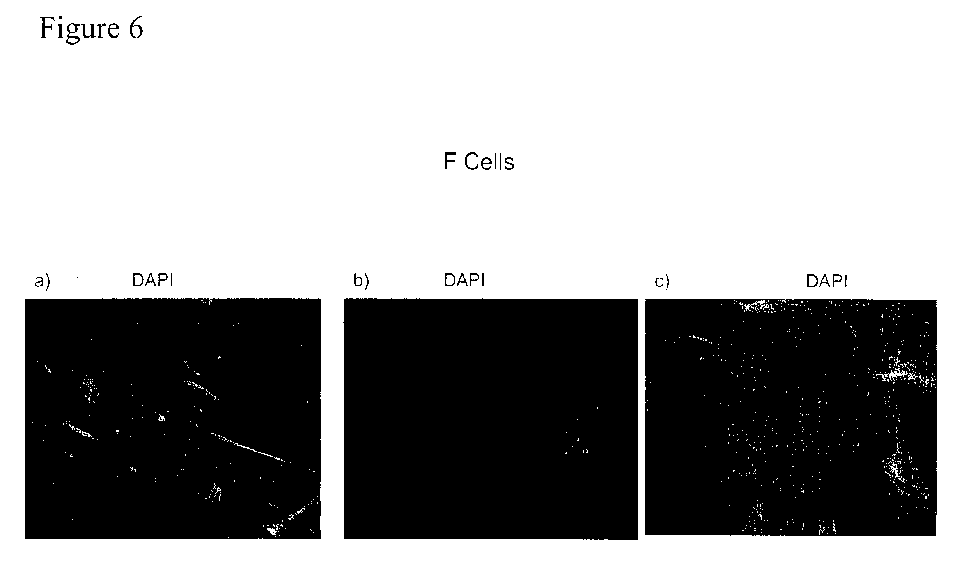

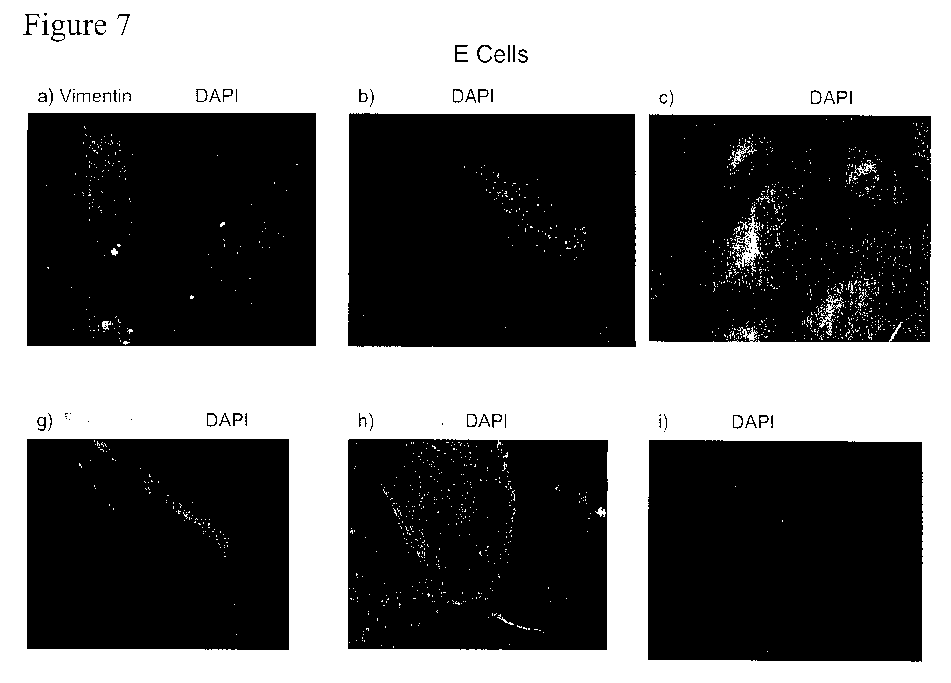

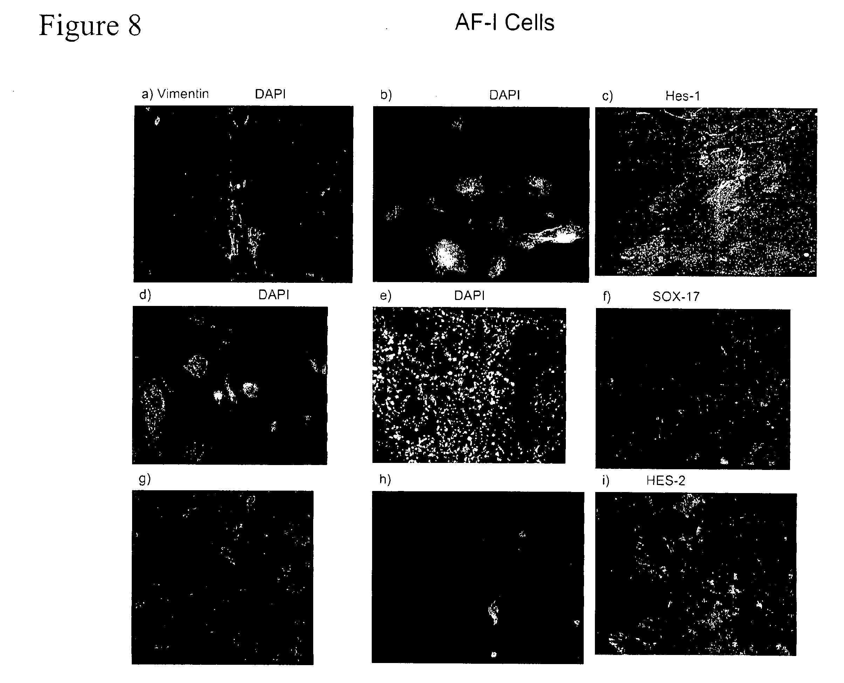

10,000 cells/cm² cells, cultured according to **Example 1**, were seeded into glass bottom 35 mm microwell dishes (Matek Corp, MA) in various growth media. Following three days in culture, the cells were fixed for 10 mins with 4% paraformaldheyde, followed by two rinses in the PBS, and addition of a permeabilization buffer containing 0.5% Triton-X (Sigma) for 5 mins at room temperature (RT) followed by additional three rinses with PBS. The fixed and permeabilized cells were blocked with either 1% bovine serum albumin (BSA) or 4% sera from the species where the secondary antibody was raised in (Goat, donkey, or rabbit). Control samples included reactions with the primary antibody omitted or where the primary antibody was replaced with corresponding immunoglobulins at the same concentration as the primary antibodies. Stained samples were rinsed with a PROLONG® antifade reagent (Invitrogen, CA) containing diamidino-2-phenylindole, dihydrochloride (DAPI) to counter stain the nucleus. Images were acquired using a Nikon Confocal Eclipse C-1 inverted microscope (Nikon, Japan) and a 10-60X objective (**Figures 6 - 8**).

### Example 5

### PCR Analysis Of Undifferentiated Cells

RNA was extracted from cells cultured in the growth media. Total RNA from human pancreas, liver, brain, gut (Ambion, INC.) NTERA cells (human embryonic carcinoma cells line, ATCC), HEK293 cells (ATCC), and human airway epithelia cells (Cambrex) were used as positive controls. Bone marrow derived mesenchymal cells (Cambrex, MD) were used as negative controls for the expression of key genes involved in pancreatic development.

*RNA extraction, purification, and cDNA synthesis*. RNA samples were purified through its binding to a silica-gel membrane (Rneasy Mini Kit, Qiagen, CA) in the presence of an ethanol-containing, high-salt buffer; while contaminants were washed away. The RNA was further purified while bound to the column by treatment with DNase I (Qiagen, CA) for 15 min. High-quality RNA was then eluted in water. Yield and purity were assessed by A260 and A280 readings on the spectrophotometer. cDNA copies were made from purified RNA using an ABI (ABI, CA) high capacity cDNA archive kit.

*Real-time PCR amplification and quantitative analysis*. Unless otherwise stated, all reagents were purchased from Applied Biosystems. Real-time PCR reactions were performed using the ABI PRISM® 7000 Sequence Detection System. TAQMAN® UNIVERSAL PCR MASTER MIX® (ABI, CA) was used with 20 ng of reverse transcribed RNA in a total reaction volume of 20 µl. Each cDNA sample was run in duplicate to correct for pipetting errors. Primers and FAM-labeled TAQMAN®probes were used at concentrations of 200 nM. The level of expression of each target gene was normalized using the pre-developed Applied Biosystem's 18S ribosomal RNA or human glyceraldehydes-3-phosphate dehydrogenase (GAPDH) endogenous control kit. Primers and probes were either designed using ABI PRISM PRIMER EXPRESS™ software or used pre-developed ABI gene analysis kit. For each gene, either one of the primers or the probe were designed to be exon-boundary spanning. This eliminated the possibility of the primers/probe binding to any genomic DNA present. The primer and probe sets are listed as following Nkx2.2 (Hs00159616), Pdx-1 (Hs00426216), Nkx6.1 (Hs00232355), Ngn3 (Hs00360700), Pax4 (Hs00173014), Pax6 (Hs00240871), Insulin (Hs00355773), Glu2 (Hs00165775), glucagon (Hs00174967), Isl-1 (Hs00158126), somatostatin (Hs00174949), FoxA2 (HNF 3-beta) (Hs00232764), HlxB9 (Hs00232128), GATA-4 (Hs00171403), HNF1β (Hs00172123), Musashi Homolog 1 (Msi-1) (Hs00159291), Hes-1 (Hs00172878), Neurotensin (NTS) (Hs00175048), Cholecystokinin (Hs00174937), AFP (Hs00173490), Secretin (Hs00360814), GIP (Hs00175030), GFAP (Hs00157674), MAP2 (Hs00159041), Olig2 (Hs0037782), Oct-4 (CGACCATCTGCCGCTTTGAG (SEQ ID NO: 1) and CCCCCTGTCCCCCA TTCCTA (SEQ ID NO: 2)); Rex-1 (CAGATCCTAAACAGCTCGCAGAAT (SEQ ID NO: 3), and GCGTACGCAAATTAAACTCCAGA(SEQ ID NO: 4); Sox17: TGGCGCAGCAGATACCA (SEQ ID NO:5), AGCGCCTTCCACGACTTG (SEQ ID NO:6) and CCAGCATCTTGCTCAACTCGGCG (SEQ ID NO:7); ABCG-2: GTTTATCCGTGGTGTGTCTGG (SEQ ID NO: 8) and CTGAGCTATAGAGGCCTGGG (SEQ ID NO: 9); SOX2: ATGCACCGCTACGACGTGA (SEQ ID NO:10) and CTTTTGCACCCCTCCCATTT (SEQ ID NO: 11). The remaining primers were designed by using the PRIMERS program (ABI, CA) and are listed in **Table V**. After an initial 50°C for 2 min, and 95°C for 10 min, samples were cycled 40 times in two stages - a denaturation step at 95°C for 15 sec, followed by an annealing/extension step at 60°C for 1 min. Data analysis was carried out using GENEAMP®7000 Sequence Detection System software. For each primer/probe set, a Cₜ value was determined as the cycle number at which the fluorescence intensity reached a specific value in the middle of the exponential region of amplification. Relative gene expression levels were calculated using the comparative Cₜ method. Briefly, for each cDNA sample, the endogenous control Cₜ value was subtracted from the gene of interest Cₜ to give the delta Cₜ value (ΔCₜ). The normalized amount of target was calculated as 2^{-ΔCt}, assuming amplification to be 100% efficiency. Final data were expressed relative to a calibrator sample. The comparative Ct method is only valid if target and endogenous control amplification efficiencies are approximately equal. Preliminary validation experiments were therefore performed for each primer/probe set by amplifying serially diluted cDNA samples and determining the ΔCₜ values. These ΔCₜ values should remain constant across the range of dilutions if amplification efficiencies are equal (**Table V**).

### Example 6

### Population doubling time

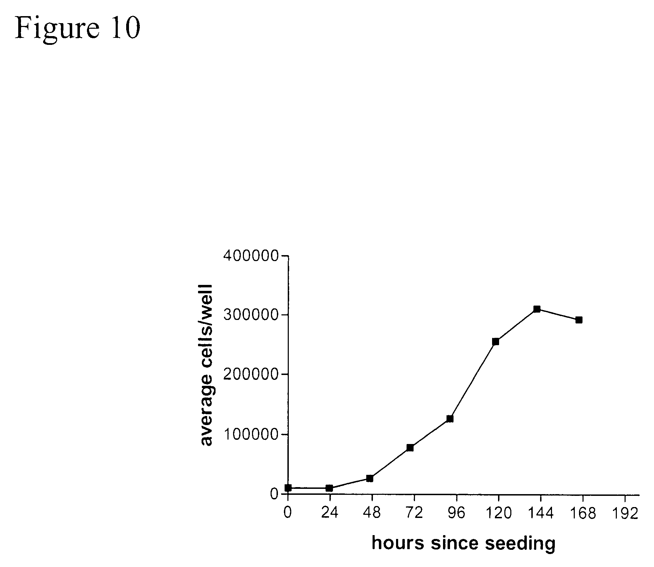

Passage 6 amniotic fluid cells (AF-I type), isolated and expanded according to Example 1 were seeded at 10000 cells/well of a 24-well tissue culture plate (Corning, MA) in growth media #11. At various time points, cells were removed from three wells of the plate using TRYPLE™ Express (Invitrogen, CA) and counted using a Guava PCA-96 cell analysis system and the VIACOUNT® reagent (Guava, CA). **Figure 10** depicts the growth curve of passage 6 cells cultured under hypoxic conditions (3% 02). The linear phase of the log plot was used to estimate the population doubling time of the cells. Population doubling time of passage 6 cells was 31 hrs.

The growth potential of the three cell populations (fibroblast, AF, and epithelial morphology) were compared over long-term cultures. **Figure 11** depicts the growth potential of AF-I, AF-II, AF-III, F, and E cells cultured in media #5. It is clear that F ("fibroblastic" amniotic fluid-derived) cells and AF cells can expand well above 50 population doublings and represent a scalable source for cell therapy applications.

### Example 7

### Telomere length of AF-I cells

The telomere length of an AF-I line isolated from a single cell by limited serial dilution was analyzed at passage 12 (approximately 50 population doublings) by using the *Telo TAGGG* Telomere Length Assay (Roche, IN) and following the manufacturer's instruction. The telomere length was analyzed for cells cultured in DMEM-LG + 10% FBS and cells cultured in Amniomax™ (Gibco) see **Figure 12**. DNA from NTERA cells served as a positive control.

### Example 8

### Karyotype analysis

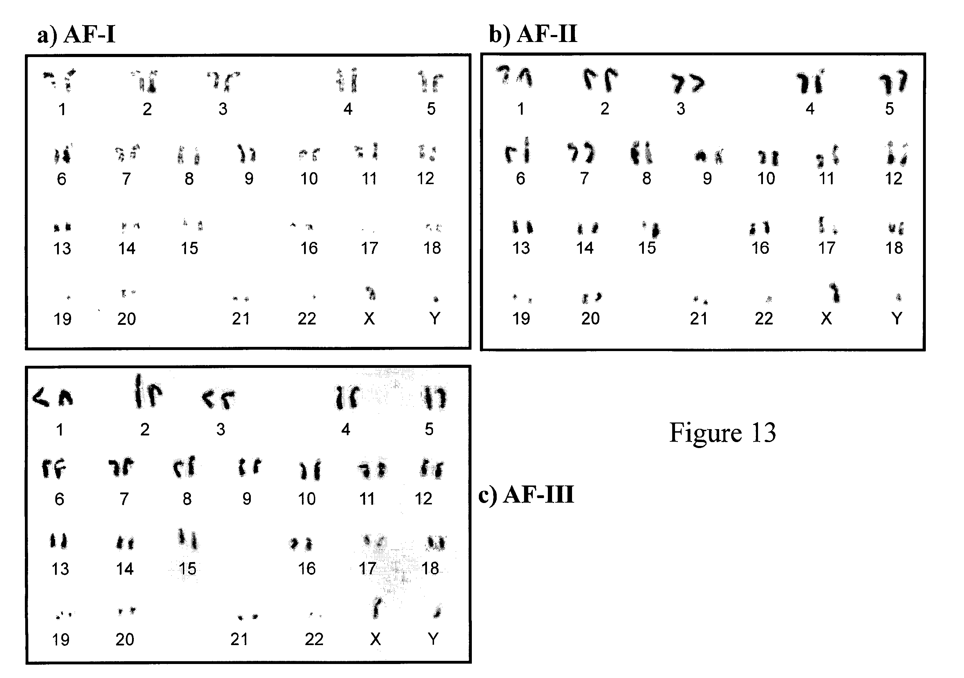

The karyotype of AF cells, isolated from mutliple donors at passage 8-10 (approximately 30 population doublings), was determined by G-band analysis. Five karyotypes were prepared and cytogenetic analysis showed that the cells had a normal autosomes and a modal chromosome number of 46. All cells analyzed also contained the X and Y-chromosomes confirming their fetal origin. **Figure 13** depicts karyotypes of amniotic fluid-derived cells (AF-I, AF-II, and AF-III) isolated from amniotic fluid obtained from 16-22 weeks of gestation.

### Example 9

### Expansion potential of AF cells derived from term amniotic fluid

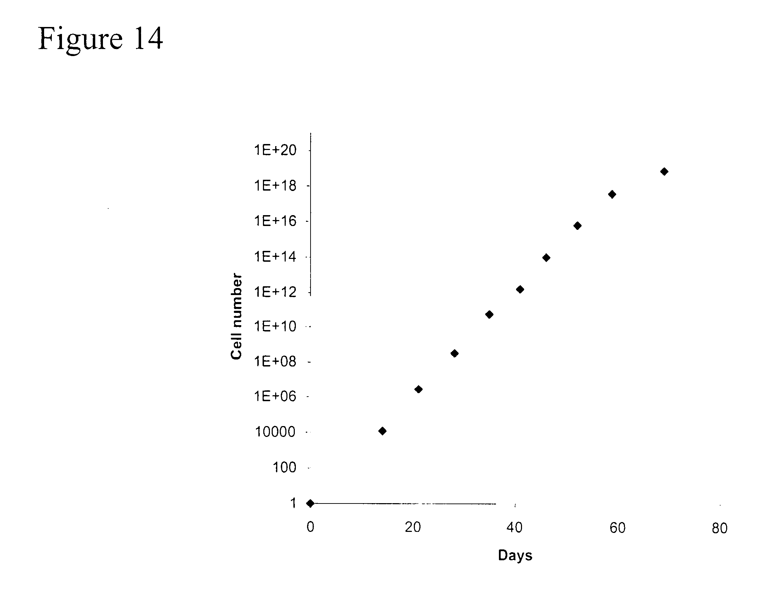

**Figure 14** depicts the expansion potential of an AF-I cell morphology derived from term amniotic fluid (∼38 weeks) and cultured in media #5. The expansion potential is very similar to the AF-I cells isolated from 16-22 wks amniotic fluid.

### Example 10

### Microarray analysis of fibroblast, epithelial, and amniotic fluid morphology cells

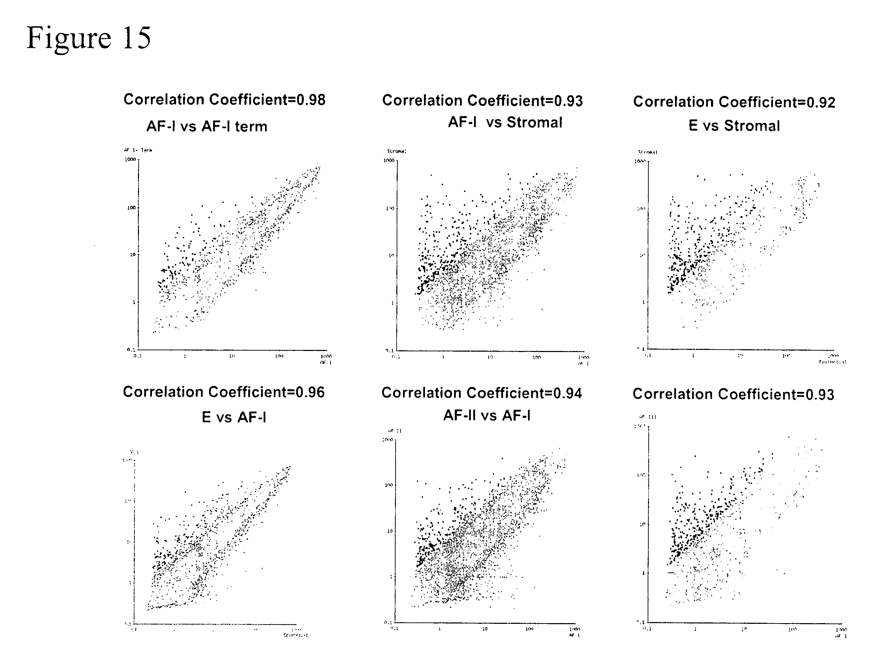

Total RNA was isolated from passage 9-11 amniotic fluid-derived fibroblast cells (F), amniotic fluid-derived epithelial cells (E), amniotic fluid-derived amniotic fluid cells (AF- I, -II, and -III lines), and amniotic fluid at term (AF term) using an RNeasy mini kit (Qiagen). The sample preparation, hybridization, and image analysis was performed according to the CodeLink™ System (GE Healthcare, Amersham Biosciences, NJ). Codelink™ Human Whole Genome arrays were used. It is comprised of approximately 55 000 30-mer probes designed to conserved exons across the transcripts of targeted genes. The chip contains ∼45000 unique Unigene IDs. Following normalization and a log transformation, data analysis was performed using OmniViz® software (MA) and GENESIFTER (VizXLabs, WA). The variance stabilizing transformation along with cross sample normalization was applied to the log transformed array dataset. The variability within each cell line and among the different cell lines was compared using the Pearson correlation coefficient. For all the samples analyzed, the correlation coefficient within a cell line was higher as compared to those between the lines. Variance in gene expression profiles between the different cell types along with the correlation coefficient between the lines are depicted in **Figure 15**. Significant differences in gene expression between the cell types were evaluated using analysis of variance and an F-test with adjusted P-value (Benjamini and Hochberg correction) of ≼ 0.05. **Tables VII A-G** list the genes that are differentially expressed at least 5-fold between the various cell types.

### Example 11

### Differentiation of cells into intestinal-like cells

The AF-I line, AFCA007 Clone A passage 14, was cultured at 10,000 cells/cm² in AMNIOMAX^{™}. At confluency, the cells were further treated for 2 weeks with a daily dose of 10 micro molar retinoic acid (RA) in AMNIOMAX^{™} media. RNA was collected at day 14 and expression of intestinal hormones (secretin, neurotensin, gastric inhibitory peptide (GIP), cholecystokinin, somatostatin, and gastrin) was assessed by using real-time PCR as outlined in Example 4. Intestinal RNA (Ambion) was used to assess relative levels of expression using the ΔΔCₜ method. **Table VIII** lists the Cₜ values and the relative level of expression of the intestinal hormones in treated and untreated samples. As shown in **Table VIII**, addition ofRA enhanced expression of the gut hormones.

### Example 12

### Differentiation of cells into multiple endocrine lineages

Cells from the cell line AFCA007 Clone A (AF-I) at passage 8 were embedded in collagen type I (Becton Dickinson, CA) with 1% growth-factor reduced matrigel matrix (Becton Dickinson), and seeded into 6-well transwell insert at 5x10⁵ cells per well. The bottom well was seeded with human aortic endothelial cells passage 6 (Cambrex. MD). Cells were cultured with DMEM medium supplemented with 5% FBS and growth factors, which includes Cyclopamine, bFGF, EGF, BMP4-7, Activin A, Exendin 4, FGF4, all-trans retinoic acid and γ-secretase inhibitors for 14 days. Cultures were fed every other day. Cells treated by all-trans retinoic acid showed the up-regulation of alpha-fetoprotein (AFP). Treatment of cells with Activin A, BMP4, or the γ-secretase inhibitor L-685,458 up-regulated the expression of HNF-3 beta. Treatment of cells with BMPs at high concentration, 50 ng/ml, also up-regualted the GATA4 expression. Treatment of cells with FGF4 at 50 ng/ml showed an up-regulation of PDX-1 expression (**Table IX**).

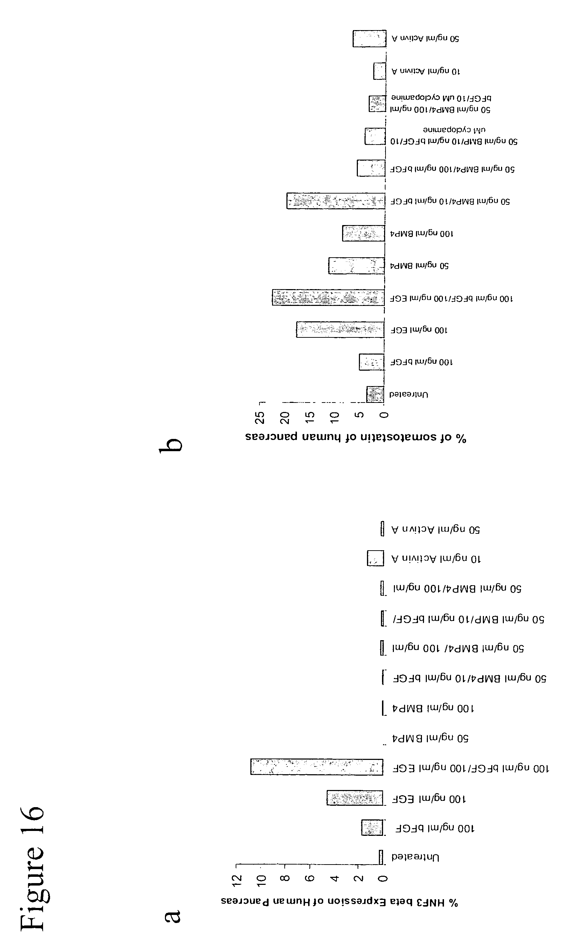

Cells from the cell line AFCA004 (E morphology) at passage 6 were seeded at 5x10⁵ cells per well of 6-well culture plates and treated with conditioned medium from confluent PANC-1 cells (ATCC, VA) in combination with different growth factors. Basic FGF, EGF and combination of bFGF and EGF enhanced the expression of HNF-3 beta ∼100 fold over untreated cells. Basic FGF, EGF and BMPs also stimulated somatostatin expression after 14 days treatment (**Figure 16****, panels a&b)**.

Taken together, these results suggest that AF cells could be differentiated into pancreatic, hepatic or intestinal lineage by treating the cells with different growth factors.

### Example 13

### Modulation of the expression of endoderm makers by inhibiting the Notch pathway

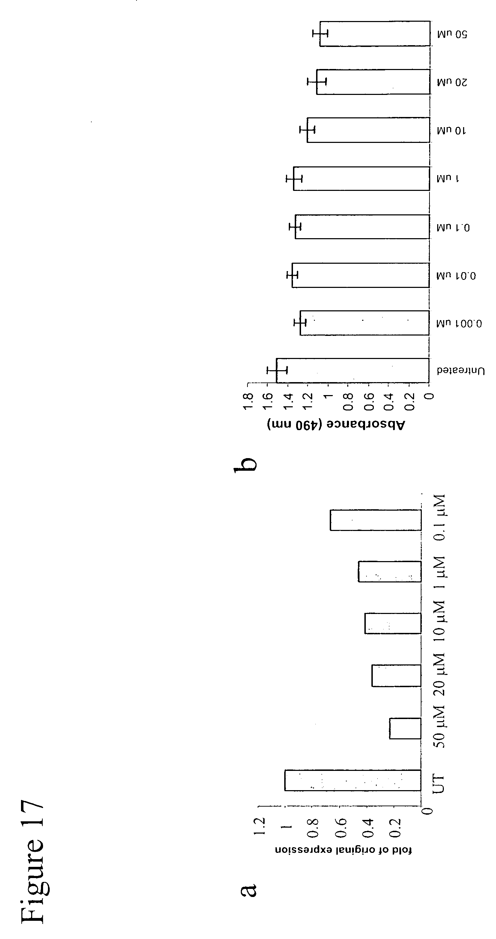

Cells from the cell line AFCA007 (AF-I) at passage 8 were treated with a range of concentrations of the notch pathway inhibitor L-685,458 (Sigma, MO) for 3 to 5 days. The cytotoxcity of L-685,458 was determined by measuring cell viability by a MTS assay (Promega, WI). Real-time PCR analysis was performed to analyze Hes-1 expression after the treatment. We found that L-685,458 showed a dose-dependent inhibitory effect on Hes-1 expression, Hes-1 is the downstream direct target of Notch pathway (**Figure 17****, panel a**). No effect on cell viability, as determined by the MTS assay, was observed following L-685,458 treatment of up to 10µM for 5 days (**Figure 17****, panel b**).

### Example 14

### Cytokine antibody array analysis for AF I and AF II cells

AFCA007 A (AF-I) and AFCA015 C (AF-II) at passage 10 were grown to approximately 70 % confluency and then cell lystaes was collected using mammalian cell lysis kit (Sigma-Aldrich, MO). Cytokine array analyss was completed using Cytokine Array panels provided by RayBiotech, GA (http://www.raybiotech.com/). **Table X** lists cytokine, cytokine and growth factor receeptor expression following normalization of the data and background subtraction. For each panel, positive and negative controls are also included. The panels were run for two different samples per cell type.

**TABLE I: COMPARISON OF THE CELL OF THE PRESENT INVENTION WITH AMNIOTIC FLUID-DERIVED CELLS OF THE ART.**

| Marker | Cell 1 | Cell 2 | Cell 3 | Cell 4 |
|---|---|---|---|---|
| CD10 | | | | - |
| CD105 | + | + | + | Weak |
| CD11 | | | - | - |
| CD117 | Weak | - | - | - |
| CD 13 | | + | | |
| CD29 (Beta 1 integrin) | | | | + |
| CD31 | | | - | |
| CD34 | Weak | - | - | - |
| CD44 | | + | + | + |
| CD45 | | - | - | |
| CD49b (Alpha 2 integrin) | | + | | |
| CD49e (Alpha 5 integrin) | | | + | |
| | | | | |
| CD73 | | | + | + |
| CD166 | | | + | |
| CD90 | + | | + | + |
| HLA ABC | + | + | + | + |
| HLA D | | - | - | - |
| Oct-4 | + | + | | + |
| SSEA-1 | - | - | | |
| SSEA-3 | + | + | | |
| SSEA-4 | + | + | | |
| Stro-1 | | - | | |
| Telomerase | + | | | |
| TRA 1-60 | | + | | |
| TRA 1-81 | | + | | |
| TRA 1-85 | | | | |
| TRA 2-49 | | | | |
| TRA 2-54 | | + | | |

**TABLE II. MEDIA FORMULATIONS USED TO CULTURE AMNIOTIC-FLUID DERIVED CELLS.**

| Media formulation | |
|---|---|
| 1 | DMEM-LG + ITS-X + 1% P/S + 2% FBS + 25 ng/ml bFGF |
| 2 | Advanced DMEM + ITS-X + 1% P/S + 1% FBS + 25 ng/ml bFGF |
| 3 | Alpha MEM: MCDB 153 (1:1) + ITS-X + 1% P/S + 1% FBS + 25 ng/ml bFGF |
| 4 | Defined Keratinocyte growth media (Gibco, NY) |
| 5 | AMNIOMAX™ complete media (Gibco, NY) |
| 6 | Chang B/C (Irvine Scientific, CA) |
| 7 | Chang D (Irvine Scientific, CA) |
| 8 | DM-KNOCKOUT™ media (Invitrogen, CA), supplemented with 20% KNOCKOUT™ serum replacement (Invitrogen, CA),10 ng/ml bFGF |
| 9 | DMEM- Low glucose, supplemented with 20% FBS |
| 10 | DMEM- Low glucose, supplemented with 5% FBS |
| 11 | DMEM- low glucose /MCDB 201 medium (1:1), supplemented with 2% defined FBS, ITS-X, 1nM dexamethasone, 100 mM ascorbic acid 2-phosphate, 10ng/ml EGF, 10ng/ml PDGF-bb and 100 mM 2-mercaptoethanol |
| 12 | 20% KNOCKOUT™ serum replacement + 80% KNOCKOUT™ DMEM, supplemented with 1 mM L-glutamine, 1% non-essential amino acids and 0.1 mM 2-mercaptoethanol |
| 13 | The medium may be conditioned overnight, on human or murine embryonic fibroblasts, human bone marrow derived stromal cells, or human placenta derived cells and supplemented with 4 ng/ml bFGF |
| 14 | high glucose DMEM, supplemented with 20% defined FBS with 0.1 mM 2- mercaptoethanol |
| 15 | Williams' E medium, 2% FBS, 1X ITS, 55uM βme, 1X Glutamax (Gibco), 1%P/S, 10ng/ml EGF, 4ng/ml bFGF, 4ng/ml dexamethasone |
| 16 | 1:1 DMEM-LG/MCDB 201, 2% FBS, ITS-X, βme 55uM,100uM ascorbic acid-2-phosphate, 4ng/ml bFGF, 10ng/ml EGF, 4ng/ml dexamethasone |

**TABLE III A: ANTIBODIES TO SURFACE RECEPTORS.**

| Antibody | Supplier | Isotype | Clone |
|---|---|---|---|
| CD117 (c-Kit) | Santa Cruz Biotechnology (CA) | Goat IgG | M-14 |
| CD117 (c-Kit) | Santa Cruz Biotechnology (CA) | Mouse IgG I | 104D2 |
| CD117 (c-Kit) | BD Pharmingen (CA) | Mouse IgG1, kappa | YB5.B8 |
| CD24 | BD Pharmingen (CA) | Mouse IgG2A, Kappa | ML5 |
| CD44 | BD Pharmingen (CA) | Mouse IgG2b, Kappa | G44-26 |
| CD45 | BD Pharmingen (CA) | Mouse IgG1, Kappa | Hi30 |
| CD49f | BD Pharmingen (CA) | Rat IgG2A, Kappa | G0H3 |
| CD73 | BD Pharmingen (CA) | Mouse IgG1, Kappa | AD2 |
| CD10 | BD Pharmingen (CA) | Mouse IgG1, Kappa | HI10a |
| CD105 | Santa Cruz (CA) | Mouse IgG1 | P3D1 |
| CD49b (Alpha 2 integrin) | BD Pharmingen (CA) | Mouse IgG2a, Kappa | 121-H6 |
| Alpha 3 integrin | Santa Cruz (CA) | Mouse IgG1 | P1B5 |
| Alpha 4 integrin (CD49d) | BD Pharmingen (CA) | Mouse IgG1, Kappa | 9F10 |
| Alpha 5 intgerin (CD49e) | BD Pharmingen (CA) | Mouse IgG1, Kappa | IIA1 |
| CD49f (Alpha 6 integrin) | BD Pharmingen (CA) | Rat IgG2a | GoH3 |
| CD29 (Beta 1 integrin) | BD Pharmingen (CA) | Mouse IgGI, Kappa | MAR4 |
| Beta 3 integrin | Santa Cruz (CA) | Mouse IgG1 | Y2/51 |
| Alpha V Beta 3 integrin (CD51/61) | BD Pharmingen (CA) | Mouse IgG1, Kappa | 23C6 |
| SSEA-3 | Chemicon (CA) | Mouse IgG3 | MC-631 |
| SSEA-4 | Chemicon (CA) | Rat IgM | MC-813-70 |
| TRA 1-60 | Chemicon (CA) | Mouse IgM | TRA 1-60 |
| TRA 1-81 | Chemicon (CA) | Mouse IgM | TRA 1-81 |
| TRA 1-85 | Chemicon (CA) | Mouse IgG1 | TRA 1-85 |
| TRA 2-54 | Chemicon (CA) | Mouse IgG1 | TRA 2-54 |
| EGF r | BD Pharmingen (CA) | Mouse IgG2b, Kappa | EGFR1 |
| EpCAM | BD Pharmingen (CA) | Mouse IgG1 | EBA-1 |
| HLA ABC | BD Pharmingen (CA) | Mouse IgG1, Kappa | G46-2.6 |
| HLA DR | BD Pharmingen (CA) | Mouse IgG2b, Kappa | TU36 |
| CD90 | BD Pharmingen (CA) | Mouse IgG1, kappa | 5E10 |

**TABLE III B: LIST OF SECONDARY CONJUGATED ANTIBODIES USED FOR FACS AND IMMUNOSTAINININGANALYSIS.**

| Secondary conjugated antibody | Supplier | Dilution |
|---|---|---|
| Goat Anti-Mouse IgG APC conjugated | Jackson ImmunoResearch (PA) | 1:200 |
| Goat Anti-Mouse IgG PE conjugated | Jackson ImmunoResearch (PA) | 1:200 |
| Donkey anti-rabbit PE or - APC conjugated | Jackson ImmunoResearch (PA) | 1:200 |
| Donkey anti-goat PE or - APC conjugated | Jackson ImmunoResearch (PA) | 1:200 |
| Goat anti-mouse IgM PE | SouthernBiotech (AL) | 1:200 |
| Goat anti-Rat IgM PE | SouthernBiotech (AL) | 1:200 |
| Goat anti-mouse IgG3 PE | SouthernBiotech (AL) | 1:200 |

**TABLE III C: LIST OF PRIMARY ANTIBODIES USED FOR IMMUNOSTAINININGANALYSIS.**

| Antibody | Supplier | Dilution |
|---|---|---|
| Beta III Tubulin | R&D Systems (MN) | 1:100 |
| C-Kit | Santa Cruz Biotechnology (CA) | 1:100 |
| Cytokeratin 18 | Sigma (MO) | 1:100 |
| Cytokeratin 8 | Sigma (MO) | 1:100 |
| Cytokeratin-7 | Santa Cruz Biotechnology (CA) | 1:100 |
| Cytokeratin 19 | Sigma (MO) | 1:100 |
| E-Cadherin | BD Transduction Laboratories (CA) | 1:100 |
| FOXA1 | Chemicon International (CA) | 1:100 |
| GATA-6 | R&D Systems (MN) | 1:100 |
| HES-1 | Chemicon International (CA) | 1:100 |
| HES-2 | Chemicon International (CA) | 1:100 |
| HNF-1 alpha | BD Transduction Laboratories (CA) | 1:100 |
| HNF-1 beta | BD Transduction Laboratories (CA) | 1:100 |
| Musashi-1 | Chemicon International (CA) | 1:100 |
| Nestin | R&D Systems (MN) | 1:100 |
| Pan-Cytokeratin (CK, 4, 5, 6, 10, 13, and 18) | Santa Cruz Biotechnology (CA) | 1:100 |
| SOX-17 | R&D Systems (MN) | 1:100 |
| SSEA-4 | Santa Cruz Biotechnology (CA) | 1:100 |
| TRA 1-81 | Santa Cruz Biotechnology (CA) | 1:100 |
| Vimentin | Santa Cruz Biotechnology (CA) | 1:100 |
| ZO-1 | BD Transduction Laboratories (CA) | 1:100 |

**TABLE IV A: CELL SURFACE RECEPTORS ON FIBROBLAST, AMNIOTIC FLUID, AND EPITHELIAL CELLS DERIVED FROM ANMNIOTIC FLUID AND GROWN UNDER HYPOXIC CONDITIONS**

| | Fibroblast cells (F) | Amniotic fluid cells (AF-I) | Amniotic fluid cells (AF-II) | Amniotic fluid cells (AF-III) | Epithelial cells (E) |
|---|---|---|---|---|---|
| Alpha 3 integrin | + | + | + | + | + |
| Beta 1 integrin | + | + | + | + | + |
| Beta 3 integrin | - | + | Weak | Weak | + |
| Alpha V Beta 3 (CD51/ 61) | - | + | Weak | Weak | + |
| C-Met | | + | + | + | |
| CD24 | Weak | + | Weak | Weak | + |
| CD10 | + | Weak | - | - | - |
| CD105 | + | Weak | + | + | - |
| CD117 | - | - | - | - | - |
| CD13 | + | Weak | + | + | - |
| CD44 | + | + | + | + | + |
| CD73 | + | + | + | + | + |
| CD90 | + | + | + | + | + |
| EpCAM | - | + | - | - | + |
| HLA ABC | + | + | + | + | + |
| HLA II-DR | - | - | - | - | - |
| TRA2-49 | - | - | - | - | - |
| SSEA-4 | + | + | + | + | + |
| SSEA-3 | - | + | - | - | + |
| TRA1-60 | - | + | - | - | + |
| TRA 1-81 | - | + | - | - | + |

**TABLE IV B: INTRACELLULAR PROTEINS OF VARIOUS AMNIOTIC FLUID CELL TYPES GROWN IN MEDIA #5**

| Marker | Fibroblast cells (F) | Amniotic fluid cells (AF-I) | Amniotic fluid cells (AF-II) | Amniotic fluid cells (AF-III) | Epithelial cells (E) |
|---|---|---|---|---|---|
| CK-19 | - | + | - | - | + |
| Vimentin | + | + | + | + | + |
| Smooth muscle actin | Weak | Weak | Weak | Weak | + |
| Beta III tubulin | + | + | + | + | + |
| Pan cytokeratin | - | + | + | - | + |
| Cytokeratin 7 | - | + | | | |
| Cytokeratin 8 | - | + | | | |
| Cytokeratin 18 | - | + | | | |
| Nestin | - | + | + | + | Weak |

**TABLE IV C: CELL SURFACE RECEPTORS ON AMNIOTIC FLUID CELLS DERIVED FROM TERM ANMNIOTIC FLUID AND GROWN UNDER HYPOXIC CONDITIONS**

| | Amniotic fluid cells (AF-I) |
|---|---|
| Alpha 3 integrin | + |
| Beta 1 integrin | + |
| Beta 3 integrin | + |
| Alpha V Beta 3 (CD51/61) | + |
| CD10 | Weak |
| CD105 | Weak |
| CD117 | - |
| CD13 | Weak |
| CD44 | + |
| CD73 | + |
| CD90 | + |
| EpCAM | + |
| HLA ABC | + |
| HLA II-DR | - |
| SSEA-4 | + |
| TRA1-60 | + |
| TRA 1-81 | + |

**TABLE V: PCR ANALYSIS OF THE AMNIOTIC FLUID-DERIVED CELLS OF THE PRESENT INVENTION**

| a. Pluripotency markers | | | | | | |
|---|---|---|---|---|---|---|
| **Markers** | **Fibroblast Cells** | **Epithelial cells** | **AF-I Cells** | **AF-II Cells** | **AF-III Cells** | **Ntera-1** |
| Oct3/4 | - | - | - | - | - | + |
| Sox-2 | - | - | - | - | - | + |
| Rex-1 | - | + | +/- | | | + |
| hTERT | - | +/- | - | - | - | + |

| b. Endocrine markers | | | | | | |
|---|---|---|---|---|---|---|
| Cell Markers | Fibroblast cells | Epithelial cell | AF_I cells | AF_II cells | AF_III cells | |
| Sox 17 | Weak | + | + | - | - | |
| GATA-6 | - | + | + | + | + | |
| GATA-4 | Weak | + | Weak | - | - | |
| HNF 1 Beta | - | + | + | + | - | |
| HNF3 beta | - | + | Weak | - | - | |
| Pdx-1 | - | - | - | - | - | |
| NGN-3 | - | - | - | - | - | |
| Musashi-1 | + | + | + | | | |
| HES-1 | + | + | + | + | + | |
| NeuroD1 | - | - | - | - | - | |
| Pax 4 | - | - | - | | | |
| Pax6 | + | + | + | - | - | |
| Secretin | - | - | - | - | - | |
| Gastric inhibitory peptide (GIP) | + | + | + | - | - | |
| Glucagon | - | - | - | - | - | |
| Somatostatin | + | + | + | - | - | |
| Cholecystokinin | - | - | - | - | - | |
| Gastrin | - | - | - | - | - | |
| Insulin | | - | Weak | - | - | |
| Nestin | + | + | + | + | + | |

| | | | | | | |
|---|---|---|---|---|---|---|
| *Weak: CT values of 35-38 cycles +: CT value <35 cycles | | | | | | |

**TABLE VI: PRESENCE OF ALL AF, E, AND F MORPHOLOGIES AT VARIOUS GESTATIONAL AGES IN SECOND TRIMESTER AMNIOTIC FLUID SAMPLES.**

| AF designation | Gestation age | Presence of AF-like morphology | Presence of E-like morphology | Presence of F-like morphology |
|---|---|---|---|---|
| AFCA001 | 17 wks | + | + | + |
| AFCA002 | 18 wks | + | + | - |
| AFCA004 | 18 wks | + | + | - |
| AFCA008 | 18 wks | + | + | - |
| AFDX001 | 19 wks | + | + | + |
| AFDX021 | 20 wks | + | + | + |
| AFDX022 | 20 wks | + | + | + |
| AFCA007 | 19 wks | + | + | - |
| AFCA009 | 18 wks | + | + | - |
| AFCA010 | 18 wks | + | + | - |
| AFCA011 | 19 wks | + | + | - |
| AFCA017 | 16 wks | + | + | - |
| AFPN003 | 20 wks | + | + | - |
| AFPN004 | 20 wks | + | + | - |
| AFND001 | 41 wks | + | + | - |

**TABLE VII A: GENES THAT WERE DIFFERENTIALLY EXPRESSED AT LEAST 5 FOLD IN FIBROBLAST VERSUS AF-I CELLS**

| Gene Identifier | Gene Name | Average fold change in fibroblast versus AF cells | Direction | adj. p-value |
|---|---|---|---|---|
| NM_002421 | Homo sapiens matrix metalloproteinase 1 (interstitial collagenase) (MMP1), mRNA | 434.28 | UP | 1.09E-03 |
| NM_144594 | Homo sapiens hypothetical protein FLJ32942 (FLJ32942), mRNA | 207.74 | UP | 2.52E-03 |
| NM_020927 | Homo sapiens KIAA1576 protein (KIAA1576), mRNA | 200.11 | UP | 5.06E-04 |
| NM_001451 | Homo sapiens forkhead box F1 (FOXF1), mRNA | 183.69 | UP | 1.52E-03 |
| AK021543 | Homo sapiens cDNA FLJ11481 fis, clone HEMBA1001803 | 131.1 | UP | 3.11E-03 |
| NM_007036 | Homo sapiens endothelial cell-specific molecule 1 (ESM1), mRNA | 120.15 | UP | 2.07E-03 |
| NM_002448 | Homo sapiens msh homeo box homolog 1 (Drosophila) (MSX1), mRNA | 112.45 | UP | 7.69E-04 |
| NM_152270 | Homo sapiens hypothetical protein FLJ34922 (FLJ34922), mRNA | 109.54 | UP | 1.63E-03 |
| NM_000474 | Homo sapiens twist homolog 1 (acrocephalosyndactyly 3; Saethre-Chotzen syndrome) (Drosophila) (TWIST1), mRNA | 102.84 | UP | 1.72E-03 |
| AK122739 | Homo sapiens cDNA FLJ16260 fis, clone IMR322006947, highly similar to Rattus norvegicus mRNA for dHand protein | 98.04 | UP | 4.82E-03 |
| AV702977 | AV702977 ADB Homo sapiens cDNA clone ADBCVD08 5, mRNA sequence | 93.67 | UP | 2.91 E-03 |
| AK026784 | Homo sapiens cDNA: FLJ23131 fis, clone LNG08502 | 87.77 | UP | 1.52E-03 |
| NM_000710 | Homo sapiens bradykinin receptor B1 (BDKRB1), mRNA | 86.86 | UP | 4.77E-03 |
| NM_000609 | Homo sapiens chemokine (C-X-C motif) ligand 12 (stromal cell-derived factor 1) (CXCL12), mRNA | 77.16 | UP | 2.97E-03 |
| NM_030781 | Homo sapiens collectin sub-family member 12 (COLEC12), transcript variant II, mRNA | 64.54 | UP | 8.99E-05 |
| NM 032638 | Homo sapiens GATA binding protein 2 (GATA2), mRNA | 63.54 | UP | 3.35E-03 |
| NM_000362 | Homo sapiens tissue inhibitor of metalloproteinase 3 (Sorsby fundus dystrophy, pseudoinflammatory) (TIMP3), mRNA | 61.32 | UP | 5.06E-03 |
| NM_198148 | Homo sapiens carboxypeptidase X (M14 family), member 2 (CPXM2), mRNA | 57.01 | UP | 4.00E-03 |
| AL831863 | Homo sapiens mRNA; cDNA DKFZp761J2017 (from clone DKFZp761J2017) | 55.07 | UP | 7.57E-03 |
| NM_002091 | Homo sapiens gastrin-releasing peptide (GRP), mRNA | 53.77 | UP | 9.01E-03 |
| NM_020404 | Homo sapiens CD164 sialomucin-like 1 (CD164L1), mRNA | 51.44 | UP | 2.22E-04 |
| NM_014178 | Homo sapiens syntaxin binding protein 6 (amisyn) (STXBP6), mRNA | 47.27 | UP | 7.17E-03 |
| NM_205855 | Homo sapiens HWKM1940 (UNQ1940), mRNA | 42.6 | UP | 2.31E-03 |
| BX089019 | BX089019 Soares_testis_NHT Homo sapiens cDNA clone IMAGp998K243513 ; IMAGE:1391375, mRNA sequence | 42.17 | UP | 8.50E-03 |
| AI124557 | am58g02.x1 Johnston frontal cortex Homo sapiens cDNA clone IMAGE:1539794 3, mRNA sequence | 37.2 | UP | 8.24E-03 |
| NM_139211 | Homo sapiens homeodomain-only protein (HOP), transcript variant 2, mRNA | 34.14 | UP | 1.45E-04 |
| NM_002593 | Homo sapiens procollagen C-endopeptidase enhancer (PCOLCE), mRNA | 30.85 | UP | 2.64E-03 |
| BQ020357 | UI-H-ED0-axk-p-07-0-Ul.s1 NCI_CGAP_ED0 Homo sapiens cDNA clone IMAGE:5830134 3, mRNA sequence | 30.74 | UP | 5.91E-03 |
| CA843592 | ir49c12.x1 HR85 islet Homo sapiens cDNA clone IMAGE:6548544 3, mRNA sequence | 29.57 | UP | 6.22E-05 |
| NM_002852 | Homo sapiens pentaxin-related gene, rapidly induced by IL-1 beta (PTX3), mRNA | 29.35 | UP | 3.55E-03 |
| NM_000089 | Homo sapiens collagen, type I, alpha 2 (COL1A2), mRNA | 28.94 | UP | 4.61E-04 |
| CD677332 | ho15f06.y1 Human Trabecular meshwork cDNA: hohphq Homo sapiens cDNA clone ho15f06 5, mRNA sequence | 28.67 | UP | 1.15E-04 |
| BC030692 | Homo sapiens ELAV (embryonic lethal, abnormal vision, Drosophila)-like 2 (Hu antigen B), mRNA (cDNA clone MGC:26319 IMAGE:4826082), complete cds | 28.22 | UP | 7.64E-03 |
| AK021543 | Homo sapiens cDNA FLJ11481 fis, clone HEMBA1001803 | 27.4 | UP | 1.70E-02 |
| AI962169 | wq45c10.x1 NCI_CGAP_GC6 Homo sapiens cDNA clone IMAGE:2474226 3, mRNA sequence | 27.1 | UP | 8.11E-03 |
| NM_000685 | Homo sapiens angiotensin II receptor, type 1 (AGTR1 transcript variant 1, mRNA | 26.59 | UP | 4.86E-03 |
| AI422199 | tf58d04.x1 NCI_CGAP_Brn23 Homo sapiens cDNA clone IMAGE:2103463 3, mRNA sequence | 26.11 | UP | 1.04E-02 |
| NM_0010022 95 | Homo sapiens GATA binding protein 3 (GATA3), transcript variant 1, mRNA | 24.62 | UP | 3.24E-03 |
| NM_000396 | Homo sapiens cathepsin K (pycnodysostosis) (CTSK), mRNA | 24.58 | UP | 4.37E-05 |
| NM_001442 | Homo sapiens fatty acid binding protein 4, adipocyte (FABP4), mRNA | 24.37 | UP | 8.07E-03 |
| NM_004460 | Homo sapiens fibroblast activation protein, alpha (FAP), mRNA | 24.15 | UP | 1.93E-04 |
| AB067499 | Homo sapiens mRNA for KIAA1912 protein, partial cds | 23.83 | UP | 2.51E-03 |
| NM_032777 | Homo sapiens G protein-coupled receptor 124 (GPR124), mRNA | 22.29 | UP | 3.83E-03 |
| U83115 | Human non-lens beta gamma-crystallin like protein (AIM1) mRNA, partial cds | 21.93 | UP | 5.28E-04 |
| AY335938 | Homo sapiens homeodomain protein IRXA1 (IRX1) mRNA, complete cds | 21.54 | UP | 2.02E-02 |
| NM_006350 | Homo sapiens follistatin (FST), transcript variant FST317, mRNA | 21.53 | UP | 2.20E-03 |
| W38393 | zb15c07.r1 Soaresfetal_lung_NbHL19W Homo sapiens cDNA clone IMAGE:302124 5, mRNA sequence | 21.16 | UP | 8.46E-05 |
| NM_021637 | Homo sapiens transmembrane protein 35 (TMEM35), mRNA | 20.78 | UP | 3.74E-04 |
| AK091731 | Homo sapiens cDNA FLJ34412 fis, clone HEART2002432 | 20.71 | UP | 1.19E-02 |
| NM_032883 | Homo sapiens chromosome 20 open reading frame 100 (C20orf100), mRNA | 20.29 | UP | 2.33E-04 |
| NM_005110 | Homo sapiens glutamine-fructose-6-phosphate transaminase 2 (GFPT2), mRNA | 19.69 | UP | 9.13E-05 |
| NM_024633 | Homo sapiens chromosome 14 open reading frame 139 (C14orf139), mRNA | 19.44 | UP | 1.05E-02 |
| NM_004811 | Homo sapiens leupaxin (LPXN), mRNA | 18.74 | UP | 1.74E-05 |
| NM_153183 | Homo sapiens nudix (nucleoside diphosphate linked moiety X)-type motif 10 (NUDT10), mRNA | 18.51 | UP | 9.88E-03 |
| NM_014459 | Homo sapiens protocadherin 17 (PCDH17), mRNA | 18.39 | UP | 1.39E-03 |
| BX115659 | BX115659 Soares_total_fetus_Nb2HF8_9w Homo sapiens cDNA clone IMAGp998C204119 ; IMAGE:1623883, mRNA sequence | 18.15 | UP | 4.07E-04 |
| NM_018013 | Homo sapiens hypothetical protein FLJ10159 (FLJ10159), mRNA | 17.64 | UP | 1.54E-02 |
| BX112628 | BX112628 Soares_fetal_lung_NbHL19W Homo sapiens cDNA clone IMAGp998A09669 ; IMAGE:299024, mRNA sequence | 16.09 | UP | 2.35E-02 |
| NM_016428 | Homo sapiens ABI gene family, member 3 (ABI3), mRNA | 16.07 | UP | 1.15E-02 |
| NM_012449 | Homo sapiens six transmembrane epithelial antigen of the prostate (STEAP), mRNA | 16.07 | UP | 1.49E-04 |
| NM_006350 | Homo sapiens follistatin (FST), transcript variant FST317, mRNA | 15.98 | UP | 6.30E-05 |
| AF052115 | Homo sapiens clone 23688 mRNA sequence | 15.92 | UP | 1.56E-02 |
| NM_004787 | Homo sapiens slit homolog 2 (Drosophila) (SLIT2), mRNA | 15.9 | UP | 4.75E-04 |
| AK091336 | Homo sapiens cDNA FLJ34017 fis, clone FCBBF2002626 | 15.59 | UP | 2.68E-02 |
| NM_006329 | Homo sapiens fibulin 5 (FBLN5), mRNA | 15.59 | UP | 7.87E-03 |
| NM_000963 | Homo sapiens prostaglandin-endoperoxide synthase 2 (prostaglandin G/H synthase and cyclooxygenase) (PTGS2), mRNA | 15.57 | UP | 8.59E-04 |
| NM_001146 | Homo sapiens angiopoietin 1 (ANGPT1), transcript variant 1, mRNA | 14.89 | UP | 9.40E-04 |
| NM_018431 | Homo sapiens docking protein 5 (DOK5), transcript variant 1, mRNA | 14.56 | UP | 8.69E-03 |
| NM_017577 | Homo sapiens hypothetical protein DKFZp434C0328 (DKFZp434C0328), mRNA | 14.47 | UP | 4.20E-03 |
| H85497 | yv88b07.r1 Soares melanocyte 2NbHM Homo sapiens cDNA clone IMAGE:249781 5, mRNA sequence | 13.8 | UP | 1.13E-04 |
| NM_001147 | Homo sapiens angiopoietin 2 (ANGPT2), mRNA | 13.76 | UP | 2.64E-02 |
| NM_000090 | Homo sapiens collagen, type III, alpha 1 (Ehlers-Danlos syndrome type IV, autosomal dominant) (COL3A1 mRNA | 13.63 | UP | 1.65E-02 |
| NM_007289 | Homo sapiens membrane metallo-endopeptidase (neutral endopeptidase, enkephalinase, CALLA, CD10) (MME), transcript variant 2b, mRNA | 13.35 | UP | 1.86E-02 |
| NM_016229 | Homo sapiens cytochrome b5 reductase b5R.2 (CYB5R2), transcript variant 1, mRNA | 13.35 | UP | 9.80E-03 |
| NM_000810 | Homo sapiens gamma-aminobutyric acid (GABA) A receptor, alpha 5 (GABRA5), mRNA | 13.07 | UP | 2.71 E-02 |
| NM_002518 | Homo sapiens neuronal PAS domain protein 2 (NPAS2), mRNA | 12.99 | UP | 1.04E-02 |
| AK093256 | Homo sapiens cDNA FLJ35937 fis, clone TEST12011480 | 12.93 | UP | 2.09E-03 |
| NM_005127 | Homo sapiens C-type (calcium dependent, carbohydrate-recognition domain) lectin, superfamily member 2 (activation-induced) (CLECSF2), mRNA | 12.82 | UP | 2.71E-02 |
| NM_006209 | Homo sapiens ectonucleotide pyrophosphatase/phosphodiesteras e 2 (autotaxin) (ENPP2), mRNA | 12.62 | UP | 2.60E-02 |
| W03013 | za02c04.r1 Soares melanocyte 2NbHM Homo sapiens cDNA clone IMAGE:291366 5 similar to contains THR.t3 THR repetitive element ;, mRNA sequence | 12.43 | UP | 7.69E-04 |
| AF131813 | Homo sapiens clone 24970 mRNA sequence | 12.17 | UP | 2.57E-02 |
| BQ025821 | UI-1-BB1 p-aye-f-10-0-ULs1 NCI_CGAP_PI6 Homo sapiens cDNA clone UI-1-BB1 p-aye-f-10-0-UI 3, mRNA sequence | 12.15 | UP | 1.30E-02 |
| AW445209 | UI-H-BI3-akc-g-11-0-UI.s1 NCI-CGAP-Sub5 Homo sapiens cDNA clone IMAGE:2733908 3, mRNA sequence | 12.09 | UP | 8.91 E-04 |
| NM_033292 | Homo sapiens caspase 1, apoptosis-related cysteine protease (interleukin 1, beta, convertase) (CASP1 transcript variant alpha, mRNA | 11.63 | UP | 2.67E-02 |
| NM_170744 | Homo sapiens unc-5 homolog B (C. elegans) (UNC5B), mRNA | 11.62 | UP | 3.68E-02 |
| NM_012204 | Homo sapiens general transcription factor IIIC, polypeptide 4, 90kDa (GTF3C4), mRNA | 11.59 | UP | 3.11E-02 |
| NM_002531 | Homo sapiens neurotensin receptor 1 (high affinity) (NTSR1), mRNA | 11.33 | UP | 2.65E-02 |
| BG118019 | 602351269F1 NIH_MGC_90 Homo sapiens cDNA clone IMAGE:4446065 5, mRNA sequence | 11.16 | UP | 8.51 E-05 |
| NM_014802 | Homo sapiens KIAA0528 gene product (KIAA0528), mRNA | 11.05 | UP | 2.84E-02 |
| BX647541 | Homo sapiens mRNA; cDNA DKFZp686P0492 (from clone DKFZp686P0492) | 10.9 | UP | 1.65E-03 |
| NM_020809 | Homo sapiens Rho GTPase activating protein 20 (ARHGAP20), mRNA | 10.85 | UP | 2.74E-02 |
| NM_016307 | Homo sapiens paired related homeobox 2 (PRRX2), mRNA | 10.85 | UP | 7.71E-05 |
| NM_057179 | Homo sapiens twist homolog 2 (Drosophila) (TWIST2), mRNA | 10.83 | UP | 7.22E-05 |
| AK128325 | Homo sapiens cDNA FLJ46467 fis, clone THYMU3022668 | 10.82 | UP | 1.20E-02 |
| BE866150 | 601679068F1 NIH_MGC_53 Homo sapiens cDNA clone IMAGE:3961768 5, mRNA sequence | 10.74 | UP | 2.16E-02 |
| BX109483 | BX109483 NCI_CGAP_Ov23 Homo sapiens cDNA clone IMAGp998C165481 ; IMAGE:2216391, mRNA sequence | 10.72 | UP | 2.65E-02 |
| C02345 | HUMGS0007544 Human adult (K.Okubo) Homo sapiens cDNA, mRNA sequence | 10.64 | UP | 2.15E-03 |
| NM_002961 | Homo sapiens S100 calcium binding protein A4 (calcium protein, calvasculin, metastasin, murine placental homolog) (S100A4), transcript variant 1, mRNA | 10.61 | UP | 1.87E-04 |
| NM_031908 | Homo sapiens C1q and tumor necrosis factor related protein 2 (C1QTNF2), mRNA | 10.13 | UP | 3.04E-02 |
| NM_013387 | Homo sapiens ubiquinol-cytochrome c reductase complex (7.2 kD) (HSPC051), transcript variant 1, mRNA | 9.95 | UP | 4.17E-04 |
| BC039369 | Homo sapiens, clone IMAGE:5271073, mRNA, partial cds | 9.76 | UP | 4.82E-03 |
| BQ934941 | AGENCOURT_8810373 NIH MGC 101 Homo sapiens cDNA clone IMAGE:6429485 5, mRNA sequence | 9.53 | UP | 2.21E-02 |
| NM_006670 | Homo sapiens trophoblast glycoprotein (TPBG), mRNA | 9.37 | UP | 2.37E-04 |
| BC046364 | Homo sapiens flavoprotein oxidoreductase MICAL3, mRNA (cDNA clone IMAGE:5737121), with apparent retained intron | 9.09 | UP | 2.55E-02 |
| NM_024600 | Homo sapiens chromosome 16 open reading frame 30 (C16orf30), mRNA | 9.09 | UP | 4.32E-02 |
| AK023647 | Homo sapiens cDNA FLJ13585 fis, clone PLACE1009150 | 8.81 | UP | 2.25E-03 |
| NM_017805 | Homo sapiens Ras interacting protein 1 (RASIP1), mRNA | 8.78 | UP | 2.38E-03 |
| NM_152399 | Homo sapiens hypothetical protein FLJ30834 (FLJ30834), mRNA | 8.73 | UP | 2.18E-02 |
| NM_002851 | Homo sapiens protein tyrosine phosphatase, receptor-type, Z polypeptide 1 (PTPRZ1), mRNA | 8.61 | UP | 5.08E-02 |
| AK024653 | Homo sapiens cDNA: FLJ21000 fis, clone CAE03359 | 8.51 | UP | 6.29E-03 |
| NM_020226 | Homo sapiens PR domain containing 8 (PRDM8), mRNA | 8.48 | UP | 1.49E-04 |
| NM_012307 | Homo sapiens erythrocyte membrane protein band 4.1-like 3 (EPB41 L3), mRNA | 8.41 | UP | 7.37E-03 |
| NM_002203 | Homo sapiens integrin, alpha 2 (CD49B, alpha 2 subunit of VLA-2 receptor) (ITGA2), mRNA | 8.3 | UP | 8.37E-05 |
| NM_203370 | Homo sapiens similar to RIKEN cDNA 6530418L21 (LOC389119), mRNA | 8.27 | UP | 8.21 E-05 |
| NM_015192 | Homo sapiens phospholipase C, beta 1 (phosphoinositide-specific) (PLCB1), transcript variant 1, mRNA | 8.26 | UP | 2.64E-03 |
| A056725 | Homo sapiens cDNA FLJ32163 fis, clone PLACE6000371 | 8.22 | UP | 1.68E-04 |
| NM_005328 | Homo sapiens hyaluronan synthase 2 (HAS2), mRNA | 8.2 | UP | 4.39E-02 |
| NM_012294 | Homo sapiens Rap guanine nucleotide exchange factor (GEF) 5 (RAPGEF5), mRNA | 8.08 | UP | 4.94E-02 |
| AA187037 | zp58e04.r1 Stratagene endothelial cell 937223 Homo sapiens cDNA clone IMAGE:624414 5, mRNA sequence | 7.95 | UP | 1.09E-02 |
| NM_001463 | Homo sapiens frizzled-related protein (FRZB), mRNA | 7.94 | UP | 1.31E-03 |
| BM468332 | AGENCOURT_6432296 NIH_MGC_71 Homo sapiens cDNA clone IMAGE:5535773 5, mRNA sequence | 7.94 | UP | 7.42E-05 |
| AK096661 | Homo sapiens cDNA FLJ39342 fis, clone OCBBF2018873 | 7.82 | UP | 2.30E-03 |
| NM 001849 | Homo sapiens collagen, type VI, alpha 2 (COL6A2), transcript variant 2C2, mRNA | 7.67 | UP | 1.10E-04 |
| BC071787 | Homo sapiens cDNA clone IMAGE:4610527, partial cds | 7.59 | UP | 1.14E-04 |
| NM_012105 | Homo sapiens beta-site APP-cleaving enzyme 2 (BACE2), transcript variant a, mRNA | 7.58 | UP | 2.00E-04 |
| NM_014217 | Homo sapiens potassium channel, subfamily K, member 2 (KCNK2), mRNA | 7.58 | UP | 4.61E-02 |
| N28431 | yx35c03.r1 Soares melanocyte 2NbHM Homo sapiens cDNA clone IMAGE:263716 5 similar to SP:PIR:S32603 S32603 collagen alpha 1(VI) chain - mouse ;, mRNA sequence | 7.47 | UP | 1.75E-02 |
| NM_012445 | Homo sapiens spondin 2, extracellular matrix protein (SPON2), mRNA | 7.22 | UP | 4.03E-02 |
| NM_000304 | Homo sapiens peripheral myelin protein 22 (PMP22), transcript variant 1, mRNA | 7.02 | UP | 4.58E-02 |
| NM_004791 | Homo sapiens integrin, beta-like 1 (with EGF-like repeat domains) (ITGBL1), mRNA | 7.02 | UP | 2.64E-02 |
| NM_053044 | Homo sapiens serine protease HTRA3 (HTRA3), mRNA | 6.86 | UP | 1.13E-03 |
| BC028245 | Homo sapiens, Similar to hypothetical gene LOC130797, clone IMAGE:5395354, mRNA | 6.86 | UP | 1.28E-02 |
| NM_023003 | Homo sapiens transmembrane 6 superfamily member 1 (TM6SF1), mRNA | 6.86 | UP | 2.12E-03 |
| AJ420536 | Homo sapiens mRNA full length insert cDNA clone EUROIMAGE 994183 | 6.79 | UP | 3.53E-04 |
| NM_152996 | Homo sapiens sialyltransferase 7 ((alpha-N-acetylneuraminyl-2,3-beta-galactosyl-1,3)-N-acetyl galactosaminide alpha-2,6-sialyltransferase) C (SIAT7C), mRNA | 6.79 | UP | 4.08E-02 |
| NM_005583 | Homo sapiens lymphoblastic leukemia derived sequence 1 (LYL1), mRNA | 6.77 | UP | 1.92E-02 |
| NM_016270 | Homo sapiens Kruppel-like factor 2 (lung) (KLF2), mRNA | 6.55 | UP | 5.02E-02 |
| NM_005397 | Homo sapiens podocalyxin-like (PODXL), mRNA | 6.53 | UP | 3.68E-03 |
| NM_006182 | Homo sapiens discoidin domain receptor family, member 2 (DDR2), mRNA | 6.52 | UP | 8.46E-05 |
| U79271 | Human clones 23920 and 23921 mRNA sequence | 6.31 | UP | 8.59E-05 |
| BC036034 | Homo sapiens endothelial differentiation, lysophosphatidic acid G-protein-coupled receptor, 2, transcript variant 2, mRNA (cDNA clone MGC:33157 IMAGE:5272431), complete cds | 6.3 | UP | 3.07E-04 |
| NM_152314 | Homo sapiens hypothetical protein MGC34830 (MGC34830), mRNA | 6.29 | UP | 2.93E-04 |
| AK095791 | Homo sapiens cDNA FLJ38472 fis, clone FEBRA2022148 | 6.27 | UP | 1.74E-03 |
| NM_004840 | Homo sapiens Rac/Cdc42 guanine nucleotide exchange factor (GEF) 6 (ARHGEF6), mRNA | 6.2 | UP | 1.32E-03 |
| AL833655 | Homo sapiens mRNA; cDNA DKFZp66700320 (from clone DKFZp667O0320) | 6.18 | UP | 3.01E-02 |
| NM_032270 | Homo sapiens factor for adipocyte differentiation 158 (FAD158), mRNA | 6.1 | UP | 6.22E-05 |
| NM_021643 | Homo sapiens tribbles homolog 2 (Drosophila) (TRIB2), mRNA | 5.97 | UP | 8.59E-05 |
| AW044647 | wy78e09.x1 Soares_NSF_F8_9W_OT_PA_P_S1 Homo sapiens cDNA clone IMAGE:2554696 3, mRNA sequence | 5.94 | UP | 4.09E-02 |
| AK125284 | Homo sapiens cDNA FLJ43294 fis, clone MESTC1000042 | 5.85 | UP | 3.94E-03 |
| NM_005069 | Homo sapiens single-minded homolog 2 (Drosophila) (SIM2), transcript variant SIM2, mRNA | 5.84 | UP | 8.35E-05 |
| NM_018836 | Homo sapiens transmembrane protein SHREW1 (SHREW1), mRNA | 5.83 | UP | 2.71E-02 |
| NM_014932 | Homo sapiens neuroligin 1 (NLGN1), mRNA | 5.83 | UP | 2.45E-03 |
| NM_006307 | Homo sapiens sushi-repeat-containing protein, X-linked (SRPX), mRNA | 5.77 | UP | 4.30E-04 |
| NM_000210 | Homo sapiens integrin, alpha 6 (ITGA6), mRNA | 5.77 | UP | 7.49E-05 |
| BG208475 | RST27977 Athersys RAGE Library Homo sapiens cDNA, mRNA sequence | 5.72 | UP | 1.46E-03 |
| N36786 | yy34e08.s1 Soares melanocyte 2NbHM Homo sapiens cDNA clone IMAGE:273158 3 similar to contains element MSR1 repetitive element ;, mRNA sequence | 5.67 | UP | 9.13E-05 |
| NM_005308 | Homo sapiens G protein-coupled receptor kinase 5 (GRK5), mRNA | 5.58 | UP | 1.35E-02 |
| AB037722 | Homo sapiens mRNA for KIAA1301 protein, partial cds | 5.58 | UP | 9.18E-04 |
| NM_012242 | Homo sapiens dickkopf homolog 1 (Xenopus laevis) (DKK1), MRNA, | 5.57 | UP | 1.68E-04 |
| NM_003068 | Homo sapiens snail homolog 2 (Drosophila) (SNAI2), mRNA | 5.53 | UP | 7.42E-05 |
| NM_080806 | Homo sapiens collagen, type XIII, alpha 1 (COL13A1), transcript variant 10, mRNA | 5.52 | UP | 8.65E-05 |
| M_173553 | Homo sapiens hypothetical protein FLJ25801 (FLJ25801), mRNA | 5.48 | UP | 1.77E-02 |
| AI085016 | ow88e06.s1 Soares_fetal_liver_spleen_1 NFLS_ S1 Homo sapiens cDNA clone IMAGE:1653922 3, mRNA sequence | 5.45 | UP | 4.77E-03 |
| NM_182728 | Homo sapiens solute carrier family 7 (cationic amino acid transporter, y+ system), member 8 (SLC7A8), transcript variant 2, mRNA | 5.34 | UP | 2.70E-02 |
| BC035066 | Homo sapiens, clone IMAGE:5259543, mRNA | 5.29 | UP | 2.24E-02 |
| NM_016247 | Homo sapiens interphotoreceptor matrix proteoglycan 2 (IMPG2), mRNA | 5.22 | UP | 2.97E-03 |
| NM_001704 | Homo sapiens brain-specific angiogenesis inhibitor 3 (BAI3), mRNA | 5.19 | UP | 1.08E-02 |
| NM_005226 | Homo sapiens endothelial differentiation, sphingolipid G-protein-coupled receptor, 3 (EDG3), mRNA | 5.18 | UP | 1.53E-04 |
| NM_017413 | Homo sapiens apelin, AGTRL1 ligand (APLN), mRNA | 5.14 | UP | 2.48E-02 |
| NM_000576 | Homo sapiens interleukin 1, beta (IL1B), mRNA | 5.1 | UP | 3.32E-03 |
| NM_012307 | Homo sapiens erythrocyte membrane protein band 4.1-like 3 (EPB41L3), mRNA | 5.09 | UP | 4.93E-04 |
| AL831835 | Homo sapiens mRNA; cDNA DKFZp547A0515 (from clone DKFZp547A0515) | 5.04 | UP | 4.14E-02 |
| NM_000955 | Homo sapiens prostaglandin E receptor 1 (subtype EP1), 42kDa (PTGER1), mRNA | 5.02 | UP | 3.12E-03 |
| NM_018658 | Homo sapiens potassium inwardly-rectifying channel, subfamily J, member 16 (KCNJ16), transcript variant 1, mRNA | 334.17 | Down | 8.59E-05 |
| NM_007038 | Homo sapiens a disintegrin-like and metalloprotease (reprolysin type) with thrombospondin type 1 motif, 5 (aggrecanase-2) (ADAMTS5), mRNA | 250.9 | Down | 3.32E-05 |
| BG219729 | RST39494 Athersys RAGE Library Homo sapiens cDNA, mRNA sequence | 182.25 | Down | 6.10E-05 |
| AB032980 | Homo sapiens mRNA for KIAA1154 protein, partial cds | 159.9 | Down | 7.49E-05 |
| NM_022454 | Homo sapiens SRY (sex determining region Y)-box 17 (SOX17), mRNA | 151.72 | Down | 3.05E-05 |
| AK130281 | Homo sapiens cDNA FLJ26771 fis, clone PRS03189 | 148.34 | Down | 6.10E-05 |
| NM_003238 | Homo sapiens transforming growth factor, beta 2 (TGFB2), mRNA | 139.18 | Down | 7.00E-05 |
| BU734212 | UI-E-CQ1-agd-e-21-0-UI.s1 UI-E-CQ1 Homo sapiens cDNA clone UI-E-CQ1-agd-e-21-0-UI 3, mRNA sequence | 135.35 | Down | 7.49E-05 |
| BM993116 | UI-H-DTO-aty-f-17-0-UI.s1 NCI_CGAP_DT0 Homo sapiens cDNA clone IMAGE:5866000 3, mRNA sequence | 134.03 | Down | 8.59E-05 |
| BX648964 | Homo sapiens mRNA; cDNA DKFZp686J0156 (from clone DKFZp686J0156) | 133.35 | Down | 1.19E-04 |
| A1765021 | wh56c02.x1 NCI_CGAP_Kid11 Homo sapiens cDNA clone IMAGE:2384738 3, mRNA sequence | 131.86 | Down | 6.22E-05 |
| D29453 | HUMNK566 Human epidermal keratinocyte Homo sapiens cDNA clone 566, mRNA sequence | 128.83 | Down | 6.30E-05 |
| NM_002521 | Homo sapiens natriuretic peptide precursor B (NPPB), mRNA | 128.09 | Down | 1.42E-04 |
| BX102632 | BX102632 NCI_CGAP _Co3 Homo sapiens cDNA clone IMAGp998J052307 ; IMAGE:928228, mRNA sequence | 123.34 | Down | 1.15E-04 |
| NM_024508 | Homo sapiens zinc finger, BED domain containing 2 (ZBED2), mRNA | 118.4 | Down | 5.18E-05 |
| NM_153026 | Homo sapiens prickle-like 1 (Drosophila) (PRICKLE1), mRNA | 106.78 | Down | 6.10E-05 |
| NM_006228 | Homo sapiens prepronociceptin (PNOC), mRNA | 102.73 | Down | 2.87E-04 |
| NM_005560 | Homo sapiens laminin, alpha 5 (LAMA5), mRNA | 99.24 | Down | 7.49E-05 |
| NM_023942 | Homo sapiens hypothetical protein MGC3036 (MGC3036), mRNA | 91.38 | Down | 6.30E-05 |
| AK096481 | Homo sapiens cDNA FLJ39162 fis, clone OCBBF2002376 | 85.12 | Down | 7.45E-05 |
| AI335277 | tb29h06.x1 NCI_CGAP_Kid12 Homo sapiens cDNA clone IMAGE:2055803 3, mRNA sequence | 84.58 | Down | 6.22E-05 |
| BF798098 | RC1-00045-021000-021-f02 CI0045 Homo sapiens cDNA, mRNA sequence | 81.7 | Down | 8.59E-05 |
| AK023631 | Homo sapiens cDNA FLJ13569 fis, clone PLACE 1008369 | 77.57 | Down | 7.00E-05 |
| NM_000927 | Homo sapiens ATP-binding cassette, sub-family B (MDR/TAP), member 1 (ABCB1), mRNA | 77.16 | Down | 2.21E-04 |
| BG197054 | RST16291 Athersys RAGE Library Homo sapiens cDNA, mRNA sequence | 76.09 | Down | 1.03E-04 |
| AK058012 | Homo sapiens cDNA FLJ25283 fis, clone STM06716 | 75.4 | Down | 6.22E-05 |
| NM_000104 | Homo sapiens cytochrome P450, family 1, subfamily B, polypeptide 1 (CYP1B1), mRNA | 70.8 | Down | 6.24E-05 |
| NM_004617 | Homo sapiens transmembrane 4 superfamily member 4 (TM4SF4), mRNA | 63.32 | Down | 1.39E-04 |
| NM_000990 | Homo sapiens ribosomal protein L27a (RPL27A), mRNA | 62.1 | Down | 5.32E-05 |
| NM_002899 | Homo sapiens retinol binding protein 1, cellular (RBP1), mRNA | 62.01 | Down | 2.87E-04 |
| NM_000582 | Homo sapiens secreted phosphoprotein 1 (osteopontin, bone sialoprotein I, early T-lymphocyte activation 1) (SPP1), mRNA | 60.53 | Down | 1.18E-04 |
| NM_002423 | Homo sapiens matrix metalloproteinase 7 (matrilysin, uterine) (MMP7), mRNA | 60.01 | Down | 1.56E-04 |
| H89053 | yw24c06.r1 Morton Fetal Cochlea Homo sapiens cDNA clone IMAGE:253162 5, mRNA sequence | 59.02 | Down | 7.45E-05 |
| AK090808 | Homo sapiens cDNA FLJ33489 fis, clone BRAMY2003585 | 58.15 | Down | 7.49E-05 |
| NM_001200 | Homo sapiens bone morphogenetic protein 2 (BMP2), mRNA | 57.05 | Down | 6.24E-05 |
| NM_001453 | Homo sapiens forkhead box C1 (FOXC1), mRNA | 56.24 | Down | 6.10E-05 |
| AK092245 | Homo sapiens cDNA FLJ34926 fis, clone NT2RP7003319, highly similar to Mus musculus neuralin mRNA | 56.11 | Down | 2.55E-04 |
| NM_005329 | Homo sapiens hyaluronan synthase 3 (HAS3), transcript variant 1, mRNA | 55.44 | Down | 7.22E-05 |
| AI244954 | qj93h05.x1 NCI_CGAP_Kid3 Homo sapiens cDNA clone IMAGE:1867065 3, mRNA sequence | 54.38 | Down | 6.22E-05 |
| NM_003287 | Homo sapiens tumor protein D52-like 1 (TPD52L1), transcript variant 1, m RNA | 53.91 | Down | 7.45E-05 |
| BF696790 | 602125323F1 NIH_MGC_56 Homo sapiens cDNA clone IMAGE:4282540 5, mRNA sequence | 49.46 | Down | 1.09E-04 |
| NM_001202 | Homo sapiens bone morphogenetic protein 4 (BMP4), transcript variant 1, mRNA | 47.81 | Down | 8.21E-05 |
| BC042028 | Homo sapiens, clone IMAGE:4794726, mRNA | 47.5 | Down | 7.49E-05 |
| AL833276 | Homo sapiens mRNA; cDNA DKFZp451D088 (from clone DKFZp451D088) | 43.28 | Down | 7.28E-05 |
| NM_184087 | Homo sapiens tripartite motif-containing 55 (TRIM55), transcript variant 4, mRNA | 42.9 | Down | 7.42E-05 |
| NM_003494 | Homo sapiens dysferlin, limb girdle muscular dystrophy 2B (autosomal recessive) (DYSF), mRNA | 42.2 | Down | 7.31E-05 |
| NM_024422 | Homo sapiens desmocollin 2 (DSC2), transcript variant Dsc2a, mRNA | 41.3 | Down | 1.96E-04 |
| AI249696 | qj64a03.x1 NCI_CGAP_Kid3 Homo sapiens cDNA clone IMAGE:1864204 3, mRNA sequence | 41.1 | Down | 1.50E-04 |
| AL833166 | Homo sapiens mRNA; cDNA DKFZp68612118 (from clone DKFZp686I2118) | 39.95 | Down | 1.89E-04 |
| NM_173505 | Homo sapiens ankyrin repeat domain 29 (ANKRD29), mRNA | 39.16 | Down | 9.13E-05 |
| NM_130435 | Homo sapiens protein tyrosine phosphatase, receptor type, E (PTPRE), transcript variant 2, mRNA | 39.16 | Down | 2.54E-04 |
| NM_016356 | Homo sapiens doublecortin domain containing 2 (DCDC2), mRNA | 38.33 | Down | 2.10E-04 |
| AF318382 | Homo sapiens pp9974 mRNA, complete cds | 38.03 | Down | 8.59E-05 |
| NM_018265 | Homo sapiens hypothetical protein FLJ10901 (FLJ10901), mRNA | 37.23 | Down | 1.50E-04 |
| NM_152487 | Homo sapiens hypothetical protein FLJ31842 (FLJ31842), mRNA | 37 | Down | 1.56E-04 |
| AK000075 | Homo sapiens cDNA FLJ20068 fis, clone COL01755 | 35.55 | Down | 2.21 E-04 |
| NM_012464 | Homo sapiens tolloid-like 1 (TLL1), mRNA | 35.05 | Down | 2.09E-03 |
| NM_184087 | Homo sapiens tripartite motif-containing 55 (TRIM55), transcript variant 4, mRNA | 34.38 | Down | 7.49E-05 |
| BU680661 | UI-CF-DU1-aaz-f-04-0-UI.s1 UI-CF-DU1 Homo sapiens cDNA clone UI-CF-DU1-aaz-f-04-0-UI 3, mRNA sequence | 34.34 | Down | 6.22E-05 |
| BF431030 | 7o18c06.x1 NCI_CGAP_Kid11 Homo sapiens cDNA clone IMAGE:3574283 3, mRNA sequence | 34.25 | Down | 1.86E-04 |
| NM_006722 | Homo sapiens microphthalmia-associated transcription factor (MITF), transcript variant 3, mRNA | 34.23 | Down | 6.30E-05 |
| NM_152284 | Homo sapiens Snf7 homologue associated with Alix 3 (Shax3), mRNA | 33.99 | Down | 7.71E-05 |
| BC037316 | Homo sapiens, clone IMAGE:5259432, mRNA | 33.46 | Down | 2.03E-04 |
| NM_002345 | Homo sapiens lumican (LUM), mRNA | 33.34 | Down | 8.46E-05 |
| NM_080743 | Homo sapiens serine-arginine repressor protein (35 kDa) (SRrp35), mRNA | 32.96 | Down | 5.23E-05 |
| NM_004221 | Homo sapiens natural killer cell transcript 4 (NK4), mRNA | 32.74 | Down | 1.45E-04 |
| NM_004496 | Homo sapiens forkhead box A1 (FOXA1), mRNA | 32.31 | Down | 2.57E-04 |
| AA738254 | nx13b02.s1 NCI_CGAP_GC3 Homo sapiens cDNA clone IMAGE:1255947 3, mRNA sequence | 32.23 | Down | 6.10E-05 |
| BC045828 | Homo sapiens zinc finger protein 608, mRNA (cDNA clone IMAGE:5262896), partial cds | 32.18 | Down | 7.49E-05 |
| NM_000599 | Homo sapiens insulin-like growth factor binding protein 5 (IGFBP5), mRNA | 32.18 | Down | 9.81E-05 |
| AF055376 | Homo sapiens short form transcription factor C-MAF (c-maf) mRNA, complete cds | 31.81 | Down | 2.01E-04 |
| NM_015236 | Homo sapiens latrophilin 3 (LPHN3), mRNA | 31.68 | Down | 9.69E-05 |
| NM_007069 | Homo sapiens HRAS-like suppressor 3 (HRASLS3), mRNA | 31.67 | Down | 7.49E-05 |
| BG221364 | RST41175 Athersys RAGE Library Homo sapiens cDNA, mRNA sequence | 31.63 | Down | 2.22E-04 |
| AI819186 | wj32d10.x1 NCI_CGAP_Kid12 Homo sapiens cDNA clone IMAGE:2404531 3, mRNA sequence | 31.49 | Down | 1.80E-04 |
| BF512544 | UI-H-BW1-amf-c-08-0-UI.s1 NCI_CGAP_Sub7 Homo sapiens cDNA clone IMAGE:3069687 3, mRNA sequence | 30.8 | Down | 7.49E-05 |
| NM_020873 | Homo sapiens leucine rich repeat neuronal 1 (LRRN1), mRNA | 30.67 | Down | 2.01E-04 |
| CA425961 | UI-H-FE1-beg-p-18-0-UI.s1 NCI_CGAP_FE1 Homo sapiens cDNA clone UI-H-FE1-beg-p-18-0-UI 3, mRNA sequence | 30.15 | Down | 1.88E-04 |
| AK000776 | Homo sapiens cDNA FLJ20769 fis, clone COL06674 | 30.11 | Down | 1.15 E-04 |
| M_001562 | Homo sapiens interleukin 18 (interferon-gamma-inducing factor) (IL18), mRNA | 29.97 | Down | 7.65E-04 |
| NM_014333 | Homo sapiens immunoglobulin superfamily, member 4 (IGSF4), mRNA | 29.77 | Down | 5.82E-04 |
| NM_030583 | Homo sapiens matrilin 2 (MATN2), transcript variant 2, mRNA | 29.05 | Down | 8.46E-05 |
| W93585 | zd95g01.s1 Soares_fetal_heart_NbHH19W Homo sapiens cDNA clone IMAGE:357264 3, mRNA sequence | 28.93 | Down | 6.22E-05 |
| NM_012198 | Homo sapiens grancalcin, EF-hand calcium binding protein (GCA), mRNA | 28.84 | Down | 9.13E-05 |
| NM_001448 | Homo sapiens glypican 4 (GPC4), mRNA | 28.72 | Down | 6.36E-04 |
| AK056882 | Homo sapiens cDNA FLJ32320 fis, clone PROST2003537 | 28.6 | Down | 4.45E-04 |
| AB033048 | Homo sapiens mRNA for KIAA1222 protein, partial cds | 28.27 | Down | 2.41E-04 |
| NM_025074 | Homo sapiens Fraser syndrome 1 (FRAS1), transcript variant 1, mRNA | 28.13 | Down | 8.92E-05 |
| NM_019000 | Homo sapiens hypothetical protein FLJ20152 (FLJ20152), mRNA | 27.54 | Down | 1.07E-04 |
| BU729783 | UI-E-CK1-afh-h-18-0-UI.s1 UI-E-CK1 Homo sapiens cDNA clone UI-E-CK1-afh-h-18-0-UI 3, mRNA sequence | 26.13 | Down | 6.30E-05 |
| NM_004862 | Homo sapiens lipopolysaccharide-induced TNF factor (LITAF), mRNA | 25.95 | Down | 8.99E-05 |
| AB011539 | Homo sapiens mRNA for MEGF6 protein (KIAA0815), partial cds | 25.67 | Down | 3.12E-04 |
| BC043195 | Homo sapiens cDNA clone IMAGE:5288757, partial cds | 25.58 | Down | 2.22E-04 |
| NM_152864 | Homo sapiens chromosome 20 open reading frame 58 (C20orf58), mRNA | 25.35 | Down | 3.00E-04 |
| AK125608 | Homo sapiens cDNA FLJ43620 fis, clone SPLEN2021701, highly similar to HLA CLASS I HISTOCOMPATIBILITY ANTIGEN, A-2 ALPHA CHAIN PRECURSOR | 25.33 | Down | 2.03E-04 |
| AL080103 | Homo sapiens mRNA; cDNA DKFZp564N2216 (from clone DKFZp564N2216) | 25.17 | Down | 2.03E-04 |
| NM_173549 | Homo sapiens hypothetical protein FLJ39553 (FLJ39553), mRNA | 24.71 | Down | 1.67E-04 |
| BG211832 | RST31404 Athersys RAGE Library Homo sapiens cDNA, mRNA sequence | 24.57 | Down | 1.02E-04 |
| NM_024726 | Homo sapiens IQ motif containing with AAA domain (IQCA), mRNA | 24.08 | Down | 7.49E-05 |
| NM_002559 | Homo sapiens purinergic receptor P2X, ligand-gated ion channel, 3 (P2RX3), mRNA | 24.04 | Down | 2.63E-04 |
| NM_018168 | Homo sapiens chromosome 14 open reading frame 105 (C14orf105), mRNA | 23.69 | Down | 1.50E-04 |
| NM_000599 | Homo sapiens insulin-like growth factor binding protein 5 (IGFBP5), mRNA | 23.5 | Down | 4.02E-04 |
| NM_003551 | Homo sapiens non-metastatic cells 5, protein expressed in (nucleoside-diphosphate kinase) (NME5), mRNA | 23.23 | Down | 1.41E-04 |
| NM_032471 | Homo sapiens protein kinase (cAMP-dependent, catalytic) inhibitor beta (PKIB), transcript variant 3, mRNA | 22.83 | Down | 1.54E-04 |
| NM_005949 | Homo sapiens metallothionein 1 F (functional) (MT1 F), mRNA | 22.22 | Down | 8.81E-05 |
| NM_018242 | Homo sapiens hypothetical protein FLJ10847 (FLJ10847), mRNA | 22.1 | Down | 2.03E-04 |
| AW151660 | xf67d04.x1 NCI_CGAP_Gas4 Homo sapiens cDNA clone IMAGE:2623111 3, mRNA sequence | 21.81 | Down | 2.71E-04 |
| AK096975 | Homo sapiens cDNA FLJ39656 fis, clone SMINT2005956 | 21.78 | Down | 2.52E-04 |
| CA306881 | UI-H-FT1-bht-n-22-0-UI.s1 NCI_CGAP_FT1 Homo sapiens cDNA clone UI-H-FT1-bht-n-22-0-UI 3, mRNA sequence | 21.69 | Down | 1.30E-04 |
| NM_016212 | Homo sapiens TP53TG3 protein (TP53TG3), mRNA | 21.67 | Down | 9.03E-05 |
| NM_178470 | Homo sapiens WD repeat domain 40B (WDR40B), mRNA | 21.63 | Down | 7.59E-05 |
| NM_014243 | Homo sapiens a disintegrin-like and metalloprotease (reprolysin type) with thrombospondin type 1 motif, 3 (ADAMTS3), mRNA | 21.47 | Down | 8.24E-05 |
| AK126467 | Homo sapiens cDNA FLJ44503 fis, clone UTERU3001158 | 21.42 | Down | 6.22E-05 |
| AW248516 | 2820632.3prime NIH_MGC_7 Homo sapiens cDNA clone IMAGE:2820632 3, mRNA sequence | 21.37 | Down | 1.23E-04 |
| NM_001263 | Homo sapiens CDP-diacylglycerol synthase (phosphatidate cytidylyltransferase) 1 (CDS1), mRNA | 21.12 | Down | 1.10E-04 |
| BM669002 | UI-E-CK1-afn-m-04-0-UI.s2 UI-E-CK1 Homo sapiens cDNA clone UI-E-CK1-afn-m-04-0-UI 3, mRNA sequence | 20.96 | Down | 7.28E-05 |
| NM_000170 | Homo sapiens glycine dehydrogenase (decarboxylating; glycine decarboxylase, glycine cleavage system protein P) (GLDC), mRNA | 20.78 | Down | 1.19E-04 |
| NM_014585 | Homo sapiens solute carrier family 40 (iron-regulated transporter), member 1 (SLC40A1), mRNA | 20.73 | Down | 8.46E-05 |
| NM_031426 | Homo sapiens chromosome 9 open reading frame 58 (C9orf58), transcript variant 1, mRNA | 20.62 | Down | 2.00E-04 |
| BM728728 | UI-E-E01-aiv-c-02-0-UI.r1 UI-E-E01 Homo sapiens cDNA clone UI-E-EO1-aiv-c-02-0-UI 5, mRNA sequence | 20.59 | Down | 1.14E-04 |
| NM_152369 | Homo sapiens hypothetical protein MGC45474 (MGC45474), mRNA | 20.57 | Down | 9.78E-05 |
| NM_175056 | Homo sapiens hypothetical protein LOC131368 (LOC131368), mRNA | 20.51 | Down | 5.42E-04 |
| NM_004524 | Homo sapiens lethal giant larvae homolog 2 (Drosophila) (LLGL2), mRNA | 20.3 | Down | 7.45E-05 |
| AI436290 | th81c01.x1 Soares_NhHMPu_S1 Homo sapiens cDNA clone IMAGE:2125056 3, mRNA sequence | 19.93 | Down | 1.23E-04 |
| AW268540 | xv51e10.x1 NCI_CGAP_Lu28 Homo sapiens cDNA clone IMAGE:2816682 3, mRNA sequence | 19.89 | Down | 1.09E-04 |
| BC015159 | Homo sapiens cDNA clone IMAGE:3885734, partial cds | 19.47 | Down | 7.49E-05 |
| NM_020130 | Homo sapiens chromosome 8 open reading frame 4 (C8orf4), mRNA | 19.43 | Down | 2.83E-04 |
| BM979825 | UI-CF-DU1-adt-f-12-0-UI.s1 UI-CF-DU1 Homo sapiens cDNA clone UI-CF-DU1-adt-f-12-0-UI 3, mRNA sequence | 19.41 | Down | 1.01E-04 |
| BM712072 | UI-E-DW1-ahc-b-11-0-UI.r1 UI-E-DW1 Homo sapiens cDNA clone UI-E-DW1-ahc-b-11-0-UI 5, mRNA sequence | 19.33 | Down | 1.19E-04 |
| CB135276 | K-EST0187371 L5HLK1 Homo sapiens cDNA clone L5HLK1-32-B12 5, mRNA sequence | 18.98 | Down | 8.97E-05 |
| T53523 | ya89h12.r1 Stratagene placenta (#937225) Homo sapiens cDNA clone IMAGE:68903 5, mRNA sequence | 18.87 | Down | 1.96E-04 |
| NM_138432 | Homo sapiens serine dehydratase-like (SDSL), mRNA | 18.84 | Down | 1.32E-04 |
| NM_023915 | Homo sapiens G protein-coupled receptor 87 (GPR87), mRNA | 18.74 | Down | 2.72E-04 |
| NM_017549 | Homo sapiens upregulated in colorectal cancer gene 1 (UCC1), mRNA | 18.73 | Down | 8.83E-05 |
| BG436244 | 602508665F1 NIH_MGC_79 Homo sapiens cDNA clone IMAGE:4605617 5, mRNA sequence | 18.7 | Down | 1.49E-04 |
| NM_024901 | Homo sapiens hypothetical protein FLJ22457 (FLJ22457), mRNA | 18.68 | Down | 1.35E-04 |
| NM_005712 | Homo sapiens HERV-H LTR-associating 1 (HHLA1), mRNA | 18.63 | Down | 1.08E-04 |
| M_198488 | Homo sapiens FLJ46072 protein (FLJ46072), mRNA | 18.58 | Down | 7.49E-05 |
| NM_020349 | Homo sapiens ankyrin repeat domain 2 (stretch responsive muscle) (ANKRD2), mRNA | 18.49 | Down | 2.00E-04 |
| NM_002148 | Homo sapiens homeo box D10 (HOXD10), mRNA | 18.41 | Down | 3.05E-05 |
| N78460 | yz76h06.r1 Soares_multiple_sclerosis_2NbHMS P Homo sapiens cDNA clone IMAGE:289019 5, mRNA sequence | 18.4 | Down | 4.02E-04 |
| NM_006598 | Homo sapiens solute carrier family 12 (potassium/chloride transporters), member 7 (SLC12A7), mRNA | 18.13 | Down | 8.59E-05 |
| BF431041 | nab31g02.x1 Soares_NSF_F8_9W_OT_PA_P_S1 Homo sapiens cDNA clone IMAGE:3267627 3, mRNA sequence | 18.11 | Down | 6.10E-05 |
| AK126467 | Homo sapiens cDNA FLJ44503 fis, clone UTERU3001158 | 17.84 | Down | 1.31E-04 |
| NM_182487 | Homo sapiens olfactomedin-like 2A (OLFML2A), mRNA | 17.77 | Down | 1.06E-04 |
| AK058012 | Homo sapiens cDNA FLJ25283 fis, clone STM06716 | 17.76 | Down | 8.82E-05 |
| NM_001252 | Homo sapiens tumor necrosis factor (ligand) superfamily, member 7 (TNFSF7), mRNA | 17.71 | Down | 2.46E-04 |
| NM_006252 | Homo sapiens protein kinase, AMP-activated, alpha 2 catalytic subunit (PRKAA2), mRNA | 17.63 | Down | 6.57E-04 |
| NM_030899 | Homo sapiens zinc finger protein 323 (ZNF323), mRNA | 17.62 | Down | 1.77E-04 |
| NM_006722 | Homo sapiens microphthalmia-associated transcription factor (MITF), transcript variant 3, mRNA | 17.53 | Down | 1.61E-04 |
| AK056431 | Homo sapiens cDNA FLJ31869 fis, clone NT2RP7002151 | 17.29 | Down | 8.46E-05 |
| BM719937 | UI-E-EJ0-ahu-a-10-0-UI.r1 UI-E-EJ0 Homo sapiens cDNA clone UI-E-EJ0-ahu-a-10-0-UI 5, mRNA sequence | 17.11 | Down | 4.37E-05 |
| NM_005855 | Homo sapiens receptor (calcitonin) activity modifying protein 1 (RAMP1), mRNA | 16.97 | Down | 1.56E-04 |
| AK124873 | Homo sapiens cDNA FLJ42883 fis, clone BRHIP3006683 | 16.87 | Down | 3.99E-04 |
| NM_001977 | Homo sapiens glutamyl aminopeptidase (aminopeptidase A) (ENPEP), mRNA | 16.61 | Down | 6.12E-04 |
| AK074181 | Homo sapiens mRNA for FLJ00254 protein | 16.54 | Down | 6.10E-05 |
| NM_000557 | Homo sapiens growth differentiation factor 5 (cartilage-derived morphogenetic protein-1) (GDF5), mRNA | 16.47 | Down | 1.49E-04 |
| AK026966 | Homo sapiens cDNA: FLJ23313 fis, clone HEP11919 | 16.36 | Down | 8.92E-05 |
| BX113319 | BX113319 NCI_CGAP_Gas4 Homo sapiens cDNA clone IMAGp998G205398 ; IMAGE:2184619, mRNA sequence | 16.19 | Down | 6.30E-05 |
| AK055362 | Homo sapiens cDNA FLJ30800 fis, clone FEBRA2001197 | 16.02 | Down | 3.05E-05 |
| NM_021102 | Homo sapiens serine protease inhibitor, Kunitz type, 2 (SPINT2), mRNA | 15.94 | Down | 2.71E-04 |
| NM_002354 | Homo sapiens tumor-associated calcium signal transducer 1 (TACSTD1), mRNA | 15.93 | Down | 9.13E-05 |
| BQ003401 | UI-H-EI1-azd-j-23-0-UI.s1 NCI_CGAP_EI1 Homo sapiens cDNA clone IMAGE:5847286 3, mRNA sequence | 15.78 | Down | 6.30E-05 |
| NM_033641 | Homo sapiens collagen, type IV, alpha 6 (COL4A6), transcript variant B, mRNA | 15.77 | Down | 5.35E-05 |
| N38753 | yy42d01.s1 Soares melanocyte 2NbHM Homo sapiens cDNA clone IMAGE:273889 3, mRNA sequence | 15.77 | Down | 3.67E-05 |
| AI420213 | te92g09.x1 NCI_CGAP_Pr28 Homo sapiens cDNA clone IMAGE:2094208 3, mRNA sequence | 15.7 | Down | 2.35E-04 |
| AK095776 | Homo sapiens cDNA FLJ38457 fis, clone FEBRA2020400 | 15.48 | Down | 3.04E-04 |
| NM_006378 | Homo sapiens sema domain, immunoglobulin domain (Ig), transmembrane domain (TM) and short cytoplasmic domain, (semaphorin) 4D (SEMA4D), mRNA | 15.33 | Down | 6.10E-05 |
| AK021801 | Homo sapiens cDNA FLJ11739 fis, clone HEMBA1005497 | 15.33 | Down | 1.38E-04 |
| BX538226 | Homo sapiens mRNA; cDNA DKFZp686E1944 (from clone DKFZp686E1944) | 15.23 | Down | 2.37E-04 |
| NM_001847 | Homo sapiens collagen, type IV, alpha 6 (COL4A6), transcript variant A, mRNA | 15.22 | Down | 7.42E-05 |
| NM_017640 | Homo sapiens leucine rich repeat containing 16 (LRRC16), mRNA | 15.21 | Down | 6.39E-05 |
| NM_007072 | Homo sapiens HERV-H LTR-associating 2 (HHLA2), mRNA | 15.04 | Down | 1.06E-04 |
| NM_052947 | Homo sapiens heart alpha-kinase (HAK), mRNA | 14.96 | Down | 2.23E-04 |
| NM_005139 | Homo sapiens annexin A3 (ANXA3), mRNA | 14.95 | Down | 6.71E-05 |
| N63415 | yy60d04.s1 Soares_multiple_sclerosis_2NbHMS P Homo sapiens cDNA clone IMAGE:277927 3 similar to contains L1.b3 L1 repetitive element;, mRNA sequence | 14.79 | Down | 2.23E-05 |
| BC052289 | Homo sapiens carboxypeptidase A4, mRNA (cDNA clone MGC:59749 IMAGE:6106874), complete cds | 14.76 | Down | 7.22E-05 |
| NM_153715 | Homo sapiens homeo box A10 (HOXA10), transcript variant 2, mRNA | 14.76 | Down | 8.35E-05 |
| NM_014936 | Homo sapiens ectonucleotide pyrophosphatase/phosphodiesteras e 4 (putative function) (ENPP4), mRNA | 14.67 | Down | 8.19E-04 |
| NM_021192 | Homo sapiens homeo box D11 (HOXD11), mRNA | 14.47 | Down | 4.53E-04 |
| BX118238 | BX118238 NCI_CGAP_Kid3 Homo sapiens cDNA clone IMAGp998L153800; IMAGE:1501598, mRNA sequence | 14.44 | Down | 5.07E-04 |
| NM 024969 | Homo sapiens TGF-beta induced apotosis protein 2 (TAIP-2), mRNA | 14.11 | Down | 1.57E-04 |
| AK125490 | Homo sapiens cDNA FLJ43501 fis, clone PEBLM2004497 | 14.06 | Down | 9.71E-04 |
| R99527 | yq79b11.s1 Soares fetal liver spleen 1 NFLS Homo sapiens cDNA clone IMAGE:201981 3, mRNA sequence | 13.98 | Down | 8.39E-04 |
| NM_001873 | Homo sapiens carboxypeptidase E (CPE), mRNA | 13.91 | Down | 9.16E-05 |
| NM_003761 | Homo sapiens vesicle-associated membrane protein 8 (endobrevin) (VAMP8), mRNA | 13.87 | Down | 3.12E-04 |
| NM_020808 | Homo sapiens signal-induced proliferation-associated 1 like 2 (SIPA1L2), mRNA | 13.84 | Down | 5.47E-04 |
| NM_152573 | Homo sapiens RAS and EF hand domain containing (RASEF), mRNA | 13.82 | Down | 5.13E-04 |
| NM_032866 | Homo sapiens cingulin-like 1 (CGNL1), mRNA | 13.78 | Down | 6.30E-05 |
| BU740051 | UI-E-EO0-ahw-n-18-0-UI.s1 UI-E-EO0 Homo sapiens cDNA clone UI-E-EO0-ahw-n-18-0-UI 3, mRNA sequence | 13.74 | Down | 4.63E-04 |
| NM_198389 | Homo sapiens lung type-I cell membrane-associated glycoprotein (T1A-2), transcript variant 2, mRNA | 13.71 | Down | 3.62E-04 |
| CA777268 | ip05d09.y1 HR85 islet Homo sapiens cDNA clone IMAGE:6134849 5, mRNA sequence | 13.63 | Down | 1.39E-04 |
| H00617 | yj25f02.s1 Soares placenta Nb2HP Homo sapiens cDNA clone IMAGE:149787 3, mRNA sequence | 13.45 | Down | 3.33E-04 |
| NM_024898 | Homo sapiens family with sequence similarity 31, member C (FAM31C), mRNA | 13.39 | Down | 2.17E-03 |
| NM_000094 | Homo sapiens collagen, type VII, alpha 1 (epidermolysis bullosa, dystrophic, dominant and recessive) (COL7A1 mRNA | 13.3 | Down | 4.93E-04 |
| NM_002837 | Homo sapiens protein tyrosine phosphatase, receptor type, B (PTPRB), mRNA | 13.25 | Down | 6.84E-04 |
| NM_018728 | Homo sapiens myosin VC (MYO5C), mRNA | 13.15 | Down | 1.64E-04 |
| NM_018659 | Homo sapiens cytokine-like protein C17 (C17), mRNA | 13.11 | Down | 1.45E-04 |
| AL834140 | Homo sapiens mRNA; cDNA DKFZp434A2029 (from clone DKFZp434A2029) | 13.08 | Down | 9.99E-05 |
| BX103476 | BX103476 NCI_CGAP_Lu5 Homo sapiens cDNA clone IMAGp998C053946 ; IMAGE:1557436, mRNA sequence | 13.02 | Down | 1.71E-04 |
| BG545305 | 602572521 F1 NIH_MGC_77 Homo sapiens cDNA clone IMAGE:4700644 5, mRNA sequence | 12.86 | Down | 6.30E-05 |
| BX647876 | Homo sapiens mRNA; cDNA DKFZp313A1525 (from clone DKFZp313A1525) | 12.83 | Down | 7.95E-05 |
| NM_030949 | Homo sapiens protein phosphatase 1, regulatory (inhibitor) subunit 14C (PPP1R14C), mRNA | 12.82 | Down | 3.35E-04 |
| NM_024423 | Homo sapiens desmocollin 3 (DSC3), transcript variant Dsc3b, mRNA | 12.81 | Down | 7.71E-05 |
| NM_014452 | Homo sapiens tumor necrosis factor receptor superfamily, member 21 (TNFRSF21), mRNA | 12.8 | Down | 2.20E-04 |
| NM_002246 | Homo sapiens potassium channel, subfamily K, member 3 (KCNK3), mRNA | 12.76 | Down | 7.59E-05 |
| NM_002427 | Homo sapiens matrix metalloproteinase 13 (collagenase 3) (MMP13), mRNA | 12.65 | Down | 4.08E-04 |
| NM_003328 | Homo sapiens TXK tyrosine kinase (TXK), mRNA | 12.61 | Down | 2.45E-04 |
| NM_014422 | Homo sapiens phosphatidylinositol (4,5) bisphosphate 5-phosphatase, A (PIB5PA), transcript variant 1, mRNA | 12.48 | Down | 8.97E-05 |
| NM_014333 | Homo sapiens immunoglobulin superfamily, member 4 (IGSF4), mRNA | 12.4 | Down | 1.04E-04 |
| AA888443 | nw74f10.s1 NCI_CGAP_Pr12 Homo sapiens cDNA clone IMAGE:1252363, mRNA sequence | 12.37 | Down | 3.96E-04 |
| BX110418 | BX110418 NCI_CGAP_Kid3 Homo sapiens cDNA clone IMAGp998C224149 ; IMAGE:1635405, mRNA sequence | 12.35 | Down | 1.54E-04 |
| NM_019102 | Homo sapiens homeo box A5 (HOXA5), mRNA | 12.33 | Down | 7.59E-05 |
| NM_016463 | Homo sapiens CXXC finger 5 (CXXC5), mRNA | 12.32 | Down | 1.96E-04 |
| NM_004572 | Homo sapiens plakophilin 2 (PKP2), transcript variant 2b, mRNA | 12.28 | Down | 4.15E-04 |
| N25875 | yw78d12.s1 Soares_placenta_8to9weeks_2NbH P8to9W Homo sapiens cDNA clone IMAGE:258359 3, mRNA sequence | 12.27 | Down | 1.02E-04 |
| NM_152694 | Homo sapiens zinc finger, CCHC domain containing 5 (ZCCHC5), mRNA | 12.22 | Down | 3.38E-04 |
| NM_021012 | Homo sapiens potassium inwardly-rectifying channel, subfamily J, member 12 (KCNJ12), mRNA | 12.22 | Down | 9.23E-04 |
| NM_017671 | Homo sapiens chromosome 20 open reading frame 42 (C20orf42), mRNA | 12.21 | Down | 1.06E-04 |
| NM_002031 | Homo sapiens fyn-related kinase (FRK), mRNA | 12.11 | Down | 2.14E-04 |
| BM976385 | UI-CF-EN1-acz-f-03-0-UI.s1 UI-CF-EN1 Homo sapiens cDNA clone UI-CF-EN1-acz-f-03-0-UI 3, mRNA sequence | 12.02 | Down | 8.11E-04 |
| NM_005296 | Homo sapiens G protein-coupled receptor 23 (GPR23), mRNA | 11.98 | Down | 1.50E-04 |
| NM_000817 | Homo sapiens glutamate decarboxylase 1 (brain, 67kDa) (GAD1 transcript variant GAD67, mRNA | 11.92 | Down | 6.22E-05 |
| AI220066 | qg84d01.x1 Soares_NFL_T_GBC_S1 Homo sapiens cDNA clone IMAGE:1841857 3, mRNA sequence | 11.88 | Down | 1.09E-04 |
| NM_005737 | Homo sapiens ADP-ribosylation factor-like 7 (ARL7), mRNA | 11.83 | Down | 1.58E-04 |
| NM_000147 | Homo sapiens fucosidase, alpha-L-1, tissue (FUCA1), mRNA | 11.82 | Down | 1.74E-03 |
| AK127437 | Homo sapiens cDNA FLJ45529 fis, clone BRTHA2027546 | 11.82 | Down | 1.68E-04 |
| NM_178033 | Homo sapiens cytochrome P450, family 4, subfamily X, polypeptide 1 (CYP4X1), mRNA | 11.81 | Down | 2.10E-04 |
| NM_173567 | Homo sapiens abhydrolase domain containing 7 (ABHD7), mRNA | 11.76 | Down | 1.75E-04 |
| AL049974 | Homo sapiens mRNA; cDNA DKFZp564B222 (from clone DKFZp564B222) | 11.74 | Down | 5.82E-04 |
| NM_000519 | Homo sapiens hemoglobin, delta (HBD), mRNA | 11.7 | Down | 9.70E-04 |
| AL359567 | Homo sapiens mRNA; cDNA DKFZp547D023 (from clone DKFZp547D023) | 11.66 | Down | 3.95E-04 |
| BF510493 | UI-H-BI4-apa-b-08-0-UI.s1 NCI_CGAP_Sub8 Homo sapiens cDNA clone IMAGE:3086558 3, mRNA sequence | 11.66 | Down | 6.30E-05 |
| NM_000961 | Homo sapiens prostaglandin 12 (prostacyclin) synthase (PTGIS), mRNA | 11.63 | Down | 5.06E-04 |
| NM_025151 | Homo sapiens RAB11 family interacting protein 1 (class I) (RAB11FIP1 transcript variant 1, mRNA | 11.6 | Down | 1.14E-04 |
| BM712945 | UI-E-EJ0-ahi-c-16-0-UI.r1 UI-E-EJ0 Homo sapiens cDNA clone UI-E-EJ0-ahi-c-16-0-UI 5, mRNA sequence | 11.6 | Down | 9.48E-05 |
| AW451831 | UI-H-B13-alk-e-12-0-UI.s1 NCI_CGAP_Sub5 Homo sapiens cDNA clone IMAGE:2737246 3, mRNA sequence | 11.54 | Down | 8.69E-04 |
| BC040701 | Homo sapiens cDNA clone IMAGE:5736259, partial cds | 11.53 | Down | 1.23E-04 |
| AL359058 | Homo sapiens mRNA full length insert cDNA clone EUROIMAGE 592473 | 11.44 | Down | 1.27E-03 |
| NM_001854 | Homo sapiens collagen, type XI, alpha 1 (COL11A1), transcript variant A, mRNA | 11.39 | Down | 7.56E-04 |
| NM_000227 | Homo sapiens laminin, alpha 3 (LAMA3), transcript variant 2, mRNA | 11.39 | Down | 8.59E-05 |
| NM_033256 | Homo sapiens protein phosphatase 1, regulatory (inhibitor) subunit 14A (PPP1R14A), mRNA | 11.36 | Down | 1.26E-04 |
| AB011538 | Homo sapiens mRNA for MEGF5, partial cds | 11.35 | Down | 4.81 E-04 |
| AL389942 | Homo sapiens mRNA full length insert cDNA clone EUROIMAGE 2005635 | 11.33 | Down | 4.78E-04 |
| NM_174900 | Homo sapiens zinc finger protein 42 (ZFP42), mRNA | 11.23 | Down | 1.29E-04 |
| BG622707 | 602647476F1 NIH_MGC_79 Homo sapiens cDNA clone IMAGE:4768963 5, mRNA sequence | 11.21 | Down | 1.49E-04 |
| NM_178177 | Homo sapiens nicotinamide nucleotide adenylyltransferase 3 (NMNAT3), mRNA | 11.19 | Down | 3.57E-04 |
| AA099748 | zI78c09.s1 Stratagene colon (#937204) Homo sapiens cDNA clone IMAGE:510736 3, mRNA sequence | 11.16 | Down | 9.87E-05 |
| H89526 | yw28b04.r1 Morton Fetal Cochlea Homo sapiens cDNA clone IMAGE:253519 5, mRNA sequence | 11.06 | Down | 2.94E-04 |
| NM_178868 | Homo sapiens chemokine-like factor super family 8 (CKLFSF8), mRNA | 10.96 | Down | 9.23E-04 |
| BE788763 | 601475864F1 NIH_MGC_68 Homo sapiens cDNA clone IMAGE:3879014 5, mRNA sequence | 10.88 | Down | 6.22E-05 |
| NM_000076 | Homo sapiens cyclin-dependent kinase inhibitor 1C (p57, Kip2) (CDKN1C), mRNA | 10.87 | Down | 7.28E-05 |
| NM_152768 | Homo sapiens hypothetical protein FLJ25378 (FLJ25378), mRNA | 10.81 | Down | 7.31E-05 |
| M60502 | Human profilaggrin mRNA, 3 end | 10.81 | Down | 8.46E-05 |
| NM_181847 | Homo sapiens amphoterin induced gene 2 (AMIGO2), mRNA | 10.78 | Down | 1.86E-03 |
| NM_005331 | Homo sapiens hemoglobin, theta 1 (HBQ1), mRNA | 10.77 | Down | 3.32E-04 |
| NM_032367 | Homo sapiens zinc finger, BED domain containing 3 (ZBED3), mRNA | 10.61 | Down | 7.71E-05 |
| NM_004574 | Homo sapiens peanut-like 2 (Drosophila) (PNUTL2), transcript variant 1, mRNA | 10.6 | Down | 3.18E-04 |
| NM_014421 | Homo sapiens dickkopf homolog 2 (Xenopus laevis) (DKK2), mRNA | 10.52 | Down | 7.31E-05 |
| NM_052923 | Homo sapiens zinc finger protein 452 (ZNF452), mRNA | 10.43 | Down | 1.06E-04 |
| NM_006379 | Homo sapiens sema domain, immunoglobulin domain (Ig), short basic domain, secreted, (semaphorin) 3C (SEMA3C), mRNA | 10.39 | Down | 6.30E-05 |
| AL137488 | Homo sapiens mRNA; cDNA DKFZp434N2030 (from clone DKFZp434N2030) | 10.37 | Down | 2.60E-04 |
| BX088936 | BX088936 Soares_testis_NHT Homo sapiens cDNA clone IMAGp998G123255 ; IMAGE:1292195, mRNA sequence | 10.34 | Down | 1.20E-03 |
| AB041269 | Homo sapiens mRNA for keratin 19, partial cds, isolate:K19-141 | 10.33 | Down | 4.02E-03 |
| AK091933 | Homo sapiens cDNA FLJ34614 fis, clone KIDNE2014268 | 10.3 | Down | 1.49E-04 |
| AF269162 | Homo sapiens c21orf7 form B mRNA, complete cds | 10.3 | Down | 1.78E-03 |
| NM_001935 | Homo sapiens dipeptidylpeptidase 4 (CD26, adenosine deaminase complexing protein 2) (DPP4), mRNA | 10.27 | Down | 1.10E-04 |
| NM_001850 | Homo sapiens collagen, type VIII, alpha 1 (COL8A1 transcript variant 1, m RNA | 10.23 | Down | 2.23E-05 |
| AI469032 | ti70a01.x1 NCI_CGAP_Kid11 Homo sapiens cDNA clone IMAGE:2137320 3, mRNA sequence | 10.2 | Down | 7.42E-05 |
| NM_003081 | Homo sapiens synaptosomal-associated protein, 25kDa (SNAP25), transcript variant 1, mRNA | 10.05 | Down | 2.34E-03 |
| BG570144 | 602591134F1 NIH_MGC_77 Homo sapiens cDNA clone IMAGE:4717761 5, mRNA sequence | 9.99 | Down | 1.09E-03 |
| BM969191 | UI-CF-EN0-acp-e-22-0-UI.s1 UI-CF-EN0 Homo sapiens cDNA clone UI-CF-EN0-acp-e-22-0-UI 3, mRNA sequence | 9.99 | Down | 1.00E-03 |
| NM_006622 | Homo sapiens polo-like kinase 2 (Drosophila) (PLK2), mRNA | 9.99 | Down | 1.45E-04 |
| NM_000860 | Homo sapiens hydroxyprostaglandin dehydrogenase 15-(NAD) (HPGD), mRNA | 9.98 | Drown | 6.39E-05 |
| AJ318805 | AJ318805 Homo sapiens adipose tissue Homo sapiens cDNA clone 2040, mRNA sequence | 9.97 | Down | 1.92E-04 |
| NM_002735 | Homo sapiens protein kinase, cAMP-dependent, regulatory, type I, beta (PRKAR1 B), mRNA | 9.93 | Down | 8.51E-06 |
| AK092114 | Homo sapiens cDNA FLJ34795 fis, clone NT2NE2005921 | 9.91 | Down | 1.57E-03 |
| NM_024608 | Homo sapiens nei endonuclease VIII-like 1 (E. coli) (NEIL1), mRNA | 9.89 | Down | 3.05E-05 |
| BE464407 | hx89g05.x1 NCI_CGAP_Kid11 Homo sapiens cDNA clone IMAGE:3195032 3, mRNA sequence | 9.88 | Down | 1.05E-03 |
| BF431460 | 7o14b05.x1 NCI_CGAP_Kid11 Homo sapiens cDNA clone IMAGE:3573849 3, mRNA sequence | 9.85 | Down | 1.19E-03 |
| BF509573 | UI-H-BI4-apf-b-11-0-UI.s1 NCI_CGAP_Sub8 Homo sapiens cDNA clone IMAGE:3086949 3, mRNA sequence | 9.82 | Down | 2.57E-04 |
| T78754 | yd01f08.r1 Soares infant brain 1NIB Homo sapiens cDNA clone IMAGE:24180 5, mRNA sequence | 9.79 | Down | 1.51E-03 |
| M_153256 | Homo sapiens chromosome 10 open reading frame 47 (C10orf47), mRNA | 9.78 | Down | 2.10E-04 |
| NM_207482 | Homo sapiens FLJ44048 protein (FLJ44048), mRNA | 9.76 | Down | 2.14E-04 |
| NM_002276 | Homo sapiens keratin 19 (KRT19), mRNA | 9.74 | Down | 3.70E-03 |
| N70752 | za72d02.s1 Soares_fetal_lung_NbHL19W Homo sapiens cDNA clone IMAGE:298083 3, mRNA sequence | 9.73 | Down | 1.49E-04 |
| NM_020962 | Homo sapiens likely ortholog of mouse neighbor of Punc E11 (NOPE), mRNA | 9.73 | Down | 8.91E-04 |
| NM_016276 | Homo sapiens serum/glucocorticoid regulated kinase 2 (SGK2), transcript variant 2, mRNA | 9.7 | Down | 4.41E-04 |
| AI355761 | qt94a11.x1 NCI_CGAP_Co14 Homo sapiens cDNA clone IMAGE:1962908 3 similar to gb:X74929 KERATIN, TYPE II CYTOSKELETAL 8 (HUMAN);, mRNA sequence | 9.7 | Down | 9.99E-05 |
| NM_003264 | Homo sapiens toll-like receptor 2 (TLR2), mRNA | 9.69 | Down | 1.86E-04 |
| NM_198495 | Homo sapiens CTAGE family, member 4 (CTAGE4), mRNA | 9.66 | Down | 3.12E-04 |
| BX097888 | BX097888 Soares_parathyroid_tumor_NbHPA Homo sapiens cDNA clone IMAGp998K064187 ; IMAGE:1650173, mRNA sequence | 9.62 | Down | 6.10E-05 |
| BU567804 | AGENCOURT_10398872 NIH_MGC_82 Homo sapiens cDNA clone IMAGE:6614502 5, mRNA sequence | 9.62 | Down | 7.71E-05 |
| NM_152423 | Homo sapiens melanoma associated antigen (mutated) 1-like 1 (MUM1L1), mRNA | 9.61 | Down | 2.45E-03 |
| NM_018330 | Homo sapiens KIAA1598 (KIAA1598), mRNA | 9.6 | Down | 1.68E-04 |
| BU584197 | 2513030T6 LIVRTUT04 Homo sapiens cDNA clone 2513030 3, mRNA sequence | 9.56 | Down | 8.83E-04 |
| NM_005264 | Homo sapiens GDNF family receptor alpha 1 (GFRA1), transcript variant 1, m RNA | 9.56 | Down | 1.19E-04 |
| NM_002245 | Homo sapiens potassium channel, subfamily K, member 1 (KCNK1), mRNA | 9.53 | Down | 2.05E-03 |
| M_178550 | Homo sapiens hypothetical protein MGC48998 (MGC48998), mRNA | 9.51 | Down | 9.03E-04 |
| NM_006863 | Homo sapiens leukocyte immunoglobulin-like receptor, subfamily A (with TM domain), member 1 (LILRA1), mRNA | 9.48 | Down | 6.22E-05 |
| M_173354 | Homo sapiens SNF1-like kinase (SNF1LK), mRNA | 9.47 | Down | 8.25E-04 |
| BC016962 | Homo sapiens, clone IMAGE:4182947, mRNA | 9.43 | Down | 2.15E-04 |
| AB046810 | Homo sapiens mRNA for KIAA1590 protein, partial cds | 9.38 | Down | 6.30E-05 |
| BX116347 | BX116347 NCI_CGAP_Kid12 Homo sapiens cDNA clone IMAGp998B215967 ; IMAGE:2401844, mRNA sequence | 9.33 | Down | 1.54E-04 |
| BU633163 | UI-H-FL1-bgt-n-07-0-UI.s1 NCI_CGAP_FL1 Homo sapiens cDNA clone UI-H-FL1-bgt-n-07-0-UI 3, mRNA sequence | 9.31 | Down | 1.46E-04 |
| AI289329 | qw28c09.x1 NCI_CGAP_Ut4 Homo sapiens cDNA clone IMAGE:1992400 3 similar to contains L1.b2 L1 repetitive element ;, mRNA sequence | 9.28 | Down | 4.80E-04 |
| BU951469 | in60a05.x3 HR85 islet Homo sapiens cDNA clone IMAGE:6126249 3, mRNA sequence | 9.21 | Down | 2.54E-03 |
| NM_002338 | Homo sapiens limbic system-associated membrane protein (LSAMP), mRNA | 9.19 | Down | 1.30E-03 |
| AL833346 | Homo sapiens mRNA; cDNA DKFZp686M2234 (from clone DKFZp686M2234) | 9.17 | Down | 3.02E-04 |
| BC040293 | Homo sapiens, clone IMAGE:4820330, mRNA | 9.1 | Down | 1.09E-03 |
| NM_003810 | Homo sapiens tumor necrosis factor (ligand) superfamily, member 10 (TNFSF10), mRNA | 9.04 | Down | 5.06E-04 |
| NM_014373 | Homo sapiens G protein-coupled receptor 160 (GPR160), mRNA | 9.01 | Down | 1.59E-04 |
| CA425405 | UI-H-FE1-bef-g-08-0-UI.s1 NCI_CGAP_FE1 Homo sapiens cDNA clone UI-H-FE1-bef-g-08-0-UI 3, mRNA sequence | 8.89 | Down | 1.74E-05 |
| AI953708 | wq47d09.x1 NCI_CGAP_GC6 Homo sapiens cDNA clone IMAGE:2474417 3, mRNA sequence | 8.87 | Down | 9.89E-03 |
| NM_017641 | Homo sapiens kinesin family member 21A (KIF21A), mRNA | 8.86 | Down | 7.22E-05 |
| NM_153377 | Homo sapiens leucine-rich repeats and immunoglobulin-like domains 3 (LRIG3), mRNA | 8.86 | Down | 2.71E-04 |
| NM_000236 | Homo sapiens lipase, hepatic (LIPC), mRNA | 8.83 | Down | 1.93E-03 |
| BC046362 | Homo sapiens voltage-dependent calcium channel gamma subunit-like protein, mRNA (cDNA clone MGC:50757 IMAGE:5221396), complete cds | 8.8 | Down | 4.71E-04 |
| AJ697972 | Homo sapiens chromosome 3 cDNA | 8.8 | Down | 7.35E-04 |
| BF509074 | UI-H-BI4-aou-b-08-0-UI.s1 NCI_CGAP_Sub8 Homo sapiens cDNA clone IMAGE:3086150 3, mRNA sequence | 8.8 | Down | 1.24E-04 |
| NM_001080 | Homo sapiens aldehyde dehydrogenase 5 family, member A1 (succinate-semialdehyde dehydrogenase) (ALDH5A1), nuclear gene encoding mitochondrial protein, transcript variant 2, mRNA | 8.79 | Down | 8.46E-05 |
| NM_002247 | Homo sapiens potassium large conductance calcium-activated channel, subfamily M, alpha member 1 (KCNMA1 mRNA | 8.78 | Down | 7.45E-05 |
| NM_022154 | Homo sapiens solute carrier family 39 (zinc transporter), member 8 (SLC39A8), mRNA | 8.77 | Down | 4.65E-05 |
| NM_144707 | Homo sapiens prominin 2 (PROM2), mRNA | 8.73 | Down | 2.01E-04 |
| AB095949 | Homo sapiens mRNA for KIAA2029 protein | 8.69 | Down | 9.23E-04 |
| AV728294 | AV728294 HTC Homo sapiens cDNA clone HTCBIE09 5, mRNA sequence | 8.68 | Down | 8.46E-05 |
| NM_003851 | Homo sapiens cellular repressor of E1A-stimulated genes 1 (CREG1), mRNA | 8.68 | Down | 2.10E-04 |
| NM_014181 | Homo sapiens HSPC159 protein (HSPC159), mRNA | 8.67 | Down | 1.10E-04 |
| NM_000349 | Homo sapiens steroidogenic acute regulator (STAR), nuclear gene encoding mitochondrial protein, transcript variant 1, mRNA | 8.66 | Down | 1.03E-04 |
| AK026740 | Homo sapiens cDNA: FLJ23087 fis, clone LNG06994, highly similar to AF161368 Homo sapiens HSPC105 mRNA | 8.65 | Down | 7.45E-04 |
| T56535 | yb33g07.r1 Stratagene fetal spleen (#937205) Homo sapiens cDNA clone IMAGE:73020 5, mRNA sequence | 8.63 | Down | 1.23E-04 |
| NM_178868 | Homo sapiens chemokine-like factor super family 8 (CKLFSF8), mRNA | 8.63 | Down | 6.22E-05 |
| NM_005130 | Homo sapiens fibroblast growth factor binding protein 1 (FGFBP1), mRNA | 8.62 | Down | 1.15E-04 |
| NM_032488 | Homo sapiens cornifelin (CNFN), mRNA | 8.6 | Down | 3.05E-05 |
| NM_052960 | Homo sapiens retinol binding protein 7, cellular (RBP7), mRNA | 8.6 | Down | 2.05E-04 |
| NM_207517 | Homo sapiens ADAMTS-like 3 (ADAMTSL3), mRNA | 8.57 | Down | 2.22E-04 |
| AK127644 | Homo sapiens cDNA FLJ45742 fis, clone KIDNE2016327 | 8.54 | Down | 3.24E-03 |
| BU727096 | UI-E-CR0-ach-e-12-0-UI.s1 UI-E-CR0 Homo sapiens cDNA clone UI-E-CR0-ach-e-12-0-UI 3, mRNA sequence | 8.53 | Down | 9.29E-04 |
| NM_015973 | Homo sapiens galanin (GAL), mRNA | 8.53 | Down | 6.30E-05 |
| NM_033514 | Homo sapiens LIM and senescent cell antigen-like domains 3 (LIMS3), mRNA | 8.51 | Down | 3.30E-04 |
| NM_138811 | Homo sapiens chromosome 7 open reading frame 31 (C7orf31 mRNA | 8.47 | Down | 8.35E-05 |
| NM_005302 | Homo sapiens G protein-coupled receptor 37 (endothelin receptor type B-like) (GPR37), mRNA | 8.47 | Down | 2.19E-03 |
| NM_173462 | Homo sapiens papilin, proteoglycan-like sulfated glycoprotein (PAPLN), mRNA | 8.46 | Down | 1.70E-04 |
| NM_032148 | Homo sapiens solute carrier family 41, member 2 (SLC41A2), mRNA | 8.44 | Down | 9.03E-05 |
| NM_000856 | Homo sapiens guanylate cyclase 1, soluble, alpha 3 (GUCY1A3), mRNA | 8.37 | Down | 1.17E-03 |
| AW591723 | xt85h10.x1 NCI_CGAP_Ut1 Homo sapiens cDNA clone IMAGE:2793283 3 similar to contains element MER32 repetitive element ;, mRNA sequence | 8.36 | Down | 4.86E-04 |
| NM_032024 | Homo sapiens chromosome 10 open reading frame 11 (C10orf11 mRNA | 8.25 | Down | 1.80E-04 |
| AK024850 | Homo sapiens cDNA: FLJ21197 fis, clone COL00201 | 8.21 | Down | 7.71E-05 |
| NM_002206 | Homo sapiens integrin, alpha 7 (ITGA7), mRNA | 8.2 | Down | 6.30E-05 |
| A1963999 | wt87g07.x1 NCI_CGAP_GC6 Homo sapiens cDNA clone IMAGE:2514492 3, mRNA sequence | 8.19 | Down | 1.61E-03 |
| NM_005567 | Homo sapiens lectin, galactoside-binding, soluble, 3 binding protein (LGALS3BP), mRNA | 8.18 | Down | 8.76E-05 |
| D87454 | Human mRNA for KIAA0265 gene, partial cds | 8.14 | Down | 1.08E-03 |
| BG564960 | 602583930F1 NIH_MGC_76 Homo sapiens cDNA clone IMAGE:4711807 5, mRNA sequence | 8.12 | Down | 7.71E-05 |
| NM_022168 | Homo sapiens interferon induced with helicase C domain 1 (IFIH1), mRNA | 8.12 | Down | 1.46E-04 |
| AK027541 | Homo sapiens cDNA FLJ14635 fis, clone NT2RP2001196 | 8.12 | Down | 2.08E-04 |
| NM_024997 | Homo sapiens activating transcription factor 7 interacting protein 2 (ATF7IP2), mRNA | 8.11 | Down | 1.65E-03 |
| AI926616 | wo48e04.x1 NCI_CGAP_Gas4 Homo sapiens cDNA clone IMAGE:2458590 3 similar to contains MER27.b2 MER27 repetitive element;, mRNA sequence | 8.08 | Down | 6.30E-05 |
| S70348 | Homo sapiens integrin beta 3 mRNA, partial cds, alternatively spliced | 8.06 | Drown | 2.21E-04 |
| NM_005725 | Homo sapiens tetraspan 2 (TSPAN-2), mRNA | 8.05 | Drown | 3.97E-03 |
| CA311343 | UI-CF-FNO-aff-b-19-0-UI.s1 UI-CF-FN0 Homo sapiens cDNA clone UI-CF-FN0-aff-b-19-0-UI 3, mRNA sequence | 8.02 | Down | 8.51E-06 |
| NM_001710 | Homo sapiens B-factor, properdin (BF), mRNA | 8.01 | Down | 1.30E-03 |
| NM_002247 | Homo sapiens potassium large conductance calcium-activated channel, subfamily M, alpha member 1 (KCNMA1 mRNA | 8 | Down | 1.29E-03 |
| BG149255 | nad25d01.x1 NCI_CGAP_Lu24 Homo sapiens cDNA clone IMAGE:3366553 3, mRNA sequence | 8 | Down | 3.85E-04 |
| NM_153634 | Homo sapiens copine VIII (CPNE8), mRNA | 7.97 | Down | 3.57E-04 |
| NM_004390 | Homo sapiens cathepsin H (CTSH), transcript variant 1, mRNA | 7.97 | Down | 7.71 E-05 |
| NM_016613 | Homo sapiens hypothetical protein DKFZp434L142 (DKFZp434L142), mRNA | 7.97 | Down | 3.72E-04 |
| NM_001845 | Homo sapiens collagen, type IV, alpha 1 (COL4A1 mRNA | 7.93 | Down | 8.65E-05 |
| NM_005502 | Homo sapiens ATP-binding cassette, sub-family A (ABC1), member 1 (ABCA1 mRNA | 7.93 | Down | 5.18E-04 |
| NM_018650 | Homo sapiens MAP/microtubule affinity-regulating kinase 1 (MARK1), mRNA | 7.92 | Down | 1.23E-04 |
| D62831 | HUM330B12B Clontech human aorta polyA+ mRNA (#6572) Homo sapiens cDNA clone GEN-330B12 5, mRNA sequence | 7.92 | Down | 9.23E-04 |
| CA313095 | UI-CF-FN0-aex-f-01-0-UI.s1 UI-CF-FN0 Homo sapiens cDNA clone UI-CF-FN0-aex-f-01-0-UI 3, mRNA sequence | 7.91 | Down | 7.42E-04 |
| BC038556 | Homo sapiens, clone IMAGE:3446976, mRNA | 7.91 | Down | 2.57E-03 |
| AI493349 | tg70f04.x1 Soares_NhHMPu_S1 Homo sapiens cDNA clone IMAGE:2114143 3, mRNA sequence | 7.9 | Down | 1.14E-04 |
| BC015108 | Homo sapiens, Similar to otoconin 90, clone IMAGE:4044247, mRNA | 7.9 | Down | 1.01E-04 |
| AI819863 | wj45h05.x1 NCI_CGAP_Lu19 Homo sapiens cDNA clone IMAGE:2405817 3, mRNA sequence | 7.87 | Down | 2.39E-03 |
| BM802920 | AGENCOURT_6457446 NIH_MGC_88 Homo sapiens cDNA clone IMAGE:5560288 5, mRNA sequence | 7.85 | Down | 1.60E-03 |
| AK057113 | Homo sapiens cDNA FLJ32551 fis, clone SPLEN1000087 | 7.83 | Down | 1.20E-03 |
| NM_005025 | Homo sapiens serine (or cysteine) proteinase inhibitor, clade I (neuroserpin), member 1 (SERPINI1), mRNA | 7.82 | Down | 2.21E-04 |
| AA195328 | zr34f08.s1 Soares_NhHMPu_S1 Homo sapiens cDNA clone IMAGE:665319 3, mRNA sequence | 7.81 | Down | 2.37E-04 |
| NM_014358 | Homo sapiens C-type (calcium dependent, carbohydrate-recognition domain) lectin, superfamily member 9 (CLECSF9), mRNA | 7.81 | Down | 5.03E-03 |
| BI493986 | df106g12.y1 Morton Fetal Cochlea Homo sapiens cDNA clone IMAGE:2538815 5, mRNA sequence | 7.8 | Down | 6.69E-04 |
| NM_152366 | Homo sapiens kelch/ankyrin repeat containing cyclin A1 interacting protein (KARCA1), transcript variant 1, mRNA | 7.79 | Down | 8.97E-05 |
| NM_015678 | Homo sapiens neurobeachin (NBEA), mRNA | 7.78 | Down | 2.12E-04 |
| NM_018424 | Homo sapiens erythrocyte membrane protein band 4.1 like 4B (EPB41L4B), mRNA | 7.77 | Down | 1.23E-04 |
| CA310979 | UI-CF-FN0-afc-c-21-0-UI.s1 UI-CF-FN0 Homo sapiens cDNA clone UI-CF-FN0-afc-c-21-0-UI 3, mRNA sequence | 7.76 | Down | 1.86E-04 |
| NM_031476 | Homo sapiens hypothetical protein DKFZp434B044 (DKFZP434B044), mRNA | 7.75 | Down | 2.20E-03 |
| NM_004086 | Homo sapiens coagulation factor C homolog, cochlin (Limulus polyphemus) (COCH), mRNA | 7.74 | Down | 2.21E-04 |
| NM_003617 | Homo sapiens regulator of G-protein signalling 5 (RGS5), mRNA | 7.73 | Down | 3.85E-04 |
| BF966833 | 602286668T1 NIH_MGC_95 Homo sapiens cDNA clone IMAGE:4375360 3, mRNA sequence | 7.73 | Down | 6.10E-05 |
| AK024270 | Homo sapiens cDNA FLJ14208 fis, clone NT2RP3003264 | 7.71 | Down | 1.13E-03 |
| NM_144587 | Homo sapiens chromosome 10 open reading frame 87 (C10orf87), mRNA | 7.71 | Down | 6.03E-04 |
| AK096288 | Homo sapiens cDNA FLJ38969 fis, clone NT2RI2002359 | 7.7 | Down | 1.49E-04 |
| BE968596 | 601649770F1 NIH_MGC_74 Homo sapiens cDNA clone IMAGE:3933472 5, mRNA sequence | 7.69 | Down | 2.39E-03 |
| AY358775 | Homo sapiens clone DNA170212 WGAR9166 (UNQ9166) mRNA, complete cds | 7.66 | Down | 2.16E-03 |
| BX103949 | BX103949 NCI_CGAP_Co3 Homo sapiens cDNA clone IMAGp998C112296 ; IMAGE:923842, mRNA sequence | 7.64 | Down | 3.18E-04 |
| NM_013410 | Homo sapiens adenylate kinase 3 (AK3), nuclear gene encoding mitochondrial protein, transcript variant 2, mRNA | 7.64 | Down | 3.07E-04 |
| NM_005204 | Homo sapiens mitogen-activated protein kinase kinase kinase 8 (MAP3K8), mRNA | 7.63 | Down | 6.82E-04 |
| NM_032211 | Homo sapiens lysyl oxidase-like 4 (LOXL4), mRNA | 7.6 | Down | 3.30E-04 |
| AA325746 | EST28794 Cerebellum II Homo sapiens cDNA 5 end, mRNA sequence | 7.57 | Down | 6.30E-05 |
| H94320 | yv18b10.s1 Soares fetal liver spleen 1NFLS Homo sapiens cDNA clone IMAGE:243067 3, mRNA sequence | 7.55 | Down | 3.35E-03 |
| BX640973 | Homo sapiens mRNA; cDNA DKFZp686B15184 (from clone DKFZp686B15184) | 7.55 | Down | 7.89E-04 |
| BQ015616 | UI-1-BC1-ajb-g-04-0-UI.s1 NCI_CGAP_PI2 Homo sapiens cDNA clone UI-1-BC1-ajb-g-04-0-UI 3, mRNA sequence | 7.54 | Down | 5.28E-04 |
| AK128715 | Homo sapiens cDNA FLJ46882 fis, clone UTERU3015844 | 7.53 | Down | 2.30E-04 |
| NM_015068 | Homo sapiens paternally expressed 10 (PEG10), mRNA | 7.52 | Down | 7.71E-05 |
| BU754480 | UI-1-BB1p-axz-h-11-0-UI.s1 NCI_CGAP_PI6 Homo sapiens cDNA clone UI-1-BB1p-axz-h-11-0-UI 3, mRNA sequence | 7.49 | Down | 6.24E-04 |
| AK074097 | Homo sapiens mRNA for FLJ00168 protein | 7.48 | Down | 1.93E-03 |
| BC033567 | Homo sapiens, clone IMAGE:4822266, mRNA | 7.48 | Down | 1.96E-04 |
| NM_018894 | Homo sapiens EGF-containing fibulin-like extracellular matrix protein 1 (EFEMP1), transcript variant 2, mRNA | 7.47 | Down | 2.67E-04 |
| BM701989 | UI-E-CQ1-aex-j-06-0-UI.r1 UI-E-CQ1 Homo sapiens cDNA clone UI-E-CQ1-aex-j-06-0-UI 5, mRNA sequence | 7.47 | Down | 4.53E-04 |
| NM_016423 | Homo sapiens zinc finger protein 219 (ZNF219), mRNA | 7.46 | Down | 1.61E-04 |
| BX117317 | BX117317 NCI_CGAP_Co3 Homo sapiens cDNA clone IMAGp998E242234 ; IMAGE:900095, mRNA sequence | 7.45 | Down | 1.13E-03 |
| NM_001993 | Homo sapiens coagulation factor III (thromboplastin, tissue factor) (F3), mRNA | 7.45 | Down | 5.82E-04 |
| CA502991 | UI-CF-FN0-afp-g-01-0-UI.s1 UI-CF-FN0 Homo sapiens cDNA clone UI-CF-FN0-afp-g-01-0-UI 3, mRNA sequence | 7.43 | Down | 2.24E-02 |
| AI686890 | tp90h02.x1 NCI_CGAP_Ut3 Homo sapiens cDNA clone IMAGE:2206611 3, mRNA sequence | 7.41 | Down | 1.49E-04 |
| AW137001 | UI-H-BI1-acu-c-05-0-UI.s1 NCI_CGAP_Sub3 Homo sapiens cDNA clone IMAGE:2715632 3, mRNA sequence | 7.4 | Down | 7.95E-05 |
| BQ021695 | UI-H-DH1-axi-f-22-0-UI.s1 NCI_CGAP_DH1 Homo sapiens cDNA clone IMAGE:5829141 3, mRNA sequence | 7.39 | Down | 6.10E-05 |
| BX640643 | Homo sapiens mRNA; cDNA DKFZp686O24114 (from clone DKFZp686O24114) | 7.36 | Down | 5.18E-05 |
| NM_004669 | Homo sapiens chloride intracellular channel 3 (CLIC3), mRNA | 7.35 | Down | 4.30E-03 |
| NM_001955 | Homo sapiens endothelin 1 (EDN1), mRNA | 7.33 | Down | 7.72E-04 |
| AB007974 | Homo sapiens mRNA, chromosome 1 specific transcript KIAA0505 | 7.33 | Down | 5.29E-03 |
| NM_198174 | Homo sapiens transcription factor CP2-like 4 (TFCP2L4), transcript variant 3, mRNA | 7.32 | Drown | 4.82E-03 |
| BX098521 | BX098521 Soares fetal liver spleen 1NFLS Homo sapiens cDNA clone IMAGp998L05118 ; IMAGE:123412, mRNA sequence | 7.3 | Down | 5.51E-03 |
| NM_004154 | Homo sapiens pyrimidinergic receptor P2Y, G-protein coupled, 6 (P2RY6), transcript variant 4, mRNA | 7.28 | Down | 3.41E-03 |
| AK123617 | Homo sapiens cDNA FLJ41623 fis, clone CTONG3009227 | 7.27 | Down | 2.26E-03 |
| BF111903 | 7I38d07.x0 Soares_NSF_F8_9W_OT_PA_P_S Homo sapiens cDNA clone IMAGE:3523644 3, mRNA sequence | 7.25 | Down | 1.34E-03 |
| NM_018655 | Homo sapiens lens epithelial protein (LENEP), mRNA | 7.24 | Down | 1.07E-04 |
| NM_139161 | Homo sapiens crumbs homolog 3 (Drosophila) (CRB3), transcript variant 2, mRNA | 7.23 | Down | 8.46E-04 |
| BC060805 | Homo sapiens hypothetical protein FLJ12788, mRNA (cDNA clone IMAGE:5266931), partial cds | 7.22 | Down | 7.45E-05 |
| CA413744 | UI-H-EZ0-bat-h-12-0-UI.s1 NCI_CGAP_Ch1 Homo sapiens cDNA clone UI-H-EZ0-bat-h-12-0-UI 3, mRNA sequence | 7.22 | Down | 6.09E-04 |
| NM_018986 | Homo sapiens SH3 domain and tetratricopeptide repeats 1 (SH3TC1), mRNA | 7.21 | Down | 2.68E-03 |
| NM_024677 | Homo sapiens hypothetical protein FLJ14001 (FLJ14001), mRNA | 7.21 | Down | 1.14E-04 |
| AL117454 | Homo sapiens mRNA; cDNA DKFZp586J1717 (from clone DKFZp586J1717) | 7.2 | Down | 2.93E-04 |
| AW450938 | UI-H-BI3-all-g-05-0-UI.s1 NCI_CGAP_Sub5 Homo sapiens cDNA clone IMAGE:2737329 3, mRNA sequence | 7.19 | Down | 1.19E-04 |
| NM_017899 | Homo sapiens hypothetical protein FLJ20607 (TSC), mRNA | 7.16 | Down | 4.80E-03 |
| S81734 | tissue transglutaminase homologue {alternatively spliced} [human, erythroleukemia cell line HEL GM06141A, mRNA, 2362 nt] | 7.16 | Down | 8.76E-05 |
| BU616749 | UI-H-FH1-bfj-a-11-0-UI.s1 NCI_CGAP_FH1 Homo sapiens cDNA clone UI-H-FH1-bfj-a-11-0-UI 3, mRNA sequence | 7.14 | Down | 2.21E-04 |
| NM_006681 | Homo sapiens neuromedin U (NMU), mRNA | 7.1 | Down | 6.52E-03 |
| AK022598 | Homo sapiens cDNA FLJ12536 fis, clone NT2RM4000265 | 7.09 | Down | 1.15E-04 |
| AW 137116 | UI-H-BI1-acp-f-03-0-UI.s1 NCI_CGAP_Sub3 Homo sapiens cDNA clone IMAGE:2715029 3, mRNA sequence | 7.07 | Down | 3.50E-03 |
| BX647876 | Homo sapiens mRNA; cDNA DKFZp313A1525 (from clone DKFZp313A1525) | 7.06 | Down | 1.41E-03 |
| NM_000597 | Homo sapiens insulin-like growth factor binding protein 2, 36kDa (IGFBP2), mRNA | 7.04 | Down | 2.99E-03 |
| CN478597 | UI-CF-FN0-aeo-g-21-0-UI.s1 UI-CF-FN0 Homo sapiens cDNA clone UI-CF-FN0-aeo-g-21-0-UI 3, mRNA sequence | 7.04 | Down | 1.43E-03 |
| CN478714 | UI-CF-FN0-afu-c-19-0-UI.s1 UI-CF-FN0 Homo sapiens cDNA clone UI-CF-FN0-afu-c-19-0-UI 3, mRNA sequence | 7.04 | Down | 3.55E-03 |
| AB020640 | Homo sapiens mRNA for KIAA0833 protein, partial cds | 7 | Down | 2.45E-03 |
| NM_022746 | Homo sapiens hypothetical protein FLJ22390 (FLJ22390), mRNA | 6.99 | Down | 9.12E-04 |
| AI905628 | CM-BT094-050299-147 BT094 Homo sapiens cDNA, mRNA sequence | 6.98 | Down | 1.54E-04 |
| NM_002193 | Homo sapiens inhibin, beta B (activin AB beta polypeptide) (INHBB), mRNA | 6.96 | Down | 7.59E-04 |
| NM_004490 | Homo sapiens growth factor receptor-bound protein 14 (GRB14), mRNA | 6.94 | Down | 1.98E-03 |
| NM_003985 | Homo sapiens tyrosine kinase, non-receptor, 1 (TNK1), mRNA | 6.93 | Down | 2.10E-04 |
| NM_000480 | Homo sapiens adenosine monophosphate deaminase (isoform E) (AMPD3), mRNA | 6.92 | Down | 1.06E-04 |
| AK094292 | Homo sapiens cDNA FLJ36973 fis, clone BRACE2006249 | 6.92 | Down | 2.03E-03 |
| BC033124 | Homo sapiens, clone IMAGE:2960615, mRNA | 6.88 | Down | 7.22E-05 |
| NM_000612 | Homo sapiens insulin-like growth factor 2 (somatomedin A) (IGF2), mRNA | 6.88 | Down | 2.60E-04 |
| BC042976 | Homo sapiens cDNA clone IMAGE:5295023, partial cds | 6.88 | Down | 2.39E-03 |
| BF445031 | nad20f02.x1 NCI_CGAP_Lu24 Homo sapiens cDNA clone IMAGE:3366266 3, mRNA sequence | 6.87 | Down | 5.18E-04 |
| NM_004170 | Homo sapiens solute carrier family 1 (neuronal/epithelial high affinity glutamate transporter, system Xag), member 1 (SLC1A1), mRNA | 6.87 | Down | 3.18E-04 |
| NM_199169 | Homo sapiens transmembrane, prostate androgen induced RNA (TMEPAI), transcript variant 2, mRNA | 6.84 | Down | 1.45E-03 |
| NM_019644 | Homo sapiens ankyrin repeat domain 7 (ANKRD7), mRNA | 6.83 | Down | 8.99E-05 |
| W20132 | zb40c10.r1 Soares_parathyroid_tumor_NbHPA Homo sapiens cDNA clone IMAGE:306066 5, mRNA sequence | 6.82 | Drown | 6.79E-03 |
| BC033116 | Homo sapiens chromodomain helicase DNA binding protein 7, mRNA (cDNA clone IMAGE:3352674), partial cds | 6.82 | Down | 8.03E-05 |
| NM_018650 | Homo sapiens MAP/microtubule affinity-regulating kinase 1 (MARK1), mRNA | 6.8 | Down | 4.97E-03 |
| BU563992 | AGENCOURT_10371176 NIH_MGC_141 Homo sapiens cDNA clone IMAGE:6601829 5, mRNA sequence | 6.79 | Down | 1.29E-02 |
| AL119769 | DKFZp761E1224_r1 761 (synonym: hamy2) Homo sapiens cDNA clone DKFZp761 E1224 5, mRNA sequence | 6.78 | Down | 2.52E-03 |
| NM_052997 | Homo sapiens ankyrin repeat domain 30A (ANKRD30A), mRNA | 6.78 | Down | 2.45E-03 |
| NM_024704 | Homo sapiens chromosome 20 open reading frame 23 (C20orf23), mRNA | 6.76 | Down | 7.45E-04 |
| NM_000495 | Homo sapiens collagen, type IV, alpha 5 (Alport syndrome) (COL4A5), transcript variant 1, mRNA | 6.74 | Down | 4.77E-03 |
| BX102895 | BX102895 Soares fetal liver spleen 1NFLS Homo sapiens cDNA clone IMAGp998I18520 ; IMAGE:242009, mRNA sequence | 6.7 | Down | 3.04E-04 |
| BU753362 | UI-1-BB1-air-h-09-0-UI.s1 NCI_CGAP_PI5 Homo sapiens cDNA clone UI-1-BB1-air-h-09-0-UI 3, mRNA sequence | 6.7 | Down | 7.42E-05 |
| AA190552 | zp86b11.s1 Stratagene HeLa cell s3 937216 Homo sapiens cDNA clone IMAGE:627069 3, mRNA sequence | 6.67 | Down | 1.26E-03 |
| NM_004024 | Homo sapiens activating transcription factor 3 (ATF3), mRNA | 6.66 | Down | 3.12E-03 |
| NM_144650 | Homo sapiens alcohol dehydrogenase, iron containing, 1 (ADHFE1), mRNA | 6.65 | Down | 1.45E-04 |
| NM_002800 | Homo sapiens proteasome (prosome, macropain) subunit, beta type, 9 (large multifunctional protease 2) (PSMB9), transcript variant 1, mRNA | 6.63 | Down | 1.70E-04 |
| NM_021977 | Homo sapiens solute carrier family 22 (extraneuronal monoamine transporter), member 3 (SLC22A3), mRNA | 6.62 | Down | 2.05E-03 |
| AW516579 | xq01f06.x1 Soares_NHCeC_cervical_tumor Homo sapiens cDNA clone IMAGE:2748611 3, mRNA sequence | 6.62 | Down | 4.65E-03 |
| AI311296 | ta48d10.x2 NCI_CGAP_Lu25 Homo sapiens cDNA clone IMAGE:2047315 3, mRNA sequence | 6.61 | Down | 1.72E-03 |
| BC040204 | Homo sapiens, clone IMAGE:4821815, mRNA | 6.61 | Down | 5.66E-04 |
| AA340011 | EST45155 Fetal skin Homo sapiens cDNA 5 end, mRNA sequence | 6.58 | Down | 6.30E-05 |
| NM_025044 | Homo sapiens bicaudal C homolog 1 (Drosophila) (BICC1), mRNA | 6.57 | Down | 6.30E-05 |
| BX111520 | BX111520 Soares placenta Nb2HP Homo sapiens cDNA clone IMAGp998L15208 ; IMAGE:141470, mRNA sequence | 6.57 | Down | 1.70E-03 |
| AW444925 | UI-H-BI3-ajz-f-09-0-UI.s1 NCI_CGAP_Sub5 Homo sapiens cDNA clone IMAGE:2733473 3, mRNA sequence | 6.52 | Down | 2.55E-03 |
| NM_003475 | Homo sapiens chromosome 11 open reading frame 13 (C11orf13), mRNA | 6.5 | Down | 5.88E-04 |
| AK124778 | Homo sapiens cDNA FLJ42788 fis, clone BRAWH3007129 | 6.48 | Down | 8.61E-04 |
| NM_024563 | Homo sapiens hypothetical protein FLJ14054 (FLJ14054), mRNA | 6.44 | Down | 4.72E-03 |
| NM_002214 | Homo sapiens integrin, beta 8 (ITGB8), mRNA | 6.43 | Down | 8.46E-05 |
| NM_178814 | Homo sapiens adaptor-related protein complex 1, sigma 3 subunit (AP1S3), mRNA | 6.42 | Down | 1.78E-04 |
| AI831068 | wj62d12.x1 NCI_CGAP_Lu19 Homo sapiens cDNA clone IMAGE:2407415 3, mRNA sequence | 6.41 | Down | 3.72E-04 |
| NM_000186 | Homo sapiens complement factor H (CFH), mRNA | 6.39 | Down | 1.74E-03 |
| NM_000216 | Homo sapiens Kallmann syndrome 1 sequence (KAL1), mRNA | 6.37 | Down | 8.59E-05 |
| NM_015478 | Homo sapiens I(3)mbt-like (Drosophila) (L3MBTL), transcript variant I, mRNA | 6.36 | Down | 7.22E-05 |
| BX096609 | BX096609 Soares retina N2b4HR Homo sapiens cDNA clone IMAGp998L12439 ; IMAGE:221339, mRNA sequence | 6.36 | Down | 1.24E-03 |
| NM_030970 | Homo sapiens hypothetical protein MGC3771 (MGC3771), mRNA | 6.34 | Down | 1.69E-04 |
| BQ007085 | UI-H-EI1-azc-k-11-0-UI.s1 NCI_CGAP_EI1 Homo sapiens cDNA clone IMAGE:5846914 3, mRNA sequence | 6.33 | Down | 1.19E-04 |
| BC039329 | Homo sapiens, clone IMAGE:5267606, mRNA | 6.33 | Down | 6.61 E-03 |
| NM_001165 | Homo sapiens baculoviral IAP repeat-containing 3 (BIRC3), transcript variant 1, mRNA | 6.33 | Down | 3.42E-03 |
| NM_004165 | Homo sapiens Ras-related associated with diabetes (RRAD), mRNA | 6.33 | Down | 4.68E-03 |
| NM_001928 | Homo sapiens D component of complement (adipsin) (DF), mRNA | 6.32 | Down | 3.68E-03 |
| NM_018670 | Homo sapiens mesoderm posterior 1 (MESP1), mRNA | 6.31 | Down | 5.76E-03 |
| NM_153229 | Homo sapiens hypothetical protein FLJ33318 (FLJ33318), mRNA | 6.31 | Down | 2.57E-03 |
| AA912845 | ol32a12.s1 Soares_NFL_T_GBC_S1 Homo sapiens cDNA clone | 6.29 | Down | 1.13E-03 |
| | IMAGE:1525150 3, mRNA sequence | | | |
| AK127437 | Homo sapiens cDNA FLJ45529 fis, clone BRTHA2027546 | 6.28 | Down | 3.37E-04 |
| NM_020836 | Homo sapiens brain-enriched guanylate kinase-associated protein (KIAA1446), mRNA | 6.27 | Down | 6.60E-03 |
| AL512697 | Homo sapiens mRNA; cDNA DKFZp547F134 (from clone DKFZp547F134) | 6.25 | Down | 1.36E-03 |
| NM_005211 | Homo sapiens colony stimulating factor 1 receptor, formerly McDonough feline sarcoma viral (v-fms) oncogene homolog (CSF1 R), mRNA | 6.24 | Down | 5.07E-04 |
| AK124776 | Homo sapiens cDNA FLJ42786 fis, clone BRAWH3006761 | 6.2 | Down | 1.15E-04 |
| NM_173662 | Homo sapiens hypothetical protein LOC285533 (LOC285533), mRNA | 6.18 | Down | 1.05E-02 |
| NM_014391 | Homo sapiens ankyrin repeat domain 1 (cardiac muscle) (ANKRD1), mRNA | 6.18 | Down | 3.52E-03 |
| NM_005860 | Homo sapiens follistatin-like 3 (secreted glycoprotein) (FSTL3), mRNA | 6.18 | Down | 5.23E-05 |
| NM_001045 | Homo sapiens solute carrier family 6 (neurotransmitter transporter, serotonin), member 4 (SLC6A4), mRNA | 6.17 | Down | 5.46E-03 |
| NM_002147 | Homo sapiens homeo box B5 (HOXB5), mRNA | 6.17 | Down | 1.05E-04 |
| AI288404 | qv89b01.x1 NCI_CGAP_Ut2 Homo sapiens cDNA clone IMAGE:1988713 3, mRNA sequence | 6.16 | Down | 3.74E-04 |
| NM_173584 | Homo sapiens hypothetical protein MGC45840 (MGC45840), mRNA | 6.16 | Down | 1.32E-04 |
| N62729 | yz76g05.s1 Soares_multiple_sclerosis_2NbHMS P Homo sapiens cDNA clone IMAGE:289016 3, mRNA sequence | 6.12 | Down | 1.37E-03 |
| AK024238 | Homo sapiens cDNA FLJ14176 fis, clone NT2RP2003101 | 6.11 | Down | 9.69E-05 |
| NM_001843 | Homo sapiens contactin 1 (CNTN1), transcript variant 1, mRNA | 6.1 | Down | 4.80E-03 |
| M_152433 | Homo sapiens kelch repeat and BTB (POZ) domain containing 3 (KBTBD3), transcript variant 1, mRNA | 6.1 | Down | 2.39E-03 |
| BX649112 | Homo sapiens mRNA; cDNA DKFZp686E02109 (from clone DKFZp686E02109) | 6.1 | Down | 2.24E-04 |
| AA411988 | zt65g11.s1 Soares_testis_NHT Homo sapiens cDNA clone IMAGE:727268 3, mRNA sequence | 6.09 | Down | 1.40E-03 |
| R80806 | yi94f01.s1 Soares placenta Nb2HP Homo sapiens cDNA clone IMAGE:146905 3, mRNA sequence | 6.08 | Down | 1.54E-04 |
| NM_002843 | Homo sapiens protein tyrosine phosphatase, receptor type, J (PTPRJ), mRNA | 6.08 | Down | 6.54E-03 |
| NM_003979 | Homo sapiens G protein-coupled receptor, family C, group 5, member A (GPCR5A), mRNA | 6.06 | Down | 8.75E-03 |
| AW 129281 | xf23a03.x1 NCI_CGAP_Kid8 Homo sapiens cDNA clone IMAGE:2618860 3 similar to gb:X58295_rna1 PLASMA GLUTATHIONE PEROXIDASE PRECURSOR (HUMAN);, mRNA sequence | 6.06 | Down | 1.70E-04 |
| NM_015345 | Homo sapiens dishevelled associated activator of morphogenesis 2 (DAAM2), mRNA | 6.05 | Down | 1.09E-03 |
| L07615 | Human neuropeptide Y receptor Y1 (NPYY1) mRNA, exon 2-3 and complete cds | 6.05 | Down | 5.51E-03 |
| NM_016179 | Homo sapiens transient receptor potential cation channel, subfamily C, member 4 (TRPC4), mRNA | 6.04 | Down | 8.59E-05 |
| NM_182797 | Homo sapiens phospholipase C, beta 4 (PLCB4), transcript variant 2, mRNA | 6.03 | Down | 4.40E-04 |
| AB032945 | Homo sapiens mRNA for KIAA1119 protein, partial cds | 6.03 | Down | 1.11E-02 |
| CA444471 | UI-H-DP0-avv-a-16-0-UI.s1 NCI_CGAP_Fs1 Homo sapiens cDNA clone UI-H-DP0-avv-a-16-0-UI 3, mRNA sequence | 6.02 | Down | 1.02E-03 |
| AK127421 | Homo sapiens cDNA FLJ45513 fis, clone BRTHA2021450 | 6.01 | Down | 1.60E-04 |
| AV736303 | AV736303 CB Homo sapiens cDNA clone CBCAJD04 5, mRNA sequence | 6.01 | Down | 8.97E-05 |
| BX537613 | Homo sapiens mRNA; cDNA DKFZp686E11117 (from clone DKFZp686E11117) | 6 | Down | 4.89E-04 |
| AB023211 | Homo sapiens mRNA for KIAA0994 protein, partial cds | 5.99 | Down | 5.50E-04 |
| NM_018349 | Homo sapiens multiple C2-domains with two transmembrane regions 2 (MCTP2), mRNA | 5.99 | Down | 2.48E-04 |
| NM_000087 | Homo sapiens cyclic nucleotide gated channel alpha 1 (CNGA1), mRNA | 5.98 | Down | 3.29E-03 |
| BF939416 | nad89c02.x1 NCI_CGAP_Pr28 Homo sapiens cDNA clone IMAGE:3410667 3, mRNA sequence | 5.97 | Down | 9.03E-04 |
| NM_013951 | Homo sapiens paired box gene 8 (PAX8), transcript variant PAX8B, mRNA | 5.97 | Down | 2.57E-03 |
| AW 195474 | xn38g09.x1 NCI_CGAP_Kid11 Homo sapiens cDNA clone IMAGE:2696032 3, mRNA sequence | 5.97 | Down | 1.68E-04 |
| R40050 | yf68h07.s1 Soares infant brain 1NIB Homo sapiens cDNA clone IMAGE:27726 3, mRNA sequence | 5.94 | Down | 5.78E-03 |
| NM_021101 | Homo sapiens claudin 1 (CLDN1), mRNA | 5.9 | Down | 2.60E-04 |
| AA602964 | no97c02.s1 NCI_CGAP_Pr2 Homo sapiens cDNA clone IMAGE:1114754, mRNA sequence | 5.9 | Down | 1.23E-03 |
| NM_006255 | Homo sapiens protein kinase C, eta (PRKCH), mRNA | 5.89 | Down | 2.78E-04 |
| NM_024103 | Homo sapiens solute carrier family 25 (mitochondrial carrier; phosphate carrier), member 23 (SLC25A23), mRNA | 5.89 | Down | 7.42E-05 |
| AK093870 | Homo sapiens cDNA FLJ36551 fis, clone TRACH2008127 | 5.88 | Down | 2.28E-02 |
| BE070450 | QV4-BT0407-020300-122-d08 BT0407 Homo sapiens cDNA, mRNA sequence | 5.85 | Down | 3.53E-04 |
| NM_003706 | Homo sapiens phospholipase A2, group IVC (cytosolic, calcium-independent) (PLA2G4C), mRNA | 5.85 | Down | 7.81E-04 |
| NM_023927 | Homo sapiens HCV NS3-transactivated protein 2 (NS3TP2), mRNA | 5.85 | Down | 2.64E-03 |
| AF519622 | Homo sapiens noncoding mRNA sequence | 5.84 | Down | 7.70E-03 |
| NM_198582 | Homo sapiens FLJ43374 protein (FLJ43374), mRNA | 5.84 | Down | 4.05E-03 |
| AK026283 | Homo sapiens cDNA: FLJ22630 fis, clone HSI06250 | 5.84 | Down | 5.32E-04 |
| CB115754 | K-EST0159876 L8SCK0 Homo sapiens cDNA clone L8SCK0-8-H08 5, mRNA sequence | 5.81 | Down | 9.71E-04 |
| BQ188285 | UI-E-EJ1-ajp-n-20-0-UI.r1 UI-E-EJ1 Homo sapiens cDNA clone UI-E-EJ1-ajp-n-20-0-UI 5, mRNA sequence | 5.8 | Down | 5.47E-03 |
| NM_003662 | Homo sapiens pirin (iron-binding nuclear protein) (PIR), mRNA | 5.78 | Down | 1.71E-04 |
| BX098660 | BX098660 Soares placenta Nb2HP Homo sapiens cDNA clone IMAGp998L03214 ; IMAGE:143762, mRNA sequence | 5.77 | Down | 6.00E-04 |
| NM_005360 | Homo sapiens v-maf musculoaponeurotic fibrosarcoma oncogene homolog (avian) (MAF), mRNA | 5.77 | Down | 2.24E-04 |
| NM_031847 | Homo sapiens microtubule-associated protein 2 (MAP2), transcript variant 4, mRNA | 5.77 | Down | 1.34E-03 |
| NM_003057 | Homo sapiens solute carrier family 22 (organic cation transporter), member 1 (SLC22A1), transcript variant 1, mRNA | 5.75 | Down | 2.42E-04 |
| AW242323 | xm96f03.x1 NCI_CGAP_Kid11 Homo sapiens cDNA clone IMAGE:2692061 3 similar to contains Alu repetitive element;, mRNA sequence | 5.74 | Down | 1.92E-04 |
| NM_052890 | Homo sapiens peptidoglycan recognition protein 2 (PGLYRP2), mRNA | 5.73 | Down | 1.14E-04 |
| NM_000593 | Homo sapiens transporter 1, ATP-binding cassette, sub-family B (MDR/TAP) (TAP1), mRNA | 5.72 | Down | 4.86E-03 |
| NM_033132 | Homo sapiens Zic family member 5 (odd-paired homolog, Drosophila) (ZIC5), mRNA | 5.71 | Down | 1.76E-04 |
| AL832380 | Homo sapiens mRNA; cDNA DKFZp451L157 (from clone DKFZp451L157) | 5.71 | Down | 1.54E-04 |
| NM_183376 | Homo sapiens arrestin domain containing 4 (ARRDC4), mRNA | 5.71 | Down | 5.88E-04 |
| NM_018700 | Homo sapiens tripartite motif-containing 36 (TRIM36), mRNA | 5.7 | Down | 1.32E-02 |
| NM_000782 | Homo sapiens cytochrome P450, family 24, subfamily A, polypeptide 1 (CYP24A1), nuclear gene encoding mitochondrial protein, mRNA | 5.69 | Down | 1.82E-02 |
| BC035805 | Homo sapiens caspase recruitment domain family, member 9, mRNA (cDNA clone IMAGE:5745585), partial cds | 5.68 | Down | 4.00E-03 |
| BC051727 | Homo sapiens cDNA clone IMAGE:5265929, partial cds | 5.68 | Down | 5.13E-04 |
| NM_022842 | Homo sapiens CUB domain-containing protein 1 (CDCP1), transcript variant 1, mRNA | 5.68 | Down | 1.85E-03 |
| NM_004335 | Homo sapiens bone marrow stromal cell antigen 2 (BST2), mRNA | 5.67 | Down | 6.01E-03 |
| NM_053039 | Homo sapiens UDP glycosyltransferase 2 family, polypeptide B28 (UGT2B28), mRNA | 5.66 | Down | 8.07E-03 |
| NM_004529 | Homo sapiens myeloid/lymphoid or mixed-lineage leukemia (trithorax homolog, Drosophila); translocated to, 3 (MLLT3), mRNA | 5.66 | Down | 4.02E-04 |
| AK093069 | Homo sapiens cDNA FLJ35750 fis, clone TEST12004539, weakly similar to Homo sapiens adlican mRNA | 5.65 | Down | 1.10E-03 |
| NM_005141 | Homo sapiens fibrinogen, B beta polypeptide (FGB), mRNA | 5.63 | Down | 7.89E-04 |
| NM_000682 | Homo sapiens adrenergic, alpha-2B-, receptor (ADRA2B), mRNA | 5.61 | Down | 1.14E-04 |
| BF512326 | UI-H-BW1-amb-g-12-0-UI.s1 NCI_CGAP_Sub7 Homo sapiens cDNA clone IMAGE:3069503 3, mRNA sequence | 5.6 | Down | 1.49E-04 |
| NM_003726 | Homo sapiens src family associated phosphoprotein 1 (SCAP1), mRNA | 5.6 | Down | 2.42E-03 |
| AK024261 | Homo sapiens cDNA FLJ14199 fis, clone NT2RP3002713 | 5.6 | Down | 4.42E-04 |
| AK125695 | Homo sapiens cDNA FLJ43707 fis, clone TESOP2001865 | 5.59 | Down | 1.10E-02 |
| NM_006074 | Homo sapiens tripartite motif-containing 22 (TRIM22), mRNA | 5.58 | Down | 1.94E-03 |
| AW591461 | x192h06.x1 NCI_CGAP_Ut1 Homo sapiens cDNA clone IMAGE:2682203 3, mRNA sequence | 5.58 | Down | 1.95E-02 |
| NM_022128 | Homo sapiens ribokinase (RBKS), mRNA | 5.58 | Down | 1.87E-04 |
| N66105 | yy65e06.s1 Soares_multiple_sclerosis_2NbHMS P Homo sapiens cDNA clone IMAGE:278434 3, mRNA sequence | 5.57 | Down | 2.84E-04 |
| BC035116 | Homo sapiens cDNA clone IMAGE:5263177, partial cds | 5.56 | Down | 5.49E-04 |
| BM988642 | UI-H-DH0-arx-p-21-0-UI.s1 NCI_CGAP_DH0 Homo sapiens cDNA clone IMAGE:5855492 3, mRNA sequence | 5.56 | Down | 2.48E-03 |
| NM_003947 | Homo sapiens huntingtin-associated protein interacting protein (duo) (HAPIP), mRNA | 5.56 | Down | 9.71E-04 |
| AI697906 | we18f06.x1 NCI_CGAP_Lu24 Homo sapiens cDNA clone IMAGE:2341475 3, mRNA sequence | 5.56 | Down | 3.52E-03 |
| AK095399 | Homo sapiens cDNA FLJ38080 fis, clone CTONG2016185 | 5.55 | Down | 7.43E-03 |
| AL110252 | Homo sapiens mRNA; cDNA DKFZp566A1046 (from clone DKFZp566A1046) | 5.54 | Down | 3.44E-03 |
| NM_024572 | Homo sapiens UDP-N-acetyl-alpha-D-galactosamine:polypeptide N-acetylgalactosaminyltransferase 14 (GalNAc-T14) (GALNT14), mRNA | 5.54 | Down | 5.93E-03 |
| NM_000775 | Homo sapiens cytochrome P450, family 2, subfamily J, polypeptide 2 (CYP2J2), mRNA | 5.53 | Down | 1.57E-03 |
| NM_018317 | Homo sapiens hypothetical protein FLJ11082 (FLJ11082), mRNA | 5.51 | Down | 5.10E-04 |
| NM_030915 | Homo sapiens likely ortholog of mouse limb-bud and heart gene (LBH), mRNA | 5.51 | Down | 6.86E-04 |
| NM_139241 | Homo sapiens FYVE, RhoGEF and PH domain containing 4 (FGD4), mRNA | 5.5 | Down | 9.42E-03 |
| CB047092 | NISC_gf08f03.x1 NCI_CGAP_Kid12 Homo sapiens cDNA clone IMAGE:3253013 3, mRNA sequence | 5.5 | Down | 5.38E-04 |
| NM_005711 | Homo sapiens EGF-like repeats and discoidin I-like domains 3 (EDIL3), mRNA | 5.5 | Down | 6.04E-04 |
| AA974968 | on02e08.s1 NCI_CGAP_Kid3 Homo sapiens cDNA clone IMAGE:1555526 3, mRNA sequence | 5.49 | Down | 1.63E-03 |
| NM_000129 | Homo sapiens coagulation factor XIII, A1 polypeptide (F13A1), mRNA | 5.49 | Down | 1.09E-02 |
| NM_007366 | Homo sapiens phospholipase A2 receptor 1, 180kDa (PLA2R1), transcript variant 1, mRNA | 5.47 | Down | 4.66E-03 |
| AL353944 | Homo sapiens mRNA; cDNA DKFZp761J1112 (from clone DKFZp761J1112) | 5.46 | Down | 5.38E-04 |
| AK095647 | Homo sapiens cDNA FLJ38328 fis, clone FCBBF3025142 | 5.46 | Down | 1.61E-03 |
| BQ188860 | UI-E-EJ1-ajx-h-03-0-UI.r1 UI-E-EJ1 Homo sapiens cDNA clone UI-E-EJ1-ajx-h-03-0-UI 5, mRNA sequence | 5.46 | Down | 3.33E-03 |
| NM_017594 | Homo sapiens DIRAS family, GTP-binding RAS-like 2 (DIRAS2), mRNA | 5.44 | Down | 1.22E-02 |
| NM_014399 | Homo sapiens transmembrane 4 superfamily member 13 (TM4SF13), mRNA | 5.43 | Down | 2.64E-04 |
| NM_033255 | Homo sapiens epithelial stromal interaction 1 (breast) (EPSTI1), mRNA | 5.43 | Down | 1.73E-03 |
| AW269270 | xs35c11.x1 NCI_CGAP_Kid11 Homo sapiens cDNA clone IMAGE:2771636 3 similar to contains L1.t3 L1 repetitive element ;, mRNA sequence | 5.42 | Down | 1.33E-03 |
| AK092456 | Homo sapiens cDNA FLJ35137 fis, clone PLACE6009419 | 5.42 | Down | 1.09E-04 |
| NM_031935 | Homo sapiens hemicentin (FIBL-6), mRNA | 5.42 | Down | 7.69E-04 |
| BU751966 | UI-1-BB0-acy-c-09-0-UI.s1 NCI_CGAP_PI4 Homo sapiens cDNA clone UI-1-BB0-acy-c-09-0-UI 3, mRNA sequence | 5.4 | Down | 1.61E-03 |
| NM_001747 | Homo sapiens capping protein (actin filament), gelsolin-like (CAPG), mRNA | 5.4 | Down | 1.33E-03 |
| NM_002260 | Homo sapiens killer cell lectin-like receptor subfamily C, member 2 (KLRC2), mRNA | 5.4 | Down | 1.21E-02 |
| AA732841 | zg77f01.s1 Soares_fetal_heart_NbHH19W Homo sapiens cDNA clone IMAGE:399385 3, mRNA sequence | 5.4 | Down | 3.48E-03 |
| AK055468 | Homo sapiens cDNA FLJ30906 fis, clone FEBRA2006055 | 5.4 | Down | 1.36E-03 |
| A1080164 | oz48c05.x1 Soares_NhHMPu_S1 Homo sapiens cDNA clone IMAGE:1678568 3, mRNA sequence | 5.38 | Down | 2.44E-03 |
| NM_005562 | Homo sapiens laminin, gamma 2 (LAMC2), transcript variant 1, mRNA | 5.38 | Down | 3.78E-04 |
| NM_080659 | Homo sapiens similar to RIKEN cDNA 2310030G06 gene (MGC14839), mRNA | 5.36 | Down | 3.81E-03 |
| NM_015085 | Homo sapiens GTPase activating Rap/RanGAP domain-like 4 (GARNL4), mRNA | 5.36 | Down | 1.86E-04 |
| BX097034 | BX097034 Soares infant brain 1 NIB Homo sapiens cDNA clone IMAGp998F14169 ; IMAGE:39685, mRNA sequence | 5.36 | Down | 8.59E-05 |
| NM_002147 | Homo sapiens homeo box B5 (HOXB5), mRNA | 5.35 | Down | 8.21E-05 |
| NM_181785 | Homo sapiens hypothetical protein LOC283537 (LOC283537), mRNA | 5.33 | Down | 3.48E-04 |
| NM_032857 | Homo sapiens lactamase, beta (LACTB), nuclear gene encoding mitochondrial protein, transcript variant 1, mRNA | 5.33 | Down | 2.45E-04 |
| BX113144 | BX113144 Soares placenta Nb2HP Homo sapiens cDNA clone IMAGp998N07225 ; IMAGE:148038, mRNA sequence | 5.32 | Down | 1.53E-03 |
| NM_014905 | Homo sapiens glutaminase (GLS), mRNA | 5.31 | Down | 1.63E-03 |
| NM_138396 | Homo sapiens membrane-associated RING-CH protein IX (MARCH-IX), mRNA | 5.31 | Down | 1.39E-04 |
| NM_004433 | Homo sapiens E74-like factor 3 (ets domain transcription factor, epithelial-specific ) (ELF3), mRNA | 5.3 | Down | 5.32E-05 |
| AA553553 | nk78d11.s1 NCI_CGAP_Sch1 Homo sapiens cDNA clone IMAGE:1019637 3, mRNA sequence | 5.27 | Down | 1.69E-03 |
| AW 188195 | xj93e12.x1 Soares_NFL_T_GBC_S1 Homo sapiens cDNA clone IMAGE:2664814 3 similar to contains element THR repetitive element;, mRNA sequence | 5.27 | Down | 5.32E-05 |
| NM_006169 | Homo sapiens nicotinamide N-methyltransferase (NNMT), mRNA | 5.26 | Down | 7.45E-05 |
| NM_000071 | Homo sapiens cystathionine-beta-synthase (CBS), mRNA | 5.26 | Down | 1.49E-03 |
| NM_001958 | Homo sapiens eukaryotic translation elongation factor 1 alpha 2 (EEF1A2), mRNA | 5.25 | Down | 5.20E-03 |
| AK055356 | Homo sapiens cDNA FLJ30794 fis, clone FEBRA2001093, weakly similar to MONOCARBOXYLATE TRANSPORTER 4 | 5.25 | Down | 8.65E-04 |
| NM_014578 | Homo sapiens ras homolog gene family, member D (RHOD), mRNA | 5.24 | Down | 5.43E-03 |
| NM_013230 | Homo sapiens CD24 antigen (small cell lung carcinoma cluster 4 antigen) (CD24), mRNA | 5.22 | Down | 7.65E-04 |
| AF086158 | Homo sapiens full length insert cDNA clone ZB72E12 | 5.2 | Down | 6.04E-03 |
| NM_004932 | Homo sapiens cadherin 6, type 2, K-cadherin (fetal kidney) (CDH6), mRNA | 5.18 | Down | 2.11E-04 |
| NM_020466 | Homo sapiens hypothetical protein dJ12208.2 (DJ12208.2), mRNA | 5.18 | Down | 2.99E-03 |
| AL117425 | Homo sapiens mRNA; cDNA DKFZp566L203 (from clone DKFZp566L203) | 5.17 | Down | 8.64E-03 |
| AI732568 | zo23d12.x5 Stratagene colon (#937204) Homo sapiens cDNA clone IMAGE:587735 3, mRNA sequence | 5.17 | Down | 9.19E-04 |
| Al150192 | qf34d12.x1 Soares_testis_NHT Homo sapiens cDNA clone IMAGE:1751927 3, mRNA sequence | 5.17 | Down | 1.21E-02 |
| AK125852 | Homo sapiens cDNA FLJ43864 fis, clone TEST14007799 | 5.17 | Down | 4.82E-03 |
| NM_017912 | Homo sapiens hect domain and RLD 6 (HERC6), mRNA | 5.15 | Down | 3.63E-04 |
| NM_006174 | Homo sapiens neuropeptide Y receptor Y5 (NPY5R), mRNA | 5.14 | Down | 1.13E-02 |
| AK025281 | Homo sapiens cDNA: FLJ21628 fis, clone COL08076 | 5.14 | Down | 9.71E-04 |
| NM_153742 | Homo sapiens cystathionase (cystathionine gamma-lyase) (CTH), transcript variant 2, mRNA | 5.12 | Down | 1.27E-04 |
| NM_003107 | Homo sapiens SRY (sex determining region Y)-box 4 (SOX4), mRNA | 5.11 | Down | 3.48E-04 |
| NM_003948 | Homo sapiens cyclin-dependent kinase-like 2 (CDC2-related kinase) (CDKL2), mRNA | 5.11 | Down | 2.05E-03 |
| NM_173660 | Homo sapiens hypothetical protein FLJ33718 (FLJ33718), mRNA | 5.11 | Down | 1.30E-03 |
| AI693580 | wd12d01.x1 NCI_CGAP_Co3 Homo sapiens cDNA clone IMAGE:2327905 3, mRNA sequence | 5.1 | Down | 6.19E-03 |
| NM_004482 | Homo sapiens UDP-N-acetyl-alpha-D-galactosamine:polypeptide N-acetylgalactosaminyltransferase 3 (GalNAc-T3) (GALNT3), mRNA | 5.1 | Down | 2.31E-03 |
| NM_030965 | Homo sapiens sialyltransferase 7 ((alpha-N-acetylneuraminyl-2,3-beta-galactosyl-1,3)-N-acetyl galactosaminide alpha-2,6-sialyltransferase) E (SIAT7E), mRNA | 5.1 | Down | 2.61E-04 |
| NM_016147 | Homo sapiens protein phosphatase methylesterase-1 (PME-1), mRNA | 5.09 | Down | 9.71E-04 |
| NM_006259 | Homo sapiens protein kinase, cGMP-dependent, type II (PRKG2), mRNA | 5.08 | Down | 1.36E-03 |
| NM_002993 | Homo sapiens chemokine (C-X-C motif) ligand 6 (granulocyte chemotactic protein 2) (CXCL6), mRNA | 5.07 | Down | 2.32E-03 |
| BE671338 | 7e49f03.x1 NCI_CGAP_Lu24 Homo sapiens cDNA clone IMAGE:3285821 3, mRNA sequence | 5.07 | Down | 1.28E-02 |
| BX647256 | Homo sapiens mRNA; cDNA DKFZp686K0753 (from clone DKFZp686K0753) | 5.07 | Down | 2.05E-02 |
| BG186566 | RST5534 Athersys RAGE Library Homo sapiens cDNA, mRNA sequence | 5.05 | Down | 8.91E-04 |
| AV652758 | AV652758 GLC Homo sapiens cDNA clone GLCDDG05 3, mRNA sequence | 5.04 | Down | 1.05E-03 |
| AI672441 | wa03c03.x1 NCI_CGAP_Kid11 Homo sapiens cDNA clone IMAGE:2296996 3, mRNA sequence | 5.04 | Down | 1.11E-02 |
| AA769642 | ob20h04.s1 NCI_CGAP_Kid5 Homo sapiens cDNA clone IMAGE:1324279 3, mRNA sequence | 5.02 | Down | 1.59E-02 |
| AL706653 | DKFZp686E1543_r1 686 (synonym: hlcc3) Homo sapiens cDNA clone DKFZp686E1543 5, mRNA sequence | 5.02 | Down | 1.09E-03 |
| AW296834 | UI-H-B12-ahz-a-10-0-UI.s1 NCI_CGAP_Sub4 Homo sapiens cDNA clone IMAGE:2728243 3, mRNA sequence | 5.01 | Down | 5.93E-03 |

**TABLE VII B: GENES THAT WERE DIFFERENTIALLY EXPRESSED AT LEAST 5 FOLD IN EPITHELIAL VERSUS FIBROBLAST CELLS**

| Gene Identifier | Gene Name | Average fold change in Epithelial versus fibroblast cells | Direction | adj. p-value |
|---|---|---|---|---|
| NM_018658 | Homo sapiens potassium inwardly-rectifying channel, subfamily J, member 16 (KCNJ16), transcript variant 1, mRNA | 481.28 | UP | 2.64E-05 |
| NM_014358 | Homo sapiens C-type (calcium dependent, carbohydrate-recognition domain) lectin, superfamily member 9 (CLECSF9), mRNA | 375.12 | UP | 6.56E-05 |
| AB032980 | Homo sapiens mRNA for KIAA1154 protein, partial cds | 331.92 | UP | 1.60E-05 |
| BG219729 | RST39494 Athersys RAGE Library Homo sapiens cDNA, mRNA sequence | 316.78 | UP | 8.39E-06 |
| NM_002899 | Homo sapiens retinol binding protein 1, cellular (RBP1), mRNA | 314.6 | UP | 5.37E-05 |
| NM_007038 | Homo sapiens a disintegrin-like and metalloprotease (reprolysin type) with thrombospondin type 1 motif, 5 (aggrecanase-2) (ADAMTS5), mRNA | 280.99 | UP | 1.26E-05 |
| NM_002423 | Homo sapiens matrix metalloproteinase 7 (matrilysin, uterine) (MMP7), mRNA | 269.82 | UP | 8.65E-06 |
| NM_001977 | Homo sapiens glutamyl aminopeptidase (aminopeptidase A) (ENPEP), mRNA | 242.27 | UP | 5.43E-05 |
| D29453 | HUMNK566 Human epidermal keratinocyte Homo sapiens cDNA clone 566, mRNA sequence | 208.36 | UP | 1.72E-05 |
| NM_024508 | Homo sapiens zinc finger, BED domain containing 2 (ZBED2), mRNA | 168.99 | UP | 8.39E-06 |
| A1335277 | tb29h06.x1 NCI_CGAP _Kid12 Homo sapiens cDNA clone IMAGE:2055803 3, mRNA sequence | 165.99 | UP | 8.46E-06 |
| BE877764 | 601486331 F1 NIH_MGC_69 Homo sapiens cDNA clone IMAGE:3888943 5, mRNA sequence | 145.52 | UP | 2.36E-05 |
| AI765021 | wh56c02.x1 NCI_CGAP _Kid11 Homo sapiens cDNA clone IMAGE:2384738 3, mRNA sequence | 145.09 | UP | 1.15E-05 |
| NM_024726 | Homo sapiens IQ motif containing with AAA domain (IQCA), mRNA | 143.09 | UP | 8.39E-06 |
| NM_004221 | Homo sapiens natural killer cell transcript 4 (NK4), mRNA | 142.15 | UP | 1.88E-05 |
| NM_019000 | Homo sapiens hypothetical protein FLJ20152 (FLJ20152), mRNA | 131.23 | UP | 1.88E-05 |
| NM_000104 | Homo sapiens cytochrome P450, family 1, subfamily B, polypeptide 1 (CYP1B1), mRNA | 127.7 | UP | 1.51E-05 |
| BM993116 | UI-H-DT0-aty-f-17-0-UI.s1 NCI_CGAP_DT0 Homo sapiens cDNA clone IMAGE:5866000 3, mRNA sequence | 127.35 | UP | 4.13E-05 |
| NM_031426 | Homo sapiens chromosome 9 open reading frame 58 (C9orf58), transcript variant 1, mRNA | 116.09 | UP | 3.82E-05 |
| NM_152369 | Homo sapiens hypothetical protein MGC45474 (MGC45474), mRNA | 112.28 | UP | 2.18E-05 |
| NM_003810 | Homo sapiens tumor necrosis factor (ligand) superfamily, member 10 (TNFSF10), mRNA | 112.19 | UP | 2.96E-05 |
| NM_023942 | Homo sapiens hypothetical protein MGC3036 (MGC3036), mRNA | 111.27 | UP | 1.15E-05 |
| NM_005560 | Homo sapiens laminin, alpha 5 (LAMA5), mRNA | 110.51 | UP | 1.37E-05 |
| NM_001453 | Homo sapiens forkhead box C1 (FOXC1), mRNA | 107.21 | UP | 1.15E-05 |
| BU734212 | UI-E-CQ1-agd-e-21-0-UI.s1 UI-E-CQ1 Homo sapiens cDNA clone UI-E-CQ1-agd-e-21-0-UI 3, mRNA sequence | 103.23 | UP | 2.89E-05 |
| AK026966 | Homo sapiens cDNA: FLJ23313 fis, clone HEP11919 | 103.17 | UP | 1.78E-05 |
| CA425961 | UI-H-FE1-beg-p-18-0-UI.s1 NCI_CGAP_FE1 Homo sapiens cDNA clone UI-H-FE1-beg-p-18-0-UI 3, mRNA sequence | 102.53 | UP | 6.21E-05 |
| AK075003 | Homo sapiens cDNA FLJ90522 fis, clone NT2RP4000108, highly similar to Human gene for neurofilament subunit NF-L | 101.8 | UP | 2.31E-04 |
| NM_018265 | Homo sapiens hypothetical protein FLJ10901 (FLJ10901), mRNA | 99.41 | UP | 3.66E-05 |
| AK058012 | Homo sapiens cDNA FLJ25283 fis, clone STM06716 | 98.88 | UP | 9.08E-06 |
| NM_004098 | Homo sapiens empty spiracles homolog 2 (Drosophila) (EMX2), mRNA | 97.96 | UP | 4.13E-05 |
| AK096481 | Homo sapiens cDNA FLJ39162 fis, clone OCBBF2002376 | 94.93 | UP | 2.83E-05 |
| NM_003238 | Homo sapiens transforming growth factor, beta 2 (TGFB2), mRNA | 91.63 | UP | 2.31E-05 |
| AI244954 | qj93h05.x1 NCI_CGAP_Kid3 Homo sapiens cDNA clone IMAGE:1867065 3, mRNA sequence | 85.24 | UP | 1.78E-05 |
| NM_173505 | Homo sapiens ankyrin repeat domain 29 (ANKRD29), mRNA | 82.89 | UP | 3.53E-05 |
| AK130281 | Homo sapiens cDNA FLJ26771 fis, clone PRS03189 | 82.02 | UP | 3.66E-06 |
| NM_153026 | Homo sapiens prickle-like 1 (Drosophila) (PRICKLE1), mRNA | 81.73 | UP | 1.78E-05 |
| AK092401 | Homo sapiens cDNA FLJ35082 fis, clone PLACE6005351 | 79.69 | UP | 1.70E-05 |
| AB011539 | Homo sapiens mRNA for MEGF6 protein (KIAA0815), partial cds | 79.31 | UP | 8.41E-05 |
| NM_016356 | Homo sapiens doublecortin domain containing 2 (DCDC2), mRNA | 78.04 | UP | 6.98E-05 |
| AK023631 | Homo sapiens cDNA FLJ13569 fis, clone PLACE1008369 | 77.68 | UP | 2.09E-05 |
| NM_024422 | Homo sapiens desmocollin 2 (DSC2), transcript variant Dsc2a, mRNA | 73.14 | UP | 7.38E-05 |
| BX648964 | Homo sapiens mRNA; cDNA DKFZp686J0156 (from clone DKFZp686J0156) | 70.68 | UP | 6.92E-05 |
| T56535 | yb33g07.r1 Stratagene fetal spleen (#937205) Homo sapiens cDNA clone IMAGE:73020 5, mRNA sequence | 69.74 | UP | 2.03E-05 |
| BG197054 | RST16291 Athersys RAGE Library Homo sapiens cDNA, mRNA sequence | 69.48 | UP | 4.96E-05 |
| NM_005949 | Homo sapiens metallothionein 1 F (functional) (MT1 F), mRNA | 68.1 | UP | 2.03E-05 |
| AK090808 | Homo sapiens cDNA FLJ33489 fis, clone BRAMY2003585 | 65.29 | UP | 2.87E-05 |
| CA306881 | UI-H-FT1-bht-n-22-0-UI.s1 NCI_CGAP_FT1 Homo sapiens cDNA clone UI-H-FT1-bht-n-22-0-UI 3, mRNA sequence | 64.98 | UP | 2.93E-05 |
| NM_001448 | Homo sapiens glypican 4 (GPC4), mRNA | 64.85 | UP | 2.45E-04 |
| AL833276 | Homo sapiens mRNA; cDNA DKFZp451D088 (from clone DKFZp451D088) | 64.7 | UP | 1.98E-05 |
| BC044843 | Homo sapiens hypothetical protein LOC339535, mRNA (cDNA clone IMAGE:5186761), partial cds | 63.8 | UP | 7.59E-05 |
| BF798098 | RC1-C10045-021000-021-f02 CI0045 Homo sapiens cDNA, mRNA sequence | 58.58 | UP | 3.04E-05 |
| H89526 | yw28b04.r1 Morton Fetal Cochlea Homo sapiens cDNA clone IMAGE:253519 5, mRNA sequence | 56.92 | UP | 3.82E-05 |
| BF509573 | UI-H-B14-apf-b-11-0-UI.s1 NCI_CGAP_Sub8 Homo sapiens cDNA clone IMAGE:3086949 3, mRNA sequence | 56.79 | UP | 2.83E-05 |
| NM_000990 | Homo sapiens ribosomal protein L27a (RPL27A), mRNA | 56.36 | UP | 1.73E-05 |
| BC042028 | Homo sapiens, clone IMAGE:4794726, mRNA | 55.75 | UP | 1.64E-05 |
| NM_003287 | Homo sapiens tumor protein D52-like 1 (TPD52L1), transcript variant 1, mRNA | 55.25 | UP | 8.46E-06 |
| NM_018168 | Homo sapiens chromosome 14 open reading frame 105 (C14orf105), mRNA | 54.64 | UP | 4.79E-05 |
| NM_152284 | Homo sapiens Snf7 homologue associated with Alix 3 (Shax3), mRNA | 54.16 | UP | 9.09E-06 |
| AK096975 | Homo sapiens cDNA FLJ39656 fis, clone SMINT2005956 | 54.14 | UP | 8.37E-05 |
| AI249696 | qj64a03.x1 NCI_CGAP_Kid3 Homo sapiens cDNA clone IMAGE:1864204 3, mRNA sequence | 53.24 | UP | 7.14E-05 |
| NM_003551 | Homo sapiens non-metastatic cells 5, protein expressed in (nucleoside-diphosphate kinase) (NME5), mRNA | 53.01 | UP | 4.75E-05 |
| BX118238 | BX118238 NCI_CGAP_Kid3 Homo sapiens cDNA clone IMAGp998L153800 ; IMAGE:1501598, mRNA sequence | 51.99 | UP | 5.64E-05 |
| BX102632 | BX102632 NCI_CGAP_Co3 Homo sapiens cDNA clone IMAGp998J052307 ; IMAGE:928228, mRNA sequence | 51.79 | UP | 9.10E-05 |
| AF055376 | Homo sapiens short form transcription factor C-MAF (c-maf) mRNA, complete cds | 51.22 | UP | 3.94E-05 |
| NM_012464 | Homo sapiens tolloid-like 1 (TLL1), mRNA | 51.19 | UP | 6.57E-04 |
| NM_030949 | Homo sapiens protein phosphatase 1, regulatory (inhibitor) subunit 14C (PPP1 R14C), mRNA | 49.96 | UP | 5.80E-05 |
| BM669002 | UI-E-CK1-afn-m-04-0-UI.s2 UI-E-CK1 Homo sapiens cDNA clone UI-E-CK1-afn-m-04-0-UI 3, mRNA sequence | 49.05 | UP | 1.78E-05 |
| NM_000582 | Homo sapiens secreted phosphoprotein 1 (osteopontin, bone sialoprotein I, early T-lymphocyte activation 1) (SPP1), mRNA | 46.79 | UP | 6.18E-05 |
| NM_006722 | Homo sapiens microphthalmia-associated transcription factor (MITF), transcript variant 3, mRNA | 46.73 | UP | 1.26E-05 |
| NM_152423 | Homo sapiens melanoma associated antigen (mutated) 1-like 1 (MUM1L1), mRNA | 46.24 | UP | 1.48E-04 |
| AA075748 | zm89e04.r1 Stratagene ovarian cancer (#937219) Homo sapiens cDNA clone IMAGE:545118 5, mRNA sequence | 45.76 | UP | 4.39E-05 |
| NM_017549 | Homo sapiens upregulated in colorectal cancer gene 1 (UCC1), mRNA | 45.18 | UP | 1.24E-05 |
| BF431030 | 7o18c06.x1 NCI_CGAP_Kid11 Homo sapiens cDNA clone IMAGE:3574283 3, mRNA sequence | 44.98 | UP | 7.75E-05 |
| NM_002245 | Homo sapiens potassium channel, subfamily K, member 1 (KCNK1), mRNA | 44.3 | UP | 4.65E-05 |
| NM_003494 | Homo sapiens dysferlin, limb girdle muscular dystrophy 2B (autosomal recessive) (DYSF), mRNA | 44.23 | UP | 1.78E-05 |
| AK074097 | Homo sapiens mRNA for FLJ00168 protein | 43.88 | UP | 7.31E-05 |
| NM_013410 | Homo sapiens adenylate kinase 3 (AK3), nuclear gene encoding mitochondrial protein, transcript variant 2, mRNA | 42.34 | UP | 2.96E-05 |
| AK000075 | Homo sapiens cDNA FLJ20068 fis, clone COL01755 | 42.18 | UP | 1.35E-04 |
| NM_001200 | Homo sapiens bone morphogenetic protein 2 (BMP2), mRNA | 41.13 | UP | 2.11E-05 |
| NM_032782 | Homo sapiens hepatitis A virus cellular receptor 2 (HAVCR2), mRNA | 41.13 | UP | 2.83E-05 |
| AW151660 | xf67d04.x1 NCI_CGAP_Gas4 Homo sapiens cDNA clone IMAGE:2623111 3, mRNA sequence | 40.6 | UP | 8.65E-05 |
| NM_130435 | Homo sapiens protein tyrosine phosphatase, receptor type, E (PTPRE), transcript variant 2, mRNA | 40.41 | UP | 1.62E-04 |
| AA738254 | nx13b02.s1 NCI_CGAP_GC3 Homo sapiens cDNA clone IMAGE:1255947 3, mRNA sequence | 40.24 | UP | 2.43E-05 |
| AK124873 | Homo sapiens cDNA FLJ42883 fis, clone BRHIP3006683 | 40.2 | UP | 1.25E-04 |
| NM_000170 | Homo sapiens glycine dehydrogenase (decarboxylating; glycine decarboxylase, glycine cleavage system protein P) (GLDC), mRNA | 40.06 | UP | 4.98E-05 |
| AK125490 | Homo sapiens cDNA FLJ43501 fis, clone PEBLM2004497 | 39.93 | UP | 1.64E-04 |
| NM_004862 | Homo sapiens lipopolysaccharide-induced TNF factor (LITAF), mRNA | 39.52 | UP | 1.37E-05 |
| NM_004617 | Homo sapiens transmembrane 4 superfamily member 4 (TM4SF4), mRNA | 39.3 | UP | 9.32E-05 |
| NM_001263 | Homo sapiens CDP-diacylglycerol synthase (phosphatidate cytidylyltransferase) 1 (CDS1), mRNA | 39.26 | UP | 3.44E-05 |
| AK000776 | Homo sapiens cDNA FLJ20769 fis, clone COL06674 | 38.12 | UP | 2.83E-05 |
| AL833166 | Homo sapiens mRNA; cDNA DKFZp68612118 (from clone DKFZp68612118) | 37.72 | UP | 1.14E-04 |
| NM_198488 | Homo sapiens FLJ46072 protein (FLJ46072), mRNA | 37.66 | UP | 2.46E-05 |
| NM_005562 | Homo sapiens laminin, gamma 2 (LAMC2), transcript variant 1, mRNA | 37.4 | UP | 8.46E-06 |
| AW268540 | xv51e10.x1 NCI_GAP_L28 Homo sapiens cDNA clone IMAGE:2816682 3, mRNA sequence | 37.26 | UP | 5.82E-05 |
| NM_012198 | Homo sapiens grancalcin, EF-hand calcium binding protein (GCA), mRNA | 36.52 | UP | 2.13E-05 |
| NM_020808 | Homo sapiens signal-induced proliferation-associated 1 like 2 (SIPA1L2), mRNA | 36.28 | UP | 9.39E-05 |
| NM_000927 | Homo sapiens ATP-binding cassette, sub-family B (MDR/TAP), member 1 (ABCB1), mRNA | 35.89 | UP | 2.20E-04 |
| AK127644 | Homo sapiens cDNA FLJ45742 fis, clone KIDNE2016327 | 35.81 | UP | 3.60E-04 |
| NM_006158 | Homo sapiens neurofilament, light polypeptide 68kDa (NEFL), mRNA | 35.78 | UP | 2.64E-05 |
| AK074181 | Homo sapiens mRNA for FLJ00254 protein | 35.75 | UP | 8.46E-06 |
| BC043195 | Homo sapiens cDNA clone IMAGE:5288757, partial cds | 35.12 | UP | 1.24E-04 |
| AL389942 | Homo sapiens mRNA full length insert cDNA clone EUROIMAGE 2005635 | 34.31 | UP | 1.37E-04 |
| BC045828 | Homo sapiens zinc finger protein 608, mRNA (cDNA clone IMAGE:5262896), partial cds | 34.02 | UP | 1.88E-05 |
| NM_153229 | Homo sapiens hypothetical protein FLJ33318 (FLJ33318), mRNA | 34.02 | UP | 9.39E-05 |
| NM_184087 | Homo sapiens tripartite motif-containing 55 (TRIM55), transcript variant 4, mRNA | 33.68 | UP | 2.18E-05 |
| NM_000216 | Homo sapiens Kallmann syndrome 1 sequence (KAL1 ), mRNA | 33.32 | UP | 1.78E-05 |
| BF696790 | 602125323F1 NIH_MGC_56 Homo sapiens cDNA clone IMAGE:4282540 5, mRNA sequence | 32.7 | UP | 5.00E-05 |
| NM_006598 | Homo sapiens solute carrier family 12 (potassium/chloride transporters), member 7 (SLC12A7), mRNA | 32.41 | UP | 8.39E-06 |
| BQ003401 | UI-H-EI1-azd-j-23-0-UIs1 NCI_CGAP_E11 Homo sapiens cDNA clone IMAGE:5847286 3, mRNA sequence | 32.41 | UP | 1.72E-05 |
| NM_003222 | Homo sapiens transcription factor AP-2 gamma (activating enhancer binding protein 2 gamma) (TFAP2C), mRNA | 32.33 | UP | 4.75E-05 |
| NM_000599 | Homo sapiens insulin-like growth factor binding protein 5 (IGFBP5), mRNA | 32.1 | UP | 5.66E-05 |
| NM_198389 | Homo sapiens lung type-I cell membrane-associated glycoprotein (T1A-2), transcript variant 2, mRNA | 31.44 | UP | 1.03E-04 |
| NM_180991 | Homo sapiens solute carrier organic anion transporter family, member 4C1 (SLCO4C1), mRNA | 30.86 | UP | 3.05E-04 |
| NM_031311 | Homo sapiens carboxypeptidase, vitellogenic-like (CPVL), transcript variant 1, mRNA | 29.97 | UP | 4.30E-05 |
| NM_052947 | Homo sapiens heart alpha-kinase (HAK), mRNA | 29.53 | UP | 4.34E-05 |
| H89053 | yw24c06.r1 Morton Fetal Cochlea Homo sapiens cDNA clone IMAGE:253162 5, mRNA sequence | 29.27 | UP | 2.78E-05 |
| AI819186 | wj32d10.x1 NCI_CGAP_Kid12 Homo sapiens cDNA clone IMAGE:2404531 3, mRNA sequence | 29.14 | UP | 1.21E-04 |
| NM_003761 | Homo sapiens vesicle-associated membrane protein 8 (endobrevin) (VAMP8), mRNA | 28.5 | UP | 7.47E-05 |
| NM_080743 | Homo sapiens serine-arginine repressor protein (35 kDa) (SRrp35), mRNA | 27.83 | UP | 1.02E-05 |
| NM_007069 | Homo sapiens HRAS-like suppressor 3 (HRASLS3), mRNA | 27.51 | UP | 6.86E-06 |
| NM_002345 | Homo sapiens lumican (LUM), mRNA | 27.17 | UP | 2.20E-05 |
| BU569937 | AGENCOURT_10399817 NIH_MGC_82 Homo sapiens cDNA clone IMAGE:6618011 5, mRNA sequence | 27.05 | UP | 3.20E-05 |
| NM_024901 | Homo sapiens hypothetical protein FLJ22457 (FLJ22457), mRNA | 27.04 | UP | 5.37E-05 |
| N25875 | yw78d12.s1 Soares_placenta_8to9weeks_2NbHP8to9W Homo sapiens cDNA clone IMAGE:258359 3, mRNA sequence | 26.94 | UP | 3.36E-05 |
| NM_152573 | Homo sapiens RAS and EF hand domain containing (RASEF), mRNA | 26.91 | UP | 4.75E-05 |
| NM_018728 | Homo sapiens myosin VC (MYO5C), mRNA | 26.81 | UP | 4.47E-05 |
| AB033048 | Homo sapiens mRNA for KIAA1222 protein, partial cds | 26.76 | UP | 1.90E-04 |
| NM_153256 | Homo sapiens chromosome 10 open reading frame 47 (C10orf47), mRNA | 26.61 | UP | 2.48E-05 |
| BG221364 | RST41175 Athersys RAGE Library Homo sapiens cDNA, mRNA sequence | 26.25 | UP | 2.06E-04 |
| NM_000094 | Homo sapiens collagen, type VII, alpha 1 (epidermolysis bullosa, dystrophic, dominant and recessive) (COL7A1), mRNA | 26.16 | UP | 6.51E-05 |
| NM_001854 | Homo sapiens collagen, type XI, alpha 1 (COL11A1), transcript variant A, mRNA | 25.88 | UP | 1.36E-04 |
| BU740051 | UI-E-EO0-ahw-n-18-0-UI.s1 UI-E-EO0 Homo sapiens cDNA clone UI-E-EO0-ahw-n-18-0-UI 3, mRNA sequence | 25.64 | UP | 5.14E-05 |
| NM_014936 | Homo sapiens ectonucleotide pyrophosphatase/phosphodiesterase 4 (putative function) (ENPP4), mRNA | 25.62 | UP | 2.82E-04 |
| NM_024898 | Homo sapiens family with sequence similarity 31, member C (FAM31C), mRNA | 25.44 | UP | 7.17E-04 |
| W93585 | zd95g01.s1 Soares_fetal_heart_NbHH19W Homo sapiens cDNA clone IMAGE:357264 3, mRNA sequence | 25.41 | UP | 1.78E-05 |
| BF510493 | UI-H-B14-apa-b-08-0-UI.s1 NCI_CGAP_Sub8 Homo sapiens cDNA clone IMAGE:3086558 3, mRNA sequence | 25.28 | UP | 2.11E-05 |
| NM_020962 | Homo sapiens likely ortholog of mouse neighbor of Punc E11 (NOPE), mRNA | 25.23 | UP | 7.99E-05 |
| NM_016212 | Homo sapiens TP53TG3 protein (TP53TG3), mRNA | 25.14 | UP | 4.07E-05 |
| NM_021012 | Homo sapiens potassium inwardly-rectifying channel, subfamily J, member 12 (KCNJ12), mRNA | 24.7 | UP | 2.10E-04 |
| NM_001935 | Homo sapiens dipeptidylpeptidase 4 (CD26, adenosine deaminase complexing protein 2) (DPP4), mRNA | 24.67 | UP | 8.46E-06 |
| NM_001993 | Homo sapiens coagulation factor III (thromboplastin, tissue factor) (F3), mRNA | 24.47 | UP | 6.12E-05 |
| NM_184087 | Homo sapiens tripartite motif-containing 55 (TRIM55), transcript variant 4, mRNA | 24.38 | UP | 8.46E-06 |
| AK056431 | Homo sapiens cDNA FLJ31869 fis, clone NT2RP7002151 | 24.34 | UP | 2.73E-05 |
| NM_000775 | Homo sapiens cytochrome P450, family 2, subfamily J, polypeptide 2 (CYP2J2), mRNA | 24.04 | UP | 3.53E-05 |
| NM_006722 | Homo sapiens microphthalmia-associated transcription factor (MITF), transcript variant 3, mRNA | 23.95 | UP | 5.23E-06 |
| NM_001977 | Homo sapiens glutamyl aminopeptidase (aminopeptidase A) (ENPEP), mRNA | 23.91 | UP | 1.88E-05 |
| AK058012 | Homo sapiens cDNA FLJ25283 fis, clone STM06716 | 23.55 | UP | 1.88E-05 |
| BU680661 | UI-CF-DU1-aaz-f-04-0-UI.s1 UI-CF-DU1 Homo sapiens cDNA clone UI-CF-DU1-aaz-f-04-0-UI 3, mRNA sequence | 23.17 | UP | 1.82E-05 |
| NM_002246 | Homo sapiens potassium channel, subfamily K, member 3 (KCNK3), mRNA | 22.96 | UP | 2.27E-05 |
| NM_052923 | Homo sapiens zinc finger protein 452 (ZNF452), mRNA | 22.72 | UP | 3.77E-05 |
| NM_014373 | Homo sapiens G protein-coupled receptor 160 (GPR160), mRNA | 22.71 | UP | 3.94E-05 |
| BX538226 | Homo sapiens mRNA; cDNA DKFZp686E1944 (from clone DKFZp686E1944) | 22.58 | UP | 1.05E-04 |
| NM_173660 | Homo sapiens hypothetical protein FLJ33718 (FLJ33718), mRNA | 22.46 | UP | 2.83E-05 |
| NM_002354 | Homo sapiens tumor-associated calcium signal transducer 1 (TACSTD1), mRNA | 21.97 | UP | 2.83E-05 |
| NM_005822 | Homo sapiens Down syndrome critical region gene 1-like 1 (DSCR1L1), mRNA | 21.78 | UP | 2.21E-04 |
| W20132 | zb40c10.r1 Soares_parathyroid_tumor_NbHPA Homo sapiens cDNA clone IMAGE:306066 5, mRNA sequence | 21.76 | UP | 4.41E-04 |
| NM_001680 | Homo sapiens FXYD domain containing ion transport regulator 2 (FXYD2), transcript variant a, mRNA | 21.75 | UP | 3.26E-05 |
| NM_006379 | Homo sapiens sema domain, immunoglobulin domain (Ig), short basic domain, secreted, (semaphorin) 3C (SEMA3C), mRNA | 21.57 | UP | 3.15E-05 |
| H23441 | ym52f11.s1 Soares infant brain 1 NIB Homo sapiens cDNA clone IMAGE:51888 3, mRNA sequence | 21.43 | UP | 3.51E-05 |
| BG211832 | RST31404 Athersys RAGE Library Homo sapiens cDNA, mRNA sequence | 21.35 | UP | 5.06E-05 |
| N51335 | yz15e08.s1 Soares_multiple_sclerosis_2NbHMSP Homo sapiens cDNA clone IMAGE:283142 3, mRNA sequence | 21.19 | UP | 6.92E-03 |
| NM_022843 | Homo sapiens protocadherin 20 (PCDH20), mRNA | 21.17 | UP | 2.03E-05 |
| NM_002521 | Homo sapiens natriuretic peptide precursor B (NPPB), mRNA | 21.01 | UP | 4.54E-04 |
| NM_021101 | Homo sapiens claudin 1 (CLDN1), mRNA | 20.92 | UP | 3.04E-05 |
| NM_004165 | Homo sapiens Ras-related associated with diabetes (RRAD), mRNA | 20.76 | UP | 6.04E-04 |
| NM_005329 | Homo sapiens hyaluronan synthase 3 (HAS3), transcript variant 1, mRNA | 20.73 | UP | 4.13E-05 |
| NM_138432 | Homo sapiens serine dehydratase-like (SDSL), mRNA | 20.41 | UP | 7.27E-05 |
| NM_004591 | Homo sapiens chemokine (C-C motif) ligand 20 (CCL20), mRNA | 20.34 | UP | 3.29E-04 |
| NM_003063 | Homo sapiens sarcolipin (SLN), mRNA | 20.26 | UP | 1.18E-04 |
| BU951469 | in60a05.x3 HR85 islet Homo sapiens cDNA clone IMAGE:6126249 3, mRNA sequence | 20.02 | UP | 4.52E-04 |
| AI436290 | th81c01.x1 Soares_NhHMPu_S1 Homo sapiens cDNA clone IMAGE:2125056 3, mRNA sequence | 19.92 | UP | 9.28E-05 |
| NM_014452 | Homo sapiens tumor necrosis factor receptor superfamily, member 21 (TNFRSF21), mRNA | 19.39 | UP | 2.96E-05 |
| AA099748 | zl78c09.s1 Stratagene colon (#937204) Homo sapiens cDNA clone IMAGE:510736 3, mRNA sequence | 19.2 | UP | 1.78E-05 |
| NM_018354 | Homo sapiens chromosome 20 open reading frame 46 (C20orf46), mRNA | 18.87 | UP | 5.05E-04 |
| NM_194298 | Homo sapiens solute carrier family 16 (monocarboxylic acid transporters), member 9 (SLC16A9), mRNA | 18.87 | UP | 9.82E-03 |
| NM_033554 | Homo sapiens major histocompatibility complex, class II, DP alpha 1 (HLA-DPA1), mRNA | 18.6 | UP | 5.61E-04 |
| NM_004524 | Homo sapiens lethal giant larvae homolog 2 (Drosophila) (LLGL2), mRNA | 18.45 | UP | 2.11E-05 |
| NM_032293 | Homo sapiens GTPase activating Rap/RanGAP domain-like 3 (GARNL3), mRNA | 18.19 | UP | 2.24E-04 |
| NM_022842 | Homo sapiens CUB domain-containing protein 1 (CDCP1), transcript variant 1, mRNA | 18.14 | UP | 1.64E-05 |
| NM_000227 | Homo sapiens laminin, alpha 3 (LAMA3), transcript variant 2, mRNA | 18.04 | UP | 3.92E-05 |
| NM_000214 | Homo sapiens jagged 1 (Alagille syndrome) (JAG1), mRNA | 18.03 | UP | 1.26E-04 |
| AK096580 | Homo sapiens cDNA FLJ39261 fis, clone OCBBF2009391 | 17.89 | UP | 3.05E-04 |
| NM_014243 | Homo sapiens a disintegrin-like and metalloprotease (reprolysin type) with thrombospondin type 1 motif, 3 (ADAMTS3), mRNA | 17.86 | UP | 1.78E-05 |
| NM_000856 | Homo sapiens guanylate cyclase 1, soluble, alpha 3 (GUCY1A3), mRNA | 17.85 | UP | 1.19E-04 |
| NM_018404 | Homo sapiens centaurin, alpha 2 (CENTA2), mRNA | 17.8 | UP | 3.05E-04 |
| NM_001165 | Homo sapiens baculoviral IAP repeat-containing 3 (BIRC3), transcript variant 1, mRNA | 17.74 | UP | 2.56E-04 |
| NM_003706 | Homo sapiens phospholipase A2, group IVC (cytosolic, calcium-independent) (PLA2G4C), mRNA | 17.65 | UP | 4.77E-05 |
| AK056882 | Homo sapiens cDNA FLJ32320 fis, clone PROST2003537 | 17.65 | UP | 4.33E-04 |
| NM_017640 | Homo sapiens leucine rich repeat containing 16 (LRRC16), mRNA | 17.43 | UP | 1.04E-04 |
| NM_000480 | Homo sapiens adenosine monophosphate deaminase (isoform E) (AMPD3), mRNA | 17.4 | UP | 1.15E-05 |
| BX103476 | BX103476 NCI_CGAP_Lu5 Homo sapiens cDNA clone IMAGp998C053946 ; IMAGE:1557436, mRNA sequence | 17.29 | UP | 7.13E-05 |
| NM_013427 | Homo sapiens Rho GTPase activating protein 6 (ARHGAP6), transcript variant 1, mRNA | 17.27 | UP | 1.48E-04 |
| S70348 | Homo sapiens integrin beta 3 mRNA, partial cds, alternatively spliced | 17.24 | UP | 3.37E-05 |
| NM_032024 | Homo sapiens chromosome 10 open reading frame 11 (C10orf11), mRNA | 16.95 | UP | 3.03E-05 |
| BM988642 | UI-H-DH0-arx-p-21-0-UI.s1 NCI_CGAP_DH0 Homo sapiens cDNA clone IMAGE:5855492 3, mRNA sequence | 16.94 | UP | 1.94E-04 |
| NM_021102 | Homo sapiens serine protease inhibitor, Kunitz type, 2 (SPINT2), mRNA | 16.94 | UP | 1.85E-04 |
| AI632692 | wa33b05.x1 NCI_CGAP_Kid11 Homo sapiens cDNA clone IMAGE:2299857 3, mRNA sequence | 16.88 | UP | 7.19E-04 |
| M_152487 | Homo sapiens hypothetical protein FLJ31842 (FLJ31842), mRNA | 16.75 | UP | 2.55E-04 |
| NM_173567 | Homo sapiens abhydrolase domain containing 7 (ABHD7), mRNA | 16.66 | UP | 2.13E-05 |
| CA502927 | UI-CF-FN0-afq-j-12-0-UI.s1 UI-CF-FN0 Homo sapiens cDNA clone UI-CF-FN0-afq-j-12-0-UI 3, mRNA sequence | 16.59 | UP | 2.35E-04 |
| NM_015888 | Homo sapiens hook homolog 1 (Drosophila) (HOOK1), mRNA | 16.44 | UP | 4.12E-05 |
| NM_178177 | Homo sapiens nicotinamide nucleotide adenylyltransferase 3 (NMNAT3), mRNA | 16.43 | UP | 1.56E-04 |
| NM_005181 | Homo sapiens carbonic anhydrase III, muscle specific (CA3), mRNA | 16.42 | UP | 4.93E-05 |
| NM_000599 | Homo sapiens insulin-like growth factor binding protein 5 (IGFBP5), mRNA | 16.27 | UP | 4.97E-04 |
| NM_006393 | Homo sapiens nebulette (NEBL), transcript variant 1, mRNA | 16.13 | UP | 6.06E-04 |
| NM_006252 | Homo sapiens protein kinase, AMP-activated, alpha 2 catalytic subunit (PRKAA2), mRNA | 16.12 | UP | 5.64E-04 |
| NM_002338 | Homo sapiens limbic system-associated membrane protein (LSAMP), mRNA | 16.11 | UP | 2.81E-04 |
| NM_006378 | Homo sapiens sema domain, immunoglobulin domain (Ig), transmembrane domain (TM) and short cytoplasmic domain, (semaphorin) 4D (SEMA4D), mRNA | 16.09 | UP | 1.78E-05 |
| AI819863 | wj45h05.x1 NCI_CGAP_Lu19 Homo sapiens cDNA clone IMAGE:2405817 3, mRNA sequence | 15.95 | UP | 3.04E-04 |
| BM979825 | UI-CF-DU1-adt-f-12-0-Ul.s1 UI-CF-DU1 Homo sapiens cDNA clone UI-CF-DU1-adt-f-12-0-UI 3, mRNA sequence | 15.9 | UP | 2.77E-05 |
| BX647876 | Homo sapiens mRNA; cDNA DKFZp313A1525 (from clone DKFZp313A1525) | 15.83 | UP | 4.39E-05 |
| NM_003730 | Homo sapiens ribonuclease T2 (RNASET2), mRNA | 15.6 | UP | 2.41E-05 |
| AL117425 | Homo sapiens mRNA; cDNA DKFZp566L203 (from clone DKFZp566L203) | 15.59 | UP | 3.31E-04 |
| AL833346 | Homo sapiens mRNA; cDNA DKFZp686M2234 (from clone DKFZp686M2234) | 15.59 | UP | 2.99E-05 |
| NM_199169 | Homo sapiens transmembrane, prostate androgen induced RNA (TMEPAI), transcript variant 2, mRNA | 15.58 | UP | 1.34E-04 |
| NM_002559 | Homo sapiens purinergic receptor P2X, ligand-gated ion channel, 3 (P2RX3), mRNA | 15.51 | UP | 9.39E-05 |
| NM_032148 | Homo sapiens solute carrier family 41, member 2 (SLC41A2), mRNA | 15.46 | UP | 2.38E-05 |
| BC021684 | Homo sapiens, clone IMAGE:3827252, mRNA | 15.41 | UP | 2.31E-05 |
| BF000009 | 7h15g04.x1 NCI_CGAP_Co16 Homo sapiens cDNA clone IMAGE:3316086 3, mRNA sequence | 15.33 | UP | 2.09E-05 |
| NM_000147 | Homo sapiens fucosidase, alpha-L-1, tissue (FUCA1), mRNA | 15.31 | UP | 9.99E-04 |
| NM_001252 | Homo sapiens tumor necrosis factor (ligand) superfamily, member 7 (TNFSF7), mRNA | 14.88 | UP | 1.85E-04 |
| NM_013322 | Homo sapiens sorting nexin 10 (SNX10), mRNA | 14.84 | UP | 2.64E-05 |
| NM_022440 | Homo sapiens mal, T-cell differentiation protein (MAL), transcript variant d, mRNA | 14.65 | UP | 2.14E-04 |
| NM_178470 | Homo sapiens WD repeat domain 40B (WDR40B), mRNA | 14.53 | UP | 3.34E-05 |
| NM_001873 | Homo sapiens carboxypeptidase E (CPE), mRNA | 14.51 | UP | 6.23E-05 |
| BM719937 | UI-E-EJ0-ahu-a-10-0-Ul.r1 UI-E-EJ0 Homo sapiens cDNA clone UI-E-EJ0-ahu-a-10-0-UI 5, mRNA sequence | 14.46 | UP | 1.64E-05 |
| NM_032551 | Homo sapiens G protein-coupled receptor 54 (GPR54), mRNA | 14.45 | UP | 5.64E-04 |
| T53523 | ya89h12.r1 Stratagene placenta (#937225) Homo sapiens cDNA clone IMAGE:68903 5, mRNA sequence | 14.42 | UP | 1.81E-04 |
| NM_173354 | Homo sapiens SNF1-like kinase (SNF1LK), mRNA | 14.19 | UP | 5.72E-05 |
| NM_014585 | Homo sapiens solute carrier family 40 (iron-regulated transporter), member 1 (SLC40A1), mRNA | 14.17 | UP | 4.06E-05 |
| N38753 | yy42d01.s1 Soares melanocyte 2NbHM Homo sapiens cDNA clone IMAGE:273889 3, mRNA sequence | 14.02 | UP | 1.15E-05 |
| NM_001710 | Homo sapiens B-factor, properdin (BF), mRNA | 13.95 | UP | 2.05E-04 |
| BC038556 | Homo sapiens, clone IMAGE:3446976, mRNA | 13.9 | UP | 4.45E-04 |
| AK125608 | Homo sapiens cDNA FLJ43620 fis, clone SPLEN2021701, highly similar to HLA CLASS I HISTOCOMPATIBILITY ANTIGEN, A-2 ALPHA CHAIN PRECURSOR | 13.88 | UP | 2.56E-04 |
| AW248516 | 2820632.3prime NIH_MGC_7 Homo sapiens cDNA clone IMAGE:2820632 3, mRNA sequence | 13.79 | UP | 2.22E-04 |
| NM_000600 | Homo sapiens interleukin 6 (interferon, beta 2) (IL6), mRNA | 13.62 | UP | 1.64E-05 |
| AK125852 | Homo sapiens cDNA FLJ43864 fis, clone TESTl4007799 | 13.46 | UP | 1.66E-04 |
| BM728728 | UI-E-EO1-aiv-c-02-0-UI.r1 UI-E-EO1 Homo sapiens cDNA clone UI-E-EO1-aiv-c-02-0-UI 5, mRNA sequence | 13.46 | UP | 2.77E-04 |
| NM_018650 | Homo sapiens MAP/microtubule affinity-regulating kinase 1 (MARK1), mRNA | 13.45 | UP | 4.39E-05 |
| NM_022154 | Homo sapiens solute carrier family 39 (zinc transporter), member 8 (SLC39A8), mRNA | 13.39 | UP | 4.78E-05 |
| AA908815 | og77h08.s1 NCI_CGAP_Ov8 Homo sapiens cDNA clone IMAGE:1454367 3, mRNA sequence | 13.23 | UP | 3.11E-05 |
| NM_016463 | Homo sapiens CXXC finger 5 (CXXC5), mRNA | 13.22 | UP | 9.67E-05 |
| AB011538 | Homo sapiens mRNA for MEGF5, partial cds | 13.19 | UP | 3.38E-04 |
| CA413744 | UI-H-EZ0-bat-h-12-0-UI.s1 NCI_CGAP_Ch1 Homo sapiens cDNA clone UI-H-EZ0-bat-h-12-0-UI 3, mRNA sequence | 13.17 | UP | 2.09E-05 |
| BM712072 | UI-E-DW1-ahc-b-11-0-UI.r1 UI-E-DW1 Homo sapiens cDNA clone UI-E-DW1-ahc-b-11-0-UI 5, mRNA sequence | 13.16 | UP | 9.39E-05 |
| BU727096 | UI-E-CR0-ach-e-12-0-UI.s1 UI-E-CR0 Homo sapiens cDNA clone UI-E-CR0-ach-e-12-0-UI 3, mRNA sequence | 13.14 | UP | 4.30E-05 |
| NM_032858 | Homo sapiens hypothetical protein FLJ14904 (FLJ14904), mRNA | 13.09 | UP | 1.83E-05 |
| BU729783 | UI-E-CK1-afh-h-18-0-UI.s1 UI-E-CK1 Homo sapiens cDNA clone UI-E-CK1-afh-h-18-0-UI 3, mRNA sequence | 12.92 | UP | 8.39E-06 |
| AB011095 | Homo sapiens mRNA for KIAA0523 protein, partial cds | 12.91 | UP | 3.44E-04 |
| BC033116 | Homo sapiens chromodomain helicase DNA binding protein 7, mRNA (cDNA clone IMAGE:3352674), partial cds | 12.87 | UP | 2.31E-05 |
| CB135276 | K-EST0187371 L5HLK1 Homo sapiens cDNA clone L5HLK1-32-B12 5, mRNA sequence | 12.75 | UP | 4.93E-05 |
| NM_182920 | Homo sapiens a disintegrin-like and metalloprotease (reprolysin type) with thrombospondin type 1 motif, 9 (ADAMTS9), transcript variant 1, mRNA | 12.73 | UP | 9.10E-05 |
| NM_000236 | Homo sapiens lipase, hepatic (LIPC), mRNA | 12.71 | UP | 1.16E-03 |
| NM_152694 | Homo sapiens zinc finger, CCHC domain containing 5 (ZCCHC5), mRNA | 12.66 | UP | 4.93E-05 |
| AB020640 | Homo sapiens mRNA for KIAA0833 protein, partial cds | 12.51 | UP | 2.31E-04 |
| NM_018330 | Homo sapiens KIAA1598 (KIAA1598), mRNA | 12.46 | UP | 6.01E-05 |
| NM_000064 | Homo sapiens complement component 3 (C3), mRNA | 12.42 | UP | 5.12E-04 |
| NM_152864 | Homo sapiens chromosome 20 open reading frame 58 (C20orf58), mRNA | 12.4 | UP | 1.66E-03 |
| AW591723 | xt85h10.x1 NCI_CGAP_Ut1 Homo sapiens cDNA clone IMAGE:2793283 3 similar to contains element MER32 repetitive element ;, mRNA sequence | 12.4 | UP | 3.71E-05 |
| NM_152768 | Homo sapiens hypothetical protein FLJ25378 (FLJ25378), mRNA | 12.28 | UP | 2.03E-05 |
| NM_152366 | Homo sapiens kelch/ankyrin repeat containing cyclin A1 interacting protein (KARCA1), transcript variant 1, mRNA | 12.19 | UP | 2.62E-05 |
| AW300043 | xs45a09.x1 NCI_CGAP _Kid11 Homo sapiens cDNA clone IMAGE:2772568 3, mRNA sequence | 12.13 | UP | 2.31E-05 |
| NM_006741 | Homo sapiens protein phosphatase 1, regulatory (inhibitor) subunit 1A (PPP1R1A), mRNA | 12.12 | UP | 2.10E-04 |
| NM_024726 | Homo sapiens IQ motif containing with AAA domain (IQCA), mRNA | 12.08 | UP | 5.33E-03 |
| NM_194284 | Homo sapiens claudin 23 (CLDN23), mRNA | 12.02 | UP | 2.87E-05 |
| AL049974 | Homo sapiens mRNA; cDNA DKFZp564B222 (from clone DKFZp564B222) | 12 | UP | 1.48E-04 |
| AI420213 | te92g09.x1 NCI_CGAP_Pr28 Homo sapiens cDNA clone IMAGE:2094208 3, mRNA sequence | 11.95 | UP | 3.59E-04 |
| NM_002800 | Homo sapiens proteasome (prosome, macropain) subunit, beta type, 9 (large multifunctional protease 2) (PSMB9), transcript variant 1, mRNA | 11.85 | UP | 4.69E-05 |
| CA391258 | cs13a10.x1 Human Retinal pigment epithelium/choroid cDNA (Un-normalized, unamplified): cs Homo sapiens cDNA clone cs13a10 3, mRNA sequence | 11.85 | UP | 3.64E-05 |
| NM_013430 | Homo sapiens gamma-glutamyltransferase 1 (GGT1), transcript variant 3, mRNA | 11.79 | UP | 1.38E-04 |
| NM_199169 | Homo sapiens transmembrane, prostate androgen induced RNA (TMEPAI), transcript variant 2, mRNA | 11.79 | UP | 8.39E-06 |
| NM_032471 | Homo sapiens protein kinase (cAMP-dependent, catalytic) inhibitor beta (PKIB), transcript variant 3, mRNA | 11.73 | UP | 1.28E-04 |
| NM_016269 | Homo sapiens lymphoid enhancer-binding factor 1 (LEF1), mRNA | 11.72 | UP | 4.57E-05 |
| NM_003851 | Homo sapiens cellular repressor of E1A-stimulated genes 1 (CREG1), mRNA | 11.61 | UP | 1.83E-05 |
| NM_003236 | Homo sapiens transforming growth factor, alpha (TGFA), mRNA | 11.61 | UP | 6.51E-05 |
| BX110418 | BX110418 NCI_CGAP_Kid3 Homo sapiens cDNA clone IMAGp998C224149 ; IMAGE:1635405, mRNA sequence | 11.59 | UP | 1.89E-05 |
| NM_006033 | Homo sapiens lipase, endothelial (LIPG), mRNA | 11.55 | UP | 8.79E-04 |
| NM_025151 | Homo sapiens RAB11 family interacting protein 1 (class I) (RAB11FIP1 transcript variant 1, mRNA | 11.5 | UP | 2.65E-05 |
| BI759570 | 603046987F1 NIH_MGC_116 Homo sapiens cDNA clone IMAGE:5187285 5, mRNA sequence | 11.49 | UP | 3.71E-05 |
| NM_013261 | Homo sapiens peroxisome proliferative activated receptor, gamma, coactivator 1, alpha (PPARGC1A), mRNA | 11.3 | UP | 2.83E-04 |
| AI830524 | wh52c02.x1 NCI_CGAP_Kid11 Homo sapiens cDNA clone IMAGE:2384354 3, mRNA sequence | 11.29 | UP | 8.65E-04 |
| NM_139161 | Homo sapiens crumbs homolog 3 (Drosophila) (CRB3), transcript variant 2, mRNA | 11.15 | UP | 1.22E-04 |
| AK055362 | Homo sapiens cDNA FLJ30800 fis, clone FEBRA2001197 | 11.13 | UP | 1.02E-05 |
| BF055156 | 7j75f03.x1 Soares_NSF_F8_9W_OT_PA_P_S1 Homo sapiens cDNA clone IMAGE:3392285 3, mRNA sequence | 11.11 | UP | 4.94E-04 |
| BC016962 | Homo sapiens, clone IMAGE:4182947, mRNA | 11.05 | UP | 1.06E-04 |
| AY358775 | Homo sapiens clone DNA170212 WGAR9166 (UNQ9166) mRNA, complete cds | 11.05 | UP | 2.32E-04 |
| NM_175056 | Homo sapiens hypothetical protein LOC131368 (LOC131368), mRNA | 10.98 | UP | 1.33E-03 |
| AI311296 | ta48d10.x2 NCI_CGAP_Lu25 Homo sapiens cDNA clone IMAGE:2047315 3, mRNA sequence | 10.93 | UP | 2.19E-04 |
| AW296834 | UI-H-BI2-ahz-a-10-0-UI.s1 NCI_CGAP_Sub4 Homo sapiens cDNA clone IMAGE:2728243 3, mRNA sequence | 10.91 | UP | 1.71E-04 |
| NM_014464 | Homo sapiens tubulointerstitial nephritis antigen (TINAG), mRNA | 10.89 | UP | 3.87E-04 |
| AK024270 | Homo sapiens cDNA FLJ14208 fis, clone NT2RP3003264 | 10.88 | UP | 3.05E-04 |
| H00617 | yj25f02.s1 Soares placenta Nb2HP Homo sapiens cDNA clone IMAGE:149787 3, mRNA sequence | 10.87 | UP | 2.09E-04 |
| AK126467 | Homo sapiens cDNA FLJ44503 fis, clone UTERU3001158 | 10.87 | UP | 5.06E-05 |
| NM_181847 | Homo sapiens amphoterin induced gene 2 (AMIGO2), mRNA | 10.86 | UP | 8.91E-04 |
| NM_014751 | Homo sapiens metastasis suppressor 1 (MTSS1), mRNA | 10.86 | UP | 2.68E-05 |
| N70752 | za72d02.s1 Soares_fetal_lung_NbHL19W Homo sapiens cDNA clone IMAGE:298083 3, mRNA sequence | 10.84 | UP | 1.34E-04 |
| NM_005302 | Homo sapiens G protein-coupled receptor 37 (endothelin receptor type B-like) (GPR37), mRNA | 10.84 | UP | 2.63E-03 |
| NM_003264 | Homo sapiens toll-like receptor 2 (TLR2), mRNA | 10.83 | UP | 1.01E-04 |
| NM_017594 | Homo sapiens DIRAS family, GTP-binding RAS-like 2 (DIRAS2), mRNA | 10.81 | UP | 7.23E-04 |
| AI688800 | wd41b03.x1 Soares_NFL_T_GBC_S1 Homo sapiens cDNA clone IMAGE:2330669 3, mRNA sequence | 10.8 | UP | 8.56E-04 |
| H08012 | yl91b08.r1 Soares infant brain 1NIB Homo sapiens cDNA clone IMAGE:45474 5, mRNA sequence | 10.8 | UP | 3.26E-04 |
| AW014126 | UI-H-Bl0-aaj-a-05-0-Ul.s1 NCI_CGAP_Sub1 Homo sapiens cDNA clone IMAGE:2709393 3, mRNA sequence | 10.79 | UP | 1.88E-05 |
| NM_014422 | Homo sapiens phosphatidylinositol (4,5) bisphosphate 5-phosphatase, A (PIB5PA), transcript variant 1, mRNA | 10.73 | UP | 4.47E-05 |
| H62713 | yr28c08.r1 Soares fetal liver spleen 1 NFLS Homo sapiens cDNA clone IMAGE:206606 5 similar to gb:X01683 ALPHA-1-ANTITRYPSIN PRECURSOR (HUMAN);, mRNA sequence | 10.73 | UP | 1.01E-03 |
| AI870547 | wl47a04.x1 NCI_CGAP_Ut1 Homo sapiens cDNA clone IMAGE:2428014 3, mRNA sequence | 10.65 | UP | 4.82E-03 |
| NM_025202 | Homo sapiens EF hand domain containing 1 (EFHD1), mRNA | 10.64 | UP | 5.59E-05 |
| N78460 | yz76h06.r1 Soares_multiple_sclerosis_2NbHMSP Homo sapiens cDNA clone IMAGE:289019 5, mRNA sequence | 10.63 | UP | 3.90E-04 |
| NM_020661 | Homo sapiens activation-induced cytidine deaminase (AICDA), mRNA | 10.63 | UP | 7.27E-05 |
| NM_173549 | Homo sapiens hypothetical protein FLJ39553 (FLJ39553), mRNA | 10.57 | UP | 3.11E-04 |
| Al767472 | wh27a07.x1 NCI_CGAP_Kid11 Homo sapiens cDNA clone IMAGE:2381940 3 similar to contains L1.t3 L1 repetitive element ;, mRNA sequence | 10.56 | UP | 8.39E-06 |
| NM_145804 | Homo sapiens ankyrin repeat and BTB (POZ) domain containing 2 (ABTB2), mRNA | 10.55 | UP | 1.41E-06 |
| NM_013230 | Homo sapiens CD24 antigen (small cell lung carcinoma cluster 4 antigen) (CD24), mRNA | 10.54 | UP | 1.12E-04 |
| NM_000758 | Homo sapiens colony stimulating factor 2 (granulocyte-macrophage) (CSF2), mRNA | 10.51 | UP | 7.66E-04 |
| AB032945 | Homo sapiens mRNA for KIAA1119 protein, partial cds | 10.48 | UP | 1.32E-03 |
| NM_024572 | Homo sapiens UDP-N-acetyl-alpha-D-galactosamine:polypeptide N-acetylgalactosaminyltransferase 14 (GalNAc-T14) (GALNT14), mRNA | 10.46 | UP | 2.62E-04 |
| NM_181332 | Homo sapiens neuroligin 4, X-linked (NLGN4X), transcript variant 2, mRNA | 10.46 | UP | 1.95E-04 |
| NM_022128 | Homo sapiens ribokinase (RBKS), mRNA | 10.45 | UP | 2.73E-05 |
| AB046810 | Homo sapiens mRNA for KIAA1590 protein, partial cds | 10.39 | UP | 2.36E-05 |
| NM_022168 | Homo sapiens interferon induced with helicase C domain 1 (IFIH1), mRNA | 10.38 | UP | 2.83E-05 |
| NM_153715 | Homo sapiens homeo box A10 (HOXA10), transcript variant 2, mRNA | 10.37 | UP | 5.50E-05 |
| BF090392 | QV3-NT0023-120900-324-b04 NT0023 Homo sapiens cDNA, mRNA sequence | 10.35 | UP | 1.25E-03 |
| NM_000880 | Homo sapiens interleukin 7 (IL7), mRNA | 10.34 | UP | 3.60E-04 |
| NM_018650 | Homo sapiens MAP/microtubule affinity-regulating kinase 1 (MARK1), mRNA | 10.29 | UP | 1.57E-04 |
| NM_017899 | Homo sapiens hypothetical protein FLJ20607 (TSC), mRNA | 10.27 | UP | 6.79E-04 |
| NM_032367 | Homo sapiens zinc finger, BED domain containing 3 (ZBED3), mRNA | 10.27 | UP | 4.12E-05 |
| NM_012206 | Homo sapiens hepatitis A virus cellular receptor 1 (HAVCR1), mRNA | 10.23 | UP | 8.15E-04 |
| AK092245 | Homo sapiens cDNA FLJ34926 fis, clone NT2RP7003319, highly similar to Mus musculus neuralin mRNA | 10.21 | UP | 1.92E-03 |
| NM_031469 | Homo sapiens SH3 domain binding glutamic acid-rich protein like 2 (SH3BGRL2), mRNA | 10.18 | UP | 1.63E-05 |
| CA311343 | UI-CF-FN0-aff-b-19-0-UI.s1 UI-CF-FN0 Homo sapiens cDNA clone UI-CF-FN0-aff-b-19-0-UI 3, mRNA sequence | 10.15 | UP | 1.89E-05 |
| NM_030923 | Homo sapiens hypothetical protein DKFZp566N034 (DKFZP566N034), mRNA | 10.15 | UP | 2.23E-05 |
| AK024261 | Homo sapiens cDNA FLJ14199 fis, clone NT2RP3002713 | 10.14 | UP | 2.08E-04 |
| NM_174900 | Homo sapiens zinc finger protein 42 (ZFP42), mRNA | 10.1 | UP | 5.14E-05 |
| NM_198495 | Homo sapiens CTAGE family, member 4 (CTAGE4), mRNA | 10.07 | UP | 4.65E-05 |
| BX105791 | BX105791 Soares_testis_NHT Homo sapiens cDNA clone IMAGp998I101824 ; IMAGE:742737, mRNA sequence | 10.06 | UP | 2.15E-03 |
| AI672441 | wa03c03.x1 NCI_CGAP _Kid11 Homo sapiens cDNA clone IMAGE:2296996 3, mRNA sequence | 10.05 | UP | 2.40E-04 |
| AK002097 | Homo sapiens cDNA FLJ11235 fis, clone PLACE1008488 | 10.01 | UP | 2.75E-05 |
| BX647256 | Homo sapiens mRNA; cDNA DKFZp686K0753 (from clone DKFZp686K0753) | 9.97 | UP | 1.81E-03 |
| AK092456 | Homo sapiens cDNA FLJ35137 fis, clone PLACE6009419 | 9.96 | UP | 5.02E-05 |
| NM_001853 | Homo sapiens collagen, type IX, alpha 3 (COL9A3), mRNA | 9.96 | UP | 2.87E-05 |
| NM_004946 | Homo sapiens dedicator of cytokinesis 2 (DOCK2), mRNA | 9.94 | UP | 3.94E-05 |
| NM_005139 | Homo sapiens annexin A3 (ANXA3), mRNA | 9.92 | UP | 5.14E-05 |
| NM_002260 | Homo sapiens killer cell lectin-like receptor subfamily C, member 2 (KLRC2), mRNA | 9.92 | UP | 5.95E-04 |
| BX649112 | Homo sapiens mRNA; cDNA DKFZp686E02109 (from clone DKFZp686E02109) | 9.89 | UP | 6.13E-05 |
| AK127437 | Homo sapiens cDNA FLJ45529 fis, clone BRTHA2027546 | 9.85 | UP | 2.09E-05 |
| NM_080659 | Homo sapiens similar to RIKEN cDNA 2310030G06 gene (MGC14839), mRNA | 9.81 | UP | 1.33E-03 |
| NM_014867 | Homo sapiens KIAA0711 gene product (KIAA0711), mRNA | 9.81 | UP | 4.12E-05 |
| BG149255 | nad25d01.x1 NCI_CGAP_Lu24 Homo sapiens cDNA clone IMAGE:3366553 3, mRNA sequence | 9.7 | UP | 1.54E-04 |
| AK022598 | Homo sapiens cDNA FLJ12536 fis, clone NT2RM4000265 | 9.7 | UP | 1.38E-02 |
| AI792194 | ov03c02.y5 NCI_CGAP_Kid3 Homo sapiens cDNA clone IMAGE:1636226 5, mRNA sequence | 9.65 | UP | 6.10E-04 |
| NM_005502 | Homo sapiens ATP-binding cassette, subfamily A (ABC1), member 1 (ABCA1), mRNA | 9.65 | UP | 4.03E-04 |
| BE070450 | QV4-BT0407-020300-122-d08 BT0407 Homo sapiens cDNA, mRNA sequence | 9.62 | UP | 5.52E-05 |
| NM_006255 | Homo sapiens protein kinase C, eta (PRKCH), mRNA | 9.56 | UP | 2.09E-05 |
| NM_005114 | Homo sapiens heparan sulfate (glucosamine) 3-O-sulfotransferase 1 (HS3ST1), mRNA | 9.55 | UP | 1.44E-04 |
| AK123427 | Homo sapiens cDNA FLJ41433 fis, clone BRHIP2007307 | 9.46 | UP | 1.99E-04 |
| NM_013430 | Homo sapiens gamma-glutamyltransferase 1 (GGT1), transcript variant 3, mRNA | 9.4 | UP | 1.15E-04 |
| AK094353 | Homo sapiens cDNA FLJ37034 fis, clone BRACE2011478 | 9.4 | UP | 3.08E-05 |
| NM_016584 | Homo sapiens interleukin 23, alpha subunit p19 (IL23A), mRNA | 9.38 | UP | 3.77E-05 |
| NM_002089 | Homo sapiens chemokine (C-X-C motif) ligand 2 (CXCL2), mRNA | 9.38 | UP | 4.55E-04 |
| NM_032023 | Homo sapiens Ras association (RaIGDS/AF-6) domain family 4 (RASSF4), transcript variant 1, mRNA | 9.38 | UP | 5.39E-05 |
| D62831 | HUM330B12B Clontech human aorta polyA+ mRNA (#6572) Homo sapiens cDNA clone GEN-330B12 5, mRNA sequence | 9.32 | UP | 9.48E-05 |
| AI963999 | wt87g07.x1 NCI_CGAP_GC6 Homo sapiens cDNA clone IMAGE:2514492 3, mRNA sequence | 9.31 | UP | 8.57E-04 |
| NM_005978 | Homo sapiens S100 calcium binding protein A2 (S100A2), mRNA | 9.31 | UP | 1.64E-05 |
| NM_006622 | Homo sapiens polo-like kinase 2 (Drosophila) (PLK2), mRNA | 9.3 | UP | 2.93E-05 |
| NM_033495 | Homo sapiens kelch-like 13 (Drosophila) (KLHL13), mRNA | 9.25 | UP | 2.38E-05 |
| L07615 | Human neuropeptide Y receptor Y1 (NPYY1) mRNA, exon 2-3 and complete cds | 9.23 | UP | 1.09E-03 |
| NM_018076 | Homo sapiens armadillo repeat containing 4 (ARMC4), mRNA | 9.16 | UP | 5.96E-04 |
| CA778369 | ip17f03.y1 HR85 islet Homo sapiens cDNA clone IMAGE:6217493 5, mRNA sequence | 9.12 | UP | 5.40E-05 |
| NM_024554 | Homo sapiens piggyBac transposable element derived 5 (PGBD5), mRNA | 9.12 | UP | 2.63E-04 |
| NM_182487 | Homo sapiens olfactomedin-like 2A (OLFML2A), mRNA | 9.04 | UP | 1.64E-04 |
| T78754 | yd01f08.r1 Soares infant brain 1NIB Homo sapiens cDNA clone IMAGE:24180 5, mRNA sequence | 9.01 | UP | 1.09E-03 |
| NM_004024 | Homo sapiens activating transcription factor 3 (ATF3), mRNA | 9 | UP | 1.50E-03 |
| NM_030583 | Homo sapiens matrilin 2 (MATN2), transcript variant 2, mRNA | 8.98 | UP | 2.15E-04 |
| NM_145032 | Homo sapiens F-box and leucine-rich repeat protein 13 (FBXL13), mRNA | 8.97 | UP | 1.78E-05 |
| NM_020130 | Homo sapiens chromosome 8 open reading frame 4 (C8orf4), mRNA | 8.93 | UP | 4.79E-04 |
| NM_005737 | Homo sapiens ADP-ribosylation factor-like 7 (ARL7), mRNA | 8.87 | UP | 1.79E-04 |
| BG675167 | 602621444F1 NCI_CGAP_Skn3 Homo sapiens cDNA clone IMAGE:4755106 5, mRNA sequence | 8.86 | UP | 7.41E-04 |
| NM_194463 | Homo sapiens ring finger protein 128 (RNF128), transcript variant 1, mRNA | 8.85 | UP | 8.97E-05 |
| NM_018349 | Homo sapiens multiple C2-domains with two transmembrane regions 2 (MCTP2), mRNA | 8.81 | UP | 8.37E-05 |
| AK026740 | Homo sapiens cDNA: FLJ23087 fis, clone LNG06994, highly similar to AF161368 Homo sapiens HSPC105 mRNA | 8.8 | UP | 3.17E-04 |
| NM_000693 | Homo sapiens aldehyde dehydrogenase 1 family, member A3 (ALDH1A3), mRNA | 8.78 | UP | 4.72E-04 |
| NM_005459 | Homo sapiens guanylate cyclase activator 1C (GUCA1C), mRNA | 8.77 | UP | 1.57E-04 |
| BF509074 | UI-H-BI4-aou-b-08-0-UI.s1 NCI_CGAP_Sub8 Homo sapiens cDNA clone IMAGE:3086150 3, mRNA sequence | 8.74 | UP | 2.11E-05 |
| AL117425 | Homo sapiens mRNA; cDNA DKFZp566L203 (from clone DKFZp566L203) | 8.69 | UP | 2.83E-04 |
| NM_006257 | Homo sapiens protein kinase C, theta (PRKCQ), mRNA | 8.64 | UP | 1.78E-05 |
| NM_005567 | Homo sapiens lectin, galactoside-binding, soluble, 3 binding protein (LGALS3BP), mRNA | 8.64 | UP | 1.26E-05 |
| NM_173508 | Homo sapiens solute carrier family 35, member F3 (SLC35F3), mRNA | 8.62 | UP | 1.04E-04 |
| AK025281 | Homo sapiens cDNA: FLJ21628 fis, clone COL08076 | 8.48 | UP | 7.96E-05 |
| NM_145252 | Homo sapiens similar to common salivary protein 1 (LOC124220), mRNA | 8.48 | UP | 1.94E-04 |
| AI764969 | wh57b02.x1 NCI_CGAP_Kid11 Homo sapiens cDNA clone IMAGE:2384811 3, mRNA sequence | 8.48 | UP | 6.82E-05 |
| NM_002313 | Homo sapiens actin binding LIM protein 1 (ABLIM1), transcript variant 1, mRNA | 8.48 | UP | 3.03E-05 |
| NM_017641 | Homo sapiens kinesin family member 21A (KIF21A), mRNA | 8.42 | UP | 2.43E-05 |
| AA564703 | nj22h06.s1 NCI_CGAP_AA1 Homo sapiens cDNA clone IMAGE:993275 3, mRNA sequence | 8.4 | UP | 6.11E-05 |
| NM_001323 | Homo sapiens cystatin E/M (CST6), mRNA | 8.37 | UP | 1.02E-04 |
| NM_014333 | Homo sapiens immunoglobulin superfamily, member 4 (IGSF4), mRNA | 8.35 | UP | 1.82E-03 |
| NM_022454 | Homo sapiens SRY (sex determining region Y)-box 17 (SOX17), mRNA | 8.33 | UP | 6.04E-05 |
| NM_000186 | Homo sapiens complement factor H (CFH), mRNA | 8.32 | UP | 1.06E-03 |
| BF512544 | UI-H-BW1-amf-c-08-0-UIs1 NCI_CGAP_Sub7 Homo sapiens cDNA clone IMAGE:3069687 3, mRNA sequence | 8.31 | UP | 1.09E-04 |
| AF036977 | Homo sapiens clone HCG IV.9 unknown mRNA | 8.27 | UP | 1.12E-03 |
| AK095500 | Homo sapiens cDNA FLJ38181 fis, clone FCBBF1000125 | 8.19 | UP | 6.56E-05 |
| NM_001955 | Homo sapiens endothelin 1 (EDN1), mRNA | 8.18 | UP | 4.43E-04 |
| NM_002053 | Homo sapiens guanylate binding protein 1, interferon-inducible, 67kDa (GBP1), mRNA | 8.17 | UP | 8.39E-06 |
| NM_031476 | Homo sapiens hypothetical protein DKFZp434B044 (DKFZP434B044), mRNA | 8.12 | UP | 8.06E-04 |
| AA844712 | ai70e12.s1 Soares_testis_NHT Homo sapiens cDNA clone IMAGE:1376206 3, mRNA sequence | 8.11 | UP | 2.36E-04 |
| BM976385 | UI-CF-EN1-acz-f-03-0-UIs1 UI-CF-EN1 Homo sapiens cDNA clone UI-CF-EN1-acz-f-03-0-UI 3, mRNA sequence | 8.11 | UP | 7.27E-04 |
| BX647876 | Homo sapiens mRNA; cDNA DKFZp313A1525 (from clone DKFZp313A1525) | 8.11 | UP | 6.87E-04 |
| NM_001845 | Homo sapiens collagen, type IV, alpha 1 (COL4A1), mRNA | 8.06 | UP | 2.72E-05 |
| NM_014900 | Homo sapiens COBL-like 1 (COBLL1), mRNA | 8.04 | UP | 9.67E-05 |
| NM_203306 | Homo sapiens hypothetical protein MGC39606 (MGC39606), mRNA | 7.98 | UP | 1.95E-03 |
| BI963896 | ie66c06.x1 Melton Normalized Human Islet 4 N4-HIS 1 Homo sapiens cDNA clone IMAGE:5671691 3, mRNA sequence | 7.97 | UP | 2.73E-05 |
| NM_005025 | Homo sapiens serine (or cysteine) proteinase inhibitor, clade I (neuroserpin), member 1 (SERPINI1), mRNA | 7.97 | UP | 3.03E-05 |
| AK024238 | Homo sapiens cDNA FLJ14176 fis, clone NT2RP2003101 | 7.96 | UP | 4.75E-05 |
| NM_005951 | Homo sapiens metallothionein 1 H (MT1 H), mRNA | 7.95 | UP | 3.19E-03 |
| CF887677 | UI-CF-FN0-aev-o-22-18-UI.r18 UI-CF-FN0 Homo sapiens cDNA clone UI-CF-FN0-aev-o-22-18-UI 5, mRNA sequence | 7.93 | UP | 4.30E-05 |
| NM_024423 | Homo sapiens desmocollin 3 (DSC3), transcript variant Dsc3b, mRNA | 7.91 | UP | 1.23E-04 |
| AI355761 | qt94a11.x1 NCI_CGAP_Co14 Homo sapiens cDNA clone IMAGE:1962908 3 similar to gb:X74929 KERATIN, TYPE II CYTOSKELETAL 8 (HUMAN);, mRNA sequence | 7.91 | UP | 6.82E-05 |
| BF445031 | nad20f02.x1 NCI_CGAP_Lu24 Homo sapiens cDNA clone IMAGE:3366266 3, mRNA sequence | 7.91 | UP | 1.78E-05 |
| CA444471 | UI-H-DP0-avv-a-16-0-UI.s1 NCI_CGAP_Fs1 Homo sapiens cDNA clone UI-H-DPO-avv-a-16-0-UI 3, mRNA sequence | 7.89 | UP | 7.69E-05 |
| NM_014942 | Homo sapiens ankyrin repeat domain 6 (ANKRD6), mRNA | 7.89 | UP | 2.49E-05 |
| AK021801 | Homo sapiens cDNA FLJ11739 fis, clone HEMBA1005497 | 7.86 | UP | 1.15E-04 |
| NM_001766 | Homo sapiens CD1 D antigen, d polypeptide (CD1 D), mRNA | 7.85 | UP | 5.10E-04 |
| NM_005360 | Homo sapiens v-maf musculoaponeurotic fibrosarcoma oncogene homolog (avian) (MAF), mRNA | 7.81 | UP | 7.59E-05 |
| NM_018700 | Homo sapiens tripartite motif-containing 36 (TRIM36), mRNA | 7.8 | UP | 2.55E-05 |
| BX640643 | Homo sapiens mRNA; cDNA DKFZp686O24114 (from clone DKFZp686O24114) | 7.79 | UP | 2.59E-05 |
| NM_005331 | Homo sapiens hemoglobin, theta 1 (HBQ1), mRNA | 7.77 | UP | 3.73E-04 |
| NM_194298 | Homo sapiens solute carrier family 16 (monocarboxylic acid transporters), member 9 (SLC16A9), mRNA | 7.77 | UP | 2.66E-04 |
| NM_012413 | Homo sapiens glutaminyl-peptide cyclotransferase (glutaminyl cyclase) (QPCT), mRNA | 7.76 | UP | 4.60E-04 |
| NM_005860 | Homo sapiens follistatin-like 3 (secreted glycoprotein) (FSTL3), mRNA | 7.76 | UP | 2.64E-05 |
| AB033029 | Homo sapiens mRNA for KIAA1203 protein, partial cds | 7.7 | UP | 8.39E-06 |
| NM_012216 | Homo sapiens midline 2 (MID2), transcript variant 1, mRNA | 7.62 | UP | 6.12E-05 |
| NM_018986 | Homo sapiens SH3 domain and tetratricopeptide repeats 1 (SH3TC1), mRNA | 7.61 | UP | 8.69E-04 |
| BM665101 | UI-E-CQ1-aew-I-09-0-UI.s1 UI-E-CQ1 Homo sapiens cDNA clone UI-E-CQ1-aew-I-09-0-UI 3, mRNA sequence | 7.58 | UP | 4.09E-05 |
| AL706653 | DKFZp686E1543_r1 686 (synonym: hlcc3) Homo sapiens cDNA clone DKFZp686E1543 5, mRNA sequence | 7.57 | UP | 2.36E-05 |
| BF920409 | QV2-NT0144-071100-463-b06 NT0144 Homo sapiens cDNA, mRNA sequence | 7.55 | UP | 6.70E-04 |
| NM_173462 | Homo sapiens papilin, proteoglycan-like sulfated glycoprotein (PAPLN), mRNA | 7.55 | UP | 5.64E-05 |
| NM_031419 | Homo sapiens nuclear factor of kappa light polypeptide gene enhancer in B-cells inhibitor, zeta (NFKBIZ), transcript variant 1, mRNA | 7.55 | UP | 1.49E-04 |
| NM_016423 | Homo sapiens zinc finger protein 219 (ZNF219), mRNA | 7.53 | UP | 1.71E-05 |
| NM_020859 | Homo sapiens Shroom-related protein (ShrmL), mRNA | 7.52 | UP | 1.26E-05 |
| NM_004932 | Homo sapiens cadherin 6, type 2, K-cadherin (fetal kidney) (CDH6), mRNA | 7.52 | UP | 1.64E-05 |
| NM_182797 | Homo sapiens phospholipase C, beta 4 (PLCB4), transcript variant 2, mRNA | 7.52 | UP | 5.49E-05 |
| AL512697 | Homo sapiens mRNA; cDNA DKFZp547F134 (from clone DKFZp547F134) | 7.5 | UP | 6.17E-05 |
| BC035116 | Homo sapiens cDNA clone IMAGE:5263177, partial cds | 7.47 | UP | 2.32E-05 |
| AL353944 | Homo sapiens mRNA; cDNA DKFZp761J1112 (from clone DKFZp761J1112) | 7.45 | UP | 1.72E-05 |
| NM_152780 | Homo sapiens hypothetical protein FLJ14503 (FLJ14503), mRNA | 7.45 | UP | 1.19E-03 |
| AA888443 | nw74f10.s1 NCI_CGAP_Pr12 Homo sapiens cDNA clone IMAGE:1252363, mRNA sequence | 7.43 | UP | 5.18E-04 |
| AW450938 | UI-H-BI3-all-g-05-0-UI.s1 NCI_CGAP_Sub5 Homo sapiens cDNA clone IMAGE:2737329 3, mRNA sequence | 7.4 | UP | 4.98E-05 |
| NM_053039 | Homo sapiens UDP glycosyltransferase 2 family, polypeptide B28 (UGT2B28), mRNA | 7.38 | UP | 1.12E-03 |
| NM_016946 | Homo sapiens F11 receptor (F11 R), transcript variant 1, mRNA | 7.36 | UP | 4.65E-05 |
| AW969742 | EST381820 MAGE resequences, MAGK Homo sapiens cDNA, mRNA sequence | 7.36 | UP | 1.15E-04 |
| BX117866 | BX117866 NCI_CGAP_GCB1 Homo sapiens cDNA clone IMAGp998N233105 ; IMAGE:1234774, mRNA sequence | 7.34 | UP | 2.20E-04 |
| NM_005739 | Homo sapiens RAS guanyl releasing protein 1 (calcium and DAG-regulated) (RASGRP1), mRNA | 7.31 | UP | 2.20E-04 |
| CB047287 | NISC_gg01h01.y1 NCI_CGAP_Kid11 Homo sapiens cDNA clone IMAGE:3253464 5, mRNA sequence | 7.25 | UP | 3.52E-05 |
| NM_013951 | Homo sapiens paired box gene 8 (PAX8), transcript variant PAX8B, mRNA | 7.25 | UP | 5.00E-05 |
| AK125695 | Homo sapiens cDNA FLJ43707 fis, clone TESOP2001865 | 7.24 | UP | 1.62E-03 |
| NM_020796 | Homo sapiens sema domain, transmembrane domain (TM), and cytoplasmic domain, (semaphorin) 6A (SEMA6A), mRNA | 7.21 | UP | 1.01E-04 |
| BM929354 | UI-E-EJ1-aje-o-19-0-UI.r1 UI-E-EJ1 Homo sapiens cDNA clone UI-E-EJ1-aje-o-19-0-UI 5, mRNA sequence | 7.19 | UP | 5.49E-05 |
| BX103949 | BX103949 NCI_CGAP_Co3 Homo sapiens cDNA clone IMAGp998C112296 ; IMAGE:923842, mRNA sequence | 7.16 | UP | 6.37E-05 |
| NM_003475 | Homo sapiens chromosome 11 open reading frame 13 (C11orf13), mRNA | 7.14 | UP | 1.56E-04 |
| BE735115 | 601566084F1 NIH_MGC_21 Homo sapiens cDNA clone IMAGE:3840837 5, mRNA sequence | 7.12 | UP | 1.41E-03 |
| NM_207517 | Homo sapiens ADAMTS-like 3 (ADAMTSL3), mRNA | 7.11 | UP | 1.26E-05 |
| BU688263 | UI-CF-EC1-aea-g-11-0-UI.s1 UI-CF-EC1 Homo sapiens cDNA clone UI-CF-EC1-aea-g-11-0-UI 3, mRNA sequence | 7.06 | UP | 2.97E-05 |
| AK022971 | Homo sapiens cDNA FLJ12909 fis, clone NT2RP2004400 | 7.06 | UP | 7.80E-05 |
| NM_001740 | Homo sapiens calbindin 2, 29kDa (calretinin) (CALB2), transcript variant CALB2, mRNA | 7.05 | UP | 1.81E-04 |
| NM_022073 | Homo sapiens egl nine homolog 3 (C. elegans) (EGLN3), mRNA | 7.04 | UP | 5.64E-04 |
| BX117230 | BX117230 NCI_CGAP_Kid11 Homo sapiens cDNA clone IMAGp998I235695 ; IMAGE:2298718, mRNA sequence | 7.03 | UP | 1.56E-03 |
| NM_002230 | Homo sapiens junction plakoglobin (JUP), transcript variant 1, mRNA | 7.03 | UP | 2.78E-05 |
| AK023793 | Homo sapiens cDNA FLJ13731 fis, clone PLACE3000142 | 7.02 | UP | 4.04E-04 |
| AL117598 | Homo sapiens mRNA; cDNA DKFZp564H1663 (from clone DKFZp564H1663) | 7 | UP | 1.59E-04 |
| NM_001958 | Homo sapiens eukaryotic translation elongation factor 1 alpha 2 (EEF1A2), mRNA | 6.99 | UP | 5.20E-04 |
| NM_007029 | Homo sapiens stathmin-like 2 (STMN2), mRNA | 6.97 | UP | 4.47E-04 |
| BE968596 | 601649770F1 NIH_MGC_74 Homo sapiens cDNA clone IMAGE:3933472 5, mRNA sequence | 6.97 | UP | 8.39E-04 |
| AK126467 | Homo sapiens cDNA FLJ44503 fis, clone UTERU3001158 | 6.97 | UP | 3.82E-05 |
| AA832510 | oe62d06.s1 NCI_CGAP_Lu5 Homo sapiens cDNA clone IMAGE:1416203 3, mRNA sequence | 6.93 | UP | 9.67E-04 |
| CF137545 | UI-HF-BN0-ane-d-05-0-UI.r1 NIH_MGC_50 Homo sapiens cDNA clone IMAGE:3092384 5, mRNA sequence | 6.93 | UP | 2.70E-03 |
| NM_004895 | Homo sapiens cold autoinflammatory syndrome 1 (CIAS1), transcript variant 1, mRNA | 6.9 | UP | 1.46E-03 |
| NM_018424 | Homo sapiens erythrocyte membrane protein band 4.1 like 4B (EPB41 L4B), mRNA | 6.9 | UP | 4.68E-05 |
| NM_004390 | Homo sapiens cathepsin H (CTSH), transcript variant 1, mRNA | 6.89 | UP | 2.72E-05 |
| NM_024603 | Homo sapiens hypothetical protein FLJ11588 (FLJ11588), mRNA | 6.87 | UP | 3.80E-05 |
| AI492941 | qz42h08.x1 NCI_CGAP_Kid11 Homo sapiens cDNA clone IMAGE:2029599 3, mRNA sequence | 6.82 | UP | 8.39E-04 |
| NM_003985 | Homo sapiens tyrosine kinase, non-receptor, 1 (TNK1), mRNA | 6.8 | UP | 4.55E-05 |
| AK095399 | Homo sapiens cDNA FLJ38080 fis, clone CTONG2016185 | 6.8 | UP | 9.23E-04 |
| BQ020597 | UI-H-DP0-avd-a-13-0-UI.s1 NCI_CGAP_Fs1 Homo sapiens cDNA clone IMAGE:5877780 3, mRNA sequence | 6.8 | UP | 4.53E-05 |
| NM_001432 | Homo sapiens epiregulin (EREG), mRNA | 6.77 | UP | 1.25E-05 |
| NM_005485 | Homo sapiens poly (ADP-ribose) polymerase family, member 3 (PARP3), transcript variant 2, mRNA | 6.76 | UP | 6.46E-05 |
| AB058769 | Homo sapiens mRNA for KIAA1866 protein, partial cds | 6.76 | UP | 9.84E-05 |
| BU753362 | UI-1-BB1-air-h-09-0-UI.s1 NCI_CGAP_PI5 Homo sapiens cDNA clone UI-1-BB1-air-h-09-0-UI 3, mRNA sequence | 6.75 | UP | 1.08E-04 |
| AL834140 | Homo sapiens mRNA; cDNA DKFZp434A2029 (from clone DKFZp434A2029) | 6.73 | UP | 4.49E-05 |
| BX092501 | BX092501 NCI_CGAP_Lu5 Homo sapiens cDNA clone IMAGp998K143946 ; IMAGE:1557637, mRNA sequence | 6.71 | UP | 2.56E-04 |
| BX640973 | Homo sapiens mRNA; cDNA DKFZp686B15184 (from clone DKFZp686B15184) | 6.71 | UP | 2.36E-04 |
| NM_015277 | Homo sapiens neural precursor cell expressed, developmentally down-regulated 4-like (NEDD4L), mRNA | 6.7 | UP | 1.60E-05 |
| NM_021192 | Homo sapiens homeo box D11 (HOXD11), mRNA | 6.69 | UP | 8.94E-04 |
| NM_032621 | Homo sapiens brain expressed X-linked 2 (BEX2), mRNA | 6.69 | UP | 1.83E-04 |
| X02851 | Human mRNA for interleukin-1 precursor (pre IL-1) | 6.68 | UP | 3.32E-04 |
| NM_014258 | Homo sapiens synaptonemal complex protein 2 (SYCP2), mRNA | 6.68 | UP | 4.96E-02 |
| NM_000682 | Homo sapiens adrenergic, alpha-2B-, receptor (ADRA2B), mRNA | 6.67 | UP | 3.64E-05 |
| BQ435580 | AGENCOURT_7836890 NIH_MGC_82 Homo sapiens cDNA clone IMAGE:6102371 5, mRNA sequence | 6.64 | UP | 1.64E-04 |
| NM_024608 | Homo sapiens nei endonuclease VIII-like 1 (E. coli) (NEIL1), mRNA | 6.63 | UP | 3.23E-05 |
| AK057113 | Homo sapiens cDNA FLJ32551 fis, clone SPLEN1000087 | 6.63 | UP | 6.06E-04 |
| NM_130897 | Homo sapiens dynein, cytoplasmic, light polypeptide 2B (DNCL2B), mRNA | 6.62 | UP | 2.40E-03 |
| N39597 | yy51e04.s1 Soares_multiple_sclerosis_2NbHMSP Homo sapiens cDNA clone IMAGE:277086 3, mRNA sequence | 6.62 | UP | 8.33E-04 |
| BU616749 | UI-H-FH1-bfj-a-11-0-UI.s1 NCI_CGAP_FH1 Homo sapiens cDNA clone UI-H-FH1-bfj-a-11-0-UI 3, mRNA sequence | 6.59 | UP | 1.93E-04 |
| AW 195474 | xn38g09.x1 NCI_CGAP_Kid11 Homo sapiens cDNA clone IMAGE:2696032 3, mRNA sequence | 6.59 | UP | 6.82E-05 |
| AK127437 | Homo sapiens cDNA FLJ45529 fis, clone BRTHA2027546 | 6.57 | UP | 1.76E-04 |
| NM_004717 | Homo sapiens diacylglycerol kinase, iota (DGKI), mRNA | 6.56 | UP | 7.54E-05 |
| BU584197 | 2513030T6 LIVRTUT04 Homo sapiens cDNA clone 2513030 3, mRNA sequence | 6.53 | UP | 8.82E-04 |
| BU634363 | UI-H-FL1-bgx-o-20-0-UI.s1 NCI_CGAP_FL1 Homo sapiens cDNA clone UI-H-FL1-bgx-o-20-0-UI 3, mRNA sequence | 6.53 | UP | 1.24E-04 |
| NM_178868 | Homo sapiens chemokine-like factor super family 8 (CKLFSF8), mRNA | 6.52 | UP | 1.59E-03 |
| NM_002247 | Homo sapiens potassium large conductance calcium-activated channel, subfamily M, alpha member 1 (KCNMA1), mRNA | 6.49 | UP | 4.49E-05 |
| AW 188195 | xj93e12.x1 Soares_NFL_T_GBC_S1 Homo sapiens cDNA clone IMAGE:2664814 3 similar to contains element THR repetitive element ;, mRNA sequence | 6.49 | UP | 3.81E-05 |
| M_153634 | Homo sapiens copine VIII (CPNE8), mRNA | 6.48 | UP | 1.72E-04 |
| NM_145205 | Homo sapiens HMG2 like (LOC127540), mRNA | 6.47 | UP | 6.39E-05 |
| M_178868 | Homo sapiens chemokine-like factor super family 8 (CKLFSF8), mRNA | 6.47 | UP | 1.15E-05 |
| U63828 | Human tissue plasminogen activator mRNA, partial cds | 6.46 | UP | 5.51E-05 |
| NM_004529 | Homo sapiens myeloid/lymphoid or mixed-lineage leukemia (trithorax homolog, Drosophila); translocated to, 3 (MLLT3), mRNA | 6.46 | UP | 8.21E-05 |
| BX647655 | Homo sapiens mRNA; cDNA DKFZp451A211 (from clone DKFZp451A211) | 6.46 | UP | 6.94E-05 |
| AW470401 | xz83g01.x1 NCI_CGAP_Lu24 Homo sapiens cDNA clone IMAGE:2870832 3 similar to contains L1.t2 L1 repetitive element ;, mRNA sequence | 6.46 | UP | 1.30E-04 |
| BG570144 | 602591134F1 NIH_MGC_77 Homo sapiens cDNA clone IMAGE:4717761 5, mRNA sequence | 6.45 | UP | 1.08E-03 |
| BX115325 | BX115325 NCI_CGAP_Kid11 Homo sapiens cDNA clone IMAGp998K085276 ; IMAGE:2137855, mRNA sequence | 6.45 | UP | 1.04E-04 |
| NM_002214 | Homo sapiens integrin, beta 8 (ITGB8), mRNA | 6.44 | UP | 6.77E-05 |
| CA314926 | UI-CF-FN0-afi-b-19-0-UI.s1 UI-CF-FN0 Homo sapiens cDNA clone UI-CF-FN0-afi-b-19-0-UI 3, mRNA sequence | 6.43 | UP | 6.61E-05 |
| AK093870 | Homo sapiens cDNA FLJ36551 fis, clone TRACH2008127 | 6.43 | UP | 1.28E-03 |
| NM_000860 | Homo sapiens hydroxyprostaglandin dehydrogenase 15-(NAD) (HPGD), mRNA | 6.42 | UP | 1.76E-05 |
| AI831068 | wj62d12.x1 NCI_CGAP_Lu19 Homo sapiens cDNA clone IMAGE:2407415 3, mRNA sequence | 6.41 | UP | 1.37E-04 |
| NM_000349 | Homo sapiens steroidogenic acute regulator (STAR), nuclear gene encoding mitochondrial protein, transcript variant 1, mRNA | 6.41 | UP | 1.26E-04 |
| NM_014181 | Homo sapiens HSPC159 protein (HSPC159), mRNA | 6.4 | UP | 1.67E-04 |
| NM_024534 | Homo sapiens hypothetical protein FLJ12684 (FLJ12684), mRNA | 6.38 | UP | 6.57E-04 |
| NM_002843 | Homo sapiens protein tyrosine phosphatase, receptor type, J (PTPRJ), mRNA | 6.38 | UP | 1.32E-03 |
| A1926616 | wo48e04.x1 NCI_CGAP_Gas4 Homo sapiens cDNA clone IMAGE:2458590 3 similar to contains MER27.b2 MER27 repetitive element ;, mRNA sequence | 6.37 | UP | 7.01E-03 |
| NM_002273 | Homo sapiens keratin 8 (KRT8), mRNA | 6.36 | UP | 2.91E-03 |
| NM_207303 | Homo sapiens attractin-like 1 (ATRNL1), mRNA | 6.35 | UP | 7.85E-04 |
| AK001007 | Homo sapiens cDNA FLJ10145 fis, clone HEMBA1003322 | 6.35 | UP | 1.82E-05 |
| CB047092 | NISC_gf08f03.xl NCI_CGAP_Kid12 Homo sapiens cDNA clone IMAGE:3253013 3, mRNA sequence | 6.33 | UP | 6.92E-05 |
| BC060805 | Homo sapiens hypothetical protein FLJ12788, mRNA (cDNA clone IMAGE:5266931), partial cds | 6.31 | UP | 2.93E-05 |
| AI359782 | qy41h10.x1 NCI_CGAP_Brn23 Homo sapiens cDNA clone IMAGE:2014627 3, mRNA sequence | 6.31 | UP | 3.23E-04 |
| CA426602 | UI-H-FE1-bef-f-11-0-UI.s1 NCI_CGAP_FE1 Homo sapiens cDNA clone UI-H-FE1-bef-f-11-0-UI 3, mRNA sequence | 6.29 | UP | 2.14E-04 |
| AK091686 | Homo sapiens cDNA FLJ34367 fis, clone FEBRA2016621 | 6.27 | UP | 1.93E-03 |
| AK091336 | Homo sapiens cDNA FLJ34017 fis, clone FCBBF2002626 | 6.26 | UP | 5.49E-05 |
| NM_015068 | Homo sapiens paternally expressed 10 (PEG10), mRNA | 6.26 | UP | 4.53E-05 |
| R79128 | yi86c12.r1 Soares placenta Nb2HP Homo sapiens cDNA clone IMAGE:146134 5, mRNA sequence | 6.23 | UP | 2.04E-04 |
| H08785 | yI94f04.s1 Soares infant brain 1NIB Homo sapiens cDNA clone IMAGE:45882 3, mRNA sequence | 6.22 | UP | 1.66E-03 |
| AI953708 | wq47d09.x1 NCI_CGAP_GC6 Homo sapiens cDNA clone IMAGE:2474417 3, mRNA sequence | 6.21 | UP | 4.58E-03 |
| NM_015236 | Homo sapiens latrophilin 3 (LPHN3), mRNA | 6.2 | UP | 5.35E-04 |
| AL833609 | Homo sapiens mRNA; cDNA DKFZp686O1267 (from clone DKFZp686O1267) | 6.19 | UP | 7.53E-04 |
| AK127421 | Homo sapiens cDNA FLJ45513 fis, clone BRTHA2021450 | 6.19 | UP | 4.68E-05 |
| NM_015085 | Homo sapiens GTPase activating Rap/RanGAP domain-like 4 (GARNL4), mRNA | 6.18 | UP | 6.82E-05 |
| NM_001305 | Homo sapiens claudin 4 (CLDN4), mRNA | 6.17 | UP | 3.48E-05 |
| NM_173078 | Homo sapiens SLIT and NTRK-like family, member 4 (SLITRK4), mRNA | 6.16 | UP | 1.08E-03 |
| BG165745 | 602344592F1 NIH_MGC_89 Homo sapiens cDNA clone IMAGE:4454470 5, mRNA sequence | 6.16 | UP | 1.20E-04 |
| NM_006074 | Homo sapiens tripartite motif-containing 22 (TRIM22), mRNA | 6.15 | UP | 1.15E-03 |
| NM_005928 | Homo sapiens milk fat globule-EGF factor 8 protein (MFGE8), mRNA | 6.15 | UP | 1.63E-02 |
| BF939703 | nac80e07.x1 NCI_CGAP_Brn23 Homo sapiens cDNA clone IMAGE:3440725 3 similar to contains MER30.t3 MER30 repetitive element ;, mRNA sequence | 6.12 | UP | 6.35E-05 |
| BX115301 | BX115301 NCI_CGAP_Kid5 Homo sapiens cDNA clone IMAGp998J093989 ; IMAGE:1574120, mRNA sequence | 6.11 | UP | 8.26E-05 |
| NM_018849 | Homo sapiens ATP-binding cassette, subfamily B (MDR/TAP), member 4 (ABCB4), transcript variant B, mRNA | 6.09 | UP | 3.23E-05 |
| NM_015478 | Homo sapiens I(3)mbt-like (Drosophila) (L3MBTL), transcript variant I, mRNA | 6.09 | UP | 2.43E-05 |
| NM_015669 | Homo sapiens protocadherin beta 5 (PCDHB5), mRNA | 6.08 | UP | 7.52E-05 |
| NM_147189 | Homo sapiens hypothetical protein MGC39325 (MGC39325), mRNA | 6.06 | UP | 3.18E-04 |
| BQ018133 | UI-H-DP0-avv-I-18-0-UI.s1 NCI_CGAP_Fs1 Homo sapiens cDNA clone IMAGE:5884961 3, mRNA sequence | 6.06 | UP | 1.78E-05 |
| N34295 | yy51e10.s1 Soares_multiple_sclerosis_2NbHMSP Homo sapiens cDNA clone IMAGE:277098 3, mRNA sequence | 6.04 | UP | 8.29E-05 |
| NM_006472 | Homo sapiens thioredoxin interacting protein (TXNIP), mRNA | 6.03 | UP | 6.87E-05 |
| NM_012472 | Homo sapiens leucine rich repeat containing 6 (LRRC6), mRNA | 6.02 | UP | 2.66E-05 |
| AK123319 | Homo sapiens cDNA FLJ41325 fis, clone BRAMY2046871 | 6 | UP | 2.55E-04 |
| AK123807 | Homo sapiens cDNA FLJ41813 fis, clone NT2RI2011450 | 5.98 | UP | 7.92E-05 |
| NM_017786 | Homo sapiens hypothetical protein FLJ20366 (FLJ20366), mRNA | 5.98 | UP | 1.85E-03 |
| NM_000212 | Homo sapiens integrin, beta 3 (platelet glycoprotein IIIa, antigen CD61) (ITGB3), mRNA | 5.97 | UP | 2.73E-05 |
| NM_000817 | Homo sapiens glutamate decarboxylase 1 (brain, 67kDa) (GAD1), transcript variant GAD67, mRNA | 5.96 | UP | 9.94E-03 |
| AK123617 | Homo sapiens cDNA FLJ41623 fis, clone CTONG3009227 | 5.92 | UP | 1.38E-03 |
| BI493986 | df106g12.y1 Morton Fetal Cochlea Homo sapiens cDNA clone IMAGE:2538815 5, mRNA sequence | 5.91 | UP | 3.68E-04 |
| NM_005264 | Homo sapiens GDNF family receptor alpha 1 (GFRA1), transcript variant 1, mRNA | 5.91 | UP | 4.09E-05 |
| CN478597 | UI-CF-FN0-aeo-g-21-0-UI.s1 UI-CF-FN0 Homo sapiens cDNA clone UI-CF-FN0-aeo-g-21-0-UI 3, mRNA sequence | 5.88 | UP | 1.13E-03 |
| BQ021695 | UI-H-DH1-axi-f-22-0-UI.s1 NCI_CGAP_DH1 Homo sapiens cDNA clone IMAGE:5829141 3, m RNA sequence | 5.88 | UP | 1.72E-04 |
| AL137698 | Homo sapiens mRNA; cDNA DKFZp434C1915 (from clone DKFZp434C1915); partial cds | 5.88 | UP | 2.03E-05 |
| NM_014905 | Homo sapiens glutaminase (GLS), mRNA | 5.88 | UP | 1.24E-04 |
| AA993234 | ot60a08.s1 Soares_testis_NHT Homo sapiens cDNA clone IMAGE:1621142 3, mRNA sequence | 5.87 | UP | 2.88E-05 |
| N38890 | yy81f12.s1 Soares_multiple_sclerosis_2NbHMSP Homo sapiens cDNA clone IMAGE:279983 3 similar to contains OFR.t3 OFR repetitive element ;, mRNA sequence | 5.87 | UP | 1.25E-04 |
| NM_197955 | Homo sapiens normal mucosa of esophagus specific 1 (NMES1), transcript variant 1, mRNA | 5.85 | UP | 1.85E-04 |
| NM_014399 | Homo sapiens transmembrane 4 superfamily member 13 (TM4SF13), mRNA | 5.84 | UP | 2.18E-05 |
| NM_024704 | Homo sapiens chromosome 20 open reading frame 23 (C20orf23), mRNA | 5.82 | UP | 1.24E-04 |
| NM_002276 | Homo sapiens keratin 19 (KRT19), mRNA | 5.8 | UP | 7.06E-03 |
| CD248442 | AGENCOURT_14095939 NIH_MGC_172 Homo sapiens cDNA 5, mRNA sequence | 5.79 | UP | 2.16E-04 |
| AK024850 | Homo sapiens cDNA: FLJ21197 fis, clone COL00201 | 5.79 | UP | 1.45E-04 |
| H83499 | ys91f12.r1 Soares retina N2b5HR Homo sapiens cDNA clone IMAGE:222191 5, mRNA sequence | 5.77 | UP | 1.37E-02 |
| NM_153377 | Homo sapiens leucine-rich repeats and immunoglobulin-like domains 3 (LRIG3), mRNA | 5.77 | UP | 2.43E-05 |
| NM_153267 | Homo sapiens MAM domain containing 2 (MAMDC2), mRNA | 5.76 | UP | 2.31 E-04 |
| NM_144595 | Homo sapiens hypothetical protein FLJ30046 (FLJ30046), mRNA | 5.76 | UP | 1.38E-03 |
| BF509155 | UI-H-BI4-aov-b-05-0-UI.s1 NCI_CGAP_Sub8 Homo sapiens cDNA clone IMAGE:3086145 3, mRNA sequence | 5.75 | UP | 6.59E-05 |
| NM_002735 | Homo sapiens protein kinase, cAMP-dependent, regulatory, type I, beta (PRKAR1 B), mRNA | 5.73 | UP | 2.73E-05 |
| NM_025044 | Homo sapiens bicaudal C homolog 1 (Drosophila) (BICC1), mRNA | 5.72 | UP | 5.81E-05 |
| BM969191 | UI-CF-EN0-acp-e-22-0-UI.s1 UI-CF-EN0 Homo sapiens cDNA clone UI-CF-EN0-acp-e-22-0-UI 3, mRNA sequence | 5.69 | UP | 1.67E-03 |
| NM_000203 | Homo sapiens iduronidase, alpha-L- (IDUA), mRNA | 5.68 | UP | 1.10E-03 |
| AI693580 | wd12d01.x1 NCI_CGAP_Co3 Homo sapiens cDNA clone IMAGE:2327905 3, mRNA sequence | 5.67 | UP | 9.23E-04 |
| NM_006169 | Homo sapiens nicotinamide N-methyltransferase (NNMT), mRNA | 5.66 | UP | 2.18E-05 |
| H46176 | yo14a11.s1 Soares adult brain N2b5HB55Y Homo sapiens cDNA clone IMAGE:177884 3, mRNA sequence | 5.65 | UP | 1.88E-05 |
| NM_024306 | Homo sapiens fatty acid 2-hydroxylase (FA2H), mRNA | 5.63 | UP | 4.19E-03 |
| BX110683 | BX110683 NCI_CGAP_GC6 Homo sapiens cDNA clone IMAGp998E175727 ; IMAGE:2310904, mRNA sequence | 5.62 | UP | 7.42E-04 |
| NM_030765 | Homo sapiens UDP-GlcNAc:betaGal beta-1,3-N-acetylglucosaminyltransferase 4 (B3GNT4), mRNA | 5.58 | UP | 1.80E-03 |
| NM_003929 | Homo sapiens RAB7, member RAS oncogene family-like 1 (RAB7L1), mRNA | 5.55 | UP | 2.14E-05 |
| AI220066 | qg84d01.x1 Soares_NFL_T_GBC_S1 Homo sapiens cDNA clone IMAGE:1841857 3, mRNA sequence | 5.54 | UP | 2.13E-05 |
| AA664452 | ae94d02.s1 Human bone marrow stromal cells Homo sapiens cDNA clone IMAGE:1026723 3 similar to contains element LTR4 repetitive element ;, mRNA sequence | 5.54 | UP | 4.13E-05 |
| NM_007237 | Homo sapiens SP140 nuclear body protein (SP140), transcript variant 1, mRNA | 5.53 | UP | 2.41E-04 |
| NM_173662 | Homo sapiens hypothetical protein LOC285533 (LOC285533), mRNA | 5.52 | UP | 3.69E-03 |
| NM_003020 | Homo sapiens secretory granule, neuroendocrine protein 1 (7B2 protein) (SGNE1), mRNA | 5.52 | UP | 2.08E-04 |
| NM_206808 | Homo sapiens citrate lyase beta like (CLYBL), transcript variant 2, mRNA | 5.52 | UP | 1.87E-04 |
| NM_004867 | Homo sapiens integral membrane protein 2A (ITM2A), mRNA | 5.51 | UP | 2.13E-05 |
| NM_006333 | Homo sapiens nuclear DNA-binding protein (C1D), transcript variant 1, mRNA | 5.51 | UP | 7.84E-04 |
| NM_014251 | Homo sapiens solute carrier family 25, member 13 (citrin) (SLC25A13), mRNA | 5.5 | UP | 5.96E-04 |
| NM_006863 | Homo sapiens leukocyte immunoglobulin-like receptor, subfamily A (with TM domain), member 1 (LILRA1), mRNA | 5.49 | UP | 6.51E-05 |
| AK057166 | Homo sapiens cDNA FLJ32604 fis, clone STOMA1000133 | 5.49 | UP | 1.85E-03 |
| NM_000076 | Homo sapiens cyclin-dependent kinase inhibitor 1C (p57, Kip2) (CDKN1C), mRNA | 5.48 | UP | 4.65E-05 |
| BC040701 | Homo sapiens cDNA clone IMAGE:5736259, partial cds | 5.47 | UP | 1.48E-04 |
| NM_152765 | Homo sapiens hypothetical protein MGC33510 (MGC33510), mRNA | 5.46 | UP | 4.76E-05 |
| NM_019034 | Homo sapiens ras homolog gene family, member F (in filopodia) (RHOF), mRNA | 5.46 | UP | 2.64E-05 |
| S81734 | tissue transglutaminase homologue {alternatively spliced} [human, erythroleukemia cell line HEL GM06141A, mRNA, 2362 nt] | 5.46 | UP | 3.92E-05 |
| BM670793 | UI-E-DX1-agv-p-03-0-UI.s1 UI-E-DX1 Homo sapiens cDNA clone UI-E-DX1-agv-p-03-0-UI 3, mRNA sequence | 5.46 | UP | 3.77E-05 |
| NM_002829 | Homo sapiens protein tyrosine phosphatase, non-receptor type 3 (PTPN3), mRNA | 5.45 | UP | 4.79E-05 |
| BM727151 | UI-E-EJ0-aij-f-05-0-UI.r1 UI-E-EJ0 Homo sapiens cDNA clone UI-E-EJ0-aij-f-05-0-UI 5, mRNA sequence | 5.45 | UP | 4.13E-05 |
| BF966833 | 602286668T1 NIH_MGC_95 Homo sapiens cDNA clone IMAGE:4375360 3, mRNA sequence | 5.45 | UP | 2.83E-05 |
| NM_004904 | Homo sapiens cAMP responsive element binding protein 5 (CREB5), mRNA | 5.44 | UP | 1.09E-04 |
| AA635788 | nr32h01.s1 NCI_CGAP_Pr22 Homo sapiens cDNA clone IMAGE:1169713 3 similar to contains Alu repetitive element;, mRNA sequence | 5.43 | UP | 2.02E-03 |
| BM701989 | UI-E-CQ1-aex-j-06-0-UI.r1 UI-E-CQ1 Homo sapiens cDNA clone UI-E-CQ1-aex-j-06-0-UI 5, mRNA sequence | 5.42 | UP | 7.55E-04 |
| NM_020665 | Homo sapiens transmembrane protein 27 (TMEM27), mRNA | 5.4 | UP | 5.46E-05 |
| NM_019034 | Homo sapiens ras homolog gene family, member F (in filopodia) (RHOF), mRNA | 5.39 | UP | 5.72E-05 |
| NM_017912 | Homo sapiens hect domain and RLD 6 (HERC6), mRNA | 5.38 | UP | 5.85E-05 |
| NM_152433 | Homo sapiens kelch repeat and BTB (POZ) domain containing 3 (KBTBD3), transcript variant 1, mRNA | 5.37 | UP | 4.29E-04 |
| CA314843 | UI-CF-FN0-afi-a-06-0-UI.s1 UI-CF-FN0 Homo sapiens cDNA clone UI-CF-FN0-afi-a-06-0-UI 3, mRNA sequence | 5.37 | UP | 2.05E-04 |
| NM_006058 | Homo sapiens TNFAIP3 interacting protein 1 (TNIP1), mRNA | 5.36 | UP | 2.25E-03 |
| BU191317 | AGENCOURT_8074912 NIH_MGC_110 Homo sapiens cDNA clone IMAGE:6086274 5, mRNA sequence | 5.36 | UP | 4.45E-02 |
| AL049437 | Homo sapiens mRNA; cDNA DKFZp586E1120 (from clone DKFZp586E1120) | 5.34 | UP | 7.78E-04 |
| NM_030952 | Homo sapiens likely ortholog of rat SNF1/AMP-activated protein kinase (SNARK), mRNA | 5.31 | UP | 4.21E-05 |
| NM_004170 | Homo sapiens solute carrier family 1 (neuronal/epithelial high affinity glutamate transporter, system Xag), member 1 (SLC1A1), mRNA | 5.31 | UP | 1.15E-04 |
| AW269776 | xv45b09.x1 Soares_NFL_T_GBC_S1 Homo sapiens cDNA clone IMAGE:2816057 3, mRNA sequence | 5.3 | UP | 1.97E-04 |
| BM977716 | UI-CF-EN1-aef-b-21-0-UI.s1 UI-CF-EN1 Homo sapiens cDNA clone UI-CF-EN1-aef-b-21-0-UI 3, mRNA sequence | 5.3 | UP | 4.01 E-03 |
| AK023658 | Homo sapiens cDNA FLJ13596 fis, clone PLACE1009637 | 5.29 | UP | 6.83E-04 |
| AF196185 | Homo sapiens atypical PKC isotype-specific interacting protein long variant mRNA, complete cds | 5.28 | UP | 4.57E-05 |
| AL045014 | DKFZp434F134_s1 434 (synonym: htes3) Homo sapiens cDNA clone DKFZp434F134 3, mRNA sequence | 5.27 | UP | 2.67E-05 |
| NM_004496 | Homo sapiens forkhead box A1 (FOXA1), mRNA | 5.27 | UP | 2.88E-03 |
| NM_033132 | Homo sapiens Zic family member 5 (odd-paired homolog, Drosophila) (ZIC5), mRNA | 5.26 | UP | 2.09E-05 |
| NM_198182 | Homo sapiens transcription factor CP2-like 2 (TFCP2L2), transcript variant 2, mRNA | 5.26 | UP | 1.35E-02 |
| NM_001657 | Homo sapiens amphiregulin (schwannoma-derived growth factor) (AREG), mRNA | 5.26 | UP | 3.99E-05 |
| R92346 | yq06b10.s1 Soares fetal liver spleen 1 NFLS Homo sapiens cDNA clone IMAGE:196123 3, mRNA sequence | 5.25 | UP | 1.16E-04 |
| BX537539 | Homo sapiens mRNA; cDNA DKFZp686A1130 (from clone DKFZp686A1130) | 5.25 | UP | 4.80E-04 |
| AB041269 | Homo sapiens mRNA for keratin 19, partial cds, isolate:K19-141 | 5.23 | UP | 1.15E-02 |
| BX090717 | BX090717 NCI_CGAP_Kid5 Homo sapiens cDNA clone IMAGp9980154699 ; IMAGE:1916390, mRNA sequence | 5.2 | UP | 9.02E-04 |
| AK025743 | Homo sapiens cDNA: FLJ22090 fis, clone HEP16084 | 5.2 | UP | 1.04E-04 |
| NM_002413 | Homo sapiens microsomal glutathione S-transferase 2 (MGST2), mRNA | 5.2 | UP | 3.34E-05 |
| AA780946 | ag99c12.s1 Gessler Wilms tumor Homo sapiens cDNA clone IMAGE:1155286 3, mRNA sequence | 5.2 | UP | 2.40E-04 |
| NM_032488 | Homo sapiens cornifelin (CNFN), mRNA | 5.2 | UP | 1.78E-05 |
| NM_024997 | Homo sapiens activating transcription factor 7 interacting protein 2 (ATF7IP2), mRNA | 5.19 | UP | 2.40E-03 |
| NM_002222 | Homo sapiens inositol 1,4,5-triphosphate receptor, type 1 (ITPR1), mRNA | 5.19 | UP | 1.41E-04 |
| NM_000784 | Homo sapiens cytochrome P450, family 27, subfamily A, polypeptide 1 (CYP27A1), nuclear gene encoding mitochondrial protein, mRNA | 5.17 | UP | 7.39E-03 |
| NM_005760 | Homo sapiens CCAAT/enhancer binding protein zeta (CEBPZ), mRNA | 5.16 | UP | 5.52E-05 |
| BQ011746 | UI-1-BC1p-atk-b-09-0-UI.s1 NCI_CGAP_PI3 Homo sapiens cDNA clone UI-1-BC1p-atk-b-09-0-UI 3, mRNA sequence | 5.16 | UP | 5.86E-04 |
| NM_032728 | Homo sapiens chromosome 9 open reading frame 67 (C9orf67), mRNA | 5.15 | UP | 1.85E-03 |
| NM_005755 | Homo sapiens Epstein-Barr virus induced gene 3 (EBI3), mRNA | 5.15 | UP | 2.33E-04 |
| AK056852 | Homo sapiens cDNA FLJ32290 fis, clone PROST2000463 | 5.15 | UP | 1.32E-03 |
| AK054990 | Homo sapiens cDNA FLJ30428 fis, clone BRACE2008941 | 5.15 | UP | 1.85E-03 |
| D54580 | HUM144G01 B Clontech human fetal brain polyA+ mRNA (#6535) Homo sapiens cDNA clone GEN-144G01 5, mRNA sequence | 5.13 | UP | 2.03E-04 |
| AL137535 | Homo sapiens mRNA; cDNA DKFZp434H2019 (from clone DKFZp434H2019) | 5.13 | UP | 2.45E-02 |
| AL110252 | Homo sapiens mRNA; cDNA DKFZp566A1046 (from clone DKFZp566A1046) | 5.12 | UP | 2.19E-03 |
| AI659523 | tt99d12.x1 NCI_CGAP_Pr28 Homo sapiens cDNA clone IMAGE:2249687 3, mRNA sequence | 5.1 | UP | 1.16E-03 |
| AW137001 | UI-H-BI1-acu-c-05-0-UI.s1 NCI_CGAP_Sub3 Homo sapiens cDNA clone IMAGE:2715632 3, mRNA sequence | 5.1 | UP | 6.90E-05 |
| BM969331 | UI-CF-DU1-aar-g-23-0-UI.s1 UI-CF-DU1 Homo sapiens cDNA clone UI-CF-DU1-aar-g-23-0-UI 3, mRNA sequence | 5.08 | UP | 3.31E-03 |
| M_178823 | Homo sapiens chromosome 6 open reading frame 165 (C6orf165), mRNA | 5.08 | UP | 3.66E-05 |
| NM_018476 | Homo sapiens brain expressed, X-linked 1 (BEX1), mRNA | 5.06 | UP | 3.05E-05 |
| BF107212 | 601824290F1 NIH_MGC_79 Homo sapiens cDNA clone IMAGE:4043879 5, mRNA sequence | 5.06 | UP | 1.75E-04 |
| NM_052890 | Homo sapiens peptidoglycan recognition protein 2 (PGLYRP2), mRNA | 5.05 | UP | 3.95E-05 |
| BX459043 | BX459043 Homo sapiens PLACENTA Homo sapiens cDNA clone CSODE011YN10 3-PRIME, mRNA sequence | 5.03 | UP | 2.88E-05 |
| NM_032857 | Homo sapiens lactamase, beta (LACTB), nuclear gene encoding mitochondrial protein, transcript variant 1, mRNA | 5.02 | UP | 1.76E-05 |
| NM_022746 | Homo sapiens hypothetical protein FLJ22390 (FLJ22390), mRNA | 5.02 | UP | 4.49E-04 |
| AK025909 | Homo sapiens cDNA: FLJ22256 fis, clone HRC02860 | 5.02 | UP | 4.54E-04 |
| NM_002247 | Homo sapiens potassium large conductance calcium-activated channel, subfamily M, alpha member 1 (KCNMA1), mRNA | 5.02 | UP | 1.33E-03 |
| AI434849 | ti13b01.x1 NCI_CGAP_Kid11 Homo sapiens cDNA clone IMAGE:2130313 3 similar to contains MER37.b1 MER37 MER37 repetitive element ;, mRNA sequence | 5.01 | UP | 2.82E-04 |
| AK091933 | Homo sapiens cDNA FLJ34614 fis, clone KIDNE2014268 | 5.01 | UP | 2.18E-05 |
| NM_004331 | Homo sapiens BCL2/adenovirus E1B 19kDa interacting protein 3-like (BNIP3L), mRNA | 5 | UP | 3.53E-05 |
| AL119769 | DKFZp761E1224_r1 761 (synonym: hamy2) Homo sapiens cDNA clone DKFZp761 E1224 5, mRNA sequence | 5 | UP | 1.92E-03 |
| NM_001451 | Homo sapiens forkhead box F1 (FOXF1), mRNA | 465.04 | Down | 2.67E-05 |
| M_144594 | Homo sapiens hypothetical protein FLJ32942 (FLJ32942), mRNA | 379.94 | Down | 1.12E-05 |
| NM_002421 | Homo sapiens matrix metalloproteinase 1 (interstitial collagenase) (MMP1), mRNA | 365.32 | Down | 2.43E-05 |
| AK026784 | Homo sapiens cDNA: FLJ23131 fis, clone LNG08502 | 331.17 | Down | 8.46E-06 |
| NM_016307 | Homo sapiens paired related homeobox 2 (PRRX2), mRNA | 309.99 | Down | 8.39E-06 |
| NM_006350 | Homo sapiens follistatin (FST), transcript variant FST317, mRNA | 232.51 | Down | 8.39E-06 |
| NM_000089 | Homo sapiens collagen, type I, alpha 2 (COL1A2), mRNA | 220.95 | Down | 2.96E-05 |
| NM_007036 | Homo sapiens endothelial cell-specific molecule 1 (ESM1), mRNA | 217.57 | Down | 2.01E-05 |
| AK021543 | Homo sapiens cDNA FLJ11481 fis, clone HEMBA1001803 | 215.51 | Down | 2.31E-05 |
| NM_004460 | Homo sapiens fibroblast activation protein, alpha (FAP), mRNA | 205.38 | Down | 1.15E-05 |
| AK122739 | Homo sapiens cDNA FLJ16260 fis, clone IMR322006947, highly similar to Rattus norvegicus mRNA for dHand protein | 190.76 | Down | 1.15E-05 |
| NM_000474 | Homo sapiens twist homolog 1 (acrocephalosyndactyly 3; Saethre-Chotzen syndrome) (Drosophila) (TWIST1), mRNA | 151.94 | Down | 5.23E-06 |
| NM_152270 | Homo sapiens hypothetical protein FLJ34922 (FLJ34922), mRNA | 144.24 | Down | 1.73E-05 |
| NM_001884 | Homo sapiens hyaluronan and proteoglycan link protein 1 (HAPLN1), mRNA | 134.45 | Down | 2.18E-05 |
| NM_000710 | Homo sapiens bradykinin receptor B1 (BDKRB1), mRNA | 132.74 | Down | 1.18E-05 |
| NM_021637 | Homo sapiens transmembrane protein 35 (TMEM35), mRNA | 126.76 | Down | 8.39E-06 |
| NM_006350 | Homo sapiens follistatin (FST), transcript variant FST317, mRNA | 106.55 | Down | 2.28E-05 |
| NM_000362 | Homo sapiens tissue inhibitor of metalloproteinase 3 (Sorsby fundus dystrophy, pseudoinflammatory) (TIMP3), mRNA | 102.59 | Down | 1.76E-05 |
| NM_002091 | Homo sapiens gastrin-releasing peptide (GRP), mRNA | 101.58 | Down | 2.46E-05 |
| NM_000609 | Homo sapiens chemokine (C-X-C motif) ligand 12 (stromal cell-derived factor 1) (CXCL12), mRNA | 97.42 | Down | 3.48E-05 |
| NM_032638 | Homo sapiens GATA binding protein 2 (GATA2), mRNA | 84.58 | Down | 1.37E-05 |
| NM_198148 | Homo sapiens carboxypeptidase X (M14 family), member 2 (CPXM2), mRNA | 77.44 | Down | 9.50E-05 |
| W38393 | zb15c07.r1 Soares_fetal_lung_NbHL19W Homo sapiens cDNA clone IMAGE:302124 5, mRNA sequence | 77.37 | Down | 1.26E-05 |
| AL831863 | Homo sapiens mRNA; cDNA DKFZp761J2017 (from clone DKFZp761J2017) | 77.09 | Down | 2.65E-05 |
| AB067499 | Homo sapiens mRNA for KIAA1912 protein, partial cds | 76.12 | Down | 3.65E-05 |
| BX089019 | BX089019 Soares_testis_NHT Homo sapiens cDNA clone IMAGp998K243513 ; IMAGE:1391375, mRNA sequence | 72.98 | Down | 1.25E-05 |
| AI962169 | wq45c10.x1 NCI_CGAP_GC6 Homo sapiens cDNA clone IMAGE:2474226 3, mRNA sequence | 65.52 | Down | 4.53E-05 |
| NM_139211 | Homo sapiens homeodomain-only protein (HOP), transcript variant 2, mRNA | 64.01 | Down | 1.72E-05 |
| CD677332 | ho15f06.y1 Human Trabecular meshwork cDNA: hohphq Homo sapiens cDNA clone ho15f06 5, mRNA sequence | 63.91 | Down | 2.35E-05 |
| NM_001442 | Homo sapiens fatty acid binding protein 4, adipocyte (FABP4), mRNA | 63.47 | Down | 1.37E-05 |
| NM_205855 | Homo sapiens HWKM1940 (UNQ1940), mRNA | 58.05 | Down | 1.89E-05 |
| W03013 | za02c04.r1 Soares melanocyte 2NbHM Homo sapiens cDNA clone IMAGE:291366 5 similar to contains THR.t3 THR repetitive element ;, mRNA sequence | 53.58 | Down | 2.67E-06 |
| NM_002852 | Homo sapiens pentaxin-related gene, rapidly induced by IL-1 beta (PTX3), mRNA | 53.13 | Down | 8.15E-04 |
| NM_024633 | Homo sapiens chromosome 14 open reading frame 139 (C14orf139), mRNA | 51.58 | Down | 3.58E-05 |
| AK021543 | Homo sapiens cDNA FLJ11481 fis, clone HEMBA1001803 | 51.28 | Down | 1.09E-04 |
| NM 002593 | Homo sapiens procollagen C-endopeptidase enhancer (PCOLCE), mRNA | 48.32 | Down | 2.03E-05 |
| AI422199 | tf58d04.x1 NCI_CGAP_Brn23 Homo sapiens cDNA clone IMAGE:2103463 3, mRNA sequence | 48.23 | Down | 4.98E-05 |
| NM_014459 | Homo sapiens protocadherin 17 (PCDH17), mRNA | 45.53 | Down | 2.11E-05 |
| NM_016588 | Homo sapiens neuritin 1 (NRN1), mRNA | 44.52 | Down | 1.78E-05 |
| NM_012242 | Homo sapiens dickkopf homolog 1 (Xenopus laevis) (DKK1), mRNA | 43.26 | Down | 7.16E-06 |
| NM_000685 | Homo sapiens angiotensin II receptor, type 1 (AGTR1), transcript variant 1, mRNA | 42.51 | Down | 2.57E-04 |
| AK056725 | Homo sapiens cDNA FLJ32163 fis, clone PLACE6000371 | 42.21 | Down | 2.67E-06 |
| AI124557 | am58g02.x1 Johnston frontal cortex Homo sapiens cDNA clone IMAGE:1539794 3, mRNA sequence | 41.42 | Down | 8.25E-03 |
| NM_020404 | Homo sapiens CD164 sialomucin-like 1 (CD164L1), mRNA | 39.82 | Down | 2.32E-05 |
| NM_015170 | Homo sapiens sulfatase 1 (SULF1), mRNA | 38.91 | Down | 2.36E-05 |
| CA843592 | ir49c12.x1 HR85 islet Homo sapiens cDNA clone IMAGE:6548544 3, mRNA sequence | 37.67 | Down | 1.25E-05 |
| AY335938 | Homo sapiens homeodomain protein IRXA1 (IRX1) mRNA, complete cds | 36.92 | Down | 3.15E-05 |
| NM_002961 | Homo sapiens S100 calcium binding protein A4 (calcium protein, calvasculin, metastasin, murine placental homolog) (S100A4), transcript variant 1, mRNA | 35.31 | Down | 6.57E-05 |
| AK091731 | Homo sapiens cDNA FLJ34412 fis, clone HEART2002432 | 34.47 | Down | 2.83E-05 |
| NM_006329 | Homo sapiens fibulin 5 (FBLN5), mRNA | 34.03 | Down | 4.55E-05 |
| BQ020357 | UI-H-ED0-axk-p-07-0-UI.s1 NCI_CGAP_ED0 Homo sapiens cDNA clone IMAGE:5830134 3, mRNA sequence | 33.2 | Down | 1.82E-05 |
| NM_018013 | Homo sapiens hypothetical protein FLJ10159 (FLJ10159), mRNA | 32.86 | Down | 1.15E-05 |
| NM_002402 | Homo sapiens mesoderm specific transcript homolog (mouse) (MEST), transcript variant 1, mRNA | 32.83 | Down | 2.14E-05 |
| BQ001571 | UI-H-DH1-awr-i-18-0-UI.s1 NCi_CGAP_DH1 Homo sapiens cDNA clone IMAGE:5893337 3, mRNA sequence | 32.34 | Down | 8.96E-06 |
| NM_032777 | Homo sapiens G protein-coupled receptor 124 (GPR124), mRNA | 31.96 | Down | 1.18E-04 |
| AK124396 | Homo sapiens cDNA FLJ42405 fis, clone ASTRO3000474 | 31.36 | Down | 2.24E-05 |
| NM_002448 | Homo sapiens msh homeo box homolog 1 (Drosophila) (MSX1), MRNA, | 31.23 | Down | 2.03E-05 |
| NM_000090 | Homo sapiens collagen, type III, alpha 1 (Ehlers-Danlos syndrome type IV, autosomal dominant) (COL3A1), mRNA | 29.77 | Down | 3.23E-04 |
| NM_001147 | Homo sapiens angiopoietin 2 (ANGPT2), mRNA | 29.68 | Down | 2.51E-05 |
| NM_001146 | Homo sapiens angiopoietin 1 (ANGPT1), transcript variant 1, mRNA | 29.26 | Down | 6.83E-05 |
| NM_000576 | Homo sapiens interleukin 1, beta (IL1B), mRNA | 29.06 | Down | 1.15E-05 |
| AF052115 | Homo sapiens clone 23688 mRNA sequence | 26.15 | Down | 1.15E-05 |
| NM_006439 | Homo sapiens mab-21-like 2 (C. elegans) (MAB21L2), mRNA | 25.51 | Down | 1.20E-04 |
| NM_016192 | Homo sapiens transmembrane protein with EGF-like and two follistatin-like domains 2 (TMEFF2), mRNA | 25.46 | Down | 2.55E-05 |
| NM_002575 | Homo sapiens serine (or cysteine) proteinase inhibitor, clade B (ovalbumin), member 2 (SERPINB2), mRNA | 24.67 | Down | 5.40E-05 |
| NM_007361 | Homo sapiens nidogen 2 (osteonidogen) (NID2), mRNA | 23.56 | Down | 2.64E-05 |
| NM 152399 | Homo sapiens hypothetical protein FLJ30834 (FLJ30834), mRNA | 23.42 | Down | 6.51E-05 |
| NM_203370 | Homo sapiens similar to RIKEN cDNA 6530418L21 (LOC389119), mRNA | 22.99 | Down | 1.43E-05 |
| NM_00100229 5 | Homo sapiens GATA binding protein 3 (GATA3), transcript variant 1, mRNA | 22.51 | Down | 1.41E-04 |
| AK093256 | Homo sapiens cDNA FLJ35937 fis, clone TESTI2011480 | 22.26 | Down | 3.04E-05 |
| U83115 | Human non-lens beta gamma-crystallin like protein (AIM1) mRNA, partial cds | 22.18 | Down | 3.77E-05 |
| NM_006475 | Homo sapiens periostin, osteoblast specific factor (POSTN), mRNA | 21.99 | Down | 7.38E-05 |
| NM_014178 | Homo sapiens syntaxin binding protein 6 (amisyn) (STXBP6), mRNA | 21.69 | Down | 3.36E-05 |
| NM_005127 | Homo sapiens C-type (calcium dependent, carbohydrate-recognition domain) lectin, superfamily member 2 (activation-induced) (CLECSF2), mRNA | 21.46 | Down | 3.71E-05 |
| NM_022475 | Homo sapiens hedgehog interacting protein (HHIP), mRNA | 21.42 | Down | 4.57E-05 |
| BX112628 | BX112628 Soares_fetal_lung_NbHL19W Homo sapiens cDNA clone IMAGp998A09669 ; IMAGE:299024, mRNA sequence | 20.99 | Down | 4.47E-05 |
| NM_000810 | Homo sapiens gamma-aminobutyric acid (GABA) A receptor, alpha 5 (GABRA5), mRNA | 20.84 | Down | 5.50E-05 |
| BQ934941 | AGENCOURT_8810373 NIH_MGC_101 Homo sapiens cDNA clone IMAGE:6429485 5, mRNA sequence | 20.45 | Down | 2.55E-05 |
| BC071787 | Homo sapiens cDNA clone IMAGE:4610527, partial cds | 20.44 | Down | 1.25E-05 |
| NM_033292 | Homo sapiens caspase 1, apoptosis-related cysteine protease (interleukin 1, beta, convertase) (CASP1), transcript variant alpha, mRNA | 19.88 | Down | 6.18E-05 |
| NM_020809 | Homo sapiens Rho GTPase activating protein 20 (ARHGAP20), mRNA | 19.54 | Down | 5.26E-05 |
| BE866150 | 601679068F1 NIH_MGC_53 Homo sapiens cDNA clone IMAGE:3961768 5, mRNA sequence | 19.3 | Down | 2.38E-05 |
| AW021686 | df26h11.y1 Morton Fetal Cochlea Homo sapiens cDNA clone IMAGE:2484717 5, mRNA sequence | 18.86 | Down | 2.59E-05 |
| BX109483 | BX109483 NCI_CGAP_Ov23 Homo sapiens cDNA clone IMAGp998C165481 ; IMAGE:2216391, mRNA sequence | 18.74 | Down | 9.84E-05 |
| NM_001463 | Homo sapiens frizzled-related protein (FRZB), mRNA | 18.63 | Down | 2.05E-04 |
| AK128325 | Homo sapiens cDNA FLJ46467 fis, clone THYMU3022668 | 18.11 | Down | 1.78E-05 |
| AK096661 | Homo sapiens cDNA FLJ39342 fis, clone OCBBF2018873 | 17.95 | Down | 5.14E-05 |
| NM_002531 | Homo sapiens neurotensin receptor 1 (high affinity) (NTSR1), MRNA, | 17.64 | Down | 5.02E-05 |
| NM_031908 | Homo sapiens C1q and tumor necrosis factor related protein 2 (C1QTNF2), mRNA | 17.61 | Down | 1.23E-04 |
| AI085016 | ow88e06.s1 Soares_fetal_liver_spleen_1NFLS_S1 Homo sapiens cDNA clone IMAGE:1653922 3, mRNA sequence | 17.36 | Down | 4.39E-05 |
| AK095791 | Homo sapiens cDNA FLJ38472 fis, clone FEBRA2022148 | 17.31 | Down | 2.76E-04 |
| NM_020927 | Homo sapiens KIAA1576 protein (KIAA1576), mRNA | 17 | Down | 2.13E-05 |
| NM_024600 | Homo sapiens chromosome 16 open reading frame 30 (C16orf30), mRNA | 16.96 | Down | 6.00E-04 |
| NM_012449 | Homo sapiens six transmembrane epithelial antigen of the prostate (STEAP), mRNA | 16.92 | Down | 5.06E-05 |
| NM_004787 | Homo sapiens slit homolog 2 (Drosophila) (SLIT2), mRNA | 16.69 | Down | 1.78E-05 |
| N95448 | zb81e11.s1 Soares_senescent_fibroblasts_NbHSF Homo sapiens cDNA clone IMAGE:310028 3, mRNA sequence | 16.55 | Down | 1.72E-05 |
| NM_004657 | Homo sapiens serum deprivation response (phosphatidylserine binding protein) (SDPR), mRNA | 16.4 | Down | 1.60E-05 |
| BC046364 | Homo sapiens flavoprotein oxidoreductase MICAL3, mRNA (cDNA clone IMAGE:5737121), with apparent retained intron | 16.35 | Down | 1.15E-05 |
| NM_053044 | Homo sapiens serine protease HTRA3 (HTRA3), mRNA | 16.29 | Down | 3.77E-05 |
| BQ011545 | UI-1-BC1p-asi-a-02-0-UI.s1 NCI_CGAP_PI3 Homo sapiens cDNA clone UI-1-BC1p-asi-a-02-0-UI 3, mRNA sequence | 16.22 | Down | 5.21E-04 |
| BC017939 | Homo sapiens, clone IMAGE:4275711, mRNA, partial cds | 15.98 | Down | 5.83E-05 |
| NM_007289 | Homo sapiens membrane metallo-endopeptidase (neutral endopeptidase, enkephalinase, CALLA, CD10) (MME), transcript variant 2b, mRNA | 15.83 | Down | 3.04E-05 |
| CA437861 | UI-H-DHO-aur-k-12-0-UI.s1 NCI_CGAP_DHO Homo sapiens cDNA clone UI-H-DHO-aur-k-12-0-UI 3, mRNA sequence | 15.77 | Down | 2.56E-04 |
| NM_006182 | Homo sapiens discoidin domain receptor family, member 2 (DDR2), mRNA | 15.71 | Down | 4.79E-05 |
| NM_145239 | Homo sapiens similar to lymphocyte antigen 6 complex, locus G5B; G5b protein; open reading frame 31 (LOC112476), mRNA | 15.64 | Down | 3.37E-05 |
| H15096 | ym29e11.r1 Soares infant brain 1 NIB Homo sapiens cDNA clone IMAGE:49250 5, mRNA sequence | 15.58 | Down | 2.20E-04 |
| NM_002851 | Homo sapiens protein tyrosine phosphatase, receptor-type, Z polypeptide 1 (PTPRZ1), mRNA | 15.57 | Drown | 1.90E-04 |
| NM_014217 | Homo sapiens potassium channel, subfamily K, member 2 (KCNK2), mRNA | 15.13 | Down | 1.31E-04 |
| NM_000963 | Homo sapiens prostaglandin-endoperoxide synthase 2 (prostaglandin G/H synthase and cyclooxygenase) (PTGS2), mRNA | 14.94 | Down | 1.88E-05 |
| BX115659 | BX115659 Soares_total_fetus_Nb2HF8_9w Homo sapiens cDNA clone IMAGp998C204119 ; IMAGE:1623883, mRNA sequence | 14.81 | Down | 2.66E-04 |
| NM_002729 | Homo sapiens hematopoietically expressed homeobox (HHEX), mRNA | 14.78 | Down | 1.09E-03 |
| A1082087 | oz52h09.x1 Soares_senescent_fibroblasts_NbHSF Homo sapiens cDNA clone IMAGE:1679009 3, mRNA sequence | 14.73 | Drown | 3.66E-05 |
| NM_018431 | Homo sapiens docking protein 5 (DOK5), transcript variant 1, mRNA | 14.67 | Down | 8.15E-04 |
| NM_004791 | Homo sapiens integrin, beta-like 1 (with EGF-like repeat domains) (ITGBL1), mRNA | 14.42 | Down | 2.18E-05 |
| AK021531 | Homo sapiens cDNA FLJ11469 fis, clone HEMBA1001658 | 14.36 | Down | 5.21E-04 |
| NM_057179 | Homo sapiens twist homolog 2 (Drosophila) (TWIST2), mRNA | 14.27 | Down | 1.25E-05 |
| NM_003619 | Homo sapiens protease, serine, 12 (neurotrypsin, motopsin) (PRSS12), mRNA | 14.1 | Down | 1.78E-05 |
| NM_002518 | Homo sapiens neuronal PAS domain protein 2 (NPAS2), mRNA | 14.05 | Down | 5.49E-05 |
| NM_000396 | Homo sapiens cathepsin K (pycnodysostosis) (CTSK), mRNA | 13.81 | Down | 2.31 E-04 |
| NM_016206 | Homo sapiens colon carcinoma related protein (FLJ38507), mRNA | 13.76 | Down | 1.81 E-04 |
| NM_021643 | Homo sapiens tribbles homolog 2 (Drosophila) (TRIB2), mRNA | 13.62 | Down | 8.39E-06 |
| NM_000955 | Homo sapiens prostaglandin E receptor 1 (subtype EP1 42kDa (PTGER1), MRNA, | 13.52 | Down | 1.70E-05 |
| NM_058187 | Homo sapiens chromosome 21 open reading frame 63 (C21orf63), mRNA | 13.44 | Down | 1.53E-04 |
| CN479391 | UI-H-DF1-aug-f-02-0-UI.s9 NCI_CGAP_DF1 Homo sapiens cDNA clone UI-H-DF1-aug-f-02-0-UI 3, mRNA sequence | 13.26 | Down | 8.65E-05 |
| NM_006209 | Homo sapiens ectonucleotide pyrophosphatase/phosphodiesterase 2 (autotaxin) (ENPP2), mRNA | 13.21 | Down | 3.28E-04 |
| NM_005584 | Homo sapiens mab-21-like 1 (C. elegans) (MAB21L1), mRNA | 12.73 | Down | 1.01E-04 |
| BM468332 | AGENCOURT 6432296 NIH_MGC_71 Homo sapiens cDNA clone IMAGE:5535773 5, mRNA sequence | 12.64 | Down | 2.13E-05 |
| NM_020353 | Homo sapiens phospholipid scramblase 4 (PLSCR4), mRNA | 12.39 | Down | 2.08E-04 |
| NM_031302 | Homo sapiens gycosyltransferase (LOC83468), mRNA | 12.39 | Down | 1.88E-03 |
| NM_005308 | Homo sapiens G protein-coupled receptor kinase 5 (GRK5), mRNA | 12.28 | Down | 1.18E-04 |
| NM_032883 | Homo sapiens chromosome 20 open reading frame 100 (C20orf100), mRNA | 12.17 | Drown | 1.78E-05 |
| NM_000110 | Homo sapiens dihydropyrimidine dehydrogenase (DPYD), mRNA | 12.16 | Down | 2.75E-05 |
| BU536871 | AGENCOURT_10224340 NIH-MGC-141 Homo sapiens cDNA clone IMAGE:6565454 5, mRNA sequence | 12.05 | Down | 4.21E-05 |
| NM_016270 | Homo sapiens Kruppel-like factor 2 (lung) (KLF2), mRNA | 11.98 | Down | 1.94E-03 |
| NM_005110 | Homo sapiens glutamine-fructose-6-phosphate transaminase 2 (GFPT2), mRNA | 11.92 | Down | 2.93E-05 |
| AK130711 | Homo sapiens cDNA FLJ27201 fis, clone SYN03133 | 11.81 | Down | 5.02E-05 |
| BM991890 | UI-H-DF1-auk-h-02-0-UI.s1 NCI_CGAP_DF1 Homo sapiens cDNA clone IMAGE:5870641 3, mRNA sequence | 11.78 | Down | 2.03E-05 |
| NM_000304 | Homo sapiens peripheral myelin protein 22 (PMP22), transcript variant 1, mRNA | 11.77 | Down | 1.04E-03 |
| BM926469 | AGENCOURT_6644776 NIH_MGC_122 Homo sapiens cDNA clone IMAGE:5766855 5, mRNA sequence | 11.53 | Down | 7.16E-06 |
| NM_001424 | Homo sapiens epithelial membrane protein 2 (EMP2), mRNA | 11.39 | Down | 5.12E-04 |
| AK093762 | Homo sapiens cDNA FLJ36443 fis, clone THYMU2012891 | 11.18 | Down | 1.34E-04 |
| NM_006288 | Homo sapiens Thy-1 cell surface antigen (THY1), mRNA | 11.17 | Down | 4.26E-04 |
| NM_006183 | Homo sapiens neurotensin (NTS), mRNA | 11.14 | Down | 8.52E-04 |
| NM_002522 | Homo sapiens neuronal pentraxin I (NPTX1), mRNA | 11.08 | Down | 3.03E-04 |
| NM_016428 | Homo sapiens ABI gene family, member 3 (ABI3), mRNA | 11.03 | Down | 2.83E-05 |
| NM_001769 | Homo sapiens CD9 antigen (p24) (CD9), mRNA | 11 | Down | 2.45E-04 |
| AL833655 | Homo sapiens mRNA; cDNA DKFZp667O0320 (from clone DKFZp667O0320) | 10.8 | Down | 1.73E-05 |
| R56121 | yg94d04.s1 Soares infant brain 1NIB Homo sapiens cDNA clone IMAGE:41388 3, mRNA sequence | 10.64 | Down | 1.04E-04 |
| NM_012445 | Homo sapiens spondin 2, extracellular matrix protein (SPON2), mRNA | 10.63 | Down | 1.36E-04 |
| NM_004811 | Homo sapiens leupaxin (LPXN), mRNA | 10.6 | Down | 2.09E-05 |
| NM_002977 | Homo sapiens sodium channel, voltage-gated, type IX, alpha (SCN9A), mRNA | 10.51 | Down | 2.09E-05 |
| BI598031 | 603248155F1 NIH_MGC_96 Homo sapiens cDNA clone IMAGE:5300149 5, mRNA sequence | 10.5 | Down | 3.34E-05 |
| NM_173553 | Homo sapiens hypothetical protein FLJ25801 (FLJ25801), MRNA, | 10.49 | Down | 2.32E-05 |
| NM_001311 | Homo sapiens cysteine-rich protein 1 (intestinal) (CRIP1), mRNA | 10.31 | Down | 2.73E-05 |
| NM_015916 | Homo sapiens family with sequence similarity 26, member B (FAM26B), mRNA | 10.28 | Down | 1.59E-04 |
| NM_005595 | Homo sapiens nuclear factor I/A (NFIA), mRNA | 10.12 | Down | 7.43E-04 |
| NM_182728 | Homo sapiens solute carrier family 7 (cationic amino acid transporter, y+ system), member 8 (SLC7A8), transcript variant 2, mRNA | 9.98 | Down | 6.78E-05 |
| NM_152996 | Homo sapiens sialyltransferase 7 ((alpha-N-acetylneuraminyl-2,3-beta-galactosyl-1,3)-N-acetyl galactosaminide alpha-2,6-sialyltransferase) C (SIAT7C), mRNA | 9.95 | Down | 1.25E-05 |
| AK091713 | Homo sapiens cDNA FLJ34394 fis, clone HCHON2000676 | 9.92 | Down | 2.31E-05 |
| AK022877 | Homo sapiens cDNA FLJ12815 fis, clone NT2RP2002546 | 9.89 | Down | 3.15E-05 |
| NM_194250 | Homo sapiens similar to C630007C17Rik protein (LOC91752), mRNA | 9.84 | Down | 7.08E-05 |
| BC039450 | Homo sapiens, clone IMAGE:5311619, mRNA | 9.77 | Down | 2.60E-04 |
| NM_031894 | Homo sapiens ferritin, heavy polypeptide-like 17 (FTHL17), mRNA | 9.76 | Down | 5.99E-04 |
| BI561641 | 603256058F1 NIH_MGC_97 Homo sapiens cDNA clone IMAGE:5298374 5, mRNA sequence | 9.68 | Down | 1.96E-03 |
| BC028245 | Homo sapiens, Similar to hypothetical gene LOC130797, clone IMAGE:5395354, mRNA | 9.68 | Down | 1.88E-05 |
| NM_031957 | Homo sapiens keratin associated protein 1-5 (KRTAP1-5), mRNA | 9.66 | Down | 2.31E-05 |
| AA947461 | ok20f03.s1 Soares_NSF_F8_9W_OT_PA_P_S1 Homo sapiens cDNA clone IMAGE:1508381 3, mRNA sequence | 9.56 | Down | 4.34E-05 |
| AK127309 | Homo sapiens cDNA FLJ45377 fis, clone BRHIP3019956 | 9.55 | Down | 1.17E-04 |
| NM_016247 | Homo sapiens interphotoreceptor matrix proteoglycan 2 (IMPG2), mRNA | 9.51 | Down | 2.09E-05 |
| NM_005574 | Homo sapiens LIM domain only 2 (rhombotin-like 1) (LMO2), mRNA | 9.46 | Down | 4.10E-05 |
| NM_004369 | Homo sapiens collagen, type VI, alpha 3 (COL6A3), transcript variant 1, mRNA | 9.36 | Down | 2.14E-05 |
| NM_003973 | Homo sapiens ribosomal protein L14 (RPL14), mRNA | 9.3 | Down | 6.77E-05 |
| NM_000459 | Homo sapiens TEK tyrosine kinase, endothelial (venous malformations, multiple cutaneous and mucosal) (TEK), mRNA | 9.25 | Down | 3.10E-04 |
| NM_005397 | Homo sapiens podocalyxin-like (PODXL), mRNA | 9.06 | Down | 6.52E-04 |
| BU675964 | UI-CF-DU1-aaf-b-24-0-Ul.s1 UI-CF-DU1 Homo sapiens cDNA clone UI-CF-DU1-aaf-b-24-0-UI 3, mRNA sequence | 9 | Down | 6.82E-05 |
| AI640484 | wa27f01.x1 NCI_CGAP_Kid11 Homo sapiens cDNA clone IMAGE:2299321 3, mRNA sequence | 8.92 | Down | 8.54E-05 |
| NM_030781 | Homo sapiens collectin sub-family member 12 (COLEC12), transcript variant II, mRNA | 8.54 | Down | 8.65E-05 |
| NM_001998 | Homo sapiens fibulin 2 (FBLN2), transcript variant 2, mRNA | 8.53 | Down | 4.60E-04 |
| BC039369 | Homo sapiens, clone IMAGE:5271073, mRNA, partial cds | 8.51 | Down | 4.85E-04 |
| AA436084 | zu03a02.r1 Soares_testis_NHT Homo sapiens cDNA clone IMAGE:730730 5 similar to contains element PTR5 PTR5 repetitive element ;, mRNA sequence | 8.51 | Down | 8.23E-05 |
| NM_015192 | Homo sapiens phospholipase C, beta 1 (phosphoinositide-specific) (PLCB1), transcript variant 1, mRNA | 8.48 | Down | 2.34E-04 |
| NM_024420 | Homo sapiens phospholipase A2, group IVA (cytosolic, calcium-dependent) (PLA2G4A), mRNA | 8.48 | Down | 2.23E-03 |
| BX095887 | BX095887 Soares_total_fetus_Nb2HF8_9w Homo sapiens cDNA clone IMAGp998G124121 ; IMAGE:1624739, mRNA sequence | 8.42 | Down | 7.38E-05 |
| NM_006417 | Homo sapiens interferon-induced protein 44 (IFI44), mRNA | 8.37 | Down | 2.46E-04 |
| AK022033 | Homo sapiens cDNA FLJ11971 fis, clone HEMBB1001208 | 8.35 | Down | 2.06E-04 |
| N69782 | yz60b07.s1 Morton Fetal Cochlea Homo sapiens cDNA clone IMAGE:287413 3, mRNA sequence | 8.02 | Down | 2.78E-04 |
| NM_023003 | Homo sapiens transmembrane 6 superfamily member 1 (TM6SF1), MRNA, | 7.93 | Down | 1.20E-04 |
| BC036034 | Homo sapiens endothelial differentiation, lysophosphatidic acid G-protein-coupled receptor, 2, transcript variant 2, mRNA (cDNA clone MGC:33157 IMAGE:5272431), complete cds | 7.86 | Down | 2.31E-05 |
| NM_005602 | Homo sapiens claudin 11 (oligodendrocyte transmembrane protein) (CLDN11), mRNA | 7.84 | Down | 2.43E-02 |
| BC039676 | Homo sapiens, clone IMAGE:5173389, mRNA | 7.74 | Down | 3.04E-05 |
| BC030692 | Homo sapiens ELAV (embryonic lethal, abnormal vision, Drosophila)-like 2 (Hu antigen B), mRNA (cDNA clone MGC:26319 IMAGE:4826082), complete cds | 7.72 | Down | 3.59E-05 |
| NM_015278 | Homo sapiens SAM and SH3 domain containing 1 (SASH1), mRNA | 7.58 | Down | 1.38E-04 |
| NM_031283 | Homo sapiens transcription factor 7-like 1 (T-cell specific, HMG-box) (TCF7L1), mRNA | 7.57 | Down | 4.12E-05 |
| BC042378 | Homo sapiens, clone IMAGE:5277693, mRNA | 7.56 | Down | 9.91E-04 |
| NM_013363 | Homo sapiens procollagen C-endopeptidase enhancer 2 (PCOLCE2), mRNA | 7.49 | Down | 5.18E-03 |
| NM_005923 | Homo sapiens mitogen-activated protein kinase kinase kinase 5 (MAP3K5), mRNA | 7.48 | Down | 2.05E-04 |
| R53688 | yg84h04.r1 Soares infant brain 1NIB Homo sapiens cDNA clone IMAGE:40175 5, mRNA sequence | 7.43 | Down | 2.31E-05 |
| AI216469 | qh07h10.x1 Soares_NFL_T_GBC_S1 Homo sapiens cDNA clone IMAGE: 1844035 3, mRNA sequence | 7.28 | Down | 4.92E-05 |
| NM_014331 | Homo sapiens solute carrier family 7, (cationic amino acid transporter, y+ system) member 11 (SLC7A11), mRNA | 7.27 | Down | 2.76E-03 |
| NM_138801 | Homo sapiens galactose mutarotase (aldose 1-epimerase) (GALM), mRNA | 7.24 | Down | 5.50E-05 |
| AL049443 | Homo sapiens mRNA; cDNA DKFZp586N2020 (from clone DKFZp586N2020) | 7.22 | Down | 2.83E-04 |
| NM_005328 | Homo sapiens hyaluronan synthase 2 (HAS2), mRNA | 7.17 | Down | 3.96E-03 |
| NM_006072 | Homo sapiens chemokine (C-C motif) ligand 26 (CCL26), mRNA | 7.12 | Down | 6.86E-05 |
| NM_020987 | Homo sapiens ankyrin 3, node of Ranvier (ankyrin G) (ANK3), transcript variant 1, mRNA | 7.09 | Down | 7.31E-05 |
| BX647541 | Homo sapiens mRNA; cDNA DKFZp686P0492 (from clone DKFZp686P0492) | 7.08 | Down | 4.47E-05 |
| AI417595 | tg79h09.x1 Soares_NhHMPu_S1 Homo sapiens cDNA clone IMAGE:2115041 3, mRNA sequence | 7.07 | Down | 7.52E-04 |
| AA158235 | zo76b02.s1 Stratagene pancreas (#937208) Homo sapiens cDNA clone IMAGE:592779 3, mRNA sequence | 7.06 | Down | 1.52E-04 |
| NM_005583 | Homo sapiens lymphoblastic leukemia derived sequence 1 (LYL1), MRNA, | 7.03 | Down | 4.69E-05 |
| BU570253 | AGENCOURT_10401698 NIH_MGC_82 Homo sapiens cDNA clone IMAGE:6618451 5, mRNA sequence | 7 | Down | 2.35E-04 |
| BM675371 | UI-E-EJ0-aht-a-07-0-UI.s1 UI-E-EJ0 Homo sapiens cDNA clone UI-E-EJ0-aht-a-07-0-UI 3, mRNA sequence | 6.95 | Down | 4.53E-05 |
| AK024653 | Homo sapiens cDNA: FLJ21000 fis, clone CAE03359 | 6.93 | Down | 6.77E-05 |
| W31037 | zb86c06.r1 Soares_senescent_fibroblasts_NbHSF Homo sapiens cDNA clone IMAGE:310474 5, mRNA sequence | 6.91 | Drown | 4.95E-05 |
| AL831835 | Homo sapiens mRNA; cDNA DKFZp547A0515 (from clone DKFZp547A0515) | 6.9 | Down | 1.53E-03 |
| NM_001849 | Homo sapiens collagen, type VI, alpha 2 (COL6A2), transcript variant 2C2, mRNA | 6.85 | Down | 3.34E-05 |
| NM_007084 | Homo sapiens SRY (sex determining region Y)-box 21 (SOX21), mRNA | 6.85 | Down | 3.88E-02 |
| NM_025107 | Homo sapiens myc target 1 (MYCT1), MRNA, | 6.82 | Down | 6.89E-05 |
| AA393981 | zt58a03.r1 Soares_testis_NHT Homo sapiens cDNA clone IMAGE:726508 5, mRNA sequence | 6.82 | Down | 4.58E-03 |
| NM_170744 | Homo sapiens unc-5 homolog B (C. elegans) (UNC5B), mRNA | 6.82 | Down | 9.92E-04 |
| AL133118 | Homo sapiens mRNA; cDNA DKFZp586N0121 (from clone DKFZp586N0121) | 6.78 | Down | 1.42E-03 |
| AK090603 | Homo sapiens cDNA FLJ33284 fis, clone ASTRO2009458 | 6.76 | Down | 1.36E-04 |
| NM_002203 | Homo sapiens integrin, alpha 2 (CD49B, alpha 2 subunit of VLA-2 receptor) (ITGA2), mRNA | 6.74 | Down | 4.93E-05 |
| NM_003713 | Homo sapiens phosphatidic acid phosphatase type 2B (PPAP2B), transcript variant 1, mRNA | 6.73 | Down | 1.06E-04 |
| NM_001401 | Homo sapiens endothelial differentiation, lysophosphatidic acid G-protein-coupled receptor, 2 (EDG2), transcript variant 1, mRNA | 6.69 | Down | 9.75E-04 |
| NM_020226 | Homo sapiens PR domain containing 8 (PRDM8), mRNA | 6.68 | Down | 2.31E-04 |
| N71963 | yz95e03.s1 Soares melanocyte 2NbHM Homo sapiens cDNA clone IMAGE:290812 3 similar to contains Alu repetitive element;, mRNA sequence | 6.63 | Down | 2.47E-04 |
| AK127536 | Homo sapiens cDNA FLJ45629 fis, clone CHONS2000797, highly similar to T-box transcription factor TBX15 | 6.6 | Down | 1.09E-04 |
| NM_006206 | Homo sapiens platelet-derived growth factor receptor, alpha polypeptide (PDGFRA), mRNA | 6.55 | Down | 6.30E-04 |
| NM_016559 | Homo sapiens peroxisomal biogenesis factor 5-like (PEX5L), mRNA | 6.55 | Down | 6.77E-04 |
| NM_012307 | Homo sapiens erythrocyte membrane protein band 4.1-like 3 (EPB41L3), mRNA | 6.51 | Down | 9.62E-04 |
| R34294 | yh84b01.s1 Soares placenta Nb2HP Homo sapiens cDNA clone IMAGE:136393 3, mRNA sequence | 6.49 | Down | 2.78E-05 |
| N36786 | yy34e08.s1 Soares melanocyte 2NbHM Homo sapiens cDNA clone IMAGE:273158 3 similar to contains element MSR1 repetitive element ;, mRNA sequence | 6.49 | Down | 6.12E-05 |
| BX113851 | BX113851 Soares_total_fetus_Nb2HF8_9w Homo sapiens cDNA clone IMAGp998G054116 ; IMAGE:1622812, mRNA sequence | 6.44 | Down | 8.41E-05 |
| BG484952 | 602503960F1 NIH_MGC_77 Homo sapiens cDNA clone IMAGE:4617278 5, mRNA sequence | 6.43 | Down | 9.06E-05 |
| NM_005434 | Homo sapiens BENE protein (BENE), mRNA | 6.4 | Down | 1.64E-05 |
| BX106577 | BX106577 Soares_NhHMPu_S1 Homo sapiens cDNA clone IMAGp998H131854 ; IMAGE:754236, mRNA sequence | 6.39 | Down | 4.12E-05 |
| NM_144617 | Homo sapiens heat shock protein, alpha-crystallin-related, B6 (HSPB6), mRNA | 6.37 | Down | 2.83E-05 |
| AK093529 | Homo sapiens cDNA FLJ36210 fis, clone THYMU2000155 | 6.29 | Down | 1.52E-04 |
| NM_022731 | Homo sapiens nuclear ubiquitous casein kinase and cyclin-dependent kinase substrate (NUCKS), mRNA | 6.27 | Down | 9.06E-05 |
| NM_018286 | Homo sapiens hypothetical protein FLJ10970 (FLJ10970), mRNA | 6.26 | Down | 1.08E-03 |
| NM_002290 | Homo sapiens laminin, alpha 4 (LAMA4), mRNA | 6.19 | Down | 6.97E-04 |
| NM_015184 | Homo sapiens phospholipase C-like 2 (PLCL2), mRNA | 6.18 | Down | 5.91E-04 |
| NM_017577 | Homo sapiens hypothetical protein DKFZp434C0328 (DKFZp434C0328), mRNA | 6.17 | Down | 3.60E-05 |
| BQ350534 | RC1-HT0256-120400-019-d06 HT0256 Homo sapiens cDNA, mRNA sequence | 6.17 | Down | 2.04E-04 |
| NM_004934 | Homo sapiens cadherin 18, type 2 (CDH18), mRNA | 6.17 | Down | 3.03E-05 |
| NM_024769 | Homo sapiens adipocyte-specific adhesion molecule (ASAM), mRNA | 6.15 | Down | 2.41E-04 |
| NM_197941 | Homo sapiens similar to ADAMTS-10 precursor (A disintegrin and metalloproteinase with thrombospondin motifs 10) (ADAM-TS 10) (ADAM-TS10) (LOC345667), mRNA | 6.14 | Down | 1.06E-04 |
| BG118019 | 602351269F1 NIH_MGC_90 Homo sapiens cDNA clone IMAGE:4446065 5, mRNA sequence | 6.14 | Down | 5.02E-05 |
| NM_005531 | Homo sapiens interferon, gamma-inducible protein 16 (IF116), mRNA | 6.13 | Down | 4.46E-04 |
| NM_005613 | Homo sapiens regulator of G-protein signalling 4 (RGS4), mRNA | 6.08 | Down | 1.04E-04 |
| NM_002166 | Homo sapiens inhibitor of DNA binding 2, dominant negative helix-loop-helix protein (ID2), mRNA | 6.07 | Down | 1.16E-03 |
| BC015720 | Homo sapiens, clone IMAGE:3909165, mRNA | 6.04 | Down | 1.15E-05 |
| BF508005 | UI-H-B14-apw-e-06-0-UI.s1 NCI_CGAP_Sub8 Homo sapiens cDNA clone IMAGE:3088978 3, mRNA sequence | 6.03 | Drown | 1.15E-05 |
| NM_007101 | Homo sapiens sarcosine dehydrogenase (SARDH), mRNA | 6.01 | Down | 4.54E-03 |
| BG025371 | 602276295F1 NIH_MGC_85 Homo sapiens cDNA clone IMAGE:4364351 5, mRNA sequence | 5.99 | Down | 6.94E-05 |
| NM_152666 | Homo sapiens hypothetical protein FLJ40773 (FLJ40773), mRNA | 5.99 | Down | 4.55E-04 |
| BM542398 | AGENCOURT_6436663 NIH_MGC_72 Homo sapiens cDNA clone IMAGE:5539574 5, mRNA sequence | 5.97 | Down | 3.34E-05 |
| AK054783 | Homo sapiens cDNA FLJ30221 fis, clone BRACE2001742 | 5.97 | Down | 2.40E-04 |
| AI651524 | wb06g07.x1 NCI_CGAP_GC6 Homo sapiens cDNA clone IMAGE:2304924 3, mRNA sequence | 5.93 | Down | 3.22E-04 |
| NM_005905 | Homo sapiens SMAD, mothers against DPP homolog 9 (Drosophila) (SMAD9), mRNA | 5.87 | Down | 4.76E-04 |
| BG818762 | 602779092F2 NCI_CGAP_Brn67 Homo sapiens cDNA clone IMAGE:4914502 5, mRNA sequence | 5.84 | Down | 1.07E-04 |
| NM_002487 | Homo sapiens necdin homolog (mouse) (NDN), mRNA | 5.83 | Down | 2.72E-05 |
| NM_000167 | Homo sapiens glycerol kinase (GK), transcript variant 2, mRNA | 5.8 | Down | 5.10E-03 |
| BC070147 | Homo sapiens cDNA clone IMAGE:4672631, containing frame-shift errors | 5.74 | Down | 7.92E-04 |
| NM_001711 | Homo sapiens biglycan (BGN), mRNA | 5.71 | Down | 4.13E-05 |
| NM_000407 | Homo sapiens glycoprotein lb (platelet), beta polypeptide (GP1BB), mRNA | 5.69 | Down | 3.03E-05 |
| N28431 | yx35c03.r1 Soares melanocyte 2NbHM Homo sapiens cDNA clone IMAGE:263716 5 similar to SP:PIR:S32603 S32603 collagen alpha 1 (VI) chain - mouse ;, mRNA sequence | 5.69 | Down | 6.03E-04 |
| NM_002615 | Homo sapiens serine (or cysteine) proteinase inhibitor, clade F (alpha-2 antiplasmin, pigment epithelium derived factor), member 1 (SERPINF1), MRNA, | 5.66 | Down | 2.02E-03 |
| H18367 | yn49d06.r1 Soares adult brain N2b5HB55Y Homo sapiens cDNA clone IMAGE:171755 3, mRNA sequence | 5.66 | Down | 4.41E-04 |
| NM_018455 | Homo sapiens uncharacterized bone marrow protein BM039 (BM039), mRNA | 5.64 | Down | 3.03E-04 |
| H24359 | ym56b03.s1 Soares infant brain 1NIB Homo sapiens cDNA clone IMAGE:52294 3, mRNA sequence | 5.61 | Down | 2.57E-04 |
| BU078105 | im64b02.y1 HR85 islet Homo sapiens cDNA clone IMAGE:6039866 5, mRNA sequence | 5.58 | Down | 3.60E-04 |
| NM_022576 | Homo sapiens phosducin (PDC), transcript variant 2, mRNA | 5.55 | Down | 9.39E-05 |
| NM_033196 | Homo sapiens hypothetical ZNF-like protein (LOC91120), mRNA | 5.54 | Down | 1.49E-04 |
| BC035066 | Homo sapiens, clone IMAGE:5259543, mRNA | 5.54 | Down | 1.65E-04 |
| R14261 | yf79d05.r1 Soares infant brain 1NIB Homo sapiens cDNA clone IMAGE:28642 5, mRNA sequence | 5.51 | Down | 2.36E-04 |
| BC042140 | Homo sapiens, clone IMAGE:5932306, mRNA | 5.48 | Down | 5.05E-05 |
| NM_005654 | Homo sapiens nuclear receptor subfamily 2, group F, member 1 (NR2F1), MRNA, | 5.48 | Down | 1.02E-05 |
| NM_182746 | Homo sapiens MCM4 minichromosome maintenance deficient 4 (S. cerevisiae) (MCM4), transcript variant 2, mRNA | 5.48 | Down | 9.96E-04 |
| BG573337 | 602595107F1 NIH_MGC_79 Homo sapiens cDNA clone IMAGE:4724521 5, mRNA sequence | 5.43 | Down | 3.72E-04 |
| NM_194250 | Homo sapiens similar to C630007C17Rik protein (LOC91752), mRNA | 5.43 | Down | 3.30E-04 |
| NM_015430 | Homo sapiens regeneration associated muscle protease (DKFZP586H2123), transcript variant 1, mRNA | 5.43 | Down | 3.49E-03 |
| NM_006868 | Homo sapiens RAB31, member RAS oncogene family (RAB31), MRNA, | 5.41 | Down | 2.11E-05 |
| BI494495 | df110f11.w1 Morton Fetal Cochlea Homo sapiens cDNA clone IMAGE:2539149 3, mRNA sequence | 5.4 | Down | 2.64E-03 |
| AW025556 | wu97g10.x1 NCI_CGAP_Kid3 Homo sapiens cDNA clone IMAGE:2528034 3, mRNA | 5.36 | Down | 1.89E-04 |
| AI793182 | sequence qz36a11.x5 NCI_CGAP_Kid11 Homo sapiens cDNA clone IMAGE:2028956 3 similar to contains L1.t3 L1 repetitive element ;, mRNA sequence | 5.34 | Down | 8.09E-04 |
| NM_006208 | Homo sapiens ectonucleotide pyrophosphatase/phosphodiesterase 1 (ENPP1), mRNA | 5.34 | Down | 2.36E-04 |
| C02345 | HUMGS0007544 Human adult (K.Okubo) Homo sapiens cDNA, mRNA sequence | 5.33 | Down | 3.45E-04 |
| NM_032297 | Homo sapiens hypothetical protein DKFZp761D112 (DKFZp761D112), mRNA | 5.32 | Down | 3.38E-04 |
| BM724062 | UI-E-E01-aiy-a-22-0-UI.r1 UI-E-E01 Homo sapiens cDNA clone UI-E-E01-aiy-a-22-0-UI 5, mRNA sequence | 5.25 | Down | 2.96E-03 |
| NM_176891 | Homo sapiens interferon epsilon 1 (IFNE1), mRNA | 5.22 | Down | 2.67E-04 |
| BX648959 | Homo sapiens mRNA; cDNA DKFZp686N2348 (from clone DKFZp686N2348) | 5.22 | Down | 4.12E-05 |
| CA771688 | io81c08.x1 HR85 islet Homo sapiens cDNA clone IMAGE:6132854 3, mRNA sequence | 5.22 | Down | 3.35E-03 |
| BC034315 | Homo sapiens hypothetical protein LOC90529, mRNA (cDNA clone IMAGE:4827425), containing frame-shift errors | 5.22 | Down | 1.90E-04 |
| W88428 | zh72g09.s1 Soares_fetal_liver_spleen_1NFLSS1 Homo sapiens cDNA clone IMAGE:417664 3, mRNA sequence | 5.22 | Down | 5.95E-04 |
| AI468014 | tj84g05.x1 Soares_NSF_F8_9W_OT_PA_P_S1 Homo sapiens cDNA clone IMAGE:2148248 3 similar to contains element TAR1 repetitive element ;, mRNA sequence | 5.21 | Down | 9.55E-04 |
| NM_197941 | Homo sapiens similar to ADAMTS-10 precursor (A disintegrin and metalloproteinase with thrombospondin motifs 10) (ADAM-TS 10) (ADAM-TS10) (LOC345667), mRNA | 5.2 | Drown | 8.12E-04 |
| U10991 | Human G2 protein mRNA, partial cds | 5.19 | Down | 7.92E-05 |
| AI888390 | wn30g10.x1 NCI_CGAP_Gas4 Homo sapiens cDNA clone IMAGE:2447010 3 similar to contains element MER8 repetitive element ;, mRNA sequence | 5.19 | Down | 2.07E-04 |
| NM_012411 | Homo sapiens protein tyrosine phosphatase, non-receptor type 22 (lymphoid) (PTPN22), transcript variant 2, mRNA | 5.19 | Down | 3.62E-04 |
| NM_030806 | Homo sapiens chromosome 1 open reading frame 21 (C1orf21), mRNA | 5.17 | Down | 1.88E-05 |
| NM_005654 | Homo sapiens nuclear receptor subfamily 2, group F, member 1 (NR2F1), mRNA | 5.17 | Down | 8.65E-05 |
| NM_153014 | Homo sapiens hypothetical protein FLJ30634 (FLJ30634), mRNA | 5.13 | Down | 1.32E-03 |
| AB020691 | Homo sapiens mRNA for KIAA0884 protein, partial cds | 5.13 | Down | 3.40E-05 |
| NM_020211 | Homo sapiens RGM domain family, member A (RGMA), mRNA | 5.1 | Down | 1.51E-04 |
| BG215747 | RST35420 Athersys RAGE Library Homo sapiens cDNA, mRNA sequence | 5.1 | Down | 1.30E-04 |
| NM_002317 | Homo sapiens lysyl oxidase (LOX), mRNA | 5.09 | Down | 1.28E-04 |
| NM_003608 | Homo sapiens G protein-coupled receptor 65 (GPR65), mRNA | 5.07 | Down | 7.41E-04 |
| NM_031305 | Homo sapiens Rho GTPase activating protein 24 (ARHGAP24), mRNA | 5.05 | Down | 1.10E-04 |
| AK124391 | Homo sapiens cDNA FLJ42400 fis, clone ASTRO2003581 | 5.03 | Down | 9.74E-05 |
| TM_005613 | Homo sapiens regulator of G-protein signalling 4 (RGS4), mRNA | 5.02 | Down | 7.55E-05 |
| NM_054027 | Homo sapiens ankylosis, progressive homolog (mouse) (ANKH), mRNA | 5 | Down | 2.64E-05 |

**TABLE VII C: GENES THAT WERE DIFFERENTIALLY EXPRESSED AT LEAST 5 FOLD IN EPITHELIAL VERSUS AF-I CELLS**

| Gene Identifier | Gene Name | Average fold change in Epithelial versus AF cells | Direction | adj. p-value |
|---|---|---|---|---|
| AK091336 | Homo sapiens cDNA FLJ34017 fis, clone FCBBF2002626 | 97.64 | UP | 6.01E-03 |
| NM_004098 | Homo sapiens empty spiracles homolog 2 (Drosophila) (EMX2), mRNA | 83.49 | UP | 8.10E-03 |
| AV702977 | AV702977 ADB Homo sapiens cDNA clone ADBCVD08 5, mRNA sequence | 82.96 | UP | 3.74E-03 |
| BE877764 | 601486331 F1 NIH_MGC_69 Homo sapiens cDNA clone IMAGE:3888943 5, mRNA sequence | 60.78 | UP | 2.79E-03 |
| AK075003 | Homo sapiens cDNA FLJ90522 fis, clone NT2RP4000108, highly similar to Human gene for neurofilament subunit NF-L | 48.1 | UP | 1.02E-02 |
| NM_014358 | Homo sapiens C-type (calcium dependent, carbohydrate-recognition domain) lectin, superfamily member 9 (CLECSF9), mRNA | 48.04 | UP | 1.99E-04 |
| AK092401 | Homo sapiens cDNA FLJ35082 fis, clone PLACE6005351 | 40.58 | UP | 1.77E-03 |
| NM_007029 | Homo sapiens stathmin-like 2 (STMN2), mRNA | 38.31 | UP | 1.31E-02 |
| AW300043 | xs45a09.x1 NCI_CGAP_Kid11 Homo sapiens cDNA clone IMAGE:2772568 3, mRNA sequence | 25.26 | UP | 1.35E-02 |
| NM_006158 | Homo sapiens neurofilament, light polypeptide 68kDa (NEFL), mRNA | 23.08 | UP | 1.76E-03 |
| NM_003222 | Homo sapiens transcription factor AP-2 gamma (activating enhancer binding protein 2 gamma) (TFAP2C), mRNA | 22.98 | UP | 1.59E-02 |
| AK023647 | Homo sapiens cDNA FLJ13585 fis, clone PLACE1009150 | 22.77 | UP | 7.48E-04 |
| BC044843 | Homo sapiens hypothetical protein LOC339535, mRNA (cDNA clone IMAGE:5186761), partial cds | 21.74 | UP | 4.67E-03 |
| BQ003501 | UI-H-E11-azd-p-06-0-UI.s1 NCI_CGAP_EI1 Homo sapiens cDNA clone IMAGE:5847413 3, mRNA sequence | 20.08 | UP | 9.54E-04 |
| AA075748 | zm89e04.r1 Stratagene ovarian cancer (#937219) Homo sapiens cDNA clone IMAGE:545118 5, mRNA sequence | 19.94 | UP | 7.10E-03 |
| BF000009 | 7h15g04.x1 NCI_CGAP_Co16 Homo sapiens cDNA clone IMAGE:3316086 3, mRNA sequence | 19.92 | UP | 7.67E-03 |
| NM_004867 | Homo sapiens integral membrane protein 2A (ITM2A), mRNA | 19.86 | UP | 2.62E-02 |
| H85497 | yv88b07.r1 Soares melanocyte 2NbHM Homo sapiens cDNA clone IMAGE:249781 5, mRNA sequence | 19.75 | UP | 7.71E-05 |
| BM713465 | UI-E-EJ0-aho-m-22-0-UI.r1 UI-E-EJ0 Homo sapiens cDNA clone UI-E-EJ0-aho-m-22-0-UI 5, mRNA sequence | 18.57 | UP | 1.68E-02 |
| BU569937 | AGENCOURT_10399817 NIH_MGC_82 Homo sapiens cDNA clone IMAGE:6618011 5, mRNA sequence | 18.3 | UP | 1.68E-02 |
| NM_032782 | Homo sapiens hepatitis A virus cellular receptor 2 (HAVCR2), mRNA | 18.05 | UP | 2.97E-03 |
| NM_013387 | Homo sapiens ubiquinol-cytochrome c reductase complex (7.2 kD) (HSPC051), transcript variant 1, mRNA | 17.68 | UP | 1.30E-04 |
| BM988338 | UI-H-DH0-asd-f-10-0-UI.s1 NCI_CGAP_DHO Homo sapiens cDNA clone IMAGE:5857545 3, mRNA sequence | 17.23 | UP | 9.27E-03 |
| BQ025821 | UI-1-BB1p-aye-f-10-0-UI.s1 NCI_CGAP_PI6 Homo sapiens cDNA clone UI-1-BB1p-aye-f-10-0-UI 3, mRNA sequence | 15.46 | UP | 1.08E-02 |
| BF515657 | UI-H-BW1-anu-e-05-0-UI.s1 NCI_CGAP_Sub7 Homo sapiens cDNA clone IMAGE:3083601 3, mRNA sequence | 15.17 | UP | 8.95E-03 |
| NM_001977 | Homo sapiens glutamyl aminopeptidase (aminopeptidase A) (ENPEP), mRNA | 14.59 | UP | 5.24E-05 |
| NM_197955 | Homo sapiens normal mucosa of esophagus specific 1 (NMES1), transcript variant 1, mRNA | 14.33 | UP | 2.54E-03 |
| NM_180991 | Homo sapiens solute carrier organic anion transporter family, member 4C1 (SLC04C1), mRNA | 13.46 | UP | 2.20E-02 |
| R44402 | yg37a01.s1 Soares infant brain 1NIB Homo sapiens cDNA clone IMAGE:34639 3 similar to contains MER35 repetitive element ;, mRNA sequence | 13.17 | UP | 1.56E-04 |
| CF137545 | UI-HF-BN0-ane-d-05-0-UI.r1 NIH_MGC_50 Homo sapiens cDNA clone IMAGE:3092384 5, mRNA sequence | 12.96 | UP | 6.05E-03 |
| NM_016269 | Homo sapiens lymphoid enhancer-binding factor 1 (LEF1), mRNA | 12.69 | UP | 7.27E-03 |
| NM_001680 | Homo sapiens FXYD domain containing ion transport regulator 2 (FXYD2), transcript variant a, mRNA | 12.65 | UP | 2.38E-03 |
| NM_003063 | Homo sapiens sarcolipin (SLN), mRNA | 12.54 | UP | 3.04E-02 |
| NM_012204 | Homo sapiens general transcription factor IIIC, polypeptide 4, 90kDa (GTF3C4), mRNA | 12.51 | UP | 3.10E-02 |
| NM_003810 | Homo sapiens tumor necrosis factor (ligand) superfamily, member 10 (TNFSF10), mRNA | 12.4 | UP | 9.19E-05 |
| NM_004591 | Homo sapiens chemokine (C-C motif) ligand 20 (CCL20), mRNA | 11.84 | UP | 3.08E-02 |
| NM_020927 | Homo sapiens KIAA1576 protein (KIAA1576), mRNA | 11.77 | UP | 6.78E-03 |
| NM_014751 | Homo sapiens metastasis suppressor 1 (MTSS1), mRNA | 11.5 | UP | 6.60E-05 |
| NM_031311 | Homo sapiens carboxypeptidase, vitellogenic-like (CPVL), transcript variant 1, mRNA | 10.36 | UP | 8.15E-03 |
| NM_005382 | Homo sapiens neurofilament 3 (150kDa medium) (NEF3), mRNA | 10.12 | UP | 4.45E-02 |
| NM_033554 | Homo sapiens major histocompatibility complex, class II, DP alpha 1 (HLA-DPA1), mRNA | 9.84 | UP | 4.35E-02 |
| AI632692 | wa33b05.x1 NCI_CGAP_Kid11 Homo sapiens cDNA clone IMAGE:2299857 3, mRNA sequence | 9.7 | UP | 3.01E-02 |
| NM_030923 | Homo sapiens hypothetical protein DKFZp566N034 (DKFZP566N034), mRNA | 9.51 | UP | 2.72E-02 |
| NM_152550 | Homo sapiens SH3 domain containing ring finger 2 (SH3RF2), mRNA | 9.31 | UP | 4.41E-02 |
| AW445209 | UI-H-BI3-akc-g-11-0-UI.s1 NCI_CGAP_Sub5 Homo sapiens cDNA clone IMAGE:2733908 3, mRNA sequence | 9.27 | UP | 1.37E-03 |
| AB011095 | Homo sapiens mRNA for KIAA0523 protein, partial cds | 8.77 | UP | 1.82E-04 |
| NM_194463 | Homo sapiens ring finger protein 128 (RNF128), transcript variant 1, mRNA | 8.75 | UP | 2.95E-03 |
| NM_013427 | Homo sapiens Rho GTPase activating protein 6 (ARHGAP6), transcript variant 1, mRNA | 8.73 | UP | 7.38E-04 |
| NM_012105 | Homo sapiens beta-site APP-cleaving enzyme 2 (BACE2), transcript variant a, mRNA | 8.67 | UP | 1.49E-04 |
| NM_000640 | Homo sapiens interleukin 13 receptor, alpha 2 (IL13RA2), mRNA | 8.29 | UP | 4.39E-02 |
| NM_001977 | Homo sapiens glutamyl aminopeptidase (aminopeptidase A) (ENPEP), mRNA | 8.16 | UP | 6.01E-03 |
| T56535 | yb33g07.r1 Stratagene fetal spleen (#937205) Homo sapiens cDNA clone IMAGE:73020 5, mRNA sequence | 8.08 | UP | 1.24E-04 |
| NM_022440 | Homo sapiens mal, T-cell differentiation protein (MAL), transcript variant d, mRNA | 8.01 | UP | 4.29E-02 |
| BM711923 | UI-E-CL1-afc-m-16-0-UI.r1 UI-E-CL1 Homo sapiens cDNA clone UI-E-CL1-afc-m-16-0-UI 5, mRNA sequence | 7.87 | UP | 8.56E-04 |
| NM_005181 | Homo sapiens carbonic anhydrase III, muscle specific (CA3), mRNA | 7.69 | UP | 1.46E-02 |
| NM_030781 | Homo sapiens collectin sub-family member 12 (COLEC12), transcript variant II, mRNA | 7.56 | UP | 8.07E-04 |
| NM_001305 | Homo sapiens claudin 4 (CLDN4), mRNA | 7.41 | UP | 3.62E-04 |
| NM_000055 | Homo sapiens butyrylcholinesterase (BCHE), mRNA | 7.26 | UP | 4.51E-03 |
| NM_153183 | Homo sapiens nudix (nucleoside diphosphate linked moiety X)-type motif 10 (NUDT10), mRNA | 7.23 | UP | 3.53E-02 |
| BI759570 | 603046987F1 NIH-MGC-116 Homo sapiens cDNA clone IMAGE:5187285 5, mRNA sequence | 7.11 | UP | 3.86E-02 |
| NM_015559 | Homo sapiens SET binding protein 1 (SETBP1), mRNA | 6.97 | UP | 3.67E-02 |
| NM_005562 | Homo sapiens laminin, gamma 2 (LAMC2), transcript variant 1, mRNA | 6.95 | UP | 2.06E-04 |
| NM_013430 | Homo sapiens gamma-glutamyltransferase 1 (GGT1), transcript variant 3, mRNA | 6.9 | UP | 3.86E-02 |
| AA908815 | og77h08.s1 NCI_CGAP_Ov8 Homo sapiens cDNA clone IMAGE:1454367 3, mRNA sequence | 6.8 | UP | 3.10E-02 |
| NM_022843 | Homo sapiens protocadherin 20 (PCDH20), mRNA | 6.73 | UP | 5.39E-03 |
| NM_018076 | Homo sapiens armadillo repeat containing 4 (ARMC4), mRNA | 6.71 | UP | 7.53E-04 |
| NM_005822 | Homo sapiens Down syndrome critical region gene 1-like 1 (DSCR1L1), mRNA | 6.67 | UP | 2.38E-02 |
| NM_006393 | Homo sapiens nebulette (NEBL), transcript variant 1, mRNA | 6.66 | UP | 4.24E-02 |
| CA391258 | cs13a10.x1 Human Retinal pigment epithelium/choroid cDNA (Unnormalized, unamplified): cs Homo sapiens cDNA clone cs13a10 3, mRNA sequence | 6.56 | UP | 9.19E-05 |
| AK026966 | Homo sapiens cDNA: FLJ23313 fis, clone HEP11919 | 6.31 | UP | 1.30E-04 |
| NM_020661 | Homo sapiens activation-induced cytidine deaminase (AICDA), mRNA | 6.12 | UP | 3.01E-02 |
| NM_004840 | Homo sapiens Rac/Cdc42 guanine nucleotide exchange factor (GEF) 6 (ARHGEF6), mRNA | 6.09 | UP | 1.19E-03 |
| BC021684 | Homo sapiens, clone IMAGE:3827252, mRNA | 6.03 | UP | 4.71E-03 |
| NM_032551 | Homo sapiens G protein-coupled receptor 54 (GPR54), mRNA | 5.96 | UP | 4.33E-02 |
| NM_024726 | Homo sapiens IQ motif containing with AAA domain (IQCA), mRNA | 5.94 | UP | 1.84E-04 |
| NM_001657 | Homo sapiens amphiregulin (schwannoma-derived growth factor) (AREG), mRNA | 5.91 | UP | 1.05E-03 |
| NM_006033 | Homo sapiens lipase, endothelial (LIPG), mRNA | 5.9 | UP | 1.76E-04 |
| NM_002089 | Homo sapiens chemokine (C-X-C motif) ligand 2 (CXCL2), mRNA | 5.88 | UP | 1.13E-04 |
| AK074097 | Homo sapiens mRNA for FLJ00168 protein | 5.87 | UP | 1.17E-03 |
| BF509573 | UI-H-BI4-apf-b-11-0-UI.s1 NCI_CGAP_Sub8 Homo sapiens cDNA clone IMAGE:3086949 3, mRNA sequence | 5.78 | UP | 1.40E-04 |
| H23441 | ym52f11.s1 Soares infant brain 1 NIB Homo sapiens cDNA clone IMAGE:51888 3, mRNA sequence | 5.77 | UP | 6.87E-04 |
| NM_031426 | Homo sapiens chromosome 9 open reading frame 58 (C9orf58), transcript variant 1, mRNA | 5.63 | UP | 3.32E-05 |
| NM_013410 | Homo sapiens adenylate kinase 3 (AK3), nuclear gene encoding mitochondrial protein, transcript variant 2, mRNA | 5.54 | UP | 1.14E-04 |
| NM_003236 | Homo sapiens transforming growth factor, alpha (TGFA), mRNA | 5.51 | UP | 3.09E-02 |
| NM_152369 | Homo sapiens hypothetical protein MGC45474 (MGC45474), mRNA | 5.46 | UP | 1.02E-04 |
| NM_153229 | Homo sapiens hypothetical protein FLJ33318 (FLJ33318), mRNA | 5.4 | UP | 1.92E-03 |
| NM_000216 | Homo sapiens Kallmann syndrome 1 sequence (KAL1), mRNA | 5.23 | UP | 1.13E-04 |
| H89526 | yw28b04.r1 Morton Fetal Cochlea Homo sapiens cDNA clone IMAGE:253519 5, mRNA sequence | 5.15 | UP | 9.54E-05 |
| AL832916 | Homo sapiens mRNA; cDNA DKFZp762I0915 (from clone DKFZp762I0915) | 5.12 | UP | 6.74E-03 |
| NM_002899 | Homo sapiens retinol binding protein 1, cellular (RBP1), mRNA | 5.07 | UP | 1.11E-03 |
| CA417015 | UI-H-FE0-bbp-d-21-0-UI.s1 NCI_CGAP_FE0 Homo sapiens cDNA clone UI-H-FE0-bbp-d-21-0-UI 3, mRNA sequence | 5.07 | UP | 1.77E-03 |
| AW263542 | xn80f01.x1 Soares_NFL_T_GBC_S1 Homo sapiens cDNA clone IMAGE:2700793 3, mRNA sequence | 5.07 | UP | 9.70E-03 |
| T47612 | yb15h03.s1 Stratagene placenta (#937225) Homo sapiens cDNA clone IMAGE:71285 3, mRNA sequence | 5.02 | UP | 7.57E-03 |
| NM_002402 | Homo sapiens mesoderm specific transcript homolog (mouse) (MEST), transcript variant 1, mRNA | 83.38 | Down | 1.30E-04 |
| NM_001884 | Homo sapiens hyaluronan and proteoglycan link protein 1 (HAPLN1), mRNA | 71.21 | Down | 5.97E-05 |
| NM 006439 | Homo sapiens mab-21-like 2 (C. elegans) (MAB21L2), mRNA | 70.58 | Down | 7.65E-05 |
| NM_001202 | Homo sapiens bone morphogenetic protein 4 (BMP4), transcript variant 1, mRNA | 50.48 | Down | 3.32E-05 |
| NM_017671 | Homo sapiens chromosome 20 open reading frame 42 (C20orf42), mRNA | 50.28 | Down | 4.04E-05 |
| AK124396 | Homo sapiens cDNA FLJ42405 fis, clone ASTRO3000474 | 45.55 | Down | 6.98E-05 |
| NM_015170 | Homo sapiens sulfatase 1 (SULF1), mRNA | 38.04 | Down | 7.58E-05 |
| BC037316 | Homo sapiens, clone IMAGE:5259432, mRNA | 34.94 | Down | 7.08E-05 |
| NM_001562 | Homo sapiens interleukin 18 (interferon-gamma-inducing factor) (IL18), mRNA | 34.48 | Down | 7.58E-05 |
| AI469032 | ti70a01.x1 NCI_CGAP_Kid11 Homo sapiens cDNA clone IMAGE:2137320 3, mRNA sequence | 28.72 | Down | 4.44E-05 |
| NM_016307 | Homo sapiens paired related homeobox 2 (PRRX2), mRNA | 28.57 | Down | 3.32E-05 |
| NM_022475 | Homo sapiens hedgehog interacting protein (HHIP), mRNA | 27 | Down | 7.14E-05 |
| NM_006072 | Homo sapiens chemokine (C-C motif) ligand 26 (CCL26), mRNA | 25.57 | Down | 5.83E-05 |
| NM_007361 | Homo sapiens nidogen 2 (osteonidogen) (NID2), mRNA | 22.81 | Down | 5.24E-05 |
| NM_006228 | Homo sapiens prepronociceptin (PNOC), mRNA | 21.34 | Down | 4.20E-04 |
| N95448 | zb81e11.s1 Soares_senescent_fibroblasts_NbHSF Homo sapiens cDNA clone IMAGE:310028 3, mRNA sequence | 20.92 | Down | 1.24E-04 |
| NM_018242 | Homo sapiens hypothetical protein FLJ10847 (FLJ10847), mRNA | 20.83 | Down | 6.60E-05 |
| N63415 | yy60d04.s1 Soares_multiple_sclerosis_2NbHMSP Homo sapiens cDNA clone IMAGE:277927 3 similar to contains L1.b3 L1 repetitive element ;, mRNA sequence | 19.2 | Down | 5.24E-05 |
| BQ001571 | UI-H-DH1-awr-i-18-0-UI.s1 NCI_CGAP _DH1 Homo sapiens cDNA clone IMAGE:5893337 3, mRNA sequence | 18.75 | Down | 6.98E-05 |
| NM_022454 | Homo sapiens SRY (sex determining region Y)-box 17 (SOX17), mRNA | 18.21 | Down | 5.83E-05 |
| BE788763 | 601475864F1 NIH_MGC_68 Homo sapiens cDNA clone IMAGE:3879014 5, mRNA sequence | 17.84 | Down | 1.13E-04 |
| BU633163 | UI-H-FL1-bgt-n-07-0-UI.s1 NCI-CGAP_FL1 Homo sapiens cDNA clone UI-H-FL1-bgt-n-07-0-UI 3, mRNA sequence | 17.41 | Down | 7.14E-05 |
| AI289329 | qw28c09.x1 NCI_CGAP_Ut4 Homo sapiens cDNA clone IMAGE:1992400 3 similar to contains L1.b2 L1 repetitive element ;, mRNA sequence | 17.1 | Down | 6.98E-05 |
| AF318382 | Homo sapiens pp9974 mRNA, complete cds | 16.45 | Down | 1.38E-04 |
| NM_022350 | Homo sapiens leukocyte-derived arginine aminopeptidase (LRAP), mRNA | 15.8 | Down | 1.10E-04 |
| BI561641 | 603256058F1 NIH_MGC_97 Homo sapiens cDNA clone IMAGE:5298374 5, mRNA sequence | 14.77 | Down | 1.94E-04 |
| NM_006350 | Homo sapiens follistatin (FST), transcript variant FST317, mRNA | 14.55 | Down | 7.14E-05 |
| NM_016588 | Homo sapiens neuritin 1 (NRN1), mRNA | 14.52 | Down | 6.98E-05 |
| CA437861 | UI-H-DH0-aur-k-12-0-UI.s1 NCI_CGAP_DH0 Homo sapiens cDNA clone UI-H-DH0-aur-k-12-0-UI 3, mRNA sequence | 14.45 | Down | 1.21E-04 |
| NM_020873 | Homo sapiens leucine rich repeat neuronal 1 (LRRN1), mRNA | 13.74 | Down | 6.60E-05 |
| NM_003385 | Homo sapiens visinin-like 1 (VSNL1), mRNA | 13.06 | Down | 7.75E-05 |
| AI640484 | wa27f01.x1 NCI_CGAP_Kid11 Homo sapiens cDNA clone IMAGE:2299321 3, mRNA sequence | 11.92 | Down | 5.48E-04 |
| AK127309 | Homo sapiens cDNA FLJ45377 fis, clone BRHIP3019956 | 11.87 | Down | 2.35E-04 |
| M60502 | Human profilaggrin mRNA, 3 end | 11.74 | Down | 7.58E-05 |
| NM_006475 | Homo sapiens periostin, osteoblast specific factor (POSTN), mRNA | 11.74 | Down | 1.19E-04 |
| NM_207482 | Homo sapiens FLJ44048 protein (FLJ44048), mRNA | 11.63 | Down | 1.45E-04 |
| NM_000519 | Homo sapiens hemoglobin, delta (HBD), mRNA | 11.61 | Down | 3.27E-04 |
| BU567804 | AGENCOURT_10398872 NIH_MGC_82 Homo sapiens cDNA clone IMAGE:6614502 5, mRNA sequence | 11.59 | Down | 5.24E-05 |
| AJ318805 | AJ318805 Homo sapiens adipose tissue Homo sapiens cDNA clone 2040, mRNA sequence | 11.59 | Down | 1.76E-04 |
| AW451831 | UI-H-BI3-alk-e-12-0-UI.s1 NCI_CGAP_Sub5 Homo sapiens cDNA clone IMAGE:2737246 3, mRNA sequence | 11.57 | Down | 1.46E-04 |
| R99527 | yq79b11.s1 Soares fetal liver spleen 1 NFLS Homo sapiens cDNA clone IMAGE:201981 3, mRNA sequence | 11.16 | Down | 1.13E-04 |
| NM_031894 | Homo sapiens ferritin, heavy polypeptide-like 17 (FTHL17), mRNA | 11.12 | Down | 4.85E-04 |
| NM_007072 | Homo sapiens HERV-H LTR-associating 2 (HHLA2), mRNA | 10.94 | Down | 1.36E-04 |
| NM_005654 | Homo sapiens nuclear receptor subfamily 2, group F, member 1 (NR2F1), mRNA | 10.91 | Down | 3.32E-05 |
| NM_004574 | Homo sapiens peanut-like 2 (Drosophila) (PNUTL2), transcript variant 1, mRNA | 10.46 | Down | 3.48E-04 |
| NM_005141 | Homo sapiens fibrinogen, B beta polypeptide (FGB), mRNA | 10.37 | Down | 4.85E-04 |
| AI905628 | CM-BT094-050299-147 BT094 Homo sapiens cDNA, mRNA sequence | 10.36 | Down | 1.49E-04 |
| BG622707 | 602647476F1 NIH_MGC_79 Homo sapiens cDNA clone IMAGE:4768963 5, mRNA sequence | 10.35 | Down | 1.73E-04 |
| W76003 | zd58g07.r1 Soares_fetal_heart_NbHH19W Homo sapiens cDNA clone IMAGE:344892 5, mRNA sequence | 10.32 | Down | 1.58E-04 |
| H15096 | ym29e11.r1 Soares infant brain 1NIB Homo sapiens cDNA clone IMAGE:49250 5, mRNA sequence | 10.23 | Down | 1.80E-04 |
| AI493349 | tg70f04.x1 Soares_NhHMPu_S1 Homo sapiens cDNA clone IMAGE:2114143 3, mRNA sequence | 9.84 | Down | 7.54E-05 |
| AK093762 | Homo sapiens cDNA FLJ36443 fis, clone THYMU2012891 | 9.76 | Down | 8.07E-04 |
| BU570253 | AGENCOURT_10401698 NIH_MGC_82 Homo sapiens cDNA clone IMAGE:6618451 5, mRNA sequence | 9.71 | Down | 3.65E-04 |
| NM_005855 | Homo sapiens receptor (calcitonin) activity modifying protein 1 (RAMP1), mRNA | 9.33 | Down | 7.08E-05 |
| NM_003328 | Homo sapiens TXK tyrosine kinase (TXK), mRNA | 9.29 | Down | 1.19E-04 |
| NM_003004 | Homo sapiens secreted and transmembrane 1 (SECTM1), mRNA | 9.23 | Down | 1.76E-04 |
| NM_005596 | Homo sapiens nuclear factor I/B (NFIB), mRNA | 9.23 | Down | 4.97E-05 |
| BC039450 | Homo sapiens, clone IMAGE:5311619, mRNA | 9.07 | Down | 1.13E-04 |
| NM_005613 | Homo sapiens regulator of G-protein signalling 4 (RGS4), mRNA | 8.99 | Down | 7.65E-05 |
| NM_003973 | Homo sapiens ribosomal protein L14 (RPL14), mRNA | 8.98 | Down | 6.60E-05 |
| BM675270 | UI-E-EJ0-ahr-j-07-0-UI.s1 UI-E-EJ0 Homo sapiens cDNA clone UI-E-EJ0-ahr-j-07-0-UI 3, mRNA sequence | 8.96 | Down | 1.34E-03 |
| NM_001311 | Homo sapiens cysteine-rich protein 1 (intestinal) (CRIP1), mRNA | 8.91 | Down | 7.14E-05 |
| NM_000087 | Homo sapiens cyclic nucleotide gated channel alpha 1 (CNGA1 mRNA | 8.76 | Down | 1.33E-03 |
| AW021686 | df26h11.y1 Morton Fetal Cochlea Homo sapiens cDNA clone IMAGE:2484717 5, mRNA sequence | 8.74 | Down | 4.04E-05 |
| NM_002031 | Homo sapiens fyn-related kinase (FRK), mRNA | 8.67 | Down | 1.14E-04 |
| NM_005279 | Homo sapiens G protein-coupled receptor 1 (GPR1), mRNA | 8.51 | Down | 4.16E-04 |
| NM_004460 | Homo sapiens fibroblast activation protein, alpha (FAP), mRNA | 8.51 | Down | 7.71E-04 |
| BE904671 | 601498784F1 NIH_MGC_70 Homo sapiens cDNA clone IMAGE:3900717 5, mRNA sequence | 8.46 | Down | 1.73E-04 |
| NM_000612 | Homo sapiens insulin-like growth factor 2 (somatomedin A) (IGF2), mRNA | 8.27 | Down | 1.19E-04 |
| AL137488 | Homo sapiens mRNA; cDNA DKFZp434N2030 (from clone DKFZp434N2030) | 8.23 | Down | 1.13E-04 |
| NM_006288 | Homo sapiens Thy-1 cell surface antigen (THY1), mRNA | 8.18 | Down | 1.43E-03 |
| NM_002837 | Homo sapiens protein tyrosine phosphatase, receptor type, B (PTPRB), mRNA | 8.15 | Down | 1.19E-03 |
| NM_030899 | Homo sapiens zinc finger protein 323 (ZNF323), mRNA | 8.08 | Down | 9.54E-05 |
| NM_002193 | Homo sapiens inhibin, beta B (activin AB beta polypeptide) (INHBB), mRNA | 8.07 | Down | 1.90E-04 |
| BX537698 | Homo sapiens mRNA; cDNA DKFZp686F09166 (from clone DKFZp686F09166) | 8.06 | Down | 1.34E-03 |
| BC033567 | Homo sapiens, clone IMAGE:4822266, mRNA | 8.04 | Down | 1.88E-04 |
| NM_052997 | Homo sapiens ankyrin repeat domain 30A (ANKRD30A), mRNA | 7.97 | Down | 1.42E-03 |
| NM_178550 | Homo sapiens hypothetical protein MGC48998 (MGC48998), mRNA | 7.82 | Down | 5.76E-04 |
| NM_178033 | Homo sapiens cytochrome P450, family 4, subfamily X, polypeptide 1 (CYP4X1), mRNA | 7.81 | Down | 8.00E-04 |
| NM_002924 | Homo sapiens regulator of G-protein signalling 7 (RGS7), mRNA | 7.8 | Down | 5.38E-04 |
| NM_012242 | Homo sapiens dickkopf homolog 1 (Xenopus laevis) (DKK1), mRNA | 7.77 | Down | 1.24E-04 |
| AA740671 | ob01h05.s1 NCI_CGAP_Kid3 Homo sapiens cDNA clone IMAGE:1322457 3, mRNA sequence | 7.74 | Down | 2.22E-03 |
| NM_005712 | Homo sapiens HERV-H LTR-associating 1 (HHLA1), mRNA | 7.67 | Down | 1.80E-04 |
| BC040293 | Homo sapiens, clone IMAGE:4820330, mRNA | 7.65 | Down | 3.10E-04 |
| NM_000089 | Homo sapiens collagen, type I, alpha 2 (COL1A2), mRNA | 7.64 | Down | 1.17E-03 |
| NM_005654 | Homo sapiens nuclear receptor subfamily 2, group F, member 1 (NR2F1), mRNA | 7.63 | Down | 2.73E-04 |
| BE465134 | hv75e11.x1 NCI_CGAP_Lu24 Homo sapiens cDNA clone IMAGE:3179276 3, mRNA sequence | 7.53 | Down | 1.28E-03 |
| NM_002522 | Homo sapiens neuronal pentraxin I (NPTX1), mRNA | 7.49 | Down | 6.53E-04 |
| AK093529 | Homo sapiens cDNA FLJ36210 fis, clone THYMU2000155 | 7.47 | Down | 1.18E-04 |
| NM_003662 | Homo sapiens pirin (iron-binding nuclear protein) (PIR), mRNA | 7.29 | Down | 6.96E-05 |
| NM_001769 | Homo sapiens CD9 antigen (p24) (CD9), mRNA | 7.23 | Down | 1.80E-04 |
| AL359567 | Homo sapiens mRNA; cDNA DKFZp547D023 (from clone DKFZp547D023) | 7.21 | Down | 1.24E-04 |
| BX647313 | Homo sapiens mRNA; cDNA DKFZp686N1593 (from clone DKFZp686N1593) | 7.19 | Down | 1.19E-04 |
| NM_003979 | Homo sapiens G protein-coupled receptor, family C, group 5, member A (GPCR5A), mRNA | 7.18 | Down | 1.63E-03 |
| AI365141 | qx96e07.x1 NCI_CGAP_GC6 Homo sapiens cDNA clone IMAGE:2010372 3, mRNA sequence | 7.04 | Down | 3.01E-04 |
| CA948963 | iq30f05.y1 HR85 islet Homo sapiens cDNA clone IMAGE: 5, mRNA sequence | 7.03 | Down | 8.55E-04 |
| NM_002977 | Homo sapiens sodium channel, voltage-gated, type IX, alpha (SCN9A), mRNA | 6.99 | Down | 5.83E-05 |
| BC033124 | Homo sapiens, clone IMAGE:2960615, mRNA | 6.86 | Down | 6.60E-05 |
| NM_003385 | Homo sapiens visinin-like 1 (VSNL1), mRNA | 6.73 | Down | 1.26E-04 |
| NM_013262 | Homo sapiens myosin regulatory light chain interacting protein (MYLIP), mRNA | 6.65 | Down | 1.66E-04 |
| NM_001843 | Homo sapiens contactin 1 (CNTN1), transcript variant 1, mRNA | 6.64 | Down | 3.37E-03 |
| NM_006727 | Homo sapiens cadherin 10, type 2 (T2-cadherin) (CDH10), mRNA | 6.59 | Down | 1.91E-03 |
| NM_016192 | Homo sapiens transmembrane protein with EGF-like and two follistatin-like domains 2 (TMEFF2), mRNA | 6.58 | Down | 9.19E-05 |
| NM_031847 | Homo sapiens microtubule-associated protein 2 (MAP2), transcript variant 4, mRNA | 6.57 | Down | 1.13E-03 |
| CB115754 | K-EST0159876 L8SCK0 Homo sapiens cDNA clone L8SCK0-8-H08 5, mRNA sequence | 6.55 | Down | 8.55E-04 |
| NM_025074 | Homo sapiens Fraser syndrome 1 (FRAS1), transcript variant 1, mRNA | 6.52 | Down | 4.97E-05 |
| NM_016206 | Homo sapiens colon carcinoma related protein (FLJ38507), mRNA | 6.45 | Down | 5.30E-04 |
| BC015159 | Homo sapiens cDNA clone IMAGE:3885734, partial cds | 6.35 | Down | 3.01E-04 |
| NM_005613 | Homo sapiens regulator of G-protein signalling 4 (RGS4), mRNA | 6.33 | Down | 1.62E-03 |
| W88428 | zh72g09.s1 Soares_fetal_liver_spleen_1NFLS_S1 Homo sapiens cDNA clone IMAGE:417664 3, mRNA sequence | 6.28 | Down | 7.53E-04 |
| NM_058187 | Homo sapiens chromosome 21 open reading frame 63 (C21orf63), mRNA | 6.16 | Down | 5.39E-03 |
| NM_020836 | Homo sapiens brain-enriched guanylate kinase-associated protein (KIAA1446), mRNA | 6.15 | Down | 3.75E-03 |
| BX107838 | BX107838 NCI_CGAP_Lu5 Homo sapiens cDNA clone IMAGp998A153853 ; IMAGE:1521686, mRNA sequence | 6.14 | Down | 1.77E-03 |
| NM_004496 | Homo sapiens forkhead box A1 (FOXA1), mRNA | 6.13 | Down | 1.94E-04 |
| NM_021637 | Homo sapiens transmembrane protein 35 (TMEM35), mRNA | 6.1 | Down | 2.12E-03 |
| NM_002521 | Homo sapiens natriuretic peptide precursor B (NPPB), mRNA | 6.1 | Down | 4.86E-04 |
| BF431041 | nab31g02.x1 Soares_NSF_F8_9W_OT_PA_P_S1 Homo sapiens cDNA clone IMAGE:3267627 3, mRNA sequence | 6.08 | Down | 7.63E-05 |
| NM_003713 | Homo sapiens phosphatidic acid phosphatase type 2B (PPAP2B), transcript variant 1, mRNA | 6.05 | Down | 6.44E-04 |
| AV728294 | AV728294 HTC Homo sapiens cDNA clone HTCBIE09 5, mRNA sequence | 6.03 | Down | 7.71E-05 |
| NM_002148 | Homo sapiens homeo box D10 (HOXD10), mRNA | 6.01 | Down | 7.14E-05 |
| NM_176891 | Homo sapiens interferon epsilon 1 (IFNE1), mRNA | 6 | Down | 7.48E-04 |
| NM_002317 | Homo sapiens lysyl oxidase (LOX), mRNA | 5.99 | Down | 3.17E-04 |
| NM_016179 | Homo sapiens transient receptor potential cation channel, subfamily C, member 4 (TRPC4), mRNA | 5.99 | Down | 9.19E-05 |
| AW134473 | UI-H-BI1-abv-a-11-0-Ul.s1 NCI_CGAP_Sub3 Homo sapiens cDNA clone IMAGE:2712885 3, mRNA sequence | 5.98 | Down | 7.04E-05 |
| NM_005130 | Homo sapiens fibroblast growth factor binding protein 1 (FGFBP1), mRNA | 5.95 | Down | 5.89E-04 |
| AW512111 | xx70e05.x1 NCI_CGAP_Lym12 Homo sapiens cDNA clone IMAGE:2849024 3 similar to contains Alu repetitive element;, mRNA sequence | 5.93 | Down | 4.97E-05 |
| NM_020349 | Homo sapiens ankyrin repeat domain 2 (stretch responsive muscle) (ANKRD2), mRNA | 5.91 | Down | 1.18E-04 |
| NM_001083 | Homo sapiens phosphodiesterase 5A, cGMP-specific (PDE5A), transcript variant 1, mRNA | 5.88 | Down | 3.74E-03 |
| H94320 | yv18b10.s1 Soares fetal liver spleen 1 NFLS Homo sapiens cDNA clone IMAGE:243067 3, mRNA sequence | 5.83 | Down | 3.18E-02 |
| NM_032461 | Homo sapiens SPANX family, member B1 (SPANXB1), mRNA | 5.8 | Down | 1.08E-03 |
| BM994473 | Ul-H-DH0-aul-1-18-0-Ul.s1 NCI_CGAP_DHO Homo sapiens cDNA clone IMAGE:5871137 3, mRNA sequence | 5.79 | Down | 1.10E-04 |
| BE540906 | 601063027F1 NIH_MGC_10 Homo sapiens cDNA clone IMAGE:3449455 5, mRNA sequence | 5.79 | Down | 4.45E-05 |
| NM_033255 | Homo sapiens epithelial stromal interaction 1 (breast) (EPSTI1), mRNA | 5.75 | Down | 8.55E-04 |
| BU616268 | UI-H-DF0-bex-n-12-0-UI.s1 NCI_CGAP_DF0 Homo sapiens cDNA clone UI-H-DF0-bex-n-12-0-UI 3, mRNA sequence | 5.73 | Down | 6.74E-04 |
| BX112170 | BX112170 Soares_fetal_heart_NbHH19W Homo sapiens cDNA clone IMAGp998O07742 ; IMAGE:327390, mRNA sequence | 5.73 | Down | 7.32E-04 |
| BC015108 | Homo sapiens, Similar to otoconin 90, clone IMAGE:4044247, mRNA | 5.72 | Down | 2.40E-03 |
| NM_000576 | Homo sapiens interleukin 1, beta (IL1B), mRNA | 5.7 | Down | 2.97E-03 |
| AW470868 | ha34h03.x1 NCI_CGAP_Kid12 Homo sapiens cDNA clone IMAGE:2875637 3, mRNA sequence | 5.7 | Down | 2.31E-03 |
| AK093069 | Homo sapiens cDNA FLJ35750 fis, clone TEST12004539, weakly similar to Homo sapiens adlican mRNA | 5.7 | Down | 1.11E-03 |
| NM_032330 | Homo sapiens calpain, small subunit 2 (CAPNS2), mRNA | 5.68 | Down | 4.44E-03 |
| U51694 | HSU51694 Human normal gingiva Homo sapiens cDNA, mRNA sequence | 5.62 | Down | 1.02E-04 |
| NM_004335 | Homo sapiens bone marrow stromal cell antigen 2 (BST2), mRNA | 5.61 | Down | 2.84E-03 |
| BF431460 | 7o14b05.x1 NCI_CGAP_Kid11 Homo sapiens cDNA clone IMAGE:3573849 3, mRNA sequence | 5.59 | Down | 7.53E-04 |
| BC046362 | Homo sapiens voltage-dependent calcium channel gamma subunit-like protein, mRNA (cDNA clone MGC:50757 IMAGE:5221396), complete cds | 5.59 | Down | 8.07E-04 |
| NM 000961 | Homo sapiens prostaglandin I2 (prostacyclin) synthase (PTGIS), mRNA | 5.57 | Down | 1.01E-03 |
| AK022877 | Homo sapiens cDNA FLJ12815 fis, clone NT2RP2002546 | 5.56 | Down | 4.04E-05 |
| NM_002277 | Homo sapiens keratin, hair, acidic, 1 (KRTHA1), mRNA | 5.51 | Down | 3.04E-04 |
| AK092245 | Homo sapiens cDNA FLJ34926 fis, clone NT2RP7003319, highly similar to Mus musculus neuralin mRNA | 5.49 | Down | 1.40E-03 |
| NM_002427 | Homo sapiens matrix metalloproteinase 13 (collagenase 3) (MMP13), mRNA | 5.47 | Down | 4.45E-04 |
| BM991890 | UI-H-DF1-auk-h-02-0-UI.s1 NCI_CGAP_DF1 Homo sapiens cDNA clone IMAGE:5870641 3, mRNA sequence | 5.39 | Down | 7.53E-04 |
| NM_002575 | Homo sapiens serine (or cysteine) proteinase inhibitor, clade B (ovalbumin), member 2 (SERPINB2), mRNA | 5.39 | Down | 9.60E-03 |
| NM_000782 | Homo sapiens cytochrome P450, family 24, subfamily A, polypeptide 1 (CYP24A1), nuclear gene encoding mitochondrial protein, mRNA | 5.36 | Down | 1.31E-02 |
| NM_016276 | Homo sapiens serum/glucocorticoid regulated kinase 2 (SGK2), transcript variant 2, mRNA | 5.34 | Down | 6.73E-04 |
| AK024689 | Homo sapiens cDNA: FLJ21036 fis, clone CAE09578 | 5.32 | Down | 1.36E-04 |
| N78829 | zb17a05.s1 Soares_fetal_lung_NbHL 19W Homo sapiens cDNA clone IMAGE:302288 3, mRNA sequence | 5.31 | Down | 6.00E-03 |
| AL080103 | Homo sapiens mRNA; cDNA DKFZp564N2216 (from clone DKFZp564N2216) | 5.26 | Down | 1.61E-04 |
| BC035400 | Homo sapiens, clone IMAGE:4822830, mRNA | 5.17 | Down | 1.13E-02 |
| AK056725 | Homo sapiens cDNA FLJ32163 fis, clone PLACE6000371 | 5.13 | Down | 3.72E-04 |
| AK025595 | Homo sapiens cDNA: FLJ21942 fis, clone HEP04527 | 5.12 | Down | 1.56E-02 |
| NM_015236 | Homo sapiens latrophilin 3 (LPHN3), mRNA | 5.11 | Down | 3.14E-04 |
| NM_012411 | Homo sapiens protein tyrosine phosphatase, non-receptor type 22 (lymphoid) (PTPN22), transcript variant 2, mRNA | 5.1 | Down | 2.78E-03 |
| AI190760 | qd61c05.x1 Soares_testis_NHT Homo sapiens cDNA clone IMAGE:1733960 3, mRNA sequence | 5.08 | Down | 2.24E-03 |
| NM_139241 | Homo sapiens FYVE, RhoGEF and PH domain containing 4 (FGD4), mRNA | 5.04 | Down | 6.92E-03 |
| NM_033066 | Homo sapiens membrane protein, palmitoylated 4 (MAGUK p55 subfamily member 4) (MPP4), mRNA | 5.04 | Down | 7.11E-03 |
| BG573337 | 602595107F1 NIH_MGC_79 Homo sapiens cDNA clone IMAGE:4724521 5, mRNA sequence | 5.03 | Down | 5.60E-03 |
| AA158235 | zo76b02.s1 Stratagene pancreas (#937208) Homo sapiens cDNA clone IMAGE:592779 3, mRNA sequence | 5.01 | Down | 7.10E-03 |
| BX092004 | BX092004 NCI_CGAP_Kid11 Homo sapiens cDNA clone IMAGp998B195924 ; IMAGE:2385330, mRNA sequence | 5.01 | Down | 1.69E-02 |
| AK058040 | Homo sapiens cDNA FLJ25311 fis, clone SYN01066 | 5 | Down | 1.76E-03 |

**TABLE VII D: GENES THAT WERE DIFFERENTIALLY EXPRESSED AT LEAST 5 FOLD IN AF-I VERSUS AF-I AT TERM CELLS**

| Gene Identifier | Gene Name | Average fold change in AF cells isolated from 2nd trimester amniotic fluid versus AF cells islolated at term | Direction | adj. p-value |
|---|---|---|---|---|
| NM_006228 | Homo sapiens prepronociceptin (PNOC), mRNA | 64.08 | UP | 1.14E-04 |
| NM_006439 | Homo sapiens mab-21-like 2 (C. elegans) (MAB21L2), mRNA | 47.74 | UP | 1.03E-04 |
| AK092245 | Homo sapiens cDNA FLJ34926 fis, clone NT2RP7003319, highly similar to Mus musculus neuralin mRNA | 44.83 | UP | 8.97E-05 |
| AK056431 | Homo sapiens cDNA FLJ31869 fis, clone NT2RP7002151 | 35.22 | UP | 5.97E-05 |
| NM_015170 | Homo sapiens sulfatase 1 (SULF1), mRNA | 30.43 | UP | 1.53E-04 |
| NM_001884 | Homo sapiens hyaluronan and proteoglycan link protein 1 (HAPLN1), mRNA | 27.82 | UP | 3.92E-05 |
| NM_002487 | Homo sapiens necdin homolog (mouse) (NDN), mRNA | 25.93 | UP | 2.07E-04 |
| BC037316 | Homo sapiens, clone IMAGE:5259432, mRNA | 25.16 | UP | 5.97E-05 |
| NM_020873 | Homo sapiens leucine rich repeat neuronal 1 (LRRN1), mRNA | 24.74 | UP | 6.46E-05 |
| H89053 | yw24c06.r1 Morton Fetal Cochlea Homo sapiens cDNA clone IMAGE:253162 5, mRNA sequence | 23.95 | UP | 6.63E-05 |
| NM_002148 | Homo sapiens homeo box D10 (HOXD10), mRNA | 20.48 | UP | 8.97E-05 |
| NM_014421 | Homo sapiens dickkopf homolog 2 (Xenopus laevis) (DKK2), mRNA | 20.41 | UP | 6.46E-05 |
| NM_016212 | Homo sapiens TP53TG3 protein (TP53TG3), mRNA | 20.08 | UP | 5.97E-05 |
| NM_080743 | Homo sapiens serine-arginine repressor protein (35 kDa) (SRrp35), mRNA | 19.58 | UP | 6.46E-05 |
| NM_000170 | Homo sapiens glycine dehydrogenase (decarboxylating; glycine decarboxylase, glycine cleavage system protein P) (GLDC), mRNA | 18.22 | UP | 9.61E-05 |
| BX647313 | Homo sapiens mRNA; cDNA DKFZp686N1593 (from clone DKFZp686N1593) | 17.47 | UP | 6.46E-05 |
| NM_007361 | Homo sapiens nidogen 2 (osteonidogen) (NID2), mRNA | 17.41 | UP | 9.75E-05 |
| NM_007072 | Homo sapiens HERV-H LTR-associating 2 (HHLA2), mRNA | 16.06 | UP | 9.61E-05 |
| NM_152359 | Homo sapiens carnitine palmitoyltransferase 1C (CPT1C), mRNA | 15.65 | UP | 2.26E-04 |
| NM_002899 | Homo sapiens retinol binding protein 1, cellular (RBP1), mRNA | 15.47 | UP | 1.40E-04 |
| NM_005531 | Homo sapiens interferon, gamma-inducible protein 16 (IFI16), mRNA | 15.18 | UP | 1.67E-04 |
| NM_021192 | Homo sapiens homeo box D11 (HOXD11), mRNA | 15.05 | UP | 1.11 E-04 |
| NM_016307 | Homo sapiens paired related homeobox 2 (PRRX2), mRNA | 14.33 | UP | 5.97E-05 |
| AA738254 | nx13b02.s1 NCI_CGAP_GC3 Homo sapiens cDNA clone IMAGE:1255947 3, mRNA sequence | 14.27 | UP | 1.64E-05 |
| BE788763 | 601475864F1 NIH_MGC_68 Homo sapiens cDNA clone IMAGE:3879014 5, mRNA sequence | 14.17 | UP | 3.92E-05 |
| NM_030627 | Homo sapiens cytoplasmic polyadenylation element binding protein 4 (CPEB4), mRNA | 14.01 | UP | 1.40E-04 |
| | BX113319 NCI_CGAP_Gas4 Homo sapiens | 13.62 | UP | 5.09E-05 |
| BX113319 | cDNA clone IMAGp998G205398 ; IMAGE:2184619, mRNA sequence | | | |
| NM_025074 | Homo sapiens Fraser syndrome 1 (FRAS1), transcript variant 1, mRNA | 13.55 | UP | 5.09E-05 |
| NM_001311 | Homo sapiens cysteine-rich protein 1 (intestinal) (CRIP1), mRNA | 13 | UP | 8.97E-05 |
| NM_138394 | Homo sapiens hypothetical protein BC008217 (LOC92906), mRNA | 12.64 | UP | 2.09E-04 |
| BF431313 | 7o11d09.x1 NCI_CGAP_Kid11 Homo sapiens cDNA clone IMAGE:3573928 3, mRNA sequence | 12.04 | UP | 1.10E-04 |
| NM_133504 | Homo sapiens decorin (DCN), transcript variant B, mRNA | 11.74 | UP | 2.01E-04 |
| BU567804 | AGENCOURT_10398872 NIH_MGC_82 Homo sapiens cDNA clone IMAGE:6614502 5, mRNA sequence | 11.67 | UP | 5.09E-05 |
| CA437861 | UI-H-DH0-aur-k-12-0-UI.s1 NCI_CGAP_DH0 Homo sapiens cDNA clone UI-H-DH0-aur-k-12-0-UI 3, mRNA sequence | 10.89 | UP | 4.99E-05 |
| BF431041 | nab31g02.x1 Soares_NSF_F8_9W_OT_PA_P_S1 Homo sapiens cDNA clone IMAGE:3267627 3, mRNA sequence | 10.78 | UP | 3.92E-05 |
| NM_138966 | Homo sapiens neuropilin (NRP) and tolloid (TLL)-like 1 (NETO1), transcript variant 3, mRNA | 10.4 | UP | 8.97E-05 |
| | UI-H-FL1-bgt-n-07-0-UI.s1 NCI_CGAP_FL1 | 10.32 | UP | 6.63E-05 |
| BU633163 | Homo sapiens cDNA clone UI-H-FL1-bgt-n-07-0-UI 3, mRNA sequence | | | |
| M60502 | Human profilaggrin mRNA, 3 end | 10.26 | UP | 1.40E-04 |
| NM_177949 | Homo sapiens armadillo repeat containing, X-linked 2 (ARMCX2), mRNA | 9.9 | UP | 1.62E-04 |
| BG622707 | 602647476F1 NIH_MGC_79 Homo sapiens cDNA clone IMAGE:4768963 5, mRNA sequence | 9.78 | UP | 1.24E-04 |
| AL834140 | Homo sapiens mRNA; cDNA DKFZp434A2029 (from clone DKFZp434A2029) | 9.75 | UP | 1.34E-04 |
| NM_020353 | Homo sapiens phospholipid scramblase 4 (PLSCR4), mRNA | 9.71 | UP | 8.97E-05 |
| AI905628 | CM-BT094-050299-147 BT094 Homo sapiens cDNA, mRNA sequence | 9.65 | UP | 1.03E-04 |
| NM_005712 | Homo sapiens HERV-H LTR-associating 1 (HHLA1), mRNA | 9.52 | UP | 1.10E-04 |
| NM_003328 | Homo sapiens TXK tyrosine kinase (TXK), mRNA | 9.49 | UP | 2.35E-04 |
| AK095573 | Homo sapiens cDNA FLJ38254 fis, clone FCBBF3000847 | 9.39 | UP | 3.92E-05 |
| NM_003973 | Homo sapiens ribosomal protein L14 (RPL14), mRNA | 9.37 | UP | 9.74E-05 |
| AL137698 | Homo sapiens mRNA; cDNA DKFZp434C1915 (from clone DKFZp434C1915); partial cds | 9.32 | UP | 1.48E-03 |
| | Homo sapiens F-box protein 9 (FBXO9), | 9.29 | UP | 1.40E-04 |
| NM_033480 | transcript variant 2, mRNA | | | |
| NM_016588 | Homo sapiens neuritin 1 (NRN1), mRNA | 9.16 | UP | 4.09E-04 |
| AK127309 | Homo sapiens cDNA FLJ45377 fis, clone BRHIP3019956 | 9 | UP | 4.86E-04 |
| NM_207482 | Homo sapiens FLJ44048 protein (FLJ44048), mRNA | 8.93 | UP | 4.37E-04 |
| BE464407 | hx89g05.x1 NCI_CGAP_Kid11 Homo sapiens cDNA clone IMAGE:3195032 3, mRNA sequence | 8.92 | UP | 6.16E-04 |
| NM_178033 | Homo sapiens cytochrome P450, family 4, subfamily X, polypeptide 1 (CYP4X1), mRNA | 8.59 | UP | 1.35E-04 |
| NM_001854 | Homo sapiens collagen, type XI, alpha 1 (COL11A1), transcript variant A, mRNA | 8.58 | UP | 4.86E-04 |
| NM_005141 | Homo sapiens fibrinogen, B beta polypeptide (FGB), mRNA | 8.52 | UP | 5.39E-04 |
| NM_022454 | Homo sapiens SRY (sex determining region Y)-box 17 (SOX17), mRNA | 8.52 | UP | 6.46E-05 |
| AI651524 | wb06g07.x1 NCI_CGAP_GC6 Homo sapiens cDNA clone IMAGE:2304924 3, mRNA sequence | 8.5 | UP | 3.47E-04 |
| AI288404 | qv89b01.x1 NCI_CGAP_Ut2 Homo sapiens cDNA clone IMAGE:1988713 3, mRNA sequence | 8.22 | UP | 3.47E-04 |
| AL359058 | Homo sapiens mRNA full length insert cDNA clone EUROIMAGE 592473 | 8.12 | UP | 1.82E-04 |
| NM_002427 | Homo sapiens matrix metalloproteinase 13 (collagenase 3) (MMP13), mRNA | 7.92 | UP | 2.93E-04 |
| AJ318805 | AJ318805 Homo sapiens adipose tissue Homo sapiens cDNA clone 2040, mRNA sequence | 7.91 | UP | 1.03E-04 |
| BC033124 | Homo sapiens, clone IMAGE:2960615, mRNA | 7.83 | UP | 6.63E-05 |
| BI561641 | 603256058F1 NIH_MGC_97 Homo sapiens cDNA clone IMAGE:5298374 5, mRNA sequence | 7.82 | UP | 1.18E-03 |
| AK054960 | Homo sapiens cDNA FLJ30398 fis, clone BRACE2008402, highly similar to Homo sapiens steroid receptor RNA activator isoform 3 mRNA | 7.67 | UP | 1.12E-03 |
| NM_174900 | Homo sapiens zinc finger protein 42 (ZFP42), mRNA | 7.64 | UP | 1.40E-04 |
| NM_007099 | Homo sapiens acid phosphatase 1, soluble (ACP1), transcript variant 2, mRNA | 7.6 | UP | 1.36E-03 |
| BC015108 | Homo sapiens, Similar to otoconin 90, clone IMAGE:4044247, mRNA | 7.54 | UP | 6.63E-05 |
| AI640484 | wa27f01.x1 NCI_CGAP_Kid11 Homo sapiens cDNA clone IMAGE:2299321 3, mRNA sequence | 7.49 | UP | 1.03E-03 |
| AK001099 | Homo sapiens cDNA FLJ10237 fis, clone HEMBB1000438 | 7.49 | UP | 9.13E-04 |
| | Homo sapiens mRNA for KIAA1211 protein, | 7.34 | UP | 7.16E-05 |
| AB033037 | partial cds | | | |
| BC033567 | Homo sapiens, clone IMAGE:4822266, mRNA | 7.29 | UP | 1.67E-04 |
| NM_001144 | Homo sapiens autocrine motility factor receptor (AMFR), transcript variant 1, mRNA | 7.23 | UP | 3.06E-04 |
| AI792194 | ov03c02.y5 NCI_CGAP_Kid3 Homo sapiens cDNA clone IMAGE:1636226 5, mRNA sequence | 7.23 | UP | 4.37E-04 |
| AI939297 | oy50g08.x5 NCI_CGAP_Brn23 Homo sapiens cDNA clone IMAGE:1669310 3, mRNA sequence | 7.21 | UP | 1.87E-04 |
| BF512544 | UI-H-BW1-amf-c-08-0-ULs1 NCI_CGAP_Sub7 Homo sapiens cDNA clone IMAGE:3069687 3, mRNA sequence | 7.17 | UP | 8.19E-05 |
| NM_005654 | Homo sapiens nuclear receptor subfamily 2, group F, member 1 (NR2F1), mRNA | 7.09 | UP | 6.63E-05 |
| H94320 | yv18b10.s1 Soares fetal liver spleen 1NFLS Homo sapiens cDNA clone IMAGE:243067 3, mRNA sequence | 6.65 | UP | 2.20E-03 |
| NM_000557 | Homo sapiens growth differentiation factor 5 (cartilage-derived morphogenetic protein-1) (GDF5), mRNA | 6.5 | UP | 1.74E-03 |
| H15096 | ym29e11.r1 Soares infant brain 1 NIB Homo sapiens cDNA clone IMAGE:49250 5, mRNA sequence | 6.48 | UP | 5.09E-05 |
| NM_002515 | Homo sapiens neuro-oncological ventral antigen 1 (NOVA1), transcript variant 1, mRNA | 6.25 | UP | 5.77E-05 |
| NM_005130 | Homo sapiens fibroblast growth factor binding protein 1 (FGFBP1), mRNA | 6.13 | UP | 1.03E-04 |
| BM675270 | UI-E-EJ0-ahr-j-07-0-UI.s1 UI-E-EJ0 Homo sapiens cDNA clone UI-E-EJ0-ahr-j-07-0-UI 3, mRNA sequence | 6.1 | UP | 1.23E-03 |
| AW268540 | xv51e10.x1 NCI_CGAP_Lu28 Homo sapiens cDNA clone IMAGE:2816682 3, mRNA sequence | 6.09 | UP | 1.09E-04 |
| NM_004390 | Homo sapiens cathepsin H (CTSH), transcript variant 1, mRNA | 6.03 | UP | 1.03E-04 |
| BM468332 | AGENCOURT_6432296 NIH_MGC_71 Homo sapiens cDNA clone IMAGE:5535773 5, mRNA sequence | 5.97 | UP | 7.63E-05 |
| NM_052997 | Homo sapiens ankyrin repeat domain 30A (ANKRD30A), mRNA | 5.87 | UP | 3.09E-03 |
| AK123319 | Homo sapiens cDNA FLJ41325 fis, clone BRAMY2046871 | 5.81 | UP | 3.81E-04 |
| NM_004235 | Homo sapiens Kruppel-like factor 4 (gut) (KLF4), mRNA | 5.77 | UP | 1.81E-04 |
| BC042976 | Homo sapiens cDNA clone IMAGE:5295023, partial cds | 5.76 | UP | 2.39E-03 |
| AA740671 | ob01h05.s1 NCI_CGAP_Kid3 Homo sapiens cDNA clone IMAGE:1322457 3, mRNA sequence | 5.61 | UP | 3.06E-03 |
| NM_000087 | Homo sapiens cyclic nucleotide gated channel alpha 1 (CNGA1), mRNA | 5.53 | UP | 2.39E-03 |
| NM_018476 | Homo sapiens brain expressed, X-linked 1 (BEX1), mRNA | 5.52 | UP | 1.07E-03 |
| BX107838 | BX107838 NCI_CGAP_Lu5 Homo sapiens cDNA clone IMAGp998A153853 ; IMAGE:1521686, mRNA | 5.41 | UP | 2.69E-03 |
| NM_145201 | Homo sapiens similar to CG3714 gene product (PP3856), mRNA | 5.36 | UP | 5.77E-05 |
| NM_005069 | Homo sapiens single-minded homolog 2 (Drosophila) (SIM2), transcript variant SIM2, mRNA | 5.34 | UP | 7.88E-05 |
| NM_004753 | Homo sapiens dehydrogenase/reductase (SDR family) member 3 (DHRS3), mRNA | 5.32 | UP | 4.71E-04 |
| NM_002338 | Homo sapiens limbic system-associated membrane protein (LSAMP), mRNA | 5.32 | UP | 6.88E-04 |
| NM_033518 | Homo sapiens solute carrier family 38, member 5 (SLC38A5), mRNA | 5.28 | UP | 3.14E-03 |
| BX117317 | BX117317 NCI_CGAP_Co3 Homo sapiens cDNA clone IMAGp998E242234 ; IMAGE:900095, mRNA sequence | 5.22 | UP | 1.36E-03 |
| CB115754 | K-EST0159876 L8SCK0 Homo sapiens cDNA clone L8SCK0-8-H08 5, mRNA sequence | 5.21 | UP | 1.22E-03 |
| AL359567 | Homo sapiens mRNA; cDNA DKFZp547D023 (from clone DKFZp547D023) | 5.18 | UP | 5.41E-04 |
| NM_020353 | Homo sapiens phospholipid scramblase 4 (PLSCR4), mRNA | 5.13 | UP | 1.36E-03 |
| NM_002515 | Homo sapiens neuro-oncological ventral antigen 1 (NOVA1), transcript variant 1, mRNA | 5.12 | UP | 9.13E-04 |
| U51694 | HSU51694 Human normal gingiva Homo sapiens cDNA, mRNA sequence | 5.11 | UP | 1.10E-04 |
| NM_006546 | Homo sapiens IGF-II mRNA-binding protein 1 (IMP-1), mRNA | 5.1 | UP | 2.50E-03 |
| BG573337 | 602595107F1 NIH_MGC_79 Homo sapiens cDNA clone IMAGE:4724521 5, mRNA sequence | 5.1 | UP | 5.54E-03 |
| NM_006727 | Homo sapiens cadherin 10, type 2 (T2-cadherin) (CDH10), mRNA | 5.01 | UP | 3.47E-03 |
| NM_014358 | Homo sapiens C-type (calcium dependent, carbohydrate-recognition domain) lectin, superfamily member 9 (CLECSF9), mRNA | 36.05 | Down | 3.68E-04 |
| NM_021013 | Homo sapiens keratin, hair, acidic, 4 (KRTHA4), mRNA | 33.84 | Down | 9.33E-04 |
| BM988338 | UI-H-DH0-asd-f-10-0-UI.s1 NCI_CGAP_DH0 Homo sapiens cDNA clone IMAGE:5857545 3, mRNA sequence | 31.23 | Down | 4.43E-03 |
| BC039369 | Homo sapiens, clone IMAGE:5271073, mRNA, partial cds | 25.01 | Down | 1.51E-03 |
| BE877764 | 601486331F1 NIH_MGC_69 Homo sapiens cDNA clone IMAGE:3888943 5, mRNA sequence | 24.69 | Down | 5.74E-03 |
| AW300043 | xs45a09.x1 NCI_CGAP_Kid11 Homo sapiens cDNA clone IMAGE:2772568 3, mRNA sequence | 21.86 | Down | 1.45E-02 |
| BQ025821 | UI-1-BB1p-aye-f-10-0-UI.s1 NCI_CGAP_PI6 Homo sapiens cDNA clone UI-1-BB1 p-aye-f-10-0-UI 3, mRNA sequence | 20.35 | Down | 7.39E-03 |
| BQ003501 | UI-H-EI1-azd-p-06-0-UI.s1 NCI_CGAP_EI1 Homo sapiens cDNA clone IMAGE:5847413 3, mRNA sequence | 19.16 | Down | 9.49E-04 |
| NM_013387 | Homo sapiens ubiquinol-cytochrome c reductase complex (7.2 kD) (HSPC051), transcript variant 1, mRNA | 18.22 | Down | 1.43E-04 |
| CA391258 | cs13a10.x1 Human Retinal pigment epithelium/choroid cDNA (Un-normalized, unamplified): cs Homo sapiens cDNA clone cs13a10 3, mRNA sequence | 17.83 | Down | 3.92E-05 |
| AV702977 | AV702977 ADB Homo sapiens cDNA clone ADBCVD08 5, mRNA sequence | 17.04 | Down | 1.41E-02 |
| NM_015714 | Homo sapiens putative lymphocyte G0/G1 switch gene (G0S2), mRNA | 15.4 | Down | 9.27E-03 |
| H85497 | yv88b07.r1 Soares melanocyte 2NbHM Homo sapiens cDNA clone IMAGE:249781 5, mRNA sequence | 14.88 | Down | 1.03E-04 |
| NM 014226 | Homo sapiens renal tumor antigen (RAGE), mRNA | 14.77 | Down | 2.43E-03 |
| AK023647 | Homo sapiens cDNA FLJ 13585 fis, clone PLACE 1009150 | 14.09 | Down | 1.22E-03 |
| AK128288 | Homo sapiens cDNA FLJ46426 fis, clone THYMU3013897 | 12.83 | Down | 2.34E-02 |
| NM_017852 | Homo sapiens NACHT, leucine rich repeat and PYD containing 2 (NALP2), mRNA | 12.69 | Down | 1.36E-03 |
| NM_024336 | Homo sapiens iroquois homeobox protein 3 (IRX3), mRNA | 12.29 | Down | 1.29E-02 |
| NM_004591 | Homo sapiens chemokine (C-C motif) ligand 20 (CCL20), mRNA | 11.11 | Down | 3.13E-02 |
| BF515657 | UI-H-BW1-anu-e-05-0-UI.s1 NCI_CGAP_Sub7 Homo sapiens cDNA clone IMAGE:3083601 3, mRNA sequence | 11.02 | Down | 1.26E-02 |
| W69644 | zd45f10.r1 Soares_fetal_heart_NbHH19W Homo sapiens cDNA clone IMAGE:343627 5, mRNA sequence | 10.54 | Down | 8.66E-03 |
| AL832916 | Homo sapiens mRNA; cDNA DKFZp762I0915 (from clone DKFZp762I0915) | 9.98 | Down | 1.94E-03 |
| NM_005739 | Homo sapiens RAS guanyl releasing protein 1 (calcium and DAG-regulated) (RASGRP1), mRNA | 9.82 | Down | 3.85E-03 |
| BF000009 | 7h15g04.x1 NCI_CGAP_Co16 Homo sapiens cDNA clone IMAGE:3316086 3, mRNA sequence | 9.64 | Down | 1.78E-02 |
| NM_012105 | Homo sapiens beta-site APP-cleaving enzyme 2 (BACE2), transcript variant a, mRNA | 9.3 | Down | 1.50E-04 |
| BU569937 | AGENCOURT_10399817 NIH_MGC_82 Homo sapiens cDNA clone IMAGE:6618011 5, mRNA sequence | 9.27 | Down | 3.61E-02 |
| NM_032782 | Homo sapiens hepatitis A virus cellular receptor 2 (HAVCR2), mRNA | 8.92 | Down | 6.61E-03 |
| NM_197955 | Homo sapiens normal mucosa of esophagus specific 1 (NMES1), transcript variant 1, mRNA | 8.82 | Down | 4.10E-03 |
| NM_003810 | Homo sapiens tumor necrosis factor (ligand) superfamily, member 10 (TNFSF10), mRNA | 8.08 | Down | 2.84E-04 |
| NM_014222 | Homo sapiens NADH dehydrogenase (ubiquinone) 1 alpha subcomplex, 8, 19kDa (NDUFA8), nuclear gene encoding mitochondrial protein, mRNA | 7.99 | Down | 1.59E-02 |
| NM_019119 | Homo sapiens protocadherin beta 9 (PCDHB9), mRNA | 7.85 | Down | 7.39E-03 |
| NM_014867 | Homo sapiens KIAA0711 gene product (KIAA0711), mRNA | 6.89 | Down | 9.61E-05 |
| BF509573 | UI-H-BI4-apf-b-11-0-UI.s1 NCI_CGAP_Sub8 Homo sapiens cDNA clone IMAGE:3086949 3, mRNA sequence | 6.85 | Down | 8.97E-05 |
| BQ012774 | UI-1-BC1p-auz-h-01-0-UI.s1 NCI_CGAP_PI3 Homo sapiens cDNA clone UI-1-BC1 p-auz-h-01-0-UI 3, mRNA sequence | 6.44 | Down | 5.30E-02 |
| NM_018836 | Homo sapiens transmembrane protein SHREW1 (SHREW1), mRNA | 6.32 | Down | 2.36E-02 |
| AW139066 | UI-H-BI1-adz-d-02-0-UI.s1 NCI_CGAP_Sub3 Homo sapiens cDNA clone IMAGE:2718387 3, mRNA sequence | 6.28 | Down | 1.03E-04 |
| NM_018076 | Homo sapiens armadillo repeat containing 4 (ARMC4), mRNA | 6.17 | Down | 2.20E-03 |
| BI759570 | 603046987F1 NIH_MGC_116 Homo sapiens cDNA clone IMAGE:5187285 5, mRNA sequence | 6.03 | Down | 5.06E-02 |
| NM_004496 | Homo sapiens forkhead box A1 (FOXA1), mRNA | 5.79 | Down | 1.03E-04 |
| NM_004473 | Homo sapiens forkhead box E1 (thyroid transcription factor 2) (FOXE1), mRNA | 5.48 | Down | 6.27E-05 |
| NM_182920 | Homo sapiens a disintegrin-like and metalloprotease (reprolysin type) with thrombospondin type 1 motif, 9 (ADAMTS9), transcript variant 1, mRNA | 5.35 | Down | 1.87E-04 |
| AI830524 | wh52c02.x1 NCI_CGAP_Kid11 Homo sapiens cDNA clone IMAGE:2384354 3, mRNA sequence | 5.34 | Down | 3.42E-02 |
| NM_004840 | Homo sapiens Rac/Cdc42 guanine nucleotide exchange factor (GEF) 6 (ARHGEF6), mRNA | 5.18 | Down | 1.41E-03 |
| AK026966 | Homo sapiens cDNA: FLJ23313 fis, clone HEP11919 | 5.09 | Down | 1.49E-04 |
| BC044843 | Homo sapiens hypothetical protein LOC339535, mRNA (cDNA clone IMAGE:5186761), partial cds | 5.03 | Down | 3.36E-02 |
| X02851 | Human mRNA for interleukin-1 precursor (pre IL-1) | 5.03 | Down | 8.60E-03 |

**TABLE VII E: GENES THAT WERE DIFFERENTIALLY EXPRESSED AT LEAST 5 FOLD IN AF-I VERSUS AF-II CELLS**

| Gene Identifier | Gene Name | Average fold change AF-I vs AF-II | Direction | adj. p-value |
|---|---|---|---|---|
| D52654 | HUM084D02B Clontech human fetal brain polyA+ mRNA (#6535) Homo sapiens cDNA clone GEN-084D02 5, mRNA sequence | 398.37 | Up | 8.54E-05 |
| NM_000090 | Homo sapiens collagen, type III, alpha 1 (Ehlers-Danlos syndrome type IV, autosomal dominant) (COL3A1 mRNA | 228.59 | Up | 5.20E-05 |
| NM_007084 | Homo sapiens SRY (sex determining region Y)-box 21 (SOX21), mRNA | 144.06 | Up | 2.10E-04 |
| AK026784 | Homo sapiens cDNA: FLJ23131 fis, clone LNG08502 | 88.3 | Up | 7.11E-05 |
| BC014344 | Homo sapiens, Similar to arylacetamide deacetylase, clone IMAGE:3934567, mRNA | 52.67 | Up | 1.31E-05 |
| BQ003501 | UI-H-EI1-azd-p-06-0-UI.s1 NCI_CGAP_EI1 Homo sapiens cDNA clone IMAGE:5847413 3, mRNA sequence | 52.33 | Up | 6.55E-05 |
| BQ025821 | UI-1-BB1p-aye-f-10-0-UI.s1 NCI_CGAP_PI6 Homo sapiens cDNA clone UI-1-BB1p-aye-f-10-0-UI 3, mRNA sequence | 45.25 | Up | 5.46E-05 |
| | Homo sapiens Ras interacting protein 1 | 43.27 | Up | 2.20E-04 |
| NM_017805 | (RASIP1), mRNA | | | |
| BF515657 | UI-H-BW1-anu-e-05-0-UI.s1 NCI_CGAP_Sub7 Homo sapiens cDNA clone IMAGE:3083601 3, mRNA sequence | 42.11 | Up | 1.69E-05 |
| NM_138961 | Homo sapiens endothelial cell adhesion molecule (ESAM), mRNA | 41.23 | Up | 7.66E-05 |
| BC046364 | Homo sapiens flavoprotein oxidoreductase MICAL3, mRNA (cDNA clone IMAGE:5737121), with apparent retained intron | 39.24 | Up | 1.47E-04 |
| AK021531 | Homo sapiens cDNA FLJ11469 fis, clone HEMBA1001658 | 36.94 | Up | 5.91E-04 |
| NM_001432 | Homo sapiens epiregulin (EREG), mRNA | 36.88 | Up | 7.51E-04 |
| NM_000474 | Homo sapiens twist homolog 1 (acrocephalosyndactyly 3; Saethre-Chotzen syndrome) (Drosophila) (TWIST1), mRNA | 36.39 | Up | 1.01E-04 |
| NM_053281 | Homo sapiens dachshund homolog 2 (Drosophila) (DACH2), mRNA | 34.9 | Up | 7.97E-06 |
| H85497 | yv88b07.r1 Soares melanocyte 2NbHM Homo sapiens cDNA clone IMAGE:249781 5, mRNA sequence | 32.03 | Up | 1.81E-04 |
| NM_033050 | Homo sapiens succinate receptor 1 (SUCNR1), mRNA | 29.47 | Up | 2.29E-04 |
| BX093329 | BX093329 Soares_parathyroid_tumor_NbHPA Homo sapiens cDNA clone IMAGp998A124183 ; IMAGE:1648403, mRNA sequence | 28.85 | Up | 6.94E-04 |
| NM_012204 | Homo sapiens general transcription factor IIIC, polypeptide 4, 90kDa (GTF3C4), mRNA | 28.2 | Up | 1.29E-04 |
| AI124557 | am58g02.x1 Johnston frontal cortex Homo sapiens cDNA clone IMAGE:1539794 3, mRNA sequence | 27.99 | Up | 4.43E-05 |
| NM_014178 | Homo sapiens syntaxin binding protein 6 (amisyn) (STXBP6), mRNA | 26.38 | Up | 9.11E-05 |
| U83115 | Human non-lens beta gamma-crystallin like protein (AIM1) mRNA, partial cds | 26.15 | Up | 3.63E-05 |
| NM_020927 | Homo sapiens KIAA1576 protein (KIAA1576), mRNA | 25.98 | Up | 2.56E-05 |
| NM_001442 | Homo sapiens fatty acid binding protein 4, adipocyte (FABP4), mRNA | 25.37 | Up | 4.39E-04 |
| BC045666 | Homo sapiens tumor protein p53 inducible protein 11, mRNA (cDNA clone IMAGE:5298525), containing frame-shift errors | 25.36 | Up | 1.84E-04 |
| NM_013387 | Homo sapiens ubiquinol-cytochrome c reductase complex (7.2 kD) (HSPC051), transcript variant 1, mRNA | 24.64 | Up | 1.47E-04 |
| AK125453 | Homo sapiens cDNA FLJ43464 fis, clone OCBBF2036225 | 23.47 | Up | 3.90E-03 |
| NM_170744 | Homo sapiens unc-5 homolog B (C. elegans) (UNC5B), mRNA | 22.66 | Up | 9.44E-05 |
| AB067499 | Homo sapiens mRNA for KIAA1912 protein, partial cds | 22.2 | Up | 2.69E-05 |
| NM_005110 | Homo sapiens glutamine-fructose-6-phosphate transaminase 2 (GFPT2), mRNA | 22 | Up | 9.53E-06 |
| CD677332 | ho15f06.y1 Human Trabecular meshwork cDNA: hohphq Homo sapiens cDNA clone ho15f06 5, mRNA sequence | 21.26 | Up | 7.83E-05 |
| NM_005525 | Homo sapiens hydroxysteroid (11-beta) dehydrogenase 1 (HSD11B1), transcript variant 1, mRNA | 20.9 | Up | 4.93E-05 |
| NM_006475 | Homo sapiens periostin, osteoblast specific factor (POSTN), mRNA | 20.5 | Up | 6.66E-04 |
| AK027128 | Homo sapiens cDNA: FLJ23475 fis, clone HSI13659 | 20.14 | Up | 1.00E-04 |
| AI962169 | wq45c10.x1 NCI_CGAP_GC6 Homo sapiens cDNA clone IMAGE:2474226 3, mRNA sequence | 19.91 | Up | 6.86E-05 |
| BM713465 | UI-E-EJ0-aho-m-22-0-UI.r1 UI-E-EJO Homo sapiens cDNA clone UI-E-EJ0-aho-m-22-0-UI 5, mRNA sequence | 19.86 | Up | 4.55E-05 |
| NM_014802 | Homo sapiens KIAA0528 gene product (KIAA0528), mRNA | 19.34 | Up | 2.92E-03 |
| NM_024336 | Homo sapiens iroquois homeobox protein 3 (IRX3), mRNA | 19.2 | Up | 4.93E-05 |
| | Homo sapiens 5-hydroxytryptamine (serotonin) | 18.89 | Up | 5.39E-05 |
| NM_000867 | receptor 2B (HTR2B), mRNA | | | |
| AF052115 | Homo sapiens clone 23688 mRNA sequence | 17.49 | Up | 8.17E-05 |
| BC016722 | Homo sapiens cDNA clone IMAGE:4075924, partial cds | 16.82 | Up | 2.28E-03 |
| NM_018013 | Homo sapiens hypothetical protein FLJ10159 (FLJ10159), mRNA | 16.78 | Up | 5.98E-06 |
| N33310 | yy39g10.s1 Soares melanocyte 2NbHM Homo sapiens cDNA clone IMAGE:273666 3, mRNA sequence | 16.64 | Up | 1.30E-04 |
| W38393 | zb15c07.r1 Soares_fetal_lung_NbHL19W Homo sapiens cDNA clone IMAGE:302124 5, mRNA sequence | 16.52 | Up | 1.05E-04 |
| R44402 | yg37a01.s1 Soares infant brain 1 NIB Homo sapiens cDNA clone IMAGE:34639 3 similar to contains MER35 repetitive element ;, mRNA sequence | 16.02 | Up | 4.93E-05 |
| NM_020226 | Homo sapiens PR domain containing 8 (PRDM8), mRNA | 15.99 | Up | 7.27E-06 |
| BU677169 | UI-CF-DU1-aaj-c-16-0-UI.s1 UI-CF-DU1 Homo sapiens cDNA clone UI-CF-DU1-aaj-c-16-0-UI 3, mRNA sequence | 15.75 | Up | 3.92E-04 |
| NM_001146 | Homo sapiens angiopoietin 1 (ANGPT1), transcript variant 1, mRNA | 15.5 | Up | 5.07E-04 |
| | Homo sapiens cDNA FLJ41881 fis, clone | 15.42 | Up | 1.03E-04 |
| AK123875 | OCBBF2021833 | | | |
| NM_000735 | Homo sapiens glycoprotein hormones, alpha polypeptide (CGA), mRNA | 15.42 | Up | 2.19E-04 |
| AW445209 | UI-H-BI3-akc-g-11-0-UI.s1 NCI_CGAP_Sub5 Homo sapiens cDNA clone IMAGE:2733908 3, mRNA sequence | 15.03 | Up | 2.89E-05 |
| BC040678 | Homo sapiens, clone IMAGE:4817707, mRNA | 14.85 | Up | 1.69E-03 |
| NM_052846 | Homo sapiens elastin microfibril interfacer 3 (EMILIN3), mRNA | 14.55 | Up | 5.78E-05 |
| NM_005320 | Homo sapiens histone 1, H1d (HIST1H1D), mRNA | 14.49 | Up | 9.77E-03 |
| U79271 | Human clones 23920 and 23921 mRNA sequence | 13.86 | Up | 7.54E-04 |
| BQ186389 | UI-E-EJ1-ajr-c-19-0-UI.r1 UI-E-EJ1 Homo sapiens cDNA clone UI-E-EJ1-ajr-c-19-0-UI 5, mRNA sequence | 13.7 | Up | 5.94E-04 |
| NM_032638 | Homo sapiens GATA binding protein 2 (GATA2), mRNA | 13.42 | Up | 1.67E-04 |
| NM_000089 | Homo sapiens collagen, type I, alpha 2 (COL1A2), mRNA | 13.37 | Up | 1.89E-03 |
| NM_139211 | Homo sapiens homeodomain-only protein (HOP), transcript variant 2, mRNA | 13.15 | Up | 1.03E-04 |
| BM663928 | UI-E-CI1-afw-p-01-0-UI.s1 UI-E-CI1 Homo sapiens cDNA clone UI-E-CI1-afw-p-01-0-UI 3, mRNA sequence | 13.08 | Up | 3.22E-04 |
| NM_021643 | Homo sapiens tribbles homolog 2 (Drosophila) (TRIB2), mRNA | 12.66 | Up | 2.40E-04 |
| NM_004411 | Homo sapiens dynein, cytoplasmic, intermediate polypeptide 1 (DNCI1), mRNA | 12.59 | Up | 1.43E-05 |
| AK128288 | Homo sapiens cDNA FLJ46426 fis, clone THYMU3013897 | 12.51 | Up | 8.11E-04 |
| AL133118 | Homo sapiens mRNA; cDNA DKFZp586N0121 (from clone DKFZp586N0121) | 12.28 | Up | 2.54E-04 |
| BQ027984 | UI-H-CO0-arg-e-03-0-UI.s1 NCI_CGAP_Sub9 Homo sapiens cDNA clone IMAGE:3106611 3, mRNA sequence | 12.2 | Up | 8.14E-05 |
| NM_001175 | Homo sapiens Rho GDP dissociation inhibitor (GDI) beta (ARHGDIB), mRNA | 11.91 | Up | 5.05E-05 |
| NM_005595 | Homo sapiens nuclear factor I/A (NFIA), mRNA | 11.71 | Up | 6.19E-04 |
| AK055518 | Homo sapiens cDNA FLJ30956 fis, clone HCASM2000202 | 11.56 | Up | 2.92E-03 |
| NM_000407 | Homo sapiens glycoprotein Ib (platelet), beta polypeptide (GP1 BB), mRNA | 11.29 | Up | 1.18E-03 |
| | Homo sapiens cDNA FLJ26796 fis, clone | 11.1 | Up | 1.24E-03 |
| AK130306 | PRS05079 | | | |
| NM_012310 | Homo sapiens kinesin family member 4A (KIF4A), mRNA | 11.08 | Up | 1.59E-02 |
| NM_058187 | Homo sapiens chromosome 21 open reading frame 63 (C21orf63), mRNA | 10.78 | Up | 5.06E-04 |
| NM_016206 | Homo sapiens colon carcinoma related protein (FLJ38507), mRNA | 10.71 | Up | 3.40E-04 |
| NM_032918 | Homo sapiens RAS-like, estrogen-regulated, growth inhibitor (RERG), mRNA | 10.69 | Up | 1.36E-04 |
| NM_053044 | Homo sapiens serine protease HTRA3 (HTRA3), mRNA | 10.65 | Up | 2.39E-04 |
| M_153355 | Homo sapiens T-cell lymphoma breakpoint associated target 1 (TCBA1), mRNA | 10.47 | Up | 8.32E-03 |
| No 152665 | Homo sapiens hypothetical protein FLJ40873 (FLJ40873), mRNA | 10.42 | Up | 2.20E-04 |
| NM_004460 | Homo sapiens fibroblast activation protein, alpha (FAP), mRNA | 10.39 | Up | 5.27E-05 |
| NM_152550 | Homo sapiens SH3 domain containing ring finger 2 (SH3RF2), mRNA | 10.29 | Up | 6.33E-04 |
| BF674238 | 602136969F1 NIH_MGC_83 Homo sapiens cDNA clone IMAGE:4273624 5, mRNA sequence | 10.27 | Up | 4.87E-02 |
| NM_005325 | Homo sapiens histone 1, H1a (HIST1H1A), mRNA | 9.97 | Up | 1.19E-03 |
| AV702977 | AV702977 ADB Homo sapiens cDNA clone ADBCVD08 5, mRNA sequence | 9.96 | Up | 4.43E-05 |
| NM_024600 | Homo sapiens chromosome 16 open reading frame 30 (C16orf30), mRNA | 9.89 | Up | 2.28E-03 |
| AK023739 | Homo sapiens cDNA FLJ13677 fis, clone PLACE1011982 | 9.82 | Up | 9.53E-06 |
| AK124699 | Homo sapiens cDNA FLJ42709 fis, clone BRAMY3007350 | 9.73 | Up | 2.38E-02 |
| NM_005239 | Homo sapiens v-ets erythroblastosis virus E26 oncogene homolog 2 (avian) (ETS2), mRNA | 9.56 | Up | 6.20E-03 |
| AK091337 | Homo sapiens cDNA FLJ34018 fis, clone FCBBF2002801 | 9.3 | Up | 6.30E-04 |
| H15096 | ym29e11.r1 Soares infant brain 1 NIB Homo sapiens cDNA clone IMAGE:49250 5, mRNA sequence | 9.17 | Up | 7.83E-05 |
| NM_018286 | Homo sapiens hypothetical protein FLJ10970 (FLJ10970), mRNA | 9.16 | Up | 2.02E-03 |
| NM_003783 | Homo sapiens UDP-Gal:betaGlcNAc beta 1,3-galactosyltransferase, polypeptide 2 (B3GALT2), mRNA | 9.04 | Up | 1.38E-02 |
| NM_032457 | Homo sapiens BH-protocadherin (brain-heart) (PCDH7), transcript variant c, mRNA | 8.94 | Up | 1.53E-04 |
| NM_015192 | Homo sapiens phospholipase C, beta 1 (phosphoinositide-specific) (PLCB1), transcript variant 1, mRNA | 8.82 | Up | 3.03E-03 |
| NM_003514 | Homo sapiens histone 1, H2am (HIST1H2AM), mRNA | 8.75 | Up | 5.60E-03 |
| AI082507 | ox55c02.s1 Soares_total_fetus_Nb2HF8_9w Homo sapiens cDNA clone IMAGE:1660226 3, mRNA sequence | 8.68 | Up | 2.98E-03 |
| NM_025107 | Homo sapiens myc target 1 (MYCT1), mRNA | 8.61 | Up | 1.37E-04 |
| BC036004 | Homo sapiens, clone IMAGE:4730399, mRNA | 8.56 | Up | 2.94E-04 |
| BF029356 | 601765592F1 NIH_MGC_53 Homo sapiens cDNA clone IMAGE:3997510 5, mRNA sequence | 8.47 | Up | 1.99E-03 |
| NM_004787 | Homo sapiens slit homolog 2 (Drosophila) (SLIT2), mRNA | 8.47 | Up | 8.47E-04 |
| CA865586 | ir42e09.x1 HR85 islet Homo sapiens cDNA clone IMAGE:6547889 3, mRNA sequence | 8.46 | Up | 8.95E-04 |
| NM_021614 | Homo sapiens potassium intermediate/small conductance calcium-activated channel, subfamily N, member 2 (KCNN2), transcript variant 1, mRNA | 8.37 | Up | 2.28E-05 |
| NM_004791 | Homo sapiens integrin, beta-like 1 (with EGF-like repeat domains) (ITGBL1 mRNA | 8.31 | Up | 2.21E-04 |
| NM_052954 | Homo sapiens cysteine and tyrosine-rich 1 (CYYR1), mRNA | 8.12 | Up | 8.96E-05 |
| NM_032883 | Homo sapiens chromosome 20 open reading frame 100 (C20orf100), mRNA | 8.1 | Up | 4.12E-04 |
| BX1065777 | BX106577 Soares_NhHMPu_S1 Homo sapiens cDNA clone IMAGp998H131854 ; IMAGE:754236, mRNA sequence | 8.06 | Up | 7.14E-05 |
| AK074131 | Homo sapiens mRNA for FLJ00204 protein | 7.95 | Up | 2.59E-04 |
| NM_024621 | Homo sapiens hypothetical protein FLJ12604 (FLJ12604), mRNA | 7.88 | Up | 4.06E-04 |
| BX647541 | Homo sapiens mRNA; cDNA DKFZp686P0492 (from clone DKFZp686P0492) | 7.84 | Up | 8.97E-05 |
| NM_145016 | Homo sapiens BXMAS2-10 (BXMAS2-10), mRNA | 7.78 | Up | 5.05E-04 |
| BU634363 | UI-H-FL1-bgx-o-20-0-UI.s1 NCI_CGAP_FL1 Homo sapiens cDNA clone UI-H-FL1-bgx-o-20-0-UI 3, mRNA sequence | 7.7 | Up | 1.84E-04 |
| AI939462 | tf23h06.x5 NCI_CGAP_Brn23 Homo sapiens cDNA clone IMAGE:2097083 3, mRNA sequence | 7.49 | Up | 1.21E-04 |
| | Homo sapiens nudix (nucleoside diphosphate | 7.47 | Up | 5.46E-05 |
| NM_153183 | linked moiety X)-type motif 10 (NUDT10), mRNA | | | |
| BQ011545 | UI-1-BC1p-asi-a-02-0-UI.s1 NCI_CGAP_PI3 Homo sapiens cDNA clone UI-1-BC1p-asi-a-02-0-UI 3, mRNA sequence | 7.43 | Up | 1.38E-03 |
| T47612 | yb15h03.s1 Stratagene placenta (#937225) Homo sapiens cDNA clone IMAGE:71285 3, mRNA sequence | 7.41 | Up | 4.13E-03 |
| NM_020809 | Homo sapiens Rho GTPase activating protein 20 (ARHGAP20), mRNA | 7.36 | Up | 2.50E-04 |
| BC042378 | Homo sapiens, clone IMAGE:5277693, mRNA | 7.25 | Up | 3.63E-03 |
| AF414442 | Homo sapiens ovarian cancer related tumor marker CA125 mRNA, complete cds | 7.11 | Up | 2.19E-03 |
| NM_006350 | Homo sapiens folfistatin (FST), transcript variant FST317, mRNA | 7.1 | Up | 5.52E-04 |
| CD723006 | oj16h02.y1 Human lacrimal gland, unamplified: oj Homo sapiens cDNA clone oj16h02 5, mRNA sequence | 7.09 | Up | 5.06E-04 |
| BC027461 | Homo sapiens cDNA clone IMAGE:2984900, containing frame-shift errors | 7.02 | Up | 4.43E-05 |
| NM_152782 | Homo sapiens Sad1 and UNC84 domain containing 1 (SUNC1), mRNA | 6.94 | Up | 1.34E-03 |
| AK125001 | Homo sapiens cDNA FLJ43011 fis, clone BRTHA2015853 | 6.91 | Up | 3.36E-03 |
| NM_152314 | Homo sapiens hypothetical protein MGC34830 (MGC34830), mRNA | 6.89 | Up | 1.08E-04 |
| NM_080872 | Homo sapiens unc-5 homolog D (C. elegans) (UNC5D), mRNA | 6.88 | Up | 3.33E-04 |
| NM_015714 | Homo sapiens putative lymphocyte G0/G1 switch gene (G0S2), mRNA | 6.86 | Up | 1.34E-03 |
| NM_014548 | Homo sapiens tropomodulin 2 (neuronal) (TMOD2), mRNA | 6.83 | Up | 5.11E-03 |
| NM_002771 | Homo sapiens protease, serine, 3 (mesotrypsin) (PRSS3), mRNA | 6.82 | Up | 1.28E-03 |
| NM_012081 | Homo sapiens elongation factor, RNA polymerase II, 2 (ELL2), mRNA | 6.75 | Up | 3.00E-03 |
| AW025556 | wu97g10.x1 NCI_CGAP_Kid3 Homo sapiens cDNA clone IMAGE:2528034 3, mRNA sequence | 6.7 | Up | 2.11E-03 |
| NM_016522 | Homo sapiens neurotrimin (HNT), mRNA | 6.7 | Up | 1.84E-04 |
| AK001279 | Homo sapiens cDNA FLJ10417 fis, clone NT2RP1000112 | 6.68 | Up | 6.41E-04 |
| NM_020993 | Homo sapiens B-cell CLL/lymphoma 7A (BCL7A), mRNA | 6.6 | Up | 1.79E-02 |
| NM_032578 | Homo sapiens myopalladin (FLJ14437), mRNA | 6.55 | Up | 5.46E-05 |
| NM_024913 | Homo sapiens hypothetical protein FLJ21986 (FLJ21986), mRNA | 6.47 | Up | 9.10E-04 |
| NM_016428 | Homo sapiens ABI gene family, member 3 (ABI3), mRNA | 6.45 | Up | 1.07E-02 |
| NM_018371 | Homo sapiens chondroitin beta1,4 N-acetylgalactosaminyltransferase (ChGn), mRNA | 6.44 | Up | 1.75E-04 |
| BM802920 | AGENCOURT_6457446 NIH_MGC_88 Homo sapiens cDNA clone IMAGE:5560288 5, mRNA sequence | 6.37 | Up | 1.34E-03 |
| NM_183245 | Homo sapiens inversin (INVS), transcript variant 2, mRNA | 6.32 | Up | 6.46E-05 |
| AA908815 | og77h08.s1 NCI_CGAP_Ov8 Homo sapiens cDNA clone IMAGE:1454367 3, mRNA sequence | 6.31 | Up | 3.33E-04 |
| BQ021661 | UI-H-DH1-axg-p-14-0-UI.s1 NCI_CGAP_DH1 Homo sapiens cDNA clone IMAGE:5828605 3, mRNA sequence | 6.28 | Up | 4.40E-04 |
| NM_017527 | Homo sapiens lymphocyte antigen 6 complex, locus K (LY6K), mRNA | 6.24 | Up | 1.36E-02 |
| NM_175887 | Homo sapiens hypothetical protein LOC222171 (LOC222171), mRNA | 6.23 | Up | 1.76E-04 |
| | UI-E-EJ1-aje-I-10-0-UI.r1 UI-E-EJ1 Homo | 6.18 | Up | 1.44E-04 |
| BM929598 | sapiens cDNA clone UI-E-EJ1-aje-I-10-0-UI 5, mRNA sequence | | | |
| AI911163 | wd24c09.x1 Soares_NFL_T_GBC_S1 Homo sapiens cDNA clone IMAGE:2329072 3, mRNA sequence | 6.17 | Up | 9.74E-03 |
| AK024653 | Homo sapiens cDNA: FLJ21000 fis, clone CAE03359 | 6.17 | Up | 2.15E-04 |
| AK124907 | Homo sapiens cDNA FLJ42917 fis, clone BRHIP3026335 | 6.15 | Up | 2.20E-03 |
| NM_001147 | Homo sapiens angiopoietin 2 (ANGPT2), mRNA | 6.14 | Up | 1.24E-04 |
| NM_001124 | Homo sapiens adrenomedullin (ADM), mRNA | 6.13 | Up | 1.72E-04 |
| AI733665 | an31g04.x5 Gessler Wilms tumor Homo sapiens cDNA clone IMAGE:1700310 3, mRNA sequence | 6.11 | Up | 5.78E-05 |
| NM_017655 | Homo sapiens PDZ domain protein GIPC2 (GIPC2), mRNA | 6.09 | Up | 1.81E-04 |
| NM_003405 | Homo sapiens tyrosine 3-monooxygenase/tryptophan 5-monooxygenase activation protein, eta polypeptide (YWHAH), mRNA | 5.96 | Up | 5.04E-03 |
| NM_000441 | Homo sapiens solute carrier family 26, member 4 (SLC26A4), mRNA | 5.95 | Up | 2.72E-02 |
| | Homo sapiens mRNA; cDNA | 5.93 | Up | 1.86E-03 |
| BX648323 | DKFZp686K10163 (from clone DKFZp686K10163) | | | |
| AW291775 | UI-H-BI2-agv-h-04-0-UI.s1 NCI_CGAP_Sub4 Homo sapiens cDNA clone IMAGE:2725855 3, mRNA sequence | 5.93 | Up | 5.03E-03 |
| NM_001546 | Homo sapiens inhibitor of DNA binding 4, dominant negative helix-loop-helix protein (ID4), mRNA | 5.9 | Up | 2.39E-03 |
| NM_152996 | Homo sapiens sialyltransferase 7 ((alpha-N-acetylneuraminyl-2,3-beta-galactosyl-1,3)-N-acetyl galactosaminide alpha-2,6-sialyltransferase) C (SIAT7C), mRNA | 5.88 | Up | 5.92E-04 |
| BM556191 | AGENCOURT_6544282 NIH_MGC_88 Homo sapiens cDNA clone IMAGE:5550173 5, mRNA sequence | 5.8 | Up | 7.12E-03 |
| NM_021242 | Homo sapiens MID1 interacting protein 1 (gastrulation specific G12-like (zebrafish)) (MID1IP1), mRNA | 5.75 | Up | 3.63E-03 |
| BI868709 | 603392040F1 NIH_MGC_90 Homo sapiens cDNA clone IMAGE:5402337 5, mRNA sequence | 5.69 | Up | 3.11E-03 |
| BM713114 | UI-E-EJ0-ahi-a-13-0-UI.r1 UI-E-EJ0 Homo sapiens cDNA clone UI-E-EJ0-ahi-a-13-0-UI 5, mRNA sequence | 5.68 | Up | 1.54E-03 |
| AL049443 | Homo sapiens mRNA; cDNA DKFZp586N2020 (from clone DKFZp586N2020) | 5.68 | Up | 4.15E-04 |
| NM_005905 | Homo sapiens SMAD, mothers against DPP homolog 9 (Drosophila) (SMAD9), mRNA | 5.65 | Up | 6.72E-04 |
| | Homo sapiens activating signal cointegrator 1 | 5.63 | Up | 6.59E-03 |
| NM_006828 | complex subunit 3 (ASCC3), mRNA | | | |
| NM_006350 | Homo sapiens follistatin (FST), transcript variant FST317, mRNA | 5.62 | Up | 9.70E-05 |
| R22189 | yh26a02.s1 Soares placenta Nb2HP Homo sapiens cDNA clone IMAGE:130826 3, mRNA sequence | 5.62 | Up | 1.08E-04 |
| NM_020226 | Homo sapiens PR domain containing 8 (PRDM8), mRNA | 5.57 | Up | 3.04E-02 |
| R01937 | ye85h04.s1 Soares fetal liver spleen 1 NFLS Homo sapiens cDNA clone IMAGE:124567 3 similar to gb:L13923 FIBRILLIN 1 PRECURSOR (HUMAN);, mRNA sequence | 5.56 | Up | 1.87E-03 |
| BM724062 | UI-E-EO1-aiy-a-22-0-UI.r1 UI-E-EO1 Homo sapiens cDNA clone UI-E-EO1-aiy-a-22-0-UI 5, mRNA sequence | 5.52 | Up | 2.40E-02 |
| NM_080671 | Homo sapiens potassium voltage-gated channel, Isk-related family, member 4 (KCNE4), mRNA | 5.51 | Up | 1.35E-02 |
| AL558097 | AL558097 Homo sapiens T CELLS (JURKAT CELL LINE) COT 10-NORMALIZED Homo sapiens cDNA clone CS0DJ002YD14 5-PRIME, mRNA sequence | 5.5 | Up | 2.82E-02 |
| AI090760 | qa65e08.x1 Soares_fetal_heart_NbHH19W Homo sapiens cDNA clone IMAGE:1691654 3, mRNA sequence | 5.49 | Up | 7.59E-03 |
| NM_030756 | Homo sapiens transcription factor 7-like 2 (T-cell specific, HMG-box) (TCF7L2), mRNA | 5.48 | Up | 4.57E-04 |
| AL832916 | Homo sapiens mRNA; cDNA DKFZp76210915 (from clone DKFZp76210915) | 5.47 | Up | 1.84E-04 |
| NM_001870 | Homo sapiens carboxypeptidase A3 (mast cell) (CPA3), mRNA | 5.46 | Up | 6.04E-04 |
| BM998303 | UI-H-DT1-awc-h-03-0-UI.s1 NCI_CGAP_DT1 Homo sapiens cDNA clone IMAGE:5887538 3, mRNA sequence | 5.45 | Up | 2.71E-04 |
| NM_004298 | Homo sapiens nucleoporin 155kDa (NUP155), transcript variant 2, mRNA | 5.45 | Up | 4.65E-02 |
| NM_138970 | Homo sapiens neurexin 3 (NRXN3), transcript variant beta, mRNA | 5.44 | Up | 1.15E-04 |
| NM_005654 | Homo sapiens nuclear receptor subfamily 2, group F, member 1 (NR2F1), mRNA | 5.43 | Up | 1.74E-04 |
| NM_012388 | Homo sapiens pallidin homolog (mouse) (PLDN), mRNA | 5.38 | Up | 1.17E-02 |
| NM_033284 | Homo sapiens transducin (beta)-like 1Y-linked (TBL1Y), transcript variant 1, mRNA | 5.38 | Up | 4.42E-02 |
| AK097648 | Homo sapiens cDNA FLJ40329 fis, clone TESTI2031418, weakly similar to TRYPSIN I-P1 PRECURSOR (EC 3.4.21.4) | 5.38 | Up | 1.83E-04 |
| AK124563 | Homo sapiens cDNA FLJ42572 fis, clone BRACE3008092 | 5.35 | Up | 4.57E-04 |
| | Homo sapiens interleukin 7 receptor (IL7R), | 5.31 | Up | 8.90E-04 |
| NM_002185 | mRNA | | | |
| BU661543 | cl73d12.z1 Hembase; Erythroid Precursor Cells (LCB:cl library) Homo sapiens cDNA clone cl73d12 5, mRNA sequence | 5.27 | Up | 6.26E-04 |
| AK092371 | Homo sapiens cDNA FLJ35052 fis, clone OCBBF2018234, highly similar to GUANINE NUCLEOTIDE-BINDING PROTEIN G(I)/G(S)/G(O) GAMMA-2 SUBUNIT (G GAMMA-I) | 5.26 | Up | 4.82E-03 |
| NM_033554 | Homo sapiens major histocompatibility complex, class II, DP alpha 1 (HLA-DPA1), mRNA | 5.24 | Up | 5.25E-04 |
| NM_153014 | Homo sapiens hypothetical protein FLJ30634 (FLJ30634), mRNA | 5.23 | Up | 2.64E-02 |
| AA455071 | aa04d03.s1 Soares_NhHMPu_S1 Homo sapiens cDNA clone IMAGE:812261 3, mRNA sequence | 5.21 | Up | 7.36E-04 |
| NM_005630 | Homo sapiens solute carrier organic anion transporter family, member 2A1 (SLC02A1), mRNA | 5.2 | Up | 1.46E-04 |
| NM_002619 | Homo sapiens platelet factor 4 (chemokine (C-X-C motif) ligand 4) (PF4), mRNA | 5.19 | Up | 3.64E-04 |
| AA232643 | zr47e10.r1 Soares_NhHMPu_S1 Homo sapiens cDNA clone IMAGE:666570 5, mRNA sequence | 5.17 | Up | 1.91E-04 |
| NM_005398 | Homo sapiens protein phosphatase 1, regulatory (inhibitor) subunit 3C (PPP1 R3C), mRNA | 5.14 | Up | 1.29E-04 |
| CA843592 | ir49c12.x1 HR85 islet Homo sapiens cDNA clone IMAGE:6548544 3, mRNA sequence | 5.13 | Up | 1.89E-04 |
| BM677978 | UI-E-EJ0-aig-o-17-0-UI.s1 UI-E-EJ0 Homo sapiens cDNA clone UI-E-EJ0-aig-o-17-0-UI 3, mRNA sequence | 5.11 | Up | 3.39E-03 |
| NM_016196 | Homo sapiens RNA binding motif protein 19 (RBM19), mRNA | 5.1 | Up | 1.84E-04 |
| BX105152 | BX105152 Soares_testis_NHT Homo sapiens cDNA clone IMAGp998J212575 ; IMAGE:1031156, mRNA sequence | 5.1 | Up | 3.96E-05 |
| NM_006329 | Homo sapiens fibulin 5 (FBLN5), mRNA | 5.06 | Up | 1.18E-03 |
| NM_001765 | Homo sapiens CD1C antigen, c polypeptide (CD1C), mRNA | 5.06 | Up | 1.54E-04 |
| NM_022768 | Homo sapiens RNA binding motif protein 15 (RBM15), mRNA | 5.06 | Up | 2.82E-04 |
| N49730 | yz06a12.s1 Soares_multiple_sclerosis _2NbHMSP Homo sapiens cDNA clone IMAGE:282238 3 similar to contains Alu repetitive element;contains element PTR5 repetitive element ;, mRNA sequence | 5.03 | Up | 9.95E-05 |
| AK124856 | Homo sapiens cDNA FLJ42866 fis, clone BRHIP2020622 | 5.02 | Up | 1.25E-03 |
| NM_001008 | Homo sapiens ribosomal protein S4, Y-linked 1 (RPS4Y1), mRNA | 456.26 | Down | 7.09E-06 |
| NM_138963 | Homo sapiens ribosomal protein S4, Y-linked 2 (RPS4Y2), mRNA | 423.01 | Down | 1.53E-06 |
| NM_022454 | Homo sapiens SRY (sex determining region Y)-box 17 (SOX17), mRNA | 404.8 | Down | 3.71E-06 |
| BG219729 | RST39494 Athersys RAGE Library Homo sapiens cDNA, mRNA sequence | 264.66 | Down | 2.05E-06 |
| H70730 | yu69e10.r1 Weizmann Olfactory Epithelium Homo sapiens cDNA clone IMAGE:239082 5, mRNA sequence | 256.95 | Down | 1.53E-06 |
| NM_004653 | Homo sapiens Smcy homolog, Y-linked (mouse) (SMCY), mRNA | 252.16 | Down | 7.51E-07 |
| AB032980 | Homo sapiens mRNA for KIAA1154 protein, partial cds | 227.36 | Down | 4.39E-05 |
| AI765021 | wh56c02.x1 NCI_CGAP_Kid11 Homo sapiens cDNA clone IMAGE:2384738 3, mRNA sequence | 212.62 | Down | 1.69E-05 |
| NM_012307 | Homo sapiens erythrocyte membrane protein band 4.1-like 3 (EPB41L3), mRNA | 192.31 | Down | 2.12E-06 |
| NM_004660 | Homo sapiens DEAD (Asp-Glu-Ala-Asp) box polypeptide 3, Y-linked (DDX3Y), mRNA | 184.34 | Down | 1.69E-05 |
| NM_013230 | Homo sapiens CD24 antigen (small cell lung carcinoma cluster 4 antigen) (CD24), mRNA | 171.2 | Down | 3.78E-07 |
| NM_184087 | Homo sapiens tripartite motif-containing 55 (TRIM55), transcript variant 4, mRNA | 166.79 | Down | 7.27E-06 |
| AK023631 | Homo sapiens cDNA FLJ13569 fis, clone PLACE 1008369 | 163.75 | Down | 2.77E-07 |
| BX102632 | BX102632 NCI_CGAP_Co3 Homo sapiens cDNA clone IMAGp998J052307 ; IMAGE:928228, mRNA sequence | 157.63 | Down | 1.88E-06 |
| NM_006228 | Homo sapiens prepronociceptin (PNOC), mRNA | 144.89 | Down | 2.50E-05 |
| NM_018658 | Homo sapiens potassium inwardly-rectifying channel, subfamily J, member 16 (KCNJ16), transcript variant 1, mRNA | 139.61 | Down | 4.43E-05 |
| NM_003411 | Homo sapiens zinc finger protein, Y-linked (ZFY), mRNA | 130.35 | Down | 2.77E-07 |
| NM_007038 | Homo sapiens a disintegrin-like and metalloprotease (reprolysin type) with thrombospondin type 1 motif, 5 (aggrecanase-2) (ADAMTS5), mRNA | 126.24 | Down | 7.83E-05 |
| NM_184087 | Homo sapiens tripartite motif-containing 55 (TRIM55), transcript variant 4, mRNA | 112.25 | Down | 1.92E-05 |
| NM_003385 | Homo sapiens visinin-like 1 (VSNL1), mRNA | 105.15 | Down | 2.05E-06 |
| NM_153634 | Homo sapiens copine VIII (CPNE8), mRNA | 101.79 | Down | 9.53E-06 |
| NM_000927 | Homo sapiens ATP-binding cassette, sub-family B (MDR/TAP), member 1 (ABCB1), mRNA | 94.49 | Down | 5.20E-05 |
| BG197054 | RST16291 Athersys RAGE Library Homo sapiens cDNA, mRNA sequence | 92.27 | Down | 2.05E-06 |
| NM_004617 | Homo sapiens transmembrane 4 superfamily member 4 (TM4SF4), mRNA | 69.46 | Down | 5.88E-05 |
| NM_000990 | Homo sapiens ribosomal protein L27a (RPL27A), mRNA | 63.45 | Down | 2.03E-04 |
| NM_014893 | Homo sapiens neuroligin 4, Y-linked (NLGN4Y), mRNA | 59.4 | Down | 2.05E-06 |
| AI249696 | qj64a03.x1 NCI_CGAP_Kid3 Homo sapiens cDNA clone IMAGE:1864204 3, mRNA sequence | 58.18 | Down | 1.92E-05 |
| AK092245 | Homo sapiens cDNA FLJ34926 fis, clone NT2RP7003319, highly similar to Mus musculus neuralin mRNA | 57.12 | Down | 2.50E-05 |
| AI335277 | tb29h06.x1 NCI_CGAP_Kid12 Homo sapiens cDNA clone IMAGE:2055803 3, mRNA sequence | 56.89 | Down | 1.80E-05 |
| NM_016356 | Homo sapiens doublecortin domain containing 2 (DCDC2), mRNA | 49.57 | Down | 3.24E-05 |
| NM_138966 | Homo sapiens neuropilin (NRP) and tolloid (TLL)-like 1 (NETO1), transcript variant 3, mRNA | 48.88 | Down | 1.41E-04 |
| | Homo sapiens desmocollin 2 (DSC2), transcript | 48.6 | Down | 1.60E-05 |
| NM_024422 | variant Dsc2a, mRNA | | | |
| NM_031862 | Homo sapiens membrane component, chromosome 17, surface marker 2 (ovarian carcinoma antigen CA125) (M17S2), transcript variant 3, mRNA | 45.75 | Down | 5.31E-05 |
| BF431030 | 7o18c06.x1 NCI_CGAP_Kid11 Homo sapiens cDNA clone IMAGE:3574283 3, mRNA sequence | 45.24 | Down | 2.90E-05 |
| NM_004496 | Homo sapiens forkhead box A1 (FOXA1), mRNA | 44.97 | Down | 2.77E-07 |
| NM_020873 | Homo sapiens leucine rich repeat neuronal 1 (LRRN1), mRNA | 42.8 | Down | 3.33E-05 |
| BF512544 | UI-H-BW1-amf-c-08-0-UI.s1 NCI_CGAP_Sub7 Homo sapiens cDNA clone IMAGE:3069687 3, mRNA sequence | 42.38 | Down | 3.24E-05 |
| CA416106 | UI-H-FEO-bbs-f-17-0-UI.s1 NCI_CGAP_FE0 Homo sapiens cDNA clone UI-H-FE0-bbs-f-17-0-UI 3, mRNA sequence | 41.22 | Down | 5.15E-05 |
| AI244954 | qj93h05.x1 NCI_CGAP_Kid3 Homo sapiens cDNA clone IMAGE:1867065 3, mRNA sequence | 40.95 | Down | 9.53E-06 |
| BF798098 | RC1-C10045-021000-021-f02 CI0045 Homo sapiens cDNA, mRNA sequence | 40.6 | Down | 6.53E-05 |
| NM_002423 | Homo sapiens matrix metalloproteinase 7 (matrilysin, uterine) (MMP7), mRNA | 39.36 | Down | 9.07E-05 |
| | UI-CF-DU1-aaz-f-04-0-UI.s1 UI-CF-DU1 Homo | 37.22 | Down | 6.19E-05 |
| BU680661 | sapiens cDNA clone UI-CF-DU1-aaz-f-04-0-UI 3, mRNA sequence | | | |
| AL080103 | Homo sapiens mRNA; cDNA DKFZp564N2216 (from clone DKFZp564N2216) | 37.11 | Down | 1.50E-05 |
| NM_025074 | Homo sapiens Fraser syndrome 1 (FRAS1), transcript variant 1, mRNA | 36.19 | Down | 7.09E-06 |
| NM_004681 | Homo sapiens eukaryotic translation initiation factor 1A, Y-linked (EIF1AY), mRNA | 36.04 | Down | 1.84E-04 |
| AK096481 | Homo sapiens cDNA FLJ39162 fis, clone OCBBF2002376 | 35.96 | Down | 4.87E-05 |
| NM_018168 | Homo sapiens chromosome 14 open reading frame 105 (C14orf105), mRNA | 34.11 | Down | 3.52E-05 |
| NM_005329 | Homo sapiens hyaluronan synthase 3 (HAS3), transcript variant 1, mRNA | 33.86 | Down | 2.25E-06 |
| NM_002527 | Homo sapiens neurotrophin 3 (NTF3), mRNA | 32.19 | Down | 1.31E-05 |
| NM_001935 | Homo sapiens dipeptidylpeptidase 4 (CD26, adenosine deaminase complexing protein 2) (DPP4), mRNA | 31.89 | Down | 1.66E-04 |
| NM_004932 | Homo sapiens cadherin 6, type 2, K-cadherin (fetal kidney) (CDH6), mRNA | 30.7 | Down | 2.59E-05 |
| BX648643 | Homo sapiens mRNA; cDNA DKFZp686O17106 (from clone DKFZp686O17106) | 30.56 | Down | 7.09E-06 |
| NM_032576 | Homo sapiens chromosome Y open reading frame 15B (CYorf15B), mRNA | 30.49 | Down | 2.05E-06 |
| NM_002276 | Homo sapiens keratin 19 (KRT19), mRNA | 29.34 | Down | 2.84E-04 |
| NM_152487 | Homo sapiens hypothetical protein FLJ31842 (FLJ31842), mRNA | 28.96 | Down | 2.08E-04 |
| NM_207446 | Homo sapiens hypothetical gene supported by AK075564; BC060873 (LOC400451), mRNA | 28.29 | Down | 9.53E-06 |
| NM_020349 | Homo sapiens ankyrin repeat domain 2 (stretch responsive muscle) (ANKRD2), mRNA | 27.27 | Down | 1.17E-05 |
| NM_012198 | Homo sapiens grancalcin, EF-hand calcium binding protein (GCA), mRNA | 27.06 | Down | 7.97E-04 |
| AK056882 | Homo sapiens cDNA FLJ32320 fis, clone PROST2003537 | 26.58 | Down | 1.63E-04 |
| NM_019000 | Homo sapiens hypothetical protein FLJ20152 (FLJ20152), mRNA | 25.35 | Down | 6.39E-05 |
| BG436244 | 602508665F1 NIH_MGC_79 Homo sapiens cDNA clone IMAGE:4605617 5, mRNA sequence | 25.2 | Down | 7.09E-06 |
| AA738254 | nx13b02.s1 NCI_CGAP_GC3 Homo sapiens cDNA clone IMAGE:1255947 3, mRNA sequence | 25.16 | Down | 9.53E-06 |
| NM_002521 | Homo sapiens natriuretic peptide precursor B (NPPB), mRNA | 24.93 | Down | 8.66E-04 |
| NM_000104 | Homo sapiens cytochrome P450, family 1, subfamily B, polypeptide 1 (CYP1B1), mRNA | 24.83 | Down | 3.50E-05 |
| NM_016946 | Homo sapiens F11 receptor (F11 R), transcript variant 1, mRNA | 24.8 | Down | 4.83E-05 |
| NM_016946 | Homo sapiens F11 receptor (F11 R), transcript variant 1, mRNA | 24.38 | Down | 2.77E-05 |
| NM_080743 | Homo sapiens serine-arginine repressor protein (35 kDa) (SRrp35), mRNA | 23.93 | Down | 1.02E-05 |
| NM_175056 | Homo sapiens hypothetical protein LOC131368 (LOC131368), mRNA | 23.5 | Down | 4.93E-05 |
| NM_000557 | Homo sapiens growth differentiation factor 5 (cartilage-derived morphogenetic protein-1) (GDF5), mRNA | 23.37 | Down | 8.14E-05 |
| NM_004221 | Homo sapiens natural killer cell transcript 4 (NK4), mRNA | 23.12 | Down | 4.24E-04 |
| BF431041 | nab31g02.x1 Soares_NSF_F8_9W_OT_PA_P_S1 Homo sapiens cDNA clone IMAGE:3267627 3, mRNA sequence | 22.5 | Down | 4.93E-05 |
| NM_178470 | Homo sapiens WD repeat domain 40B (WDR40B), mRNA | 21.85 | Down | 4.54E-05 |
| AI651524 | wb06g07.x1 NCI_CGAP_GC6 Homo sapiens cDNA clone IMAGE:2304924 3, mRNA sequence | 21.78 | Down | 4.59E-05 |
| CB047092 | NISC_gf08f03.x1 NCI_CGAP_Kid12 Homo sapiens cDNA clone IMAGE:3253013 3, mRNA sequence | 21.63 | Down | 5.93E-06 |
| AA449137 | zx03d12.r1 Soares_total_fetus_Nb2HF8_9w Homo sapiens cDNA clone IMAGE:785399 5, mRNA sequence | 21.62 | Down | 2.08E-04 |
| H89053 | yw24c06.r1 Morton Fetal Cochlea Homo sapiens cDNA clone IMAGE:253162 5, mRNA sequence | 21.41 | Down | 4.16E-03 |
| BX509117 | DKFZp686C09280_r1 686 (synonym: hlcc3) Homo sapiens cDNA clone DKFZp686C09280 5, mRNA sequence | 20.95 | Down | 2.31E-05 |
| NM_00082 | Homo sapiens secreted phosphoprotein 1 5 (osteopontin, bone sialoprotein I, early T-lymphocyte activation 1) (SPP1), mRNA | 20.66 | Down | 6.44E-03 |
| AK124873 | Homo sapiens cDNA FLJ42883 fis, clone BRHIP3006683 | 20.37 | Down | 4.83E-05 |
| AK000075 | Homo sapiens cDNA FLJ20068 fis, clone COL01755 | 20.18 | Down | 5.17E-04 |
| NM_032471 | Homo sapiens protein kinase (cAMP-dependent, catalytic) inhibitor beta (PKIB), transcript variant 3, mRNA | 20.17 | Down | 5.05E-04 |
| AK095776 | Homo sapiens cDNA FLJ38457 fis, clone FEBRA2020400 | 19.57 | Down | 1.69E-05 |
| | Homo sapiens a disintegrin-like and | 19.13 | Down | 9.32E-05 |
| NM_182920 | metalloprotease (reprolysin type) with thrombospondin type 1 motif, 9 (ADAMTS9), transcript variant 1, mRNA | | | |
| NM_002273 | Homo sapiens keratin 8 (KRT8), mRNA | 18.92 | Down | 2.49E-05 |
| NM_002246 | Homo sapiens potassium channel, subfamily K, member 3 (KCNK3), mRNA | 18.9 | Down | 1.35E-05 |
| NM_024726 | Homo sapiens IQ motif containing with AAA domain (IQCA), mRNA | 18.77 | Down | 5.32E-04 |
| AF055376 | Homo sapiens short form transcription factor C-MAF (c-maf) mRNA, complete cds | 18.38 | Down | 1.54E-03 |
| AB033048 | Homo sapiens mRNA for KIAA1222 protein, partial cds | 18.16 | Down | 7.70E-05 |
| AW511222 | hd44d11.x1 Soares_NFL_T_GBC_S1 Homo sapiens cDNA clone IMAGE:2912373 3 similar to contains Alu repetitive element;, mRNA sequence | 17.66 | Down | 7.79E-04 |
| NM_004165 | Homo sapiens Ras-related associated with diabetes (RRAD), mRNA | 17.32 | Down | 3.10E-04 |
| NM_005202 | Homo sapiens collagen, type VIII, alpha 2 (COL8A2), mRNA | 17.22 | Down | 1.48E-04 |
| NM_002515 | Homo sapiens neuro-oncological ventral antigen 1 (NOVA1), transcript variant 1, mRNA | 17.01 | Down | 4.66E-06 |
| NM_005130 | Homo sapiens fibroblast growth factor binding protein 1 (FGFBP1), MRNA | 16.84 | Down | 9.53E-06 |
| AK098071 | Homo sapiens cDNA FLJ40752 fis, clone TRACH2000972 | 16.57 | Down | 1.90E-04 |
| AK024238 | Homo sapiens cDNA FLJ14176 fis, clone NT2RP2003101 | 16.33 | Down | 1.60E-05 |
| R99527 | yq79b11.s1 Soares fetal liver spleen 1 NFLS Homo sapiens cDNA clone IMAGE:201981 3, mRNA sequence | 16.15 | Down | 7.97E-06 |
| NM_021102 | Homo sapiens serine protease inhibitor, Kunitz type, 2 (SPINT2), mRNA | 15.87 | Down | 4.35E-05 |
| BQ003401 | UI-H-EI1-azd-j-23-0-UI.s1 NCI_CGAP _EI1 Homo sapiens cDNA clone IMAGE:5847286 3, mRNA sequence | 15.77 | Down | 2.50E-04 |
| BC035656 | Homo sapiens hypothetical protein LOC285835, mRNA (cDNA clone IMAGE:5588650), partial cds | 15.49 | Down | 153E-06 |
| T56535 | yb33g07.r1 Stratagene fetal spleen (#937205) Homo sapiens cDNA clone IMAGE:73020 5, mRNA sequence | 15.46 | Down | 3.03E-05 |
| NM_152864 | Homo sapiens chromosome 20 open reading frame 58 (C20orf58), mRNA | 15.18 | Down | 1.00E-03 |
| NM_001448 | Homo sapiens glypican 4 (GPC4), mRNA | 15.04 | Down | 2.32E-04 |
| | Homo sapiens crumbs homolog 3 (Drosophila) | 14.99 | Down | 1.08E-04 |
| NM_139161 | (CRB3), transcript variant 2, mRNA | | | |
| BQ924832 | AGENCOURT_8840265 Lupski_sciatic_nerve Homo sapiens cDNA clone IMAGE:6205036 5, mRNA sequence | 14.93 | Down | 1.30E-05 |
| NM_016276 | Homo sapiens serum/glucocorticoid regulated kinase 2 (SGK2), transcript variant 2, mRNA | 14.91 | Down | 2.20E-05 |
| CA437861 | UI-H-DH0-aur-k-12-0-UI.s1 NCI_CGAP_DH0 Homo sapiens cDNA clone UI-H-DH0-aur-k-12-0-UI 3, mRNA sequence | 14.78 | Down | 9.11E-05 |
| H89526 | yw28b04.r1 Morton Fetal Cochlea Homo sapiens cDNA clone IMAGE:253519 5, mRNA sequence | 14.74 | Down | 3.24E-05 |
| BU633163 | UI-H-FL1-bgt-n-07-0-UI.s1 NCI_CGAP_FL1 Homo sapiens cDNA clone UI-H-FL1-bgt-n-07-0-UI 3, mRNA sequence | 14.62 | Down | 3.96E-05 |
| NM_000519 | Homo sapiens hemoglobin, delta (HBD), mRNA | 14.61 | Down | 1.34E-05 |
| NM_020962 | Homo sapiens likely ortholog of mouse neighbor of Punc E11 (NOPE), mRNA | 14.48 | Down | 6.78E-05 |
| NM_004862 | Homo sapiens lipopolysaccharide-induced TNF factor (LITAF), mRNA | 14.37 | Down | 3.22E-04 |
| NM_002837 | Homo sapiens protein tyrosine phosphatase, receptor type, B (PTPRB), mRNA | 14.3 | Down | 1.61E-05 |
| NM_024898 | Homo sapiens family with sequence similarity 31, member C (FAM31C), mRNA | 14.27 | Down | 3.50E-05 |
| AF269162 | Homo sapiens c21orf7 form B mRNA, complete cds | 14.09 | Down | 1.28E-04 |
| NM_003385 | Homo sapiens visinin-like 1 (VSNL1), mRNA | 14.08 | Down | 1.92E-05 |
| BC041412 | Homo sapiens heat shock 70kDa protein 12A, mRNA (cDNA clone IMAGE:5285193), partial cds | 14.04 | Down | 2.40E-04 |
| AI493349 | tg70f04.x1 Soares_NhHMPu_S1 Homo sapiens cDNA clone IMAGE:2114143 3, mRNA sequence | 13.92 | Down | 1.43E-05 |
| BE295468 | 601174523F1 NIH_MGC_17 Homo sapiens cDNA clone IMAGE:3529924 5, mRNA sequence | 13.86 | Down | 9.65E-04 |
| NM_014936 | Homo sapiens ectonucleotide pyrophosphatase/phosphodiesterase 4 (putative function) (ENPP4), mRNA | 13.83 | Down | 6.53E-05 |
| NM_207517 | Homo sapiens ADAMTS-like 3 (ADAMTSL3), mRNA | 13.79 | Down | 3.05E-05 |
| NM_025245 | Homo sapiens pre-B-cell leukemia transcription factor 4 (PBX4), mRNA | 13.77 | Down | 2.73E-04 |
| NM_173508 | Homo sapiens solute carrier family 35, member F3 (SLC35F3), mRNA | 13.69 | Down | 3.06E-05 |
| NM_002578 | Homo sapiens p21 (CDKN1A)-activated kinase 3 (PAK3), mRNA | 13.61 | Down | 1.46E-04 |
| NM_016588 | Homo sapiens neuritin 1 (NRN1), mRNA | 13.56 | Down | 6.51E-05 |
| NM_198389 | Homo sapiens lung type-I cell membrane-associated glycoprotein (T1A-2), transcript variant 2, mRNA | 13.52 | Down | 6.51E-05 |
| NM_173505 | Homo sapiens ankyrin repeat domain 29 (ANKRD29), mRNA | 13.38 | Down | 5.46E-05 |
| NM_152573 | Homo sapiens RAS and EF hand domain containing (RASEF), mRNA | 13.3 | Down | 4.40E-04 |
| NM_020130 | Homo sapiens chromosome 8 open reading frame 4 (C8orf4), mRNA | 13.11 | Down | 5.06E-04 |
| N63415 | yy60d04.s1 Soares_multiple_sclerosis_2NbHMSP Homo sapiens cDNA clone IMAGE:277927 3 similar to contains L1.b3 L1 repetitive element ;, mRNA sequence | 13.03 | Down | 4.99E-04 |
| BX648207 | Homo sapiens mRNA; cDNA DKFZp686E16168 (from clone DKFZp686E16168) | 12.84 | Down | 3.91E-04 |
| NM_178012 | Homo sapiens tubulin, beta polypeptide paralog (MGC8685), mRNA | 12.7 | Down | 2.28E-04 |
| NM_152737 | Homo sapiens hypothetical protein MGC33993 (MGC33993), mRNA | 12.61 | Down | 9.53E-06 |
| AI126888 | qb95d06.x1 Soares_fetal_heart_NbHH19W Homo sapiens cDNA clone IMAGE:1707851 3, mRNA sequence | 12.43 | Down | 4.50E-05 |
| NM_005712 | Homo sapiens HERV-H LTR-associating 1 (HHLA1), mRNA | 12.39 | Down | 1.42E-04 |
| NM_002515 | Homo sapiens neuro-oncological ventral antigen 1 (NOVA1), transcript variant 1, mRNA | 12.32 | Down | 2.77E-05 |
| H23441 | ym52f11.s1 Soares infant brain 1NIB Homo sapiens cDNA clone IMAGE:51888 3, mRNA sequence | 12.28 | Down | 3.96E-05 |
| AL137698 | Homo sapiens mRNA; cDNA DKFZp434C1915 (from clone DKFZp434C1915); partial cds | 12.24 | Down | 5.21E-05 |
| AB023211 | Homo sapiens mRNA for KIAA0994 protein, partial cds | 12.15 | Down | 3.52E-05 |
| AL389942 | Homo sapiens mRNA full length insert cDNA clone EUROIMAGE 2005635 | 12.03 | Down | 1.88E-04 |
| AL359058 | Homo sapiens mRNA full length insert cDNA clone EUROIMAGE 592473 | 11.96 | Down | 1.92E-05 |
| NM_173549 | Homo sapiens hypothetical protein FLJ39553 (FLJ39553), mRNA | 11.93 | Down | 4.52E-05 |
| NM_002031 | Homo sapiens fyn-related kinase (FRK), mRNA | 11.64 | Down | 4.39E-05 |
| AB011095 | Homo sapiens mRNA for KIAA0523 protein, partial cds | 11.59 | Down | 2.43E-05 |
| NM_178033 | Homo sapiens cytochrome P450, family 4, subfamily X, polypeptide 1 (CYP4X1), mRNA | 11.58 | Down | 7.70E-05 |
| NM_003383 | Homo sapiens very low density lipoprotein receptor (VLDLR), mRNA | 11.43 | Down | 1.30E-04 |
| AK125490 | Homo sapiens cDNA FLJ43501 fis, clone PEBLM2004497 | 11.25 | Down | 5.37E-04 |
| NM_004982 | Homo sapiens potassium inwardly-rectifying channel, subfamily J, member 8 (KCNJ8), mRNA | 11 | Down | 5.37E-05 |
| NM_016307 | Homo sapiens paired related homeobox 2 (PRRX2), mRNA | 10.96 | Down | 9.84E-04 |
| NM_019644 | Homo sapiens ankyrin repeat domain 7 (ANKRD7), mRNA | 10.92 | Down | 1.08E-04 |
| AL834140 | Homo sapiens mRNA; cDNA DKFZp434A2029 (from clone DKFZp434A2029) | 10.85 | Down | 3.86E-04 |
| NM_020944 | Homo sapiens glucosidase, beta (bile acid) 2 (GBA2), mRNA | 10.85 | Down | 3.37E-04 |
| CN478597 | UI-CF-FN0-aeo-g-21-0-UI.s1 UI-CF-FN0 Homo sapiens cDNA clone UI-CF-FN0-aeo-g-21-0-UI 3, mRNA sequence | 10.84 | Down | 3.83E-05 |
| AW603401 | RC0-CN0025-010200-012-d01 CN0025 Homo sapiens cDNA, mRNA sequence | 10.78 | Down | 6.72E-04 |
| | Homo sapiens cyclin-dependent kinase inhibitor | 10.73 | Down | 1.42E-04 |
| NM_000076 | 1C (p57, Kip2) (CDKN1C), mRNA | | | |
| AI355761 | qt94a11.x1 NCI_CGAP_Co14 Homo sapiens cDNA clone IMAGE:1962908 3 similar to gb:X74929 KERATIN, TYPE II CYTOSKELETAL 8 (HUMAN);, mRNA sequence | 10.63 | Down | 1.08E-04 |
| AW172903 | xj05e04.x1 NCI_CGAP_Ut2 Homo sapiens cDNA clone IMAGE:2656350 3, mRNA sequence | 10.58 | Down | 9.44E-05 |
| AB041269 | Homo sapiens mRNA for keratin 19, partial cds, isolate:K19-141 | 10.55 | Down | 1.35E-03 |
| BC042028 | Homo sapiens, clone IMAGE:4794726, mRNA | 10.53 | Down | 2.71E-02 |
| NM_030949 | Homo sapiens protein phosphatase 1, regulatory (inhibitor) subunit 14C (PPP1R14C). mRNA | 10.51 | Down | 2.17E-04 |
| BX103476 | BX103476 NCI_CGAP_Lu5 Homo sapiens cDNA clone IMAGp998C053946 ; IMAGE:1557436, mRNA sequence | 10.49 | Down | 4.88E-02 |
| NM_174900 | Homo sapiens zinc finger protein 42 (ZFP42), mRNA | 10.38 | Down | 1.88E-04 |
| NM_152768 | Homo sapiens hypothetical protein FLJ25378 (FLJ25378), mRNA | 10.35 | Down | 8.16E-04 |
| NM_021977 | Homo sapiens solute carrier family 22 (extraneuronal monoamine transporter), member 3 (SLC22A3), mRNA | 10.27 | Down | 1.97E-04 |
| BF509573 | UI-H-B14-apf-b-11-0-UI.s1 NCI_CGAP_Sub8 Homo sapiens cDNA clone IMAGE:3086949 3, mRNA sequence | 10.23 | Down | 1.43E-05 |
| BX648964 | Homo sapiens mRNA; cDNA DKFZp686J0156 (from clone DKFZp686J0156) | 10.19 | Down | 6.44E-03 |
| NM_002338 | Homo sapiens limbic system-associated membrane protein (LSAMP), mRNA | 10.15 | Down | 7.21E-05 |
| NM_006561 | Homo sapiens CUG triplet repeat, RNA binding protein 2 (CUGBP2), mRNA | 10.13 | Down | 1.75E-03 |
| AK123319 | Homo sapiens cDNA FLJ41325 fis, clone BRAMY2046871 | 10.08 | Down | 3.96E-05 |
| NM_004753 | Homo sapiens dehydrogenase/reductase (SDR family) member 3 (DHRS3), mRNA | 9.91 | Down | 1.59E-03 |
| BE788763 | 601475864F1 NIH_MGC_68 Homo sapiens cDNA clone IMAGE:3879014 5, mRNA sequence | 9.88 | Down | 3.14E-04 |
| NM_030594 | Homo sapiens cytoplasmic polyadenylation element binding protein 1 (CPEB1), mRNA | 9.86 | Down | 1.93E-03 |
| NM_002462 | Homo sapiens myxovirus (influenza virus) resistance 1, interferon-inducible protein p78 (mouse) (MX1), mRNA | 9.8 | Down | 3.75E-04 |
| S69208 | troponin T [human, skeletal and cardiac muscle, mRNA, 932 nt] | 9.79 | Down | 1.10E-03 |
| NM_005360 | Homo sapiens v-maf musculoaponeurotic fibrosarcoma oncogene homolog (avian) (MAF), mRNA | 9.76 | Down | 9.46E-04 |
| NM_030583 | Homo sapiens matrilin 2 (MATN2), transcript variant 2, mRNA | 9.67 | Down | 1.92E-05 |
| NM_012464 | Homo sapiens tolloid-like 1 (TLL1), mRNA | 9.66 | Down | 1.76E-02 |
| NM_004490 | Homo sapiens growth factor receptor-bound protein 14 (GRB14), mRNA | 9.65 | Down | 8.22E-04 |
| NM_015236 | Homo sapiens latrophilin 3 (LPHN3), mRNA | 9.65 | Down | 3.96E-05 |
| AK024261 | Homo sapiens cDNA FLJ14199 fis, clone NT2RP3002713 | 9.62 | Down | 1.63E-04 |
| NM_207482 | Homo sapiens FLJ44048 protein (FLJ44048), mRNA | 9.49 | Down | 6.53E-05 |
| BM969191 | UI-CF-EN0-acp-e-22-0-UI.s1 UI-CF-EN0 Homo sapiens cDNA clone UI-CF-EN0-acp-e-22-0-UI 3, mRNA sequence | 9.4 | Down | 2.21E-04 |
| M60502 | Human profilaggrin mRNA, 3 end | 9.34 | Down | 5.78E-05 |
| NM_001710 | Homo sapiens B-factor, properdin (BF), mRNA | 9.32 | Down | 8.70E-05 |
| NM_005114 | Homo sapiens heparan sulfate (glucosamine) 3-O-sulfotransferase 1 (HS3ST1), mRNA | 9.3 | Down | 1.05E-04 |
| NM_003985 | Homo sapiens tyrosine kinase, non-receptor, 1 (TNK1), mRNA | 9.3 | Down | 1.00E-04 |
| NM_001874 | Homo sapiens carboxypeptidase M (CPM), transcript variant 1, mRNA | 9.24 | Down | 5.54E-04 |
| AJ697972 | Homo sapiens chromosome 3 cDNA | 9.15 | Down | 3.55E-04 |
| BM701989 | UI-E-CQ1-aex-j-06-0-UI.r1 UI-E-CQ1 Homo sapiens cDNA clone UI-E-CQ1-aex-j-06-0-UI 5, mRNA sequence | 9.09 | Down | 8.72E-03 |
| BX640973 | Homo sapiens mRNA; cDNA DKFZp686B15184 (from clone DKFZp686B15184) | 9.09 | Down | 1.29E-04 |
| NM_002313 | Homo sapiens actin binding LIM protein 1 (ABLIM1), transcript variant 1, mRNA | 9.05 | Down | 1.23E-04 |
| BX486208 | DKFZp686J07250_r1 686 (synonym: hlcc3) Homo sapiens cDNA clone DKFZp686J07250 5, mRNA sequence | 8.94 | Down | 9.28E-04 |
| NM_004024 | Homo sapiens activating transcription factor 3 (ATF3), mRNA | 8.93 | Down | 9.73E-04 |
| S70348 | Homo sapiens integrin beta 3 mRNA, partial cds, alternatively spliced | 8.92 | Down | 6.01E-05 |
| NM_198495 | Homo sapiens CTAGE family, member 4 (CTAGE4), mRNA | 8.89 | Down | 1.28E-04 |
| D86975 | Homo sapiens mRNA for KIAA0222 gene, partial cds | 8.88 | Down | 3.55E-04 |
| NM_001854 | Homo sapiens collagen, type XI, alpha 1 (COL11A1), transcript variant A, mRNA | 8.87 | Down | 6.78E-05 |
| NM_000212 | Homo sapiens integrin, beta 3 (platelet glycoprotein IIIa, antigen CD61) (ITGB3), mRNA | 8.84 | Down | 7.53E-05 |
| AL117578 | Homo sapiens mRNA; cDNA DKFZp434C128 (from clone DKFZp434C128) | 8.8 | Down | 3.22E-04 |
| NM_198174 | Homo sapiens transcription factor CP2-like 4 (TFCP2L4), transcript variant 3, mRNA | 8.75 | Down | 8.93E-05 |
| BU727096 | UI-E-CR0-ach-e-12-0-UI.s1 UI-E-CR0 Homo sapiens cDNA clone UI-E-CR0-ach-e-12-0-UI 3, mRNA sequence | 8.71 | Down | 5.91E-04 |
| NM_005949 | Homo sapiens metallothionein 1 F (functional) (MT1 F), mRNA | 8.67 | Down | 3.29E-03 |
| BX537613 | Homo sapiens mRNA; cDNA DKFZp686E11117 (from clone DKFZp686E11117) | 8.65 | Down | 7.70E-05 |
| NM_014358 | Homo sapiens C-type (calcium dependent, carbohydrate-recognition domain) lectin, superfamily member 9 (CLECSF9), mRNA | 8.57 | Down | 7.11E-05 |
| NM_000817 | Homo sapiens glutamate decarboxylase 1 (brain, 67kDa) (GAD1 transcript variant GAD67, mRNA | 8.55 | Down | 2.77E-05 |
| NM_005450 | Homo sapiens noggin (NOG), mRNA | 8.49 | Down | 2.75E-03 |
| NM_144707 | Homo sapiens prominin 2 (PROM2), mRNA | 8.49 | Down | 2.72E-04 |
| AK074181 | Homo sapiens mRNA for FLJ00254 protein | 8.46 | Down | 7.83E-05 |
| NM_032882 | Homo sapiens paraneoplastic antigen like 6A (PNMA6A), mRNA | 8.44 | Down | 2.57E-05 |
| NM_004479 | Homo sapiens fucosyltransferase 7 (alpha (1,3) fucosyltransferase) (FUT7), mRNA | 8.39 | Down | 1.73E-05 |
| NM_000856 | Homo sapiens guanylate cyclase 1, soluble, alpha 3 (GUCY1A3), mRNA | 8.31 | Down | 1.63E-04 |
| NM_016542 | Homo sapiens Mst3 and SOK1-related kinase (MST4), mRNA | 8.26 | Down | 6.03E-04 |
| NM_021044 | Homo sapiens desert hedgehog homolog (Drosophila) (DHH), mRNA | 8.23 | Down | 6.01E-04 |
| NM_000313 | Homo sapiens protein S (alpha) (PROS1), mRNA | 8.23 | Down | 4.08E-04 |
| NM_000227 | Homo sapiens laminin, alpha 3 (LAMA3), transcript variant 2, mRNA | 8.15 | Down | 2.89E-05 |
| AA010611 | zi09f09.s1 Soares_fetal_liver_spleen_1NFLS_S1 Homo sapiens cDNA clone IMAGE:430313 3, mRNA sequence | 8.13 | Down | 4.99E-04 |
| NM_004433 | Homo sapiens E74-like factor 3 (ets domain transcription factor, epithelial-specific) (ELF3), mRNA | 8.07 | Down | 6.84E-05 |
| NM_000860 | Homo sapiens hydroxyprostaglandin dehydrogenase 15-(NAD) (HPGD), mRNA | 8.02 | Down | 1.49E-04 |
| BG208475 | RST27977 Athersys RAGE Library Homo sapiens cDNA, mRNA sequence | 8 | Down | 2.90E-03 |
| N75271 | yz74h12.r1 Soares_multiple_sclerosis_2NbHMSP Homo sapiens cDNA clone IMAGE:288839 5, mRNA sequence | 8 | Down | 3.67E-03 |
| NM_022912 | Homo sapiens chromosome 2 open reading frame 23 (C2orf23), mRNA | 8 | Down | 9.93E-05 |
| AL706653 | DKFZp686E1543_r1 686 (synonym: hlcc3) Homo sapiens cDNA clone DKFZp686E1543 5, mRNA sequence | 7.98 | Down | 9.86E-05 |
| AA411988 | zt65g11.s1 Soares_testis_NHT Homo sapiens cDNA clone IMAGE:727268 3, mRNA sequence | 7.95 | Down | 9.11E-05 |
| NM_020152 | Homo sapiens chromosome 21 open reading frame 7 (C21orf7), mRNA | 7.95 | Down | 4.35E-05 |
| AK125608 | Homo sapiens cDNA FLJ43620 fis, clone SPLEN2021701, highly similar to HLA CLASS I HISTOCOMPATIBILITY ANTIGEN, A-2 ALPHA CHAIN PRECURSOR | 7.93 | Down | 1.87E-03 |
| CA306881 | UI-H-FT1-bht-n-22-0-UI.s1 NCI_CGAP_FT1 Homo sapiens cDNA clone UI-H-FT1-bht-n-22-0-UI 3, mRNA sequence | 7.9 | Down | 7.95E-04 |
| AI640484 | wa27f01.x1 NCI_CGAP_Kid11 Homo sapiens cDNA clone IMAGE:2299321 3, mRNA sequence | 7.87 | Down | 3.33E-04 |
| AI819863 | wj45h05.x1 NCI_CGAP_Lu19 Homo sapiens cDNA clone IMAGE:2405817 3, mRNA sequence | 7.86 | Down | 5.42E-05 |
| AK124702 | Homo sapiens cDNA FLJ42712 fis, clone BRAMY3008044 | 7.86 | Down | 2.13E-04 |
| NM_014333 | Homo sapiens immunoglobulin superfamily, member 4 (IGSF4), mRNA | 7.8 | Down | 5.41E-03 |
| H94320 | yv18b10.s1 Soares fetal liver spleen 1 NFLS Homo sapiens cDNA clone IMAGE:243067 3, mRNA sequence | 7.8 | Down | 4.43E-05 |
| NM_022103 | Homo sapiens hypothetical zinc finger protein FLJ14011 (FLJ14011), mRNA | 7.77 | Down | 3.50E-04 |
| NM 016179 | Homo sapiens transient receptor potential cation channel, subfamily C, member 4 (TRPC4), mRNA | 7.75 | Down | 6.04E-04 |
| AF108093 | Homo sapiens IA-2 gene, intron 18 | 7.74 | Down | 1.92E-05 |
| NM_001332 | Homo sapiens catenin (cadherin-associated protein), delta 2 (neural plakophilin-related arm-repeat protein) (CTNND2), mRNA | 7.73 | Down | 5.06E-04 |
| NM_002206 | Homo sapiens integrin, alpha 7 (ITGA7), mRNA | 7.73 | Down | 2.33E-03 |
| BF510493 | UI-H-B14-apa-b-08-0-UI.s1 NCI_CGAP_Sub8 Homo sapiens cDNA clone IMAGE:3086558 3, mRNA sequence | 7.68 | Down | 2.89E-05 |
| BU951469 | in60a05.x3 HR85 islet Homo sapiens cDNA clone IMAGE:6126249 3, mRNA sequence | 7.63 | Down | 1.74E-04 |
| AI819186 | wj32d10.x1 NCI_CGAP_Kid12 Homo sapiens cDNA clone IMAGE:2404531 3, mRNA sequence | 7.62 | Down | 5.86E-04 |
| NM_013259 | Homo sapiens transgelin 3 (TAGLN3), transcript variant 1, mRNA | 7.61 | Down | 3.22E-04 |
| AI942360 | wo80c06.x1 NCI_CGAP_Kid11 Homo sapiens cDNA clone IMAGE:2461642 3, mRNA sequence | 7.57 | Down | 6.84E-05 |
| BX098521 | BX098521 Soares fetal liver spleen 1 NFLS Homo sapiens cDNA clone IMAGp998L05118 ; IMAGE:123412, mRNA sequence | 7.55 | Down | 1.31E-05 |
| NM_152495 | Homo sapiens cornichon homolog 3 (Drosophila) (CNIH3), mRNA | 7.55 | Down | 5.20E-05 |
| NM_018728 | Homo sapiens myosin VC (MYO5C), mRNA | 7.54 | Down | 1.30E-04 |
| NM_004086 | Homo sapiens coagulation factor C homolog, cochlin (Limulus polyphemus) (COCH), mRNA | 7.51 | Down | 5.92E-04 |
| BM683698 | UI-E-EJ1-ajh-k-08-0-UI.s1 UI-E-EJ1 Homo sapiens cDNA clone UI-E-EJ1-ajh-k-08-0-UI 3, mRNA sequence | 7.48 | Down | 6.46E-05 |
| NM_000593 | Homo sapiens transporter 1, ATP-binding cassette, sub-family B (MDR/TAP) (TAP1), mRNA | 7.47 | Down | 1.05E-04 |
| | Homo sapiens cDNA FLJ36973 fis, clone | 7.44 | Down | 5.42E-05 |
| AK094292 | BRACE2006249 | | | |
| NM_031442 | Homo sapiens transmembrane 4 superfamily member 10 (TM4SF10), mRNA | 7.42 | Down | 1.87E-03 |
| AK074097 | Homo sapiens mRNA for FLJ00168 protein | 7.41 | Down | 4.15E-04 |
| NM_005302 | Homo sapiens G protein-coupled receptor 37 (endothelin receptor type B-like) (GPR37), mRNA | 7.33 | Down | 1.15E-03 |
| NM_018349 | Homo sapiens multiple C2-domains with two transmembrane regions 2 (MCTP2), mRNA | 7.3 | Down | 5.46E-05 |
| NM_014399 | Homo sapiens transmembrane 4 superfamily member 13 (TM4SF13), mRNA | 7.28 | Down | 7.73E-03 |
| NM_002899 | Homo sapiens retinol binding protein 1, cellular (RBP1), mRNA | 7.28 | Down | 1.79E-03 |
| NM_015441 | Homo sapiens olfactomedin-like 2B (OLFML2B), mRNA | 7.24 | Down | 5.84E-04 |
| NM_206808 | Homo sapiens citrate lyase beta like (CLYBL), transcript variant 2, mRNA | 7.22 | Down | 9.35E-05 |
| NM_207380 | Homo sapiens FLJ43339 protein (FLJ43339), mRNA | 7.19 | Down | 1.69E-03 |
| NM_000147 | Homo sapiens fucosidase, alpha-L- 1, tissue (FUCA1), mRNA | 7.17 | Down | 4.23E-02 |
| NM_002670 | Homo sapiens plastin 1 (I isoform) (PLS1), mRNA | 7.16 | Down | 2.28E-02 |
| NM_000620 | Homo sapiens nitric oxide synthase 1 (neuronal) (NOS1), mRNA | 7.15 | Down | 1.46E-04 |
| NM_018242 | Homo sapiens hypothetical protein FLJ10847 (FLJ10847), mRNA | 7.14 | Down | 1.48E-03 |
| N78460 | yz76h06.r1 Soares_multiple_sclerosis_2NbHMSP Homo sapiens cDNA clone IMAGE:289019 5, mRNA sequence | 7.11 | Down | 2.28E-02 |
| NM_001001430 | Homo sapiens troponin T2, cardiac (TNNT2), transcript variant 2, mRNA | 7.04 | Down | 9.11E-05 |
| NM_003979 | Homo sapiens G protein-coupled receptor, family C, group 5, member A (GPCR5A), mRNA | 6.95 | Down | 4.47E-05 |
| NM_003328 | Homo sapiens TXK tyrosine kinase (TXK), mRNA | 6.94 | Down | 1.44E-04 |
| AK128715 | Homo sapiens cDNA FLJ46882 fis, clone UTERU3015844 | 6.94 | Down | 3.34E-03 |
| NM_000961 | Homo sapiens prostaglandin I2 (prostacyclin) synthase (PTGIS), mRNA | 6.91 | Down | 5.88E-04 |
| NM_052839 | Homo sapiens pannexin 2 (PANX2), mRNA | 6.9 | Down | 7.48E-05 |
| AF119903 | Homo sapiens PRO2834 mRNA, complete cds | 6.89 | Down | 2.43E-05 |
| NM_001505 | Homo sapiens G protein-coupled receptor 30 (GPR30), mRNA | 6.84 | Down | 1.15E-03 |
| AW137116 | UI-H-B1I-acp-f-03-0-UI.s1 NCI_CGAP_Sub3 Homo sapiens cDNA clone IMAGE:2715029 3, mRNA sequence | 6.83 | Down | 3.08E-05 |
| NM_052960 | Homo sapiens retinol binding protein 7, cellular (RBP7), mRNA | 6.79 | Down | 3.15E-03 |
| NM_003107 | Homo sapiens SRY (sex determining region Y)-box 4 (SOX4), mRNA | 6.77 | Down | 3.33E-04 |
| NM_004170 | Homo sapiens solute carrier family 1 (neuronal/epithelial high affinity glutamate transporter, system Xag), member 1 (SLC1A1), mRNA | 6.77 | Down | 3.33E-04 |
| CB115754 | K-EST0159876 L8SCK0 Homo sapiens cDNA clone L8SCK0-8-H08 5, mRNA sequence | 6.75 | Down | 1.69E-05 |
| AK026740 | Homo sapiens cDNA: FLJ23087 fis, clone LNG06994, highly similar to AF161368 Homo sapiens HSPC105 mRNA | 6.75 | Down | 1.05E-04 |
| NM_002487 | Homo sapiens necdin homolog (mouse) (NDN), mRNA | 6.75 | Down | 6.29E-04 |
| M27161 | Human MHC class I CD8 alpha-chain (Leu-2/T8) gene, complete cds | 6.73 | Down | 4.96E-03 |
| AK025003 | Homo sapiens cDNA: FLJ21350 fis, clone COL02751 | 6.7 | Down | 4.15E-04 |
| NM_000693 | Homo sapiens aldehyde dehydrogenase 1 family, member A3 (ALDH1A3), mRNA | 6.69 | Down | 1.41E-02 |
| NM_014900 | Homo sapiens COBL-like 1 (COBLL1), mRNA | 6.67 | Down | 1.17E-02 |
| BG545305 | 602572521 F1 NIH_MGC_77 Homo sapiens cDNA clone IMAGE:4700644 5, mRNA sequence | 6.66 | Down | 8.24E-04 |
| NM_014421 | Homo sapiens dickkopf homolog 2 (Xenopus laevis) (DKK2), mRNA | 6.66 | Down | 2.17E-02 |
| NM_005139 | Homo sapiens annexin A3 (ANXA3), mRNA | 6.64 | Down | 2.04E-04 |
| BX111520 | BX111520 Soares placenta Nb2HP Homo sapiens cDNA clone IMAGp998L15208 ; IMAGE:141470, mRNA sequence | 6.64 | Down | 7.70E-05 |
| AK090808 | Homo sapiens cDNA FLJ33489 fis, clone BRAMY2003585 | 6.63 | Down | 1.08E-04 |
| D62831 | HUM330B12B Clontech human aorta polyA+ mRNA (#6572) Homo sapiens cDNA clone GEN-330B12 5, mRNA sequence | 6.58 | Down | 1.02E-03 |
| H18652 | ym45e04.s1 Soares infant brain 1NIB Homo sapiens cDNA clone IMAGE:50948 3, mRNA sequence | 6.57 | Down | 1.00E-03 |
| | UI-H-EI1-ayu-m-09-0-UI.s1 NCI_CGAP_EI1 | 6.56 | Down | 1.26E-04 |
| BQ002165 | Homo sapiens cDNA clone IMAGE:5843888 3, mRNA sequence | | | |
| NM_018076 | Homo sapiens armadillo repeat containing 4 (ARMC4), mRNA | 6.54 | Down | 1.72E-04 |
| H41942 | yo60a08.r1 Soares breast 3NbHBst Homo sapiens cDNA clone IMAGE:182294 5 similar to contains Alu repetitive element;contains LTR9 repetitive element ;, mRNA sequence | 6.53 | Down | 3.48E-03 |
| AJ318805 | AJ318805 Homo sapiens adipose tissue Homo sapiens cDNA clone 2040, mRNA sequence | 6.5 | Down | 1.54E-04 |
| NM_032866 | Homo sapiens cingulin-like 1 (CGNL1), mRNA | 6.46 | Down | 1.84E-03 |
| CA434164 | UI-H-DHO-arv-a-17-0-UI.s1 NCI_CGAP_DH0 Homo sapiens cDNA clone UI-H-DH0-arv-a-17-0-UI 3, mRNA sequence | 6.44 | Down | 2.82E-04 |
| AW006864 | ws15d04.x1 NCI_CGAP_Kid11 Homo sapiens cDNA clone IMAGE:2497255 3, mRNA sequence | 6.39 | Down | 2.77E-05 |
| NM_014932 | Homo sapiens neuroligin 1 (NLGN1), mRNA | 6.39 | Drown | 2.59E-05 |
| BX538226 | Homo sapiens mRNA; cDNA DKFZp686E1944 (from clone DKFZp686E1944) | 6.38 | Down | 1.74E-04 |
| NM_016651 | Homo sapiens dapper homolog 1, antagonist of beta-catenin (xenopus) (DACT1), mRNA | 6.38 | Down | 5.05E-04 |
| NM_015678 | Homo sapiens neurobeachin (NBEA), mRNA | 6.34 | Down | 4.17E-03 |
| NM_022842 | Homo sapiens CUB domain-containing protein 1 (CDCP1), transcript variant 1, mRNA | 6.32 | Down | 1.12E-03 |
| AF130079 | Homo sapiens clone FLC0578 PRO2852 mRNA, complete cds | 6.32 | Down | 1.74E-02 |
| AL117425 | Homo sapiens mRNA; cDNA DKFZp566L203 (from clone DKFZp566L203) | 6.29 | Down | 8.54E-05 |
| NM_005141 | Homo sapiens fibrinogen, B beta polypeptide (FGB), mRNA | 6.28 | Down | 3.43E-04 |
| AW590139 | hg33d12.x1 NCI_CGAP_GC6 Homo sapiens cDNA clone IMAGE:2947415 3, mRNA sequence | 6.27 | Down | 1.29E-04 |
| BE968596 | 601649770F1 NIH_MGC_74 Homo sapiens cDNA clone IMAGE:3933472 5, mRNA sequence | 6.26 | Down | 6.39E-05 |
| AF220263 | Homo sapiens MOST2 mRNA, complete cds | 6.25 | Down | 1.10E-03 |
| NM_000876 | Homo sapiens insulin-like growth factor 2 receptor (IGF2R), mRNA | 6.25 | Down | 1.08E-03 |
| NM_006681 | Homo sapiens neuromedin U (NMU), mRNA | 6.25 | Down | 1.07E-04 |
| AK024865 | Homo sapiens cDNA: FLJ21212 fis, clone COL00502 | 6.24 | Down | 1.05E-04 |
| NM_024508 | Homo sapiens zinc finger, BED domain containing 2 (ZBED2), mRNA | 6.22 | Down | 8.82E-04 |
| NM_177949 | Homo sapiens armadillo repeat containing, X-linked 2 (ARMCX2), mRNA | 6.18 | Down | 1.20E-02 |
| NM_001257 | Homo sapiens cadherin 13, H-cadherin (heart) (CDH13), mRNA | 6.17 | Down | 8.46E-03 |
| NM_001200 | Homo sapiens bone morphogenetic protein 2 (BMP2), mRNA | 6.15 | Down | 3.33E-04 |
| AK125695 | Homo sapiens cDNA FLJ43707 fis, clone TESOP2001865 | 6.15 | Down | 6.53E-05 |
| BC033567 | Homo sapiens, clone IMAGE:4822266, mRNA | 6.14 | Down | 3.14E-04 |
| AB011539 | Homo sapiens mRNA for MEGF6 protein (KIAA0815), partial cds | 6.14 | Down | 1.49E-03 |
| NM_024697 | Homo sapiens hypothetical protein FLJ22419 (FLJ22419), mRNA | 6.13 | Down | 2.72E-04 |
| BC040701 | Homo sapiens cDNA clone IMAGE:5736259, partial cds | 6.13 | Down | 1.89E-03 |
| NM_001889 | Homo sapiens crystallin, zeta (quinone reductase) (CRYZ), mRNA | 6.12 | Down | 1.97E-02 |
| BX108092 | BX108092 Soares_testis_NHT Homo sapiens cDNA clone IMAGp998_164460 ; IMAGE:1754967, mRNA sequence | 6.08 | Down | 1.01E-04 |
| T65315 | yc79h02.s1 Soares infant brain 1 NIB Homo sapiens cDNA clone IMAGE:22228 3, mRNA sequence | 6.08 | Down | 1.52E-04 |
| BX095811 | BX095811 Soares_testis_NHT Homo sapiens cDNA clone IMAGp998E084494 ; IMAGE:1837423, mRNA sequence | 6.07 | Down | 1.68E-04 |
| NM_012413 | Homo sapiens glutaminyl-peptide cyclotransferase (glutaminyl cyclase) (QPCT), mRNA | 6.07 | Down | 4.25E-02 |
| AI311296 | ta48d10.x2 NCI_CGAP_Lu25 Homo sapiens cDNA clone IMAGE:2047315 3, mRNA sequence | 6.07 | Down | 4.15E-04 |
| NM_138811 | Homo sapiens chromosome 7 open reading frame 31 (C7orf31), mRNA | 6.06 | Down | 1.54E-04 |
| BM988642 | UI-H-DH0-arx-p-21-0-UI.s1 NCI_CGAP_DH0 Homo sapiens cDNA clone IMAGE:5855492 3, mRNA sequence | 6.05 | Down | 1.28E-04 |
| NM_016613 | Homo sapiens hypothetical protein DKFZp434L142 (DKFZp434L142), mRNA | 6.04 | Down | 1.25E-02 |
| W20132 | zb40c10.r1 Soares_parathyroid_tumor_NbHPA Homo sapiens cDNA clone IMAGE:306066 5, mRNA sequence | 6.01 | Down | 7.83E-05 |
| NM_001785 | Homo sapiens cytidine deaminase (CDA), mRNA | 6.01 | Down | 1.16E-04 |
| | Homo sapiens RAB11 family interacting protein | 6 | Down | 1.76E-02 |
| NM_025151 | 1 (class I) (RAB11FIP1), transcript variant 1, mRNA | | | |
| AF519622 | Homo sapiens noncoding mRNA sequence | 5.98 | Down | 1.26E-04 |
| NM_152284 | Homo sapiens Snf7 homologue associated with Alix 3 (Shax3), mRNA | 5.97 | Down | 7.70E-05 |
| D29453 | HUMNK566 Human epidermal keratinocyte Homo sapiens cDNA clone 566, mRNA sequence | 5.97 | Down | 3.58E-04 |
| AI220066 | qg84d01.x1 Soares_NFL_T_GBC_S1 Homo sapiens cDNA clone IMAGE:1841857 3, mRNA sequence | 5.96 | Down | 9.78E-04 |
| AK026235 | Homo sapiens cDNA: FLJ22582 fis, clone HSI02576 | 5.94 | Down | 1.10E-03 |
| BX102869 | BX102869 Soares fetal liver spleen 1 NFLS Homo sapiens cDNA clone IMAGp998P17421 ; IMAGE:214528, mRNA sequence | 5.92 | Down | 2.81E-04 |
| NM_002354 | Homo sapiens tumor-associated calcium signal transducer 1 (TACSTD1), mRNA | 5.92 | Down | 6.65E-04 |
| CA313095 | UI-CF-FN0-aex-f-01-0-UI.s1 UI-CF-FNO Homo sapiens cDNA clone UI-CF-FN0-aex-f-01-0-UI 3, mRNA sequence | 5.91 | Down | 1.51E-04 |
| BX641086 | Homo sapiens mRNA; cDNA DKFZp686G2469 (from clone DKFZp686G2469) | 5.9 | Down | 1.07E-04 |
| AK021493 | Homo sapiens cDNA FLJ11431 fis, clone HEMBA1001094 | 5.88 | Down | 2.79E-04 |
| NM_152369 | Homo sapiens hypothetical protein MGC45474 (MGC45474), mRNA | 5.87 | Down | 4.40E-04 |
| L07615 | Human neuropeptide Y receptor Y1 (NPYY1) mRNA, exon 2-3 and complete cds | 5.87 | Down | 3.91E-04 |
| BX503694 | DKFZp686M02112_s1 686 (synonym: hlcc3) Homo sapiens cDNA clone DKFZp686M02112 3, mRNA sequence | 5.84 | Down | 2.17E-04 |
| NM_021101 | Homo sapiens claudin 1 (CLDN1), mRNA | 5.82 | Down | 1.24E-04 |
| NM_005737 | Homo sapiens ADP-ribosylation factor-like 7 (ARL7), mRNA | 5.8 | Down | 3.58E-04 |
| NM_005264 | Homo sapiens GDNF family receptor alpha 1 (GFRA1), transcript variant 1, mRNA | 5.8 | Down | 4.54E-05 |
| AB020640 | Homo sapiens mRNA for KIAA0833 protein, partial cds | 5.79 | Down | 7.11E-05 |
| AK022598 | Homo sapiens cDNA FLJ12536 fis, clone NT2RM4000265 | 5.78 | Down | 2.20E-04 |
| NM_173660 | Homo sapiens hypothetical protein FLJ33718 (FLJ33718), mRNA | 5.76 | Down | 2.95E-04 |
| NM_052923 | Homo sapiens zinc finger protein 452 (ZNF452), mRNA | 5.71 | Down | 3.90E-03 |
| NM_000797 | Homo sapiens dopamine receptor D4 (DRD4), mRNA | 5.71 | Down | 4.44E-03 |
| NM_014917 | Homo sapiens netrin G1 (NTNG1), mRNA | 5.7 | Down | 2.50E-04 |
| NM_053039 | Homo sapiens UDP glycosyltransferase 2 family, polypeptide B28 (UGT2B28), mRNA | 5.7 | Down | 9.11E-05 |
| NM_006266 | Homo sapiens ral guanine nucleotide dissociation stimulator (RALGDS), mRNA | 5.68 | Down | 9.21E-03 |
| NM_003264 | Homo sapiens toll-like receptor 2 (TLR2), mRNA | 5.67 | Down | 1.51E-04 |
| AA017035 | ze37c10.s1 Soares retina N2b4HR Homo sapiens cDNA clone IMAGE:361170 3 similar to contains Alu repetitive element;contains element MER37 repetitive element ;, mRNA sequence | 5.66 | Down | 1.17E-04 |
| NM_005901 | Homo sapiens SMAD, mothers against DPP homolog 2 (Drosophila) (SMAD2), transcript variant 1, mRNA | 5.65 | Down | 3.84E-02 |
| NM_000576 | Homo sapiens interleukin 1, beta (IL1B), mRNA | 5.64 | Down | 6.69E-05 |
| NM_013322 | Homo sapiens sorting nexin 10 (SNX10), mRNA | 5.63 | Down | 4.81E-04 |
| AJ406941 | Homo sapiens partial mRNA for keratin associated protein 4.9 (KRTAP4.9 gene) | 5.62 | Down | 5.80E-05 |
| NM_001562 | Homo sapiens interleukin 18 (interferon-gamma-inducing factor) (IL18), mRNA | 5.61 | Down | 5.82E-03 |
| NM_173567 | Homo sapiens abhydrolase domain containing 7 (ABHD7), mRNA | 5.59 | Down | 1.18E-04 |
| F36108 | HSPD33448 HM3 Homo sapiens cDNA clone sH4-000003-1/E09, mRNA sequence | 5.57 | Down | 7.05E-04 |
| NM_145804 | Homo sapiens ankyrin repeat and BTB (POZ) domain containing 2 (ABTB2), mRNA | 5.56 | Down | 1.76E-03 |
| BQ002466 | UI-H-EI1-ayw-k-24-0-UI.s1 NCI_CGAP_EI1 Homo sapiens cDNA clone IMAGE:5844623 3, mRNA Sequence | 5.56 | Down | 1.46E-04 |
| NM_173662 | Homo sapiens hypothetical protein LOC285533 (LOC285533), mRNA | 5.56 | Down | 3.01E-04 |
| NM_000129 | Homo sapiens coagulation factor XIII, A1 polypeptide (F13A1), mRNA | 5.53 | Down | 1.05E-04 |
| NM_000584 | Homo sapiens interleukin 8 (IL8), mRNA | 5.51 | Down | 1.82E-03 |
| BM664445 | UI-E-CL1-afa-p-05-0-UI.s1 UI-E-CL1 Homo sapiens cDNA clone UI-E-CL1-afa-p-05-0-UI 3, mRNA sequence | 5.5 | Down | 6.13E-04 |
| NM_006863 | Homo sapiens leukocyte immunoglobulin-like receptor, subfamily A (with TM domain), member 1 (LILRA1), mRNA | 5.48 | Down | 3.09E-03 |
| NM_006722 | Homo sapiens microphthalmia-associated transcription factor (MITF), transcript variant 3, mRNA | 5.46 | Down | 5.71E-03 |
| BC033124 | Homo sapiens, clone IMAGE:2960615, mRNA | 5.46 | Down | 3.64E-04 |
| NM_014978 | Homo sapiens sortilin-related VPS10 domain containing receptor 3 (SORCS3), mRNA | 5.46 | Down | 1.26E-04 |
| AF007143 | Homo sapiens clone 23738 mRNA sequence | 5.46 | Down | 2.05E-04 |
| NM_001089 | Homo sapiens ATP-binding cassette, sub-family A (ABC1), member 3 (ABCA3), mRNA | 5.46 | Down | 4.75E-05 |
| NM_004524 | Homo sapiens lethal giant larvae homolog 2 (Drosophila) (LLGL2), mRNA | 5.46 | Down | 2.73E-04 |
| AW591461 | x192h06.x1 NCI_CGAP_Ut1 Homo sapiens cDNA clone IMAGE:2682203 3, mRNA sequence | 5.45 | Down | 1.29E-04 |
| NM_007072 | Homo sapiens HERV-H LTR-associating 2 (HHLA2), mRNA | 5.45 | Down | 1.18E-03 |
| NM_181718 | Homo sapiens hypothetical protein LOC253982 (LOC253982), mRNA | 5.44 | Down | 6.64E-03 |
| AI559193 | tq42h01.x1 NCI_CGAP_Ut1 Homo sapiens cDNA clone IMAGE:2211505 3, mRNA sequence | 5.44 | Down | 2.64E-03 |
| | 7k31b04.x1 NCI_CGAP_Ov18 Homo sapiens | 5.43 | Down | 4.72E-03 |
| BF058471 | cDNA clone IMAGE:3477054 3 similar to contains element MER17 repetitive element ;, mRNA sequence | | | |
| AK130181 | Homo sapiens cDNA FLJ26671 fis, clone MPG03325 | 5.42 | Down | 4.50E-05 |
| NM_178039 | Homo sapiens Rab6-interacting protein 2 (ELKS), transcript variant delta, mRNA | 5.41 | Down | 9.73E-04 |
| NM_019025 | Homo sapiens spermine oxidase (SMOX), transcript variant 5, mRNA | 5.4 | Down | 1.11E-02 |
| BX108809 | BX108809 Soares fetal liver spleen 1 N FLS Homo sapiens cDNA clone IMAGp998L05373 ; IMAGE:195988, mRNA sequence | 5.4 | Down | 4.43E-02 |
| NM_019607 | Homo sapiens hypothetical protein FLJ11267 (FLJ11267), mRNA | 5.4 | Down | 1.76E-02 |
| NM_001343 | Homo sapiens disabled homolog 2, mitogen-responsive phosphoprotein (Drosophila) (DAB2), mRNA | 5.39 | Down | 3.11E-03 |
| NM_000087 | Homo sapiens cyclic nucleotide gated channel alpha 1 (CNGA1), mRNA | 5.34 | Down | 6.52E-04 |
| NM_031894 | Homo sapiens ferritin, heavy polypeptide-like 17 (FTHL17), mRNA | 5.34 | Down | 4.47E-03 |
| NM_031419 | Homo sapiens nuclear factor of kappa light polypeptide gene enhancer in B-cells inhibitor, zeta (NFKBIZ), transcript variant 1, mRNA | 5.33 | Down | 7.27E-03 |
| BG199496 | RST18780 Athersys RAGE Library Homo sapiens cDNA, mRNA sequence | 5.3 | Down | 4.14E-04 |
| NM_001958 | Homo sapiens eukaryotic translation elongation factor 1 alpha 2 (EEF1A2), mRNA | 5.29 | Down | 1.97E-04 |
| NM_001878 | Homo sapiens cellular retinoic acid binding protein 2 (CRABP2), mRNA | 5.29 | Down | 5.17E-04 |
| AW014126 | UI-H-BI0-aaj-a-05-0-UI.s1 NCI_CGAP_Sub1 Homo sapiens cDNA clone IMAGE:2709393 3, mRNA sequence | 5.29 | Down | 9.98E-04 |
| NM_006072 | Homo sapiens chemokine (C-C motif) ligand 26 (CCL26), mRNA | 5.28 | Down | 5.45E-03 |
| X70287 | H.sapiens gene for thioredoxin, exons 2 and 3 | 5.27 | Down | 2.75E-02 |
| NM_014867 | Homo sapiens KIAA0711 gene product (KIAA0711), mRNA | 5.27 | Down | 1.44E-03 |
| BX100098 | BX100098 Soares_pregnant uterus_NbHPU Homo sapiens cDNA clone IMAGp998C021198 ; IMAGE:502201, mRNA sequence | 5.26 | Down | 2.59E-04 |
| AK055334 | Homo sapiens cDNA FLJ30772 fis, clone FEBRA2000757, moderately similar to Homo sapiens BM-009 mRNA | 5.24 | Down | 8.92E-04 |
| BU567804 | AGENCOURT_10398872 NH_MGC_82 Homo sapiens cDNA clone IMAGE:6614502 5, mRNA sequence | 5.24 | Down | 2.28E-02 |
| NM_006332 | Homo sapiens interferon, gamma-inducible protein 30 (IFI30), mRNA | 5.24 | Down | 1.75E-03 |
| BF111903 | 7138d07.x1 Soares_NSF_F8_9W_OT_PA_P_S1 Homo sapiens cDNA clone IMAGE:3523644 3, mRNA sequence | 5.23 | Down | 4.43E-05 |
| NM_006727 | Homo sapiens cadherin 10, type 2 (T2-cadherin) (CDH10), mRNA | 5.2 | Down | 1.84E-04 |
| NM_003810 | Homo sapiens tumor necrosis factor (ligand) superfamily, member 10 (TNFSF10), mRNA | 5.19 | Down | 2.91E-03 |
| AK127421 | Homo sapiens cDNA FLJ45513 fis, clone BRTHA2021450 | 5.18 | Down | 8.00E-05 |
| NM_024897 | Homo sapiens progestin and adipoQ receptor family member VI (PAQR6), transcript variant 1, mRNA | 5.18 | Down | 2.69E-05 |
| BU729783 | UI-E-CK1-afh-h-18-0-UI.s1 UI-E-CK1 Homo sapiens cDNA clone UI-E-CK1-afh-h-18-0-UI 3, mRNA sequence | 5.17 | Down | 1.52E-04 |
| BX107838 | BX107838 NCI_CGAP_Lu5 Homo sapiens cDNA clone IMAGp998A153853 ; IMAGE:1521686, mRNA sequence | 5.16 | Down | 4.26E-04 |
| NM_004335 | Homo sapiens bone marrow stromal cell antigen 2 (BST2), mRNA | 5.16 | Down | 7.11E-05 |
| AK021637 | Homo sapiens cDNA FLJ11575 fis, clone HEMBA1003531 | 5.15 | Down | 6.53E-05 |
| NM_014890 | Homo sapiens downregulated in ovarian cancer 1 (DOC1), transcript variant 2, mRNA | 5.15 | Down | 9.70E-05 |
| NM_003E97 | Homo sapiens TGFB inducible early growth response 2 (TIEG2), mRNA | 5.14 | Down | 1.19E-03 |
| AI684824 | wa86a12.x1 Soares_NFL_T_GBC_S1 Homo sapiens cDNA clone IMAGE:2303038 3, mRNA sequence | 5.13 | Down | 9.14E-04 |
| NM_002522 | Homo sapiens neuronal pentraxin I (NPTX1), mRNA | 5.12 | Down | 1.52E-03 |
| NM_002250 | Homo sapiens potassium intermediate/small conductance calcium-activated channel, subfamily N, member 4 (KCNN4), mRNA | 5.12 | Down | 1.28E-04 |
| NM_001305 | Homo sapiens claudin 4 (CLDN4), mRNA | 5.11 | Down | 3.26E-03 |
| BC015108 | Homo sapiens, Similar to otoconin 90, clone IMAGE:4044247, mRNA | 5.1 | Down | 2.17E-04 |
| N51335 | yz15e08.s1 Soares_multiple_sclerosis_2NbHMSP Homo sapiens cDNA clone IMAGE:283142 3, mRNA sequence | 5.1 | Down | 2.89E-05 |
| NM_001002295 | Homo sapiens GATA binding protein 3 (GATA3), transcript variant 1, mRNA | 5.06 | Down | 3.85E-03 |
| NM_013281 | Homo sapiens fibronectin leucine rich transmembrane protein 3 (FLRT3), transcript variant 1, mRNA | 5.05 | Down | 5.04E-03 |
| AI623139 | tu89b07.x1 NCI_CGAP_Gas4 Homo sapiens cDNA clone IMAGE:2258197 3, mRNA sequence | 5.04 | Down | 4.25E-03 |
| NM_022168 | Homo sapiens interferon induced with helicase C domain 1 (IFIH1), mRNA | 5.04 | Down | 2.48E-03 |
| BC05172 7 | Homo sapiens cDNA clone IMAGE:5265929, partial cds | 5.02 | Down | 5.42E-04 |

**TABLE VII F: GENES THAT WERE DIFFERENTIALLY EXPRESSED AT LEAST 5 FOLD IN AF-I VERSUS AF-III CELLS**

| Gene Identifier | Gene Name | Average fold change in AF-I cells versus AF-III cells | Direction | adj. p-value |
|---|---|---|---|---|
| NM_080872 | Homo sapiens unc-5 homolog D (C. elegans) (UNC5D), mRNA | 331.21 | Up | 5.22E-06 |
| NM_000090 | Homo sapiens collagen, type III, alpha 1 (Ehlers-Danlos syndrome type IV, autosomal dominant) (COL3A1), mRNA | 310.82 | Up | 3.56E-06 |
| NM_138961 | Homo sapiens endothelial cell adhesion molecule (ESAM), mRNA | 262 | Up | 6.27E-06 |
| AK021531 | Homo sapiens cDNA FLJ11469 fis, clone HEMBA1001658 | 205.55 | Up | 3.10E-06 |
| NM_000474 | Homo sapiens twist homolog 1 (acrocephalosyndactyly 3; Saethre-Chotzen syndrome) (Drosophila) (TWIST1), mRNA | 116.12 | Up | 2.29E-05 |
| BX089554 | BX089554 Soares placenta Nb2HP Homo sapiens cDNA clone IMAGp998P07210 ; IMAGE:142326, mRNA sequence | 97.02 | Up | 3.10E-06 |
| NM_018013 | Homo sapiens hypothetical protein FLJ10159 (FLJ10159), mRNA | 88.82 | Up | 3.10E-06 |
| M_182798 | Homo sapiens hypothetical protein FLJ39155 (FLJ39155), transcript variant 2, mRNA | 86.73 | Up | 3.10E-06 |
| NM_052954 | Homo sapiens cysteine and tyrosine-rich 1 (CYYR1), mRNA | 85.9 | Up | 3.10E-06 |
| NM_000867 | Homo sapiens 5-hydroxytryptamine (serotonin) receptor 2B (HTR2B), mRNA | 74.02 | Up | 3.19E-06 |
| NM_014178 | Homo sapiens syntaxin binding protein 6 (amisyn) (STXBP6), mRNA | 71.46 | Up | 8.25E-06 |
| U83115 | Human non-lens beta gamma-crystallin like protein (AIM1) mRNA, partial cds | 66.41 | Up | 8.66E-06 |
| CA429135 | UI-H-FH1-bfh-k-22-0-UI.s1 NCI CGAP FH1 Homo sapiens cDNA clone UI-H-FH1-bfh-k-22-0-UI 3, mRNA sequence | 66.03 | Up | 2.19E-06 |
| BQ003501 | UI-H-EI1-azd-p-06-0-UI.s1 NCI_CGAP_EI1 Homo sapiens cDNA clone IMAGE:5847413 3, mRNA sequence | 54.48 | Up | 1.35E-05 |
| BX105152 | BX105152 Soares_testis_NHT Homo sapiens cDNA clone IMAGp998J212575 ; IMAGE:1031156, mRNA sequence | 50.41 | Up | 5.63E-06 |
| NM_006393 | Homo sapiens nebulette (NEBL), transcript variant 1, mRNA | 50.35 | Up | 1.43E-05 |
| NM_001432 | Homo sapiens epiregulin (EREG), mRNA | 49.79 | Up | 6.21E-06 |
| AI124557 | am58g02.x1 Johnston frontal cortex Homo sapiens cDNA clone IMAGE:1539794 3, mRNA sequence | 45.53 | Up | 3.19E-06 |
| NM_018286 | Homo sapiens hypothetical protein FLJ10970 (FLJ10970), mRNA | 44.54 | Up | 1.84E-05 |
| AI962169 | wq45c10.x1 NCI_CGAP_GC6 Homo sapiens cDNA clone IMAGE:2474226 3, mRNA sequence | 44.13 | Up | 3.20E-05 |
| NM_003381 | Homo sapiens vasoactive intestinal peptide (VIP), transcript variant 1, mRNA | 42.63 | Up | 1.22E-05 |
| NM_018371 | Homo sapiens chondroitin beta1,4 N-acetylgalactosaminyltransferase (ChGn), mRNA | 42.53 | Up | 2.67E-05 |
| NM_181481 | Homo sapiens chromosome 18 open reading frame 1 (C18orf1), transcript variant a1, mRNA | 41.33 | Up | 6.27E-06 |
| BQ025821 | UI-1-BB1p-aye-f-10-0-UI.s1 NCI_CGAP_PI6 Homo sapiens cDNA clone UI-1-BB1p-aye-f-10-0-UI 3, mRNA sequence | 39.96 | Up | 3.10E-06 |
| NM_152754 | Homo sapiens sema domain, immunoglobulin domain (Ig), short basic domain, secreted, (semaphorin) 3D (SEMA3D), mRNA | 37.45 | Up | 3.17E-05 |
| NM_002928 | Homo sapiens regulator of G-protein signalling 16 (RGS16), mRNA | 36.89 | Up | 2.02E-05 |
| H85497 | yv88b07.r1 Soares melanocyte 2NbHM Homo sapiens cDNA clone IMAGE:249781 5, mRNA sequence | 35.37 | Up | 8.96E-05 |
| BF515657 | UI-H-BW1-anu-e-05-0-UI.s1 NCI_CGAP_Sub7 Homo sapiens cDNA clone IMAGE:3083601 3, mRNA sequence | 34.85 | Up | 5.22E-06 |
| BE465760 | hw22f09.x1 NCI_CGAP_Kid11 Homo sapiens cDNA clone IMAGE:3183689 3, mRNA sequence | 34.39 | Up | 1.39E-05 |
| AK027128 | Homo sapiens cDNA: FLJ23475 fis, clone HSI13659 | 33.21 | Up | 4.37E-06 |
| BC040678 | Homo sapiens, clone IMAGE:4817707, mRNA | 32.1 | Up | 3.37E-05 |
| NM_001864 | Homo sapiens cytochrome c oxidase subunit VIIa polypeptide 1 (muscle) (COX7A1), mRNA | 30.71 | Up | 1.69E-05 |
| AK096708 | Homo sapiens cDNA FLJ39389 fis, clone PLACE6003621 | 28.39 | Up | 8.85E-06 |
| NM_005308 | Homo sapiens G protein-coupled receptor kinase 5 (GRK5), mRNA | 27.96 | Up | 1.34E-05 |
| CF137545 | UI-HF-BN0-ane-d-05-0-UI.r1 NIH_MGC_50 Homo sapiens cDNA clone IMAGE:3092384 5, mRNA sequence | 27.55 | Up | 2.39E-05 |
| NM_053281 | Homo sapiens dachshund homolog 2 (Drosophila) (DACH2), mRNA | 25.26 | Up | 5.22E-05 |
| NM_005595 | Homo sapiens nuclear factor I/A (NFIA), mRNA | 23.76 | Up | 2.51E-05 |
| BM685124 | UI-E-EJ1-ajl-I-13-0-UI.s1 UI-E-EJ1 Homo sapiens cDNA clone UI-E-EJ1-ajl-I-13-0-UI 3, mRNA sequence | 23.64 | Up | 1.16E-05 |
| | Homo sapiens dynein, cytoplasmic, | 23.55 | Up | 4.37E-06 |
| NM_004411 | intermediate polypeptide 1 (DNCI1), mRNA | | | |
| NM_032918 | Homo sapiens RAS-like, estrogen-regulated, growth inhibitor (RERG), mRNA | 23 | Up | 3.96E-06 |
| NM_020997 | Homo sapiens left-right determination factor 1 (LEFTY1), mRNA | 22.48 | Up | 9.37E-06 |
| N33310 | yy39g10.s1 Soares melanocyte 2NbHM Homo sapiens cDNA clone IMAGE:273666 3, mRNA sequence | 22.37 | Up | 1.57E-05 |
| BM713465 | UI-E-EJ0-aho-m-22-0-UI.r1 UI-E-EJ0 Homo sapiens cDNA clone UI-E-EJ0-aho-m-22-0-UI 5, mRNA sequence | 21.7 | Up | 8.98E-06 |
| AK095053 | Homo sapiens cDNA FLJ37734 fis, clone BRHIP2020842 | 21.49 | Up | 2.37E-05 |
| BC016722 | Homo sapiens cDNA clone IMAGE:4075924, partial cds | 20.91 | Up | 7.23E-04 |
| AW445209 | UI-H-B13-akc-g-11-0-UI.s1 NCI_CGAP_Sub5 Homo sapiens cDNA clone IMAGE:2733908 3, mRNA sequence | 20.04 | Up | 6.27E-06 |
| AI032876 | ow13g03.x1 Soares_parathyroid_tumor_NbHPA Homo sapiens cDNA clone IMAGE:1646740 3, mRNA sequence | 19.56 | Up | 2.18E-05 |
| BG389328 | 602413981F1 NIH_MGC_92 Homo sapiens cDNA clone IMAGE:4522269 5, mRNA sequence | 18.94 | Up | 9.37E-06 |
| | Homo sapiens T-cell lymphoma breakpoint | 18.74 | Up | 5.63E-06 |
| M_153355 | associated target 1 (TCBA1), mRNA | | | |
| BX089554 | BX089554 Soares placenta Nb2HP Homo sapiens cDNA clone IMAGp998P07210 ; IMAGE:142326, mRNA sequence | 18.36 | Up | 1.12E-06 |
| NM_002518 | Homo sapiens neuronal PAS domain protein 2 (NPAS2), mRNA | 17.94 | Up | 2.19E-06 |
| AI342246 | qt26g09.x1 Soares_pregnant_uterus_NbHPU Homo sapiens cDNA clone IMAGE:1949152 3, mRNA sequence | 17.68 | Up | 8.37E-06 |
| NM_170744 | Homo sapiens unc-5 homolog B (C. elegans) (UNC5B), mRNA | 17.32 | Up | 1.99E-05 |
| AI951740 | wv38h09.x1 NCI_CGAP_Ov18 Homo sapiens cDNA clone IMAGE:2531873 3, mRNA sequence | 17.24 | Up | 8.84E-05 |
| NM_005110 | Homo sapiens glutamine-fructose-6-phosphate transaminase 2 (GFPT2), mRNA | 16.65 | Up | 8.86E-06 |
| NM_015564 | Homo sapiens leucine rich repeat transmembrane neuronal 2 (LRRTM2), mRNA | 16.45 | Up | 2.72E-05 |
| BC043411 | Homo sapiens, clone IMAGE:6155889, mRNA | 16.38 | Up | 3.10E-06 |
| M_153183 | Homo sapiens nudix (nucleoside diphosphate linked moiety X)-type motif 10 (NUDT10), mRNA | 16.25 | Up | 1.45E-05 |
| NM_005320 | Homo sapiens histone 1, H1d (HIST1H1D), mRNA | 16.24 | Up | 4.43E-04 |
| W93585 | zd95g01.s1 Soares_fetal_heart_NbHH19W Homo sapiens cDNA clone IMAGE:357264 3, mRNA sequence | 16.22 | Up | 1.39E-05 |
| NM_002771 | Homo sapiens protease, serine, 3 (mesotrypsin) (PRSS3), mRNA | 16.15 | Up | 3.20E-05 |
| NM_013387 | Homo sapiens ubiquinol-cytochrome c reductase complex (7.2 kD) (HSPC051), transcript variant 1, mRNA | 16.09 | Up | 3.10E-06 |
| NM_001853 | Homo sapiens collagen, type IX, alpha 3 (COL9A3), mRNA | 15.86 | Up | 4.01E-05 |
| NM_014802 | Homo sapiens KIAA0528 gene product (KIAA0528), mRNA | 15.81 | Up | 5.11E-05 |
| AF216077 | Homo sapiens clone HB-2 mRNA sequence | 15.64 | Up | 2.71E-05 |
| AI686652 | tu35d06.x1 NCI_CGAP_Pr28 Homo sapiens cDNA clone IMAGE:2253035 3, mRNA sequence | 15.12 | Up | 5.07E-06 |
| AI086279 | oz40h01.s1 Soares_NhHMPu_S1 Homo sapiens cDNA clone IMAGE:1677841 3, mRNA sequence | 14.96 | Up | 2.47E-05 |
| NM_001146 | Homo sapiens angiopoietin 1 (ANGPT1), transcript variant 1, mRNA | 14.38 | Up | 1.29E-05 |
| NM_021643 | Homo sapiens tribbles homolog 2 (Drosophila) (TRIB2), mRNA | 14.32 | Up | 6.48E-06 |
| NM_020927 | Homo sapiens KIAA1576 protein (KIAA1576), mRNA | 14.14 | Up | 1.61E-05 |
| W69644 | zd45f10.r1 Soares_fetal_heart_NbHH19W Homo sapiens cDNA clone IMAGE:343627 5, mRNA sequence | 14.01 | Up | 1.22E-05 |
| NM_012204 | Homo sapiens general transcription factor IIIC, polypeptide 4, 90kDa (GTF3C4), mRNA | 13.95 | Up | 1.41E-05 |
| BM663928 | UI-E-CI1-afw-p-01-0-UI.s1 UI-E-CI1 Homo sapiens cDNA clone UI-E-CI1-afw-p-01-0-UI 3, mRNA sequence | 13.53 | Up | 8.04E-05 |
| BM724062 | UI-E-E01-aiy-a-22-0-UI.r1 UI-E-EO1 Homo sapiens cDNA clone UI-E-EO1-aiy-a-22-0-UI 5, mRNA sequence | 13.51 | Up | 3.19E-06 |
| H51050 | yp84e11.s1 Soares fetal liver spleen 1 NFLS Homo sapiens cDNA clone IMAGE:194156 3, mRNA sequence | 13.39 | Up | 1.94E-05 |
| BX111353 | BX111353 NCI_CGAP_Kid3 Homo sapiens cDNA clone IMAGp9980094576 ; IMAGE:1869152, mRNA sequence | 13.38 | Up | 3.92E-05 |
| NM_054027 | Homo sapiens ankylosis, progressive homolog (mouse) (ANKH), mRNA | 13.27 | Up | 3.33E-05 |
| NM_152550 | Homo sapiens SH3 domain containing ring finger 2 (SH3RF2), mRNA | 13.06 | Up | 2.23E-05 |
| NM_033082 | Homo sapiens cytokine induced protein 29 kDa (CIP29), mRNA | 12.87 | Up | 5.63E-06 |
| | zr47e10.r1 Soares_NhHMPu_S1 Homo sapiens | 12.47 | Up | 6.41E-05 |
| AA232643 | cDNA clone IMAGE:666570 5, mRNA sequence | | | |
| BX103846 | BX103846 NCI_CGAP _Lu24 Homo sapiens cDNA clone IMAGp998F125811 ; IMAGE:2342027, mRNA sequence | 12.4 | Up | 1.39E-05 |
| NM_016140 | Homo sapiens brain specific protein (CGI-38), mRNA | 12.26 | Up | 2.71E-05 |
| AW043793 | wy76d11.x1 Soares_NSF_F8_9W_OT_PA_P_S1 Homo sapiens cDNA clone IMAGE:2554485 3 similar to contains element MER18 repetitive element ;, mRNA sequence | 12.04 | Up | 6.21E-06 |
| NM_020226 | Homo sapiens PR domain containing 8 (PRDM8), mRNA | 12.01 | Up | 5.32E-06 |
| BX113590 | BX113590 Soares_fetal_heart_NbHH19W Homo sapiens cDNA clone IMAGp998A14871 ; IMAGE:376597, mRNA sequence | 11.97 | Up | 3.09E-05 |
| AK127644 | Homo sapiens cDNA FLJ45742 fis, clone KIDNE2016327 | 11.91 | Up | 7.34E-05 |
| BC046364 | Homo sapiens flavoprotein oxidoreductase MICAL3, mRNA (cDNA clone IMAGE:5737121), with apparent retained intron | 11.88 | Up | 1.87E-05 |
| AI821210 | ne08e05.y5 NCI_CGAP_Co3 Homo sapiens cDNA clone IMAGE:880640 5, mRNA sequence | 11.79 | Up | 4.15E-03 |
| AK128288 | Homo sapiens cDNA FLJ46426 fis, clone THYMU3013897 | 11.76 | Up | 2.88E-04 |
| NM_033641 | Homo sapiens collagen, type IV, alpha 6 (COL4A6), transcript variant B, mRNA | 11.56 | Up | 2.05E-05 |
| NM_004460 | Homo sapiens fibroblast activation protein, alpha (FAP), mRNA | 11.54 | Up | 1.40E-05 |
| A1792426 | qe47a12.y5 Soares_fetal_lung_NbHL19W Homo sapiens cDNA clone IMAGE:1742110 5 similar to contains MER22.b3 MER18 repetitive element;, mRNA sequence | 11.41 | Up | 1.33E-03 |
| NM_018374 | Homo sapiens hypothetical protein FLJ11273 (FLJ11273), mRNA | 11.23 | Up | 3.24E-04 |
| BM802920 | AGENCOURT_6457446 NIH_MGC_88 Homo sapiens cDNA clone IMAGE:5560288 5, mRNA sequence | 11.11 | Up | 8.59E-05 |
| AA043255 | zk49f07.s1 Soares_pregnant_uterus_NbHPU Homo sapiens cDNA clone IMAGE:486181 3, mRNA sequence | 11.1 | Up | 2.64E-05 |
| NM_001847 | Homo sapiens collagen, type IV, alpha 6 (COL4A6), transcript variant A, mRNA | 10.98 | Up | 9.89E-06 |
| AK124778 | Homo sapiens cDNA FLJ42788 fis, clone BRAWH3007129 | 10.95 | Up | 9.27E-05 |
| AK130306 | Homo sapiens cDNA FLJ26796 fis, clone PRS05079 | 10.86 | Up | 1.02E-04 |
| AK023739 | Homo sapiens cDNA FLJ13677 fis, clone PLACE1011982 | 10.83 | Up | 6.24E-05 |
| | Homo sapiens mRNA; cDNA DKFZp451B0818 | 10.8 | Up | 5.63E-06 |
| AL832624 | (from clone DKFZp451 B0818) | | | |
| BC014344 | Homo sapiens, Similar to arylacetamide deacetylase, clone IMAGE:3934567, mRNA | 10.68 | Up | 2.37E-05 |
| NM_000955 | Homo sapiens prostaglandin E receptor 1 (subtype EP1), 42kDa (PTGER1), mRNA | 10.66 | Up | 1.14E-05 |
| CD001963 | C24B1 subtracted cochlea cDNA library Homo sapiens cDNA, mRNA sequence | 10.47 | Up | 7.71E-06 |
| NM_032261 | Homo sapiens chromosome 21 open reading frame 56 (C21orf56), mRNA | 10.42 | Up | 4.73E-06 |
| BC036004 | Homo sapiens, clone IMAGE:4730399, mRNA | 10.3 | Up | 3.06E-05 |
| NM_006408 | Homo sapiens anterior gradient 2 homolog (Xenopus laevis) (AGR2), mRNA | 10.28 | Up | 4.23E-05 |
| AB037805 | Homo sapiens mRNA for KIAA1384 protein, partial cds | 10.24 | Up | 8.03E-05 |
| BF964783 | 602268030F1 NIH_MGC_81 Homo sapiens cDNA clone IMAGE:4356246 5, mRNA sequence | 10.02 | Up | 1.41E-05 |
| NM_000963 | Homo sapiens prostaglandin-endoperoxide synthase 2 (prostaglandin G/H synthase and cyclooxygenase) (PTGS2), mRNA | 9.91 | Up | 5.63E-06 |
| | Homo sapiens cDNA FLJ35222 fis, clone | 9.88 | Up | 4.65E-02 |
| AK092541 | PROST2000835 | | | |
| NM_007361 | Homo sapiens nidogen 2 (osteonidogen) (NID2), mRNA | 9.68 | Up | 5.30E-06 |
| NM_013231 | Homo sapiens fibronectin leucine rich transmembrane protein 2 (FLRT2), mRNA | 9.58 | Up | 1.43E-05 |
| NM_002667 | Homo sapiens phospholamban (PLN), mRNA | 9.55 | Up | 9.83E-04 |
| AF414442 | Homo sapiens ovarian cancer related tumor marker CA125 mRNA, complete cds | 9.51 | Up | 1.11E-03 |
| AK125453 | Homo sapiens cDNA FLJ43464 fis, clone OCBBF2036225 | 9.48 | Up | 1.15E-02 |
| W38393 | zb15c07.r1 Soares_fetal_lung_NbHL19W Homo sapiens cDNA clone IMAGE:302124 5, mRNA sequence | 9.39 | Up | 5.52E-05 |
| NM_152775 | Homo sapiens hypothetical protein MGC33607 (MGC33607), mRNA | 9.29 | Up | 2.35E-05 |
| AK091731 | Homo sapiens cDNA FLJ34412 fis, clone HEART2002432 | 9.07 | Up | 2.06E-05 |
| AK025909 | Homo sapiens cDNA: FLJ22256 fis, clone HRC02860 | 9.05 | Up | 4.45E-05 |
| D62676 | HUM313C12B Clontech human aorta polyA+ mRNA (#6572) Homo sapiens cDNA clone GEN-313C12 5, mRNA sequence | 9.02 | Up | 1.38E-03 |
| | | | | |
| AK129955 | Homo sapiens cDNA FLJ26445 fis, clone KDN02608 | 8.98 | Up | 8.52E-05 |
| R22189 | yh26a02.s1 Soares placenta Nb2HP Homo sapiens cDNA clone IMAGE:130826 3, mRNA sequence | 8.94 | Up | 2.29E-05 |
| AW963062 | EST375135 MAGE resequences, MAGH Homo sapiens cDNA, mRNA sequence | 8.9 | Up | 1.76E-04 |
| AW044502 | wx22g05.x1 NCI_CGAP_Kid11 Homo sapiens cDNA clone IMAGE:2544440 3, mRNA sequence | 8.89 | Up | 1.35E-05 |
| NM_014222 | Homo sapiens NADH dehydrogenase (ubiquinone) 1 alpha subcomplex, 8, 19kDa (NDUFA8), nuclear gene encoding mitochondrial protein, mRNA | 8.88 | Up | 4.45E-05 |
| AK124562 | Homo sapiens cDNA FLJ42571 fis, clone BRACE3008036 | 8.82 | Up | 8.86E-06 |
| NM_002261 | Homo sapiens killer cell lectin-like receptor subfamily C, member 3 (KLRC3), transcript variant NKG2-E, mRNA | 8.75 | Up | 9.45E-05 |
| BC012900 | Homo sapiens, clone IMAGE:3881549, mRNA | 8.71 | Up | 1.61E-04 |
| R53688 | yg84h04.r1 Soares infant brain 1 NIB Homo sapiens cDNA clone IMAGE:40175 5, mRNA sequence | 8.49 | Up | 1.74E-05 |
| AL133118 | Homo sapiens mRNA; cDNA DKFZp586N0121 (from clone DKFZp586N0121) | 8.47 | Up | 6.83E-05 |
| NM_002260 | Homo sapiens killer cell lectin-like receptor subfamily C, member 2 (KLRC2), mRNA | 8.44 | Up | 1.61E-04 |
| NM_002421 | Homo sapiens matrix metalloproteinase 1 (interstitial collagenase) (MMP1), mRNA | 8.34 | Up | 5.17E-04 |
| NM_005328 | Homo sapiens hyaluronan synthase 2 (HAS2), mRNA | 8.18 | Up | 2.35E-04 |
| NM_152423 | Homo sapiens melanoma associated antigen (mutated) 1-like 1 (MUM1L1), mRNA | 8.12 | Up | 6.31E-05 |
| NM_004848 | Homo sapiens chromosome 1 open reading frame 38 (C1 orf38), mRNA | 8.05 | Up | 5.83E-05 |
| NM_006617 | Homo sapiens nestin (NES), mRNA | 8.01 | Up | 8.50E-06 |
| C02345 | HUMGS0007544 Human adult (K.Okubo) Homo sapiens cDNA, mRNA sequence | 8 | Up | 3.09E-05 |
| NM_000900 | Homo sapiens matrix Gla protein (MGP), mRNA | 7.97 | Up | 3.88E-05 |
| N54656 | yz08h05.r1 Soares_multiple_sclerosis_2NbHMSP Homo sapiens cDNA clone IMAGE:282489 5, mRNA sequence | 7.97 | Up | 4.17E-05 |
| AK095726 | Homo sapiens cDNA FLJ38407 fis, clone FEBRA2008859 | 7.97 | Up | 1.84E-05 |
| BC027461 | Homo sapiens cDNA clone IMAGE:2984900, containing frame-shift errors | 7.95 | Up | 2.18E-04 |
| AK096061 | Homo sapiens cDNA FLJ38742 fis, clone KIDNE2012009 | 7.91 | Up | 1.32E-05 |
| NM_001001931 | Homo sapiens mitochondrial tumor suppressor 1 (MTUS1), nuclear gene encoding mitochondrial protein, transcript variant 4, mRNA | 7.84 | Up | 1.22E-05 |
| NM_133504 | Homo sapiens decorin (DCN), transcript variant B, mRNA | 7.82 | Up | 6.70E-06 |
| NM_018427 | Homo sapiens RRN3 RNA polymerase I transcription factor homolog (yeast) (RRN3), mRNA | 7.76 | Up | 1.39E-05 |
| NM_006528 | Homo sapiens tissue factor pathway inhibitor 2 (TFPI2), mRNA | 7.73 | Up | 3.10E-06 |
| CN371168 | 17000600077294 GRN_PREHEP Homo sapiens cDNA 5, mRNA sequence | 7.63 | Up | 1.83E-05 |
| AI911957 | wd78b01.x1 NCI_CGAP_Lu24 Homo sapiens cDNA clone IMAGE:2337673 3, mRNA sequence | 7.61 | Up | 1.82E-05 |
| NM_058187 | Homo sapiens chromosome 21 open reading frame 63 (C21orf63), mRNA | 7.59 | Up | 9.08E-05 |
| NM_004235 | Homo sapiens Kruppel-like factor 4 (gut) (KLF4), mRNA | 7.52 | Up | 1.43E-05 |
| AL832916 | Homo sapiens mRNA; cDNA DKFZp76210915 (from clone DKFZp76210915) | 7.51 | Up | 6.70E-06 |
| BG118019 | 602351269F1 NIH_MGC_90 Homo sapiens cDNA clone IMAGE:4446065 5, mRNA sequence | 7.49 | Up | 1.43E-05 |
| AW770283 | h176b02.x1 NCI_CGAP_Kid13 Homo sapiens cDNA clone IMAGE:3007083 3, mRNA sequence | 7.41 | Up | 1.38E-04 |
| T47612 | yb15h03.s1 Stratagene placenta (#937225) Homo sapiens cDNA clone IMAGE:71285 3, mRNA sequence | 7.35 | Up | 9.37E-06 |
| BM998303 | UI-H-DT1-awc-h-03-0-UI.s1 NCI_CGAP_DT1 Homo sapiens cDNA clone IMAGE:5887538 3, mRNA sequence | 7.31 | Up | 2.23E-05 |
| AK092048 | Homo sapiens cDNA FLJ34729 fis, clone MESAN2006401 | 7.26 | Up | 1.57E-05 |
| BX647541 | Homo sapiens mRNA; cDNA DKFZp686P0492 (from clone DKFZp686P0492) | 7.24 | Up | 2.71E-05 |
| AI033863 | cow10e02.x1 Soares_parathyroid_tumor_NbHPA Homo sapiens cDNA clone IMAGE:1646426 3, mRNA sequence | 7.16 | Up | 7.24E-06 |
| NM_004657 | Homo sapiens serum deprivation response (phosphatidylserine binding protein) (SDPR), mRNA | 7.16 | Up | 3.17E-05 |
| BF222640 | 7p57c03.x1 NCI_CGAP_Pr28 Homo sapiens cDNA clone IMAGE:3649901 3, mRNA sequence | 7.13 | Up | 1.37E-04 |
| BU620793 | UI-H-FL1-bfx-d-10-0-UI.s1 NCI_CGAP_FL1 Homo sapiens cDNA clone UI-H-FL1-bfx-d-10-0-UI 3, mRNA sequence | 7.11 | Up | 1.46E-05 |
| AA102553 | zn26a04.s1 Stratagene neuroepithelium NT2RAMI 937234 Homo sapiens cDNA clone IMAGE:548526 3, mRNA sequence | 7.01 | Up | 4.89E-05 |
| NM_021242 | Homo sapiens MID1 interacting protein 1 (gastrulation specific G12-like (zebrafish)) (MID1IP1), mRNA | 6.96 | Up | 1.32E-04 |
| CB045035 | NISC_gc08b02.x1 NCI_CGAP_Co17 Homo sapiens cDNA clone IMAGE:3217875 3, mRNA sequence | 6.94 | Up | 1.32E-04 |
| NM_007063 | Homo sapiens TBC1 domain family, member 8 (with GRAM domain) (TBC1D8), mRNA | 6.92 | Up | 7.24E-06 |
| H25898 | yl55b10.r1 Soares breast 3NbHBst Homo sapiens cDNA clone IMAGE:162139 5, mRNA sequence | 6.91 | Up | 5.94E-04 |
| AK091337 | Homo sapiens cDNA FLJ34018 fis, clone FCBBF2002801 | 6.89 | Up | 1.05E-04 |
| BE877764 | 601486331 F1 NIH_MGC_69 Homo sapiens cDNA clone IMAGE:3888943 5, mRNA sequence | 6.88 | Up | 2.25E-04 |
| BE503916 | hz35g01.x1 NCI_CGAP_GC6 Homo sapiens cDNA clone IMAGE:3210000 3, mRNA sequence | 6.73 | Up | 2.28E-05 |
| NM_024993 | Homo sapiens leucine rich repeat transmembrane neuronal 4 (LRRTM4), mRNA | 6.69 | Up | 2.67E-05 |
| | Homo sapiens nidogen 2 (osteonidogen) | 6.65 | Up | 2.63E-04 |
| NM_007361 | (NID2), mRNA | | | |
| BC070147 | Homo sapiens cDNA clone IMAGE:4672631, containing frame-shift errors | 6.65 | Up | 1.35E-05 |
| BC045666 | Homo sapiens tumor protein p53 inducible protein 11, mRNA (cDNA clone IMAGE:5298525), containing frame-shift errors | 6.62 | Up | 1.80E-04 |
| NM_017787 | Homo sapiens chromosome 10 open reading frame 26 (C10orf26), mRNA | 6.59 | Up | 3.69E-05 |
| BQ267806 | ij94e04.x1 Human insulinoma Homo sapiens cDNA clone IMAGE:5779278 3, mRNA sequence | 6.59 | Up | 1.39E-04 |
| NM_032578 | Homo sapiens myopalladin (FLJ14437), mRNA | 6.57 | Up | 1.43E-05 |
| AK124699 | Homo sapiens cDNA FLJ42709 fis, clone BRAMY3007350 | 6.55 | Up | 1.67E-05 |
| NM_000921 | Homo sapiens phosphodiesterase 3A, cGMP-inhibited (PDE3A), mRNA | 6.51 | Up | 3.13E-05 |
| CB048134 | NISC_gj03a09.y1 NCI_CGAP_Pr28 Homo sapiens cDNA clone IMAGE:3270448 5, mRNA sequence | 6.47 | Up | 8.86E-06 |
| CA843592 | ir49c12.x1 HR85 islet Homo sapiens cDNA clone IMAGE:6548544 3, mRNA sequence | 6.44 | Up | 3.85E-05 |
| NM_024633 | Homo sapiens chromosome 14 open reading frame 139 (C14orf139), mRNA | 6.42 | Up | 2.45E-04 |
| BX640685 | Homo sapiens mRNA; cDNA DKFZp686M08112 (from clone DKFZp686M08112) | 6.41 | Up | 3.09E-05 |
| NM_005398 | Homo sapiens protein phosphatase 1, regulatory (inhibitor) subunit 3C (PPP1 R3C), mRNA | 6.38 | Up | 1.22E-05 |
| NM_001957 | Homo sapiens endothelin receptor type A (EDNRA), mRNA | 6.36 | Up | 3.08E-05 |
| NM_003248 | Homo sapiens thrombospondin 4 (THBS4), mRNA | 6.35 | Up | 2.71E-05 |
| BQ188758 | UI-E-EJ1-ajx-a-20-0-UI.r1 UI-E-EJ1 Homo sapiens cDNA clone UI-E-EJ1-ajx-a-20-0-UI 5, mRNA sequence | 6.28 | Up | 5.68E-03 |
| AI830524 | wh52c02.x1 NCI_CGAP_Kid11 Homo sapiens cDNA clone IMAGE:2384354 3, mRNA sequence | 6.28 | Up | 2.77E-05 |
| BG818762 | 602779092F2 NCI_CGAP_Brn67 Homo sapiens cDNA clone IMAGE:4914502 5, mRNA sequence | 6.28 | Up | 1.57E-05 |
| NM_024420 | Homo sapiens phospholipase A2, group IVA (cytosolic, calcium-dependent) (PLA2G4A), mRNA | 6.26 | Up | 3.90E-04 |
| NM_173552 | Homo sapiens hypothetical protein MGC33365 (MGC33365), mRNA | 6.25 | Up | 1.39E-05 |
| BQ189707 | UI-E-EJ1-aka-o-12-0-UI.r1 UI-E-EJ1 Homo sapiens cDNA clone UI-E-EJ1-aka-o-12-0-UI 5, mRNA sequence | 6.25 | Up | 2.62E-05 |
| NM_015559 | Homo sapiens SET binding protein 1 (SETBP1), mRNA | 6.21 | Up | 1.83E-03 |
| T15720 | IB1788 Infant brain, Bento Soares Homo sapiens cDNA 3end, mRNA sequence | 6.17 | Up | 2.02E-05 |
| AW291013 | UI-H-BI2-agj-f-01-0-UI.s1 NCI_CGAP_Sub4 Homo sapiens cDNA clone IMAGE:2724601 3, mRNA sequence | 6.15 | Up | 3.80E-05 |
| NM_024336 | Homo sapiens iroquois homeobox protein 3 (IRX3), mRNA | 6.12 | Up | 9.89E-06 |
| NM_025107 | Homo sapiens myc target 1 (MYCT1), mRNA | 6.11 | Up | 5.67E-05 |
| NM_144665 | Homo sapiens sestrin 3 (SESN3), mRNA | 6.11 | Up | 1.39E-04 |
| AA908815 | og77h08.s1 NCI_CGAP_Ov8 Homo sapiens cDNA clone IMAGE:1454367 3, mRNA sequence | 6.07 | Up | 2.25E-04 |
| NM_024621 | Homo sapiens hypothetical protein FLJ12604 (FLJ12604), mRNA | 6.05 | Up | 2.61E-04 |
| AI335546 | qt24d07.x1 NCI_CGAP_GC4 Homo sapiens cDNA clone IMAGE:1948525 3, mRNA sequence | 6.02 | Up | 8.66E-06 |
| AB007954 | Homo sapiens mRNA, chromosome 1 specific transcript KIAA0485 | 5.99 | Up | 5.46E-05 |
| NM_033292 | Homo sapiens caspase 1, apoptosis-related cysteine protease (interleukin 1, beta, convertase) (CASP1), transcript variant alpha, mRNA | 5.95 | Up | 4.51E-05 |
| BX112737 | BX112737 Soares placenta Nb2HP Homo sapiens cDNA clone IMAGp998H17189 ; IMAGE:134080, mRNA sequence | 5.94 | Up | 3.13E-06 |
| NM_005239 | Homo sapiens v-ets erythroblastosis virus E26 oncogene homolog 2 (avian) (ETS2), mRNA | 5.94 | Up | 1.14E-04 |
| BM677978 | UI-E-EJ0-aig-o-17-0-UI.s1 UI-E-EJ0 Homo sapiens cDNA clone UI-E-EJ0-aig-o-17-0-UI 3, mRNA sequence | 5.91 | Up | 7.10E-05 |
| BX648604 | Homo sapiens mRNA; cDNA DKFZp686M11213 (from clone DKFZp686M11213) | 5.91 | Up | 1.15E-04 |
| BC041997 | Homo sapiens, clone IMAGE:5310819, mRNA | 5.91 | Up | 4.16E-04 |
| NM_145239 | Homo sapiens similar to lymphocyte antigen 6 complex, locus G5B; G5b protein; open reading frame 31 (LOC112476), mRNA | 5.85 | Up | 5.37E-05 |
| NM_015149 | Homo sapiens ral guanine nucleotide dissociation stimulator-like 1 (RGL1), mRNA | 5.85 | Up | 2.32E-05 |
| NM_030756 | Homo sapiens transcription factor 7-like 2 (T-cell specific, HMG-box) (TCF7L2), mRNA | 5.85 | Up | 3.88E-05 |
| BU626144 | UI-H-FG1-bgq-i-08-0-UI.s1 NCI_CGAP_FG1 Homo sapiens cDNA clone UI-H-FG1-bgq-i-08-0-UI 3, mRNA sequence | 5.85 | Up | 1.70E-04 |
| BM556191 | AGENCOURT_6544282 NIH_MGC_88 Homo sapiens cDNA clone IMAGE:5550173 5, mRNA sequence | 5.83 | Up | 1.85E-05 |
| BM931867 | UI-E-EJ1-ajk-n-19-0-UI.r1 UI-E-EJ1 Homo sapiens cDNA clone UI-E-EJ1-ajk-n-19-0-UI 5, mRNA sequence | 5.81 | Up | 2.62E-05 |
| BG576442 | 602595685F1 NIH_MGC_87 Homo sapiens cDNA clone IMAGE:4708725 5, mRNA sequence | 5.8 | Up | 7.12E-05 |
| AB096240 | Homo sapiens LOH11CR1A gene, loss of heterozygosity, 11, chromosomal region 1 gene A product | 5.78 | Up | 1.63E-03 |
| NM_003763 | Homo sapiens syntaxin 16 (STX16), transcript variant 2, mRNA | 5.72 | Up | 7.02E-05 |
| BQ013869 | UI-1-BC1p-alg-a-03-0-UI.s1 NCI_CGAP_PI3 Homo sapiens cDNA clone UI-1-BC1 p-alg-a-03-0-UI 3, mRNA sequence | 5.69 | Up | 1.22E-05 |
| NM_005795 | Homo sapiens calcitonin receptor-like (CALCRL), mRNA | 5.68 | Up | 3.82E-05 |
| BM690053 | UI-E-CK1-abr-c-12-0-UI.r1 UI-E-CK1 Homo sapiens cDNA clone UI-E-CK1-abr-c-12-0-UI 5, mRNA sequence | 5.64 | Up | 2.37E-05 |
| AL832779 | Homo sapiens mRNA; cDNA DKFZp686H157 (from clone DKFZp686H157) | 5.64 | Up | 7.39E-06 |
| CA425350 | UI-H-DF0-bel-I-04-0-UI.s1 NCI_CGAP_DF0 Homo sapiens cDNA clone UI-H-DF0-bel-I-04-0-UI 3, mRNA sequence | 5.63 | Up | 2.47E-05 |
| AA398628 | zt74e03.s1 Soares_testis_NHT Homo sapiens cDNA clone IMAGE:728092 3, mRNA sequence | 5.62 | Up | 1.87E-04 |
| BX093329 | BX093329 Soares_parathyroid_tumor_NbHPA Homo sapiens cDNA clone IMAGp998A124183 ; IMAGE:1648403, mRNA sequence | 5.61 | Up | 7.11E-05 |
| NM_006573 | Homo sapiens tumor necrosis factor (ligand) superfamily, member 13b (TNFSF13B), mRNA | 5.58 | Up | 1.02E-05 |
| AK124699 | Homo sapiens cDNA FLJ42709 fis, clone BRAMY3007350 | 5.53 | Up | 1.72E-04 |
| AL709606 | DKFZp686M125_r1 686 (synonym: hlcc3) Homo sapiens cDNA clone DKFZp686M125 5, mRNA sequence | 5.53 | Up | 1.80E-04 |
| BQ022721 | UI-1-BB1p-axt-g-01-0-UI.s1 NCI_CGAP_PI6 Homo sapiens cDNA clone UI-1-BB1p-axt-g-01-0-UI 3, mRNA sequence | 5.5 | Up | 2.79E-03 |
| AA613572 | nq22g06.s1 NCI_CGAP_Co10 Homo sapiens cDNA clone IMAGE:1144666 3, mRNA sequence | 5.47 | Up | 4.31E-04 |
| R38944 | yd06g09.s1 Soares infant brain 1 NIB Homo sapiens cDNA clone IMAGE:25061 3 similar to contains Alu repetitive element;, mRNA sequence | 5.45 | Up | 4.34E-05 |
| AK124563 | Homo sapiens cDNA FLJ42572 fis, clone BRACE3008092 | 5.43 | Up | 9.13E-06 |
| BC037929 | Homo sapiens cDNA clone IMAGE:5284659, partial cds | 5.42 | Up | 7.14E-04 |
| AK096536 | Homo sapiens cDNA FLJ39217 fis, clone OCBBF2006639, moderately similar to ZINC FINGER PROTEIN 84 | 5.41 | Up | 3.17E-06 |
| AL049443 | Homo sapiens mRNA; cDNA DKFZp586N2020 (from clone DKFZp586N2020) | 5.41 | Up | 2.83E-04 |
| NM_004951 | Homo sapiens Epstein-Barr virus induced gene 2 (lymphocyte-specific G protein-coupled receptor) (EBI2), mRNA | 5.39 | Up | 4.33E-04 |
| NM_021153 | Homo sapiens cadherin 19, type 2 (CDH19), mRNA | 5.37 | Up | 3.26E-05 |
| BX106577 | BX106577 Soares_NhHMPu_S1 Homo sapiens cDNA clone IMAGp998H131854 ; IMAGE:754236, mRNA sequence | 5.37 | Up | 2.51E-05 |
| AW148979 | xf07g08.x1 NCI_CGAP_Kid8 Homo sapiens cDNA clone IMAGE:2617406 3, mRNA sequence | 5.36 | Up | 2.78E-04 |
| AI024717 | ov68h06.x1 Soares_testis_NHT Homo sapiens cDNA clone IMAGE:1642523 3, mRNA sequence | 5.35 | Up | 3.75E-05 |
| AF131784 | Homo sapiens clone 25194 mRNA sequence | 5.34 | Up | 2.14E-05 |
| AK092375 | Homo sapiens cDNA FLJ35056 fis, clone OCBBF2018581 | 5.32 | Up | 6.05E-05 |
| NM_002615 | Homo sapiens serine (or cysteine) proteinase inhibitor, clade F (alpha-2 antiplasmin, pigment epithelium derived factor), member 1 (SERPINF1), mRNA | 5.3 | Up | 1.74E-04 |
| NM_016206 | Homo sapiens colon carcinoma related protein (FLJ38507), mRNA | 5.3 | Up | 2.44E-04 |
| BQ346857 | RC1-NT0033-250800-018-g09 NT0033 Homo sapiens cDNA, mRNA sequence | 5.29 | Up | 4.05E-05 |
| BQ026175 | UI-1-BB1p-akc-h-10-0-UI.s1 NCI_CGAP_PI6 Homo sapiens cDNA clone UI-1-BB1p-akc-h-10-0-UI 3, mRNA sequence | 5.29 | Up | 7.29E-04 |
| NM_033067 | Homo sapiens DMRT-like family B with proline-rich C-terminal, 1 (DMRTB1), mRNA | 5.28 | Up | 2.03E-04 |
| AK127847 | Homo sapiens cDNA FLJ45950 fis, clone PLACE7008136 | 5.27 | Up | 6.27E-06 |
| AI024323 | ov67g08.x1 Soares_testis_NHT Homo sapiens cDNA clone IMAGE:1642430 3, mRNA sequence | 5.27 | Up | 1.14E-04 |
| NM_022768 | Homo sapiens RNA binding motif protein 15 (RBM15), mRNA | 5.27 | Up | 3.30E-04 |
| BC016780 | Homo sapiens, clone IMAGE:4106389, mRNA | 5.24 | Up | 2.73E-03 |
| CA427170 | UI-H-DF0-bey-f-20-0-UI.s1 NCI_CGAP_DF0 Homo sapiens cDNA clone UI-H-DF0-bey-f-20-0-UI 3, mRNA sequence | 5.23 | Up | 2.60E-04 |
| BX537862 | Homo sapiens mRNA; cDNA DKFZp779M0552 (from clone DKFZp779M0552) | 5.23 | Up | 5.42E-05 |
| NM_022658 | Homo sapiens homeo box C8 (HOXC8), mRNA | 5.22 | Up | 4.37E-04 |
| | 601237381 F1 NIH_MGC_44 Homo sapiens | 5.21 | Up | 1.74E-04 |
| BE378852 | cDNA clone IMAGE:3609140 5, mRNA sequence | | | |
| AK074131 | Homo sapiens mRNA for FLJ00204 protein | 5.18 | Up | 5.59E-05 |
| BX098131 | BX098131 Soares_testis_NHT Homo sapiens cDNA clone IMAGp9980101830 ; IMAGE:745185, mRNA sequence | 5.15 | Up | 1.32E-05 |
| AI798732 | we91h12.x1 Soares_NFL_T_GBC_S1 Homo sapiens cDNA clone IMAGE:2348519 3, mRNA sequence | 5.14 | Up | 5.68E-04 |
| NM_017577 | Homo sapiens hypothetical protein DKFZp434C0328 (DKFZp434C0328), mRNA | 5.13 | Up | 3.63E-04 |
| NM_015541 | Homo sapiens leucine-rich repeats and immunoglobulin-like domains 1 (LRIG1), mRNA | 5.12 | Up | 1.04E-04 |
| NM_032622 | Homo sapiens ligand of numb-protein X (LNX), mRNA | 5.12 | Up | 1.74E-04 |
| NM_003500 | Homo sapiens acyl-Coenzyme A oxidase 2, branched chain (ACOX2), mRNA | 5.11 | Up | 1.34E-05 |
| NM_152270 | Homo sapiens hypothetical protein FLJ34922 (FLJ34922), mRNA | 5.07 | Up | 4.93E-05 |
| BM695626 | UI-E-CQ1-aew-i-06-0-UI.r1 UI-E-CQ1 Homo sapiens cDNA clone UI-E-CQ1-aew-i-06-0-UI 5, mRNA sequence | 5.06 | Up | 1.10E-03 |
| NM_014548 | Homo sapiens tropomodulin 2 (neuronal) (TMOD2), mRNA | 5.05 | Up | 4.23E-03 |
| BC036223 | Homo sapiens, clone IMAGE:5272183, mRNA | 5.04 | Up | 6.69E-05 |
| NM_173554 | Homo sapiens chromosome 10 open reading frame 107 (C10orf107), mRNA | 5.04 | Up | 8.63E-05 |
| NM_018409 | Homo sapiens hypothetical protein DKFZp761O0113 (DKFZp761O0113), mRNA | 5.03 | Up | 3.39E-04 |
| BF509492 | UI-H-B14-aoz-c-06-0-UI.s1 NCI_CGAP_Sub8 Homo sapiens cDNA clone IMAGE:3086579 3, mRNA sequence | 5.01 | Up | 1.99E-03 |
| BQ574344 | UI-H-EZ1-baz-b-10-0-UI.s1 NCI_CGAP_Ch2 Homo sapiens cDNA clone UI-H-EZ1-baz-b-10-0-UI 3, mRNA sequence | 5.01 | Up | 7.24E-06 |
| NM_022454 | Homo sapiens SRY (sex determining region Y)-box 17 (SOX17), mRNA | 366.7 | Down | 3.10E-06 |
| BG219729 | RST39494 Athersys RAGE Library Homo sapiens cDNA, mRNA sequence | 268.82 | Down | 2.19E-06 |
| NM_152737 | Homo sapiens hypothetical protein MGC33993 (MGC33993), mRNA | 250.88 | Down | 1.66E-06 |
| BX102632 | BX102632 NCI_CGAP_Co3 Homo sapiens cDNA clone IMAGp998J052307 ; IMAGE:928228, mRNA sequence | 188.6 | Down | 1.29E-06 |
| NM_002521 | Homo sapiens natriuretic peptide precursor B (NPPB), mRNA | 184.64 | Down | 3.10E-06 |
| NM_013230 | Homo sapiens CD24 antigen (small cell lung carcinoma cluster 4 antigen) (CD24), mRNA | 160.77 | Down | 4.30E-06 |
| AK023631 | Homo sapiens cDNA FLJ13569 fis, clone PLACE1008369 | 154.91 | Down | 5.78E-07 |
| NM_006169 | Homo sapiens nicotinamide N-methyltransferase (NNMT), mRNA | 141.97 | Down | 1.12E-06 |
| NM_006228 | Homo sapiens prepronociceptin (PNOC), mRNA | 138.62 | Down | 1.22E-05 |
| AI765021 | wh56c02.x1 NCI_CGAP_Kid11 Homo sapiens cDNA clone IMAGE:2384738 3, mRNA sequence | 130.91 | Down | 6.27E-06 |
| NM_004617 | Homo sapiens transmembrane 4 superfamily member 4 (TM4SF4), mRNA | 111.06 | Down | 1.43E-05 |
| AK096481 | Homo sapiens cDNA FLJ39162 fis, clone OCBBF2002376 | 102.43 | Down | 3.17E-06 |
| NM_007038 | Homo sapiens a disintegrin-like and metalloprotease (reprolysin type) with thrombospondin type 1 motif, 5 (aggrecanase-2) (ADAMTS5), mRNA | 95.94 | Down | 6.14E-06 |
| AB032980 | Homo sapiens mRNA for KIAA1154 protein, partial cds | 85.12 | Down | 9.37E-06 |
| AK092245 | Homo sapiens cDNA FLJ34926 fis, clone NT2RP7003319, highly similar to Mus musculus neuralin mRNA | 70.22 | Down | 2.04E-05 |
| NM_002276 | Homo sapiens keratin 19 (KRT19), mRNA | 59.59 | Down | 2.19E-06 |
| AI249696 | qj64a03.x1 NCI_CGAP_Kid3 Homo sapiens cDNA clone IMAGE:1864204 3, mRNA sequence | 56.92 | Down | 9.37E-06 |
| D29453 | HUMNK566 Human epidermal keratinocyte Homo sapiens cDNA clone 566, mRNA sequence | 53.31 | Down | 5.14E-06 |
| NM_004753 | Homo sapiens dehydrogenase/reductase (SDR family) member 3 (DHRS3), mRNA | 50.19 | Down | 1.41E-05 |
| NM_012464 | Homo sapiens tolloid-like 1 (TLL1), mRNA | 48.62 | Down | 1.72E-04 |
| NM_080743 | Homo sapiens serine-arginine repressor protein (35 kDa) (SRrp35), mRNA | 48.46 | Down | 1.34E-06 |
| NM_004496 | Homo sapiens forkhead box A1 (FOXA1), mRNA | 47.75 | Down | 3.10E-06 |
| BF512544 | UI-H-BW1-amf-c-08-0-UI.s1 NCI_CGAP_Sub7 Homo sapiens cDNA clone IMAGE:3069687 3, mRNA sequence | 46.87 | Down | 6.56E-06 |
| NM_016356 | Homo sapiens doublecortin domain containing 2 (DCDC2), mRNA | 46.71 | Down | 1.48E-05 |
| NM_020873 | Homo sapiens leucine rich repeat neuronal 1 (LRRN1), mRNA | 45.32 | Down | 3.17E-06 |
| | Homo sapiens neuropilin (NRP) and tolloid | 44.78 | Down | 1.84E-05 |
| NM_138966 | (TLL)-like 1 (NETO1), transcript variant 3, mRNA | | | |
| NM_000582 | Homo sapiens secreted phosphoprotein 1 (osteopontin, bone sialoprotein I, early T-lymphocyte activation 1) (SPP1), mRNA | 43.26 | Down | 9.71E-06 |
| BU680661 | UI-CF-DU1-aaz-f-04-0-UI.s1 UI-CF-DU1 Homo sapiens cDNA clone UI-CF-DU1-aaz-f-04-0-UI 3, mRNA sequence | 43.24 | Down | 3.19E-06 |
| NM_184087 | Homo sapiens tripartite motif-containing 55 (TRIM55), transcript variant 4, mRNA | 41.43 | Down | 3.19E-06 |
| NM_004221 | Homo sapiens natural killer cell transcript 4 (NK4), mRNA | 40.53 | Down | 1.66E-05 |
| CA416106 | UI-H-FE0-bbs-f-17-0-UI.s1 NCI_CGAP_FE0 Homo sapiens cDNA clone UI-H-FE0-bbs-f-17-0-UI 3, mRNA sequence | 40.33 | Down | 2.77E-05 |
| NM_006439 | Homo sapiens mab-21-like 2 (C. elegans) (MAB21L2), mRNA | 40.02 | Down | 3.10E-05 |
| BF431030 | 7o18c06.x1 NCI_CGAP_Kid11 Homo sapiens cDNA clone IMAGE:3574283 3, mRNA sequence | 39.89 | Down | 1.22E-05 |
| NM_025074 | Homo sapiens Fraser syndrome 1 (FRAS1), transcript variant 1, mRNA | 39.81 | Down | 3.82E-06 |
| BG197054 | RST16291 Athersys RAGE Library Homo sapiens cDNA, mRNA sequence | 39.23 | Down | 3.73E-05 |
| BG165745 | 602344592F1 NIH_MGC_89 Homo sapiens cDNA clone IMAGE:4454470 5, mRNA sequence | 37.22 | Down | 1.39E-04 |
| NM_002527 | Homo sapiens neurotrophin 3 (NTF3), mRNA | 34.83 | Down | 5.63E-06 |
| NM_004982 | Homo sapiens potassium inwardly-rectifying channel, subfamily J, member 8 (KCNJ8), mRNA | 33.22 | Down | 3.22E-05 |
| NM_018168 | Homo sapiens chromosome 14 open reading frame 105 (C14orf105), mRNA | 31.87 | Down | 1.84E-05 |
| BQ003401 | UI-H-E11-azd-j-23-0-UI.s1 NCI_CGAP_EI1 Homo sapiens cDNA clone IMAGE:5847286 3, mRNA sequence | 31.63 | Down | 8.86E-06 |
| BC037316 | Homo sapiens, clone IMAGE:5259432, mRNA | 30.99 | Down | 2.19E-06 |
| NM_016212 | Homo sapiens TP53TG3 protein (TP53TG3), mRNA | 30.74 | Down | 1.57E-05 |
| BQ375719 | QV2-TN0173-021100-454-g06 TN0173 Homo sapiens cDNA, mRNA sequence | 30.59 | Down | 1.84E-05 |
| NM_014333 | Homo sapiens immunoglobulin superfamily, member 4 (IGSF4), mRNA | 30.19 | Down | 2.42E-05 |
| NM_005712 | Homo sapiens HERV-H LTR-associating 1 (HHLA1), mRNA | 29.97 | Down | 1.62E-05 |
| NM_001955 | Homo sapiens endothelin 1 (EDN1), mRNA | 29.8 | Down | 1.41E-05 |
| NM_0025 59 | Homo sapiens purinergic receptor P2X, ligand-gated ion channel, 3 (P2RX3), mRNA | 28.92 | Down | 1.84E-05 |
| NM_0018 85 | Homo sapiens crystallin, alpha B (CRYAB), mRNA | 28.49 | Down | 6.27E-06 |
| NM_0056 02 | Homo sapiens claudin 11 (oligodendrocyte transmembrane protein) (CLDN11), mRNA | 28.14 | Down | 5.78E-07 |
| NM_0009 27 | Homo sapiens ATP-binding cassette, sub-family B (MDR/TAP), member 1 (ABCB1), mRNA | 27.84 | Down | 8.93E-06 |
| NM_0033 85 | Homo sapiens visinin-like 1 (VSNL1), mRNA | 27.75 | Down | 9.37E-06 |
| NM_0203 49 | Homo sapiens ankyrin repeat domain 2 (stretch responsive muscle) (ANKRD2), mRNA | 27.68 | Down | 2.19E-06 |
| NM_1524 87 | Homo sapiens hypothetical protein FLJ31842 (FLJ31842), mRNA | 27.59 | Down | 3.71E-05 |
| NM_0178 19 | Homo sapiens RNA (guanine-9-) methyltransferase domain containing 1 (RG9MTD1), mRNA | 26.6 | Down | 1.99E-05 |
| AA44913 7 | zx03d12.r1 Soares_total_fetus_Nb2HF8_9w Homo sapiens cDNA clone IMAGE:785399 5, mRNA sequence | 26.24 | Down | 1.67E-05 |
| AW5112 22 | hd44d11.x1 Soares_NFL_T_GBC_S1 Homo sapiens cDNA clone IMAGE:2912373 3 similar to contains Alu repetitive element;, mRNA sequence | 26.15 | Down | 9.71E-06 |
| AI469032 | ti70a01.x1 NCI_CGAP_Kid11 Homo sapiens cDNA clone IMAGE:2137320 3, mRNA sequence | 25.39 | Down | 3.10E-06 |
| NM_0308 99 | Homo sapiens zinc finger protein 323 (ZNF323), mRNA | 25.07 | Down | 1.43E-05 |
| BG54530 5 | 602572521 F1 NIH_MGC_77 Homo sapiens cDNA clone IMAGE:4700644 5, mRNA sequence | 24.4 | Down | 3.68E-06 |
| NM_1840 87 | Homo sapiens tripartite motif-containing 55 (TRIM55), transcript variant 4, mRNA | 24.35 | Down | 9.37E-06 |
| NM_0060 74 | Homo sapiens tripartite motif-containing 22 (TRIM22), mRNA | 24.19 | Down | 1.55E-04 |
| NM_1526 94 | Homo sapiens zinc finger, CCHC domain containing 5 (ZCCHC5), mRNA | 24.15 | Down | 8.30E-05 |
| NM_1750 56 | Homo sapiens hypothetical protein LOC131368 (LOC131368), mRNA | 23.96 | Down | 2.36E-05 |
| NM_0152 36 | Homo sapiens latrophilin 3 (LPHN3), mRNA | 23.83 | Down | 9.13E-06 |
| AK12487 3 | Homo sapiens cDNA FLJ42883 fis, clone BRHIP3006683 | 23.58 | Down | 1.95E-05 |
| NM_0015 62 | Homo sapiens interleukin 18 (interferon-gamma-inducing factor) (IL18), mRNA | 23.38 | Down | 4.81E-05 |
| | Homo sapiens TatD DNase domain containing | 23.12 | Down | 6.00E-05 |
| NM_0320 26 | 1 (TATDN1), mRNA | | | |
| NM_1527 03 | Homo sapiens chromosome 7 open reading frame 6 (C7orf6), mRNA | 22.98 | Down | 1.82E-05 |
| NM_0326 03 | Homo sapiens lysyl oxidase-like 3 (LOXL3), mRNA | 22.16 | Down | 1.17E-05 |
| BX11582 5 | BX115825 Soares_NhHMPu_S1 Homo sapiens cDNA clone IMAGp998C134603 ; IMAGE:1879236, mRNA sequence | 22.02 | Down | 3.22E-05 |
| NM_0014 48 | Homo sapiens glypican 4 (GPC4), mRNA | 21.86 | Down | 8.50E-06 |
| BC00858 0 | Homo sapiens, clone IMAGE:4179986, mRNA, partial cds | 21.22 | Down | 3.19E-06 |
| NM_0022 73 | Homo sapiens keratin 8 (KRT8), mRNA | 21.19 | Down | 1.29E-05 |
| NM_0050 69 | Homo sapiens single-minded homolog 2 (Drosophila) (SIM2), transcript variant SIM2, mRNA | 20.97 | Down | 3.19E-06 |
| NM_0529 47 | Homo sapiens heart alpha-kinase (HAK), mRNA | 20.9 | Down | 1.22E-05 |
| AB04126 9 | Homo sapiens mRNA for keratin 19, partial cds, isolate: K19-141 | 20.57 | Down | 3.49E-05 |
| NM_002578 | Homo sapiens p21 (CDKN1A)-activated kinase 3 (PAK3), mRNA | 20.36 | Down | 3.19E-06 |
| NM_0247 26 | Homo sapiens IQ motif containing with AAA domain (IQCA), mRNA | 20.18 | Down | 1.02E-04 |
| NM_0186 58 | Homo sapiens potassium inwardly-rectifying channel, subfamily J, member 16 (KCNJ16), transcript variant 1, mRNA | 19.93 | Down | 3.44E-05 |
| BU56780 4 | AGENCOURT_10398872 NIH_MGC_82 Homo sapiens cDNA clone IMAGE:6614502 5, mRNA sequence | 19.73 | Down | 6.27E-06 |
| NM_0053 29 | Homo sapiens hyaluronan synthase 3 (HAS3), transcript variant 1, mRNA | 19.66 | Down | 3.19E-06 |
| BX50911 7 | DKFZp686C09280_r1 686 (synonym: hlcc3) Homo sapiens cDNA clone DKFZp686C09280 5, mRNA sequence | 19.48 | Down | 8.85E-06 |
| NM_0033 92 | Homo sapiens wingless-type MMTV integration site family, member 5A (WNT5A), mRNA | 19.4 | Down | 3.19E-06 |
| NM_0248 98 | Homo sapiens family with sequence similarity 31, member C (FAM31C), mRNA | 19.07 | Down | 6.27E-06 |
| AF31838 2 | Homo sapiens pp9974 mRNA, complete cds | 19 | Down | 6.27E-06 |
| AL11757 8 | Homo sapiens mRNA; cDNA DKFZp434C128 (from clone DKFZp434C128) | 18.69 | Down | 3.17E-06 |
| BU63316 3 | UI-H-FL1-bgt-n-07-0-UI.s1 NCI_CGAP_FL1 Homo sapiens cDNA clone UI-H-FL1-bgt-n-07-0-UI 3, mRNA sequence | 18.57 | Down | 9.42E-06 |
| AB01153 9 | Homo sapiens mRNA for MEGF6 protein (KIAA0815), partial cds | 18.38 | Down | 4.22E-05 |
| NM_0006 93 | Homo sapiens aldehyde dehydrogenase 1 family, member A3 (ALDH1A3), mRNA | 18.37 | Down | 4.97E-05 |
| AA19532 8 | zr34f08.s1 Soares_NhHMPu_S1 Homo sapiens cDNA clone IMAGE:665319 3, mRNA sequence | 18.33 | Down | 9.56E-06 |
| AL08010 3 | Homo sapiens mRNA; cDNA DKFZp564N2216 (from clone DKFZp564N2216) | 18.18 | Down | 3.85E-05 |
| NM_0201 30 | Homo sapiens chromosome 8 open reading frame 4 (C8orf4), mRNA | 17.97 | Down | 1.19E-04 |
| M_1829 20 | Homo sapiens a disintegrin-like and metalloprotease (reprolysin type) with thrombospondin type 1 motif, 9 (ADAMTS9), transcript variant 1, mRNA | 17.95 | Down | 5.64E-05 |
| AK02426 1 | Homo sapiens cDNA FLJ14199 fis, clone NT2RP3002713 | 17.68 | Down | 2.24E-05 |
| NM_0051 30 | Homo sapiens fibroblast growth factor binding protein 1 (FGFBP1), mRNA | 17.55 | Down | 5.73E-06 |
| BF43104 1 | nab31g02.x1 Soares_NSF_F8_9W_OT_PA_P_S1 Homo sapiens cDNA clone IMAGE:3267627 3, mRNA sequence | 17.25 | Down | 1.56E-05 |
| BM992049 | UI-H-DF1-auf-e-22-0-UI.s1 NCI_CGAP_DF1 Homo sapiens cDNA clone IMAGE:5868669 3, mRNA sequence | 16.81 | Drown | 8.93E-06 |
| NM_0015 54 | Homo sapiens cysteine-rich, angiogenic inducer, 61 (CYR61), mRNA | 16.43 | Down | 6.87E-04 |
| AK09352 9 | Homo sapiens cDNA FLJ36210 fis, clone THYMU2000155 | 16.29 | Down | 1.16E-05 |
| NM_1983 89 | Homo sapiens lung type-I cell membrane-associated glycoprotein (T1A-2), transcript variant 2, mRNA | 16.15 | Down | 2.62E-05 |
| NM_0044 90 | Homo sapiens growth factor receptor-bound protein 14 (GRB14), mRNA | 16.02 | Down | 1.52E-04 |
| NM_0019 99 | Homo sapiens fibrillin 2 (congenital contractural arachnodactyly) (FBN2), mRNA | 16 | Down | 4.45E-05 |
| NM_0124 13 | Homo sapiens glutaminyl-peptide cyclotransferase (glutaminyl cyclase) (QPCT), mRNA | 15.51 | Down | 1.39E-05 |
| NM_1784 70 | Homo sapiens WD repeat domain 40B (WDR40B), mRNA | 15.46 | Down | 2.73E-05 |
| BM71294 5 | UI-E-EJ0-ahi-c-16-0-UI.r1 UI-E-EJ0 Homo sapiens cDNA clone UI-E-EJ0-ahi-c-16-0-UI 5, mRNA sequence | 15.36 | Down | 4.73E-06 |
| NM_0188 94 | Homo sapiens EGF-containing fibulin-like extracellular matrix protein 1 (EFEMP1), transcript variant 2, mRNA | 15.15 | Down | 5.35E-05 |
| BM71207 2 | UI-E-DW1-ahc-b-11-0-UI.r1 UI-E-DW1 Homo sapiens cDNA clone UI-E-DW1-ahc-b-11-0-UI 5, mRNA sequence | 15.1 | Down | 1.93E-05 |
| NM_0005 19 | Homo sapiens hemoglobin, delta (HBD), mRNA | 14.92 | Down | 5.14E-06 |
| NM_0162 76 | Homo sapiens serum/glucocorticoid regulated kinase 2 (SGK2), transcript variant 2, mRNA | 14.87 | Down | 3.13E-06 |
| BF11171 0 | 7147c10.x1 Soares_NSF_F8_9W_OT_PA_P_S1 Homo sapiens cDNA clone IMAGE:3524371 3, mRNA sequence | 14.78 | Down | 9.84E-05 |
| AF26916 2 | Homo sapiens c21orf7 form B mRNA, complete cds | 14.77 | Down | 5.11E-05 |
| NM_2034 18 | Homo sapiens Down syndrome critical region gene 1 (DSCR1), transcript variant 3, mRNA | 14.75 | Down | 9.03E-04 |
| NM_1735 05 | Homo sapiens ankyrin repeat domain 29 (ANKRD29), mRNA | 14.71 | Down | 8.60E-05 |
| NM_0325 11 | Homo sapiens chromosome 6 open reading frame 168 (C6orf168), mRNA | 14.65 | Down | 1.60E-05 |
| NM_1527 68 | Homo sapiens hypothetical protein FLJ25378 (FLJ25378), mRNA | 14.59 | Down | 3.47E-05 |
| NM_0028 37 | Homo sapiens protein tyrosine phosphatase, receptor type, B (PTPRB), mRNA | 14.53 | Down | 8.08E-06 |
| NM_0006 82 | Homo sapiens adrenergic, alpha-2B-, receptor (ADRA2B), mRNA | 14.52 | Down | 5.46E-05 |
| NM_015 41 | Homo sapiens olfactomedin-like 2B 4 (OLFML2B), mRNA | 14.35 | Down | 3.75E-05 |
| NM_006 75 | Homo sapiens periostin, osteoblast specific 4 factor (POSTN), mRNA | 14.33 | Down | 4.23E-05 |
| BG4362 4 | 602508665F1 NIH_MGC_79 Homo sapiens 4 cDNA clone IMAGE:4605617 5, mRNA sequence | 14.19 | Down | 3.75E-05 |
| NM_025 45 | Homo sapiens pre-B-cell leukemia transcription 2 factor 4 (PBX4), mRNA | 14.1 | Down | 1.41E-05 |
| NM_0005 57 | Homo sapiens growth differentiation factor 5 (cartilage-derived morphogenetic protein-1) (GDF5), mRNA | 14.06 | Down | 7.14E-05 |
| NM_079 23 | Homo sapiens myosin, light polypeptide 6, 4 alkali, smooth muscle and non-muscle (MYL6), transcript variant 2, mRNA | 14.03 | Drown | 2.37E-05 |
| NM_2075 17 | Homo sapiens ADAMTS-like 3 (ADAMTSL3), mRNA | 13.93 | Down | 1.39E-05 |
| NM_152 73 | Homo sapiens RAS and EF hand domain 5 containing (RASEF), mRNA | 13.93 | Down | 1.72E-04 |
| NM_0176 33 | Homo sapiens family with sequence similarity 46, member A (FAM46A), mRNA | 13.92 | Down | 3.30E-06 |
| NM_016 88 | Homo sapiens neuritin 1 (NRN1), mRNA 5 | 13.79 | Down | 1.39E-05 |
| NM_0209 62 | Homo sapiens likely ortholog of mouse neighbor of Punc E11 (NOPE), mRNA | 13.78 | Down | 2.37E-05 |
| NM_0324 61 | Homo sapiens SPANX family, member B1 (SPANXB1), mRNA | 13.57 | Down | 1.09E-05 |
| BE78876 3 | 601475864F1 NIH_MGC_68 Homo sapiens cDNA clone IMAGE:3879014 5, mRNA sequence | 13.56 | Down | 1.39E-05 |
| AK12549 0 | Homo sapiens cDNA FLJ43501 fis, clone PEBLM2004497 | 13.39 | Down | 3.11E-04 |
| NM_0005 84 | Homo sapiens interleukin 8 (IL8), mRNA | 13.37 | Down | 1.66E-05 |
| M_1458 02 | Homo sapiens septin 6 (SEPT6), transcript variant V, mRNA | 13.04 | Down | 9.89E-06 |
| R99527 | yq79b11.s1 Soares fetal liver spleen 1NFLS Homo sapiens cDNA clone IMAGE:201981 3, mRNA sequence | 13.01 | Down | 3.09E-05 |
| NM_0325 10 | Homo sapiens par-6 partitioning defective 6 homolog gamma (C. elegans) (PARD6G), mRNA | 12.84 | Down | 3.42E-05 |
| NM_0005 99 | Homo sapiens insulin-like growth factor binding protein 5 (IGFBP5), mRNA | 12.83 | Down | 1.87E-05 |
| NM_0008 76 | Homo sapiens insulin-like growth factor 2 receptor (IGF2R), mRNA | 12.74 | Down | 3.58E-04 |
| AL389942 | Homo sapiens mRNA full length insert cDNA clone EUROIMAGE 2005635 | 12.52 | Down | 1.20E-04 |
| AJ69797 2 | Homo sapiens chromosome 3 cDNA | 12.43 | Down | 1.17E-04 |
| H23441 | ym52f11.s1 Soares infant brain 1NIB Homo sapiens cDNA clone IMAGE:51888 3, mRNA sequence | 12.39 | Down | 2.04E-05 |
| NM_0162 01 | Homo sapiens angiomotin like 2 (AMOTL2), mRNA | 12.38 | Down | 1.31E-03 |
| NM_0051 39 | Homo sapiens annexin A3 (ANXA3), mRNA | 12.27 | Down | 3.10E-06 |
| W85910 | zh52b10.s1 Soares_fetal_liver_spleen_1NFLS_S1 Homo sapiens cDNA clone IMAGE:415675 3, mRNA sequence | 12.27 | Down | 4.22E-05 |
| NM_0048 62 | Homo sapiens lipopolysaccharide-induced TNF factor (LITAF), mRNA | 12.27 | Down | 1.03E-04 |
| TM_1780 33 | Homo sapiens cytochrome P450, family 4, subfamily X, polypeptide 1 (CYP4X1), mRNA | 12.13 | Down | 3.22E-05 |
| NM_0201 52 | Homo sapiens chromosome 21 open reading frame 7 (C21orf7), mRNA | 12.12 | Down | 4.27E-06 |
| NM_0041 65 | Homo sapiens Ras-related associated with diabetes (RRAD), mRNA | 11.95 | Down | 1.10E-04 |
| NM_005450 | Homo sapiens noggin (NOG), mRNA | 11.93 | Down | 7.24E-06 |
| NM_0002 12 | Homo sapiens integrin, beta 3 (platelet glycoprotein IIIa, antigen CD61) (ITGB3), mRNA | 11.9 | Down | 8.68E-05 |
| BX64896 4 | Homo sapiens mRNA; cDNA DKFZp686J0156 (from clone DKFZp686J0156) | 11.85 | Down | 1.22E-05 |
| NM_0314 76 | Homo sapiens hypothetical protein DKFZp434B044 (DKFZP434B044), mRNA | 11.84 | Down | 1.04E-05 |
| NM_1452 01 | Homo sapiens similar to CG3714 gene product (PP3856), mRNA | 11.57 | Down | 1.39E-05 |
| NM_0161 79 | Homo sapiens transient receptor potential cation channel, subfamily C, member 4 (TRPC4), mRNA | 11.56 | Down | 2.61 E-04 |
| NM_0052 02 | Homo sapiens collagen, type VIII, alpha 2 (COL8A2), mRNA | 11.39 | Down | 2.61 E-04 |
| AW0216 86 | df26h11.y1 Morton Fetal Cochlea Homo sapiens cDNA clone IMAGE:2484717 5, mRNA sequence | 11.2 | Down | 2.21 E-05 |
| BC04141 2 | Homo sapiens heat shock 70kDa protein 12A, mRNA (cDNA clone IMAGE:5285193), partial cds | 11.17 | Down | 2.88E-04 |
| NM_0044 33 | Homo sapiens E74-like factor 3 (ets domain transcription factor, epithelial-specific) (ELF3), mRNA | 11.14 | Down | 2.80E-05 |
| NM_0011 44 | Homo sapiens autocrine motility factor receptor (AMFR), transcript variant 1, mRNA | 11.14 | Down | 5.52E-05 |
| NM_006 63 | Homo sapiens leukocyte immunoglobulin-like 8 receptor, subfamily A (with TM domain), member 1 (LILRA1), mRNA | 11.04 | Down | 2.72E-05 |
| BX11331 9 | BX113319 NCI_CGAP_Gas4 Homo sapiens cDNA clone IMAGp998G205398 ; IMAGE:2184619, mRNA sequence | 10.97 | Down | 2.51E-05 |
| NM_002 62 | Homo sapiens myxovirus (influenza virus) 4 resistance 1, interferon-inducible protein p78 (mouse) (MX1), mRNA | 10.93 | Down | 1.17E-04 |
| AB01109 5 | Homo sapiens mRNA for KIAA0523 protein, partial cds | 10.88 | Down | 8.65E-06 |
| NM_001 54 | Homo sapiens collagen, type XI, alpha 1 8 (COL11A1), transcript variant A, mRNA | 10.81 | Down | 9.37E-06 |
| NM_178 34 | Homo sapiens layilin (LOC143903), mRNA 8 | 10.8 | Down | 5.28E-06 |
| NM_0121 98 | Homo sapiens grancalcin, EF-hand calcium binding protein (GCA), mRNA | 10.71 | Down | 1.57E-05 |
| NM 2074 82 | Homo sapiens FLJ44048 protein (FLJ44048), mRNA | 10.69 | Down | 3.75E-05 |
| NM_000 17 | Homo sapiens glutamate decarboxylase 1 8 (brain, 67kDa) (GAD1), transcript variant GAD67, mRNA | 10.65 | Down | 1.35E-05 |
| NM_004 89 | Homo sapiens nuclear factor (erythroid-derived 2 2)-like 3 (NFE2L3), mRNA | 10.42 | Down | 4.53E-05 |
| NM_0070 72 | Homo sapiens HERV-H LTR-associating 2 (HHLA2), mRNA | 10.41 | Down | 7.19E-06 |
| AI355761 | qt94a11.x1 NCI_CGAP_Co14 Homo sapiens cDNA clone IMAGE:1962908 3 similar to gb:X74929 KERATIN, TYPE II CYTOSKELETAL 8 (HUMAN);, mRNA sequence | 10.38 | Down | 2.80E-05 |
| NM_0219 77 | Homo sapiens solute carrier family 22 (extraneuronal monoamine transporter), member 3 (SLC22A3), mRNA | 10.3 | Down | 1.20E-04 |
| AL13769 8 | Homo sapiens mRNA; cDNA DKFZp434C1915 (from clone DKFZp434C1915); partial cds | 10.26 | Down | 1.30E-04 |
| BM97638 5 | UI-CF-EN1-acz-f-03-0-UI.s1 UI-CF-EN1 Homo sapiens cDNA clone UI-CF-EN1-acz-f-03-0-UI 3, mRNA sequence | 10.2 | Down | 1.22E-05 |
| AK12331 9 | Homo sapiens cDNA FLJ41325 fis, clone BRAMY2046871 | 10.05 | Down | 1.84E-05 |
| BX45904 3 | BX459043 Homo sapiens PLACENTA Homo sapiens cDNA clone CS0DE011YN10 3-PRIME, mRNA sequence | 9.97 | Down | 3.27E-04 |
| NM_0002 27 | Homo sapiens laminin, alpha 3 (LAMA3), transcript variant 2, mRNA | 9.83 | Down | 5.83E-06 |
| AI335277 | tb29h06.x1 NCI_CGAP_Kid12 Homo sapiens cDNA clone IMAGE:2055803 3, mRNA sequence | 9.8 | Down | 7.24E-06 |
| NM_2073 80 | Homo sapiens FLJ43339 protein (FLJ43339), mRNA | 9.73 | Down | 4.97E-05 |
| NM_0033 28 | Homo sapiens TXK tyrosine kinase (TXK), mRNA | 9.7 | Down | 1.64E-05 |
| NM_0006 12 | Homo sapiens insulin-like growth factor 2 (somatomedin A) (IGF2), mRNA | 9.68 | Down | 2.36E-05 |
| NM_0053 02 | Homo sapiens G protein-coupled receptor 37 (endothelin receptor type B-like) (GPR37), mRNA | 9.63 | Down | 5.63E-06 |
| NM_0023 38 | Homo sapiens limbic system-associated membrane protein (LSAMP), mRNA | 9.62 | Down | 4.14E-05 |
| NM_0252 39 | Homo sapiens programmed cell death 1 ligand 2 (PDCD1 LG2), mRNA | 9.57 | Down | 7.52E-05 |
| NM_0004 78 | Homo sapiens alkaline phosphatase, liver/bone/kidney (ALPL), mRNA | 9.56 | Down | 1.39E-05 |
| AJ31880 5 | AJ318805 Homo sapiens adipose tissue Homo sapiens cDNA clone 2040, mRNA sequence | 9.55 | Down | 5.32E-05 |
| BG62270 7 | 602647476F1 NIH_MGC_79 Homo sapiens cDNA clone IMAGE:4768963 5, mRNA sequence | 9.54 | Down | 2.76E-05 |
| CN47859 7 | UI-CF-FN0-aeo-g-21-0-UI.s1 UI-CF-FN0 Homo sapiens cDNA clone UI-CF-FN0-aeo-g-21-0-UI 3, mRNA sequence | 9.49 | Down | 1.39E-05 |
| BF79809 8 | RC1-CI0045-021000-021-f02 CI0045 Homo sapiens cDNA, mRNA sequence | 9.46 | Down | 2.31 E-05 |
| NM_0008 56 | Homo sapiens guanylate cyclase 1, soluble, alpha 3 (GUCY1A3), mRNA | 9.42 | Down | 7.20E-05 |
| AL11976 9 | DKFZp761E1224_r1 761 (synonym: hamy2) Homo sapiens cDNA clone DKFZp761 E1224 5, mRNA sequence | 9.33 | Down | 5.73E-06 |
| NM_0055 96 | Homo sapiens nuclear factor I/B (NFIB), mRNA | 9.32 | Down | 2.30E-05 |
| AW 1344 73 | UI-H-BI1-abv-a-11-0-UI.s1 NCI_CGAP_Sub3 Homo sapiens cDNA clone IMAGE:2712885 3, mRNA sequence | 9.29 | Down | 1.31E-04 |
| BX53769 8 | Homo sapiens mRNA; cDNA DKFZp686F09166 (from clone DKFZp686F09166) | 9.28 | Down | 4.13E-06 |
| NM_0244 23 | Homo sapiens desmocollin 3 (DSC3), transcript variant Dsc3b, mRNA | 9.27 | Down | 2.36E-05 |
| NM_0060 72 | Homo sapiens chemokine (C-C motif) ligand 26 (CCL26), mRNA | 9.27 | Down | 9.91E-06 |
| NM_1735 08 | Homo sapiens solute carrier family 35, member F3 (SLC35F3), mRNA | 9.24 | Down | 5.64E-05 |
| NM_1780 12 | Homo sapiens tubulin, beta polypeptide paralog (MGC8685), mRNA | 9.24 | Down | 2.21E-04 |
| NM_0332 90 | Homo sapiens midline 1 (Opitz/BBB syndrome) (MID1), transcript variant 3, mRNA | 9.18 | Down | 8.22E-04 |
| NM_018265 | Homo sapiens hypothetical protein FLJ10901 (FLJ10901), mRNA | 8.95 | Down | 1.17E-04 |
| H89526 | yw28b04.r1 Morton Fetal Cochlea Homo sapiens cDNA clone IMAGE:253519 5, mRNA sequence | 8.91 | Down | 1.74E-05 |
| NM_0009 61 | Homo sapiens prostaglandin 12 (prostacyclin) synthase (PTGIS), mRNA | 8.89 | Down | 2.82E-04 |
| AI942360 | wo80c06.x1 NCI CGAP Kid11 Homo sapiens cDNA clone IMAGE:2461642 3, mRNA sequence | 8.88 | Down | 2.07E-05 |
| BX53761 3 | Homo sapiens mRNA; cDNA DKFZp686E11117 (from clone DKFZp686E11117) | 8.86 | Down | 1.59E-05 |
| NM_0143 58 | Homo sapiens C-type (calcium dependent, carbohydrate-recognition domain) lectin, superfamily member 9 (CLECSF9), mRNA | 8.84 | Down | 2.21E-05 |
| AK02399 9 | Homo sapiens cDNA FLJ13937 fis, clone Y79AA1000805 | 8.83 | Down | 1.32E-04 |
| NM_0006 92 | Homo sapiens aldehyde dehydrogenase 1 family, member B1 (ALDH1B1), nuclear gene encoding mitochondrial protein, mRNA | 8.8 | Down | 4.89E-05 |
| NM_0331 36 | Homo sapiens fibroblast growth factor 1 (acidic) (FGF1), transcript variant 2, mRNA | 8.8 | Down | 6.22E-05 |
| AK00007 5 | Homo sapiens cDNA FLJ20068 fis, clone COL01755 | 8.8 | Down | 1.85E-05 |
| NM_0186 59 | Homo sapiens cytokine-like protein C17 (C17), mRNA | 8.77 | Down | 7.06E-05 |
| AK05533 4 | Homo sapiens cDNA FLJ30772 fis, clone FEBRA2000757, moderately similar to Homo sapiens BM-009 mRNA | 8.59 | Down | 5.63E-06 |
| NM_0039 79 | Homo sapiens G protein-coupled receptor, family C, group 5, member A (GPCR5A), mRNA | 8.53 | Down | 4.21E-06 |
| NM_0142 43 | Homo sapiens a disintegrin-like and metalloprotease (reprolysin type) with thrombospondin type 1 motif, 3 (ADAMTS3), mRNA | 8.5 | Down | 5.37E-05 |
| CA43786 1 | UI-H-DH0-aur-k-12-0-UI.s1 NCI_CGAP_DH0 Homo sapiens cDNA clone UI-H-DH0-aur-k-12-0-UI 3, mRNA sequence | 8.44 | Down | 7.74E-05 |
| NM_0186 63 | Homo sapiens peroxisomal membrane protein 2, 22kDa (PXMP2), mRNA | 8.43 | Down | 5.70E-04 |
| AI953708 | wq47d09.x1 NCI_CGAP_GC6 Homo sapiens cDNA clone IMAGE:2474417 3, mRNA sequence | 8.39 | Down | 4.14E-05 |
| BC03431 5 | Homo sapiens hypothetical protein LOC90529, mRNA (cDNA clone IMAGE:4827425), containing frame-shift errors | 8.37 | Down | 1.84E-04 |
| CB11575 4 | K-EST0159876 L8SCK0 Homo sapiens cDNA clone L8SCK0-8-H08 5, mRNA sequence | 8.36 | Down | 7.40E-06 |
| NM_0050 26 | Homo sapiens phosphoinositide-3-kinase, catalytic, delta polypeptide (PIK3CD), mRNA | 8.3 | Down | 1.40E-04 |
| AL70665 3 | DKFZp686E1543_r1 686 (synonym: hlcc3) Homo sapiens cDNA clone DKFZp686E1543 5, mRNA sequence | 8.28 | Down | 2.59E-05 |
| M60502 | Human profilaggrin mRNA, 3 end | 8.28 | Down | 2.62E-05 |
| NM_0529 97 | Homo sapiens ankyrin repeat domain 30A (ANKRD30A), mRNA | 8.28 | Down | 4.24E-06 |
| NM_0202 47 | Homo sapiens chaperone, ABC1 activity of bc1 complex like (S. pombe) (CABC1), mRNA | 8.26 | Down | 9.37E-06 |
| NM_0180 76 | Homo sapiens armadillo repeat containing 4 (ARMC4), mRNA | 8.22 | Down | 4.95E-05 |
| NM_0071 67 | Homo sapiens zinc finger protein 258 (ZNF258), mRNA | 8.22 | Down | 6.47E-04 |
| NM_0166 13 | Homo sapiens hypothetical protein DKFZp434L142 (DKFZp434L142), mRNA | 8.21 | Down | 1.46E-05 |
| NM_0165 42 | Homo sapiens Mst3 and SOK1-related kinase (MST4), mRNA | 8.19 | Down | 1.06E-04 |
| NM_0005 81 | Homo sapiens glutathione peroxidase 1 (GPX1), transcript variant 1, mRNA | 8.18 | Down | 3.53E-04 |
| AK09211 4 | Homo sapiens cDNA FLJ34795 fis, clone NT2NE2005921 | 8.11 | Down | 3.10E-06 |
| AK12477 6 | Homo sapiens cDNA FLJ42786 fis, clone BRAWH3006761 | 8.11 | Down | 2.02E-05 |
| AK12470 2 | Homo sapiens cDNA FLJ42712 fis, clone BRAMY3008044 | 8.1 | Down | 6.75E-06 |
| NM_0033 85 | Homo sapiens visinin-like 1 (VSNL1), mRNA | 8.1 | Down | 3.19E-06 |
| NM_0309 15 | Homo sapiens likely ortholog of mouse limb-bud and heart gene (LBH), mRNA | 8.09 | Down | 3.86E-05 |
| CD72379 8 | oj26f04.y1 Human lacrimal gland, unamplified: oj Homo sapiens cDNA clone oj26f04 5, mRNA sequence | 8.08 | Down | 2.04E-04 |
| BE96859 6 | 601649770F1 NIH_MGC_74 Homo sapiens cDNA clone IMAGE:3933472 5, mRNA sequence | 8.05 | Down | 2.29E-05 |
| AB02321 1 | Homo sapiens mRNA for KIAA0994 protein, partial cds | 8.05 | Down | 1.74E-04 |
| NM_0040 45 | Homo sapiens ATX1 antioxidant protein 1 homolog (yeast) (ATOX1), mRNA | 8.02 | Down | 1.42E-04 |
| AK07409 7 | Homo sapiens mRNA for FLJ00168 protein | 7.99 | Down | 2.58E-04 |
| M_1817 18 | Homo sapiens hypothetical protein LOC253982 (LOC253982), mRNA | 7.98 | Down | 2.03E-04 |
| NM_0041 54 | Homo sapiens pyrimidinergic receptor P2Y, G-protein coupled, 6 (P2RY6), transcript variant 4, mRNA | 7.94 | Down | 9.37E-06 |
| NM_0143 91 | Homo sapiens ankyrin repeat domain 1 (cardiac muscle) (ANKRD1), mRNA | 7.92 | Down | 1.03E-04 |
| NM_0806 59 | Homo sapiens similar to RIKEN cDNA 2310030G06 gene (MGC14839), mRNA | 7.89 | Down | 2.67E-05 |
| NM_0123 19 | Homo sapiens solute carrier family 39 (zinc transporter), member 6 (SLC39A6), mRNA | 7.87 | Down | 4.83E-04 |
| NM_1391 61 | Homo sapiens crumbs homolog 3 (Drosophila) (CRB3), transcript variant 2, mRNA | 7.86 | Down | 2.20E-03 |
| BX64829 9 | Homo sapiens mRNA; cDNA DKFZp686J04125 (from clone DKFZp686J04125) | 7.85 | Down | 1.74E-05 |
| H94320 | yv18b10.s1 Soares fetal liver spleen 1NFLS Homo sapiens cDNA clone IMAGE:243067 3, mRNA sequence | 7.84 | Down | 8.68E-05 |
| NM_0024 23 | Homo sapiens matrix metalloproteinase 7 (matrilysin, uterine) (MMP7), mRNA | 7.83 | Down | 4.28E-04 |
| AK05801 2 | Homo sapiens cDNA FLJ25283 fis, clone STM06716 | 7.7 | Down | 5.51E-05 |
| T56535 | yb33g07.r1 Stratagene fetal spleen (#937205) Homo sapiens cDNA clone IMAGE:73020 5, mRNA sequence | 7.69 | Down | 2.72E-05 |
| NM_0020 53 | Homo sapiens guanylate binding protein 1, interferon-inducible, 67kDa (GBP1), mRNA | 7.68 | Down | 9.91E-06 |
| NM_0017 10 | Homo sapiens B-factor, properdin (BF), mRNA | 7.64 | Down | 4.89E-05 |
| AK127421 | Homo sapiens cDNA FLJ45513 fis, clone BRTHA2021450 | 7.63 | Down | 8.90E-05 |
| NM_0160 61 | Homo sapiens yippee-like 5 (Drosophila) (YPEL5), mRNA | 7.58 | Down | 5.26E-04 |
| NM_0068 55 | Homo sapiens KDEL (Lys-Asp-Glu-Leu) endoplasmic reticulum protein retention receptor 3 (KDELR3), transcript variant 1, mRNA | 7.57 | Down | 4.41E-04 |
| AI311296 | ta48d10.x2 NCI_CGAP_Lu25 Homo sapiens cDNA clone IMAGE:2047315 3, mRNA sequence | 7.55 | Down | 1.93E-04 |
| NM_0038 10 | Homo sapiens tumor necrosis factor (ligand) superfamily, member 10 (TNFSF10), mRNA | 7.55 | Down | 4.68E-04 |
| AI819863 | wj45h05.x1 NCI_CGAP_Lu19 Homo sapiens cDNA clone IMAGE:2405817 3, mRNA sequence | 7.54 | Down | 2.62E-05 |
| NM_0000 76 | Homo sapiens cyclin-dependent kinase inhibitor 1C (p57, Kip2) (CDKN1C), mRNA | 7.53 | Down | 2.31E-05 |
| NM_0037 61 | Homo sapiens vesicle-associated membrane protein 8 (endobrevin) (VAMP8), mRNA | 7.53 | Down | 6.69E-05 |
| NM_0034 75 | Homo sapiens chromosome 11 open reading frame 13 (C11orf13), mRNA | 7.5 | Down | 1.89E-04 |
| NM_1984 95 | Homo sapiens CTAGE family, member 4 (CTAGE4), mRNA | 7.47 | Down | 2.61 E-05 |
| AL117454 | Homo sapiens mRNA; cDNA DKFZp586J1717 (from clone DKFZp586J1717) | 7.4 | Down | 2.32E-04 |
| NM_0251 49 | Homo sapiens hypothetical protein FLJ20920 (FLJ20920), mRNA | 7.4 | Down | 1.67E-05 |
| NM_002E 22 | Homo sapiens neuronal pentraxin I (NPTX1), mRNA | 7.37 | Down | 2.29E-05 |
| NM_0028 67 | Homo sapiens RAB3B, member RAS oncogene family (RAB3B), mRNA | 7.37 | Down | 3.28E-05 |
| AK02163 7 | Homo sapiens cDNA FLJ11575 fis, clone HEMBA1003531 | 7.36 | Down | 4.12E-05 |
| AL35905 8 | Homo sapiens mRNA full length insert cDNA clone EUROIMAGE 592473 | 7.35 | Down | 1.12E-05 |
| AF10809 3 | Homo sapiens IA-2 gene, intron 18 | 7.33 | Down | 5.57E-05 |
| AK02623 5 | Homo sapiens cDNA: FLJ22582 fis, clone HSI02576 | 7.32 | Down | 1.55E-04 |
| NM_0221 03 | Homo sapiens hypothetical zinc finger protein FLJ14011 (FLJ14011), mRNA | 7.31 | Down | 6.09E-05 |
| NM_0019 77 | Homo sapiens glutamyl aminopeptidase (aminopeptidase A) (ENPEP), mRNA | 7.22 | Down | 9.96E-05 |
| BX11152 0 | BX111520 Soares placenta Nb2HP Homo sapiens cDNA clone IMAGp998L15208 ; IMAGE:141470, mRNA sequence | 7.21 | Down | 7.14E-06 |
| NM_0174 56 | Homo sapiens pleckstrin homology, Sec7 and coiled-coil domains 1 (cytohesin 1) (PSCD1), transcript variant 2, mRNA | 7.2 | Down | 1.76E-03 |
| NM_0139 62 | Homo sapiens neuregulin 1 (NRG1), transcript variant GGF2, mRNA | 7.19 | Down | 2.88E-04 |
| S70348 | Homo sapiens integrin beta 3 mRNA, partial cds, alternatively spliced | 7.18 | Down | 2.47E-05 |
| U51694 | HSU51694 Human normal gingiva Homo sapiens cDNA, mRNA sequence | 7.13 | Down | 6.48E-06 |
| NM_0012 99 | Homo sapiens calponin 1, basic, smooth muscle (CNN1), mRNA | 7.07 | Down | 2.91 E-05 |
| NM_0017 85 | Homo sapiens cytidine deaminase (CDA), mRNA | 7.02 | Down | 3.22E-05 |
| NM_0052 96 | Homo sapiens G protein-coupled receptor 23 (GPR23), mRNA | 6.94 | Down | 1.65E-04 |
| NM_0305 94 | Homo sapiens cytoplasmic polyadenylation element binding protein 1 (CPEB1), mRNA | 6.94 | Down | 5.63E-06 |
| BX11314 4 | BX113144 Soares placenta Nb2HP Homo sapiens cDNA clone IMAGp998N07225 ; IMAGE:148038, mRNA sequence | 6.93 | Down | 4.14E-05 |
| AB02064 0 | Homo sapiens mRNA for KIAA0833 protein, partial cds | 6.91 | Down | 5.63E-06 |
| AF24457 1 | Homo sapiens clone L49 HERV-K-T47-like long terminal repeat sequence | 6.86 | Down | 1.29E-04 |
| NM_0051 41 | Homo sapiens fibrinogen, B beta polypeptide (FGB), mRNA | 6.85 | Down | 2.24E-04 |
| NM_0530 01 | Homo sapiens odd-skipped-related 2A protein (OSR2), mRNA | 6.84 | Down | 2.30E-05 |
| AA19572 7 | zr33a09.r1 Soares_NhHMPu_S1 Homo sapiens cDNA clone IMAGE:665176 5, mRNA sequence | 6.82 | Down | 2.73E-05 |
| NM_0011 65 | Homo sapiens baculoviral IAP repeat-containing 3 (BIRC3), transcript variant 1, mRNA | 6.81 | Down | 2.02E-04 |
| NM_0055 12 | Homo sapiens glycoprotein A repetitions predominant (GARP), mRNA | 6.81 | Down | 2.00E-04 |
| NM_0000 87 | Homo sapiens cyclic nucleotide gated channel alpha 1 (CNGA1), mRNA | 6.81 | Down | 5.65E-05 |
| NM_0019 93 | Homo sapiens coagulation factor III (thromboplastin, tissue factor) (F3), mRNA | 6.74 | Down | 1.42E-04 |
| AF04121 0 | Homo sapiens midline 1 fetal kidney isoform 3 (MID1) mRNA, partial cds | 6.73 | Down | 6.70E-05 |
| NM_0010 83 | Homo sapiens phosphodiesterase 5A, cGMP-specific (PDE5A), transcript variant 1, mRNA | 6.73 | Down | 7.71E-06 |
| NM_0124 11 | Homo sapiens protein tyrosine phosphatase, non-receptor type 22 (lymphoid) (PTPN22), transcript variant 2, mRNA | 6.72 | Down | 2.36E-05 |
| NM_0309 65 | Homo sapiens sialyltransferase 7 ((alpha-N-acetylneuraminyl-2,3-beta-galactosyl-1,3)-N-acetyl galactosaminide alpha-2,6-sialyltransferase) E (SIAT7E), mRNA | 6.71 | Down | 3.90E-04 |
| NM_0008 52 | Homo sapiens glutathione S-transferase pi (GSTP1), mRNA | 6.7 | Down | 6.35E-05 |
| BC04636 2 | Homo sapiens voltage-dependent calcium channel gamma subunit-like protein, mRNA (cDNA clone MGC:50757 IMAGE:5221396), complete cds | 6.7 | Down | 1.38E-05 |
| NM_1445 69 | Homo sapiens SPOC domain containing 1 (SPOCD1), mRNA | 6.66 | Down | 5.03E-05 |
| NM_0060 58 | Homo sapiens TNFAIP3 interacting protein 1 (TNIP1), mRNA | 6.61 | Down | 1.01E-03 |
| S54641 | HZF-16=Kruppel-related zinc finger gene homolog {alternatively spliced} [human, hepatoblastoma cell line, HEP-G2, mRNA, 2080 nt] | 6.59 | Down | 6.25E-04 |
| NM_0180 23 | Homo sapiens YEATS domain containing 2 (YEATS2), mRNA | 6.58 | Down | 5.94E-04 |
| NM_0021 93 | Homo sapiens inhibin, beta B (activin AB beta polypeptide) (INHBB), mRNA | 6.56 | Down | 5.11E-05 |
| NM_0314 26 | Homo sapiens chromosome 9 open reading frame 58 (C9orf58), transcript variant 1, mRNA | 6.55 | Down | 4.93E-04 |
| AI244954 | qj93h05.x1 NCI_CGAP_Kid3 Homo sapiens cDNA clone IMAGE:1867065 3, mRNA sequence | 6.53 | Down | 5.14E-06 |
| BX53754 7 | Homo sapiens mRNA; cDNA DKFZp686N1640 (from clone DKFZp686N1640) | 6.51 | Down | 1.44E-04 |
| NM_0009 81 | Homo sapiens ribosomal protein L19 (RPL19), mRNA | 6.49 | Down | 1.39E-04 |
| AK05801 2 | Homo sapiens cDNA FLJ25283 fis, clone STM06716 | 6.48 | Down | 1.57E-05 |
| NM_0245 63 | Homo sapiens hypothetical protein FLJ14054 (FLJ14054), mRNA | 6.48 | Down | 2.40E-04 |
| NM_0018 43 | Homo sapiens contactin 1 (CNTN1), transcript variant 1, mRNA | 6.47 | Down | 1.91E-05 |
| L07615 | Human neuropeptide Y receptor Y1 (NPYY1) mRNA, exon 2-3 and complete cds | 6.46 | Down | 2.22E-04 |
| NM_0133 22 | Homo sapiens sorting nexin 10 (SNX10), mRNA | 6.44 | Down | 7.33E-05 |
| NM_0143 33 | Homo sapiens immunoglobulin superfamily, member 4 (IGSF4), mRNA | 6.41 | Down | 2.37E-05 |
| NM_1735 49 | Homo sapiens hypothetical protein FLJ39553 (FLJ39553), mRNA | 6.41 | Down | 1.42E-05 |
| BF50992 5 | UI-H-BI4-aph-c-10-0-UI.s1 NCI_CGAP_Sub8 Homo sapiens cDNA clone IMAGE:3087355 3, mRNA sequence | 6.39 | Down | 4.05E-05 |
| NM_003597 | Homo sapiens TGFB inducible early growth response 2 (TIEG2), mRNA | 6.39 | Down | 1.22E-05 |
| NM_152 69 | Homo sapiens hypothetical protein MGC45474 3 (MGC45474), mRNA | 6.39 | Down | 3.75E-05 |
| NM_1447 07 | Homo sapiens prominin 2 (PROM2), mRNA 7 | 6.38 | Down | 1.04E-04 |
| NM_0040 86 | Homo sapiens coagulation factor C homolog, cochlin (Limulus polyphemus) (COCH), mRNA | 6.37 | Down | 1.91E-05 |
| NM 0201 27 | Homo sapiens tuftelin 1 (TUFT1), mRNA | 6.37 | Down | 2.93E-03 |
| NM_0001 04 | Homo sapiens cytochrome P450, family 1, subfamily B, polypeptide 1 (CYP1B1), mRNA | 6.35 | Down | 1.46E-05 |
| NM_2068 08 | Homo sapiens citrate lyase beta like (CLYBL), transcript variant 2, mRNA | 6.32 | Down | 5.22E-05 |
| NM_0035 67 | Homo sapiens breast cancer anti-estrogen resistance 3 (BCAR3), mRNA | 6.31 | Down | 1.39E-05 |
| NM 0036 17 | Homo sapiens regulator of G-protein signalling 5 (RGS5), mRNA | 6.29 | Down | 2.36E-04 |
| AK13018 1 | Homo sapiens cDNA FLJ26671 fis, clone MPG03325 | 6.28 | Down | 6.69E-05 |
| NM_0159 54 | Homo sapiens CGI-26 protein (CGI-26), mRNA | 6.27 | Down | 2.50E-04 |
| NM_0019 45 | Homo sapiens diphtheria toxin receptor (heparin-binding epidermal growth factor-like growth factor) (DTR), mRNA | 6.27 | Down | 2.56E-03 |
| NM_0005 97 | Homo sapiens insulin-like growth factor binding protein 2, 36kDa (IGFBP2), mRNA | 6.26 | Down | 2.29E-04 |
| NM_0209 92 | Homo sapiens PDZ and LIM domain 1 (elfin) (PDLIM1), mRNA | 6.25 | Down | 8.97E-05 |
| NM_0005 76 | Homo sapiens interleukin 1, beta (IL1B), mRNA | 6.22 | Down | 2.34E-05 |
| NM_0228 18 | Homo sapiens microtubule-associated protein 1 light chain 3 beta (MAP1LC3B), mRNA | 6.22 | Down | 1.18E-03 |
| NM_0012 57 | Homo sapiens cadherin 13, H-cadherin (heart) (CDH13), mRNA | 6.21 | Down | 4.57E-04 |
| AI697906 | we18f06.x1 NCI_CGAP_Lu24 Homo sapiens cDNA clone IMAGE:2341475 3, mRNA sequence | 6.21 | Down | 2.37E-05 |
| AK12569 5 | Homo sapiens cDNA FLJ43707 fis, clone TESOP2001865 | 6.21 | Down | 1.16E-05 |
| NM_0057 25 | Homo sapiens tetraspan 2 (TSPAN-2), mRNA | 6.21 | Down | 7.32E-05 |
| AI694344 | wd45f11.x1 Soares_NFL_T_GBC_S1 Homo sapiens cDNA clone IMAGE:2331117 3 similar to contains MER13.b1 MER13 repetitive etement;, mRNA sequence | 6.2 | Down | 1.44E-04 |
| NM_0013 33 | Homo sapiens cathepsin L2 (CTSL2), mRNA | 6.19 | Down | 3.43E-04 |
| AI623139 | tu89b07.x1 NCI_CGAP_Gas4 Homo sapiens cDNA clone IMAGE:2258197 3, mRNA sequence | 6.18 | Down | 1.38E-04 |
| NM_0067 27 | Homo sapiens cadherin 10, type 2 (T2-cadherin) (CDH10), mRNA | 6.17 | Down | 6.43E-05 |
| M80899 | Human novel protein AHNAK mRNA, partial sequence | 6.16 | Down | 6.38E-04 |
| NM_0122 13 | Homo sapiens malonyl-CoA decarboxylase (MLYCD), mRNA | 6.15 | Down | 3.70E-05 |
| NM_0166 51 | Homo sapiens dapper homolog 1, antagonist of beta-catenin (xenopus) (DACT1), mRNA | 6.14 | Down | 6.41E-05 |
| NM_0001 29 | Homo sapiens coagulation factor XIII, A1 polypeptide (F13A1), mRNA | 6.14 | Down | 9.71E-06 |
| NM_0064 72 | Homo sapiens thioredoxin interacting protein (TXNIP), mRNA | 6.11 | Down | 1.43E-03 |
| BU72709 6 | UI-E-CR0-ach-e-12-0-UI.s1 UI-E-CR0 Homo sapiens cDNA clone UI-E-CR0-ach-e-12-0-UI 3, mRNA sequence | 6.09 | Down | 7.54E-05 |
| AF07057 1 | Homo sapiens clone 24739 mRNA sequence | 6.04 | Down | 8.86E-05 |
| NM_018120 | Homo sapiens armadillo repeat containing 1 (ARMC1), mRNA | 6.02 | Down | 2.51E-03 |
| CA41484 7 | UI-H-EZ0-bar-b-22-0-UI.s1 NCI_CGAP_Ch1 Homo sapiens cDNA clone UI-H-EZ0-bar-b-22-0-UI 3, mRNA sequence | 6.02 | Down | 4.69E-05 |
| AW4747 73 | xy06f10.x1 NCI_CGAP_Lym12 Homo sapiens cDNA clone IMAGE:2852395 3, mRNA sequence | 6.01 | Down | 8.88E-05 |
| NM_0068 51 | Homo sapiens GLI pathogenesis-related 1 (glioma) (GLIPR1), mRNA | 6.01 | Down | 2.98E-05 |
| NM_0249 97 | Homo sapiens activating transcription factor 7 interacting protein 2 (ATF7IP2), mRNA | 6 | Down | 1.65E-04 |
| NM_0039 32 | Homo sapiens suppression of tumorigenicity 13 (colon carcinoma) (Hsp70 interacting protein) (ST13), mRNA | 6 | Down | 4.81 E-04 |
| T53523 | ya89h12.r1 Stratagene placenta (#937225) Homo sapiens cDNA clone IMAGE:68903 5, mRNA sequence | 5.99 | Down | 1.60E-04 |
| NM_0169 46 | Homo sapiens F11 receptor (F11 R), transcript variant 1, mRNA | 5.99 | Down | 7.76E-04 |
| AJ40694 1 | Homo sapiens partial mRNA for keratin associated protein 4.9 (KRTAP4.9 gene) | 5.99 | Down | 1.74E-05 |
| BF51049 3 | UI-H-BI4-apa-b-08-0-UI.s1 NCI_CGAP_Sub8 Homo sapiens cDNA clone IMAGE:3086558 3, mRNA sequence | 5.93 | Down | 3.17E-05 |
| AK09443 6 | Homo sapiens cDNA FLJ37117 fis, clone BRACE2022270 | 5.92 | Down | 1.58E-04 |
| NM_032 73 | Homo sapiens Cbl-interacting protein Sts-1 8 (STS-1), mRNA | 5.92 | Down | 1.67E-05 |
| NM_015 77 | Homo sapiens retinoic acid induced 14 (RAI14), 5 mRNA | 5.91 | Down | 3.19E-03 |
| NM_014 02 | Homo sapiens KIAA0408 (KIAA0408), mRNA | 5.91 | Down | 5.11E-05 |
| AB00794 0 | Homo sapiens mRNA for KIAA0471 protein, partial cds | 5.88 | Down | 1.65E-04 |
| NM_0044 79 | Homo sapiens fucosyltransferase 7 (alpha (1,3) fucosyltransferase) (FUT7), mRNA | 5.87 | Down | 3.82E-05 |
| BC03559 9 | Homo sapiens, clone IMAGE:3871970, mRNA, partial cds | 5.86 | Down | 9.99E-05 |
| NM_0013 32 | Homo sapiens catenin (cadherin-associated protein), delta 2 (neural plakophilin-related arm-repeat protein) (CTNND2), mRNA | 5.84 | Down | 1.73E-05 |
| NM 0019 95 | Homo sapiens acyl-CoA synthetase long-chain family member 1 (ACSL1 mRNA | 5.83 | Down | 4.26E-04 |
| NM_0530 39 | Homo sapiens UDP glycosyltransferase 2 family, polypeptide B28 (UGT2B28), mRNA | 5.82 | Down | 4.93E-05 |
| BC01510 8 | Homo sapiens, Similar to otoconin 90, clone IMAGE:4044247, mRNA | 5.81 | Down | 5.63E-06 |
| | Homo sapiens potassium intermediate/small | 5.8 | Down | 1.60E-05 |
| NM_0022 50 | conductance calcium-activated channel, subfamily N, member 4 (KCNN4), mRNA | | | |
| AL11742 5 | Homo sapiens mRNA; cDNA DKFZp566L203 (from clone DKFZp566L203) | 5.76 | Down | 6.05E-05 |
| NM_0148 67 | Homo sapiens KIAA0711 gene product (KIAA0711), mRNA | 5.74 | Down | 7.24E-06 |
| BM47382 3 | AGENCOURT_6484255 NIH_MGC_72 Homo sapiens cDNA clone IMAGE:5538225 5, mRNA sequence | 5.73 | Down | 2.36E-05 |
| NM_0245 58 | Homo sapiens chromosome 14 open reading frame 138 (C14orf138), mRNA | 5.73 | Down | 8.48E-04 |
| NM_0055 78 | Homo sapiens LIM domain containing preferred translocation partner in lipoma (LPP), mRNA | 5.71 | Down | 7.52E-05 |
| AK12417 5 | Homo sapiens cDNA FLJ42181 fis, clone THYMU2031368 | 5.7 | Down | 2.30E-03 |
| NM_0132 81 | Homo sapiens fibronectin leucine rich transmembrane protein 3 (FLRT3), transcript variant 1, mRNA | 5.69 | Down | 1.38E-05 |
| AA68929 2 | nv66c11.s1 NCI_CGAP_GCB1 Homo sapiens cDNA clone IMAGE:1234772 3, mRNA sequence | 5.69 | Down | 1.42E-04 |
| NM_0026 22 | Homo sapiens prefoldin 1 (PFDN1), mRNA | 5.69 | Down | 1.69E-03 |
| BM695043 | UI-E-CL1-aez-k-18-0-UI.r1 UI-E-CL1 Homo sapiens cDNA clone UI-E-CL1-aez-k-18-0-UI 5, mRNA sequence | 5.68 | Down | 2.37E-03 |
| NM_0071 45 | Homo sapiens zinc finger protein 146 (ZNF146), mRNA | 5.67 | Down | 6.48E-04 |
| NM_0040 78 | Homo sapiens cysteine and glycine-rich protein 1 (CSRP1), mRNA | 5.67 | Down | 2.33E-04 |
| N75271 | yz74h12.r1 Soares_multiple_sclerosis_2NbHMSP Homo sapiens cDNA clone IMAGE:288839 5, mRNA sequence | 5.67 | Down | 7.47E-05 |
| AL35394 4 | Homo sapiens mRNA; cDNA DKFZp761J1112 (from clone DKFZp761 J1112) | 5.66 | Down | 3.88E-05 |
| AL04796 0 | DKFZp586B0623_r1 586 (synonym: hute1) Homo sapiens cDNA clone DKFZp586B0623, mRNA sequence | 5.63 | Down | 1.65E-04 |
| AK13109 2 | Homo sapiens mRNA for FLJ00307 protein | 5.63 | Down | 2.25E-04 |
| NM_0208 08 | Homo sapiens signal-induced proliferation-associated 1 like 2 (SIPA1L2), mRNA | 5.63 | Down | 8.36E-04 |
| NM_0034 94 | Homo sapiens dysferlin, limb girdle muscular dystrophy 2B (autosomal recessive) (DYSF), mRNA | 5.6 | Down | 5.21E-05 |
| NM_0328 83 | Homo sapiens chromosome 20 open reading frame 100 (C20orf100), mRNA | 5.58 | Down | 1.93E-05 |
| NM_006722 | Homo sapiens microphthalmia-associated transcription factor (MITF), transcript variant 3, mRNA | 5.57 | Down | 1.99E-04 |
| NM_0149 78 | Homo sapiens sortilin-related VPS10 domain containing receptor 3 (SORCS3), mRNA | 5.57 | Down | 4.68E-05 |
| BX11041 8 | BX110418 NCI_CGAP_Kid3 Homo sapiens cDNA clone IMAGp998C224149 ; IMAGE:1635405, mRNA sequence | 5.56 | Down | 5.10E-05 |
| NM_0040 87 | Homo sapiens discs, large homolog 1 (Drosophila) (DLG1), mRNA | 5.56 | Down | 1.21E-04 |
| NM_0005 61 | Homo sapiens glutathione S-transferase M1 (GSTM1), transcript variant 1, mRNA | 5.54 | Down | 5.63E-06 |
| NM_0067 85 | Homo sapiens mucosa associated lymphoid tissue lymphoma translocation gene 1 (MALT1), transcript variant 1, mRNA | 5.53 | Down | 2.39E-04 |
| NM_0528 39 | Homo sapiens pannexin 2 (PANX2), mRNA | 5.52 | Down | 2.09E-05 |
| NM_0250 85 | Homo sapiens transcriptional coactivator tubedown-100 (TBDN100), transcript variant 2, mRNA | 5.51 | Down | 1.35E-03 |
| NM_0244 22 | Homo sapiens desmocollin 2 (DSC2), transcript variant Dsc2a, mRNA | 5.5 | Down | 7.12E-05 |
| NM_0174 48 | Homo sapiens lactate dehydrogenase C (LDHC), transcript variant 2, mRNA | 5.5 | Down | 8.50E-06 |
| AK12803 6 | Homo sapiens cDNA FLJ46155 fis, clone TESTI4001517, moderately similar to Keratin, type I cytoskeletal 18 | 5.5 | Down | 2.58E-03 |
| NM_0326 23 | Homo sapiens ovary-specific acidic protein (OSAP), mRNA | 5.47 | Down | 5.65E-04 |
| BX11634 7 | BX116347 NCI_CGAP_Kid12 Homo sapiens cDNA clone IMAGp998B215967 ; IMAGE:2401844, mRNA sequence | 5.47 | Down | 1.65E-04 |
| NM_0020 31 | Homo sapiens fyn-related kinase (FRK), mRNA | 5.45 | Down | 2.62E-05 |
| CA41374 4 | UI-H-EZO-bat-h-12-0-UI.s1 NCI_CGAP_Ch1 Homo sapiens cDNA clone UI-H-EZ0-bat-h-12-0-UI 3, mRNA sequence | 5.45 | Down | 1.32E-04 |
| T78754 | yd01f08.r1 Soares infant brain 1NIB Homo sapiens cDNA clone IMAGE:24180 5, mRNA sequence | 5.45 | Down | 1.14E-04 |
| NM_0050 82 | Homo sapiens tripartite motif-containing 25 (TRIM25), mRNA | 5.44 | Down | 4.71E-04 |
| NM_0328 57 | Homo sapiens lactamase, beta (LACTB), nuclear gene encoding mitochondrial protein, transcript variant 1, mRNA | 5.43 | Down | 6.74E-04 |
| NM_1736 62 | Homo sapiens hypothetical protein LOC285533 (LOC285533), mRNA | 5.43 | Down | 1.72E-04 |
| NM_0021 66 | Homo sapiens inhibitor of DNA binding 2, dominant negative helix-loop-helix protein (ID2), mRNA | 5.43 | Down | 2.13E-04 |
| NM_0005 93 | Homo sapiens transporter 1, ATP-binding cassette, sub-family B (MDR/TAP) (TAP1), mRNA | 5.42 | Down | 5.84E-04 |
| | Ul-H-DH0-arx-p-21-0-UI.s1 NCI_CGAP_DH0 | 5.42 | Down | 2.89E-05 |
| BM98864 2 | Homo sapiens cDNA clone IMAGE:5855492 3, mRNA sequence | | | |
| NM_0249 01 | Homo sapiens hypothetical protein FLJ22457 (FLJ22457), mRNA | 5.41 | Down | 4.87E-04 |
| NM_0178 23 | Homo sapiens dual specificity phosphatase 23 (DUSP23), mRNA | 5.41 | Down | 5.55E-04 |
| BX10783 8 | BX107838 NCI_CGAP_Lu5 Homo sapiens cDNA clone IMAGp998A153853 ; IMAGE:1521686, mRNA sequence | 5.39 | Down | 2.44E-04 |
| AW 1729 03 | xj05e04.x1 NCI_CGAP_Ut2 Homo sapiens cDNA clone IMAGE:2656350 3, mRNA sequence | 5.39 | Down | 1.67E-05 |
| BX11132 1 | BX111321 Soares_pregnant_uterus_NbHPU Homo sapiens cDNA clone IMAGp998C221112 ; IMAGE:469197, mRNA sequence | 5.39 | Down | 1.46E-03 |
| NM_0332 11 | Homo sapiens hypothetical gene supported by AF038182; BC009203 (LOC90355), mRNA | 5.38 | Down | 1.15E-04 |
| D62831 | HUM330B12B Clontech human aorta polyA+ mRNA (#6572) Homo sapiens cDNA clone GEN-330B12 5, mRNA sequence | 5.37 | Down | 5.80E-03 |
| NM_0010 69 | Homo sapiens tubulin, beta polypeptide (TUBB), mRNA | 5.36 | Down | 4.71E-04 |
| NM_0043 24 | Homo sapiens BCL2-associated X protein (BAX), transcript variant beta, mRNA | 5.36 | Down | 8.14E-04 |
| AV736303 | AV736303 CB Homo sapiens cDNA clone CBCAJD04 5, mRNA sequence | 5.36 | Down | 4.51E-05 |
| AK13153 2 | Homo sapiens cDNA FLJ16761 fis, clone BRAMY3008096 | 5.34 | Down | 9.55E-05 |
| NM_0208 59 | Homo sapiens Shroom-related protein (ShrmL), mRNA | 5.34 | Down | 1.21E-04 |
| NM_0025 75 | Homo sapiens serine (or cysteine) proteinase inhibitor, clade B (ovalbumin), member 2 (SERPINB2), mRNA | 5.34 | Down | 8.70E-05 |
| NM_0175 99 | Homo sapiens transmembrane protein vezatin (VEZATIN), mRNA | 5.33 | Down | 6.62E-04 |
| BX11607 1 | BX116071 Soares_pregnant_uterus_NbHPU Homo sapiens cDNA clone IMAGp998L201165 ; IMAGE:489763, mRNA sequence | 5.33 | Down | 1.39E-04 |
| NM_0246 77 | Homo sapiens hypothetical protein FLJ14001 (FLJ14001), mRNA | 5.32 | Down | 5.07E-06 |
| AL83277 9 | Homo sapiens mRNA; cDNA DKFZp686H157 (from clone DKFZp686H157) | 5.32 | Down | 4.37E-04 |
| NM_0025 47 | Homo sapiens oligophrenin 1 (OPHN1), mRNA | 5.32 | Down | 5.03E-05 |
| NM_0143 22 | Homo sapiens opsin 3 (encephalopsin, panopsin) (OPN3), mRNA | 5.3 | Down | 4.04E-04 |
| NM_0030 57 | Homo sapiens solute carrier family 22 (organic cation transporter), member 1 (SLC22A1), transcript variant 1, mRNA | 5.29 | Down | 1.92E-04 |
| BM969191 | UI-CF-EN0-acp-e-22-0-UI.s1 UI-CF-EN0 Homo sapiens cDNA clone UI-CF-EN0-acp-e-22-0-UI 3, mRNA sequence | 5.28 | Down | 4.06E-04 |
| S69208 | troponin T [human, skeletal and cardiac muscle, mRNA, 932 nt] | 5.28 | Down | 7.73E-04 |
| NM_0312 16 | Homo sapiens SEH1-like (S. cerevisiae) (SEH1L), mRNA | 5.27 | Down | 1.17E-03 |
| NM_0029 67 | Homo sapiens scaffold attachment factor B (SAFB), mRNA | 5.27 | Down | 5.52E-04 |
| NM_0028 43 | Homo sapiens protein tyrosine phosphatase, receptor type, J (PTPRJ), mRNA | 5.27 | Down | 5.63E-06 |
| NM_0186 64 | Homo sapiens Jun dimerization protein p21SNFT (SNFT), mRNA | 5.27 | Down | 3.06E-05 |
| NM_0209 44 | Homo sapiens glucosidase, beta (bile acid) 2 (GBA2), mRNA | 5.25 | Down | 3.07E-04 |
| BM98720 0 | UI-H-CO0-aql-b-05-0-UI.s1 NCI_CGAP_Sub9 Homo sapiens cDNA clone IMAGE:3104192 3, mRNA sequence | 5.24 | Down | 8.03E-05 |
| NM_0239 15 | Homo sapiens G protein-coupled receptor 87 (GPR87), mRNA | 5.23 | Down | 2.05E-04 |
| NM_0156 78 | Homo sapiens neurobeachin (NBEA), mRNA | 5.23 | Down | 2.95E-05 |
| NM_004438 | Homo sapiens EPH receptor A4 (EPHA4), mRNA | 5.22 | Down | 1.93E-05 |
| NM_0022 06 | Homo sapiens integrin, alpha 7 (ITGA7), mRNA | 5.22 | Down | 2.36E-05 |
| AW5914 61 | xI92h06.x1 NCI_CGAP_Ut1 Homo sapiens cDNA clone IMAGE:2682203 3, mRNA sequence | 5.22 | Down | 7.14E-05 |
| AK05603 3 | Homo sapiens cDNA FLJ31471 fis, clone NT2NE2001435 | 5.19 | Down | 3.29E-04 |
| NM_0182 35 | Homo sapiens CNDP dipeptidase 2 (metallopeptidase M20 family) (CNDP2), mRNA | 5.19 | Down | 3.74E-03 |
| AV72829 4 | AV728294 HTC Homo sapiens cDNA clone HTCBIE09 5, mRNA sequence | 5.19 | Down | 3.05E-04 |
| NM_0247 25 | Homo sapiens hypothetical protein FLJ23518 (FLJ23518), mRNA | 5.18 | Down | 2.56E-03 |
| NM_0018 74 | Homo sapiens carboxypeptidase M (CPM), transcript variant 1, mRNA | 5.18 | Down | 4.89E-05 |
| W30761 | zb76g12.r1 Soares_senescent_fibroblasts_NbHSF Homo sapiens cDNA clone IMAGE:309574 5, mRNA sequence | 5.15 | Down | 1.61E-04 |
| BM74199 7 | K-EST0014724 S7SNU719 Homo sapiens cDNA clone S7SNU719-7-F05 5, mRNA sequence | 5.14 | Down | 1.15E-02 |
| NM_017594 | Homo sapiens DIRAS family, GTP-binding RAS-like 2 (DIRAS2), mRNA | 5.14 | Down | 1.38E-03 |
| NM_0223 43 | Homo sapiens chromosome 9 open reading frame 19 (C9orf19), mRNA | 5.13 | Down | 2.67E-05 |
| NM_0032 87 | Homo sapiens tumor protein D52-like 1 (TPD52L1 transcript variant 1, mRNA | 5.13 | Down | 2.71E-05 |
| BC06710 6 | Homo sapiens putative G protein coupled receptor, mRNA (cDNA clone MGC:71222 IMAGE:6645890), complete cds | 5.12 | Down | 2.13E-04 |
| NM_1818 28 | Homo sapiens neurofibromin 2 (bilateral acoustic neuroma) (NF2), transcript variant 5, mRNA | 5.12 | Down | 1.99E-03 |
| NM_0052 64 | Homo sapiens GDNF family receptor alpha 1 (GFRA1), transcript variant 1, mRNA | 5.11 | Down | 4.23E-05 |
| AK09215 7 | Homo sapiens cDNA FLJ34838 fis, clone NT2NE2010654 | 5.11 | Down | 6.46E-05 |
| NM_0229 12 | Homo sapiens chromosome 2 open reading frame 23 (C2orf23), mRNA | 5.09 | Down | 5.42E-05 |
| NM_0529 37 | Homo sapiens similar to hypothetical protein FLJ10883 (LOC115294), mRNA | 5.09 | Down | 1.38E-04 |
| BM66545 2 | UI-E-CQ1-aex-n-03-0-UI.s1 UI-E-CQ1 Homo sapiens cDNA clone UI-E-CQ1-aex-n-03-0-UI 3, mRNA sequence | 5.08 | Down | 3.24E-04 |
| NM_0038 31 | Homo sapiens RIO kinase 3 (yeast) (RIOK3), transcript variant 1, mRNA | 5.08 | Down | 1.64E-04 |
| NM_1816 89 | Homo sapiens neuronatin (NNAT), transcript variant 2, mRNA | 5.08 | Down | 7.40E-06 |
| BC03312 4 | Homo sapiens, clone IMAGE:2960615, mRNA | 5.07 | Down | 3.52E-05 |
| NM_0160 03 | Homo sapiens WIPI49-like protein 2 (DKFZP434J154), transcript variant 2, mRNA | 5.07 | Down | 6.36E-03 |
| BC03855 6 | Homo sapiens, clone IMAGE:3446976, mRNA | 5.06 | Down | 2.32E-04 |
| AW0695 77 | cr47c06.x1 Human bone marrow stromal cells Homo sapiens cDNA clone HBMSC_cr47c06 3, mRNA sequence | 5.06 | Down | 2.08E-03 |
| NM_0000 55 | Homo sapiens butyrylcholinesterase (BCHE), mRNA | 5.05 | Down | 1.20E-05 |
| NM_0056 02 | Homo sapiens claudin 11 (oligodendrocyte transmembrane protein) (CLDN11), mRNA | 5.04 | Down | 7.46E-04 |
| NM_0309 52 | Homo sapiens likely ortholog of rat SNF1/AMP-activated protein kinase (SNARK), mRNA | 5.03 | Down | 8.60E-05 |
| AK09237 9 | Homo sapiens cDNA FLJ35060 fis, clone OCBBF2018828 | 5.03 | Down | 1.67E-05 |
| AK09501 3 | Homo sapiens cDNA FLJ37694 fis, clone BRHIP2015224 | 5.01 | Down | 1.87E-04 |
| NM_1452 34 | Homo sapiens chordin-like 1 (CHRDL1), mRNA | 5.01 | Down | 1.11E-05 |
| NM_1992 87 | Homo sapiens similar to RIKEN cDNA 3110023B02 (MGC16597), mRNA | 5 | Down | 9.27E-05 |
| NM_0251 51 | Homo sapiens RAB11 family interacting protein 1 (class I) (RAB11FIP1), transcript variant 1, mRNA | 5 | Down | 4.32E-05 |

**TABLE VII G: GENES THAT WERE DIFFERENTIALLY EXPRESSED AT LEAST 5 FOLD IN AF-II VERSUS AF-III CELLS**

| Gene Identifier | Gene Name | Average fold change AF-II vs AF-III | Direction | adj. p-value |
|---|---|---|---|---|
| NM_0034 11 | Homo sapiens zinc finger protein, Y-linked (ZFY), mRNA | 328.28 | Up | 1.97E-06 |
| NM_1536 34 | Homo sapiens copine VIII (CPNE8), mRNA | 286.5 | Up | 6.16E-06 |
| H70730 | yu69e10.r1 Weizmann Olfactory Epithelium Homo sapiens cDNA clone IMAGE:239082 5, mRNA sequence | 272.44 | Up | 1.97E-06 |
| NM_1389 63 | Homo sapiens ribosomal protein S4, Y-linked 2 (RPS4Y2), mRNA | 265.55 | Up | 1.56E-06 |
| NM_0046 53 | Homo sapiens Smcy homolog, Y-linked (mouse) (SMCY), mRNA | 246.17 | Up | 1.00E-06 |
| NM_0046 60 | Homo sapiens DEAD (Asp-Glu-Ala-Asp) box polypeptide 3, Y-linked (DDX3Y), mRNA | 244.93 | Up | 1.17E-05 |
| NM_0123 07 | Homo sapiens erythrocyte membrane protein band 4.1-like 3 (EPB41L3), mRNA | 207.61 | Up | 1.83E-06 |
| BX08955 4 | BX089554 Soares placenta Nb2HP Homo sapiens cDNA clone IMAGp998P07210 ; IMAGE:142326, mRNA sequence | 119.7 | Up | 2.95E-06 |
| NM_0010 08 | Homo sapiens ribosomal protein S4, Y-linked 1 (RPS4Y1), mRNA | 112.79 | Up | 2.02E-05 |
| NM_0148 93 | Homo sapiens neuroligin 4, Y-linked (NLGN4Y), mRNA | 93.96 | Up | 9.61E-06 |
| NM_1827 98 | Homo sapiens hypothetical protein FLJ39155 (FLJ39155), transcript variant 2, mRNA | 71.46 | Up | 1.36E-05 |
| NM_0808 72 | Homo sapiens unc-5 homolog D (C. elegans) (UNC5D), mRNA | 48.16 | Up | 5.41E-05 |
| BX64864 3 | Homo sapiens mRNA; cDNA DKFZp686O17106 (from clone DKFZp686O17106) | 47.84 | Up | 2.95E-06 |
| NM_0029 28 | Homo sapiens regulator of G-protein signalling 16 (RGS16), mRNA | 47.75 | Up | 2.99E-05 |
| AB03780 5 | Homo sapiens mRNA for KIAA1384 protein, partial cds | 45.54 | Up | 7.68E-06 |
| NM_0318 62 | Homo sapiens membrane component, chromosome 17, surface marker 2 (ovarian carcinoma antigen CA125) (M17S2), transcript variant 3, mRNA | 44.02 | Up | 5.15E-05 |
| CA42913 5 | UI-H-FH1-bfh-k-22-0-UI.s1 NCI_CGAP_FH1 Homo sapiens cDNA clone UI-H-FH1-bfh-k-22-0-UI 3, mRNA sequence | 43.87 | Up | 2.90E-06 |
| BC04341 | Homo sapiens, clone IMAGE:6155889, mRNA | 42.03 | Up | 6.97E-06 |
| 1 | | | | |
| NM_0046 81 | Homo sapiens eukaryotic translation initiation factor 1A, Y-linked (EIF1AY), mRNA | 41.7 | Up | 2.24E-04 |
| NM_1814 81 | Homo sapiens chromosome 18 open reading frame 1 (C18orf1), transcript variant a1, mRNA | 39.6 | Up | 9.61E-06 |
| BE46576 0 | hw22f09.x1 NCI_CGAP_Kid11 Homo sapiens cDNA clone IMAGE:3183689 3, mRNA sequence | 39.51 | Up | 1.50E-05 |
| NM_0026 67 | Homo sapiens phospholamban (PLN), mRNA | 37.56 | Up | 1.87E-05 |
| AK09807 1 | Homo sapiens cDNA FLJ40752 fis, clone TRACH2000972 | 34.71 | Up | 1.02E-04 |
| NM_0018 64 | Homo sapiens cytochrome c oxidase subunit VIIa polypeptide 1 (muscle) (COX7A1), mRNA | 33.43 | Up | 1.08E-06 |
| AF05537 6 | Homo sapiens short form transcription factor C-MAF (c-maf) mRNA, complete cds | 32.71 | Up | 9.41E-04 |
| NM_0049 32 | Homo sapiens cadherin 6, type 2, K-cadherin (fetal kidney) (CDH6), mRNA | 31.51 | Up | 1.37E-05 |
| NM_0025 15 | Homo sapiens neuro-oncological ventral antigen 1 (NOVA1), transcript variant 1, mRNA | 30.4 | Up | 1.97E-06 |
| NM_0183 49 | Homo sapiens multiple C2-domains with two transmembrane regions 2 (MCTP2), mRNA | 29.49 | Up | 1.68E-05 |
| NM_0009 90 | Homo sapiens ribosomal protein L27a (RPL27A), mRNA | 28.23 | Up | 4.25E-04 |
| BX11135 3 | BX111353 NCI_CGAP_Kid3 Homo sapiens cDNA clone IMAGp998O094576 ; IMAGE:1869152, mRNA sequence | 27.4 | Up | 3.27E-06 |
| NM_0325 76 | Homo sapiens chromosome Y open reading frame 15B (CYorf15B), mRNA | 27.08 | Up | 3.10E-06 |
| NM_0209 97 | Homo sapiens left-right determination factor 1 (LEFTY1), mRNA | 26.55 | Up | 1.42E-05 |
| NM_0064 08 | Homo sapiens anterior gradient 2 homolog (Xenopus laevis) (AGR2), mRNA | 25.93 | Up | 2.36E-05 |
| BQ92483 2 | AGENCOURT_8840265 Lupski_sciatic_nerve Homo sapiens cDNA clone IMAGE:6205036 5, mRNA sequence | 24.29 | Up | 2.00E-06 |
| NM_0190 00 | Homo sapiens hypothetical protein FLJ20152 (FLJ20152), mRNA | 23.73 | Up | 7.10E-05 |
| BC03565 6 | Homo sapiens hypothetical protein LOC285835, mRNA (cDNA clone IMAGE:5588650), partial cds | 23.25 | Up | 1.82E-06 |
| AI032876 | ow13g03.x1 Soares_parathyroid_tumor_NbHPA Homo sapiens cDNA clone IMAGE:1646740 3, mRNA sequence | 22.96 | Up | 5.20E-06 |
| NM_0013 05 | Homo sapiens claudin 4 (CLDN4), mRNA | 21.95 | Up | 3.72E-04 |
| BX08955 4 | BX089554 Soares placenta Nb2HP Homo sapiens cDNA clone IMAGp998P07210 ; IMAGE:142326, mRNA sequence | 20.73 | Up | 2.00E-06 |
| CF13754 5 | UI-HF-BN0-ane-d-05-0-UI.r1 NIH_MGC_50 Homo sapiens cDNA clone IMAGE:3092384 5, mRNA sequence | 20.24 | Up | 7.57E-05 |
| CB04709 2 | NISC_gf08f03.x1 NCI_CGAP_Kid12 Homo sapiens cDNA clone IMAGE:3253013 3, mRNA sequence | 20.2 | Up | 4.77E-06 |
| NM_0051 14 | Homo sapiens heparan sulfate (glucosamine) 3-O-sulfotransferase 1 (HS3ST1), mRNA | 19.91 | Up | 2.92E-05 |
| BQ43078 8 | AGENCOURT_7776027 NIH_MGC_68 Homo sapiens cDNA clone IMAGE:6024295 5, mRNA sequence | 19.78 | Up | 9.59E-04 |
| AK02423 8 | Homo sapiens cDNA FLJ14176 fis, clone NT2RP2003101 | 19.33 | Up | 2.32E-05 |
| NM_0010 01931 | Homo sapiens mitochondrial tumor suppressor 1 (MTUS1), nuclear gene encoding mitochondrial protein, transcript variant 4, mRNA | 18.91 | Up | 2.90E-06 |
| NM_1524 23 | Homo sapiens melanoma associated antigen (mutated) 1-like 1 (MUM1L1), mRNA | 18.66 | Up | 1.97E-06 |
| BX11359 0 | BX113590 Soares_fetal_heart_NbHH19W Homo sapiens cDNA clone IMAGp998A14871 ; IMAGE:376597, mRNA sequence | 18.22 | Up | 3.83E-05 |
| NM_0025 | Homo sapiens neuronal PAS domain protein 2 (NPAS2), mRNA | 17.82 | Up | 3.88E-05 |
| 18 | | | | |
| AK12871 5 | Homo sapiens cDNA FLJ46882 fis, clone UTERU3015844 | 17.2 | Up | 1.17E-03 |
| AA73825 4 | nx13b02.s1 NCI_CGAP_GC3 Homo sapiens cDNA clone IMAGE:1255947 3, mRNA sequence | 16.61 | Up | 6.24E-06 |
| NM_0033 81 | Homo sapiens vasoactive intestinal peptide (VIP), transcript variant 1, mRNA | 16.44 | Up | 5.33E-06 |
| AI951740 | wv38h09.x1 NCI_CGAP_Ov18 Homo sapiens cDNA clone IMAGE:2531873 3, mRNA sequence | 14.99 | Up | 6.49E-03 |
| NM_0063 93 | Homo sapiens nebulette (NEBL), transcript variant 1, mRNA | 14.87 | Up | 1.84E-03 |
| NM_0009 63 | Homo sapiens prostaglandin-endoperoxide synthase 2 (prostaglandin G/H synthase and cyclooxygenase) (PTGS2), mRNA | 14.83 | Up | 3.02E-03 |
| H89053 | yw24c06.r1 Morton Fetal Cochlea Homo sapiens cDNA clone IMAGE:253162 5, mRNA sequence | 14.76 | Up | 6.87E-03 |
| D86975 | Homo sapiens mRNA for KIAA0222 gene, partial cds | 14.7 | Up | 2.87E-04 |
| AA10255 3 | zn26a04.s1 Stratagene neuroepithelium NT2RAMI 937234 Homo sapiens cDNA clone IMAGE:548526 3, mRNA sequence | 13.56 | Up | 2.86E-04 |
| AK09670 8 | Homo sapiens cDNA FLJ39389 fis, clone PLACE6003621 | 13.38 | Up | 2.90E-05 |
| BM68512 4 | UI-E-EJ1-ajl-I-13-0-UI.s1 UI-E-EJ1 Homo sapiens cDNA clone UI-E-EJ1-ajl-I-13-0-UI 3, mRNA sequence | 12.74 | Up | 2.49E-04 |
| AI220066 | qg84d01.x1 Soares_NFL_T_GBC_S1 Homo sapiens cDNA clone IMAGE:1841857 3, mRNA sequence | 12.47 | Up | 1.90E-04 |
| AI342246 | qt26g09.x1 Soares_pregnant_uterus_NbHPU Homo sapiens cDNA clone IMAGE:1949152 3, mRNA sequence | 12.29 | Up | 7.15E-04 |
| NM_0245 01 | Homo sapiens homeo box D1 (HOXD1), mRNA | 12.11 | Up | 9.73E-06 |
| NM_0163 07 | Homo sapiens paired related homeobox 2 (PRRX2), mRNA | 12 | Up | 9.50E-04 |
| AB02069 1 | Homo sapiens mRNA for KIAA0884 protein, partial cds | 11.69 | Up | 1.47E-04 |
| BE29546 8 | 601174523F1 NIH_MGC_17 Homo sapiens cDNA clone IMAGE:3529924 5, mRNA sequence | 11.64 | Up | 1.47E-03 |
| BU95146 9 | in60a05.x3 HR85 islet Homo sapiens cDNA clone IMAGE:6126249 3, mRNA sequence | 11.37 | Up | 3.28E-05 |
| M_1981 74 | Homo sapiens transcription factor CP2-like 4 (TFCP2L4), transcript variant 3, mRNA | 11.26 | Up | 3.35E-05 |
| NM_0024 21 | Homo sapiens matrix metalloproteinase 1 (interstitial collagenase) (MMP1), mRNA | 11.12 | Up | 5.10E-04 |
| TM_1453 13 | Homo sapiens RasGEF domain family, member 1A (RASGEF1A), mRNA | 10.64 | Up | 2.38E-05 |
| NM_0009 01 | Homo sapiens nuclear receptor subfamily 3, group C, member 2 (NR3C2), mRNA | 10.62 | Up | 3.93E-06 |
| AL55252 7 | AL552527 Homo sapiens PLACENTA COT 25-NORMALIZED Homo sapiens cDNA clone CSODI067YL24 3-PRIME, mRNA sequence | 10.61 | Up | 3.88E-03 |
| NM_0529 54 | Homo sapiens cysteine and tyrosine-rich 1 (CYYR1), mRNA | 10.58 | Up | 7.22E-05 |
| AF51962 2 | Homo sapiens noncoding mRNA sequence | 10.39 | Up | 1.49E-04 |
| NM_0066 81 | Homo sapiens neuromedin U (NMU), mRNA | 10.37 | Up | 2.92E-05 |
| W93585 | zd95g01.s1 Soares_fetal_heart_NbHH19W Homo sapiens cDNA clone IMAGE:357264 3, mRNA sequence | 10.27 | Up | 2.01E-04 |
| BX10515 2 | BX105152 Soares_testis_NHT Homo sapiens cDNA clone IMAGp998J212575 ; IMAGE:1031156, mRNA sequence | 9.89 | Up | 4.37E-05 |
| AV70923 2 | AV709232 ADC Homo sapiens cDNA clone ADCAJB11 5, mRNA sequence | 9.89 | Up | 1.56E-04 |
| W69644 | zd45f10.r1 Soares_fetal_heart_NbHH19W Homo sapiens cDNA clone IMAGE:343627 5, mRNA sequence | 9.89 | Up | 1.90E-05 |
| NM_0018 78 | Homo sapiens cellular retinoic acid binding protein 2 (CRABP2), mRNA | 9.82 | Up | 2.65E-04 |
| NM_1527 54 | Homo sapiens sema domain, immunoglobulin domain (Ig), short basic domain, secreted, (semaphorin) 3D (SEMA3D), mRNA | 9.78 | Up | 6.57E-04 |
| BE87776 4 | 601486331 F1 NIH_MGC_69 Homo sapiens cDNA clone IMAGE:3888943 5, mRNA sequence | 9.59 | Up | 1.90E-04 |
| AK02590 9 | Homo sapiens cDNA: FLJ22256 fis, clone HRC02860 | 9.52 | Up | 8.65E-05 |
| NM_0155 59 | Homo sapiens SET binding protein 1 (SETBP1), mRNA | 9.3 | Up | 3.69E-04 |
| AI493349 | tg70f04.x1 Soares_NhHMPu_S1 Homo sapiens cDNA clone IMAGE:2114143 3, mRNA sequence | 9.21 | Up | 1.86E-05 |
| AK12995 5 | Homo sapiens cDNA FLJ26445 fis, clone KDN02608 | 9.2 | Up | 1.41E-04 |
| BX09852 1 | BX098521 Soares fetal liver spleen 1 NFLS Homo sapiens cDNA clone IMAGp998L05118 ; IMAGE:123412, mRNA sequence | 9.06 | Up | 9.68E-04 |
| AK09429 2 | Homo sapiens cDNA FLJ36973 fis, clone BRACE2006249 | 9.05 | Up | 3.44E-05 |
| AK09577 | Homo sapiens cDNA FLJ38457 fis, clone FEBRA2020400 | 8.98 | Up | 1.13E-05 |
| 6 | | | | |
| NM_0182 42 | Homo sapiens hypothetical protein FLJ10847 (FLJ10847), mRNA | 8.98 | Up | 8.02E-04 |
| AW0068 64 | ws15d04.x1 NCI_CGAP_Kid11 Homo sapiens cDNA clone IMAGE:2497255 3, mRNA sequence | 8.93 | Up | 1.50E-05 |
| BX10384 6 | BX103846 NCI_CGAP_Lu24 Homo sapiens cDNA clone IMAGp998F125811 ; IMAGE:2342027, mRNA sequence | 8.89 | Up | 1.38E-04 |
| NM_0244 22 | Homo sapiens desmocollin 2 (DSC2), transcript variant Dsc2a, mRNA | 8.84 | Up | 3.19E-05 |
| NM_0324 71 | Homo sapiens protein kinase (cAMP-dependent, catalytic) inhibitor beta (PKIB), transcript variant 3, mRNA | 8.77 | Up | 1.35E-03 |
| AK12477 8 | Homo sapiens cDNA FLJ42788 fis, clone BRAWH3007129 | 8.62 | Up | 4.29E-04 |
| H25898 | yl55b10.r1 Soares breast 3NbHBst Homo sapiens cDNA clone IMAGE:162139 5, mRNA sequence | 8.52 | Up | 1.29E-04 |
| NM_0155 64 | Homo sapiens leucine rich repeat transmembrane neuronal 2 (LRRTM2), mRNA | 8.5 | Up | 3.01E-03 |
| AI126888 | qb95d06.x1 Soares_fetal_heart_NbHH19W Homo sapiens cDNA clone IMAGE:1707851 3, mRNA sequence | 8.5 | Up | 6.30E-05 |
| BC04202 8 | Homo sapiens, clone IMAGE:4794726, mRNA | 8.46 | Up | 3.65E-02 |
| NM_0065 61 | Homo sapiens CUG triplet repeat, RNA binding protein 2 (CUGBP2), mRNA | 8.37 | Up | 2.52E-03 |
| NM_0046 75 | Homo sapiens ras homolog gene family, member I (ARHI), mRNA | 8.36 | Up | 4.99E-04 |
| BE50391 6 | hz35g01.x1 NCI_CGAP_GC6 Homo sapiens cDNA clone IMAGE:3210000 3, mRNA sequence | 8.3 | Up | 1.00E-05 |
| NM_2074 46 | Homo sapiens hypothetical gene supported by AK075564; BC060873 (LOC400451), mRNA | 8.28 | Up | 2.72E-05 |
| AK09173 1 | Homo sapiens cDNA FLJ34412 fis, clone HEART2002432 | 8.08 | Up | 9.36E-05 |
| NM_0022 61 | Homo sapiens killer cell lectin-like receptor subfamily C, member 3 (KLRC3), transcript variant NKG2-E, mRNA | 8.07 | Up | 2.69E-04 |
| AW2709 28 | xs06c05.x1 NCI_CGAP_Kid11 Homo sapiens cDNA clone IMAGE:2768840 3, mRNA sequence | 7.99 | Up | 4.03E-05 |
| NM_0050 79 | Homo sapiens tumor protein D52 (TPD52), mRNA | 7.97 | Up | 4.79E-05 |
| NM_0013 53 | Homo sapiens aldo-keto reductase family 1, member C1 (dihydrodiol dehydrogenase 1; 20-alpha (3-alpha)-hydroxysteroid dehydrogenase) (AKR1C1), mRNA | 7.88 | Up | 3.67E-04 |
| AI830524 | wh52c02.x1 NCI_CGAP_Kid11 Homo sapiens cDNA clone IMAGE:2384354 3, mRNA sequence | 7.82 | Up | 1.23E-03 |
| BX64820 7 | Homo sapiens mRNA; cDNA DKFZp686E16168 (from clone DKFZp686E16168) | 7.82 | Up | 9.59E-04 |
| AW0437 93 | wy76d11.x1 Soares_NSF_F8_9W_OT_PA_P_S1 Homo sapiens cDNA clone IMAGE:2554485 3 similar to contains element MER18 repetitive element ;, mRNA sequence | 7.79 | Up | 4.64E-05 |
| N63415 | yy60d04.s1 Soares_multiple_sclerosis_2NbHMSP Homo sapiens cDNA clone IMAGE:277927 3 similar to contains L1.b3 L1 repetitive element ;, mRNA sequence | 7.76 | Up | 1.14E-03 |
| NM_0022 60 | Homo sapiens killer cell lectin-like receptor subfamily C, member 2 (KLRC2), mRNA | 7.76 | Up | 3.65E-04 |
| NM_0048 48 | Homo sapiens chromosome 1 open reading frame 38 (C1orf38), mRNA | 7.75 | Up | 5.34E-04 |
| NM_0249 93 | Homo sapiens leucine rich repeat transmembrane neuronal 4 (LRRTM4), mRNA | 7.53 | Up | 3.35E-05 |
| AI821210 | ne08e05.y5 NCI_CGAP_Co3 Homo sapiens cDNA clone IMAGE:880640 5, mRNA sequence | 7.51 | Up | 5.58E-04 |
| BM66444 5 | UI-E-CL1-afa-p-05-0-UI.s1 UI-E-CL1 Homo sapiens cDNA clone UI-E-CL1-afa-p-05-0-UI 3, mRNA sequence | 7.4 | Up | 5.30E-04 |
| NM_0196 44 | Homo sapiens ankyrin repeat domain 7 (ANKRD7), mRNA | 7.39 | Up | 2.24E-04 |
| BU62079 3 | UI-H-FL1-bfx-d-10-0-UI.s1 NCI_CGAP_FL1 Homo sapiens cDNA clone UI-H-FL1-bfx-d-10-0-UI 3, mRNA sequence | 7.35 | Up | 1.13E-05 |
| NM_002 46 | Homo sapiens potassium channel, 2 subfamily K, member 3 (KCNK3), mRNA | 7.31 | Up | 1.68E-05 |
| NM_031 42 | Homo sapiens transmembrane 4 4 superfamily member 10 (TM4SF10), mRNA | 7.13 | Up | 2.31E-03 |
| BX09250 1 | BX092501 NCI_CGAP_Lu5 Homo sapiens cDNA clone IMAGp998K143946 ; IMAGE:1557637, mRNA sequence | 7.12 | Up | 1.69E-03 |
| NM_1779 49 | Homo sapiens armadillo repeat containing, X-linked 2 (ARMCX2), mRNA | 7.09 | Up | 9.21E-03 |
| NM_0183 76 | Homo sapiens nipsnap homolog 3B (C. elegans) (NIPSNAP3B), mRNA | 7.03 | Up | 2.53E-02 |
| AK09376 2 | Homo sapiens cDNA FLJ36443 fis, clone THYMU2012891 | 7.02 | Up | 1.23E-03 |
| NM_0186 58 | Homo sapiens potassium inwardly-rectifying channel, subfamily J, member 16 (KCNJ16), transcript variant 1, mRNA | 7.01 | Up | 6.28E-04 |
| AB03294 5 | Homo sapiens mRNA for KIAA1119 protein, partial cds | 6.98 | Up | 1.11E-04 |
| NM_0318 94 | Homo sapiens ferritin, heavy polypeptide-like 17 (FTHL17), mRNA | 6.97 | Up | 3.42E-03 |
| | UI-H-BI1-aee-a-12-0-UI.s1 | 6.96 | Up | 3.35E-05 |
| AW 1398 91 | NCI-CGAP-Sub3 Homo sapiens cDNA clone IMAGE:2719006 3, mRNA sequence | | | |
| AK12456 2 | Homo sapiens cDNA FLJ42571 fis, clone BRACE3008036 | 6.9 | Up | 2.57E-05 |
| NM_0019 35 | Homo sapiens dipeptidylpeptidase 4 (CD26, adenosine deaminase complexing protein 2) (DPP4), mRNA | 6.9 | Up | 1.08E-03 |
| AI686652 | tu35d06.x1 NCI_CGAP_Pr28 Homo sapiens cDNA clone IMAGE:2253035 3, mRNA sequence | 6.87 | Up | 4.03E-06 |
| NM_1840 87 | Homo sapiens tripartite motif-containing 55 (TRIM55), transcript variant 4, mRNA | 6.85 | Up | 1.68E-05 |
| NM_0020 89 | Homo sapiens chemokine (C-X-C motif) ligand 2 (CXCL2), mRNA | 6.81 | Up | 9.44E-05 |
| NM_0168 31 | Homo sapiens period homolog 3 (Drosophila) (PER3), mRNA | 6.81 | Up | 6.24E-06 |
| NM_0025 15 | Homo sapiens neuro-oncological ventral antigen 1 (NOVA1), transcript variant 1, mRNA | 6.79 | Up | 4.79E-05 |
| BC01290 0 | Homo sapiens, clone IMAGE:3881549, mRNA | 6.77 | Up | 1.84E-02 |
| NM_0529 23 | Homo sapiens zinc finger protein 452 (ZNF452), mRNA | 6.75 | Up | 2.98E-03 |
| NM_0218 | Homo sapiens DnaJ (Hsp40) homolog, subfamily C, member 12 (DNAJC12), | 6.73 | Up | 5.46E-04 |
| 00 | transcript variant 1, mRNA | | | |
| AK05688 2 | Homo sapiens cDNA FLJ32320 fis, clone PROST2003537 | 6.71 | Up | 9.55E-04 |
| BF70068 4 | 602128672F1 NIH_MGC_56 Homo sapiens cDNA clone IMAGE:4285673 5, mRNA sequence | 6.7 | Up | 4.03E-06 |
| NM_1446 64 | Homo sapiens hypothetical protein MGC33371 (MGC33371), mRNA | 6.66 | Up | 1.64E-03 |
| AL83277 9 | Homo sapiens mRNA; cDNA DKFZp686H157 (from clone DKFZp686H157) | 6.62 | Up | 8.14E-05 |
| NM_0183 71 | Homo sapiens chondroitin beta1,4 N-acetylgalactosaminyltransferase (ChGn), mRNA | 6.6 | Up | 6.54E-04 |
| NM_0143 99 | Homo sapiens transmembrane 4 superfamily member 13 (TM4SF13), mRNA | 6.56 | Up | 9.52E-03 |
| AA74876 2 | ny06h10.s1 NCI_CGAP_GCB1 Homo sapiens cDNA clone IMAGE:1271011 3, mRNA sequence | 6.54 | Up | 3.49E-03 |
| AF21607 7 | Homo sapiens clone HB-2 mRNA sequence | 6.5 | Up | 4.90E-05 |
| AA04325 5 | zk49f07.s1 Soares_pregnant_uterus_NbHPU Homo sapiens cDNA clone IMAGE:486181 3, mRNA sequence | 6.49 | Up | 5.44E-04 |
| NM_1528 | Homo sapiens chromosome 20 open reading frame 58 (C20orf58), mRNA | 6.44 | Up | 2.32E-03 |
| 64 | | | | |
| NM_0328 66 | Homo sapiens cingulin-like 1 (CGNL1), mRNA | 6.4 | Up | 3.69E-03 |
| AK05499 0 | Homo sapiens cDNA FLJ30428 fis, clone BRACE2008941 | 6.37 | Up | 3.89E-05 |
| NM_1389 61 | Homo sapiens endothelial cell adhesion molecule (ESAM), mRNA | 6.35 | Up | 2.70E-04 |
| AI244954 | qj93h05.x1 NCI_CGAP_Kid3 Homo sapiens cDNA clone IMAGE:1867065 3, mRNA sequence | 6.27 | Up | 3.90E-05 |
| NM_0198 50 | Homo sapiens neuronal guanine nucleotide exchange factor (NGEF), mRNA | 6.25 | Up | 4.99E-04 |
| NM_0073 61 | Homo sapiens nidogen 2 (osteonidogen) (NID2), mRNA | 6.15 | Up | 7.03E-03 |
| NM_0001 70 | Homo sapiens glycine dehydrogenase (decarboxylating; glycine decarboxylase, glycine cleavage system protein P) (GLDC), mRNA | 6.13 | Up | 2.12E-05 |
| BG57203 9 | 602592506F1 NIH_MGC_79 Homo sapiens cDNA clone IMAGE:4719888 5, mRNA sequence | 6.09 | Up | 1.13E-05 |
| NM_0057 95 | Homo sapiens calcitonin receptor-like (CALCRL), mRNA | 6.03 | Up | 3.13E-02 |
| BQ26780 | ij94e04.x1 Human insulinoma Homo sapiens cDNA clone IMAGE:5779278 3, | 6.02 | Up | 6.53E-04 |
| 6 | mRNA sequence | | | |
| BC03622 3 | Homo sapiens, clone IMAGE:5272183, mRNA | 6 | Up | 3.15E-04 |
| NM_004 70 | Homo sapiens collagen, type XII, alpha 1 3 (COL12A1), transcript variant long, mRNA | 5.96 | Up | 1.14E-02 |
| NM_0053 60 | Homo sapiens v-maf musculoaponeurotic fibrosarcoma oncogene homolog (avian) (MAF), mRNA | 5.95 | Up | 2.41E-03 |
| AB03304 8 | Homo sapiens mRNA for KIAA1222 protein, partial cds | 5.94 | Up | 3.49E-04 |
| NM_017 86 | Homo sapiens hypothetical protein 7 FLJ20366 (FLJ20366), mRNA | 5.93 | Up | 2.24E-04 |
| AW5151 14 | xu91g11.x1 NCI_CGAP_Ut2 Homo sapiens cDNA clone IMAGE:2809124 3, mRNA sequence | 5.92 | Up | 1.11E-04 |
| AK09854 3 | Homo sapiens cDNA FLJ25677 fis, clone TST04054 | 5.84 | Up | 4.99E-03 |
| NM_004 35 | Homo sapiens Kruppel-like factor 4 (gut) 2 (KLF4), mRNA | 5.83 | Up | 6.54E-04 |
| AI335277 | tb29h06.x1 NCI_CGAP_Kid12 Homo sapiens cDNA clone IMAGE:2055803 3, mRNA sequence | 5.81 | Up | 1.24E-04 |
| AK09500 4 | Homo sapiens cDNA FLJ37685 fis, clone BRHIP2013972 | 5.76 | Up | 3.79E-03 |
| BQ00715 6 | UI-H-EI1-azc-k-08-0-Ul.s1 NCI_CGAP_EI1 Homo sapiens cDNA clone IMAGE:5846911 3, mRNA sequence | 5.68 | Up | 1.24E-04 |
| NM_0168 24 | Homo sapiens adducin 3 (gamma) (ADD3), transcript variant 1, mRNA | 5.68 | Up | 6.32E-04 |
| AA99433 0 | ou33h05.s1 Soares_NFL_T_GBC_S1 Homo sapiens cDNA clone IMAGE:1628121 3, mRNA sequence | 5.67 | Up | 9.57E-05 |
| NM_0169 46 | Homo sapiens F11 receptor (F11 R), transcript variant 1, mRNA | 5.65 | Up | 7.59E-05 |
| NM_0053 08 | Homo sapiens G protein-coupled receptor kinase 5 (GRK5), mRNA | 5.63 | Up | 7.59E-05 |
| BG38932 8 | 602413981 F1 NIH_MGC_92 Homo sapiens cDNA clone IMAGE:4522269 5, mRNA sequence | 5.61 | Up | 1.31E-04 |
| CN37116 8 | 17000600077294 GRN_PREHEP Homo sapiens cDNA 5, mRNA sequence | 5.57 | Up | 1.94E-02 |
| AK02153 1 | Homo sapiens cDNA FLJ11469 fis, clone HEMBA1001658 | 5.56 | Up | 7.81E-03 |
| NM_0018 53 | Homo sapiens collagen, type IX, alpha 3 (COL9A3), mRNA | 5.56 | Up | 6.94E-05 |
| BC03792 9 | Homo sapiens cDNA clone IMAGE:5284659, partial cds | 5.56 | Up | 9.99E-04 |
| | oz40h01.s1 Soares_NhHMPu_S1 Homo | 5.54 | Up | 1.20E-03 |
| AI086279 | sapiens cDNA clone IMAGE:1677841 3, mRNA sequence | | | |
| BF43347 9 | 7q53g05.x1 NCI_CGAP_Lu24 Homo sapiens cDNA clone IMAGE:3702320 3 similar to TR:Q9Y7P8 Q9Y7P8 HYPOTHETICAL 11.7 KD PROTEIN. ;, mRNA sequence | 5.48 | Up | 1.23E-04 |
| AI024717 | ov68h06.x1 Soares_testis_NHT Homo sapiens cDNA clone IMAGE:1642523 3, mRNA sequence | 5.46 | Up | 1.59E-04 |
| NM_0330 82 | Homo sapiens cytokine induced protein 29 kDa (CIP29), mRNA | 5.45 | Up | 9.59E-04 |
| W56431 | zc57f06.r1 Soares_parathyroid_tumor_NbHPA Homo sapiens cDNA clone IMAGE:326435 5, mRNA sequence | 5.42 | Up | 3.76E-04 |
| NM_1445 87 | Homo sapiens chromosome 10 open reading frame 87 (C10orf87), mRNA | 5.41 | Up | 1.47E-04 |
| BX10148 9 | BX101489 Soares_NFL_T_GBC_S Homo sapiens cDNA clone IMAGp998O165825 ; IMAGE:2347623, mRNA sequence | 5.37 | Up | 1.71E-05 |
| NM_0140 59 | Homo sapiens response gene to complement 32 (RGC32), mRNA | 5.32 | Up | 1.48E-04 |
| AI939617 | tm62e06.x5 NCI_CGAP_Brn25 Homo sapiens cDNA clone IMAGE:2162722 3, mRNA sequence | 5.3 | Up | 1.84E-03 |
| NM_0049 51 | Homo sapiens Epstein-Barr virus induced gene 2 (lymphocyte-specific G protein-coupled receptor) (EBI2), mRNA | 5.3 | Up | 8.80E-04 |
| AK09505 3 | Homo sapiens cDNA FLJ37734 fis, clone BRHIP2020842 | 5.3 | Up | 2.53E-04 |
| NM_0244 20 | Homo sapiens phospholipase A2, group IVA (cytosolic, calcium-dependent) (PLA2G4A), mRNA | 5.3 | Up | 1.56E-02 |
| NM_0180 13 | Homo sapiens hypothetical protein FLJ10159 (FLJ10159), mRNA | 5.29 | Up | 2.12E-05 |
| BX10185 0 | BX101850 Soares infant brain 1NIB Homo sapiens cDNA clone IMAGp998M14139 ; IMAGE:26298, mRNA sequence | 5.25 | Up | 7.59E-05 |
| C18094 | C18094 Human placenta cDNA (TFujiwara) Homo sapiens cDNA clone GEN-557D07 5, mRNA sequence | 5.23 | Up | 1.00E-05 |
| BU63433 2 | UI-H-FL1-bgx-k-02-0-UI.s1 NCI_CGAP_FL Homo sapiens cDNA clone UI-H-FL1-bgx-k-02-0-UI 3, mRNA sequence | 5.22 | Up | 2.86E-04 |
| AI033863 | ow10e02.x1 Soares_parathyroid_tumor_NbHPA Homo sapiens cDNA clone IMAGE:1646426 3, mRNA sequence | 5.18 | Up | 4.89E-03 |
| BG57014 4 | 602591134F1 NIH_MGC_77 Homo sapiens cDNA clone IMAGE:4717761 5, mRNA sequence | 5.18 | Up | 4.36E-04 |
| NM_0184 27 | Homo sapiens RRN3 RNA polymerase I transcription factor homolog (yeast) (RRN3), mRNA | 5.17 | Up | 1.68E-03 |
| BX48620 | DKFZp686J07250_r1 686 (synonym: hlcc3) Homo sapiens cDNA clone | 5.14 | Up | 1.31E-03 |
| 8 | DKFZp686J07250 5, mRNA sequence | | | |
| NM_0149 36 | Homo sapiens ectonucleotide pyrophosphatase/phosphodiesterase 4 (putative function) (ENPP4), mRNA | 5.14 | Up | 3.43E-04 |
| AF08613 4 | Homo sapiens full length insert cDNA clone ZA88B06 | 5.14 | Up | 4.00E-04 |
| NM_0018 47 | Homo sapiens collagen, type IV, alpha 6 (COL4A6), transcript variant A, mRNA | 5.12 | Up | 1.03E-03 |
| AK09572 6 | Homo sapiens cDNA FLJ38407 fis, clone FEBRA2008859 | 5.1 | Up | 1.35E-04 |
| BQ01386 9 | UI-1-BC1p-alg-a-03-0-UI.s1 NCI_CGAP_PI3 Homo sapiens cDNA clone UI-1-BC1p-alg-a-03-0-UI 3, mRNA sequence | 5.09 | Up | 1.42E-05 |
| NM_2073 03 | Homo sapiens attractin-like 1 (ATRNL1), mRNA | 5.04 | Up | 4.36E-04 |
| CB04728 7 | NISC_gg01h01.y1 NCI_CGAP_Kid11 Homo sapiens cDNA clone IMAGE:3253464 5, mRNA sequence | 5.04 | Up | 1.16E-03 |
| NM_0024 23 | Homo sapiens matrix metalloproteinase 7 (matrilysin, uterine) (MMP7), mRNA | 5.02 | Up | 6.94E-05 |
| AW2626 83 | xq93c06.x1 NCI_CGAP_Brn53 Homo sapiens cDNA clone IMAGE:2758186 3, mRNA sequence | 5.02 | Up | 3.19E-05 |
| NM_0132 | Homo sapiens transgelin 3 (TAGLN3), transcript variant 1, mRNA | 5.01 | Up | 8.63E-04 |
| 59 | | | | |
| D52654 | HUM084D02B Clontech human fetal brain polyA+ mRNA (#6535) Homo sapiens cDNA clone GEN-084D02 5, mRNA sequence | 449.36 | Down | 9.61E-06 |
| NM_0064 75 | Homo sapiens periostin, osteoblast specific factor (POSTN), mRNA | 293.88 | Down | 9.16E-05 |
| NM_0064 39 | Homo sapiens mab-21-like 2 (C. elegans) (MAB21L2), mRNA | 199.11 | Down | 3.53E-05 |
| NM_0178 05 | Homo sapiens Ras interacting protein 1 (RASIP1 mRNA | 153.9 | Down | 9.55E-05 |
| NM_0061 69 | Homo sapiens nicotinamide N-methyltransferase (NNMT), mRNA | 91.55 | Down | 1.57E-05 |
| AB06749 9 | Homo sapiens mRNA for KIAA1912 protein, partial cds | 69.67 | Down | 1.13E-05 |
| NM_0070 84 | Homo sapiens SRY (sex determining region Y)-box 21 (SOX21), mRNA | 55.37 | Down | 8.65E-05 |
| NM_0320 26 | Homo sapiens TatD DNase domain containing 1 (TATDN1), mRNA | 52.52 | Down | 2.24E-04 |
| NM_0178 19 | Homo sapiens RNA (guanine-9-) methyltransferase domain containing 1 (RG9MTD1), mRNA | 47.78 | Down | 2.09E-04 |
| NM_0328 | Homo sapiens chromosome 20 open reading frame 100 (C20orf100), mRNA | 45.19 | Down | 8.58E-05 |
| 83 | | | | |
| BQ37571 9 | QV2-TN0173-021100-454-g06 TN0173 Homo sapiens cDNA, mRNA sequence | 44.03 | Down | 1.41E-04 |
| BC03731 6 | Homo sapiens, clone IMAGE:5259432, mRNA | 34.66 | Down | 2.12E-05 |
| BC04237 8 | Homo sapiens, clone IMAGE:5277693, mRNA | 32.65 | Down | 6.44E-04 |
| NM_0188 94 | Homo sapiens EGF-containing fibulin-like extracellular matrix protein 1 (EFEMP1), transcript variant 2, mRNA | 30.19 | Down | 2.74E-05 |
| NM_0330 50 | Homo sapiens succinate receptor 1 (SUCNR1), mRNA | 30.08 | Down | 1.95E-04 |
| NM_1526 94 | Homo sapiens zinc finger, CCHC domain containing 5 (ZCCHC5), mRNA | 29.85 | Down | 7.57E-05 |
| NM_0529 97 | Homo sapiens ankyrin repeat domain 30A (ANKRD30A), mRNA | 28.91 | Down | 5.75E-04 |
| AK02678 4 | Homo sapiens cDNA: FLJ23131 fis, clone LNG08502 | 27.95 | Down | 8.14E-05 |
| NM_0018 85 | Homo sapiens crystallin, alpha B (CRYAB), mRNA | 26.31 | Down | 5.48E-05 |
| NM_0056 02 | Homo sapiens claudin 11 (oligodendrocyte transmembrane protein) (CLDN11), mRNA | 23.71 | Down | 1.13E-05 |
| NM_0014 42 | Homo sapiens fatty acid binding protein 4, adipocyte (FABP4), mRNA | 23.48 | Down | 4.05E-04 |
| NM_0216 14 | Homo sapiens potassium intermediate/small conductance calcium-activated channel, subfamily N, member 2 (KCNN2), transcript variant 1, mRNA | 23.14 | Down | 9.61E-06 |
| AK12387 5 | Homo sapiens cDNA FLJ41881 fis, clone OCBBF2021833 | 22.73 | Down | 9.17E-05 |
| R44402 | yg37a01.s1 Soares infant brain 1NIB Homo sapiens cDNA clone IMAGE:34639 3 similar to contains MER35 repetitive element ;, mRNA sequence | 22.27 | Down | 2.38E-05 |
| AK09501 3 | Homo sapiens cDNA FLJ37694 fis, clone BRHIP2015224 | 20.95 | Down | 9.77E-04 |
| NM_0021 85 | Homo sapiens interleukin 7 receptor (IL7R), mRNA | 20.88 | Down | 4.62E-04 |
| NM_0162 12 | Homo sapiens TP53TG3 protein (TP53TG3), mRNA | 20.63 | Down | 6.56E-05 |
| NM_1527 37 | Homo sapiens hypothetical protein MGC33993 (MGC33993), mRNA | 19.89 | Down | 6.16E-06 |
| BF00248 9 | 7h07e07.x1 NCI_CGAP_Co16 Homo sapiens cDNA clone IMAGE:3315300 3, mRNA sequence | 19.88 | Down | 8.65E-05 |
| NM_0055 | Homo sapiens hydroxysteroid (11-beta) dehydrogenase 1 (HSD11B1), transcript | 18.27 | Down | 3.53E-05 |
| 25 | variant 1, mRNA | | | |
| AF31838 2 | Homo sapiens pp9974 mRNA, complete cds | 17.67 | Down | 3.89E-05 |
| BX11582 5 | BX115825 Soares_NhHMPu_S1 Homo sapiens cDNA clone IMAGp998C134603 ; IMAGE:1879236, mRNA sequence | 17.5 | Down | 1.67E-04 |
| NM_0006 12 | Homo sapiens insulin-like growth factor 2 (somatomedin A) (IGF2), mRNA | 16.41 | Down | 4.79E-05 |
| AK09211 4 | Homo sapiens cDNA FLJ34795 fis, clone NT2NE2005921 | 16.29 | Down | 2.18E-05 |
| AI469032 | ti70a01.x1 NCI_CGAP_Kid11 Homo sapiens cDNA clone IMAGE:2137320 3, mRNA sequence | 15.92 | Down | 9.36E-05 |
| NM_0011 75 | Homo sapiens Rho GDP dissociation inhibitor (GDI) beta (ARHGDIB), mRNA | 15.57 | Down | 4.62E-05 |
| AF05211 5 | Homo sapiens clone 23688 mRNA sequence | 15.11 | Down | 1.03E-04 |
| CD67733 2 | ho15f06.y1 Human Trabecular meshwork cDNA: hohphq Homo sapiens cDNA clone ho15f06 5, mRNA sequence | 14.89 | Down | 8.06E-05 |
| NM_0180 77 | Homo sapiens RNA binding motif protein 28 (RBM28), mRNA | 14.72 | Down | 9.00E-05 |
| NM_0007 35 | Homo sapiens glycoprotein hormones, alpha polypeptide (CGA), mRNA | 14.51 | Down | 2.62E-04 |
| N49730 | yz06a12.s1 | 13.53 | Down | 2.76E-05 |
| | Soares_multiple_sclerosis_2NbHMSP Homo sapiens cDNA clone IMAGE:282238 3 similar to contains Alu repetitive element;contains element PTR5 repetitive element ;, mRNA sequence | | | |
| NM_0063 50 | Homo sapiens follistatin (FST), transcript variant FST317, mRNA | 13.34 | Down | 2.53E-04 |
| AK05551 8 | Homo sapiens cDNA FLJ30956 fis, clone HCASM2000202 | 13.09 | Down | 2.63E-03 |
| NM_0161 47 | Homo sapiens protein phosphatase methylesterase-1 (PME-1), mRNA | 13.04 | Down | 1.84E-02 |
| NM_0324 57 | Homo sapiens BH-protocadherin (brain-heart) (PCDH7), transcript variant c, mRNA | 13.01 | Down | 7.57E-05 |
| BX64829 9 | Homo sapiens mRNA; cDNA DKFZp686J04125 (from clone DKFZp686J04125) | 12.94 | Down | 1.82E-04 |
| BC03431 5 | Homo sapiens hypothetical protein LOC90529, mRNA (cDNA clone IMAGE:4827425), containing frame-shift errors | 12.94 | Down | 2.72E-04 |
| AA19532 8 | zr34f08.s1 Soares_NhHMPu_S1 Homo sapiens cDNA clone IMAGE:665319 3, mRNA sequence | 12.73 | Down | 7.59E-04 |
| AI082507 | ox55c02.s1 Soares_total_fetus_Nb2HF8_9w Homo sapiens cDNA clone IMAGE:1660226 3, mRNA sequence | 12.67 | Down | 5.54E-05 |
| NM_0157 14 | Homo sapiens putative lymphocyte G0/G1 switch gene (G0S2), mRNA | 12.17 | Down | 7.45E-04 |
| BM99204 9 | UI-H-DF1-auf-e-22-0-UI.s1 NCI_CGAP_DF1 Homo sapiens cDNA clone IMAGE:5868669 3, mRNA sequence | 12.09 | Down | 8.65E-05 |
| NM_0164 28 | Homo sapiens ABI gene family, member 3 (ABI3), mRNA | 12.02 | Down | 3.62E-04 |
| BQ02798 4 | UI-H-CO0-arg-e-03-0-UI.s1 NCI_CGAP_Sub9 Homo sapiens cDNA clone IMAGE:3106611 3, mRNA sequence | 11.97 | Down | 7.10E-05 |
| M_1527 82 | Homo sapiens Sad1 and UNC84 domain containing 1 (SUNC1 mRNA | 11.76 | Down | 6.37E-04 |
| CD72379 8 | oj26f04.y1 Human lacrimal gland, unamplified: oj Homo sapiens cDNA clone oj26f04 5, mRNA sequence | 11.66 | Down | 1.59E-04 |
| NM_0331 36 | Homo sapiens fibroblast growth factor 1 (acidic) (FGF1), transcript variant 2, mRNA | 11.19 | Down | 5.11E-03 |
| BU68826 3 | UI-CF-EC1-aea-g-11-0-UI.s1 UI-CF-EC1 Homo sapiens cDNA clone UI-CF-EC1-aea-g-11-0-UI 3, mRNA sequence | 11.07 | Down | 2.89E-04 |
| NM_0143 91 | Homo sapiens ankyrin repeat domain 1 (cardiac muscle) (ANKRD1), mRNA | 11.05 | Down | 9.03E-03 |
| NM_0239 15 | Homo sapiens G protein-coupled receptor 87 (GPR87), mRNA | 10.71 | Down | 4.91E-05 |
| | Homo sapiens programmed cell death 1 | 10.6 | Down | 1.03E-03 |
| NM_0252 39 | ligand 2 (PDCD1 LG2), mRNA | | | |
| NM_0033 92 | Homo sapiens wingless-type MMTV integration site family, member 5A (WNT5A), mRNA | 10.52 | Down | 1.11E-02 |
| NM_0308 99 | Homo sapiens zinc finger protein 323 (ZNF323), mRNA | 10.28 | Down | 1.90E-03 |
| NM_0015 54 | Homo sapiens cysteine-rich, angiogenic inducer, 61 (CYR61), mRNA | 10.27 | Down | 5.12E-03 |
| AB00233 3 | Human mRNA for KIAA0335 gene, partial cds | 10.22 | Down | 4.90E-05 |
| NM_0052 33 | Homo sapiens EPH receptor A3 (EPHA3), transcript variant 1, mRNA | 10.14 | Down | 2.91E-03 |
| NM_0246 00 | Homo sapiens chromosome 16 open reading frame 30 (C16orf30), mRNA | 10.11 | Down | 3.10E-03 |
| NM_0326 38 | Homo sapiens GATA binding protein 2 (GATA2), mRNA | 9.99 | Down | 2.21E-04 |
| U79271 | Human clones 23920 and 23921 mRNA sequence | 9.88 | Down | 5.41E-05 |
| NM_0174 48 | Homo sapiens lactate dehydrogenase C (LDHC), transcript variant 2, mRNA | 9.74 | Down | 2.07E-04 |
| NM_0012 99 | Homo sapiens calponin 1, basic, smooth muscle (CNN1), mRNA | 9.63 | Down | 3.67E-04 |
| AI939462 | tf23h06.x5 NCI_CGAP_Brn23 Homo sapiens cDNA clone IMAGE:2097083 3, mRNA sequence | 9.62 | Down | 8.51E-05 |
| NM_178E 34 | Homo sapiens layilin (LOC143903), mRNA | 9.59 | Down | 1.13E-05 |
| AF26908 8 | Homo sapiens breast cancer antigen NY-BR-1.1 mRNA, partial cds | 9.42 | Down | 2.10E-03 |
| NM_0019 45 | Homo sapiens diphtheria toxin receptor (heparin-binding epidermal growth factor-like growth factor) (DTR), mRNA | 9.37 | Down | 2.21E-02 |
| NM_0047 91 | Homo sapiens integrin, beta-like 1 (with EGF-like repeat domains) (ITGBL1), mRNA | 9.24 | Down | 2.86E-04 |
| NM_0006 82 | Homo sapiens adrenergic, alpha-2B-, receptor (ADRA2B), mRNA | 9.19 | Down | 9.68E-04 |
| NM_0122 42 | Homo sapiens dickkopf homolog 1 (Xenopus laevis) (DKK1), mRNA | 9.18 | Down | 2.05E-03 |
| NM_0019 98 | Homo sapiens fibulin 2 (FBLN2), transcript variant 2, mRNA | 9.07 | Down | 5.02E-04 |
| BG16574 5 | 602344592F1 NIH_MGC_89 Homo sapiens cDNA clone IMAGE:4454470 5, mRNA sequence | 9.04 | Down | 1.30E-03 |
| D29453 | HUMNK566 Human epidermal keratinocyte Homo sapiens cDNA clone 566, mRNA sequence | 8.93 | Down | 2.86E-04 |
| | oj16h02.y1 Human lacrimal gland, | 8.93 | Down | 9.36E-05 |
| CD72300 6 | unamplified: oj Homo sapiens cDNA clone oj16h02 5, mRNA sequence | | | |
| NM_1785 50 | Homo sapiens hypothetical protein MGC48998 (MGC48998), mRNA | 8.87 | Down | 3.76E-04 |
| NM_0162 01 | Homo sapiens angiomotin like 2 (AMOTL2), mRNA | 8.62 | Down | 2.48E-02 |
| AW2917 75 | UI-H-BI2-agv-h-04-0-UI.s1 NCI_CGAP_Sub4 Homo sapiens cDNA clone IMAGE:2725855 3, mRNA sequence | 8.5 | Down | 3.03E-03 |
| NM_1527 03 | Homo sapiens chromosome 7 open reading frame 6 (C7orf6), mRNA | 8.38 | Down | 1.58E-04 |
| NM_0018 43 | Homo sapiens contactin 1 (CNTN1), transcript variant 1, mRNA | 8.32 | Down | 5.41E-05 |
| AL11745 4 | Homo sapiens mRNA; cDNA DKFZp586J1717 (from clone DKFZp586J1717) | 8.26 | Down | 5.79E-05 |
| BX09200 4 | BX092004NCI_CGAP_Kid11 Homo sapiens cDNA clone IMAGp998B195924 ; IMAGE:2385330, mRNA sequence | 8.18 | Down | 4.67E-04 |
| NM_1452 01 | Homo sapiens similar to CG3714 gene product (PP3856), mRNA | 8.16 | Down | 1.64E-03 |
| BX53769 8 | Homo sapiens mRNA; cDNA DKFZp686F09166 (from clone DKFZp686F09166) | 8.15 | Down | 7.27E-04 |
| NM_2034 | Homo sapiens Down syndrome critical region gene 1 (DSCR1), transcript variant | 8.12 | Down | 2.96E-02 |
| 18 | 3, mRNA | | | |
| NM_0244 23 | Homo sapiens desmocollin 3 (DSC3), transcript variant Dsc3b, mRNA | 7.93 | Down | 2.54E-03 |
| AK09352 9 | Homo sapiens cDNA FLJ36210 fis, clone THYMU2000155 | 7.9 | Down | 9.50E-04 |
| AK13153 2 | Homo sapiens cDNA FLJ16761 fis, clone BRAMY3008096 | 7.89 | Down | 1.84E-03 |
| NM_0325 11 | Homo sapiens chromosome 6 open reading frame 168 (C6orf168), mRNA | 7.42 | Down | 3.49E-03 |
| NM_0025 21 | Homo sapiens natriuretic peptide precursor B (NPPB), mRNA | 7.4 | Down | 4.45E-03 |
| BU62632 6 | UI-H-DF0-bet-p-17-0-UI.s1 NCI_CGAP_DF0 Homo sapiens cDNA clone UI-H-DF0-bet-p-17-0-UI 3, mRNA sequence | 7.34 | Down | 7.03E-05 |
| NM_0011 44 | Homo sapiens autocrine motility factor receptor (AMFR), transcript variant 1, mRNA | 7.33 | Down | 1.03E-03 |
| NM_0015 41 | Homo sapiens heat shock 27kDa protein 2 (HSPB2), mRNA | 7.32 | Down | 3.96E-03 |
| NM_0050 69 | Homo sapiens single-minded homolog 2 (Drosophila) (SIM2), transcript variant SIM2, mRNA | 7.31 | Down | 1.23E-03 |
| AI937359 | wp76c02.x1 NCI_CGAP_Brn25 Homo sapiens cDNA clone IMAGE:2467682 3, | 7.31 | Down | 1.96E-03 |
| | mRNA sequence | | | |
| NM_0153 45 | Homo sapiens dishevelled associated activator of morphogenesis 2 (DAAM2), mRNA | 7.29 | Down | 2.18E-05 |
| NM_0000 89 | Homo sapiens collagen, type I, alpha 2 (COL1A2), mRNA | 7.25 | Down | 6.07E-03 |
| NM_0151 50 | Homo sapiens raft-linking protein (RAFTLIN), mRNA | 7.24 | Down | 2.87E-03 |
| BX11331 9 | BX113319 NCI_CGAP_Gas4 Homo sapiens cDNA clone IMAGp998G205398 ; IMAGE:2184619, mRNA sequence | 7.24 | Down | 1.28E-03 |
| M_1993 29 | Homo sapiens solute carrier family 43, member 3 (SLC43A3), mRNA | 7.19 | Down | 2.31E-03 |
| AV70297 7 | AV702977 ADB Homo sapiens cDNA clone ADBCVD08 5, mRNA sequence | 7.11 | Down | 5.21E-05 |
| NM_0245 12 | Homo sapiens leucine rich repeat containing 2 (LRRC2), mRNA | 7.09 | Down | 2.65E-04 |
| NM_0176 55 | Homo sapiens PDZ domain protein GIPC2 (GIPC2), mRNA | 7.06 | Down | 2.04E-04 |
| NM_0144 77 | Homo sapiens chromosome 20 open reading frame 10 (C20orf10), mRNA | 7.05 | Down | 4.10E-03 |
| NM_0025 59 | Homo sapiens purinergic receptor P2X, ligand-gated ion channel, 3 (P2RX3), mRNA | 7.03 | Down | 4.63E-03 |
| BM97638 5 | UI-CF-EN1-acz-f-03-0-UI.s1 UI-CF-EN1 Homo sapiens cDNA clone UI-CF-EN1-acz-f-03-0-UI 3, mRNA sequence | 7.01 | Down | 9.77E-04 |
| BX64832 3 | Homo sapiens mRNA; cDNA DKFZp686K10163 (from clone DKFZp686K10163) | 6.94 | Down | 8.05E-04 |
| BC00858 0 | Homo sapiens, clone IMAGE:4179986, mRNA, partial cds | 6.93 | Down | 5.41E-05 |
| NM_0070 34 | Homo sapiens DnaJ (Hsp40) homolog, subfamily B, member 4 (DNAJB4), mRNA | 6.72 | Down | 2.98E-02 |
| AW0255 56 | wu97g10.x1 NCI_CGAP_Kid3 Homo sapiens cDNA clone IMAGE:2528034 3, mRNA sequence | 6.7 | Down | 2.31E-03 |
| NM_0035 14 | Homo sapiens histone 1, H2am (HIST1H2AM), mRNA | 6.7 | Down | 9.11E-03 |
| NM_0326 03 | Homo sapiens lysyl oxidase-like 3 (LOXL3), mRNA | 6.65 | Down | 2.58E-03 |
| AW1726 52 | xj80f06.x1 Soares_NFL_T_GBC_S1 Homo sapiens cDNA clone IMAGE:2663555 3, mRNA sequence | 6.62 | Down | 2.17E-04 |
| NM_0063 50 | Homo sapiens follistatin (FST), transcript variant FST317, mRNA | 6.57 | Down | 7.59E-05 |
| BQ01858 6 | UI-H-DH1-awu-c-08-0-UI.s1 NCI_CGAP_DH1 Homo sapiens cDNA clone IMAGE:5823679 3, mRNA sequence | 6.43 | Down | 3.44E-03 |
| | nae30h12.x1 Lupski_sympathetic_trunk | 6.43 | Down | 8.65E-05 |
| BF44815 8 | Homo sapiens cDNA clone IMAGE:4090607 3, mRNA sequence | | | |
| NM_0015 57 | Homo sapiens interleukin 8 receptor, beta (IL8RB), mRNA | 6.38 | Down | 2.96E-03 |
| NM_0021 66 | Homo sapiens inhibitor of DNA binding 2, dominant negative helix-loop-helix protein (ID2), mRNA | 6.37 | Down | 2.10E-03 |
| NM_0806 71 | Homo sapiens potassium voltage-gated channel, Isk-related family, member 4 (KCNE4), mRNA | 6.36 | Down | 1.17E-02 |
| BM71294 5 | UI-E-EJ0-ahi-c-16-0-UI.r1 UI-E-EJ0 Homo sapiens cDNA clone UI-E-EJ0-ahi-c-16-0-UI 5, mRNA sequence | 6.33 | Down | 3.62E-04 |
| BM99189 0 | UI-H-DF1-auk-h-02-0-UI.s1 NCI_CGAP_DF1 Homo sapiens cDNA clone IMAGE:5870641 3, mRNA sequence | 6.32 | Down | 8.14E-05 |
| NM_0178 23 | Homo sapiens dual specificity phosphatase 23 (DUSP23), mRNA | 6.26 | Down | 9.36E-03 |
| NM_0324 61 | Homo sapiens SPANX family, member B1 (SPANXB1), mRNA | 6.26 | Down | 5.62E-03 |
| NM_00601 27 | Homo sapiens exonuclease 1 (EXO1), transcript variant 1, mRNA | 6.26 | Down | 7.81E-03 |
| NM_0035 45 | Homo sapiens histone 1, H4e (HIST1 H4E), mRNA | 6.19 | Down | 7.48E-03 |
| NM_0014 | Homo sapiens forkhead box F1 (FOXF1), mRNA | 6.17 | Down | 1.02E-03 |
| 51 | | | | |
| NM_001 55 | Homo sapiens endothelin 1 (EDN1), mRNA 9 | 6.16 | Down | 2.54E-03 |
| NM_003 17 | Homo sapiens regulator of G-protein 6 signalling 5 (RGS5), mRNA | 6.07 | Down | 9.24E-03 |
| NM_0211 03 | Homo sapiens thymosin, beta 10 (TMSB10), mRNA | 6.06 | Down | 2.32E-02 |
| NM_0014 24 | Homo sapiens epithelial membrane protein 2 (EMP2), mRNA | 6.06 | Down | 2.23E-03 |
| BG35457 9 | CDCA6 Cell division cycle associated gene 6 Human cDNA expression libraries Homo sapiens cDNA clone 407614, mRNA sequence | 5.96 | Down | 1.40E-03 |
| BM66545 2 | UI-E-CQ1-aex-n-03-0-UI.s1 UI-E-CQ1 Homo sapiens cDNA clone UI-E-CQ1-aex-n-03-0-UI 3, mRNA sequence | 5.94 | Down | 2.64E-04 |
| NM_0794 23 | Homo sapiens myosin, light polypeptide 6, alkali, smooth muscle and non-muscle (MYL6), transcript variant 2, mRNA | 5.87 | Down | 3.58E-02 |
| AW1344 73 | UI-H-BI1-abv-a-11-0-UI.s1 NCI_CGAP_Sub3 Homo sapiens cDNA clone IMAGE:2712885 3, mRNA sequence | 5.85 | Down | 1.03E-03 |
| NM_0323 34 | Homo sapiens hypothetical protein MGC14595 (MGC14595), mRNA | 5.79 | Down | 9.11E-03 |
| BF02935 | 601765592F1 NIH_MGC_53 Homo sapiens cDNA clone IMAGE:3997510 5, mRNA | 5.78 | Down | 3.55E-03 |
| 6 | sequence | | | |
| NM_1758 87 | Homo sapiens hypothetical protein LOC222171 (LOC222171), mRNA | 5.72 | Down | 3.51E-04 |
| NM_1392 41 | Homo sapiens FYVE, RhoGEF and PH domain containing 4 (FGD4), mRNA | 5.72 | Down | 3.12E-03 |
| NM_0159 15 | Homo sapiens spastic paraplegia 3A (autosomal dominant) (SPG3A), mRNA | 5.7 | Down | 2.15E-03 |
| NM_0529 37 | Homo sapiens similar to hypothetical protein FLJ10883 (LOC115294), mRNA | 5.69 | Down | 7.86E-03 |
| NM_0152 51 | Homo sapiens KIAA0431 protein (KIAA0431), mRNA | 5.6 | Down | 3.87E-04 |
| NM_0219 56 | Homo sapiens glutamate receptor, ionotropic, kainate 2 (GRIK2), transcript variant 1, mRNA | 5.58 | Down | 2.31E-03 |
| NM_0175 27 | Homo sapiens lymphocyte antigen 6 complex, locus K (LY6K), mRNA | 5.57 | Down | 1.69E-02 |
| NM_0151 92 | Homo sapiens phospholipase C, beta 1 (phosphoinositide-specific) (PLCB1), transcript variant 1, mRNA | 5.53 | Down | 7.81E-03 |
| NM_0202 23 | Homo sapiens family with sequence similarity 20, member C (FAM20C), mRNA | 5.51 | Down | 1.19E-03 |
| NM_0026 19 | Homo sapiens platelet factor 4 (chemokine (C-X-C motif) ligand 4) (PF4), mRNA | 5.51 | Down | 3.31E-04 |
| NM_0020 53 | Homo sapiens guanylate binding protein 1, interferon-inducible, 67kDa (GBP1), mRNA | 5.51 | Down | 1.27E-03 |
| H55853 | yq94c05.r1 Soares fetal liver spleen 1 NFLS Homo sapiens cDNA clone IMAGE:203432 5, mRNA sequence | 5.5 | Down | 6.78E-03 |
| NM_0009 47 | Homo sapiens primase, polypeptide 2A, 58kDa (PRIM2A), mRNA | 5.48 | Down | 8.13E-03 |
| NM_0055 96 | Homo sapiens nuclear factor I/B (NFIB), mRNA | 5.46 | Down | 1.00E-04 |
| NM_0142 85 | Homo sapiens exosome component 2 (EXOSC2), mRNA | 5.45 | Down | 1.45E-02 |
| NM_0046 23 | Homo sapiens tetratricopeptide repeat domain 4 (TTC4), mRNA | 5.43 | Down | 1.84E-02 |
| NM_0528 46 | Homo sapiens elastin microfibril interfacer 3 (EMILIN3), mRNA | 5.43 | Down | 1.11 E-04 |
| AW1523 68 | xg63e03.x1 NCI_CGAP_Ut4 Homo sapiens cDNA clone IMAGE:2633020 3 similar to contains Alu repetitive element;, mRNA sequence | 5.41 | Down | 5.48E-04 |
| NM_0004 75 | Homo sapiens nuclear receptor subfamily 0, group B, member 1 (NR0B1 mRNA | 5.39 | Down | 1.85E-03 |
| NM_0123 94 | Homo sapiens prefoldin 2 (PFDN2), mRNA | 5.38 | Down | 3.93E-02 |
| NM_0190 18 | Homo sapiens hypothetical protein FLJ11127 (FLJ11127), mRNA | 5.32 | Down | 1.05E-02 |
| NM_0042 98 | Homo sapiens nucleoporin 155kDa (NUP155), transcript variant 2, mRNA | 5.3 | Down | 5.08E-02 |
| M_0035 16 | Homo sapiens histone 2, H2aa (HIST2H2AA), mRNA | 5.3 | Down | 3.90E-02 |
| NM_0043 24 | Homo sapiens BCL2-associated X protein (BAX), transcript variant beta, mRNA | 5.27 | Down | 2.42E-02 |
| NM_0060 12 | Homo sapiens CIpP caseinolytic protease, ATP-dependent, proteolytic subunit homolog (E. coli) (CLPP), nuclear gene encoding mitochondrial protein, mRNA | 5.22 | Down | 3.42E-03 |
| NM 0219 68 | Homo sapiens histone 1, H4j (HIST1H4J), mRNA | 5.19 | Down | 1.84E-02 |
| BF11171 0 | 7147c10.x1 Soares_NSF_F8_9W_OT_PA_P_S1 Homo sapiens cDNA clone IMAGE:3524371 3, mRNA sequence | 5.19 | Down | 1.23E-03 |
| NM_0068 37 | Homo sapiens COP9 constitutive photomorphogenic homolog subunit 5 (Arabidopsis) (COPS5), mRNA | 5.16 | Down | 3.83E-02 |
| NM_0018 84 | Homo sapiens hyaluronan and proteoglycan link protein 1 (HAPLN1), mRNA | 5.15 | Down | 6.63E-03 |
| NM_0186 | Homo sapiens peroxisomal membrane protein 2, 22kDa (PXMP2), mRNA | 5.14 | Down | 1.79E-02 |
| 63 | | | | |
| BX09332 9 | BX093329 Soares_parathyroid_tumor_NbHPA Homo sapiens cDNA clone IMAGp998A124183 ; IMAGE:1648403, mRNA sequence | 5.14 | Down | 9.07E-03 |
| NM_0010 01992 | Homo sapiens ubiquitin specific protease 16 (USP16), transcript variant 2, mRNA | 5.13 | Down | 1.18E-02 |
| NM_0034 05 | Homo sapiens tyrosine 3-monooxygenase/tryptophan 5-monooxygenase activation protein, eta polypeptide (YWHAH), mRNA | 5.11 | Down | 1.09E-02 |
| NM_0032 38 | Homo sapiens transforming growth factor, beta 2 (TGFB2), mRNA | 5.09 | Down | 2.36E-03 |
| T95596 | ye40b03.s1 Soares fetal liver spleen 1 NFLS Homo sapiens cDNA clone IMAGE:120173 3, mRNA sequence | 5.08 | Down | 3.51E-04 |
| NM_0047 53 | Homo sapiens dehydrogenase/reductase (SDR family) member 3 (DHRS3), mRNA | 5.07 | Down | 6.56E-03 |
| NM_0001 65 | Homo sapiens gap junction protein, alpha 1, 43kDa (connexin 43) (GJA1), mRNA | 5.04 | Down | 2.07E-03 |
| H99504 | yx25g05.s1 Soares melanocyte 2NbHM Homo sapiens cDNA clone IMAGE:262808 3, mRNA sequence | 5.04 | Down | 1.13E-03 |
| NM_0124 64 | Homo sapiens tolloid-like 1 (TLL1), mRNA | 5.03 | Down | 3.47E-02 |
| | Homo sapiens heart alpha-kinase (HAK), | 5.02 | Down | 1.06E-04 |
| NM_0529 47 | mRNA | | | |

**TABLE VIII: REAL TIME PCR DATA FOR DIFFERENTIATED AF-I CELLS**

| Hormone | Ct values for AFCA007 Clone A cultured for 2 wks in Amniomax | Ct values for AFCA007 Clone A cultured for 2 wks in Amniomax + 10 micro molar retinoic acid | ΔΔCₜ for AFCA007 Clone A cultured for 2 wks in Amniomax | ΔΔCₜ for AFCA007 Clone A cultured for 2 wks in Amniomax + 10 micro molar retinoic acid |
|---|---|---|---|---|
| GIP | 34 | 31 | 0.00062 | 0.004 |
| Neurotensin | 37 | 35 | 0.000183 | 0.0004 |
| Gastrin | No expression | 36 | | 0.0004 |
| Secretin | 32 | 32 | 0.02 | 0.014 |
| Somatostatin | 31 | 28 | 0.002 | 0.014 |
| CCK | 33 | 31 | 0.003 | 0.008 |
| GAPDH | 22 | 22 | | |

| | | | | |
|---|---|---|---|---|
| *: For ΔΔCₜ analysis, the positive control was human intestinal RNA | | | | |

**TABLE IX: PCR ANALYSIS OF THE EXPRESSION OF ENDODERM MARKERS IN AFCA007 (P8) AFTER 14-DAYS OF DIFFERENTIATION ASSAY**

| Treatment | HNF3β | Pdx-1 | GATA4 | Glu-2 | HNF1α | AFP |
|---|---|---|---|---|---|---|
| Untreated | - | - | - | + | - | - |
| FGF4 50 ng/ml | + | + | + | + | + | |
| All-trans RA (10 µM) | + | - | + | + | + | + |
| L-685,458 (10 µM) | + | | + | + | + | |
| Cyclopamine (10 µM) | | | + | + | | |
| BMP5 (5 ng/ml) | + | | + | + | + | |
| BMP6 (50 ng/ml) | + | | + | + | + | |
| Excendin 4 (10 nM) | + | | + | + | + | |
| BMP7 (10 ng/ml) | | | + | + | + | |
| BMP4 (50 ng/ml) | + | | | + | + | |

**TABLE X: CYTOKINE, CYTOKINE AND GROWTH FACTOR RECEPTOR EXPRESSION LEVEL FOR AFCA007 A (AF-I) AND AFCA015 C (AF-II) CELLS**

| | **AFCA007 A** | **AFCA007 A** | **AFCA015 C** | **AFCA015 C** |
|---|---|---|---|---|
| **POS** | 23,029.50 | 22,843.43 | 23,281.28 | 23,086.51 |
| **NEG** | 1.00 | 0.98 | 0.86 | 0.86 |
| **Angiogenin** | 44.50 | 89.98 | 101.17 | 26.71 |
| **BDNF** | 702.50 | 670.09 | 590.28 | 411.52 |
| **BLC** | 276.50 | 365.08 | 192.90 | 0.86 |
| **BMP-4** | 66.00 | 96.36 | 126.89 | 117.22 |
| **BMP-6** | 135.00 | 182.17 | 247.34 | 133.53 |
| **CK beta 8-1** | 139.50 | 100.28 | 80.16 | 43.87 |
| **CNTF** | 235.50 | 234.64 | 82.73 | 0.86 |
| **EGF** | 125.50 | 106.66 | 66.01 | 0.86 |
| **Eotaxin** | 90.00 | 95.87 | 152.61 | 99.21 |
| **Eotaxin**-**2** | 635.00 | 554.86 | 528.55 | 290.54 |
| **Eotaxin-3** | 174.50 | 245.43 | 246.48 | 170.42 |
| **FGF-6** | 422.00 | 411.67 | 318.07 | 213.32 |
| **FGF-7** | 378.50 | 348.41 | 225.05 | 15.12 |
| **Flt-3 Ligand** | 298.00 | 264.56 | 146.18 | 66.60 |
| **Fractalkine** | 545.50 | 399.41 | 330.50 | 105.21 |
| **GCP**-**2** | 323.00 | 266.52 | 182.18 | 0.86 |
| **GDNF** | 249.00 | 213.56 | 100.74 | 15.55 |
| **GM-CSF** | 1,003.50 | 687.75 | 663.15 | 343.31 |
| **I-309** | 108.00 | 90.47 | 91.74 | 36.57 |
| **IFN-gamma** | 227.50 | 189.04 | 217.34 | 7.40 |
| **IGFBP-1** | 156.00 | 197.37 | 182.61 | 37.00 |
| **IGFBP-2** | 627.00 | 415.10 | 383.66 | 281.53 |
| **IGFBP-4** | 154.00 | 136.57 | 103.74 | 0.86 |
| **IGF-I** | 121.00 | 200.32 | 218.62 | 191.01 |
| **IL-10** | 474.50 | 379.30 | 320.22 | 64.89 |
| **IL**-**13** | 768.00 | 627.43 | 636.57 | 184.58 |
| **IL**-**15** | 829.50 | 542.60 | 558.56 | 226.62 |
| **IL-16** | 243.50 | 235.62 | 146.18 | 2.25 |
| **IL**-**1alpha** | 1,206.00 | 1,058.47 | 1,048.95 | 662.05 |
| **IL**-**1beta** | 349.00 | 282.21 | 258.49 | 64.89 |
| **IL-1ra** | 278.50 | 337.13 | 225.05 | 111.65 |
| **IL-2** | 684.50 | 581.34 | 489.97 | 254.50 |
| **IL-3** | 1,052.50 | 1,066.31 | 1,075.53 | 904.86 |
| **IL-4** | 396.00 | 404.80 | 383.23 | 224.47 |
| **IL-5** | 763.00 | 676.96 | 754.89 | 303.41 |
| **IL-6** | 1,172.00 | 1,113.88 | 485.25 | 327.43 |
| **IL-7** | 675.00 | 567.61 | 473.68 | 241.63 |
| **Leptin** | 269.50 | 267.99 | 229.77 | 118.08 |
| **LIGHT** | 295.50 | 254.75 | 214.33 | 85.48 |
| **MCP-1** | 1,027.00 | 901.06 | 1,080.25 | 990.24 |
| **MCP**-**2** | 546.00 | 461.20 | 389.66 | 120.66 |
| **MCP-3** | 378.50 | 372.93 | 288.49 | 85.91 |
| **MCP-4** | 642.50 | 532.30 | 390.95 | 159.69 |
| **M-CSF** | 692.00 | 566.63 | 504.11 | 267.37 |
| **MDC** | 446.50 | 442.07 | 312.07 | 138.24 |
| **MIG** | 760.00 | 725.51 | 701.30 | 363.47 |
| **MIP**-**1**-**delta** | 290.50 | 347.92 | 295.78 | 117.65 |
| **MIP**-**3**-**alpha** | 406.50 | 399.90 | 311.64 | 64.46 |
| **NAP**-**2** | 216.50 | 241.51 | 208.33 | 44.29 |
| **NT-3** | 45.50 | 124.31 | 61.30 | 0.86 |
| **PARC** | 181.50 | 208.16 | 138.46 | 0.86 |
| **PDGF-BB** | 594.00 | 313.10 | 126.03 | 152.83 |
| **RANTES** | 418.50 | 350.86 | 248.63 | 108.21 |
| **SCF** | 307.50 | 152.26 | 84.45 | 45.58 |
| **SDF-1** | 654.50 | 594.09 | 237.48 | 116.37 |
| **TARC** | 227.50 | 264.56 | 148.75 | 2.25 |
| **TGF-beta 1** | 824.50 | 750.02 | 693.59 | 407.23 |
| **TGF-beta 3** | 278.00 | 341.54 | 277.35 | 57.16 |
| **TNF**-**alpha** | 804.50 | 968.73 | 992.37 | 552.23 |
| **TNF**-**beta** | 549.50 | 510.23 | 888.20 | 285.82 |
| **POS** | 24,267.79 | 23,873.40 | 25,002.74 | 25,119.43 |
| **NEG** | 1.00 | 0.89 | 0.78 | 0.78 |
| **Acrp30** | 29.13 | 0.89 | 100.74 | 98.12 |
| **AgRP** | 1.00 | 0.89 | 19.30 | 0.78 |
| **Angiopoietin**-**2** | 144.13 | 84.27 | 62.74 | 58.01 |
| **Amphiregulin** | 1.00 | 0.89 | 0.78 | 0.78 |
| **Axl** | 1,478.63 | 882.74 | 542.12 | 536.93 |
| **bFGF** | 5,455.13 | 4,386.44 | 4,419.49 | 7,225.00 |
| **b-NGF** | 94.13 | 8.27 | 69.72 | 58.79 |
| **BTC** | 1.00 | 0.89 | 0.78 | 0.78 |
| **CCL-28** | 141.13 | 76.23 | 0.78 | 0.78 |
| **CTACK** | 383.13 | 315.86 | 304.37 | 297.86 |
| **Dtk** | 90.13 | 51.19 | 48.77 | 48.67 |
| **EGF-R** | 1,730.13 | 851.89 | 1,363.21 | 1,485.42 |
| **ENA-78** | 207.13 | 109.31 | 514.97 | 192.34 |
| **Fas/TNFRSF6** | 4,740.63 | 1,570.79 | 1,026.94 | 575.87 |
| **FGF-4** | 342.13 | 141.50 | 156.21 | 88.77 |
| **FGF-9** | 267.13 | 166.98 | 140.69 | 100.07 |
| **GCSF** | 320.63 | 151.78 | 152.33 | 115.25 |
| **GITR-Ligand** | 183.63 | 112.44 | 170.17 | 81.77 |
| **GITR** | 211.13 | 116.91 | 63.51 | 18.30 |
| **GRO** | 10,097.13 | 9,584.54 | 2,380.16 | 1,317.60 |
| **GRO-alpha** | 1,061.13 | 754.43 | 356.73 | 124.60 |
| **HCC-4** | 90.63 | 18.55 | 67.00 | 8.18 |
| **HGF** | 1.00 | 0.89 | 265.19 | 333.29 |
| **ICAM-1** | 11,257.63 | 6,308.40 | 2,116.03 | 1,415.33 |
| **ICAM**-**3** | 75.13 | 19.89 | 54.98 | 61.52 |
| **IGFBP-3** | 603.63 | 406.61 | 435.46 | 346.92 |
| **IGFBP-6** | 523.63 | 334.63 | 253.56 | 176.77 |
| **IGF-I SR** | 929.13 | 526.87 | 484.72 | 463.73 |
| **IL-1 R4/ST2** | 330.63 | 297.97 | 272.95 | 171.71 |
| **IL-1 RI** | 57.13 | 40.91 | 40.24 | 16.74 |
| **IL-11** | 192.13 | 106.63 | 79.80 | 19.86 |
| **IL-12 p40** | 134.13 | 113.33 | 99.19 | 93.06 |
| **IL**-**12 p70** | 183.63 | 165.64 | 166.29 | 85.27 |
| **IL-17** | 47.13 | 0.89 | 0.78 | 0.78 |
| **IL-2 Rapha** | 229.13 | 158.04 | 115.87 | 36.21 |
| **IL-6 R** | 158.63 | 30.62 | 55.75 | 46.72 |
| **IL**-**8** | 2,792.13 | 2,894.57 | 528.93 | 164.70 |
| **I-TAC** | 499.63 | 184.42 | 139.53 | 89.16 |
| **Lymphotactin** | 581.63 | 277.86 | 295.45 | 215.71 |
| **MIF** | 2,280.63 | 1,794.32 | 1,532.31 | 1,042.71 |
| **MIP-1alpha** | 557.13 | 448.19 | 319.49 | 261.65 |
| **MIP-1beta** | 193.13 | 133.45 | 182.58 | 225.44 |
| **MIP**-**3beta** | 360.13 | 203.19 | 126.34 | 101.23 |
| **MSP**-**alpha** | 251.13 | 174.13 | 121.69 | 89.94 |
| **NT-4** | 115.13 | 27.94 | 2.62 | 0.78 |
| **Osteoprotegerin** | 2,391.13 | 1,761.24 | 1,558.30 | 1,582.37 |
| **Oncostatin M** | 541.13 | 340.45 | 179.48 | 98.51 |
| **PIGF** | 88.13 | 50.74 | 40.24 | 11.29 |
| **sgp130** | 604.13 | 503.63 | 369.92 | 379.24 |
| **sTNF RII** | 17.63 | 0.89 | 0.78 | 8.57 |
| **sTNF-RI** | 903.63 | 551.91 | 685.63 | 469.18 |
| **TECK** | 255.63 | 91.87 | 140.69 | 100.46 |
| **TIMP-1** | 1,099.63 | 732.53 | 661.58 | 832.85 |
| **TIMP-2** | 11,468.63 | 7,205.68 | 14,022.74 | 14,546.57 |
| **Thrombopoietin** | 580.13 | 364.59 | 405.21 | 281.90 |
| **TRAIL R3** | 1,881.13 | 728.50 | 988.93 | 1,186.39 |
| **TRAIL R4** | 437.13 | 412.42 | 172.89 | 233.23 |
| **uPAR** | 1,055.13 | 631.49 | 668.56 | 960.95 |
| **VEGF** | 418.13 | 293.95 | 290.79 | 452.44 |
| **VEGF-D** | 39.13 | 0.89 | 20.85 | 12.46 |
| **POS** | 17,605.50 | 17,898.76 | 18,624.45 | 19,358.48 |
| **NEG** | 1.00 | 1.04 | 1.02 | 1.09 |
| **Activin A** | 7.00 | 106.51 | 131.02 | 216.38 |
| **ALCAM** | 227.00 | 372.00 | 229.19 | 194.01 |
| **B7-1(CD80)** | 18.00 | 138.72 | 100.34 | 3.55 |
| **BMP-5** | 42.50 | 129.37 | 90.63 | 42.29 |
| **BMP-7** | 72.00 | 136.12 | 111.59 | 1.09 |
| **Cardiotrophin-1** | 578.00 | 1,221.99 | 498.65 | 233.30 |
| **CD14** | 118.00 | 231.72 | 134.09 | 180.37 |
| **CXCL**-**16** | 1.00 | 187.56 | 201.07 | 175.45 |
| **DR6 (TNFRSF21)** | 107.00 | 382.39 | 190.84 | 1.09 |
| **Endoglin** | 1,213.50 | 735.17 | 1,168.45 | 1,359.16 |
| **ErbB3** | 183.50 | 269.13 | 226.63 | 167.81 |
| **E-Selectin** | 150.00 | 250.94 | 178.06 | 82.68 |
| **Fas Ligand** | 134.00 | 269.65 | 162.72 | 88.14 |
| **ICAM-2** | 364.50 | 416.16 | 273.16 | 203.83 |
| **IGF-II** | 172.00 | 206.26 | 140.22 | 89.77 |
| **IL-1 R II** | 178.00 | 226.52 | 190.33 | 124.70 |
| **IL-10 Rbeta** | 124.50 | 111.18 | 91.14 | 194.56 |
| **IL**-**13 Ralpha2** | 94.50 | 117.94 | 80.40 | 1.09 |
| **IL**-**18 BPalpha** | 3.00 | 132.49 | 234.82 | 1.09 |
| **IL-18 Rbeta** | 59.50 | 191.71 | 112.61 | 1.09 |
| **IL**-**2 Ralpha** | 128.00 | 156.90 | 149.94 | 50.48 |
| **IL**-**2 Rbeta** | 1.00 | 57.15 | 196.98 | 1.09 |
| **IL**-**2 Rgamma** | 1.00 | 1.04 | 1.02 | 1.09 |
| **IL**-**21R** | 153.50 | 166.78 | 146.36 | 70.67 |
| **IL**-**5 Ralpha** | 133.00 | 166.78 | 150.96 | 21.01 |
| **IL-9** | 169.00 | 184.96 | 155.56 | 57.58 |
| **IP-10** | 91.50 | 211.46 | 161.19 | 94.14 |
| **LAP** | 3,066.00 | 2,460.08 | 5,775.28 | 6,813.80 |
| **Leptin R** | 225.50 | 223.41 | 168.35 | 230.03 |
| **LIF** | 93.50 | 181.84 | 495.07 | 217.48 |
| **L**-**Selectin** | 87.50 | 103.91 | 122.84 | 1.09 |
| **M-CSF R** | 1.00 | 193.27 | 65.57 | 167.27 |
| **MMP-1** | 206.50 | 170.93 | 135.11 | 379.56 |
| **MMP-13** | 158.50 | 149.11 | 119.26 | 57.58 |
| **MMP-9** | 1.00 | 137.16 | 56.37 | 167.81 |
| **MPIF-1** | 1.00 | 291.47 | 1.02 | 1.09 |
| **NGF R** | 1.00 | 123.13 | 388.21 | 1.09 |
| **PDGF AA** | 331.50 | 311.21 | 470.01 | 493.62 |
| **PDGF-AB** | 48.50 | 111.70 | 137.67 | 248.58 |
| **PDGF Ralpha** | 1.00 | 184.96 | 174.48 | 1.09 |
| **PDGF Rbeta** | 1,199.50 | 859.86 | 3,513.79 | 4,030.00 |
| **PECAM-1** | 41.50 | 138.72 | 280.32 | 190.74 |
| **Prolactin** | 285.00 | 272.77 | 195.44 | 59.76 |
| **SCFR** | 64.50 | 245.75 | 128.46 | 88.68 |
| **SDF-1beta** | 425.50 | 364.73 | 248.11 | 452.14 |
| **Siglec**-**5** | 152.50 | 153.79 | 1.02 | 1.09 |
| **TGF**-**alpha** | 69.50 | 227.04 | 140.74 | 166.72 |
| **TGF beta2** | 173.50 | 138.72 | 182.15 | 118.70 |
| **Tie**-**1** | 8.50 | 124.69 | 373.89 | 101.78 |
| **Tie-2** | 153.00 | 90.92 | 231.24 | 18.28 |
| **TIMP-4** | 49.50 | 97.68 | 93.70 | 17.74 |
| **VE-Cadherin** | 77.00 | 139.24 | 131.02 | 85.41 |
| **VEGF R2** | 59.50 | 164.70 | 101.37 | 78.86 |
| **VEGF R3** | 37.50 | 121.06 | 222.54 | 54.85 |

**TABLE XI: PROTEINS CAPABLE OF DETECTION BY ANTIBODY ARRAYS**

| **Cytokine** | **Full Name** |
|---|---|
| **Activin A** | EDF(erythroid differentiation factor), FRP(Follicle stimulating hormone releasing protein), Restrictin-P, WEHI-MIF(WEHI mesoderm inducing factor) |
| **AGRP** | Agouti related protein |
| **ALCAM** | Activated leukocyte cell adhesion molecule |
| **Amphiregulin** | Amphiregulin |
| **ANG** | Angiogenin |
| **Angiopoietin-1** | Angiopoietin-1 |
| **Angiopoietin-like Factor** | Angiopoietin-like Factor |
| **Angiostatin** | Angiostatin |
| **APRIL** | A proliferation-inducing ligand |
| **AXL** | A protein tyrosine kinase also called UFO or ark. The ligand for this receptor is GPF (growth-potentiating factor). The ligand is called also axl stimulatory factor,which is identical with one of the gas genes, gas-6. |
| **B7**-**1(CD80)** | B7-1(CD80) |
| **BAFF** | B-cell-activating factor belonging to the TNF family |
| **BDNF** | Brain-derived neurotrophic factor |
| **Betacellulin** | Betacellulin |
| **beta**-**Galactosidase** | beta-Galactosidase |
| **beta-NGF** | Nerve growth factor-beta |
| **bFGF** | Basic fibroblast growth factor |
| **BLC** | B-lymphocyte chemoattractant |
| **BMP**-**2** | Bone morphogenetic proteins -2 |
| **BMP**-**4** | Bone morphogenetic proteins -4 |
| **BMP**-**5** | Bone morphogenetic proteins -5 |
| **BMP-6** | Bone morphogenetic proteins -6 |
| **BMP**-**7** | Bone morphogenetic proteins -7 |
| **BTC** | Betacellulin |
| **CCL28** | CCK-1 |
| **CD27** | Cluster of Differentiation 27 |
| **CD30** | Cluster of Differentiation 30 |
| **CD40** | Cluster of Differentiation 40 |
| **CD40 Ligand** | Cluster of Differentiation 40 ligand |
| **Ck beta 8-1** | Chemokine-beta-8 |
| **CNTF** | Ciliary neuronotrophic factor, ciliary neurotrophic factor |
| **Cripto-1** | CRGF (Cripto growth factor) |
| **CTACK** | CTAC (Cuteaneous T-Cell Attracting Chemokine); Skinkine; Eskine |
| **CTLA-4** | Cytotoxic T-lymphocyte associated antigen 4 |
| **CXCL16** | CXC Chemokine ligand 16 |
| **Dkk**-**4** | Dickkopf-4 |
| **DR6** | Death Receptor 6 |
| **Dtk** | Developmental tyrosine kinase |
| **EGF** | Epidermal growth factor |
| **EGR-R** | Epidermal growth factor related protein |
| **ENA-78** | Epithelial neutrophil-activating protein 78, epithelial cellderived neutrophil attractant-78 |
| **Endostatin** | Endostatin |
| **Eotaxin** | Eotaxin |
| **Eotaxin-2** | MPIF-2 (Myeloid progenitor inhibitory factor-2 ),Chemokine-beta-6 |
| **Eotaxin**-**3** | MIP-4-alpha (macrophage inflammatory protein-4-alpha), TSC-1 (thymic stroma chemokine-1) |
| **Erythropoietin** | Erythropoietin |
| **E-Selectin** | E-Selectin |
| **Fas Ligand** | FasL, CD95 ligand, Apo-1 ligand |
| Fas/TNFRSF6 | **Fas or cd95** |
| **FGF-2** | Fibroblast growth factor-2 |
| **FGF**-**4** | Fibroblast growth factor-4 |
| **FGF**-**6** | Fibroblast growth factor-6 |
| **FGF**-**7** | Fibroblast growth factor-7 |
| **FGF**-**8** | Fibroblast growth factor-8 |
| **FGF**-**9** | Fibroblast growth factor-9 |
| FKN or FK | **Fractalkine** |
| **Flt**-**3 Ligand** | fms-like tyrosine kinase-3 Ligand (also known as STK-1 Ligand) |
| **Follistatin** | Follicle stimulating hormone suppressing protein (FSP) |
| **GCP**-**2** | Granulocyte Chemotactic Protein 2 |
| **GCSF** | Granulocyte-colony Stimulating Factor |
| **GDF-15** | growth/differentiation factor-15 |
| **GDNF** | Glial-derived Neurotrophic Factor |
| **GITR** | Glucocorticoid induced tumor necrosis factor receptor |
| **GITR**-**Ligand** | Glucocorticoid induced tumor necrosis factor receptor |
| **GM**-**CSF** | Granulocyte-macrophage colony stimulating factor |
| **GRO** | Growth Related Oncogene |
| | |
| **GRO**-**a** | Growth Related Oncogene-Alpha |
| **HB**-**EGF** | Heparin-binding Epidermal Growth factor |
| **HCC**-**4** | Hemofiltrate CC Chemokine 4 |
| **HGF** | Hepatocyte growth factor |
| **HPTA** | Hepatopoeitin A |
| **HRG** | Heregulin/NDF/GGF/Neuregulin |
| **HRG-alpha** | Heregulin/NDF/GGF/Neuregulin |
| **HRG-beta** | Heregulin/NDF/GGF/Neuregulin |
| **HVEM** | Herpesvirus entry mediator |
| **I**-**309** | I-309 |
| **ICAM**-**1** | Intercellular Adhesion Molecule 1 |
| **ICAM-2** | Intercellular Adhesion Molecule 2 |
| **ICAM**-**3** | Intercellular Adhesion Molecule 3 |
| **IFN-alpha** | Interferon alpha |
| **IFN**-**beta** | Interferon beta |
| **IFN**-**gamma** | Interferon gamma |
| **IFN**-**omega** | Interferon omega |
| **IGFBP-1** | Insulin-like growth factor binding proteins 1 |
| **IGFBP-2** | Insulin-like growth factor binding proteins 2 |
| **IGFBP-3** | Insulin-like growth factor binding proteins 3 |
| **IGFBP-4** | Insulin-like growth factor binding proteins 4 |
| **IGFBP**-**6** | Insulin-like growth factor binding proteins 6 |
| **IGF**-**I SR** | Insulin like growth factor I soluble receptor |
| **IGF**-**I** | Insulin-like growth factor-1 |
| **IGF**-**II** | Insulin-like growth factor II |
| **IL**-**1 R1** | Interleukin 1 receptor-related protein-1 |
| **IL**-**1 R4/ST2** | Interleukin 1 receptor 4, also known as ST2 |
| **IL**-**10** | Interleukin 10 |
| **IL**-**10 R alpha** | Interleukin 10 soluble receptor alpha |
| **IL**-**10 R beta** | Interleukin 10 receptor beta |
| **IL-11** | Interleukin 11 |
| **IL-12** | Interleukin 12 |
| **IL**-**12 p40** | Interleukin 12p40 |
| **IL12p70** | Interleukin 12p70 |
| **IL**-**13** | Interleukin 13 |
| **IL**-**15** | Interleukin 15 |
| **IL-16** | Interleukin 16 |
| **IL**-**17** | Interleukin 17 |
| **IL**-**18 BPa** | Interleukin 18 binding protein A |
| **IL**-**18 R alpha** | Interleukin 18 receptor alpha |
| **IL**-**18 R beta** | Interleukin 18 receptor beta |
| **IL**-**18** | Interleukin 18 |
| **IL-1a** | Interleukin I alpha |
| **IL**-**1b** | Interleukin I beta |
| **IL-1ra** | Interleukin 1 receptor antagonist |
| **IL**-**2** | Interleukin 2 |
| **IL**-**2 R alpha** | Interleukin 2 soluble receptor alpha |
| **IL-2 R beta** | Interleukin 2 soluble receptor beta |
| **IL-2 R gamma** | Interleukin 2 receptor gamma |
| **IL**-**21 R** | Interleukin 21 receptor |
| **IL**-**3** | Interleukin 3 |
| **IL-4** | Interleukin 4 |
| **IL**-**5** | Interleukin 5 |
| **IL**-**5 R alpha** | Interleukin 5 receptor alpha |
| **IL**-**6** | Interleukin 6 |
| **IL**-**7** | Interleukin 7 |
| **IL-8** | Interleukin 8 |
| **IL-9** | Interleukin 9 |
| **IL**-**9 R** | Interleukin 9 receptor |
| **Inhibin A** | Inhibin A |
| **Inhibin B** | Inhibin B |
| **IP**-**10** | IFN - inducible Protein 10, Immune Protein 10 |
| **I-TAC** | Interferon-inducible T cell Alpha Chemoattractant |
| **LAP** | Liver activator protein |
| **TGF-LAP** | Tumor growth factor - latency associated peptide |
| **LT-BP-1** | Latent TGF-beta binding protein 1 |
| **LECT2** | Leukocyte cell-derived chemotaxin-2 |
| **LEPTIN R** | LEPTIN receptor |
| **LFA**-**1 alpha** | Lymphocyte function-associated antigen 1 alpha |
| **LIF** | Leukemia Inhibitoty Factor |
| **LIGHT** | LIGHT |
| **LIX** | LPS induced C-X-C chemokine |
| **L**-**Selectin** | L-Selectin |
| **Lymphotactin** | Lymphotactin |
| **MAC-1** | Macrophage galactose-specific lectin-1 |
| **MMP**-**13** | Matrix Metalloproteinase 13 |
| **MMP**-**19** | Matrix Metalloproteinase 19 |
| **MCP**-**1** | Monocyte Chemoattractant Protein 1 |
| **MCP**-**2** | Monocyte Chemoattractant Protein 2 |
| **MCP**-**3** | Monocyte Chemoattractant Protein 3 |
| **MCP-4** | Monocyte Chemoattractant Protein 4 |
| **MCSF** | Macrophage-colony Stimuating Factor |
| **M-CSF R** | Macrophage colony stimulating factor receptor |
| **MDC** | Macrophage-derived Chemokine |
| **MIF** | Macrophage Migration Inhibitory Factor |
| **MIG** | Monokine induced by gamma interferon |
| **MIP-1a** | Macrophage Inflammatory Protein 1 alpha |
| **MIP-1b** | Macrophage Inflammatory Protein 1 beta |
| **MIP-1d** | Macrophage Inflammatory Protein 1delta |
| **MIP-2** | Macrophage Inflammatory Protein 2 |
| **MIP-3 alpha** | Macrophage Inflammatory Protein 3 alpha |
| **MIP-3 beta** | Macrophage Inflammatory Protein 3 beta |
| **MMP**-**1** | Matrix Metalloproteinase 1 |
| **MMP-10** | Matrix Metalloproteinase 10 |
| **MMP**-**11 (Stromelysin-3)** | Matrix Metalloproteinase 11 |
| **MMP**-**12** | Matrix Metalloproteinase 12 |
| **MMP**-**13** | Matrix Metalloproteinase 13 |
| **MMP-14** | Matrix Metalloproteinase 14 |
| **MMP**-**2** | Matrix Metalloproteinase 2 |
| **MMP**-**20** | Matrix Metalloproteinase 20 |
| **MMP**-**3** | Matrix metalloproteinase 3 |
| **MMP**-**7** | Matrix Metalloproteinase 7 |
| **MMP**-**8** | Matrix Metalloproteinase 8 |
| **MMP**-**9** | Matrix Metalloproteinase 9 |
| **MPIF**-**1** | Myeloid Progenitor Inhibitory Factor 1 |
| **MSP a**-**chain** | Macrophage stimulating protein a-chain |
| **MSP b-chain** | Macrophage stimulating protein b-chain |
| **NAP-2** | Neutrophil Activating Peptide 2 |
| **NGF** | Nerve growth factor |
| **NGF R** | Nerve growth factor receptor |
| **NT-3** | Neurotrophin-4 |
| **NT-4** | Neurotrophin-3 |
| **OPG** | Osteoprotegerin |
| **OSM** | Oncostatin M |
| **PARC** | Pulmonary and Activation-Regulated Chemokine |
| **PDGF R alpha** | Platelet-derived growth factor receptor alpha |
| | |
| **PDGF R beta** | Platelet-derived growth factor receptor beta |
| **PDGF**-**AA** | Platelet-derived Growth Factor AA |
| **PDGF-BB** | Platelet-derived Growth Factor BB |
| **PECAM-1** | Platelet endothelial cell adhesion molecule |
| **PF4** | Platelet factor 4 |
| **PIGF** | Placenta growth Factor |
| **Prolactin** | Prolactin |
| **P-selectin** | P-selectin |
| **RANTES** | Regulated upon activation, normal T-cell expressed, and presumably secreted |
| **SCF** | Stem Call Factor |
| **SCF R** | Stem cell factor receptor |
| **SDF-1** | Stromal cell-derived factor |
| **SLPI** | Secretory leukocyte protease inhibitor |
| **SMDF** | Sensory and Motor Neuron-derived Factor |
| **SPARC** | Osteonectin |
| **ST2** | Interleukin 1 receptor 4 |
| **TARC** | Thymus and Activation-Regulated Chemokine |
| **TECK** | Thymus-expressed Chemokine |
| **TGFa** | Tumor Necrosis Factor Alpha |
| **TGF**-**a** | Transforming growth factor alpha |
| **TGF**-**beta 1** | Tumor Necrosis Facyor beta1 |
| **TGF**-**beta 2** | Tumor Necrosis Facyor beta2 |
| **TGF**-**beta 3** | Tumor Necrosis Facyor beta3 |
| **Thrombospondin (TSP)** | Thrombospondin |
| **Thymopoietin** | Thymopoietin (Tpo) |
| **Tie**-**1** | Tyrosine kinase with Ig and EGF homology domains 1 |
| **Tie**-**2** | Tyrosine kinase with immunoglobulin and epidermal growth factor homology domain 2 |
| **TIMP-1** | Tissue inhibitor of metalloproteinases-1 |
| **TIMP-2** | Tissue inhibitor of metalloproteinases-2 |
| **TIMP-3** | Tissue inhibitor of metalloproteinase-3 |
| **TIMP-3** | Tissue inhibitor of metalloproteinases-3 |
| **TIMP-4** | Tissue inhibitor of metalloproteinase-4 |
| **TNF-a** | Tumor necrosis factor-alpha |
| **TNF-b** | Tumor necrosis factor-beta |
| **TPO** | Thrombopoietin |
| **TRAIL R1** | TNF-related apoptosis-inducing ligand receptor 1 |
| **TRAIL R2** | TNF-related apoptosis-inducing ligand receptor 2 |
| **TRAIL** | TNF-related apoptosis inducing ligand |
| **TRANCE** | Tumor necrosis factor-related activation-induced cytokine |
| **TSLP** | Thymic stromal derived lymphopoietin |
| **uPA** | Urokinase plasminogen activator Ab |
| **uPAR** | Urokinase plasminogen activator receptor |
| **VCAM-1** | Vascular cell adhesion molecule (VCAM-1, CD106, or INCAM-110) |
| **VE-Cadherin** | Vascular epithelial cadherin |
| **VEGF** | Vascular Endothelial Growth Factor |
| **VEGF R2** | Vascular endothelial growth factor receptor 2 |
| **VEGF R3** | Vascular endothelial growth factor receptor 3 |
| **VEGF-B** | Vascular Endothelial Growth Factor B |
| **VEGF**-**C** | Vascular Endothelial Growth Factor C |
| **VEGF**-**D** | Vascular Endothelial Growth Factor D |

## Claims

1. A substantially pure population of amniotic fluid-derived cells, wherein said cells are:
capable of differentiating into cells displaying the characteristics of the beta-cell lineage,
substantially negative for the expression of the CD117 and Oct-4 protein markers, wherein the markers are not present or expressed in at least 70% of the total cell population,
substantially positive for the expression of GATA-6 and SSEA-4, wherein
GATA-6 and SSEA-4 are present or expressed in at least about 50% of the total cell population, and
substantially either
i) positive for the expression of SOX-17 and cytokeratin, wherein SOX-17 and cytokeratin are present or expressed in at least about 50% of the total cell population;
ii) negative for the expression of SOX-17, wherein SOX-17 is not present or expressed in at least 70% of the total cell population, and positive in the expression of cytokeratin, wherein cytokeratin is present or expressed in at least about 50% of the total cell population; or
iii) negative for the expression of SOX-17 and cytokeratin, wherein SOX-17 and cytokeratin are not present or expressed in at least 70% of the total cell population.

2. The population of amniotic fluid-derived cells according to claim 1, wherein said cells are
substantially positive for the expression of SOX-17 and cytokeratin, wherein SOX-17 and cytokeratin are present or expressed in at least about 50% of the total cell population, and
substantially positive for the expression of the HNF-1 beta, HNF-3 beta and GATA-6 protein markers, wherein the markers are present or expressed in at least about 50% of the total cell population.

3. The population of amniotic fluid-derived cells according to claim 1, wherein said cells are substantially
negative for the expression of SOX-17, wherein SOX-17 is not present or expressed in at least 70% of the total cell population, and positive in the expression of cytokeratin, wherein cytokeratin is present or expressed in at least about 50% of the total cell population, and
do not express the markers HNF-3 beta and GATA-4.

4. The population of amniotic fluid-derived cells according to claim 3, wherein said cells do not express any of the markers HNF-3 beta, GATA-4 or Tra2-54.

5. The population of amniotic fluid-derived cells according to claim 1, wherein said cells are substantially
negative for the expression of SOX-17 and cytokeratin, wherein SOX-17 and cytokeratin are not present or expressed in at least about 70% of the total cell population, and
do not express the markers HNF-3 beta and GATA-4.

6. The population of amniotic fluid-derived cells according to claim 5, wherein said cells do not express any of the markers HNF-3 beta, GATA-4 or Tra2-54.

7. The population of amniotic fluid-derived cells according to any of the preceding claims, wherein said cells are substantially positive for the expression of the HES-1 gene, wherein HES-1 is present or expressed in at least about 50% of the total cell population.

8. The population of amniotic fluid-derived cells according to any of the preceding claims, capable of propagating in vitro.

9. The population of amniotic fluid-derived cells according to any of the preceding claims, capable of propagating in vitro under hypoxic conditions.

10. The population of amniotic fluid-derived cells according to any of the preceding claims, capable of differentiating into a gut hormone-producing cell.

11. A population of amniotic fluid-derived cells as defined in any one of claims 1 to 10, for use in a method of treating a patient with diabetes mellitus or at risk of developing diabetes.

12. In vitro use of the population of cells according to any one of claims 1 to 10, in a method wherein the cells of said population are differentiated into pancreatic hormone producing cells.

## Patentansprüche

1. Im Wesentlichen reine Population von aus Fruchtwasser gewonnenen Zellen, wobei die Zellen:
dazu fähig sind, zu Zellen zu differenzieren, die die Charakteristika der beta-Zelle-Linie zeigen,
im Wesentlichen negativ für die Expression der CD117- und Oct-4-Proteinmarker sind, wobei die Marker in mindestens 70% der Zellgesamtpopulation nicht vorhanden sind bzw. exprimiert werden,
im Wesentlichen positiv für die Expression von GATA-6 und SSEA-4 sind, wobei GATA-6 und SSEA-4 in mindestens etwa 50% der Zellgesamtpopulation vorhanden sind bzw. exprimiert werden, und
im Wesentlichen entweder
i) positiv für die Expression von SOX-17 und Cytokeratin sind, wobei SOX-17 und Cytokeratin in mindestens etwa 50% der Zellgesamtpopulation vorhanden sind oder exprimiert werden,
ii) negativ für die Expression von SOX-17 sind, wobei SOX-17 in mindestens 70% der Zellgesamtpopulation nicht vorhanden ist bzw. exprimiert wird, und positiv für die Expression von Cytokeratin sind, wobei Cytokeratin in mindestens etwa 50% der Zellgesamtpopulation vorhanden ist bzw. exprimiert wird, oder
iii) negativ für die Expression von SOX-17 und Cytokeratin sind, wobei SOX-17 und Cytokeratin in mindestens 70% der Zellgesamtpopulation nicht vorhanden sind bzw. exprimiert werden.

2. Population von aus Fruchtwasser gewonnenen Zellen nach Anspruch 1, wobei die Zellen
im Wesentlichen positiv für die Expression von SOX-17 und Cytokeratin sind, wobei SOX-17 und Cytokeratin in mindestens etwa 50% der Zellgesamtpopulation vorhanden sind bzw. exprimiert werden, und
im Wesentlichen positiv für die Expression von HNF-1-beta-, HNF-3-beta- und GATA-6-Proteinmarkern sind, wobei die Marker in mindestens etwa 50% der Zellgesamtpopulation vorhanden sind bzw. exprimiert werden.

3. Population von aus Fruchtwasser gewonnenen Zellen nach Anspruch 1, wobei die Zellen im Wesentlichen
negativ für die Expression von SOX-17 sind, wobei SOX-17 in mindestens 70% der Zellgesamtpopulation nicht vorhanden ist bzw. exprimiert wird, und positiv für die Expression von Cytokeratin sind, wobei Cytokeratin in mindestens etwa 50% der Zellgesamtpopulation vorhanden ist bzw. exprimiert wird, und
die Marker HNF-3-beta und GATA-4 nicht exprimieren.

4. Population von aus Fruchtwasser gewonnenen Zellen nach Anspruch 3, wobei die Zellen keinen der Marker HNF-3-beta, GATA-4 bzw. Tra2-54 exprimieren.

5. Population von aus Fruchtwasser gewonnenen Zellen nach Anspruch 1, wobei die Zellen im Wesentlichen
negativ für die Expression von SOX-17 und Cytokeratin sind, wobei SOX-17 und Cytokeratin in mindestens etwa 70% der Zellgesamtpopulation nicht vorhanden sind bzw. exprimiert werden, und
die Marker HNF-3-beta und GATA-4 nicht exprimieren.

6. Population von aus Fruchtwasser gewonnenen Zellen nach Anspruch 5, wobei die Zellen keinen der Marker HNF-3-beta, GATA-4 bzw. Tra2-54 exprimieren.

7. Population von aus Fruchtwasser gewonnenen Zellen nach einem der vorhergehenden Ansprüche, wobei die Zellen im Wesentlichen positiv für die Expression des HES-1-Gens sind, wobei HES-1 in mindestens etwa 50% der Zellgesamtpopulation vorhanden ist bzw. exprimiert wird.

8. Population von aus Fruchtwasser gewonnenen Zellen nach einem der vorhergehenden Ansprüche, fähig zur in-vitro-Fortpflanzung.

9. Population von aus Fruchtwasser gewonnenen Zellen nach einem der vorhergehenden Ansprüche, fähig zur in-vitro-Fortpflanzung unter hypoxischen Bedingungen.

10. Population von aus Fruchtwasser gewonnenen Zellen nach einem der vorhergehenden Ansprüche, fähig zur Differenzierung zu einer hormonproduzierenden Eingeweidezelle.

11. Population von aus Fruchtwasser gewonnenen Zellen nach einem der Ansprüche 1 bis 10 zur Verwendung in einem Verfahren zur Behandlung eines Patienten mit Diabetes mellitus bzw. eines Patienten, bei dem ein Risiko des Auftretens von Diabetes besteht.

12. In-vitro-Anwendung der Population von Zellen nach einem der Ansprüche 1 bis 10 in einem Verfahren, bei dem die Zellen der Population zu hormonproduzierenden Bauchspeicheldrüsenzellen differenziert werden.

## Revendications

1. Population sensiblement pure de cellules dérivées de liquide amniotique, dans laquelle lesdites cellules sont :
capables de se différencier en cellules présentant les caractéristiques du lignage de cellules bêta,
sensiblement négatives pour l'expression des marqueurs protéiques CD117 et Oct-4, où les marqueurs ne sont pas présents ou exprimés dans au moins 70 % de la population totale de cellules,
sensiblement positives pour l'expression de GATA-6 et SSEA-4, où GATA-6 et SSEA-4 sont présents ou exprimés dans au moins environ 50 % de la population totale de cellules, et sensiblement
i) positives pour l'expression de SOX-17 et de cytokératine, où SOX-17 et la cytokératine sont présents ou exprimés dans au moins environ 50 % de la population totale de cellules ;
ii) négatives pour l'expression de SOX-17, où SOX-17 n'est pas présent ou exprimé dans au moins 70 % de la population totale de cellules, et positives pour l'expression de cytokératine, où la cytokératine est présente ou exprimée dans au moins environ 50 % de la population totale de cellules ; ou
iii) négatives pour l'expression de SOX-17 et de cytokératine, où SOX-17 et la cytokératine ne sont pas présents ou exprimés dans au moins 70 % de la population totale de cellules.

2. Population de cellules dérivées de liquide amniotique selon la revendication 1, dans laquelle lesdites cellules sont
sensiblement positives pour l'expression de SOX-17 et de cytokératine, où SOX-17 et la cytokératine sont présents ou exprimés dans au moins environ 50 % de la population totale de cellules, et
sensiblement positives pour l'expression des marqueurs protéiques HNF-1 bêta, HNF-3 bêta et GATA-6, où les marqueurs sont présents ou exprimés dans au moins environ 50 % de la population totale de cellules.

3. Population de cellules dérivées de liquide amniotique selon la revendication 1, dans laquelle lesdites cellules sont sensiblement
négatives pour l'expression de SOX-17, où SOX-17 n'est pas présent ou exprimé dans au moins 70 % de la population totale de cellules, et positives dans l'expression de cytokératine, où la cytokératine est présente ou exprimée dans au moins environ 50 % de la population totale de cellules, et
n'expriment pas les marqueurs HNF-3 bêta et GATA-4.

4. Population de cellules dérivées de liquide amniotique selon la revendication 3, dans laquelle
lesdites cellules n'expriment aucun des marqueurs HNF-3 bêta, GATA-4 ou Tra2-54.

5. Population de cellules dérivées de liquide amniotique selon la revendication 1, dans laquelle lesdites cellules sont sensiblement
négatives pour l'expression de SOX-17 et de cytokératine, où SOX-17 et la cytokératine ne sont pas présents ou exprimés dans au moins environ 70 % de la population totale de cellules, et
n'expriment pas les marqueurs HNF-3 bêta et GATA-4.

6. Population de cellules dérivées de liquide amniotique selon la revendication 5, dans laquelle lesdites cellules n'expriment aucun des marqueurs HNF-3 bêta, GATA-4 ou Tra2-54.

7. Population de cellules dérivées de liquide amniotique selon l'une quelconque des revendications précédentes, dans laquelle lesdites cellules sont sensiblement positives pour l'expression du gène HES-1, où HES-1 est présent ou exprimé dans au moins environ 50 % de la population totale de cellules.

8. Population de cellules dérivées de liquide amniotique selon l'une quelconque des revendications précédentes, capable de se propager *in vitro.*

9. Population de cellules dérivées de liquide amniotique selon l'une quelconque des revendications précédentes, capable de se propager *in vitro* dans des conditions hypoxiques.

10. Population de cellules dérivées de liquide amniotique selon l'une quelconque des revendications précédentes, capable de se différencier en cellule productrice d'hormone intestinale.

11. Population de cellules dérivées de liquide amniotique telle que définie dans l'une quelconque des revendications 1 à 10, pour utilisation dans un procédé de traitement d'un patient ayant un diabète sucré ou à risque de développer un diabète.

12. Utilisation *in vitro* de la population de cellules selon l'une quelconque des revendications 1 à 10, dans un procédé dans lequel les cellules de ladite population sont différenciées en cellules productrices d'hormone pancréatiques.
